# EUROPEAN PATENT APPLICATION

(11) **EP 4 303 216 A1**
(43) Date of publication of application: **10.01.2024**
(21) Application number: 22762614.0
(22) Date of filing: 03.03.2022
(51) Int. Cl.: C07D 401/12, C07D 401/14, C07D 403/14, C07D 405/14, C07D 471/00, C07D 471/04, C07D 487/00, C07D 487/04, C07D 498/04, A61K 31/437, A61K 31/444, A61K 31/517, A61K 31/519, A61K 31/551, A61K 31/554, A61P 35/00

(54) **FUSED RING SUBSTITUTED SIX-MEMBERED HETEROCYCLIC COMPOUND, PREPARATION METHOD THEREFOR AND USE THEREOF**

(30) Priority: 03.03.2021 CN 202110234333; 18.05.2021 CN 202110542264; 10.09.2021 CN 202111062604; 04.01.2022 CN 202210004944
(71) Applicant: Genfleet Therapeutics (Shanghai) Inc., Shanghai 201203 (CN); Zhejiang Genfleet Therapeutics Co., Ltd., Shaoxing Binhai New City, Zhejiang 312000 (CN)
(72) Inventor: ZHOU, Fusheng, Shanghai 201203 (CN); ZHAO, Jichen, Shanghai 201203 (CN); HE, Wan, Shanghai 201203 (CN); LIN, Chonglan, Shanghai 201203 (CN); PENG, Ling, Shanghai 201203 (CN); YANG, Huabin, Shanghai 201203 (CN); LI, Zhen, Shanghai 201203 (CN); LAN, Xiaoling, Shanghai 201203 (CN); GE, Mei, Shanghai 201203 (CN); DING, Qian, Shanghai 201203 (CN); YU, Xiang, Shanghai 201203 (CN); LAN, Jiong, Shanghai 201203 (CN); LU, Qiang, Shanghai 201203 (CN)
(74) Representative: Elzaburu S.L.P.
(86) International application number: PCT/CN2022/079153
(87) International publication number: WO 2022/184152

(57) **Abstract**

A compound having HPK1 inhibitory activity as shown in formula (IA) or (IC), and a pharmaceutically acceptable salt, a stereoisomer, a solvate or a prodrug thereof, and a pharmaceutical composition containing the compound, and use of same in preparation of a drug for preventing and/or treating diseases or conditions associated with HPK1 activity.

## Description

### Technical Field

The present disclosure belongs to the field of medicine, and in particular relates to a fused ring substituted six-membered heterocyclic compound, a preparation method therefor and use thereof.

### Background

Hematopoietic progenitor kinase 1 (HPK1, also known as MAP4K1) is a hematopoietic-specific serine/threonine protein kinase belonging to the MAP4K family of mammalian ste20-related protein kinases. HPK1 is predominantly expressed in hematopoietic tissues and cells. There are three activation modes of HPK1, namely serine phosphorylation, threonine phosphorylation, or tyrosine phosphorylation. Studies have shown that in vitro HPK1-/-T cells have a low TCR activation threshold, proliferate robustly, and produce more Thl cytokines. In a mouse experimental autoimmune encephalomyelitis (EAE) model, more severe autoimmune symptoms may occur when HPK1-/-mice are immunized with peptides derived from myelin oligodendrocyte glycoprotein (MOG). Furthermore, in a Lewis lung cancer tumor model that produces PGE2, HPK1-knockout mice develop tumors significantly more slowly than wild-type mice. In addition, a large number of studies have shown that HPK1 can bind to various linker proteins, such as SLP-76 family, CARD11, HIS, HIP-55, GRB2 family, LAT, CRK family, etc., to interact and activate the JNK/SAPK signaling pathway of hematopoietic stem cells, thereby negatively regulating the TCR pathway. Because of the important role of HPK1 in immunity, HPK1 inhibitors play an important role in malignant solid tumors or hematological cancers (e.g. acute myeloid leukemia, urothelial carcinoma, breast cancer, colon cancer, lung cancer, pancreatic cancer, melanoma), autoimmune diseases (e.g. systemic lupus erythematosus, psoriatic arthritis), and inflammatory responses. At present, there is no drug targeting HPK1 on the market. To better meet the enormous clinical demand, we aim to develop more effective HPK1 inhibitors.

### Summary of the Invention

The present disclosure provides an efficient HPK1 inhibitor with a novel structure, which has the advantages of high activity, good selectivity, and low toxicity and side effects, with good physicochemical properties and pharmaceutical properties.

In a first aspect of the present disclosure, provided is a compound of formula (IA), or a pharmaceutically acceptable salt, a stereoisomer, a solvate or a prodrug thereof: 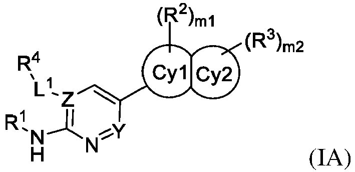 wherein
Y is CH or N;
R¹ is H, C₁₋₆ alkyl, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-NR^{d}-S(=O)₂-R^{c}, -C₁₋₄ alkyl-NR^{d}-S(=O)₂-NR^{a}R^{b}, -C₁₋₄ alkyl-C(=O)-NR^{a}R^{b}, -C₁₋₄ alkyl-S(=O)₂-R^{c}, -C₁₋₄ alkyl-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -S(=O)₂-R^{c}, or -S(=O)₂-NR^{a}R^{b}; wherein the C₁₋₆ alkyl and the C₁₋₆ alkoxy are each independently optionally substituted with halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8-to 10-membered bicyclic heteroaryl each independently contains 1, 2, 3, or 4 heteroatom(s) independently selected from N, O, and S as ring atoms;
Z, L¹ and R⁴ are selected from one of the following combinations:
   (a) Z is N; -L¹-R⁴ is absent;
   (b) Z is C;
L¹ is a bond, -C₁₋₄ alkyl-, -C₃₋₆ monocyclic cycloalkyl-, -O-, -S-, -NH-, -C(=O)-, -S(=O)-, or -S(=O)₂-;
R⁴ is selected from the group consisting of H, halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C₁₋₄ alkyl-hydroxyl, -C₁₋₄ alkyl-cyano, -C₁₋₄ alkyl-carboxyl, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3-to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}; the C₁₋₆ alkyl and the C₁₋₆ alkoxy are each independently optionally substituted with halogen, deuterium, cyano, or hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8-to 10-membered bicyclic heteroaryl each independently contains 1, 2, 3, or 4 heteroatom(s) independently selected from N, O, and S as ring atoms; 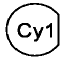 is phenyl or 5- or 6-membered monocyclic heteroaryl; the 5- or 6-membered monocyclic heteroaryl contains 1, 2, or 3 heteroatom(s) independently selected from N, O, and S as ring atoms;
the phenyl or the 5- or 6-membered monocyclic heteroaryl are each independently optionally substituted with m1 R² group(s); m1 is 1, 2, 3, or 4;
the R² is 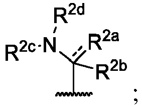 wherein
R^{2a} is H, deuterium, C₁₋₆ alkyl, phenyl, or deuterated C₁₋₆ alkyl, and R^{2b} is H, deuterium, C₁₋₆ alkyl, or deuterated C₁₋₆ alkyl; or R^{2a} and R^{2b} together with the carbon atom to which the R^{2a} and R^{2b} are attached form a C₃₋₆ monocyclic cycloalkyl group; and the bond between R^{2a} and the carbon atom is a single bond; R^{2c} is H, deuterium, C₁₋₆ alkyl, deuterated C₁₋₆ alkyl, -C₁₋₄ alkyl-C₃₋₆ monocyclic cycloalkyl, or C₃₋₆ monocyclic cycloalkyl; R^{2d} is H, deuterium, C₁₋₆ alkyl, deuterated C₁₋₆ alkyl, C₃₋₂₀ cycloalkyl, or 3- to 20-membered heterocyclyl; or
when R^{2a} and R^{2d} together with the nitrogen atom to which the R^{2a} and R^{2d} are attached form a 3- to 20-membered heterocyclyl group, a 5- or 6-membered monocyclic heteroaryl group, or an 8- to 10-membered bicyclic heteroaryl group, R^{2c} is H, deuterium, C₁₋₆ alkyl, or deuterated C₁₋₆ alkyl; R^{2b} is H, deuterium, C₁₋₆ alkyl, or deuterated C₁₋₆ alkyl, and the bond between R^{2a} and the carbon atom is a single bond; or R^{2b} is absent, and the bond between R^{2a} and the carbon atom is a double bond; the 3- to 20-membered heterocyclyl, the 5- or 6-membered monocyclic heteroaryl, or the 8- to 10-membered bicyclic heteroaryl each independently contains one nitrogen atom and optionally 1 or 2 heteroatom(s) independently selected from N, O, and S as ring atoms; and the 3- to 20-membered heterocyclyl, the 5- or 6-membered monocyclic heteroaryl, and the 8- to 10-membered bicyclic heteroaryl are each independently optionally substituted with 1, 2, 3, or 4 group(s) selected from group S1; or
when R^{2c} and R^{2d} together with the nitrogen atom to which the R^{2c} and R^{2d} are attached form a 3- to 20-membered heterocyclyl group, a 5- or 6-membered monocyclic heteroaryl group, or an 8- to 10-membered bicyclic heteroaryl group, R^{2a} is H, deuterium, C₁₋₆ alkyl, or deuterated C₁₋₆ alkyl, R^{2b} is H, deuterium, C₁₋₆ alkyl, or deuterated C₁₋₆ alkyl, and the bond between R^{2a} and the carbon atom is a single bond; the 3- to 20-membered heterocyclyl, the 5- or 6-membered monocyclic heteroaryl, or the 8- to 10-membered bicyclic heteroaryl each independently contains one nitrogen atom and optionally 1 or 2 heteroatom(s) independently selected from N, O, and S as ring atoms; and the 3- to 20-membered heterocyclyl, the 5- or 6-membered monocyclic heteroaryl, and the 8- to 10-membered bicyclic heteroaryl are each independently optionally substituted with 1, 2, 3, or 4 group(s) selected from group S1; or
the R² is 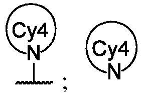 is 3- to 20-membered heterocyclyl, 5- or 6-membered monocyclic heteroaryl, or 8- to 10-membered bicyclic heteroaryl containing one nitrogen atom and attached to other moieties of a molecule through the nitrogen atom; 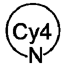 further optionally contains 1 or 2 heteroatom(s) independently selected from N, O, and S as ring atoms; the 3- to 20-membered heterocyclyl, the 5- or 6-membered monocyclic heteroaryl, and the 8- to 10-membered bicyclic heteroaryl are each independently optionally substituted with 1, 2, 3, or 4 group(s) selected from group S1; 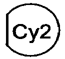
is phenyl or 5- or 6-membered monocyclic heteroaryl; the 5- or 6-membered monocyclic heteroaryl contains 1, 2, or 3 heteroatom(s) independently selected from N, O, and S as ring atoms; the phenyl and the 5- or 6-membered monocyclic heteroaryl are each independently optionally substituted with m2 R³ group(s);
m2 is 1, 2, 3, or 4; each R³ is independently selected from the group consisting of H, oxo (=O), halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C₁₋₄ alkyl-carboxy, -C(=O)-C₁₋₆ alkyl, -C(=O)O-C₁₋₆ alkyl, -C(=O)-C₁₋₄ alkyl-hydroxy, -C(=O)-C₁₋₄ alkyl-C₁₋₆ alkoxy, -C(=O)-C₁₋₄ alkyl-O-C₃₋₂₀ cycloalkyl, -C(=O)-C₃₋₂₀ cycloalkyl, -C(=O)O-C₃₋₂₀ cycloalkyl, -C(=O)-C₁₋₄ alkyl-C₃₋₂₀ cycloalkyl, -C(=O)-3- to 20-membered heterocyclyl, -C(=O)-C₁₋₄ alkyl-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)(C₁₋₆ alkyl)₂, -P(=O)(C₁₋₆ alkyl)₂; wherein the C₁₋₆ alkyl and the C₁₋₆ alkoxy are each independently optionally substituted with 1, 2, 3, or 4 group(s) selected from halogen, deuterium, cyano, 5- to 6-membered heterocyclyl, and hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S2; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; or 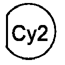
is C₅₋₇ monocyclic cycloalkyl or 5-, 6- or 7-membered monocyclic heterocyclyl; the 5-, 6- or 7-membered heterocyclyl each independently contains 1, 2, or 3 heteroatom(s) independently selected from N, O, and S as ring atoms; the C₅₋₇ monocyclic cycloalkyl and the 5-, 6- or 7-membered monocyclic heterocyclyl are each independently optionally substituted with m2 R³ group(s);
m2 is 1, 2, 3, or 4; each R³ is independently selected from the group consisting of H, oxo (=O), halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C1-4 alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C₁₋₄ alkyl-carboxy, -C(=O)-C₁₋₆ alkyl, -C(=O)O-C₁₋₆ alkyl, -C(=O)-C₁₋₄ alkyl-hydroxy, -C(=O)-C₁₋₄ alkyl-C₁₋₆ alkoxy, -C(=O)-C₁₋₄ alkyl-O-C₃₋₂₀ cycloalkyl, -C(=O)-C₃₋₂₀ cycloalkyl, -C(=O)O-C₃₋₂₀ cycloalkyl, -C(=O)-C₁₋₄ alkyl-C₃₋₂₀ cycloalkyl, -C(=O)-3- to 20-membered heterocyclyl, -C(=O)-C₁₋₄ alkyl-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)(C₁₋₆ alkyl)₂, -P(=O)(C₁₋₆ alkyl)₂, =CR^{3a}R^{3b}; wherein two hydrogen atoms attached to a same carbon atom can be substituted with two R³ groups such that the two R³ groups together with the carbon atom to which the two R³ groups are attached form a C₃₋₆ monocyclic cycloalkyl group or a 3- to 6-membered monocyclic heterocyclyl group; or two hydrogen atoms attached to different carbon atoms are substituted with two R³ groups, and the two R³ groups join to form -CH₂-, -CH₂CH₂-, or -CH₂CH₂CH₂-; wherein the C₁₋₆ alkyl and the C₁₋₆ alkoxy are each independently optionally substituted with 1, 2, 3, or 4 group(s) selected from halogen, deuterium, cyano, 5- to 6-membered heterocyclyl, and hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S2; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; wherein R^{3a} and R^{3b} are each independently hydrogen, halogen, cyano, C₁₋₆ alkyl, halogenated C₁₋₆ alkyl, C₆₋₁₄ aryl, or 5- or 6-membered monocyclic heteroaryl; the C₆₋₁₄ aryl or the 5- or 6-membered monocyclic heteroaryl is optionally substituted with 1, 2, or 3 group(s) selected from halogen, cyano, C₁₋₄ alkyl, C₁₋₄ alkoxy, halogenated C₁₋₄ alkyl, halogenated C₁₋₄ alkoxy; or R^{3a} and R^{3b} together with the carbon atom to which the R^{3a} and R^{3b} are attached form a C₃₋₆ monocyclic cycloalkyl group or a 3- to 6-membered monocyclic heterocyclyl group; the C₃₋₆ monocyclic cycloalkyl or the 3- to 6-membered monocyclic heterocyclyl is optionally substituted with 1, 2, or 3 group(s) selected from halogen, cyano, C₁₋₄ alkyl, C₁₋₄ alkoxy, halogenated C₁₋₄ alkyl, halogenated C₁₋₄ alkoxy, C₆₋₁₄ aryl, 5- or 6-membered monocyclic heteroaryl;
among the above groups, each group from group S1 is independently selected from the group consisting of oxo (=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -0-8- to 10-membered bicyclic heteroaryl, -C=C-C₃₋₂₀ cycloalkyl, -C=C-3- to 20-membered heterocyclyl, -C=C-C₆₋₁₄ aryl, -C=C-5- or 6-membered monocyclic heteroaryl, -C=C-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -S(=O)₂-5- or 6-membered monocyclic heteroaryl, -S(=O)₂-8- to 10-membered bicyclic heteroaryl, -C(=O)O-C₁₋₆ alkyl, -C(=O)O-C₃₋₂₀ cycloalkyl, -C(=O)O-3- to 20-membered heterocyclyl, -C(=O)O-5- or 6-membered monocyclic heteroaryl, -C(=O)O-8- to 10-membered bicyclic heteroaryl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}; wherein the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S3; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; among the above groups, each group from group S2 is independently selected from the group consisting of oxo (=O), halogen, cyano, hydroxyl, carboxyl, nitro, C₁₋₆ alkyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-3- to 20-membered heterocyclyl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8-to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -S(=O)₂-5- or 6-membered monocyclic heteroaryl, -S(=O)₂-8- to 10-membered bicyclic heteroaryl, -C(=O)O-C₁₋₆ alkyl, -C(=O)O-C₃₋₂₀ cycloalkyl, -C(=O)O-3- to 20-membered heterocyclyl, -C(=O)O-5- or 6-membered monocyclic heteroaryl, -C(=O)O-8- to 10-membered bicyclic heteroaryl, -NR^{a1}R^{b1}, -C(=O)-NR^{a1}R^{b1}, -OR^{c1}, -C₁₋₄ alkyl-C(=O)O-C₁₋₆ alkyl, -C₁₋₄ alkyl-C(=O)O-C₃₋₂₀ cycloalkyl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}; wherein the C₁₋₆ alkyl, the C₁₋₆ alkoxy, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S3; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms;
among the above groups, each R^{a}, R^{b}, R^{a1}, and R^{b1} is independently H, C₁₋₆ alkyl, halogenated C₁₋₆ alkyl, deuterated C₁₋₆ alkyl, -C₁₋₄ alkyl-hydroxy, -C₁₋₄ alkyl-cyano, -C₁₋₄ alkyl-halogenated C₁₋₆ alkyl, -C₁₋₄ alkyl-deuterated C₁₋₆ alkyl, -C₁₋₄ alkyl-C₁₋₆ alkoxy, -C₁₋₄ alkyl-halogenated C₁₋₆ alkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C(=O)-C₁₋₆ alkyl, -C(=O)-C₃₋₆ monocyclic cycloalkyl, -C(=O)-3- to 6-membered monocyclic heterocyclyl; wherein the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂; or
each R^{a} and R^{b} together with the nitrogen atom to which the R^{a} and R^{b} are attached form a 3- to 20-membered heterocyclyl group; each R^{a1} and R^{b1} together with the nitrogen atom to which the R^{a1} and R^{b1} are attached form a 3- to 20-membered heterocyclyl group; wherein the 3- to 20-membered heterocyclyl is each independently optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂;
among the above groups, each R^{d} and R^{d1} is independently H, C₁₋₆ alkyl, or deuterated C₁₋₆ alkyl;
among the above groups, each R^{c} and R^{c1}is independently H, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -C₁₋₄ alkyl-halogenated C₁₋₆ alkyl, -C₁₋₄ alkyl-deuterated C₁₋₆ alkyl, -C₁₋₄ alkyl-C₁₋₆ alkoxy, -C₁₋₄ alkyl-halogenated C₁₋₆ alkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂;
among the above groups, each group from group S3 is independently selected from the group consisting of oxo (C=O), cyano, halogen, hydroxy, carboxy, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂, 3- to 6-membered monocyclic heterocyclyl;
among the above groups, the -C₁₋₄ alkyl- or the -C₃₋₆ monocyclic cycloalkyl- is unsubstituted; or hydrogen atoms of the -C₁₋₄ alkyl- are each independently substituted with a group selected from halogen, cyano, hydroxy, C₁₋₆ alkyl, halogenated C₁₋₆ alkyl, deuterated C₁₋₆ alkyl, -CH₂-hydroxy, -CH₂-cyano, and phenyl, or two hydrogen atoms attached to a same carbon atom of the C₁₋₄ alkyl are simultaneously substituted with -(CH₂)ⱼ- to form a cycloalkyl group, wherein j is 2, 3, 4, 5, or 6; hydrogen atoms of the -C₃₋₆ monocyclic cycloalkyl- are each independently substituted with a group selected from halogen, cyano, hydroxy, C₁₋₆ alkyl, halogenated C₁₋₆ alkyl, deuterated C₁₋₆ alkyl.

In an embodiment, the compound is a compound of formula (IA-1); 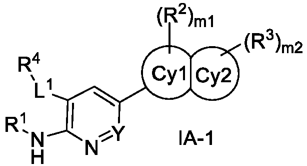
wherein R¹, Y, 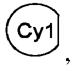 R², m1, 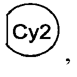 R³, and m2 are as defined in formula (IA);
L¹ is a bond, -C₁₋₄ alkyl-, -C₃₋₆ monocyclic cycloalkyl-, -O-, -S-, -NH-, -C(=O)-, -S(=O)-, or -S(=O)₂-;
R⁴ is selected from the group consisting of H, halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C₁₋₄ alkyl-hydroxyl, -C₁₋₄ alkyl-cyano, -C₁₋₄ alkyl-carboxyl, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3-to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)2-R^{c}, -S(=O)₂-NR^{a}R^{b}, -NR^{d}-S(=O)₂-NR^{a}R^{b}, -NR^{a}R^{b}, -OR^{c}; the C₁₋₆ alkyl and the C₁₋₆ alkoxy are each independently optionally substituted with halogen, deuterium, cyano, or hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8-to 10-membered bicyclic heteroaryl each independently contains 1, 2, 3, or 4 heteroatom(s) independently selected from N, O, and S as ring atoms; wherein R^{a}, R^{b}, R^{c}, R^{d}, and group S1 are each as defined in formula (IA).

In an embodiment, the compound is the compound of formula (IA-1); wherein R¹, Y, 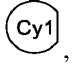 R², m1, 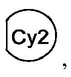 R³, and m2 are as defined in formula (IA); L¹ is a bond or -C(=O)-; R⁴ is C₃₋₂₀ cycloalkyl, 3- to 20-membered heterocyclyl, C₆₋₁₄ aryl, 5- or 6-membered monocyclic heteroaryl, or 8- to 10-membered bicyclic heteroaryl; wherein the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; or L¹ is a bond; R⁴ is H.

In an embodiment, in formula (IA-1), Y is a carbon atom.

In an embodiment, the compound is a compound of formula (IA1), a compound of formula (IA2), a compound of formula (IA3), a compound of formula (IA4), a compound of formula (IA5), or a compound of formula (IA6); 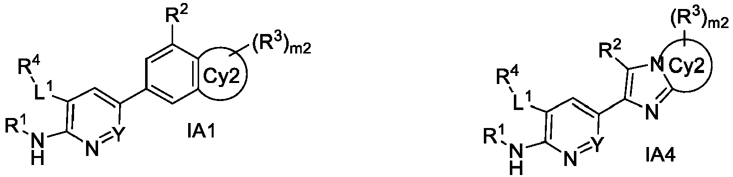 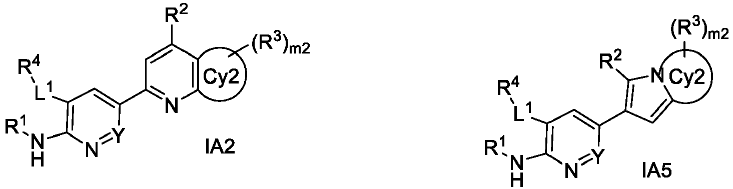 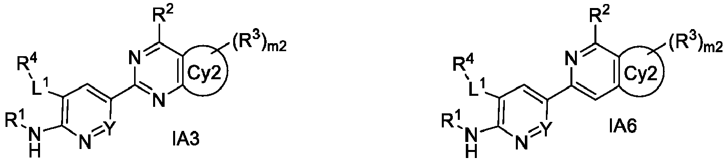 in each formula, Y, L¹, R¹, R², 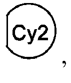 R³, R⁴, and m2 are each independently as defined in formula (IA-1).

In an embodiment, Y is each independently a carbon atom in the compound of formula (IA1), the compound of (IA2), the compound of (IA3), the compound of (IA4), the compound of (IA5), or the compound of (IA6).

In an embodiment, the compound is a compound of formula (IB); 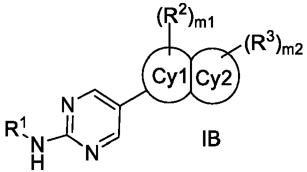 wherein R¹, 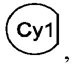 R², m1, 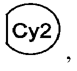 R³, and m2 are as defined in formula IA.

In an embodiment, the compound is a compound of formula (IB1), a compound of formula (IB2), a compound of formula (IB3), a compound of formula (IB4), a compound of formula (IB5), or a compound of formula (IB6); 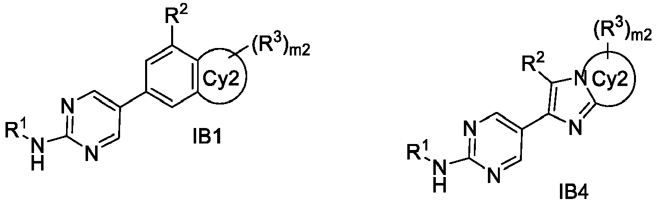 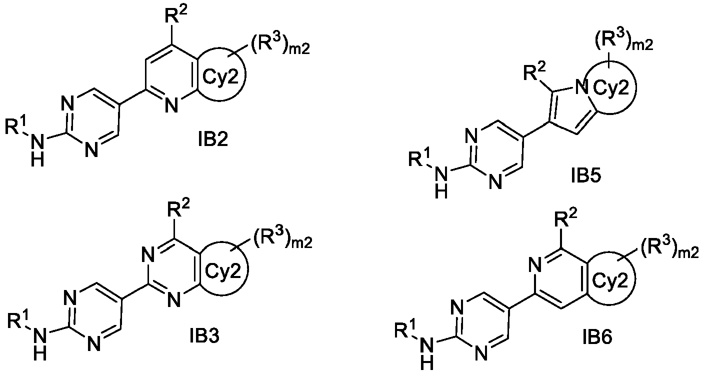 in each formula, R¹, R², 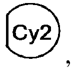 R³, and m2 are each independently as defined in formula IB.

In an embodiment, R¹ is 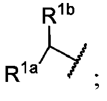 wherein R^{1a} is C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -C₁₋₄ alkyl-hydroxy, -C₁₋₄ alkyl-cyano, -C₁₋₄ alkyl-C₁₋₆ alkoxy, -C₁₋₄ alkyl-halogenated C₁₋₆ alkoxy, or -C₁₋₄ alkyl-deuterated C₁₋₆ alkoxy; R^{1b} is H, halogen, C₁₋₃ alkyl, halogenated C₁₋₃ alkyl, deuterated C₁₋₃ alkyl, C₁₋₃ alkoxy, halogenated C₁₋₃ alkoxy, or deuterated C₁₋₃ alkoxy.

In an embodiment, R¹ is 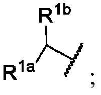 wherein R^{1a} is C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl; R^{1b} is H, halogen, C₁₋₃ alkyl, halogenated C₁₋₃ alkyl, deuterated C₁₋₃ alkyl, C₁₋₃ alkoxy, halogenated C₁₋₃ alkoxy, or deuterated C₁₋₃ alkoxy; wherein the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are each independently optionally substituted with 1, 2, 3, or 4 group(s) selected from group S1.

In an embodiment, R¹ is H, C₁₋₄ alkyl, C₁₋₄ alkoxy, -C₁₋₂ alkyl-hydroxy, -C₁₋₂ alkyl-cyano, -C₁₋₂ alkyl-carboxy, -C₁₋₂ alkyl-C₁₋₄ alkyl, -C₁₋₂ alkyl-C₁₋₄ alkoxy, C₃₋₆ monocyclic cycloalkyl, 3- to 6-membered monocyclic heterocyclyl, 7- to 11-membered spiro-heterocyclyl, 6- to 10-membered fused heterocyclyl, 6- to 14-membered bridged heterocyclyl, phenyl, 5- or 6-membered monocyclic heteroaryl, 8- to 10-membered bicyclic heteroaryl, -C₁₋₂ alkyl-C₃₋₆ monocyclic cycloalkyl, -C₁₋₂ alkyl-O-C₃₋₆ monocyclic cycloalkyl, -C₁₋₂ alkyl-3- to 6-membered monocyclic heterocyclyl, -C₁₋₂ alkyl-O-3- to 6-membered monocyclic heterocyclyl, -C₁₋₂ alkyl-7- to 11-membered spiro-heterocyclyl, -C₁₋₂ alkyl-O-7- to 11-membered spiro-heterocyclyl, -C₁₋₂ alkyl-6- to 10-membered fused heterocyclyl, -C₁₋₂ alkyl-O-6- to 10-membered fused heterocyclyl, -C₁₋₂ alkyl-6- to 14-membered bridged heterocyclyl, -C₁₋₂ alkyl-O-6- to 14-membered bridged heterocyclyl, -C₁₋₂ alkyl-phenyl, -C₁₋₂ alkyl-O-phenyl, -C₁₋₂ alkyl-5- or 6-membered monocyclic heteroaryl, -C₁₋₂ alkyl-O-5- or 6-membered monocyclic heteroaryl, -C₁₋₂ alkyl-8- to 10-membered bicyclic heteroaryl, -C₁₋₂ alkyl-O-8- to 10-membered bicyclic heteroaryl, -C₁₋₂ alkyl-NR^{a}R^{b}, -C₁₋₂ alkyl-NR^{d}-C(=O)-R^{c}, -C₁₋₂ alkyl-NR^{d}-C(=O)-NR^{a}R^{b}, -C₁₋₂ alkyl-NR^{d}-S(=O)₂-R^{c}, -C₁₋₂ alkyl-NR^{d}-S(=O)₂-NR^{a}R^{b}, -C₁₋₂ alkyl-C(=O)-NR^{a}R^{b}, -C₁₋₂ alkyl-S(=O)₂-R^{c}, -C₁₋₂ alkyl-S(=O)₂-NR^{a}R^{b}, -C(=O)-C₁₋₄ alkyl, -C(=O)-C₃₋₆ monocyclic cycloalkyl, -C(=O)-3- to 6-membered monocyclic heterocyclyl, -C(=O)-7- to 11-membered spiro-heterocyclyl, -C(=O)-6- to 10-membered fused heterocyclyl, -C(=O)-6- to 14-membered bridged heterocyclyl, -C(=O)-phenyl, -C(=O)-5- or 6-membered monocyclic heteroaryl, -C(=O)-8- to 10-membered bicyclic heteroaryl, -C(=O)-NR^{a}R^{b}, -S(=O)₂-R^{c}, or -S(=O)₂-NR^{a}R^{b}; wherein the C₁₋₄ alkyl and the C₁₋₄ alkoxy are each independently optionally substituted with 1, 2, or 3 group(s) selected from halogen, deuterium, cyano, or hydroxy; the C₃₋₆ monocyclic cycloalkyl, the 3- to 6-membered monocyclic heterocyclyl, the 7- to 11-membered spiro-heterocyclyl, the 6- to 10-membered fused heterocyclyl, the 6- to 14-membered bridged heterocyclyl, the phenyl, the 5- or 6-membered monocyclic heteroaryl, and the 8- to 10-membered bicyclic heteroaryl are each independently optionally substituted with 1, 2, 3, or 4 group(s) selected from group S1.

In an embodiment, R¹ is phenyl; the phenyl is optionally substituted with 1, 2, 3, or 4 group(s) selected from group S1.

In an embodiment, R⁴ is C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl; wherein the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are each independently optionally substituted with 1, 2, 3, or 4 group(s) selected from group S1.

In an embodiment, R⁴ is 3- to 6-membered monocyclic heterocyclyl; the 3- to 6-membered monocyclic heterocyclyl is selected from aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran; the 3- to 6-membered monocyclic heterocyclyl is optionally substituted with 1, 2, 3, or 4 group(s) selected from group S1.

In an embodiment, R⁴ is 5- or 6-membered monocyclic heteroaryl; the 5- or 6-membered monocyclic heteroaryl is selected from the group consisting of thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine; the 5- or 6-membered monocyclic heteroaryl is optionally substituted with 1, 2, 3, or 4 group(s) selected from group S1.

In an embodiment, the compound of formula (IA), or a pharmaceutically acceptable salt, a stereoisomer, a solvate or a prodrug thereof, is prepared by a method comprising: 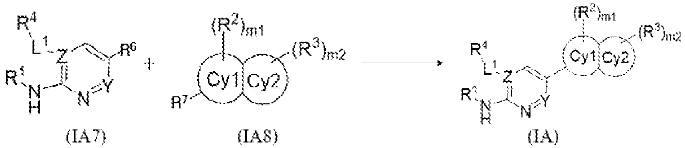 wherein Y, Z, R¹, L¹, R⁴, R², 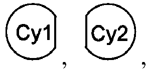 m1, m2, and R³ are as defined above. R⁶ and R⁷ are different groups, optionally selected from -CHO, -COCH₂, -COOC₂H₅, -OCH₃, -CN, -NO₂, -F, -Cl, Br, a boronic acid group, or a boronate group.

The compound of formula (IA), or a pharmaceutically acceptable salt, a stereoisomer, a solvate or a prodrug thereof, is prepared by a Suzuki reaction of R⁶ in a compound of formula (IA7) with R⁷ in a compound of formula (IA8) in the presence of a palladium catalyst, or
by a Suzuki reaction of a compound without one or more of -R¹, -R¹-NH, -L¹-R⁴ or -R⁴ groups in the compound of formula (IA7) with a compound without the -R² and/or -R³ groups in the compound of formula (IA8) to obtain an intermediate free of the -R¹, -R¹-NH, -L¹-R⁴, -R⁴, -R² and/or -R³ groups, followed by a substitution reaction of the intermediate with a compound having the -R¹, -R¹-NH, -L¹-R⁴, -R⁴, -R² and/or -R³ groups. In a second aspect of the present disclosure, provided is a compound of formula (IC), or a pharmaceutically acceptable salt, a stereoisomer, a solvate or a prodrug thereof: 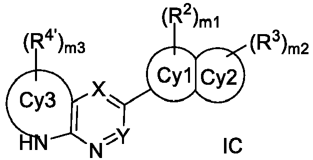 wherein
X is CR⁵ or N; Y is CH or N; and X and Y cannot both be N; R⁵ is H, C₁₋₆ alkyl, or C₃₋₆ monocyclic cycloalkyl; 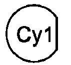 is phenyl or 5- or 6-membered monocyclic heteroaryl; the 5- or 6-membered monocyclic heteroaryl contains 1, 2, or 3 heteroatom(s) independently selected from N, O, and S as ring atoms; the phenyl or the 5- or 6-membered monocyclic heteroaryl are each independently optionally substituted with m1 R² group(s); m1 is 1, 2, 3, or 4;
the R² is 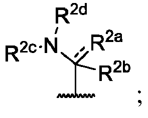 wherein
R^{2a} is H, deuterium, C₁₋₆ alkyl, phenyl, or deuterated C₁₋₆ alkyl, and R^{2b} is H, deuterium, C₁₋₆ alkyl, or deuterated C₁₋₆ alkyl; or R^{2a} and R^{2b} together with the carbon atom to which the R^{2a} and R^{2b} are attached form a C₃₋₆ monocyclic cycloalkyl group, and a bond between R^{2a} and the carbon atom is a single bond; R^{2c} is H, deuterium, C₁₋₆ alkyl, deuterated C₁₋₆ alkyl, -C₁₋₄ alkyl-C₃₋₆ monocyclic cycloalkyl, or C₃₋₆ monocyclic cycloalkyl; R^{2d} is H, deuterium, C₁₋₆ alkyl, deuterated C₁₋₆ alkyl, C₃₋₂₀ cycloalkyl, or 3- to 20-membered heterocyclyl; or
when R^{2a} and R^{2d} together with the nitrogen atom to which the R^{2a} and R^{2d} are attached form a 3- to 20-membered heterocyclyl group, a 5- or 6-membered monocyclic heteroaryl group, or an 8- to 10-membered bicyclic heteroaryl group, R^{2c} is H, deuterium, C₁₋₆ alkyl, or deuterated C₁₋₆ alkyl; R^{2b} is H, deuterium, C₁₋₆ alkyl, or deuterated C₁₋₆ alkyl, and the bond between R^{2a} and the carbon atom is a single bond; or R^{2b} is absent, and the bond between R^{2a} and the carbon atom is a double bond; the 3- to 20-membered heterocyclyl, the 5- or 6-membered monocyclic heteroaryl, or the 8- to 10-membered bicyclic heteroaryl each independently contains one nitrogen atom and optionally 1 or 2 heteroatom(s) independently selected from N, O, and S as ring atoms; and the 3- to 20-membered heterocyclyl, the 5- or 6-membered monocyclic heteroaryl, and the 8- to 10-membered bicyclic heteroaryl are each independently optionally substituted with 1, 2, 3, or 4 group(s) selected from group S1; or
when R^{2c} and R^{2d} together with the nitrogen atom to which the R^{2c} and R^{2d} are attached form a 3- to 20-membered heterocyclyl group, a 5- or 6-membered monocyclic heteroaryl group, or an 8- to 10-membered bicyclic heteroaryl group, R^{2a} is H, deuterium, C₁₋₆ alkyl, or deuterated C₁₋₆ alkyl; R^{2b} is H, deuterium, C₁₋₆ alkyl, or deuterated C₁₋₆ alkyl; and the bond between R^{2a} and the carbon atom is a single bond; the 3- to 20-membered heterocyclyl, the 5- or 6-membered monocyclic heteroaryl, or the 8- to 10-membered bicyclic heteroaryl each independently contains one nitrogen atom and optionally 1 or 2 heteroatom(s) independently selected from N, O, and S as ring atoms; and the 3- to 20-membered heterocyclyl, the 5- or 6-membered monocyclic heteroaryl, and the 8- to 10-membered bicyclic heteroaryl are each independently optionally substituted with 1, 2, 3, or 4 group(s) selected from group S1; or
the R² is 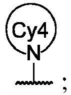 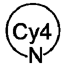 is 3- to 20-membered heterocyclyl, 5- or 6-membered monocyclic heteroaryl, or 8-to 10-membered bicyclic heteroaryl containing one nitrogen atom and attached to other moieties of a molecule through the nitrogen atom; 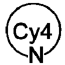 further optionally contains 1 or 2 heteroatom(s) independently selected from N, O, and S as ring atoms; the 3- to 20-membered heterocyclyl, the 5- or 6-membered monocyclic heteroaryl, and the 8- to 10-membered bicyclic heteroaryl are each independently optionally substituted with 1, 2, 3, or 4 group(s) selected from group S1; 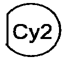
is phenyl or 5- or 6-membered monocyclic heteroaryl; the 5- or 6-membered monocyclic heteroaryl contains 1, 2, or 3 heteroatom(s) independently selected from N, O, and S as ring atoms; the phenyl and the 5- or 6-membered monocyclic heteroaryl are each independently optionally substituted with m2 R³ group(s);
m2 is 1, 2, 3, or 4; each R³ is independently selected from the group consisting of H, oxo (=O), halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C₁₋₄ alkyl-carboxy, -C(=O)-C₁₋₆ alkyl, -C(=O)O-C₁₋₆ alkyl, -C(=O)-C₁₋₄ alkyl-hydroxy, -C(=O)-C₁₋₄ alkyl-C₁₋₆ alkoxy, -C(=O)-C₁₋₄ alkyl-O-C₃₋₂₀ cycloalkyl, -C(=O)-C₃₋₂₀ cycloalkyl, -C(=O)O-C₃₋₂₀ cycloalkyl, -C(=O)-C₁₋₄ alkyl-C₃₋₂₀ cycloalkyl, -C(=O)-3- to 20-membered heterocyclyl, -C(=O)-C₁₋₄ alkyl-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)(C₁₋₆ alkyl)₂, -P(=O)(C₁₋₆ alkyl)₂; wherein the C₁₋₆ alkyl and the C₁₋₆ alkoxy are each independently optionally substituted with 1, 2, 3, or 4 group(s) selected from halogen, deuterium, cyano, 5- to 6-membered heterocyclyl, and hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S2; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; or 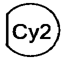
is C₅₋₇ monocyclic cycloalkyl or 5-, 6- or 7-membered monocyclic heterocyclyl; the 5-, 6- or 7-membered heterocyclyl each independently contains 1, 2, or 3 heteroatom(s) independently selected from N, O, and S as ring atoms; the C₅₋₇ monocyclic cycloalkyl and the 5-, 6- or 7-membered monocyclic heterocyclyl are each independently optionally substituted with m2 R³ group(s);
m2 is 1, 2, 3, or 4; each R³ is independently selected from the group consisting of H, oxo (=O), halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C₁₋₄ alkyl-carboxy, -C(=O)-C₁₋₆ alkyl, -C(=O)O-C₁₋₆ alkyl, -C(=O)-C₁₋₄ alkyl-hydroxy, -C(=O)-C₁₋₄ alkyl-C₁₋₆ alkoxy, -C(=O)-C₁₋₄ alkyl-O-C₃₋₂₀ cycloalkyl, -C(=O)-C₃₋₂₀ cycloalkyl, -C(=O)O-C₃₋₂₀ cycloalkyl, -C(=O)-C₁₋₄ alkyl-C₃₋₂₀ cycloalkyl, -C(=O)-3- to 20-membered heterocyclyl, -C(=O)-C₁₋₄ alkyl-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)(C₁₋₆ alkyl)₂, -P(=O)(C₁₋₆ alkyl)₂, =CR^{3a}R^{3b}; wherein two hydrogen atoms attached to a same carbon atom can be substituted with two R³ groups such that the two R³ groups together with the carbon atom to which the two R³ groups are attached form a C₃₋₆ monocyclic cycloalkyl group or a 3- to 6-membered monocyclic heterocyclyl group; or two hydrogen atoms attached to different carbon atoms are substituted with two R³ groups, and the two R³ groups join to form -CH₂-, -CH₂CH₂-, or -CH₂CH₂CH₂-; wherein the C₁₋₆ alkyl and the C₁₋₆ alkoxy are each independently optionally substituted with 1, 2, 3, or 4 group(s) selected from halogen, deuterium, cyano, 5- to 6-membered heterocyclyl, and hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S2; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; wherein R^{3a} and R^{3b} are each independently hydrogen, halogen, cyano, C₁₋₆ alkyl, halogenated C₁₋₆ alkyl, C₆₋₁₄ aryl, or 5- or 6-membered monocyclic heteroaryl; the C₆₋₁₄ aryl or the 5- or 6-membered monocyclic heteroaryl is optionally substituted with 1, 2, or 3 group(s) selected from halogen, cyano, C₁₋₄ alkyl, C₁₋₄ alkoxy, halogenated C₁₋₄ alkyl, halogenated C₁₋₄ alkoxy; or R^{3a} and R^{3b} together with the carbon atom to which the R^{3a} and R^{3b} are attached form a C₃₋₆ monocyclic cycloalkyl group or a 3- to 6-membered monocyclic heterocyclyl group; the C₃₋₆ monocyclic cycloalkyl or the 3- to 6-membered monocyclic heterocyclyl is optionally substituted with 1, 2, or 3 group(s) selected from halogen, cyano, C₁₋₄ alkyl, C₁₋₄ alkoxy, halogenated C₁₋₄ alkyl, halogenated C₁₋₄ alkoxy, C₆₋₁₄ aryl, 5- or 6-membered monocyclic heteroaryl; 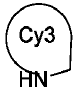
is 5- to 7-membered heteroaromatic ring; wherein the m3 hydrogen atom(s) of 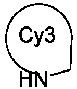 are optionally substituted with m3 R^{4'} group(s); m3 is 1, 2, 3, or 4; R^{4'} is selected from the group consisting of H, oxo (C=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, 5- or 6-membered monocyclic heteroaryl, 8- to 10-membered bicyclic heteroaryl, -C=C-C₃₋₂₀ cycloalkyl, -C=C-3- to 20-membered heterocyclyl, -C-C-C₆₋₁₄ aryl, -C=C-5- or 6-membered monocyclic heteroaryl, -C=C-8- to 10-membered bicyclic heteroaryl, -C=C-C(=O)-NR^{a}R^{b}, -C≡C-C₁₋₄ alkyl-C(=O)-NR^{a}R^{b}, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)-(C₁₋₆ alkyl)₂, -P(=O)-(C₁₋₆ alkyl)₂; wherein the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; or 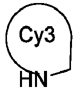
is 5- to 7-membered heterocyclic ring; wherein the m3 hydrogen atom(s) of 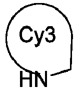 are optionally substituted with m3 R^{4'} group(s); m3 is 1, 2, 3, or 4; R^{4'} is selected from the group consisting of H, oxo (C=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C=C-C₃₋₂₀ cycloalkyl, -C=C-3- to 20-membered heterocyclyl, -C-C-C₆₋₁₄ aryl, -C=C-5- or 6-membered monocyclic heteroaryl, -C=C-8- to 10-membered bicyclic heteroaryl, -C=C-C(=O)-NR^{a}R^{b}, -C≡C-C₁₋₄ alkyl-C(=O)-NR^{a}R^{b}, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3-to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)-(C₁₋₆ alkyl)₂, -P(=O)-(C₁₋₆ alkyl)₂; the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms;
among the above groups, each group from group S1 is independently selected from the group consisting of oxo (=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C=C-C₃₋₂₀ cycloalkyl, -C=C-3- to 20-membered heterocyclyl, -C-C-C₆₋₁₄ aryl, -C=C-5- or 6-membered monocyclic heteroaryl, -C=C-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -Cl-a alkyl-S(=O)2-NR^{a1}R^{b1}, -C₁₋₄ alkyl-NR^{d1}-S(=O)2-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}; wherein the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S3; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; among the above groups, each group from group S2 is independently selected from the group consisting of oxo (=O), halogen, cyano, hydroxyl, carboxyl, nitro, C₁₋₆ alkyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-3- to 20-membered heterocyclyl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8-to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -S(=O)₂-5- or 6-membered monocyclic heteroaryl, -S(=O)₂-8- to 10-membered bicyclic heteroaryl, -C(=O)O-C₁₋₆ alkyl, -C(=O)O-C₃₋₂₀ cycloalkyl, -C(=O)O-3- to 20-membered heterocyclyl, -C(=O)O-5- or 6-membered monocyclic heteroaryl, -C(=O)O-8- to 10-membered bicyclic heteroaryl, -NR^{a1}R^{b1}, -C(=O)-NR^{a1}R^{b1}, -OR^{c1}, -C₁₋₄ alkyl-C(=O)O-C₁₋₆ alkyl, -C₁₋₄ alkyl-C(=O)O-C₃₋₂₀ cycloalkyl, -C₁₋₄ alkyl-NR^{a1}R^{b1}, -C₁₋₄ alkyl-C(=O)-NR^{a1}R^{b1}, -C=C-C(=O)-NR^{a1}R^{b1}, -C-C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}; wherein the C₁₋₆ alkyl, the C₁₋₆ alkoxy, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S3; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms;
among the above groups, each R^{a}, R^{b}, R^{a1}, and R^{b1} is independently H, C₁₋₆ alkyl, halogenated C₁₋₆ alkyl, deuterated C₁₋₆ alkyl, -C₁₋₄ alkyl-hydroxy, -C₁₋₄ alkyl-cyano, -C₁₋₄ alkyl-halogenated C₁₋₆ alkyl, -C₁₋₄ alkyl-deuterated C₁₋₆ alkyl, -C₁₋₄ alkyl-C₁₋₆ alkoxy, -C₁₋₄ alkyl-halogenated C₁₋₆ alkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8-to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C(=O)-C₁₋₆ alkyl, -C(=O)-C₃₋₆ monocyclic cycloalkyl, -C(=O)-3- to 6-membered monocyclic heterocyclyl; wherein the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂; or
each R^{a} and R^{b} together with the nitrogen atom to which the R^{a} and R^{b} are attached form a 3- to 20-membered heterocyclyl group; each R^{a1} and R^{b1} together with the nitrogen atom to which the R^{a1} and R^{b1} are attached form a 3- to 20-membered heterocyclyl group; wherein the 3- to 20-membered heterocyclyl is each independently optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂;
among the above groups, each R^{d} and R^{d1} are independently H, C₁₋₆ alkyl, or deuterated C₁₋₆ alkyl;
among the above groups, each R^{c} and R^{c1} are independently H, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -C₁₋₄ alkyl-halogenated C₁₋₆ alkyl, -C₁₋₄ alkyl-deuterated C₁₋₆ alkyl, -C₁₋₄ alkyl-C₁₋₆ alkoxy, -C₁₋₄ alkyl-halogenated C₁₋₆ alkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂;
among the above groups, each group from group S3 is independently selected from the group consisting of oxo (C=O), cyano, halogen, hydroxy, carboxy, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂, 3- to 6-membered monocyclic heterocyclyl;
among the above groups, the -C₁₋₄ alkyl- or the -C₃₋₆ monocyclic cycloalkyl- is unsubstituted, or hydrogen atoms of the -C₁₋₄ alkyl- are each independently substituted with a group selected from halogen, cyano, hydroxy, C₁₋₆ alkyl, halogenated C₁₋₆ alkyl, deuterated C₁₋₆ alkyl, -CH₂-hydroxy, -CH₂-cyano, and phenyl, or two hydrogen atoms attached to a same carbon atom of the C₁₋₄ alkyl are substituted with -(CH₂)ⱼ- to form a cycloalkyl group, wherein j is 2, 3, 4, 5, or 6; hydrogen atoms of the -C₃₋₆ monocyclic cycloalkyl- are each independently substituted with a group selected from halogen, cyano, hydroxy, C₁₋₆ alkyl, halogenated C₁₋₆ alkyl, deuterated C₁₋₆ alkyl.

In an embodiment, R^{4'} is C₁₋₆ alkyl or C₁₋₆ alkoxy; the C₁₋₆ alkyl and the C₁₋₆ alkoxy are each independently optionally substituted with 1, 2, or 3 group(s) selected from halogen, deuterium, cyano, or hydroxyl;
In an embodiment, R^{4'} is C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl; wherein the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are each independently optionally substituted with 1, 2, 3, or 4 group(s) selected from group S1.

In an embodiment, when R^{4'} is 3- to 6-membered monocyclic heterocyclyl, the 3- to 6-membered monocyclic heterocyclyl is selected from aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran; the 3- to 6-membered monocyclic heterocyclyl is optionally substituted with 1, 2, 3, or 4 group(s) selected from group S1.

In an embodiment, when R^{4'} is 5- or 6-membered monocyclic heteroaryl, the 5- or 6-membered monocyclic heteroaryl is selected from the group consisting of thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine; the 5- or 6-membered monocyclic heteroaryl is optionally substituted with 1, 2, 3, or 4 group(s) selected from group S1.

In an embodiment, the compound is a compound of formula (IC1);

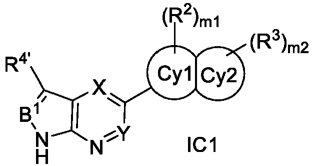

Wherein X, Y, 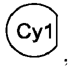 R², m1, 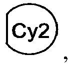 R³, and m2 are as defined in formula IC; B¹ is CH or N;
R^{4'} is selected from the group consisting of H, oxo (C=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, 5- or 6-membered monocyclic heteroaryl, 8- to 10-membered bicyclic heteroaryl, -C=C-C₃₋₂₀ cycloalkyl, -C=C-3- to 20-membered heterocyclyl, -C-C-C₆₋₁₄ aryl, -C=C-5- or 6-membered monocyclic heteroaryl, -C=C-8- to 10-membered bicyclic heteroaryl, -C=C-C(=O)-NR^{a}R^{b}, -C≡C-C₁₋₄ alkyl-C(=O)-NR^{a}R^{b}, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3-to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)-(C₁₋₆ alkyl)₂, -P(=O)-(C₁₋₆ alkyl)₂; wherein the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; wherein R^{a}, R^{b}, R^{c}, R^{d}, and group S1 are each as defined in formula IC.

In an embodiment, the compound is a compound of formula (ICla), a compound of formula (IC1b), a compound of formula (IC1c), a compound of formula (IC1d), a compound of formula (ICle), or a compound of formula (IClf); 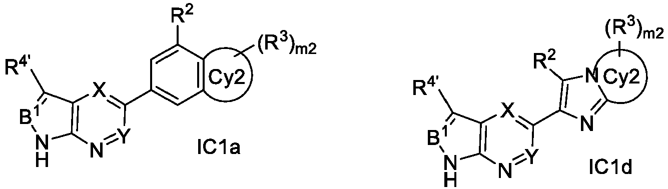 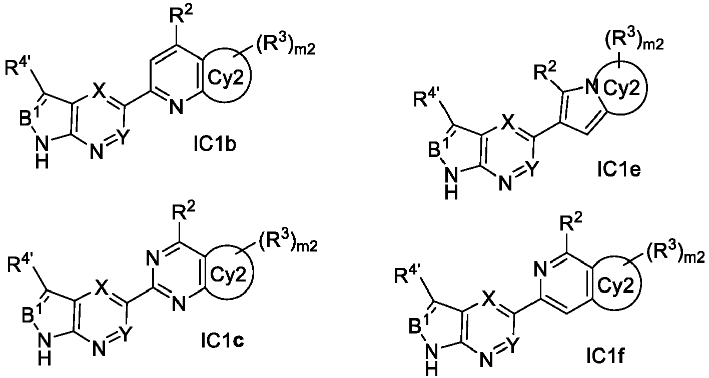 in each formula, X, Y, R², 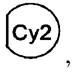 R³, m2, B¹, and R^{4'} are each independently as defined in formula IC1.

In an embodiment, in the compound of formula (IC1), the compound of formula (ICla), the compound of formula (IC1b), the compound of formula (IC1c), the compound of formula (ICld), the compound of formula (ICle), or the compound of formula (IClf), X and Y are each independently CH.

In an embodiment, in the compound of formula (IC1), the compound of formula (ICla), the compound of formula (IC1b), the compound of formula (IC1c), the compound of formula (ICld), the compound of formula (ICle), or the compound of formula (IClf), B¹ is each independently CH.

In an embodiment, in the compound of formula (IC1), the compound of formula (ICla), the compound of formula (IC1b), the compound of formula (IC1c), the compound of formula (ICld), the compound of formula (ICle), or the compound of formula (IClf), B¹ is each independently N.

In an embodiment, in the compound of formula (IC1), the compound of formula (ICla), the compound of formula (IC1b), the compound of formula (IC1c), the compound of formula (ICld), the compound of formula (ICle), or the compound of formula (IClf), R^{4'} is methyl.

In an embodiment, the compound is a compound of formula (IC3); 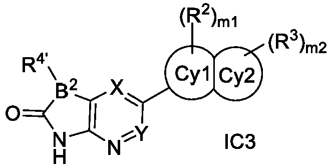 wherein X, Y, 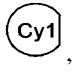 R², m1, 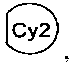 R³, and m2 are as defined in formula IC; B² is N or CH;
R^{4'} is selected from the group consisting of H, oxo (C=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C=C-C₃₋₂₀ cycloalkyl, -C=C-3- to 20-membered heterocyclyl, -C-C-C₆₋₁₄ aryl, -C=C-5- or 6-membered monocyclic heteroaryl, -C=C-8- to 10-membered bicyclic heteroaryl, -C=C-C(=O)-NR^{a}R^{b}, -C≡C-C₁₋₄ alkyl-C(=O)-NR^{a}R^{b}, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3-to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)-(C₁₋₆ alkyl)₂, -P(=O)-(C₁₋₆ alkyl)₂; the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; wherein R^{a}, R^{b}, R^{c}, R^{d}, and group S1 are each as defined in formula IC.

In an embodiment, the compound is a compound of formula (IC3a), a compound of formula (IC3b), a compound of formula (IC3c), a compound of formula (IC3d), a compound of formula (IC3e), or a compound of formula (IC3f); 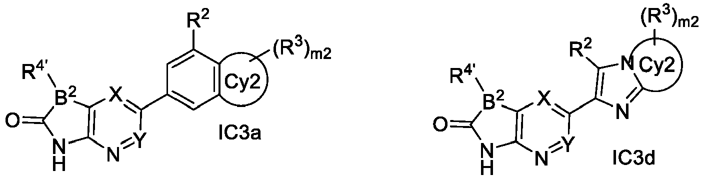 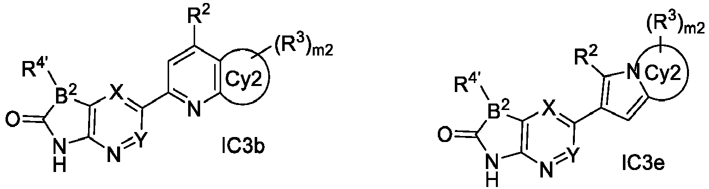 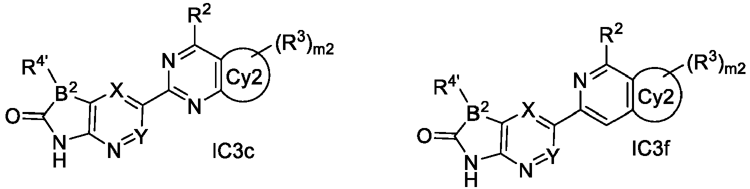 in each formula, X, Y, R², 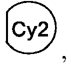 R³, m2, B², and R^{4'} are each independently as defined in formula IC3.

In an embodiment, in the compound of formula (IC3), the compound of formula (IC3a), the compound of formula (IC3b), the compound of formula (IC3c), the compound of formula (IC3d), the compound of formula (IC3e), or the compound of formula (IC3f), X and Y are each independently a carbon atom.

In an embodiment, in the compound of formula (IC3), the compound of formula (IC3a), the compound of formula (IC3b), the compound of formula (IC3c), the compound of formula (IC3d), the compound of formula (IC3e), or the compound of formula (IC3f), B² is each independently CH.

In an embodiment, in the compound of formula (IC3), the compound of formula (IC3a), the compound of formula (IC3b), the compound of formula (IC3c), the compound of formula (IC3d), the compound of formula (IC3e), or the compound of formula (IC3f), B² is each independently N.

In an embodiment, the compound is a compound of formula (IC4); 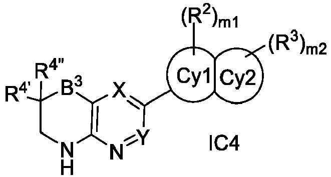
wherein X, Y, 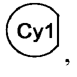 R², m1, 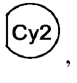 R³, and m2 are as defined in formula IC; B³ is CHR^{4‴}, NR^{4‴}, or O;
R^{4'}, R^{4"}, and R^{4‴} are each independently selected from the group consisting of H, oxo (C=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C≡C-C₃₋₂₀ cycloalkyl, -C=C-3- to 20-membered heterocyclyl, -C≡C-C₆₋₁₄ aryl, -C=C-5- or 6-membered monocyclic heteroaryl, -C=C-8- to 10-membered bicyclic heteroaryl, -C≡C-C(=O)-NR^{a}R^{b}, -C≡C-C₁₋₄ alkyl-C(=O)-NR^{a}R^{b}, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)-(C₁₋₆ alkyl)₂, -P(=O)-(C₁₋₆ alkyl)₂; the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; or
R^{4'} and R^{4"} together with the carbon atom to which the R^{4'} and R^{4"} are attached form a carbonyl group (C=O);
wherein R^{a}, R^{b}, R^{c}, R^{d}, and group S1 are each as defined in formula IC.

In an embodiment, the compound is a compound of formula (IC4a), a compound of formula (IC4b), a compound of formula (IC4c), a compound of formula (IC4d), a compound of formula (IC4e), or a compound of formula (IC4f); 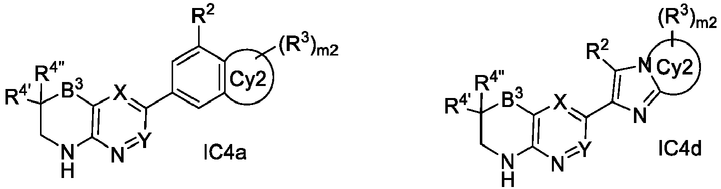 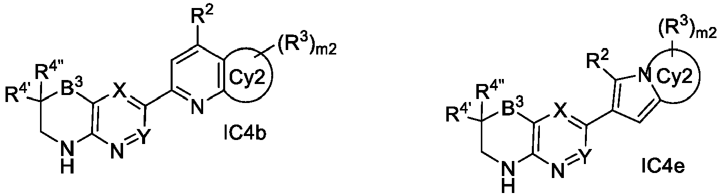 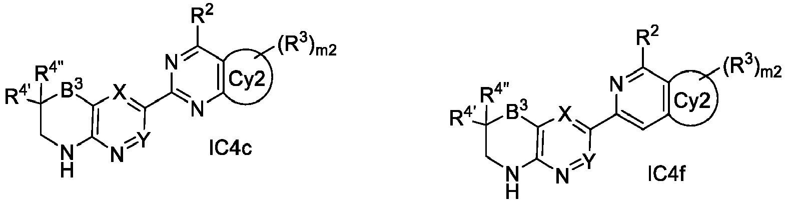 in each formula, X, Y, R², 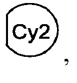 R³, m2, B³, R⁴, and R^{4"} are each independently as defined in formula IC4.

In an embodiment, in the compound of formula (IC4), the compound of formula (IC4a), the compound of formula (IC4b), the compound of formula (IC4c), the compound of formula (IC4d), the compound of formula (IC4e), or the compound of formula (IC4f), X and Y are each independently a carbon atom.

In an embodiment, in the compound of formula (IC4), the compound of formula (IC4a), the compound of formula (IC4b), the compound of formula (IC4c), the compound of formula (IC4d), the compound of formula (IC4e), or the compound of formula (IC4f), B³ is each independently CHR^{4‴}; R^{4‴} is as defined above.

In an embodiment, in the compound of formula (IC4), the compound of formula (IC4a), the compound of formula (IC4b), the compound of formula (IC4c), the compound of formula (IC4d), the compound of formula (IC4e), or the compound of formula (IC4f), B³ is each independently NR^{4‴}; R^{4‴} is as defined above.

In an embodiment, in the compound of formula (IC4), the compound of formula (IC4a), the compound of formula (IC4b), the compound of formula (IC4c), the compound of formula (IC4d), the compound of formula (IC4e), or the compound of formula (IC4f), B³ is each independently O.

In an embodiment, 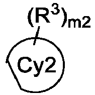 is selected from the group consisting of 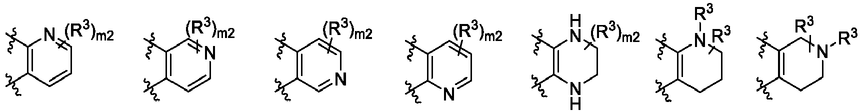 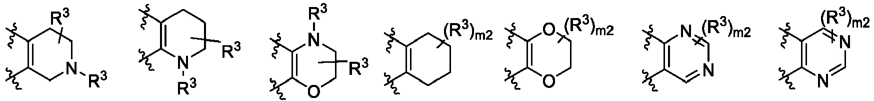 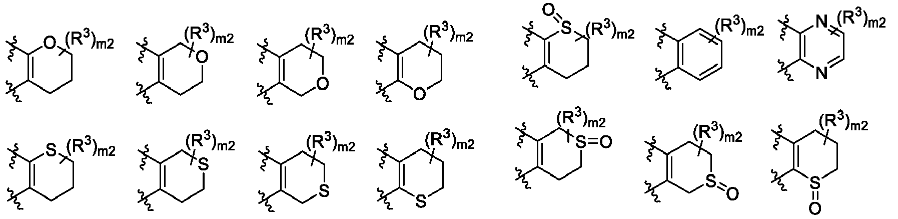 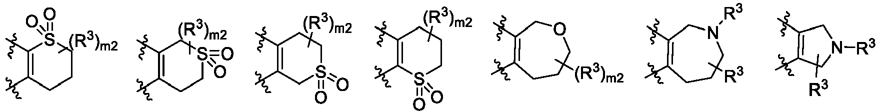 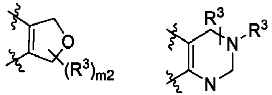 in each formula, each R³ and m2 are independently as defined above.

In an embodiment, 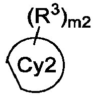 is selected from the group consisting of 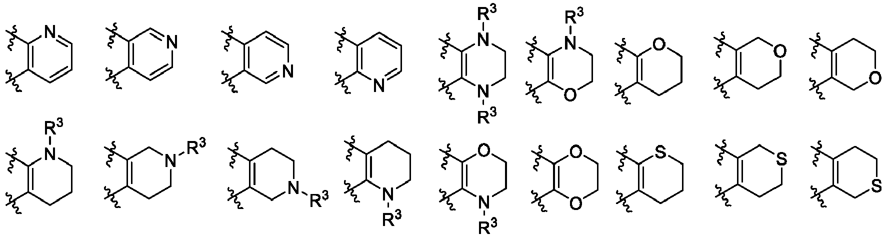 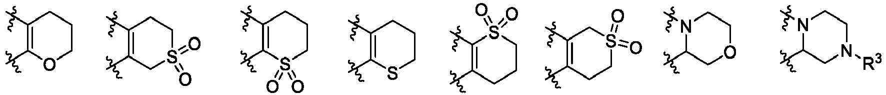 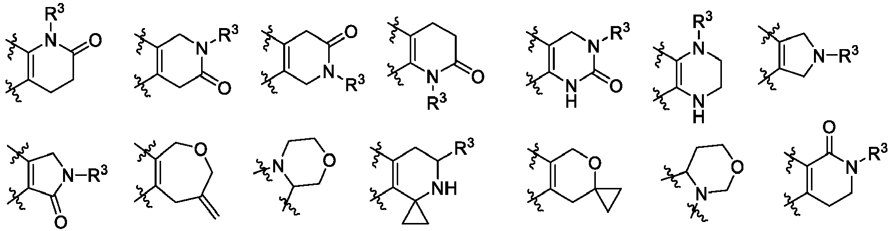 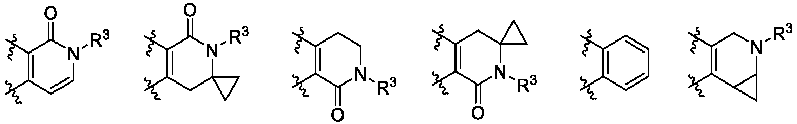 in each formula, each R³ is independently as defined above.

In an embodiment, 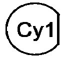 is selected from the group consisting of 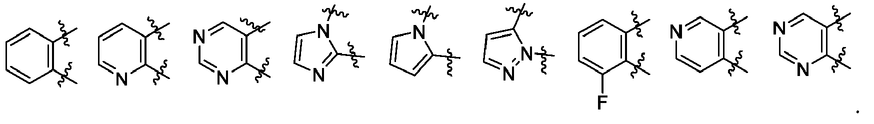 .

In an embodiment, 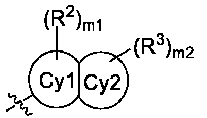 is selected from the group consisting of

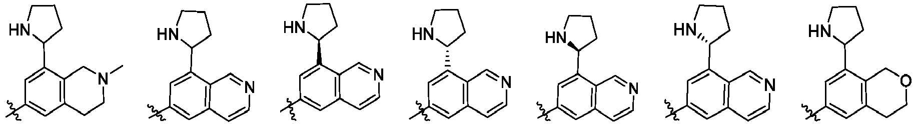

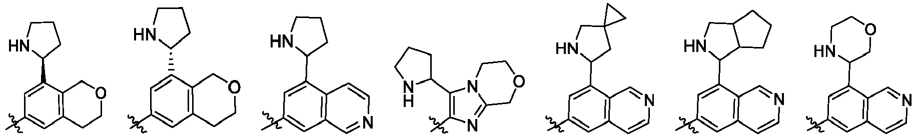

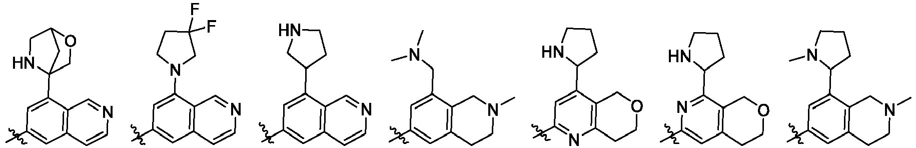

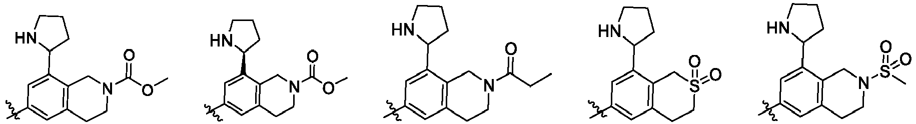

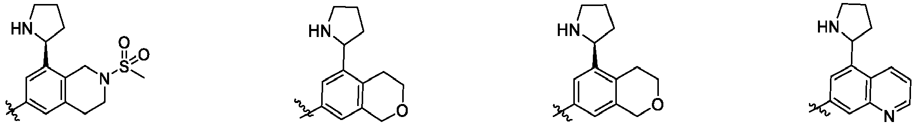

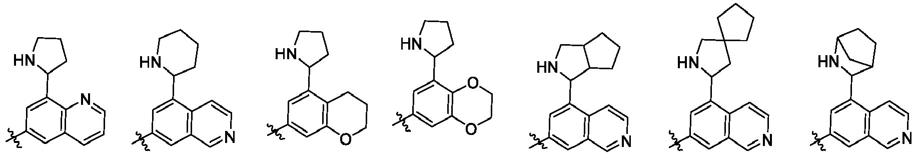

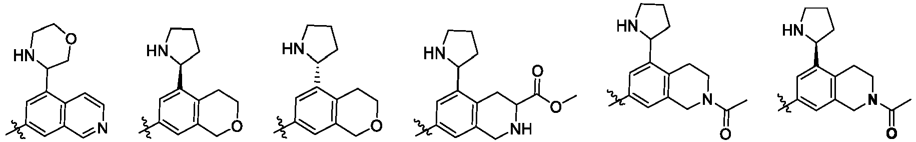

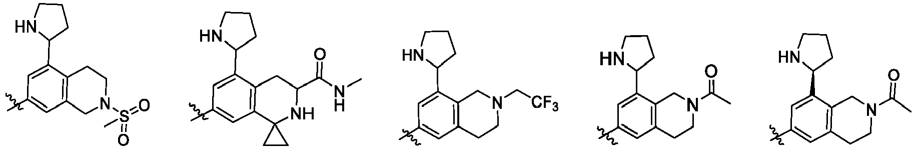

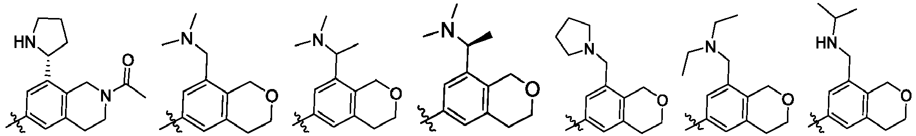

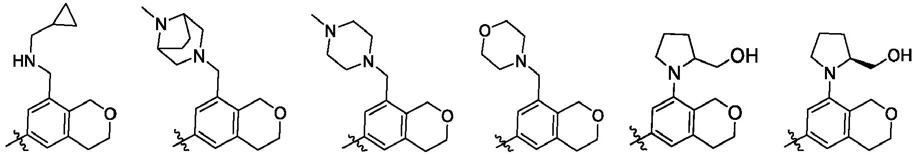

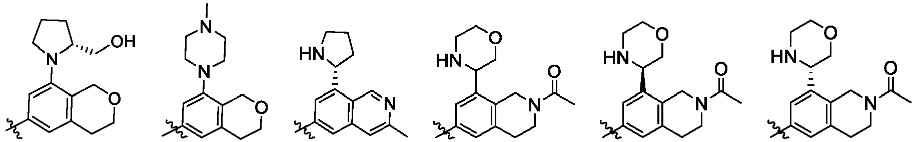

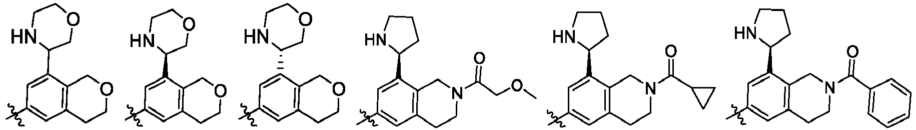

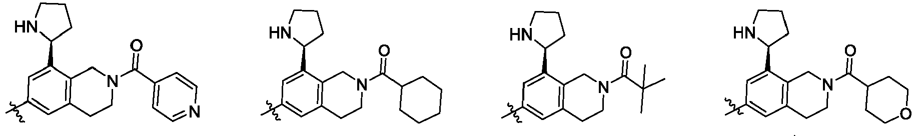

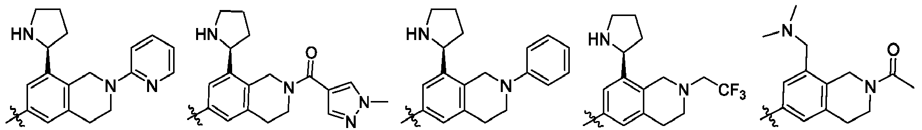

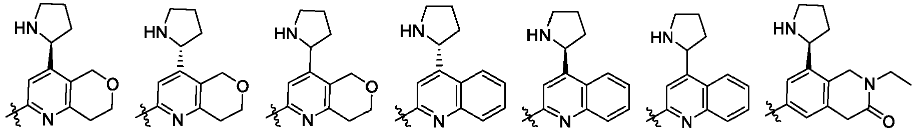

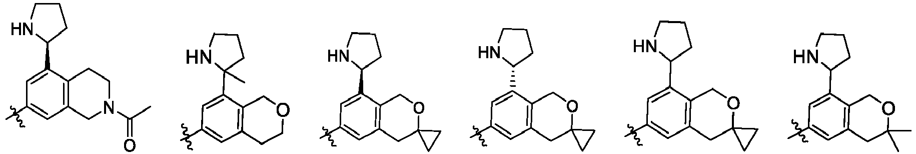

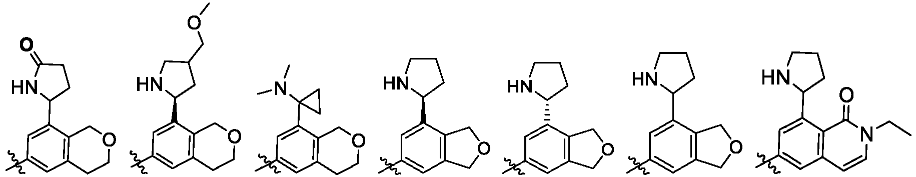

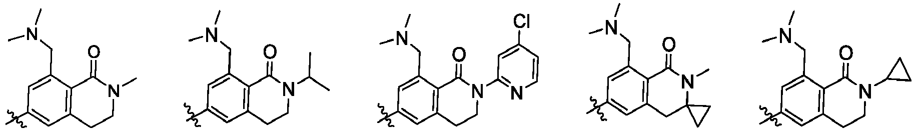

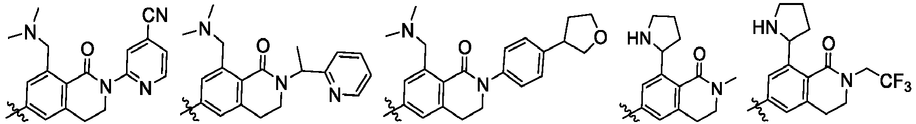

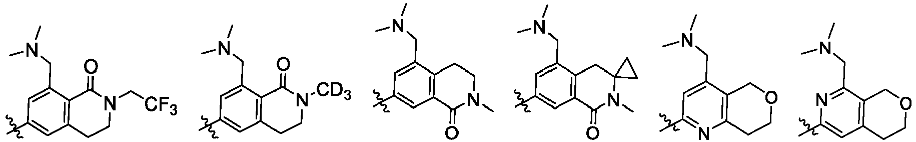

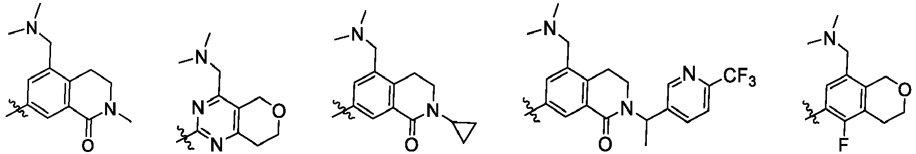

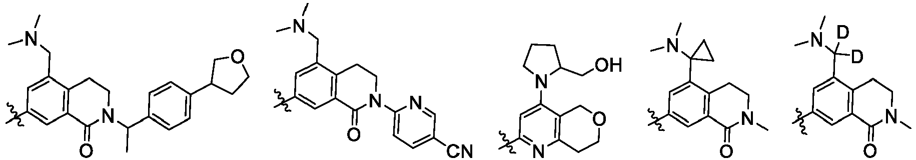

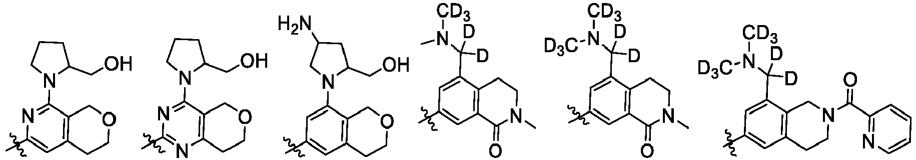

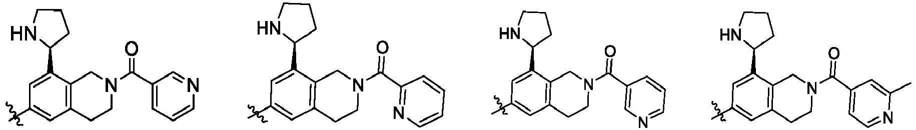

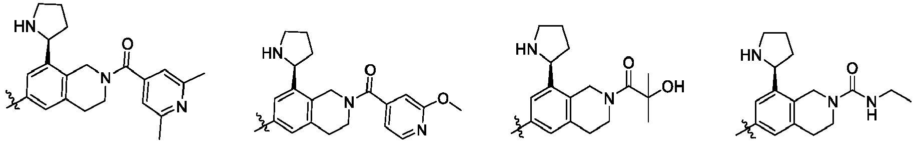

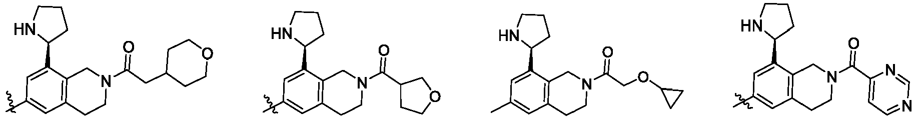

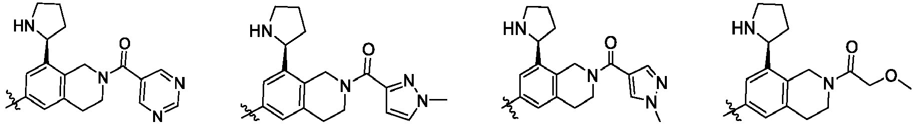

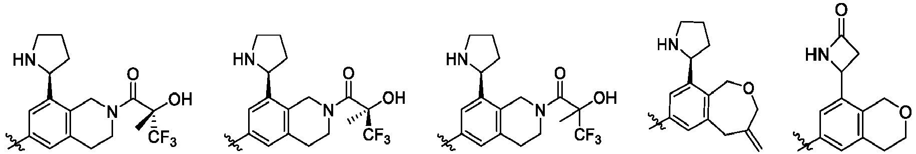

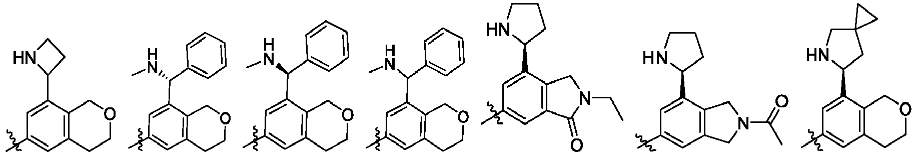

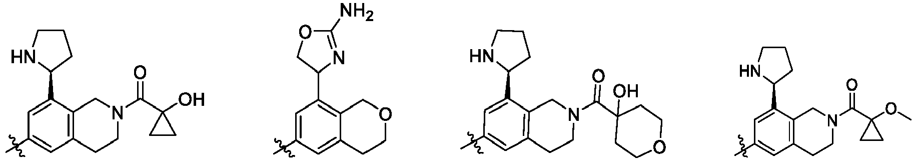

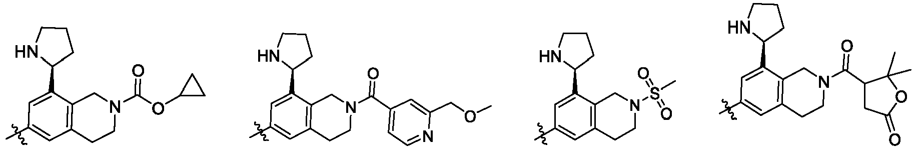

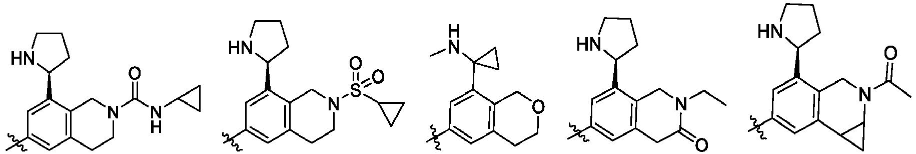

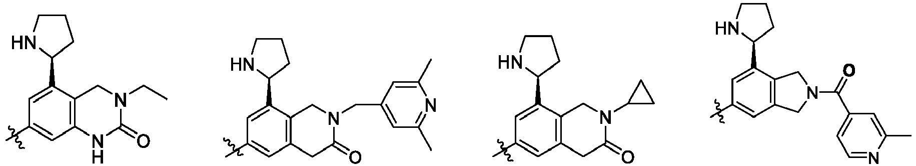

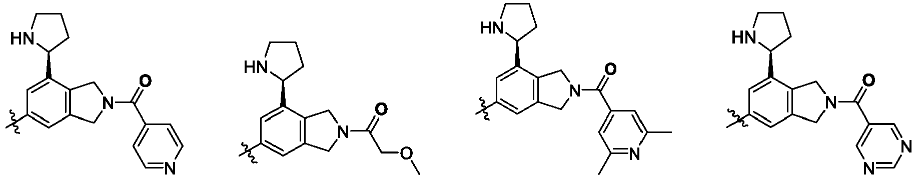

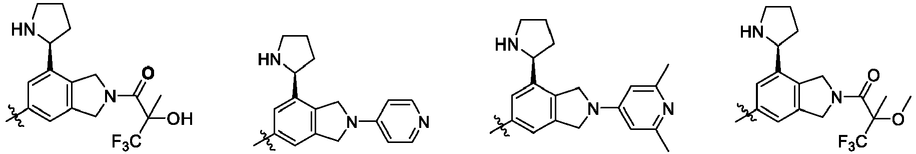

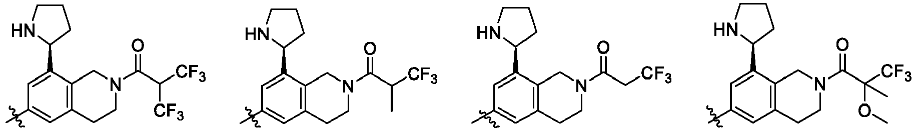

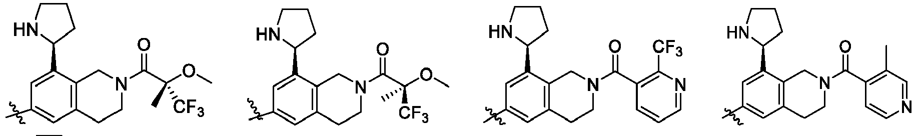

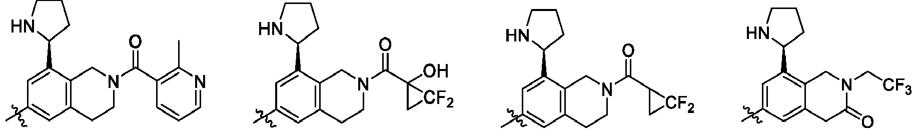

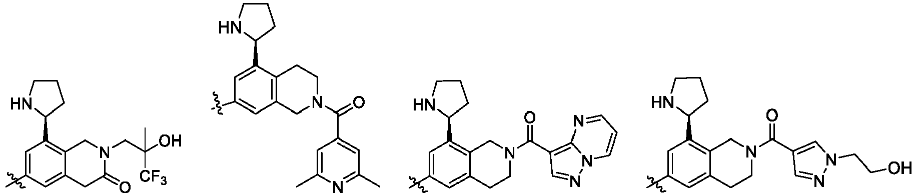

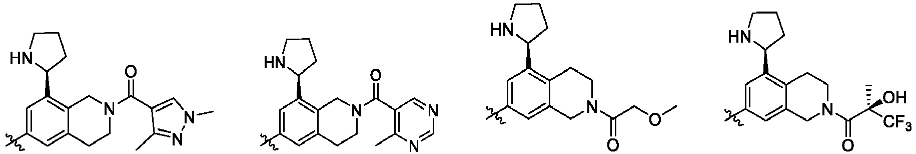

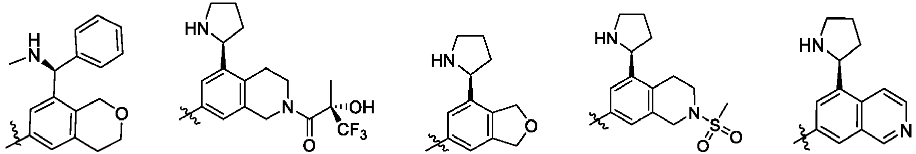

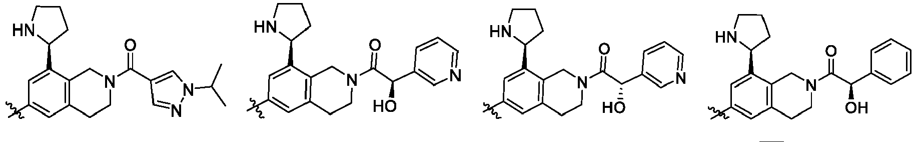

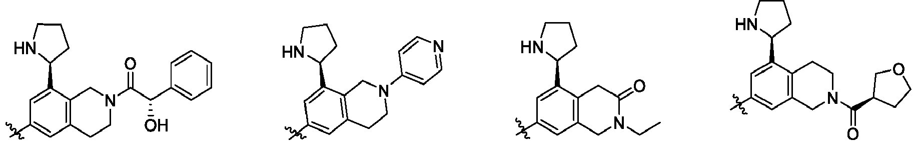

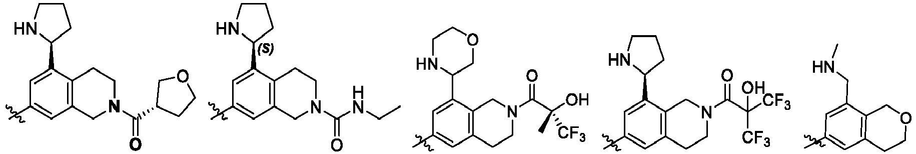

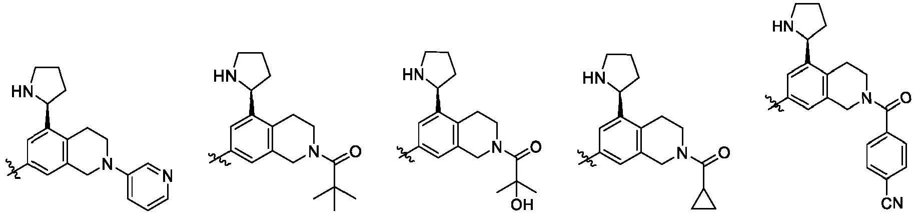

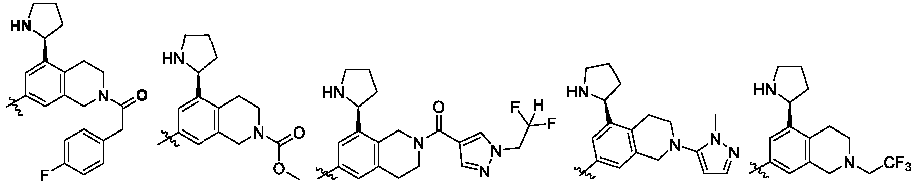

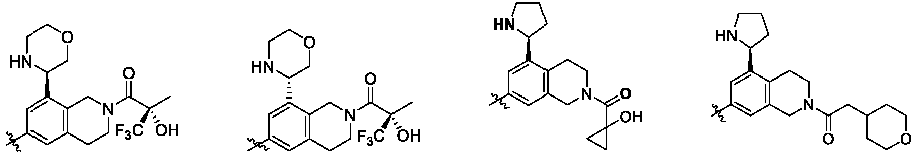

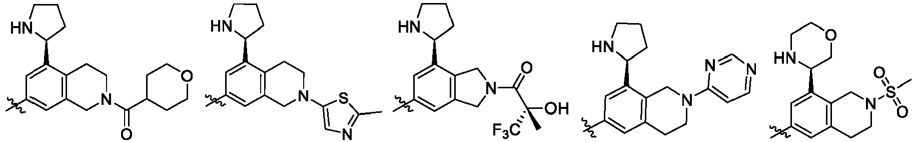

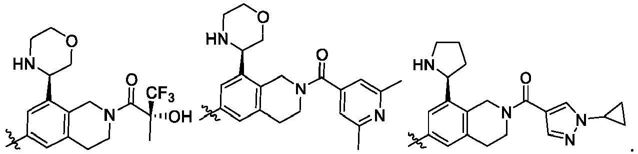

In an embodiment, 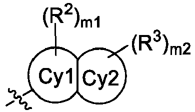 is selected from the group consisting of

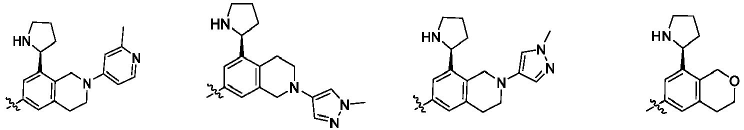

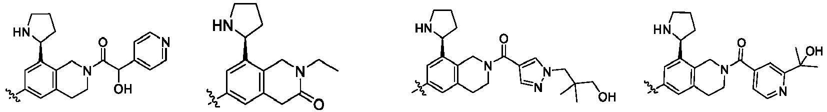

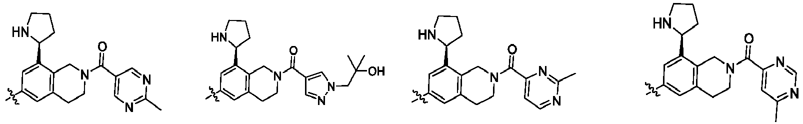

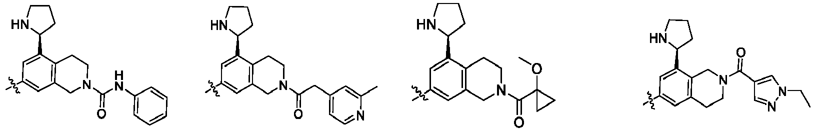

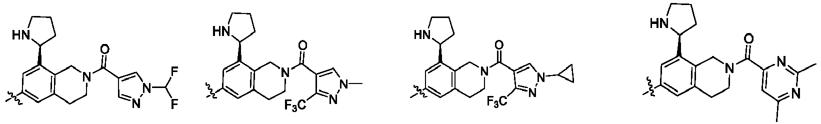

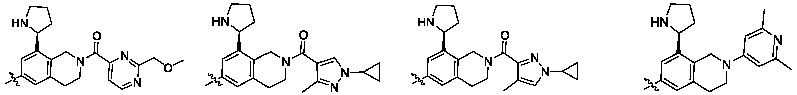

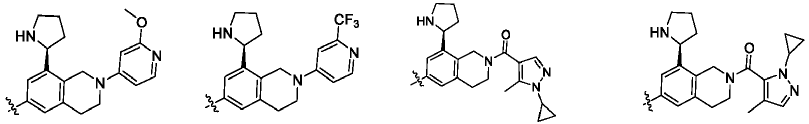

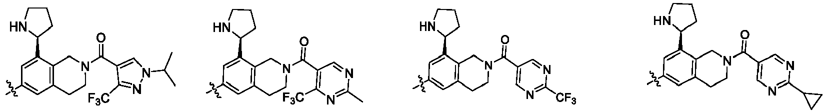

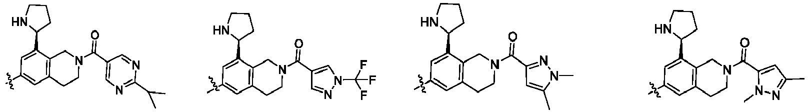

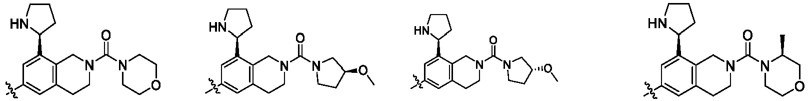

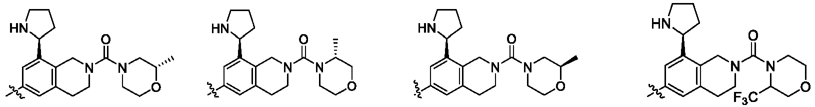

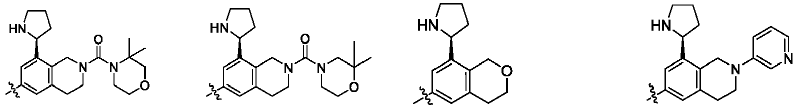

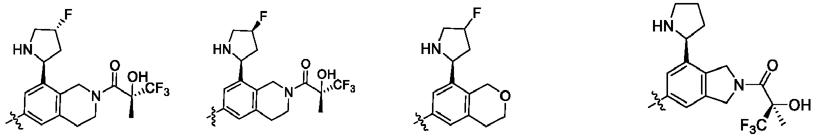

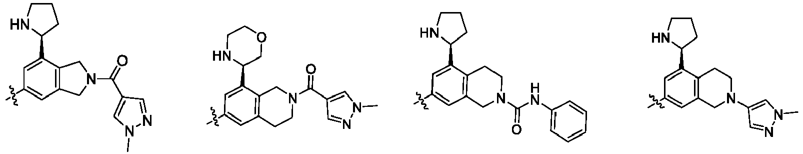

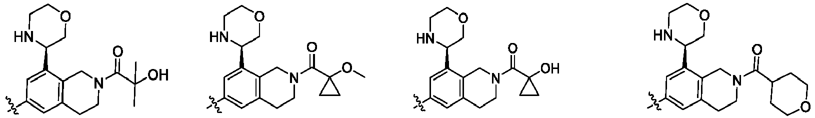

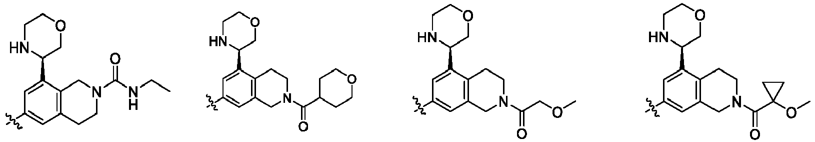

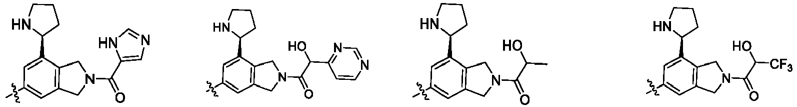

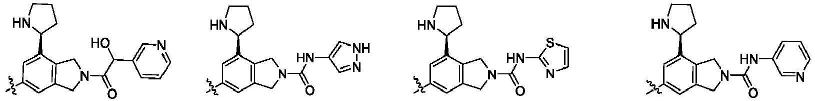

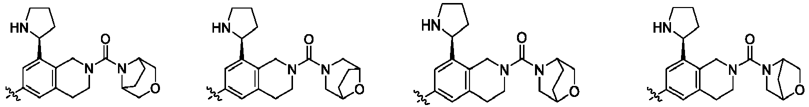

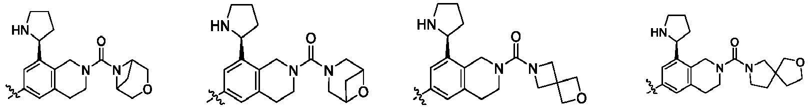

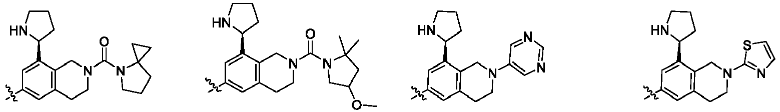

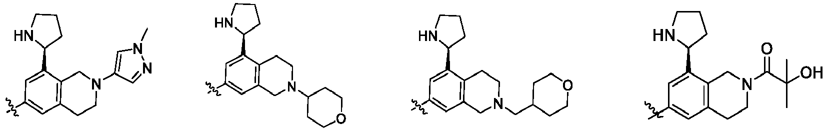

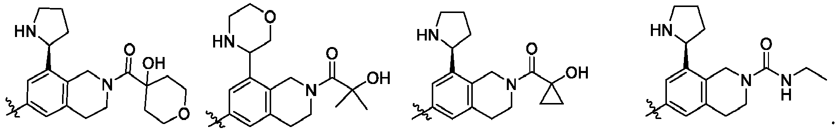

In an embodiment, R² is selected from the group consisting of 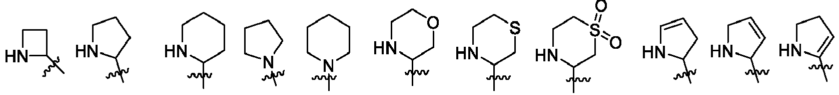 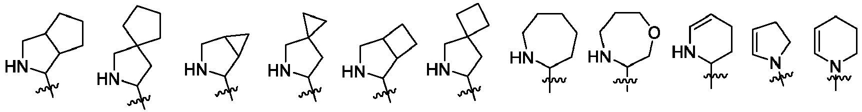 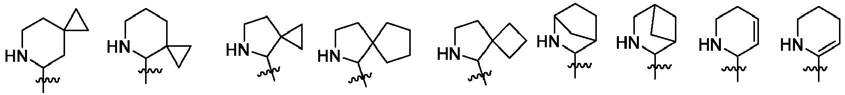 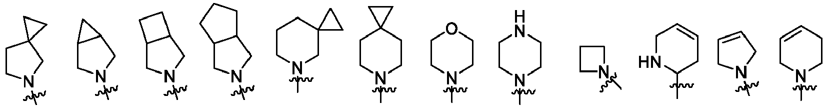 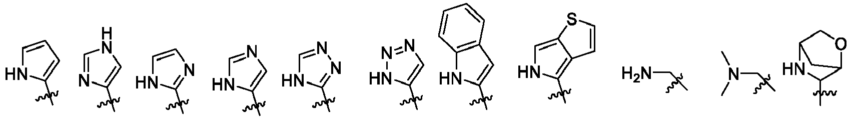 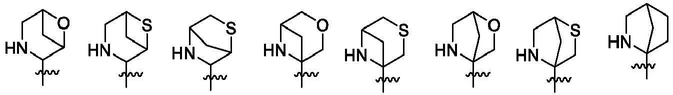 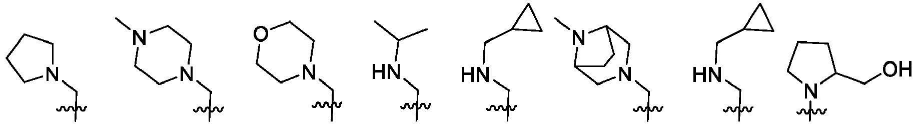 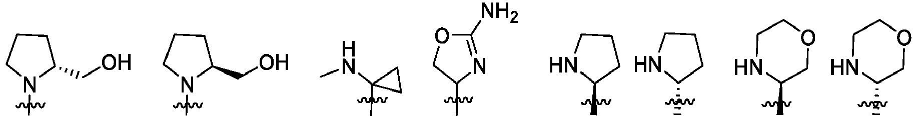 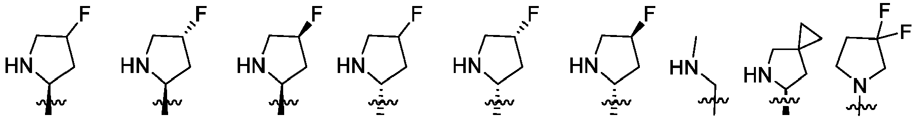 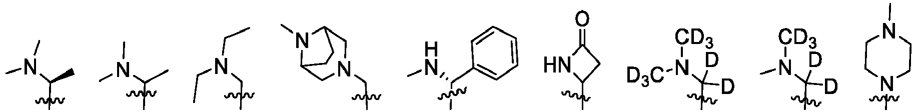 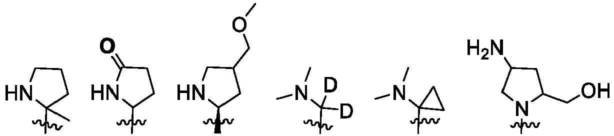 hydrogen atoms of each of the above groups may be each independently optionally substituted with 1, 2, 3, or 4 group(s) selected from group S1.

In an embodiment, R² is selected from the group consisting of 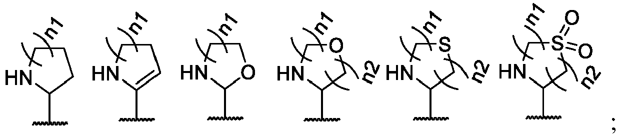 wherein each n1 and n2 are independently 0, 1, 2, or 3; hydrogen atoms of each of the above groups may be each independently optionally substituted with 1, 2, 3, or 4 group(s) selected from group S1.

In an embodiment, R² is selected from the group consisting of 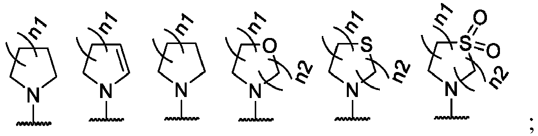 wherein each n1 and n2 are independently 0, 1, 2, or 3; hydrogen atoms of each of the above groups may be each independently optionally substituted with 1, 2, 3, or 4 group(s) selected from group S1.

In an embodiment, R² is 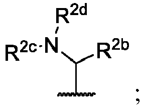 wherein R^{2b} is H or methyl;
R^{2c} and R^{2d} are each independently H, C₁₋₆ alkyl, C₃₋₆ monocyclic cycloalkyl, or 3- to 6-membered monocyclic heterocyclyl; or R^{2c} and R^{2d} together with the nitrogen atom to which the R^{2c} and R^{2d} are attached form a 3- to 6-membered monocyclic heterocyclyl group, a 5- or 6-membered monocyclic heteroaryl group, or an 8- to 10-membered bicyclic heteroaryl group; the 3- to 6-membered monocyclic heterocyclyl, the 5- or 6-membered monocyclic heteroaryl, and the 8- to 10-membered bicyclic heteroaryl each independently contains one nitrogen atom and optionally 1 or 2 heteroatom(s) independently selected from N, O, and S as ring atoms; and the 3- to 6-membered monocyclic heterocyclyl, the 5- or 6-membered monocyclic heteroaryl, and the 8- to 10-membered bicyclic heteroaryl are each independently optionally substituted with 1, 2, 3, or 4 group(s) selected from group S1.

In an embodiment, 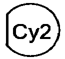 is phenyl or 5- or 6-membered monocyclic heteroaryl; R³ is selected from the group consisting of H, oxo (=O), halogen, cyano, hydroxyl, carboxyl, nitro, C₁₋₄ alkyl, C₁₋₄ alkoxy, C₃₋₆ monocyclic cycloalkyl, 3- to 6-membered monocyclic heterocyclyl, 7- to 11-membered spiro-heterocyclyl, 6- to 10-membered fused heterocyclyl, 6- to 14-membered bridged heterocyclyl, phenyl, 5- or 6-membered monocyclic heteroaryl, 8- to 10-membered bicyclic heteroaryl, -O-C₃₋₆ monocyclic cycloalkyl, -O-3- to 6-membered monocyclic heterocyclyl, -O-7- to 11-membered spiro-heterocyclyl, -O-6- to 10-membered fused heterocyclyl, -O-6- to 14-membered bridged heterocyclyl, -O-phenyl, -O-5- or 6-membered monocyclic heteroaryl, -O-8- to 10-membered bicyclic heteroaryl, -C₁₋₂ alkyl-hydroxy, -C₁₋₂ alkyl-cyano, -C₁₋₂ alkyl-C₁₋₄ alkyl, -C₁₋₂ alkyl-C₁₋₄ alkoxy, -C₁₋₂ alkyl-C₃₋₆ monocyclic cycloalkyl, -C₁₋₂ alkyl-O-C₃₋₆ monocyclic cycloalkyl, -C₁₋₂ alkyl-3-to 6-membered monocyclic heterocyclyl, -C₁₋₂ alkyl-O-3- to 6-membered monocyclic heterocyclyl, -C₁₋₂ alkyl-7- to 11-membered spiro-heterocyclyl, -C₁₋₂ alkyl-6- to 10-membered fused heterocyclyl, -C₁₋₂ alkyl-6- to 14-membered bridged heterocyclyl, -C₁₋₂ alkyl-O-7- to 11-membered spiro-heterocyclyl, -C₁₋₂ alkyl-O-6- to 10-membered fused heterocyclyl, -C₁₋₂ alkyl-O-6- to 14-membered bridged heterocyclyl, -C₁₋₂ alkyl-phenyl, -C₁₋₂ alkyl-O-phenyl, -C₁₋₂ alkyl-5- or 6-membered monocyclic heteroaryl, -C₁₋₂ alkyl-O-5- or 6-membered monocyclic heteroaryl, -C₁₋₂ alkyl-8- to 10-membered bicyclic heteroaryl, -C₁₋₂ alkyl-O-8- to 10-membered bicyclic heteroaryl, -C₁₋₂ alkyl-NR^{a}R^{b}, -C₁₋₂ alkyl-C(=O)-NR^{a}R^{b}, -C₁₋₂ alkyl-NR^{d}-C(=O)-R^{c}, -C₁₋₂ alkyl-NR^{d}-C(=O)-NR^{a}R^{b}, -C₁₋₂ alkyl-S(=O)₂-R^{c}, -C₁₋₂ alkyl-NR^{d}-S(=O)₂-R^{c}, -C₁₋₂ alkyl-S(=O)₂-NR^{a}R^{b}, -C₁₋₂ alkyl-NR^{d}-S(=O)₂-NR^{a}R^{b}, -C₁₋₂ alkyl-carboxy, -C(=O)-C₁₋₄ alkyl, -C(=O)-C₃₋₆ monocyclic cycloalkyl, -C(=O)-3- to 6-membered monocyclic heterocyclyl, -C(=O)-7- to 11-membered spiro-heterocyclyl, -C(=O)-6- to 10-membered fused heterocyclyl, -C(=O)-6- to 14-membered bridged heterocyclyl, -C(=O)-phenyl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₂ alkyl-P(=O)(C₁₋₄ alkyl)₂, -P(=O)(C₁₋₄ alkyl)₂; wherein the C₁₋₄ alkyl and the C₁₋₄ alkoxy are each independently optionally substituted with halogen, deuterium, cyano, or hydroxy; the C₃₋₆ monocyclic cycloalkyl, the 3- to 6-membered monocyclic heterocyclyl, the 7- to 11-membered spiro-heterocyclyl, the 6- to 10-membered fused heterocyclyl, the 6- to 14-membered bridged heterocyclyl, the phenyl, the 5- or 6-membered monocyclic heteroaryl, and the 8- to 10-membered bicyclic heteroaryl are each independently optionally substituted with 1, 2, 3, or 4 group(s) selected from group S2; the 3- to 6-membered monocyclic heterocyclyl, the 7- to 11-membered spiro-heterocyclyl, the 6- to 10-membered fused heterocyclyl, the 6- to 14-membered bridged heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; R^{a}, R^{b}, R^{d}, and R^{c} are as defined above.

In an embodiment, 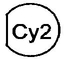 is C₅₋₇ monocyclic cycloalkyl or 5-, 6- or 7-membered monocyclic heterocyclyl; R³ is selected from the group consisting of H, oxo (=O), halogen, cyano, hydroxyl, carboxyl, nitro, C₁₋₄ alkyl, C₁₋₄ alkoxy, C₃₋₆ monocyclic cycloalkyl, 3- to 6-membered monocyclic heterocyclyl, 7- to 11-membered spiro-heterocyclyl, 6- to 10-membered fused heterocyclyl, 6- to 14-membered bridged heterocyclyl, phenyl, 5- or 6-membered monocyclic heteroaryl, 8- to 10-membered bicyclic heteroaryl, -O-C₃₋₆ monocyclic cycloalkyl, -O-3- to 6-membered monocyclic heterocyclyl, -O-7- to 11-membered spiro-heterocyclyl, -O-6- to 10-membered fused heterocyclyl, -O-6- to 14-membered bridged heterocyclyl, -O-phenyl, -O-5- or 6-membered monocyclic heteroaryl, -O-8- to 10-membered bicyclic heteroaryl, -C₁₋₂ alkyl-hydroxy, -C₁₋₂ alkyl-cyano, -C₁₋₂ alkyl-C₁₋₄alkyl, -C₁₋₂ alkyl-C₁₋₄ alkoxy, -C₁₋₂ alkyl-C₃₋₆ monocyclic cycloalkyl, -C₁₋₂ alkyl-O-C₃₋₆ monocyclic cycloalkyl, -C₁₋₂ alkyl-3- to 6-membered monocyclic heterocyclyl, -C₁₋₂ alkyl-O-3- to 6-membered monocyclic heterocyclyl, -C₁₋₂ alkyl-7- to 11-membered spiro-heterocyclyl, -C₁₋₂ alkyl-6- to 10-membered fused heterocyclyl, -C₁₋₂ alkyl-6- to 14-membered bridged heterocyclyl, -C₁₋₂ alkyl-O-7- to 11-membered spiro-heterocyclyl, -C₁₋₂ alkyl-O-6- to 10-membered fused heterocyclyl, -C₁₋₂ alkyl-O-6- to 14-membered bridged heterocyclyl, -C₁₋₂ alkyl-phenyl, -C₁₋₂ alkyl-O-phenyl, -C₁₋₂ alkyl-5- or 6-membered monocyclic heteroaryl, -C₁₋₂ alkyl-O-5- or 6-membered monocyclic heteroaryl, -C₁₋₂ alkyl-8- to 10-membered bicyclic heteroaryl, -C₁₋₂ alkyl-O-8- to 10-membered bicyclic heteroaryl, -C₁₋₂ alkyl-NR^{a}R^{b}, -C₁₋₂ alkyl-C(=O)-NR^{a}R^{b}, -C₁₋₂ alkyl-NR^{d}-C(=O)-R^{c}, -C₁₋₂ alkyl-NR^{d}-C(=O)-NR^{a}R^{b}, -C₁₋₂ alkyl-S(=O)₂-R^{c}, -C₁₋₂ alkyl-NR^{d}-S(=O)₂-R^{c}, -C₁₋₂ alkyl-S(=O)₂-NR^{a}R^{b}, -C₁₋₂ alkyl-NR^{d}-S(=O)₂-NR^{a}R^{b}, -C₁₋₂ alkyl-carboxy, -C(=O)-C₁₋₄ alkyl, -C(=O)-C₃₋₆ monocyclic cycloalkyl, -C(=O)-3- to 6-membered monocyclic heterocyclyl, -C(=O)-7- to 11-membered spiro-heterocyclyl, -C(=O)-6- to 10-membered fused heterocyclyl, -C(=O)-6- to 14-membered bridged heterocyclyl, -C(=O)-phenyl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)(C₁₋₄ alkyl)₂, -P(=O)(C₁₋₄ alkyl)₂; wherein two hydrogen atoms attached to a same carbon atom can be substituted with two R³ groups such that the two R³ groups together with the carbon atom to which the two R³ groups are attached form a C₃₋₆ monocyclic cycloalkyl group or a 3-to 6-membered monocyclic heterocyclyl group; or two hydrogen atoms attached to different carbon atoms are substituted with two R³ groups, and the two R³ groups join to form -CH₂- or -CH₂CH₂-; wherein the C₁₋₄ alkyl and the C₁₋₄ alkoxy are each independently optionally substituted with halogen, deuterium, cyano, or hydroxy; the C₃₋₆ monocyclic cycloalkyl, the 3- to 6-membered monocyclic heterocyclyl, the 7- to 11-membered spiro-heterocyclyl, the 6- to 10-membered fused heterocyclyl, the 6- to 14-membered bridged heterocyclyl, the phenyl, the 5- or 6-membered monocyclic heteroaryl, and the 8- to 10-membered bicyclic heteroaryl are each independently optionally substituted with 1, 2, 3, or 4 group(s) selected from group S2; the 3- to 6-membered monocyclic heterocyclyl, the 7- to 11-membered spiro-heterocyclyl, the 6- to 10-membered fused heterocyclyl, the 6- to 14-membered bridged heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; R^{a}, R^{b}, R^{d}, and R^{c} are as defined above.

In an embodiment, 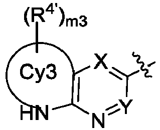 is selected from the group consisting of

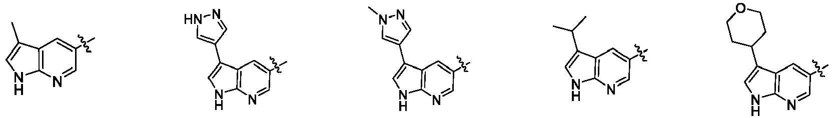

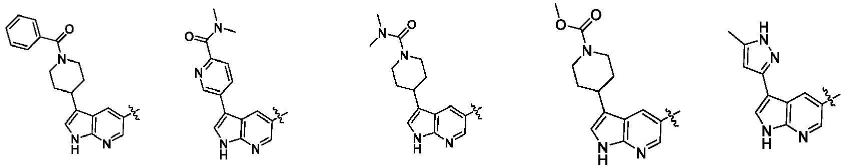

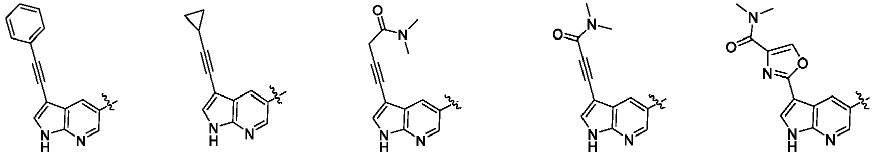

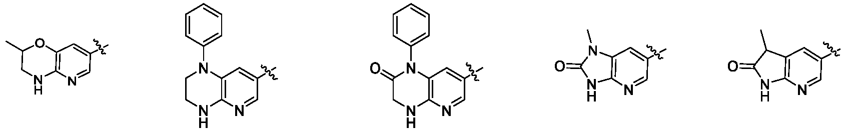

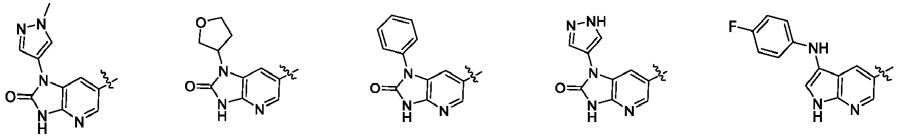

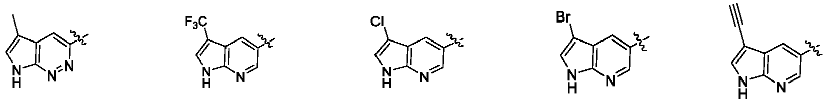

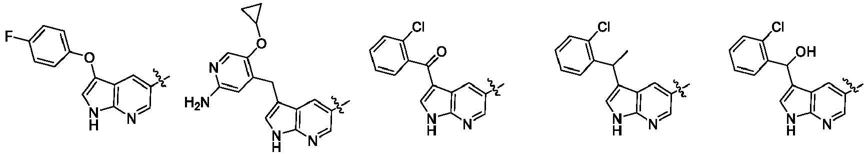

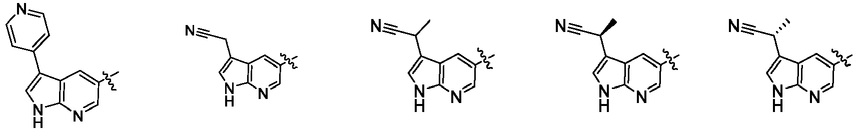

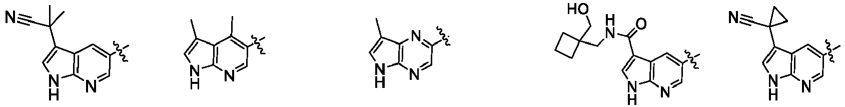

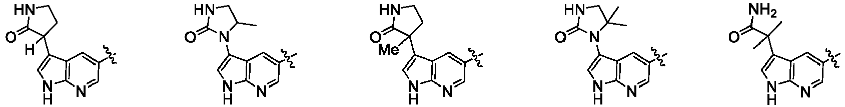

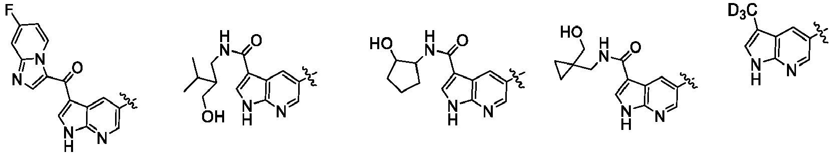

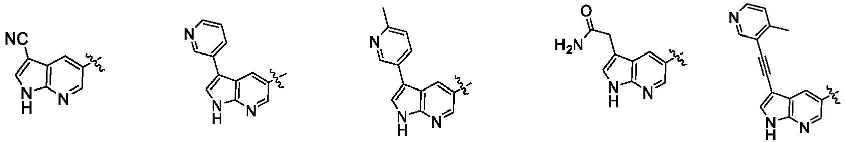

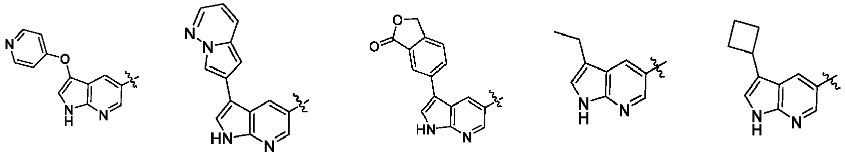

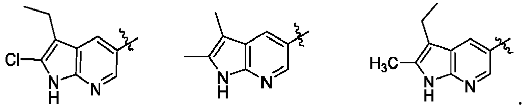

In an embodiment, each group from group S1 is independently selected from the group consisting of oxo (=O), halogen, cyano, hydroxyl, carboxyl, nitro, C₁₋₄ alkyl, C₂₋₄ alkenyl, C₂₋₄ alkynyl, C₁₋₄ alkoxy, C₃₋₆ monocyclic cycloalkyl, 3- to 20-membered heterocyclyl, phenyl, 5- or 6-membered monocyclic heteroaryl, 8- to 10-membered bicyclic heteroaryl, -O-C₃₋₆ monocyclic cycloalkyl, -O-3- to 20-membered heterocyclyl, -O-phenyl, -O-5- or 6-membered monocyclic heteroaryl, -O-8- to 10-membered bicyclic heteroaryl, -C=C-C₃₋₆ monocyclic cycloalkyl, -C=C-3- to 20-membered heterocyclyl, -C=C-phenyl, -C=C-5- or 6-membered monocyclic heteroaryl, -C=C-8- to 10-membered bicyclic heteroaryl, -C₁₋₂ alkyl-hydroxy, -C₁₋₂ alkyl-cyano, -C₁₋₂ alkyl-C₁₋₄ alkyl, -C₁₋₂ alkyl-C₁₋₄ alkoxy, -C₁₋₂ alkyl-C₃₋₆ monocyclic cycloalkyl, -C₁₋₂ alkyl-O-C₃₋₆ monocyclic cycloalkyl, -C₁₋₂ alkyl-3-to 20-membered heterocyclyl, -C₁₋₂ alkyl-O-3- to 20-membered heterocyclyl, -C₁₋₂ alkyl-phenyl, -C₁₋₂ alkyl-O-phenyl, -C₁₋₂ alkyl-5- or 6-membered monocyclic heteroaryl, -C₁₋₂ alkyl-O-5- or 6-membered monocyclic heteroaryl, -C₁₋₂ alkyl-8- to 10-membered bicyclic heteroaryl, -C₁₋₂ alkyl-O-8- to 10-membered bicyclic heteroaryl, -S(=O)₂-C₁₋₄ alkyl, -S(=O)₂-C₃₋₆ monocyclic cycloalkyl, -S(=O)₂-3- to 20-membered heterocyclyl, -C(=O)O-C₁₋₄ alkyl, -C(=O)O-C₃₋₆ monocyclic cycloalkyl, -C(=O)-C₁₋₄ alkyl, -C(=O)-C₃₋₆ monocyclic cycloalkyl, -C(=O)-phenyl, -NR^{a1}R^{b1}, -C(=O)-NR^{a1}R^{b1}, -OR^{c1}, -C₁₋₂ alkyl-S(=O)₂-C₁₋₄ alkyl, -C₁₋₂ alkyl-S(=O)₂-C₃₋₆ monocyclic cycloalkyl, -C₁₋₂ alkyl-S(=O)₂-3- to 20-membered heterocyclyl, -C₁₋₂ alkyl-C(=O)O-C₁₋₄ alkyl, -C₁₋₂ alkyl-C(=O)O-C₃₋₆ monocyclic cycloalkyl, -C₁₋₂ alkyl-C(=O)-C₁₋₄ alkyl, -C₁₋₂ alkyl-C(=O)-C₃₋₆ monocyclic cycloalkyl, -C₁₋₂ alkyl-C(=O)-phenyl, -C(=O)-5- or 6-membered monocyclic heteroaryl, -C(=O)-8- to 10-membered bicyclic heteroaryl, -C₁₋₂ alkyl-NR^{a1}R^{b1}, -C₁₋₂ alkyl-C(=O)-NR^{a1}R^{b1}, -C≡C-C(=O)-NR^{a1}R^{b1}, -C≡C-C₁₋₂ alkyl-C(=O)-NR^{a1}R^{b1}, -C₁₋₂ alkyl-OR^{c1}, -C₁₋₂ alkyl-P(=O)-(C₁₋₄ alkyl)₂, -P(=O)-(C₁₋₄ alkyl)₂, -C₁₋₂ alkyl-NR^{d1}-C(=O)-R^{c1}, -C₁₋₂ alkyl-NR^{d1}-C(=O)-NR^{a1}R^{b1}, -C₁₋₂ alkyl-NR^{d1}-S(=O)₂-R^{c1}, -C₁₋₂ alkyl-S(=O)₂-NR^{a1}R^{b1}, -C₁₋₂ alkyl-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}; wherein the C₁₋₄ alkyl, the C₁₋₄ alkoxy, the C₂₋₄ alkenyl, and the C₂₋₄ alkynyl are each independently optionally substituted with 1, 2, or 3 group(s) selected from halogen, deuterium, cyano, or hydroxyl; the C₃₋₆ monocyclic cycloalkyl, the 3- to 20-membered heterocyclyl, the phenyl, the 5- or 6-membered monocyclic heteroaryl, and the 8- to 10-membered bicyclic heteroaryl are each independently optionally substituted with 1, 2, 3, or 4 group(s) selected from group S3; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms.

In an embodiment, each R^{a}, R^{b}, R^{a1}, and R^{b1} is independently H, C₁₋₄ alkyl, halogenated C₁₋₄ alkyl, deuterated C₁₋₄ alkyl, -C₁₋₂ alkyl-hydroxy, -C₁₋₂ alkyl-cyano, -C₁₋₂ alkyl-halogenated C₁₋₄ alkyl, -C₁₋₂ alkyl-deuterated C₁₋₄ alkyl, -C₁₋₂ alky1-C₁₋₄ alkoxy, -C₁₋₂ alkyl-halogenated C₁₋₄ alkoxy, -C₁₋₂ alkyl-deuterated C₁₋₄ alkoxy, C₃₋₆ monocyclic cycloalkyl, -C₁₋₂ alkyl-C₃₋₆ monocyclic cycloalkyl, -C₁₋₂ alkyl-O-C₃₋₆ monocyclic cycloalkyl, 3- to 6-membered monocyclic heterocyclyl, -C₁₋₂ alkyl-3- to 6-membered monocyclic heterocyclyl, -C₁₋₂ alkyl-O-3- to 6-membered monocyclic heterocyclyl, phenyl, -C₁₋₂ alkyl-phenyl, 5- or 6-membered monocyclic heteroaryl, -C₁₋₂ alkyl-5- or 6-membered monocyclic heteroaryl, 8- to 10-membered bicyclic heteroaryl, -C₁₋₂ alkyl-8-to 10-membered bicyclic heteroaryl, -S(=O)₂-C₁₋₄ alkyl, -S(=O)₂-C₃₋₆ monocyclic cycloalkyl, -S(=O)₂-3- to 6-membered monocyclic heterocyclyl, -C(=O)-C₁₋₄ alkyl, -C(=O)-C₃₋₆ monocyclic cycloalkyl, -C(=O)-3- to 6-membered monocyclic heterocyclyl; wherein the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are optionally substituted with 1 or 2 group(s) selected from the group consisting of halogen, hydroxyl, carboxyl, nitro, C₁₋₄ alkyl, halogenated C₁₋₄ alkyl, deuterated C₁₋₄ alkyl, C₁₋₄ alkoxy, halogenated C₁₋₄ alkoxy, deuterated C₁₋₄ alkoxy, -NH₂, -NHC₁₋₄ alkyl, -N(C₁₋₄ alkyl)₂, -C₁₋₂ alkyl-P(=O)-(C₁₋₄ alkyl)₂, -P(=O)-(C₁₋₄ alkyl)₂; or
each R^{a} and R^{b} together with the nitrogen atom to which the R^{a} and R^{b} are attached form a 3- to 20-membered heterocyclyl group; each R^{a1} and R^{b1} together with the nitrogen atom to which the R^{a1} and R^{b1} are attached form a 3- to 20-membered heterocyclyl group; wherein the 3- to 20-membered heterocyclyl is each independently optionally substituted with 1 or 2 group(s) selected from the group consisting of halogen, hydroxyl, carboxyl, nitro, C₁₋₄ alkyl, halogenated C₁₋₄ alkyl, deuterated C₁₋₄ alkyl, C₁₋₄ alkoxy, halogenated C₁₋₄ alkoxy, deuterated C₁₋₄ alkoxy, -NH₂, -NHC₁₋₄ alkyl, -N(C₁₋₄ alkyl)₂, -C₁₋₂ alkyl-P(=O)-(C₁₋₄ alkyl)₂, -P(=O)-(C₁₋₄ alkyl)₂.

In an embodiment, each R^{d} and R^{d1} is independently H, C₁₋₄ alkyl, or deuterated C₁₋₄ alkyl. In an embodiment, each R^{c} and R^{c1} is independently H, C₁₋₄ alkyl, halogenated C₁₋₄ alkyl, deuterated C₁₋₄ alkyl, C₁₋₄ alkoxy, halogenated C₁₋₄ alkoxy, deuterated C₁₋₄ alkoxy, -C₁₋₂ alkyl-halogenated C₁₋₄ alkyl, -C₁₋₂ alkyl-deuterated C₁₋₄ alkyl, -C₁₋₂ alkyl-C₁₋₄ alkoxy, -C₁₋₂ alkyl-halogenated C₁₋₄ alkoxy, -C₁₋₂ alkyl-deuterated C₁₋₄ alkoxy, C₃₋₆ monocyclic cycloalkyl, -C₁₋₂ alkyl-C₃₋₆ monocyclic cycloalkyl, -C₁₋₂ alkyl-O-C₃₋₆ monocyclic cycloalkyl, 3- to 6-membered monocyclic heterocyclyl, -C₁₋₂ alkyl-3- to 6-membered monocyclic heterocyclyl, -C₁₋₂ alkyl-O-3- to 6-membered monocyclic heterocyclyl, phenyl, -C₁₋₂ alkyl-phenyl, 5- or 6-membered monocyclic heteroaryl, -C₁₋₂ alkyl-5- or 6-membered monocyclic heteroaryl, 8- to 10-membered bicyclic heteroaryl, or -C₁₋₂ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are optionally substituted with 1 or 2 group(s) selected from the group consisting of halogen, hydroxyl, carboxyl, nitro, C₁₋₄ alkyl, halogenated C₁₋₄ alkyl, deuterated C₁₋₄ alkyl, C₁₋₄ alkoxy, halogenated C₁₋₄ alkoxy, deuterated C₁₋₄ alkoxy, -NH₂, -NHC₁₋₄ alkyl, -N(C₁₋₄ alkyl)₂, -C₁₋₂ alkyl-P(=O)-(C₁₋₄ alkyl)₂, -P(=O)-(C₁₋₄ alkyl)₂.

In an embodiment, each group from group S3 is independently selected from the group consisting of oxo (C=O), halogen, hydroxy, carboxy, nitro, C₁₋₄ alkyl, halogenated C₁₋₄ alkyl, deuterated C₁₋₄ alkyl, C₁₋₄ alkoxy, halogenated C₁₋₄ alkoxy, deuterated C₁₋₄ alkoxy, -NH₂, -NHC₁₋₄ alkyl, -N(C₁₋₄ alkyl)₂, -C₁₋₂ alkyl-P(=O)-(C₁₋₄ alkyl)₂, -P(=O)-(C₁₋₄ alkyl)₂. In an embodiment, the -C₁₋₂ alkyl- or -C₃₋₆ monocyclic cycloalkyl- is unsubstituted; or hydrogen atoms of the -C₁₋₂ alkyl- are each independently substituted with a group selected from halogen, cyano, hydroxy, C₁₋₄ alkyl, halogenated C₁₋₄ alkyl, deuterated C₁₋₄ alkyl, -CH₂-hydroxy, -CH₂-cyano, and phenyl, or two hydrogen atoms attached to a same carbon atom of the C₁₋₂ alkyl are substituted with -(CH₂)ⱼ- to form a cycloalkyl group, wherein j is 2, 3, 4, 5, or 6; hydrogen atoms of the -C₃₋₆ monocyclic cycloalkyl- are each independently substituted with a group selected from halogen, cyano, hydroxy, C₁₋₄ alkyl, halogenated C₁₋₄ alkyl, deuterated C₁₋₄ alkyl.

In an embodiment, among the above groups, the 3- to 20-membered heterocyclyl is selected from the group consisting of 3- to 6-membered monocyclic heterocyclyl, 7- to 11-membered spiro-heterocyclyl, 6- to 10-membered fused heterocyclyl, 6- to 14-membered bridged heterocyclyl.

As described herein, 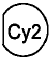 may be referred to as a Cy2 ring. 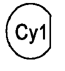 may be referred to as a Cy1 ring. 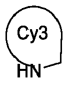 may be referred to as a Cy3 ring. 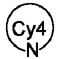 may be referred to as a Cy4 ring.

In an embodiment, the compound of formula (IC), or a pharmaceutically acceptable salt, a stereoisomer, or a solvate thereof, is prepared by a method comprising: 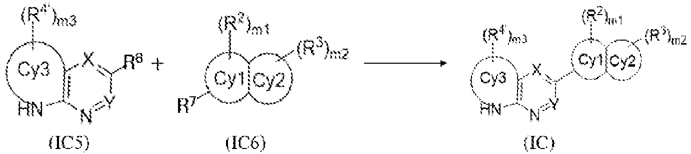 wherein X, Y, R², R³, R^{4'}, 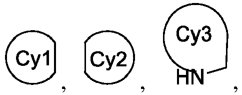 m1, m2, and m3 are as defined above.

R⁶ and R⁷ are different groups, optionally selected from -CHO, -COCH₂, -COOC₂H₅, -OCH₃, -CN, -NO₂, -F, -Cl, Br, a boronic acid group, or a boronate group.

The compound of formula (IC), or a pharmaceutically acceptable salt, a stereoisomer, a solvate or a prodrug thereof, is prepared by a Suzuki reaction of R⁶ in a compound of formula (IC5) with R⁷ in a compound of formula (IC6) in the presence of a palladium catalyst, or by a Suzuki reaction of a compound without an -R⁴ group in the compound of formula (IC5) with a compound without the -R² and/or -R³ groups in the compound of formula (IC6) to obtain an intermediate free of the -R², -R³ or -R⁴ groups, followed by a substitution reaction of the intermediate with a compound having the -R², -R³ or -R⁴ groups.

In an embodiment, a method for preparing the compound of formula (IC), or a pharmaceutically acceptable salt, a stereoisomer, a solvate or a prodrug thereof, comprising: 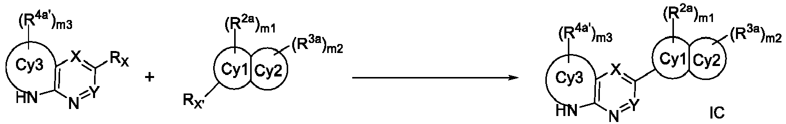 wherein X, Y, 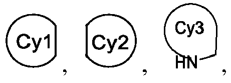 m1, m2, and m3 are as defined above.

R^{2a} is selected from R² or an intermediate group forming R²; R^{3a} is selected from R³ or an intermediate group forming R^{3a}; R^{4a'} is selected from R^{4a} or an intermediate group forming R^{4a}; R_{X} and R_{X'} are selected from a halogen atom, a boronic acid group, or a boronate group; provided that when Rx is selected from a halogen atom, R_{X'} is selected from a boronic acid group or a boronate group; when R_{X'} is selected from a halogen atom, Rx is selected from a boronic acid group or a boronate group.

Preferably, the boronic acid group or the boronate group is selected from 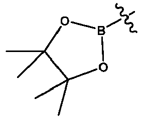 or -B(OH)₂.

In an embodiment, the compound of formula (IA) is selected from the group consisting of

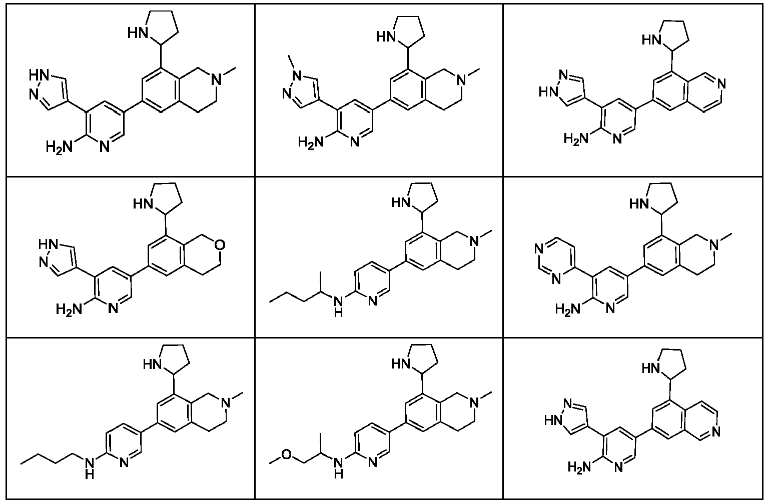

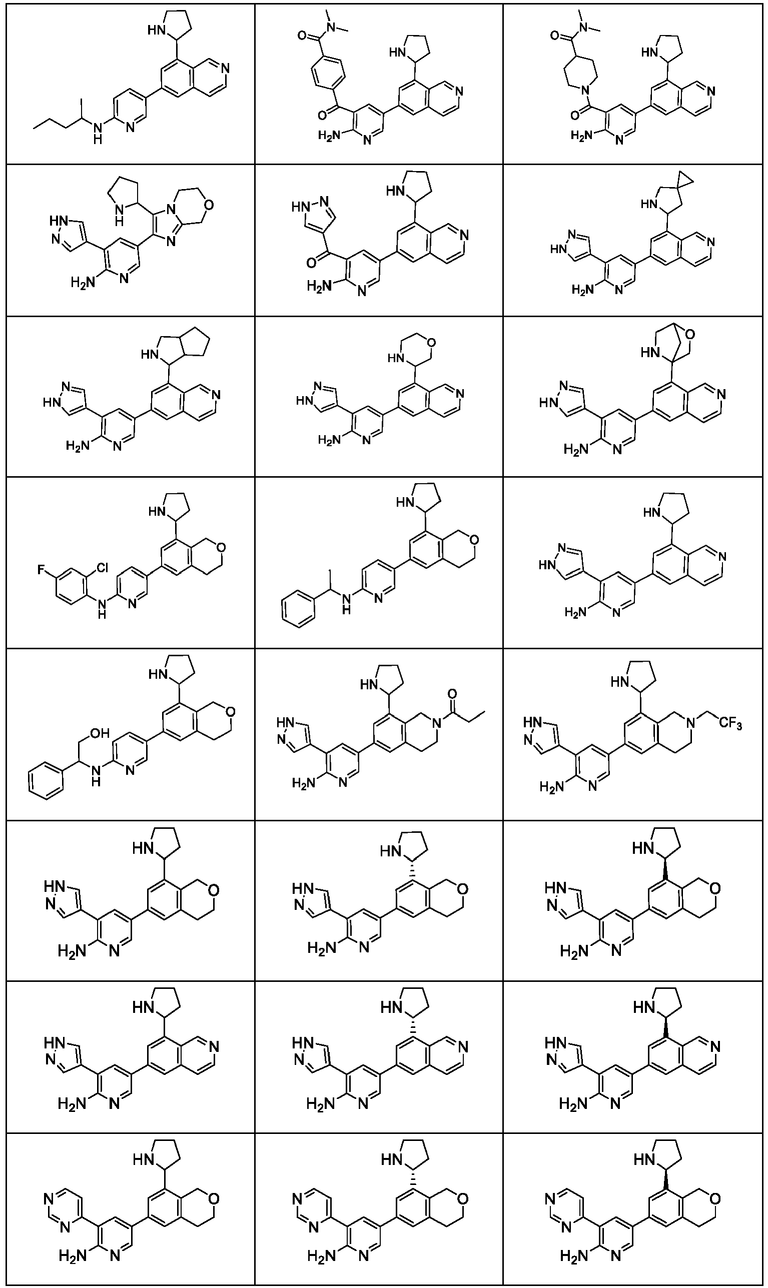

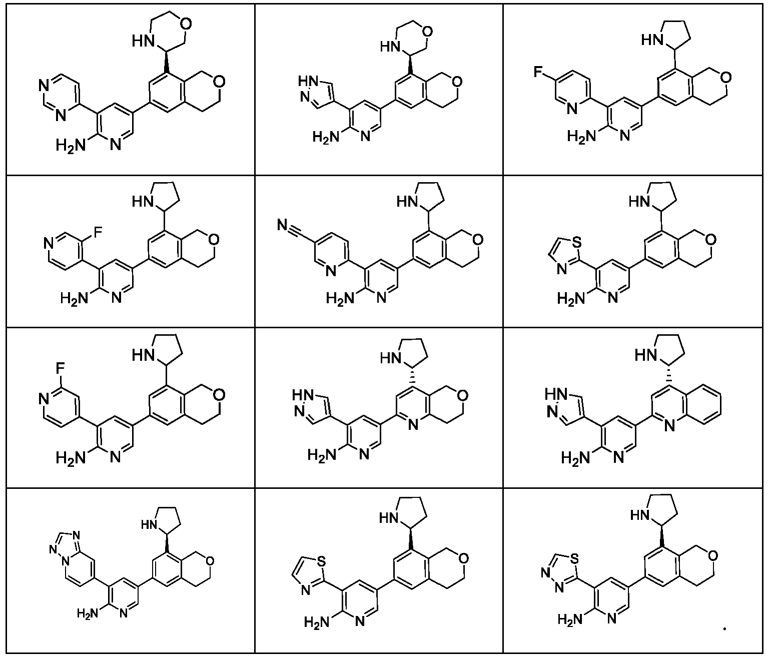

In an embodiment, the compound of formula (IC) is selected from the group consisting of

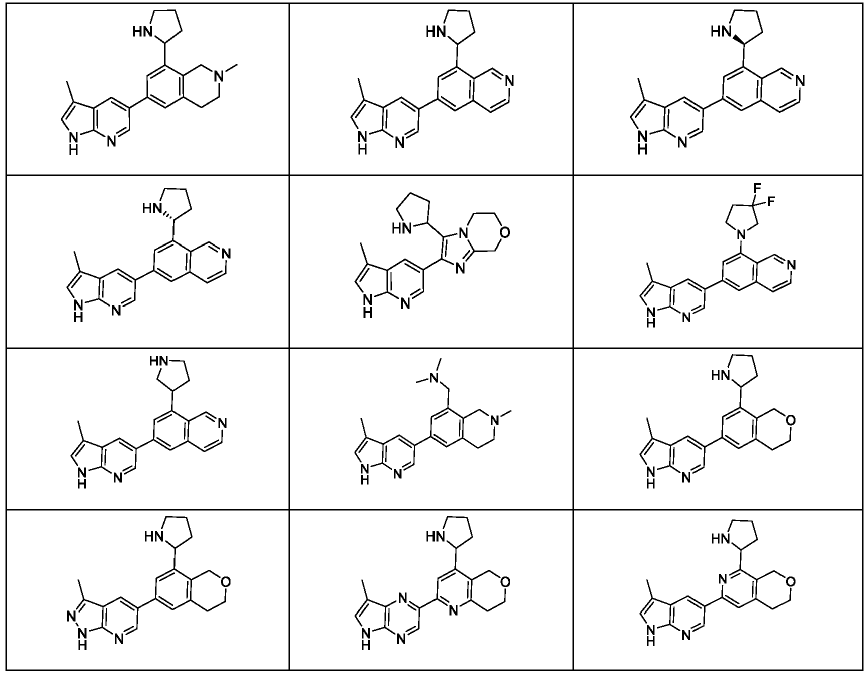

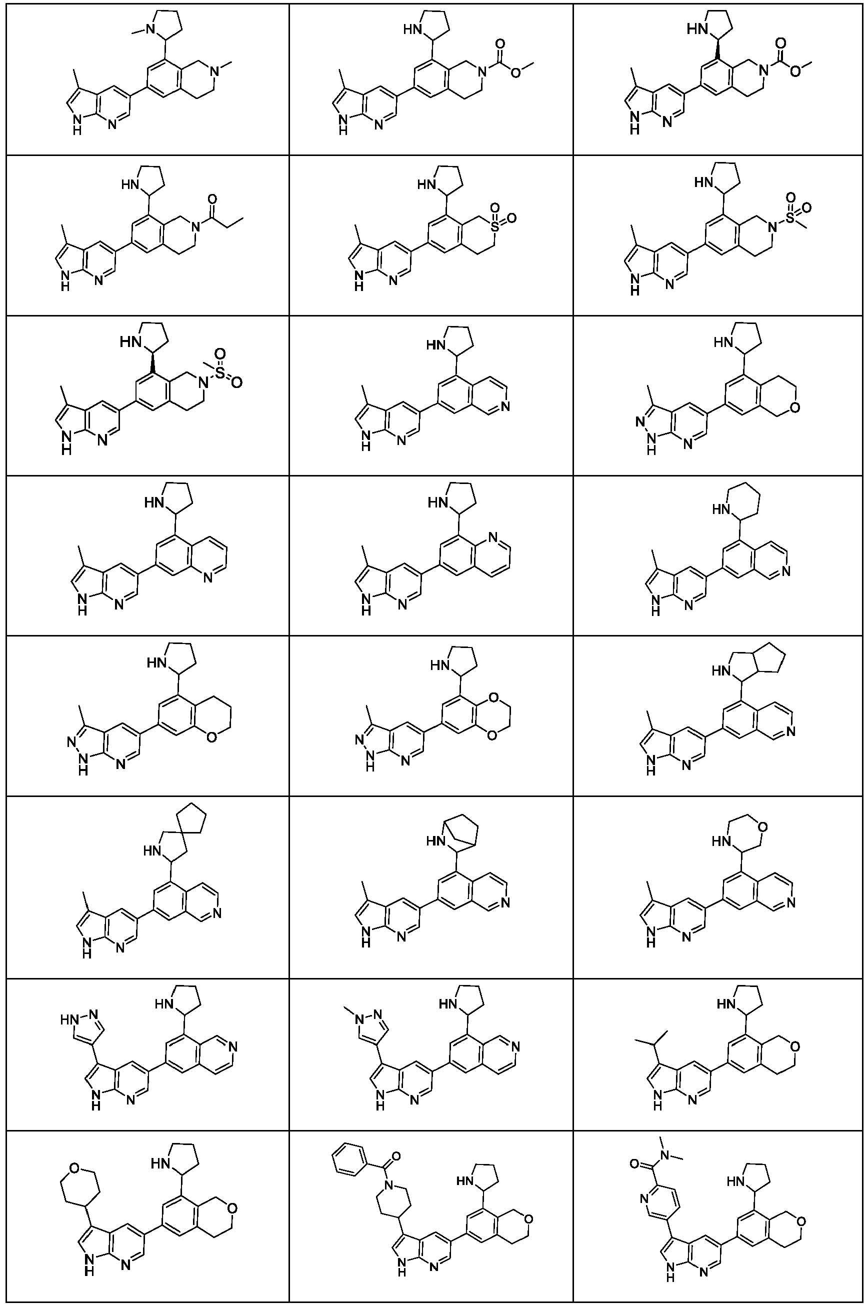

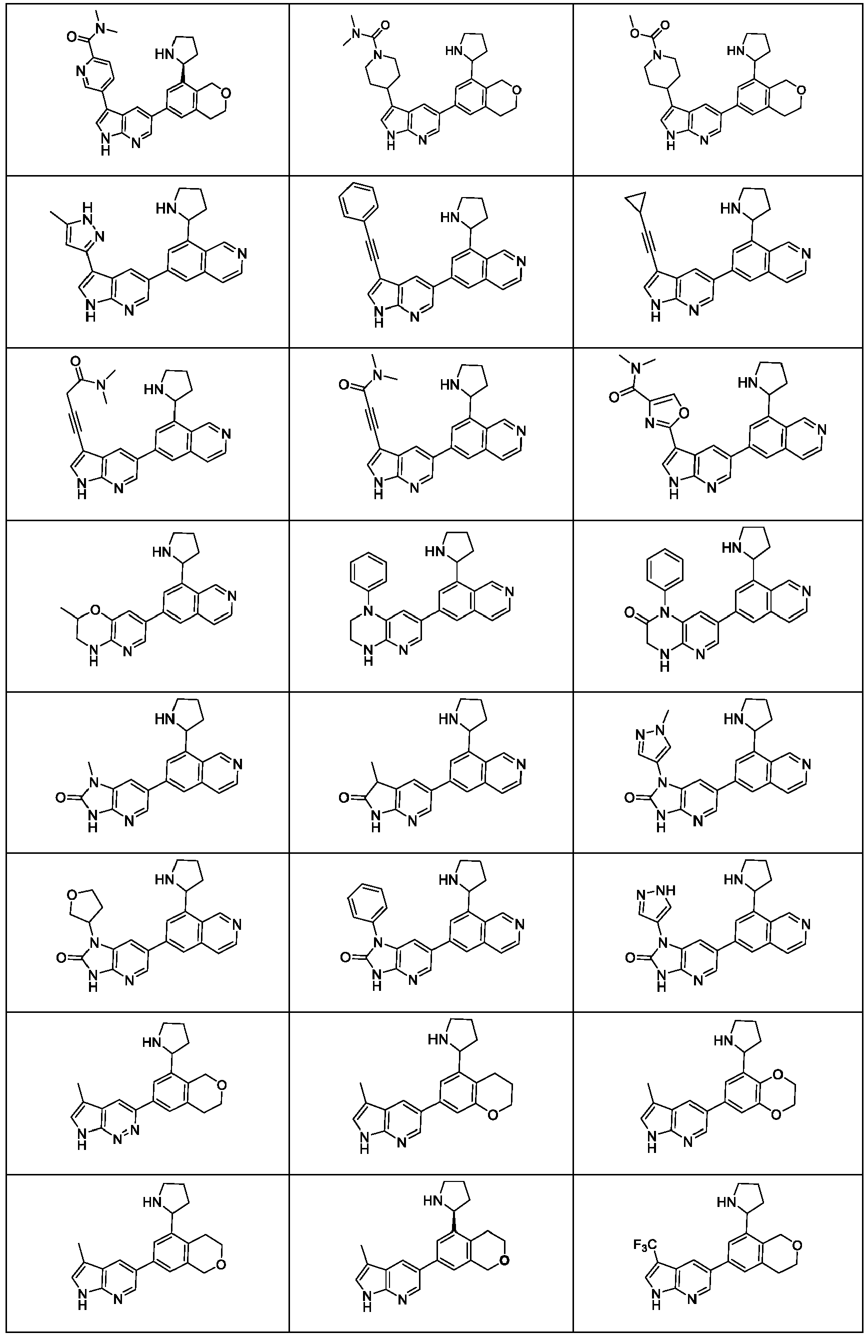

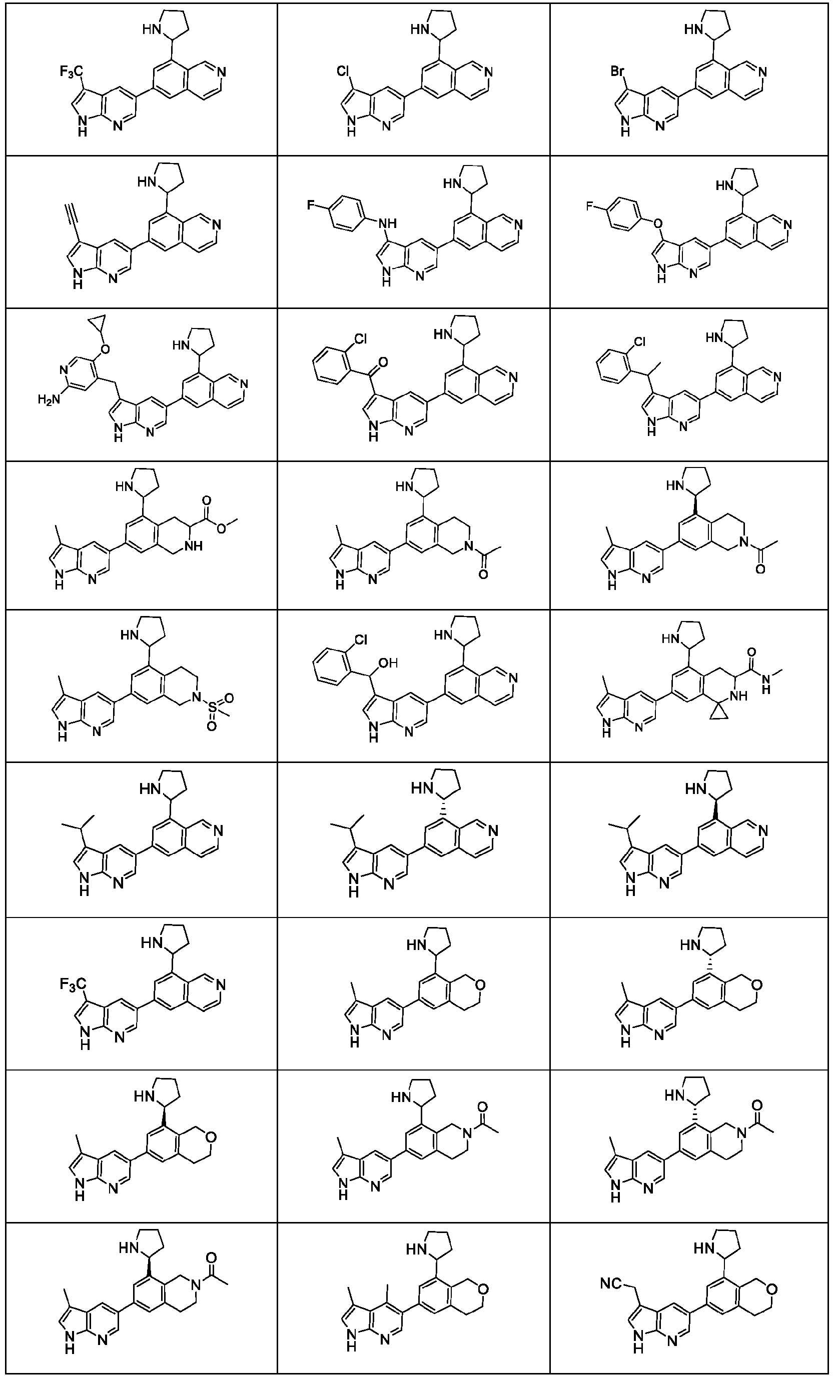

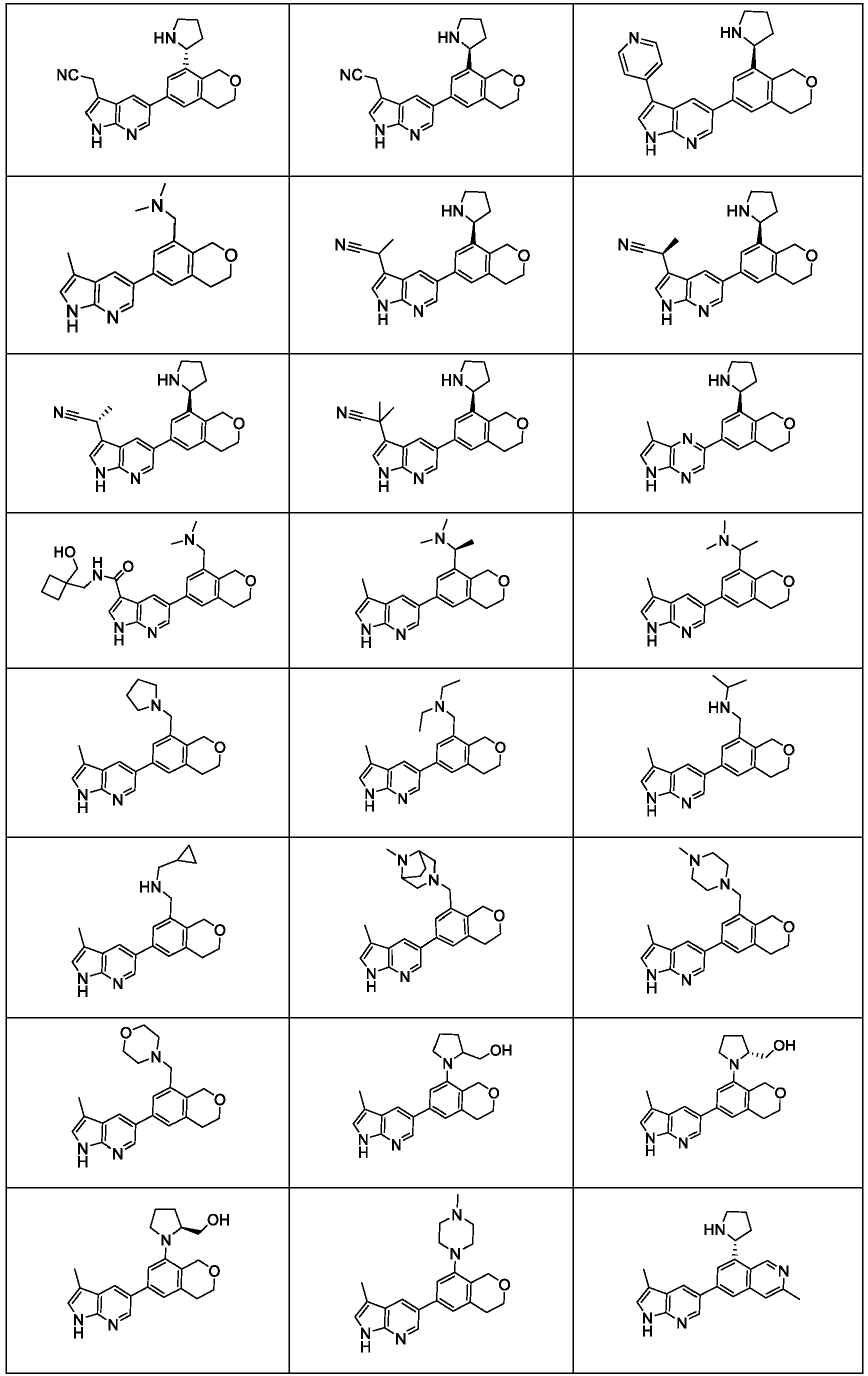

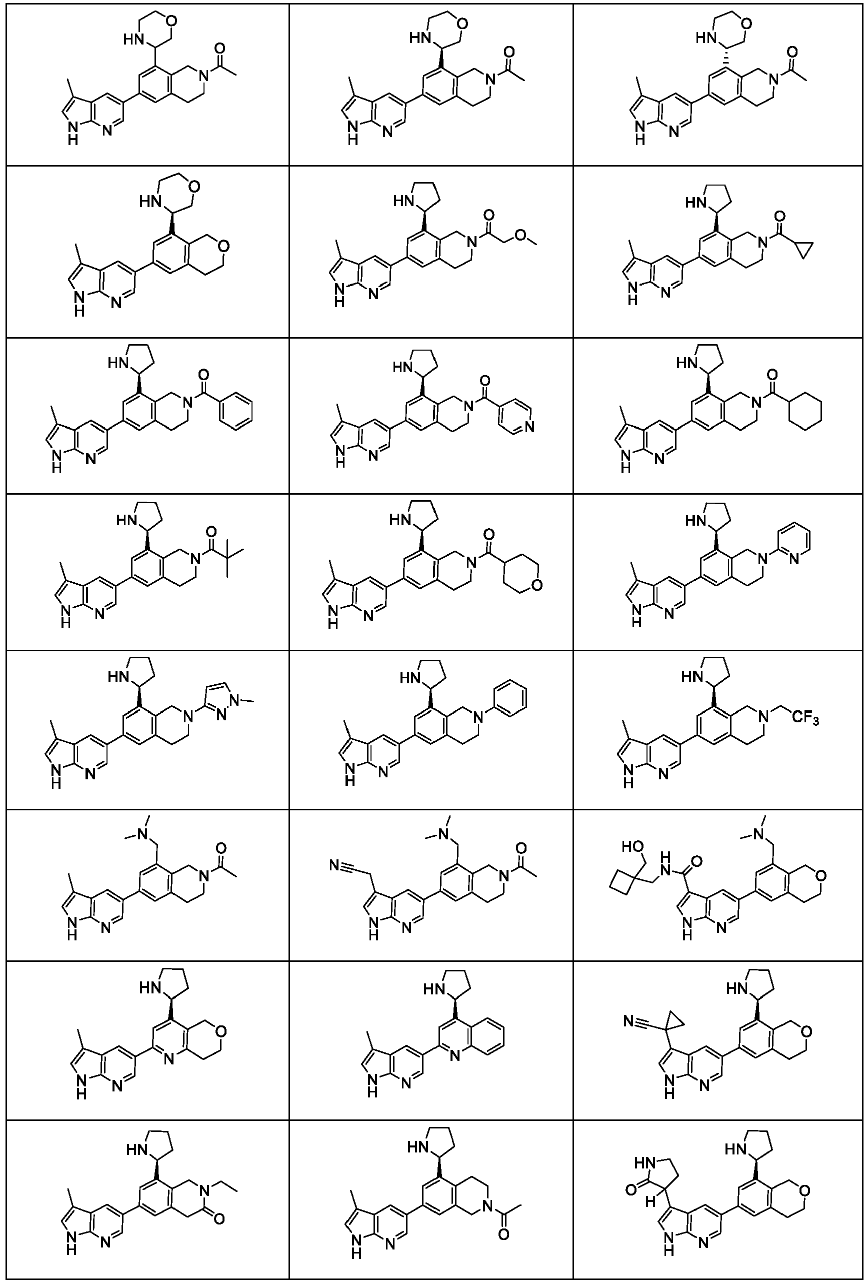

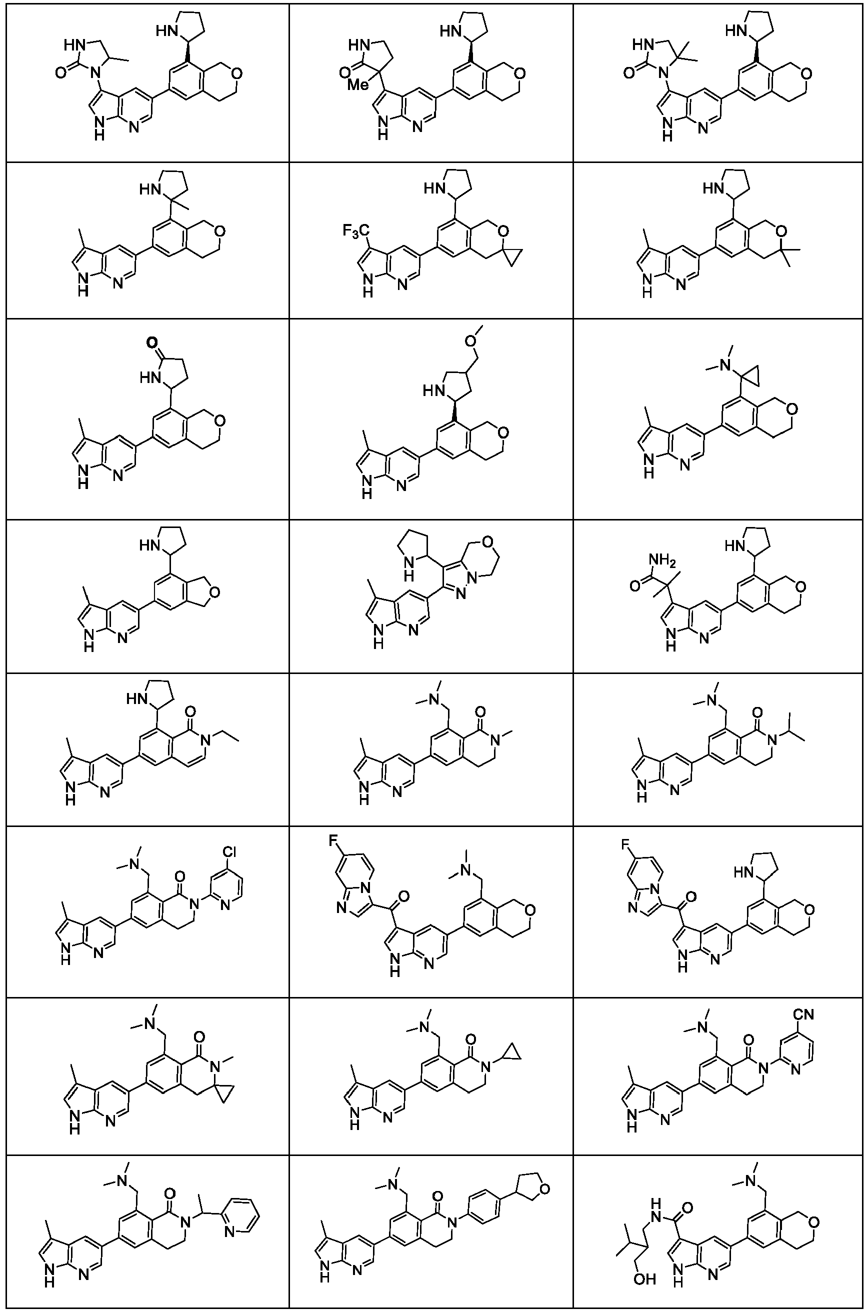

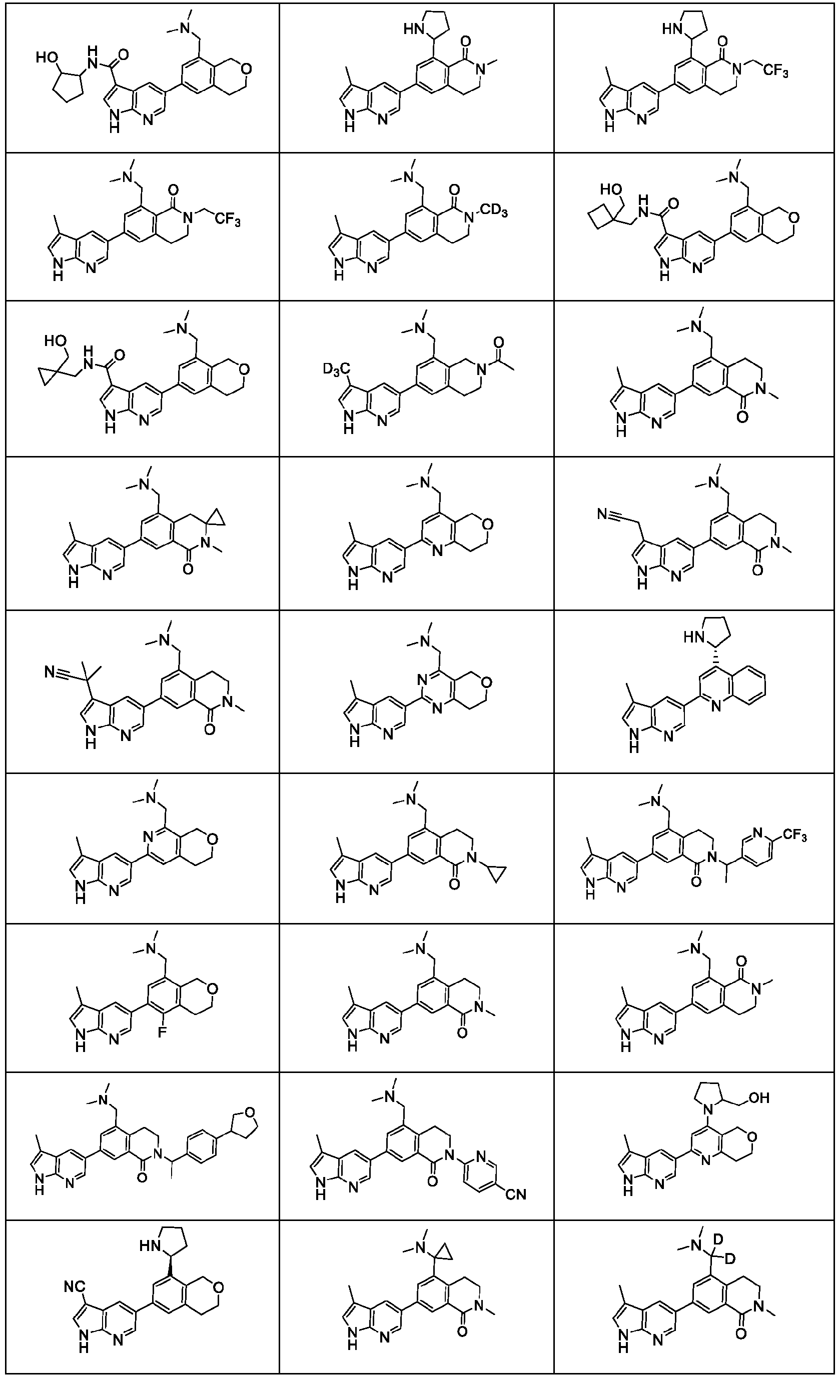

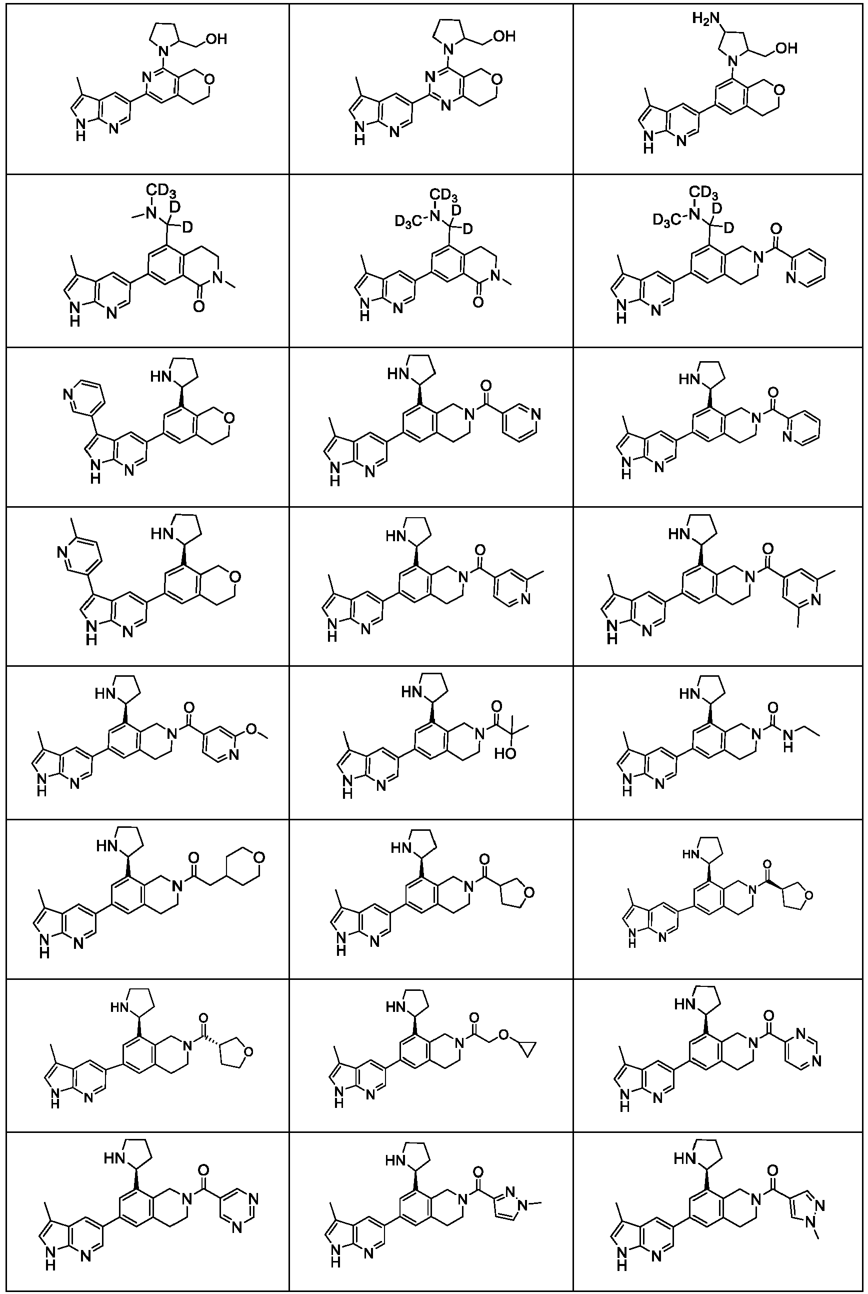

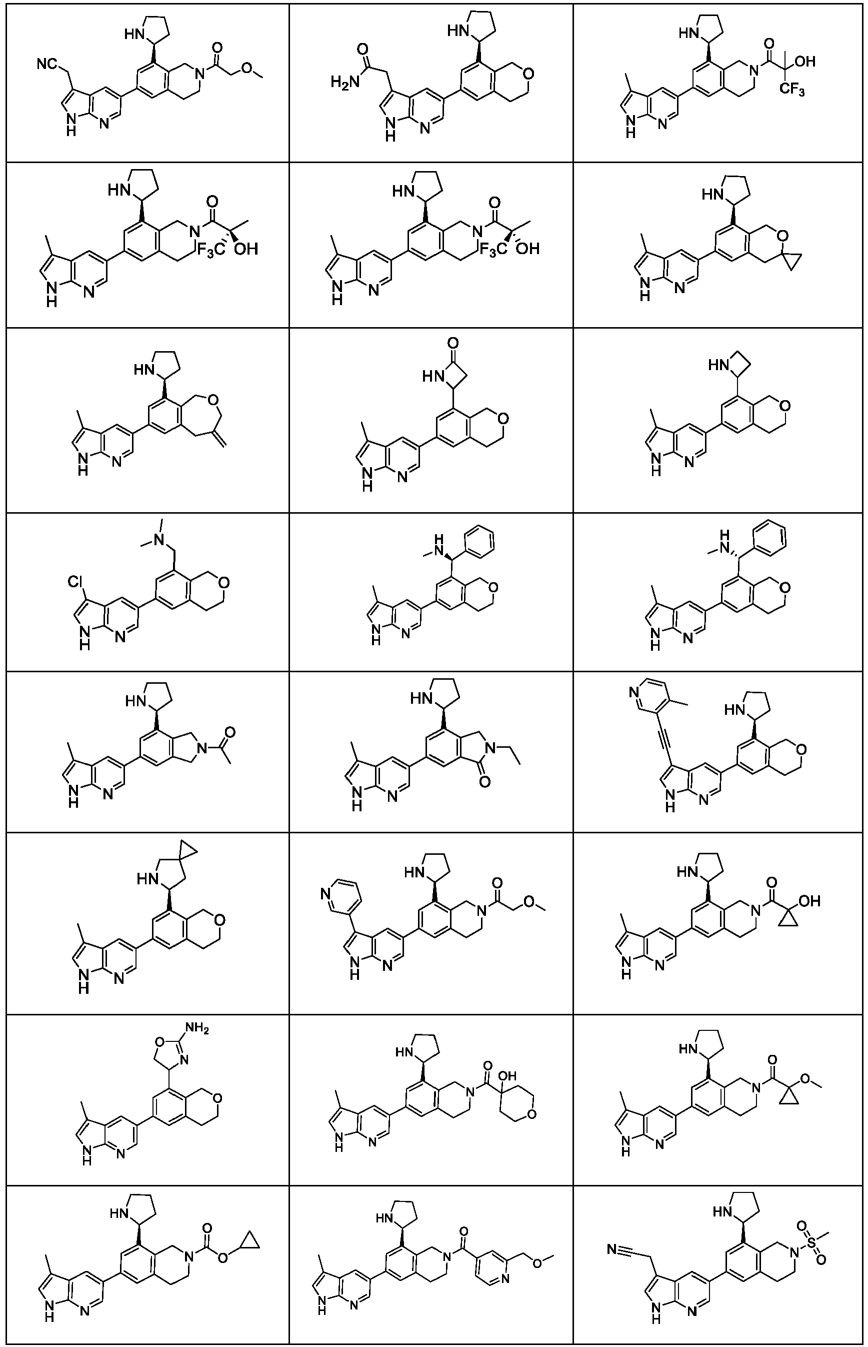

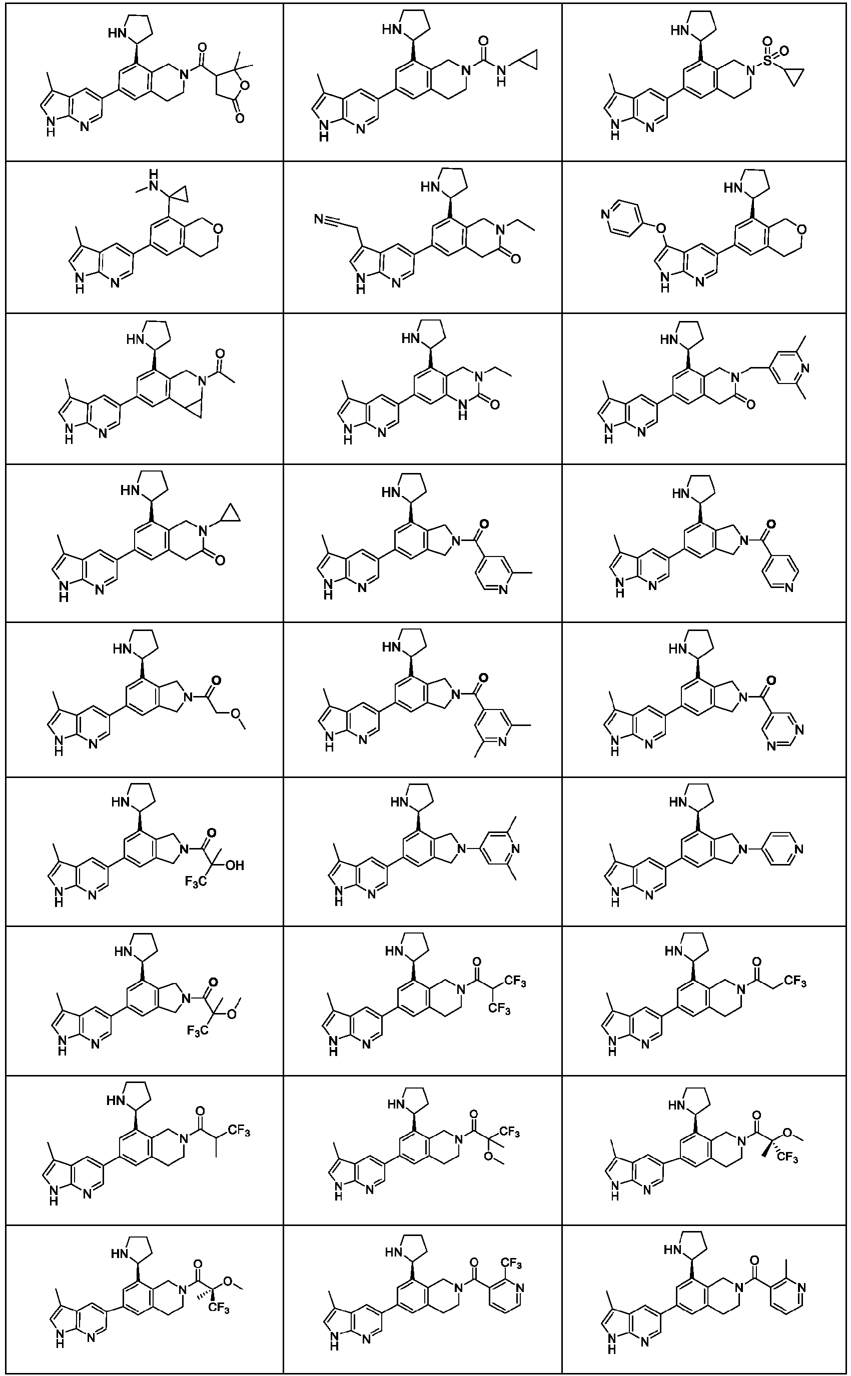

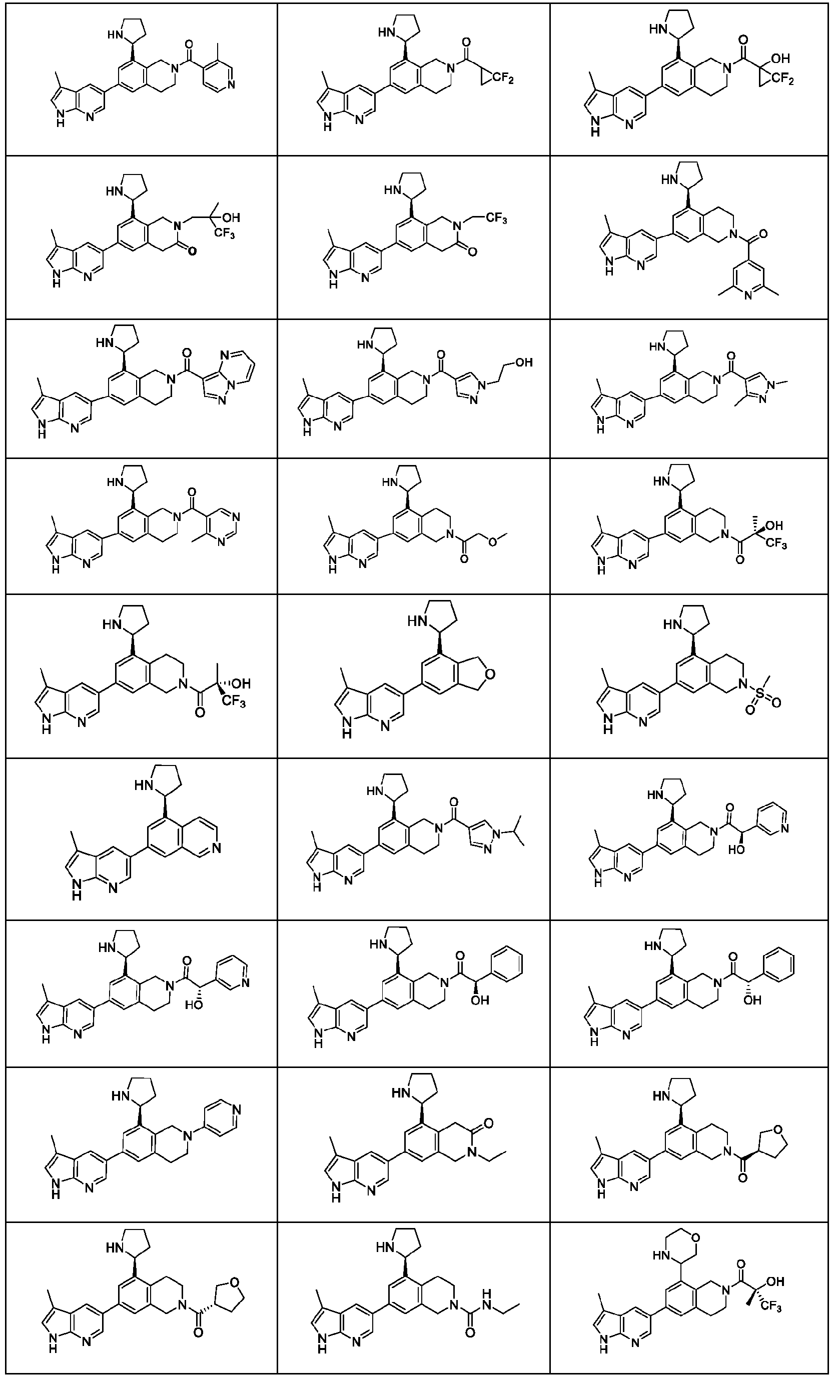

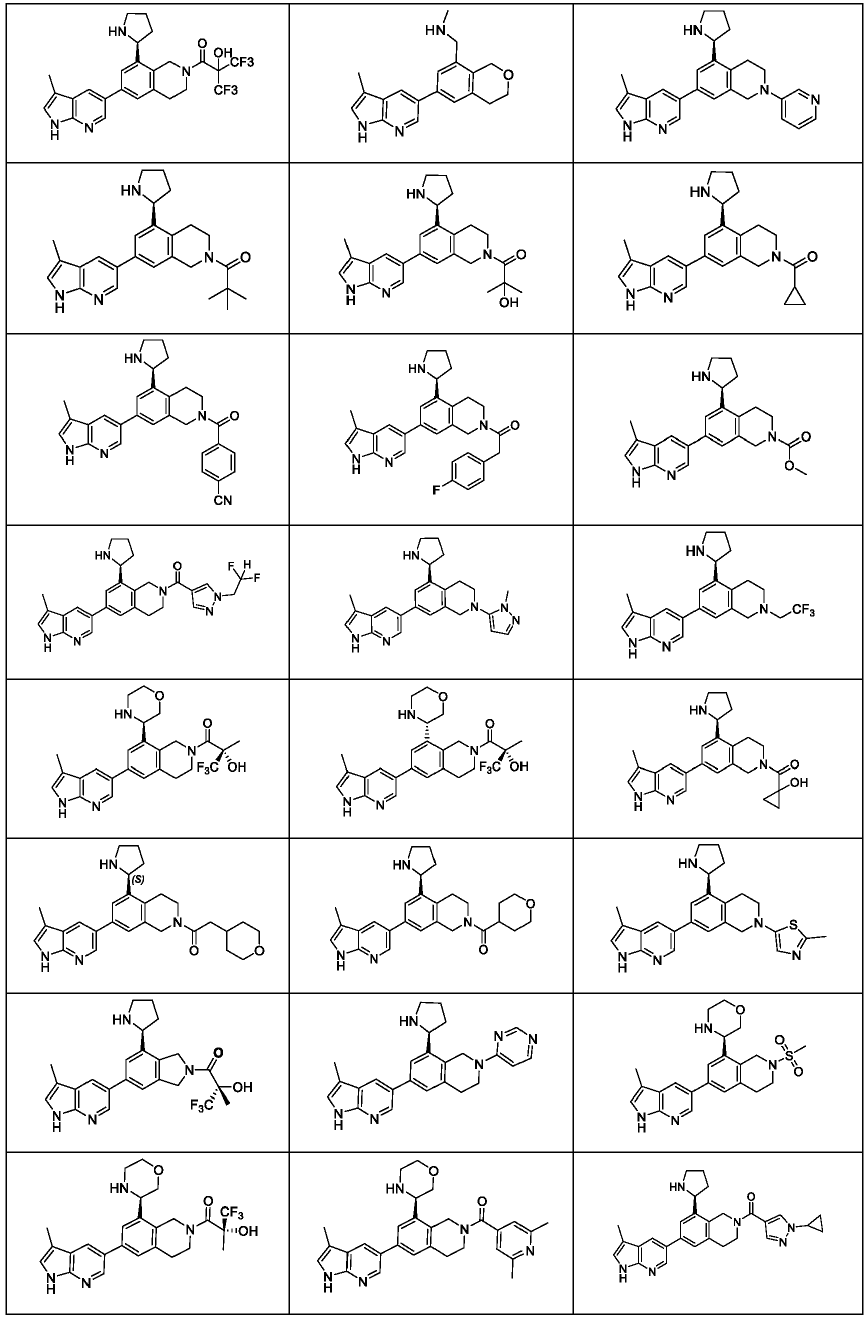

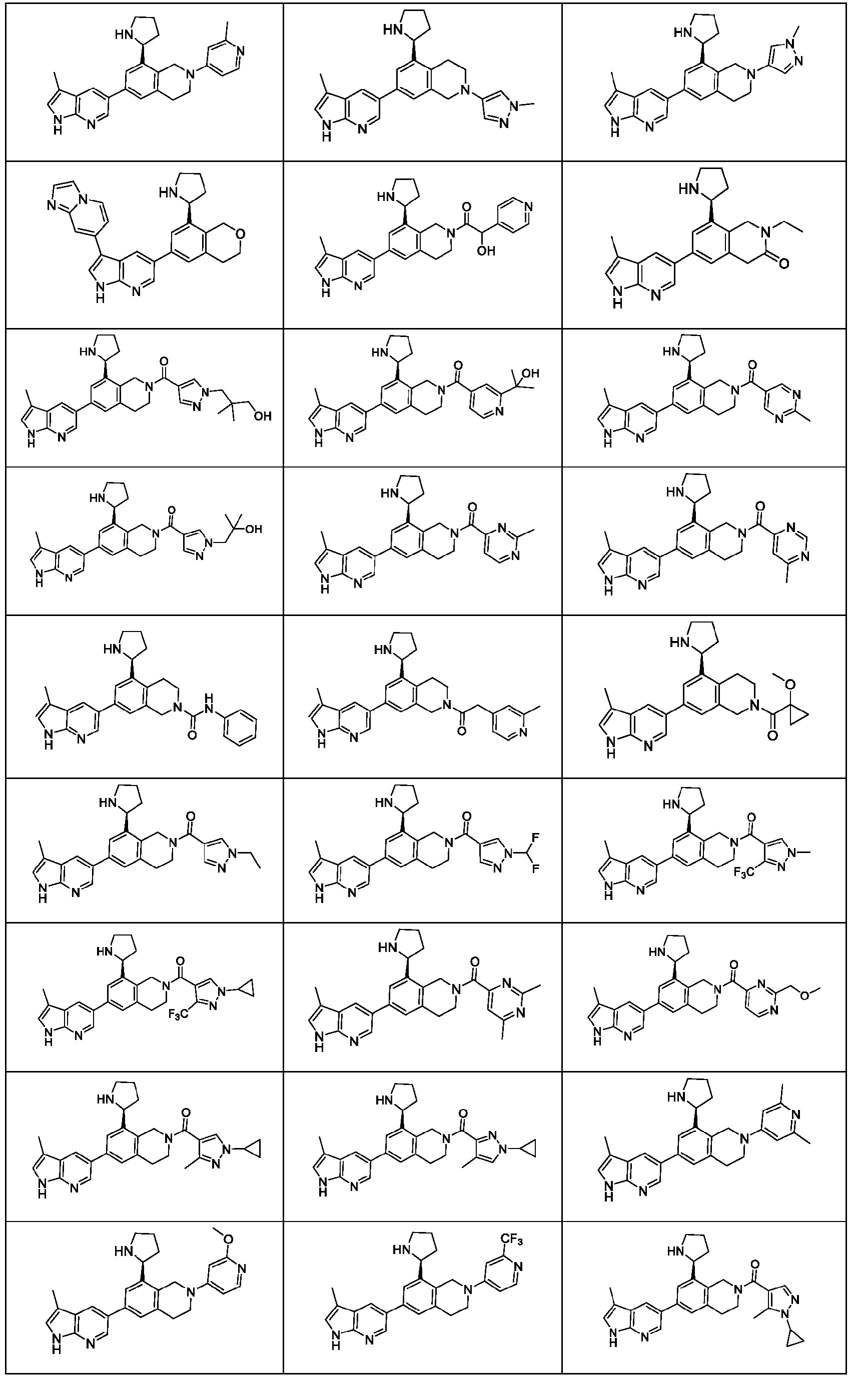

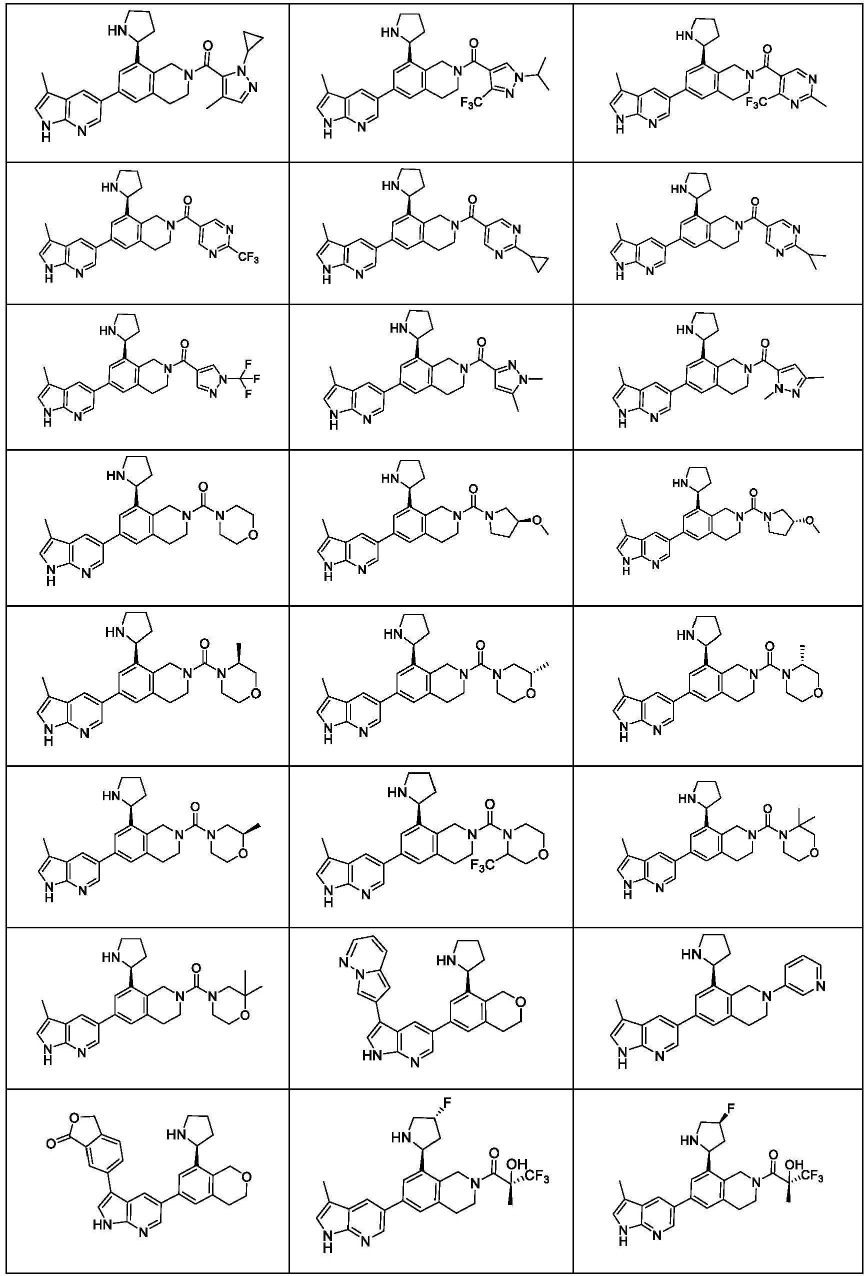

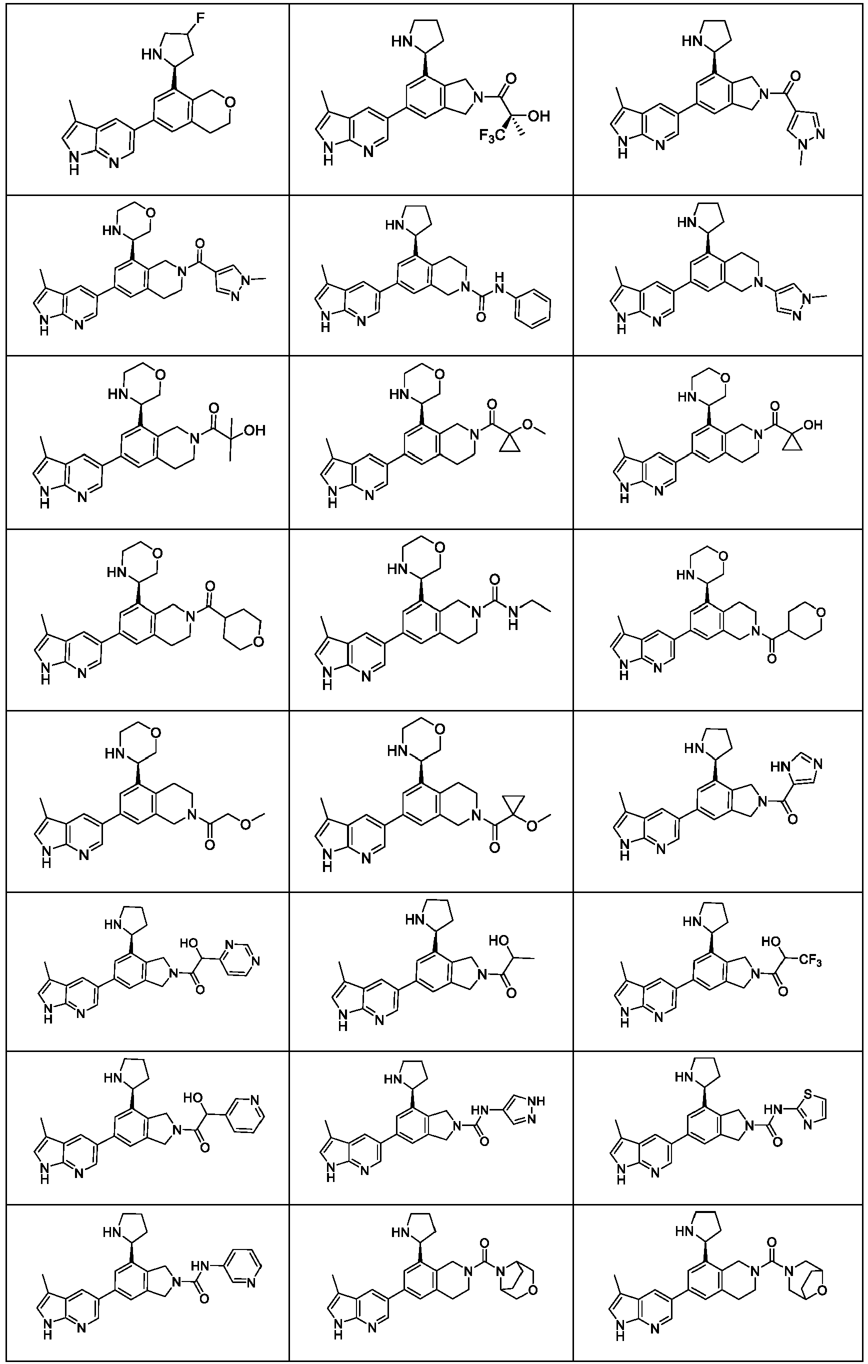

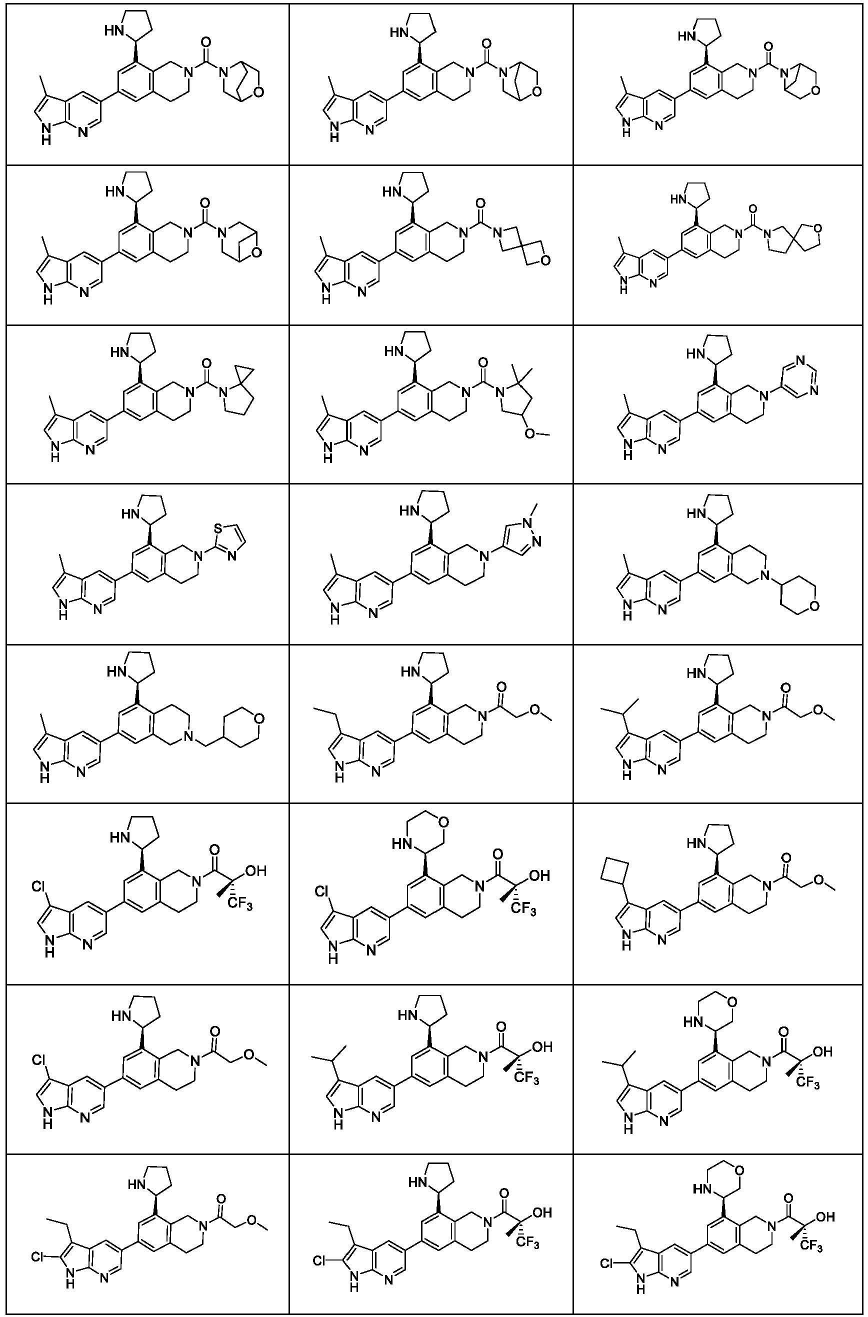

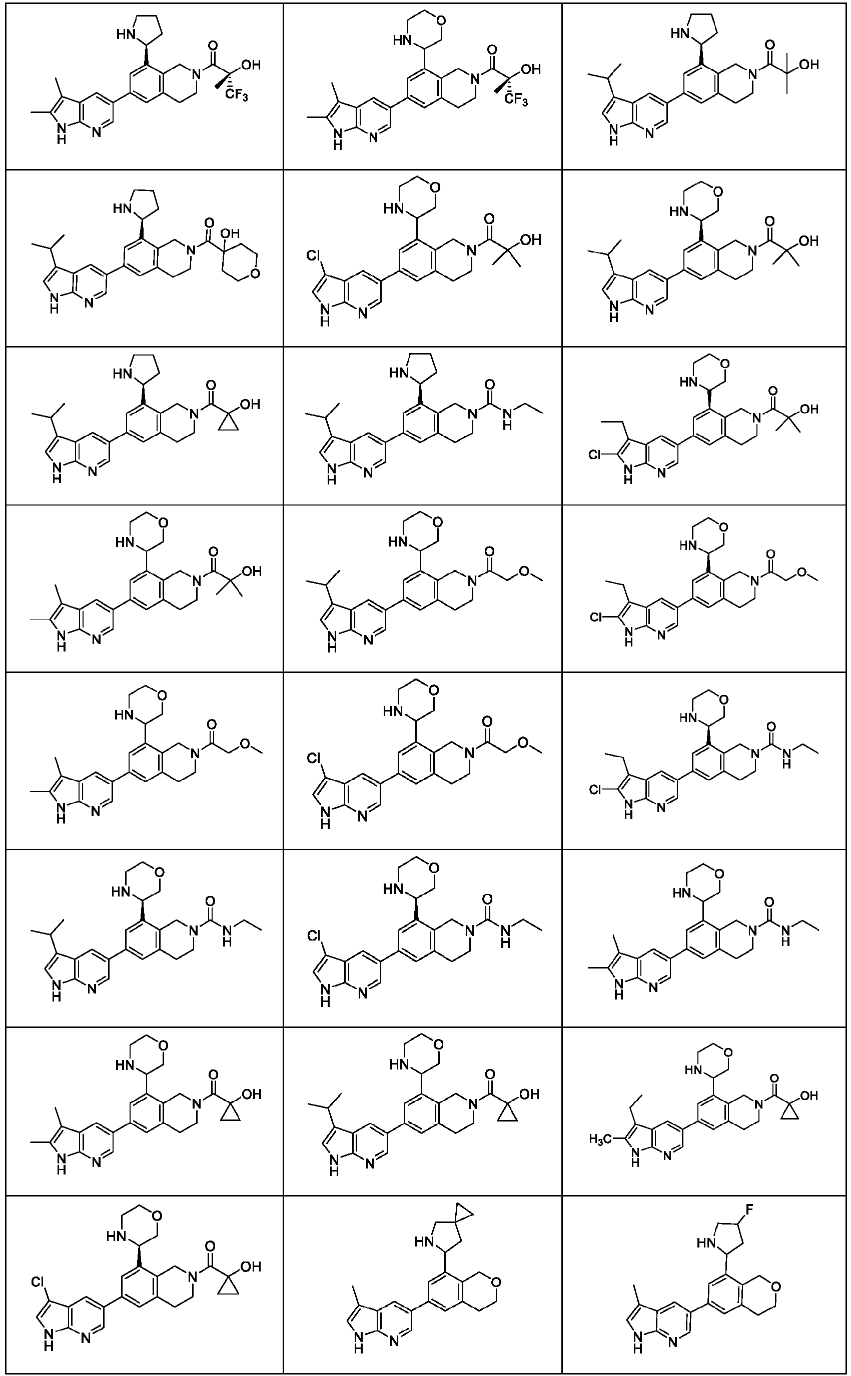

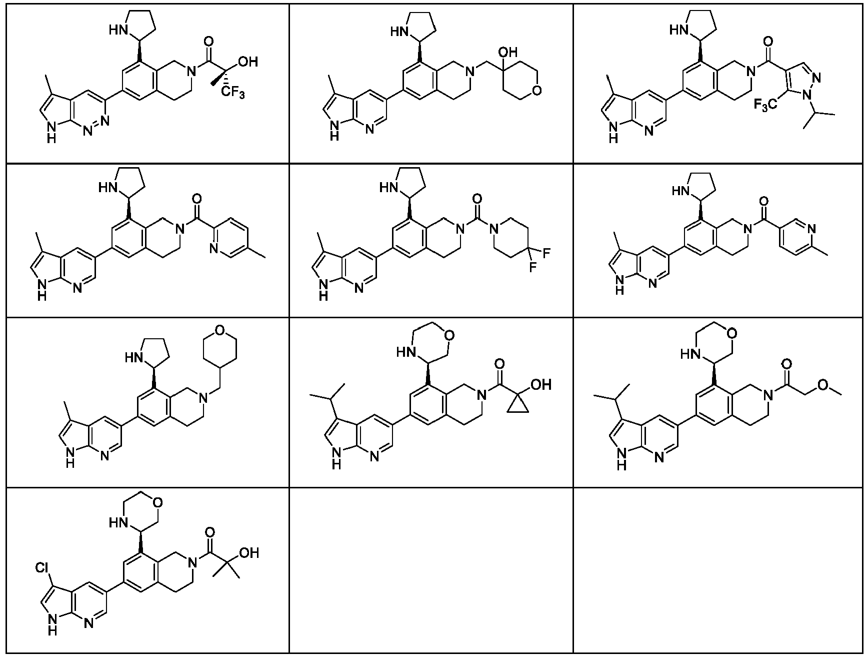

In a third aspect of the present disclosure, provided is a pharmaceutical composition comprising the compound, or the pharmaceutically acceptable salt, the stereoisomer, the solvate or the prodrug thereof of the first or second aspects described above; and a pharmaceutically acceptable carrier.

As used herein, the term "pharmaceutically acceptable carrier" refers to any formulation or carrier medium capable of delivering an effective amount of an active agent of the present disclosure without interfering with the biological activity of the active agent and without causing toxicity and side effects to the host or subject. The representative carriers include water, oil, vegetables and minerals, cream bases, lotion bases, ointment bases, and the like. These bases include suspending agents, tackifiers, transdermal enhancers, and the like. Their formulations are well known to those skilled in the cosmetic or topical pharmaceutical arts.

In an embodiment of the present disclosure, the pharmaceutical composition can be administered in any of the following ways: oral, spray inhalation, rectal, nasal, buccal, topical, parenteral such as subcutaneous, intravenous, intramuscular, intraperitoneal, intrathecal, intraventricular, intrasternal and intracranial injection or infusion, or via an explanted reservoir. Oral, intraperitoneal, or intravenous administration is preferred. When administered orally, the compounds of the present disclosure may be formulated in any orally acceptable dosage form including, but not limited to, tablets, capsules, aqueous solutions, or aqueous suspensions. Carriers used in the tablets generally include lactose and corn starch, and lubricating agents such as magnesium stearate may also be added. Diluents used in the capsules generally include lactose and dried corn starch. Aqueous suspensions are generally used by mixing active ingredients with suitable emulsifiers and suspending agents. If desired, some sweetening, flavoring, or coloring agents may also be added to the above oral dosage forms. In the case of topical administration, especially in the treatment of affected surfaces or organs readily accessible by topical external application, such as neurological diseases of the eye, skin or lower intestinal tract, the compounds of the present disclosure may be formulated in various dosage forms of topical administration depending on the affected surfaces or organs concerned. In the case of topical administration to the eye, the compounds of the present disclosure may be formulated in a dosage form of micronized suspensions or solutions in a carrier such as isotonic sterile saline with a certain pH, with or without a preservative such as benzylalkanolate chloride. For ophthalmic use, the compounds may also be formulated in an ointment such as petrolatum. When administered topically to the skin, the compounds of the present disclosure may be formulated in a suitable ointment, lotion, or cream in which active ingredients are suspended or dissolved in one or more carriers. Carriers that may be used in ointments include, but are not limited to, mineral oil, liquid petrolatum, white petrolatum, propylene glycol, polyethylene oxide, polypropylene oxide, emulsifying wax, and water; carriers that may be used in lotions or creams include, but are not limited to, mineral oil, sorbitan monostearate, Tween 60, cetyl esters wax, hexadecenyl aromatic alcohol, 2-octyldodecanol, benzyl alcohol, and water. The compounds of the present disclosure may also be administered in the form of sterile injectable formulations, including sterile injectable aqueous solutions, oil suspensions, or sterile injectable solutions. Carriers and solvents that may be used include water, Ringer's solution, and isotonic sodium chloride solution. In addition, sterilized non-volatile oils may be used as a solvent or suspending medium, such as monoglyceride or diglyceride.

In another aspect of the present disclosure, provided is uses of the compound, or the pharmaceutically acceptable salt, the stereoisomer, the solvate or the prodrug thereof of the first or second aspect described above, or the pharmaceutical composition of the third aspect described above, in preparation of a drug for preventing and/or treating diseases or conditions; the diseases or conditions are associated with HPK1 activity.

In another aspect of the present disclosure, provided is uses of the compound, or the pharmaceutically acceptable salt, the stereoisomer, the solvate or the prodrug thereof of the first or second aspect described above, or the pharmaceutical composition of the third aspect described above, in preparation of HPK1 inhibitors.

In another aspect of the disclosure, provided is a method for treating cancer, comprising a step of administering a therapeutically effective amount of the compound, or the pharmaceutically acceptable salt, the stereoisomer, the solvate or the prodrug thereof of the first or second aspect described above, or any combination thereof, or the pharmaceutical composition of the third aspect described above, to a subject in need.

The present disclosure provides a method for regulating (e.g. inhibiting or activating) HPK1 activity by contacting HPK1 with the compound or the pharmaceutically acceptable salt thereof of the present disclosure. In some embodiments, the contacting may be administering the compounds provided herein or a pharmaceutically acceptable salt thereof, to a subject. In certain embodiments, the compounds of the present disclosure, or a pharmaceutically acceptable salt thereof, may be used for therapeutic administration to enhance, stimulate and/or increase immunity against cancer. For example, a method for treating diseases or conditions associated with HPK1 activity may comprise administering a therapeutically effective amount of the compounds provided herein or a pharmaceutically acceptable salt thereof, to a subject in need. The compound of the present disclosure may be used alone, in combination with other agents or therapies, or as an adjuvant or novel adjuvant for the treatment of diseases or conditions including cancer. For the uses described herein, any compound of the present disclosure may be used, including any embodiment thereof.

In some embodiments, the diseases or conditions associated with HPK1 activity are cancer.

In some embodiments, examples of the cancer include, but are not limited to, bone cancer, pancreatic cancer, skin cancer, head and neck cancer, malignant melanoma of the skin or eye, uterine cancer, ovarian cancer, rectal cancer, anal region cancer, gastric cancer, testicular cancer, uterine cancer, fallopian tube cancer, endometrial cancer, endometrial cancer, cervical cancer, vaginal cancer, vulval cancer, Hodgkin's disease, non-Hodgkin's lymphoma, esophageal cancer, small intestine cancer, endocrine system cancer, thyroid cancer, parathyroid cancer, adrenal cancer, soft tissue sarcoma, urethral cancer, penile cancer, chronic or acute leukemia (including acute myeloid leukemia, chronic myeloid leukemia, acute lymphoblastic leukemia, chronic lymphocytic leukemia), childhood solid tumor, lymphocytic lymphoma, bladder cancer, kidney cancer, or urethral carcinoma, renal pelvis cancer, central nervous system (CNS) tumors, primary CNS lymphoma, tumor angiogenesis, spinal axis tumor, brain stem glioma, pituitary adenoma, Kaposi's sarcoma, epidermoid cancer, squamous cell carcinoma, T-cell lymphoma, environmentally-induced cancers (including those induced by asbestos), and combinations of such cancers.

In some embodiments, examples of the cancer include, but are not limited to, melanoma (e.g. metastatic malignant melanoma), kidney cancer (e.g. clear cell carcinoma), prostate cancer (e.g. hormone-refractory prostate adenocarcinoma), breast cancer, triple-negative breast cancer, colon cancer, and lung cancer (e.g. non-small cell lung cancer and small cell lung cancer).

In some embodiments, examples of the cancer include, but are not limited to, solid tumors (e.g. prostate cancer, colon cancer, esophageal cancer, endometrial cancer, ovarian cancer, uterine cancer, kidney cancer, liver cancer, pancreatic cancer, gastric cancer, breast cancer, lung cancer, head and neck cancer, thyroid cancer, glioblastoma, sarcoma, bladder cancer, and the like), hematological cancer (e.g. lymphoma, leukemia, such as acute lymphoblastic leukemia (ALL), acute myeloid leukemia (AML), chronic lymphocytic leukemia (CLL), chronic myeloid leukemia (CML), DLBCL, mantle cell lymphoma, non-Hodgkin's lymphoma (including relapsed or refractory NHL and relapsed follicular lymphoma), Hodgkin's lymphoma or multiple myeloma), and combinations of such cancers.

In some embodiments, examples of the cancer include, but are not limited to, hematological cancer, sarcoma, lung cancer, gastrointestinal cancer, genitourinary tract cancer, liver cancer, bone cancer, nervous system cancer, gynecological cancer, and skin cancer.

Examples of hematological cancers include lymphoma and leukemia, such as acute lymphoblastic leukemia (ALL), acute myeloid leukemia (AML), acute promyelocytic leukemia (APL), chronic lymphocytic leukemia (CLL), chronic myeloid leukemia (CML), diffuse large B-cell lymphoma (DLBCL), mantle cell lymphoma, non-Hodgkin's lymphoma (including relapsed or refractory NHL and relapsed follicular lymphoma), Hodgkin's lymphoma, myeloproliferative disorders (e.g. primary myelofibrosis (PMF), polycythemia vera (PV), essential thrombocythemia (ET)), myelodysplastic syndrome (MDS), T-cell acute lymphoblastic lymphoma (T-ALL), multiple myeloma, cutaneous T-cell lymphoma, Waldenstrom's macroglobulinemia, hairy cell lymphoma, chronic myeloid lymphoma, and Burkitt's lymphoma.

Examples of sarcomas include chondrosarcoma, Ewing's sarcoma, osteosarcoma, rhabdomyosarcoma, angiosarcoma, fibrosarcoma, liposarcoma, myxoma, rhabdomyoma, rhabdomyosarcoma, fibroma, lipoma, hamartoma, and teratoma. Examples of lung cancers include non-small cell lung cancer (NSCLC), small cell lung cancer, bronchial carcinoma (squamous cell, undifferentiated small cell, undifferentiated large cell, adenocarcinoma), alveolar (bronchiolar) carcinoma, bronchial adenoma, chondroma-like hamartoma, and mesothelioma.

Examples of gastrointestinal cancers include esophageal cancer, gastric cancer, pancreatic cancer, small intestine cancer, large intestine cancer, and colorectal cancer.

Examples of genitourinary tract cancers include kidney cancer, bladder and urethral cancer, prostate cancer, and testicular cancer.

Examples of liver cancers include hepatoma (hepatocellular carcinoma), cholangiocarcinoma, hepatoblastoma, angiosarcoma, hepatocellular adenoma, and hemangioma.

Examples of bone cancers include, for example, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor, chordoma, osteochondrofibroma (osteocartilaginous exostosis), benign chondroma, chondroblastoma, chondromyxoid fibroma, osteoid osteoma, and giant cell tumor.

Examples of nervous system cancers include skull cancer (osteoma, hemangioma, granuloma, xanthoma, osteitis deformans), meningeal carcinoma (meningioma, meningiosarcoma, gliomatosis), brain cancer (astrocytoma, medulloblastoma, glioma, ependymoma, embryoma (pinealoma), glioblastoma, glioblastoma multiforme, oligodendroglioma, schwannoma, retinoblastoma, congenital tumors) and spinal cord cancer (neurofibroma, meningioma, glioma, sarcoma), as well as neuroblastoma and Lhermitte-Duclos disease.

Examples of gynecological cancers include uterine cancer (endometrial cancer), cervical cancer, ovarian cancer, granulosa-membrane cell tumor, Sertoli-Leydig cell tumor, dysgerminoma, malignant teratoma), vulval cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma), and fallopian tube cancer).

Examples of skin cancers include melanoma, basal cell carcinoma, squamous cell carcinoma, Kaposi's sarcoma,Merkel cell skin cancer, dysplastic nevus, lipoma, hemangioma, dermatofibroma, and keloids.

In some embodiments, the diseases or conditions associated with HPK1 activity include, but are not limited to, sickle cell disease (e.g. sickle cell anemia), triple-negative breast cancer (TNBC), myelodysplastic syndrome, testicular cancer, biliary tract cancer, esophageal cancer, and urothelial cancer.

Examples of head and neck cancers include glioblastoma, melanoma, rhabdomyosarcoma, lymphosarcoma, osteosarcoma, squamous cell carcinoma, adenocarcinoma, oral cancer, laryngeal cancer, nasopharyngeal cancer, nasal and lateral nasal cancer, thyroid cancer, and parathyroid cancer.

As used herein, the term "subject" refers to animals, particularly mammals, preferably a human.

As used herein, the term "effective amount" or "therapeutically effective amount" refers to an amount of the drug or agent that is nontoxic but sufficient to achieve a desired effect. In an embodiment of the present disclosure, the amount of a given drug in the treatment of a patient according to the present disclosure depends on several factors, such as the specific dosing regimen, the type and severity of diseases or disorders, the uniqueness (e.g. weight) of the subject or host in need of treatment, but may be routinely determined by methods known in the art according to the particular circumstances, including, for example, the specific drug that has been employed, the route of administration, the condition being treated, and the subject or host being treated. In general, in terms of doses used for adult human treatment, the administration dose typically ranges from 0.02-5000 mg/day, such as about 1-1500 mg/day. The desired dose may be conveniently presented in one dose or as divided doses administered simultaneously (or over a short period of time) or at appropriate intervals, such as two, three, four, or more divided doses per day. It will be understood by those skilled in the art that although the above dose ranges are given, the specific effective amount may be appropriately adjusted depending on the patient's condition in conjunction with the physician's diagnosis.

As used herein, the term "pharmaceutically acceptable salt" refers to a salt of the compound of the present disclosure that is pharmaceutically acceptable and that possesses the pharmacological activity of the parent compound. Such salts include acid addition salts formed with inorganic acids such as nitric acid, phosphoric acid, carbonic acid, and the like, or with organic acids such as propionic acid, hexanoic acid, cyclopentanoic acid, glycolic acid, pyruvic acid, gluconic acid, stearic acid, muconic acid, and the like; or salts formed when an acidic proton present on the parent compound is substituted with a metal ion such as an alkali metal ion or an alkaline earth metal ion; or coordination compounds formed with organic bases such as ethanolamine. The pharmaceutically acceptable salts of the present disclosure may be synthesized from the parent compounds containing an acid radical or a basic group by a conventional chemical method. Generally, such salts are prepared by reacting these compounds in free acid or base form with a stoichiometric amount of the appropriate base or acid in water or an organic solvent or a mixture of both. Generally, nonaqueous media such as ether, ethyl acetate, ethanol, isopropanol, or acetonitrile are preferred. In addition to salt forms, the compounds provided by the present disclosure may also exist in the form of prodrugs. Prodrugs of the compounds described herein are readily converted to the compounds of the present disclosure by chemical changes under physiological conditions. In addition, the prodrugs may be converted to the compounds of the present disclosure by chemical or biochemical methods in vivo.

As used herein, the terms "solvent compound" and "solvate" refer to a material formed by the combination of the compound of the present disclosure and a pharmaceutically acceptable solvent. Pharmaceutically acceptable solvents include acetic acid, and the like. Solvent compounds include both stoichiometric amounts of solvent compounds and non-stoichiometric amounts of solvent compounds. Certain compounds of the present disclosure may exist in non-solvated or solvated forms. In general, the solvated forms are equivalent to non-solvated forms and are encompassed within the scope of the present disclosure.

As used herein, the term "stereoisomer" includes conformational isomers and configurational isomers, wherein the configurational isomers primarily include cis-trans isomers and optical isomers. The compounds described in the present disclosure may exist in the form of stereoisomers and thus encompass all possible stereoisomeric forms, including, but not limited to, cis-trans isomers, tautomers, enantiomers, diastereomers, atropisomers, and the like. The compounds described in the present disclosure may also exist in the form of any combination or any mixture of the above stereoisomers, such as equivalent mixtures of mesomers, racemates and atropisomers, for example, single enantiomers, single diastereomers, or a mixture of the above, or single atropisomers or a mixture thereof. When the compounds described in the present disclosure contain olefinic double bonds, they include cis-isomers and trans-isomers, as well as any combination thereof, unless specified otherwise. The atropisomers of the present disclosure are stereoisomers based on axial or planar chirality resulting from restricted intramolecular rotation. And as drugs, stereoisomers having excellent activity are preferred. The compounds of the present disclosure have optical isomers derived from asymmetric carbons, and the like. If necessary, individual isomers may be resolved by methods known in the art, for example, crystallization or chiral chromatography, and the like.

As used herein, the term "alkyl" refers to a straight or branched chain-saturated aliphatic hydrocarbon group. The term "C₁₋₂₀ alkyl" refers to a straight or branched alkyl group having 1 to 20 carbon atom(s). Preferred is C₁₋₁₀ alkyl. More preferred is C₁₋₆ alkyl (i.e. a straight or branched alkyl group having 1, 2, 3, 4, 5, or 6 carbon atom(s)). More preferred is C₁₋₄ alkyl. More preferred is C₁₋₃ alkyl. Specific examples include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and various branched isomers thereof, and the like.

As used herein, the term "alkoxy" refers to a group having an -O-alkyl structure, wherein alkyl is defined as described above. The term "C₁₋₁₀ alkoxy" refers to an alkoxy group having 1 to 10 carbon atom(s). Preferred is C₁₋₆ alkoxy. More preferred is C₁₋₄ alkoxy. More preferred is C₁₋₃ alkoxy. Specific examples include, but are not limited to, methoxy, ethoxy, n-propoxy, isopropoxy, n-butoxy, tert-butoxy, isobutoxy, n-pentoxy, and the like. As used herein, the term "alkenyl" refers to an alkyl group having one or more carbon-carbon double bond(s) at any position in the chain as defined above. The term "C₂₋₈ alkenyl" refers to an alkenyl group having 2 to 8 carbon atoms and at least one (e.g. 1 to 2) carbon-carbon double bond(s). Preferred is C₂₋₆ alkenyl (i.e. an alkenyl group having 2 to 6 carbon atoms and 1 to 2 carbon-carbon double bond(s)). More preferred is C₂₋₄ alkenyl (i.e. an alkenyl group having 2 to 4 carbon atoms and 1 to 2 carbon-carbon double bond(s)). Specific examples include, but are not limited to, ethenyl, 1-propenyl, 2-propenyl, 1-, 2-, or 3-butenyl, pentenyl, hexenyl, butadienyl, and the like.

As used herein, the term "alkynyl" refers to an alkyl group having one or more carbon-carbon triple bond(s) at any position in the chain as defined above. The term "C₂₋₈ alkynyl" refers to an alkynyl group having 2 to 8 carbon atoms and at least one (e.g. 1 to 2) carbon-carbon triple bond(s). Preferred is C₂₋₆ alkynyl (i.e. an alkynyl group having 2 to 6 carbon atoms and 1 to 2 carbon-carbon triple bond(s)). More preferred is C₂₋₄ alkynyl (i.e. an alkynyl group having 2 to 4 carbon atoms and 1 to 2 carbon-carbon triple bond(s)). Specific examples include, but are not limited to, ethynyl, 1-propynyl, 2-propynyl, 1-, 2-, or 3-butynyl, and the like.

As used herein, the term "halogen" refers to fluorine, chlorine, bromine, or iodine.

As used herein, the term "halogenated" refers to fluoro, chloro, bromo, or iodo.

As used herein, the term "halogenated alkyl" refers to an alkyl group in which one or more (e.g. 1, 2, 3, 4, or 5) hydrogen atom(s) are substituted with halogens, wherein the alkyl is defined as described above. The term "halogenated C₁₋₁₀ alkyl" refers to a halogenated alkyl group having 1 to 10 carbon atom(s). Preferred is halogenated C₁₋₆ alkyl. More preferred is halogenated C₁₋₄ alkyl. More preferred is halogenated C₁₋₃ alkyl. Specific examples include, but are not limited to, monochloromethyl, dichloromethyl, trichloromethyl, monochloroethyl, 1,2-dichloroethyl, trichloroethyl, monobromoethyl, monofluoromethyl, difluoromethyl, trifluoromethyl, monofluoroethyl, difluoroethyl, trifluoroethyl, and the like.

As used herein, the term "halogenated alkoxy" refers to an alkoxy group in which one or more (e.g. 1, 2, 3, 4, or 5) hydrogen atom(s) are substituted with halogens, wherein the alkoxy is defined as described above. The term "halogenated C₁₋₁₀ alkoxy" refers to a halogenated alkoxy group having 1 to 10 carbon atom(s). Preferred is halogenated C₁₋₆ alkoxy. More preferred examples are halogenated C₁₋₄ alkoxy. More preferred examples are halogenated C₁₋₃ alkoxy. Specific examples include, but are not limited to, trifluoromethoxy, trifluoroethoxy, monofluoromethoxy, monofluoroethoxy, difluoromethoxy, difluoroethoxy, and the like.

As used herein, the term "deuterated" refers to a group in which one or more hydrogen atom(s) are substituted with deuterium atoms.

As used herein, the term "deuterated alkyl" refers to an alkyl group in which one or more (e.g. 1, 2, 3, 4, or 5) hydrogen atom(s) are substituted with deuterium atoms, wherein the alkyl is defined as described above. The term "deuterated C₁₋₁₀ alkyl" refers to a deuterated alkyl group having 1 to 10 carbon atom(s). Preferred is deuterated C₁₋₆ alkyl. More preferred is deuterated C₁₋₄ alkyl. More preferred is deuterated C₁₋₃ alkyl. Specific examples include, but are not limited to, mono-deuterated methyl, di-deuterated methyl, tri-deuterated methyl, mono-deuterated ethyl, 1,2-di-deuterated ethyl, tri-deuterated ethyl, and the like.

As used herein, the term "deuterated alkoxy" refers to an alkoxy group in which one or more (e.g. 1, 2, 3, 4, or 5) hydrogen atom(s) are substituted with deuterium atoms, wherein the alkoxy is defined as described above. The term "deuterated C₁₋₁₀ alkoxy" refers to a deuterated alkoxy group having 1 to 10 carbon atom(s). Preferred is deuterated C₁₋₆ alkoxy. More preferred is deuterated C₁₋₄ alkoxy. More preferred is deuterated C₁₋₃ alkoxy. Specific examples include, but are not limited to, tri-deuterated methoxy, tri-deuterated ethoxy, mono-deuterated methoxy, mono-deuterated ethoxy, di-deuterated methoxy, di-deuterated ethoxy, and the like.

As used herein, the terms "cycloalkyl" and "cycloalkyl ring" are used interchangeably, and refer to a saturated monocyclic or polycyclic cyclic hydrocarbon group, including, for example, monocyclic cycloalkyl, spiro-cycloalkyl, fused cycloalkyl, and bridged cycloalkyl. The ring carbon atoms of the cycloalkyl group described in the present disclosure may be optionally substituted with 1, 2, or 3 oxo group(s) to form a cyclic ketone structure. The term "3- to 20-membered cycloalkyl" or "C₃₋₂₀ cycloalkyl" refers to a cycloalkyl group having 3 to 20 ring carbon atoms, including monocyclic cycloalkyl, spiro-cycloalkyl, fused cycloalkyl, and bridged cycloalkyl. Preferred is C₃₋₁₂ cycloalkyl, C₅₋₂₀ spiro-cycloalkyl, C₅₋₂₀ fused cycloalkyl, or C₅₋₂₀ bridged cycloalkyl. More preferred is C₃₋₈ monocyclic cycloalkyl.

The terms "C₃₋₈ monocyclic cycloalkyl" and "3- to 8-membered monocyclic cycloalkyl" refer to a saturated monocyclic cyclic hydrocarbon group having 3- to 8-ring carbon atoms. Preferred is C₃₋₆ monocyclic cycloalkyl (i.e. 3- to 6-membered monocyclic cycloalkyl) or C₄₋₆ monocyclic cycloalkyl (i.e. 4- to 6-membered monocyclic cycloalkyl). More preferred is C₃, C₄, C₅, or C₆ monocyclic cycloalkyl. Specific examples of the monocyclic cycloalkyl include, but are not limited to, cyclopropyl, cyclobutyl, cyclopentyl, cyclohexyl, cycloheptyl, cyclooctyl, and the like.

As used herein, the terms "spiro-cycloalkyl" and "spiro-cycloalkyl ring" refer to a polycyclic cyclic hydrocarbon group formed by sharing one carbon atom (referred to as a spiro-atom) between two or more monocyclic rings. The spiro-cycloalkyl groups are classified into mono-spiro-cycloalkyl, di-spiro-cycloalkyl, and poly-spiro-cycloalkyl groups, depending on the number of spiro-atoms shared between rings. The term "5- to 20-membered spiro-cycloalkyl" or "C₅₋₂₀ spiro-cycloalkyl" refers to a polycyclic cyclic hydrocarbon group having 5 to 20 ring carbon atoms, wherein the monocyclic ring that shares the spiro-atom is a 3- to 8-membered monocyclic cycloalkyl ring. Preferred is 6- to 14-membered (i.e. C₆₋₁₄) spiro-cycloalkyl. More preferred is 6- to 14-membered mono-spiro-cycloalkyl. More preferred is 7- to 11-membered (i.e. C₇₋₁₁) spiro-cycloalkyl. More preferred is 7- to 11-membered mono-spiro-cycloalkyl. Most preferred are 7-membered (4-membered monocyclic cycloalkyl ring/4-membered monocyclic cycloalkyl ring), 8-membered (4-membered monocyclic cycloalkyl ring/5-membered monocyclic cycloalkyl ring), 9-membered (4-membered monocyclic cycloalkyl ring/6-membered monocyclic cycloalkyl ring, 5-membered monocyclic cycloalkyl ring/5-membered monocyclic cycloalkyl ring), 10-membered (5-membered monocyclic cycloalkyl ring/6-membered monocyclic cycloalkyl ring), or 11-membered (6-membered monocyclic cycloalkyl ring/6-membered monocyclic cycloalkyl ring) mono-spiro-cycloalkyl. Specific examples of the spiro-cycloalkyl include, but are not limited to:

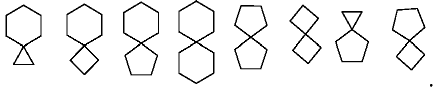

These spiro-cycloalkyl groups may be attached to other moieties of a molecule through any one of the ring atoms.

As used herein, the terms "fused cycloalkyl" and "fused cycloalkyl ring" refer to a polycyclic cyclic hydrocarbon group formed by two or more monocyclic rings sharing an adjacent pair of carbon atoms. The fused cycloalkyl groups may be classified into bicyclic, tricyclic, tetracyclic, or polycyclic fused cycloalkyl groups, depending on the number of rings formed. The term "5- to 20-membered fused cycloalkyl" or "C₅₋₂₀ fused cycloalkyl" refers to a polycyclic cyclic hydrocarbon group having 5 to 20 ring carbon atoms, wherein the monocyclic ring that shares the adjacent pair of carbon atoms is a 3- to 8-membered monocyclic cycloalkyl ring. Preferred is 6- to 14-membered (i.e. C₆₋₁₄) fused cycloalkyl. More preferred is 6- to 14-membered di-fused cycloalkyl. More preferred is 7- to 10-membered (i.e. C₇₋₁₀) fused cycloalkyl. More preferred is 7- to 10-membered di-fused cycloalkyl. Most preferred is 8-membered (a 5-membered monocyclic cycloalkyl ring fused to a 5-membered monocyclic cycloalkyl ring), 9-membered (a 5-membered monocyclic cycloalkyl ring fused to a 6-membered monocyclic cycloalkyl ring), or 10-membered (a 6-membered monocyclic cycloalkyl ring fused to a 6-membered monocyclic cycloalkyl ring) di-fused cycloalkyl. Specific examples of the fused cycloalkyl include, but are not limited to:

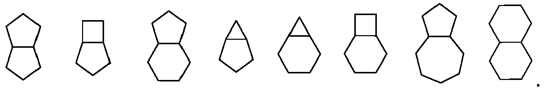

These fused cycloalkyl groups may be attached to other moieties of a molecule through any one of the ring atoms.

As used herein, the terms "bridged cycloalkyl" and "bridged cycloalkyl ring" refer to a polycyclic cyclic hydrocarbon group formed by sharing two carbon atoms that are not directly attached between two or more monocyclic rings. The bridged cycloalkyl groups may be classified into bicyclic, tricyclic, tetracyclic, or polycyclic bridged cycloalkyl groups, depending on the number of rings formed. The terms "5- to 20-membered bridged cycloalkyl" and "C₅₋₂₀ bridged cycloalkyl" refer to a polycyclic cyclic hydrocarbon group having 5 to 20 ring carbon atoms, wherein any two rings share two carbon atoms that are not directly attached. Preferred is 6- to 14-membered (i.e. C₆₋₁₄) bridged cycloalkyl. More preferred is 7- to 10-membered (i.e. C₇₋₁₀) bridged cycloalkyl. Specific examples of the bridged cycloalkyl include, but are not limited to:

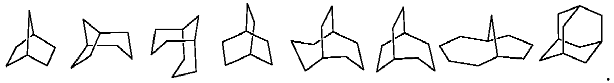

These bridged cycloalkyl groups may be attached to other moieties of a molecule through any one of the ring atoms.

As used herein, the term "halogenated cycloalkyl" refers to a cycloalkyl group in which one or more (e.g. 1, 2, 3, 4, or 5) hydrogen atom(s) are substituted with halogens, wherein the cycloalkyl is defined as described above.

As used herein, the term "halogenated C₃₋₈ monocyclic cycloalkyl" refers to a halogenated monocyclic cycloalkyl group having 3 to 8 ring carbon atoms. Preferred is halogenated C₃₋₆ monocyclic cycloalkyl. More preferred are halogenated C₃, halogenated C₄, halogenated C₅, or halogenated C₆ monocyclic cycloalkyl. Specific examples include, but are not limited to, trifluorocyclopropyl, monofluorocyclopropyl, monofluorocyclohexyl, difluorocyclopropyl, difluorocyclohexyl, and the like.

As used herein, the terms "heterocyclyl" and "heterocyclyl ring" are used interchangeably, and refer to a saturated or partially unsaturated monocyclic or polycyclic cyclic hydrocarbon group, including, for example, monocyclic heterocyclyl, spiro-heterocyclyl, fused heterocyclyl, and bridged heterocyclyl. The ring carbon atoms of the heterocyclyl group described in the present disclosure may be optionally substituted with 1, 2, or 3 oxo group(s) to form a cyclic ketone, cyclic lactone, or cyclic lactam structure. The term "3- to 20-membered heterocyclyl" refers to a saturated or partially unsaturated monocyclic or polycyclic cyclic hydrocarbon group having 3 to 20 ring atoms, wherein one or more (preferably 1, 2, 3, or 4) ring atom(s) are heteroatoms selected from nitrogen, oxygen, or S(=O)_{m'} (where m' is an integer from 0 to 2), but excluding the ring moieties -O-O-, -O-S- or -S-S-, the other ring atoms being carbon. When the ring atom is a nitrogen atom, the nitrogen atom may be substituted or unsubstituted (i.e. N or NR, wherein R is hydrogen or other substituent as already defined herein). The 3- to 20-membered heterocyclyl group described in the present disclosure includes monocyclic heterocyclyl (e.g. 3- to 8-membered monocyclic heterocyclyl), 5- to 20-membered spiro-heterocyclyl, 5- to 20-membered fused heterocyclyl, and 5- to 20-membered bridged heterocyclyl.

As used herein, the terms "3- to 8-membered monocyclic heterocyclyl" and "3- to 8-membered monocyclic heterocyclyl ring" refer to a saturated or partially unsaturated monocyclic cyclic hydrocarbon group having 3 to 8 ring atoms of which 1, 2 or 3 are heteroatoms selected from nitrogen, oxygen, or S(=O)_{m'} (wherein m' is an integer from 0 to 2). Preferred is a 3- to 6-membered monocyclic heterocyclyl group having 3 to 6 ring atoms of which 1 or 2 are heteroatoms. More preferred is a 4- to 6-membered monocyclic heterocyclyl group having 4 to 6 ring atoms of which 1 or 2 are heteroatoms. More preferred is a 5- or 6-membered monocyclic heterocyclyl group having 5 or 6 ring atoms of which 1 or 2 are heteroatoms. When the heteroatom is a nitrogen atom, the nitrogen atom may be substituted or unsubstituted (i.e. N or NR, wherein R is hydrogen or other substituent as already defined herein). When the heteroatom is a sulfur atom, the sulfur atom may be optionally oxidized (i.e. S(=O)_{m'}, wherein m' is an integer from 0 to 2). The ring carbon atoms of the monocyclic heterocyclyl group may be optionally substituted with 1, 2, or 3 oxo group(s) to form a cyclic ketone, cyclic lactone, or cyclic lactam structure. Specific examples of the monocyclic heterocyclyl include, but are not limited to, aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholin-1,1-dioxide, tetrahydropyran, 1,2-dihydroazete, 1,2-dihydrooxacyclobutadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrole-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridine-2,6-(1H, 3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine, 1,2,3,4-tetrahydropyrimidine, and the like.

As used herein, the term "3- to 6-membered nitrogen-containing heterocyclyl" refers to a saturated or partially unsaturated monocyclic cyclic hydrocarbon group having 3 to 6 ring atoms of which 1 is a nitrogen atom, and the other 1 or 2 ring atom(s) are heteroatoms selected from nitrogen, oxygen, or S(=O)_{m'} (wherein m' is an integer from 0 to 2). Specific examples include, but are not limited to, azacyclopropyl, azacyclobutyl, azacyclopentyl (i.e. tetrahydropyrrole), azacyclohexyl (i.e. hexahydropyridine), morpholinyl, piperazinyl, oxazolidine.

As used herein, the term "3- to 8-membered monocyclic heterocycloalkyl" refers to a saturated monocyclic cyclic hydrocarbon group having 3 to 8 ring atoms of which 1 or 2 are heteroatoms. Preferred is 3- to 6-membered monocyclic heterocycloalkyl, i.e. a saturated monocyclic cyclic hydrocarbon group having 3 to 6 ring atoms of which 1 or 2 are heteroatoms. Specific examples of the heterocycloalkyl include, but are not limited to, azacyclopropyl, oxiranyl, azetidinyl, oxetanyl, oxazolidinyl, 1,3-dioxolanyl, dioxanyl, imidazolidinyl, tetrahydrofuranyl, tetrahydrothiophenyl, tetrahydropyrrolyl, piperidinyl, piperazinyl, morpholinyl, thiomorpholinyl, thiomorpholine-1,1-dioxide, tetrahydropyranyl, 1,4-oxazepanyl, 1,3-oxazepanyl, 1,3-oxazinanyl, hexahydropyrimidinyl, 1,4-dioxanyl. The two ring atoms, including C-C and N-C, attached in the above monocyclic heterocyclyl ring may optionally be fused to the cycloalkyl, heterocyclyl, aryl, or heteroaryl as defined in the present disclosure, such as a monocyclic cycloalkyl ring, monocyclic heterocyclyl ring, monocyclic aryl ring, and 5- or 6-membered monocyclic heteroaryl ring, to form a fused polycyclic ring. The 2 ring atoms attached in the monocyclic heterocyclyl forming a fused ring with other rings are preferably C-C.

As used herein, the terms "spiro-heterocyclyl" and "spiro-heterocyclyl ring" refer to a polycyclic heterocyclyl group formed by sharing one carbon atom (referred to as a spiro-atom) between two or more saturated or partially unsaturated monocyclic rings, wherein one or more (e.g. 1, 2, or 3) ring atom(s) are heteroatoms selected from nitrogen, oxygen, or S(=O)_{m'} (wherein m' is an integer from 0 to 2), the other ring atoms being carbon. When the heteroatom is a nitrogen atom, the nitrogen atom may be substituted or unsubstituted (i.e. N or NR, wherein R is hydrogen or other substituent as already defined herein). Each monocyclic ring may contain one or more double bond(s), but none of the rings have a completely conjugated π-electron system. The spiro-heterocyclyl groups are classified into mono-spiro-heterocyclyl, bi-spiro-heterocyclyl, or poly-spiro-heterocyclyl groups, depending on the number of spiro-atoms shared between rings. The term "5- to 20-membered spiro-heterocyclyl" refers to a spiro-heterocyclyl group having 5 to 20 ring atoms, wherein one of the monocyclic rings that share spiro-atoms is a 3- to 8-membered monocyclic heterocyclyl ring and the other monocyclic ring is a 3- to 8-membered monocyclic heterocyclyl ring or a 3- to 8-membered monocyclic cycloalkyl ring. Preferred is a 6- to 14-membered spiro-heterocyclyl group having 6 to 14 ring atoms of which 1 or 2 are heteroatoms. More preferred is a 7- to 11-membered spiro-heterocyclyl group having 7 to 11 ring atoms of which 1 or 2 are heteroatoms. Most preferred is 7-membered (4-membered monocyclic heterocyclyl ring/4-membered monocyclic heterocyclyl ring or 4-membered monocyclic heterocyclyl ring/4-membered monocyclic cycloalkyl or 4-membered monocyclic cycloalkyl ring/4-membered monocyclic heterocyclyl ring), 8-membered (4-membered monocyclic heterocyclyl ring/5-membered monocyclic heterocyclyl ring), 9-membered (4-membered monocyclic heterocyclyl ring/6-membered monocyclic heterocyclyl ring, 5-membered monocyclic heterocyclyl ring/5-membered monocyclic heterocyclyl ring), 10-membered (5-membered monocyclic heterocyclyl ring/6-membered monocyclic heterocyclyl ring), or 11-membered (6-membered monocyclic heterocyclyl ring/6-membered monocyclic heterocyclyl ring) mono-spiro-heterocyclyl. Specific examples of the spiro-heterocyclyl include, but are not limited to:

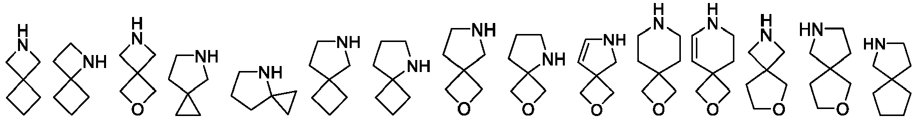

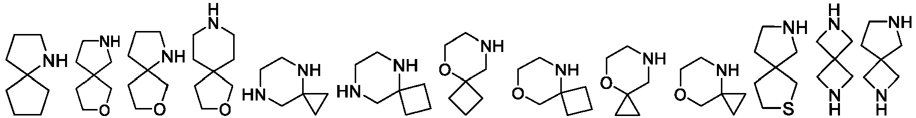

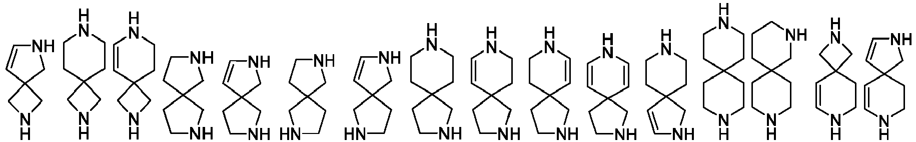

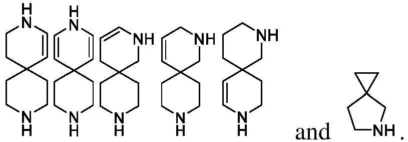

These spiro-heterocyclyl groups may be attached to other moieties of a molecule through any one of the suitable ring atoms.

As used herein, the terms "fused heterocyclyl" and "fused heterocyclyl ring" refer to a polycyclic heterocyclyl group formed by two or more saturated or partially unsaturated monocyclic rings sharing an adjacent pair of carbon atoms, wherein one or more (e.g. 1, 2, or 3) ring atom(s) are heteroatoms selected from nitrogen, oxygen, or S(=O)_{m'} (wherein m' is an integer from 0 to 2), the other ring atoms being carbon. When the heteroatom is a nitrogen atom, the nitrogen atom may be substituted or unsubstituted (i.e. N or NR, wherein R is hydrogen or other substituent as already defined herein). Each monocyclic ring may contain one or more double bond(s), but none of the rings have a completely conjugated π-electron system. The shared adjacent ring atom pairs may be C-C or N-C. The fused heterocyclyl groups may be classified into bicyclic, tricyclic, tetracyclic, or polycyclic fused heterocyclyl groups, depending on the number of rings formed. The term "5- to 20-membered fused heterocyclyl" refers to a fused heterocyclyl group having 5 to 20 ring atoms, wherein the monocyclic ring that shares adjacent ring atom pairs is a 3- to 8-membered monocyclic heterocyclyl ring. Preferred is a 6- to 14-membered fused heterocyclyl group having 6 to 14 ring atoms of which 1 or 2 are heteroatoms. More preferred is a 6- to 10-membered fused heterocyclyl group having 6 to 10 ring atoms of which 1 or 2 are heteroatoms. More preferred is an 8- to 10-membered fused heterocyclyl group having 8 to 10 ring atoms of which 1 or 2 are heteroatoms. Most preferred is an 8-membered (a 5-membered monocyclic heterocyclyl ring fused to a 5-membered monocyclic heterocyclyl ring), 9-membered (a 5-membered monocyclic heterocyclyl ring fused to a 6-membered monocyclic heterocyclyl ring), or 10-membered (a 6-membered monocyclic heterocyclyl ring fused to a 6-membered monocyclic heterocyclyl ring) bicyclic fused heterocyclyl group. Specific examples of the fused heterocyclyl include, but are not limited to:

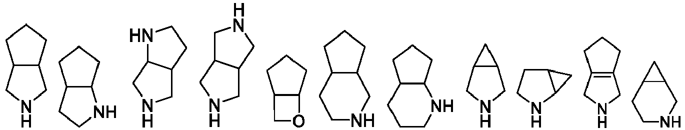

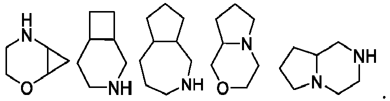

These fused heterocyclyl groups may be attached to other moieties of a molecule through any one of the suitable ring atoms.

As used herein, the terms "bridged heterocyclyl" and "bridged heterocyclyl ring" refer to a polycyclic heterocyclyl group formed by two or more saturated or partially unsaturated monocyclic rings sharing two ring atoms that are not directly attached, wherein one or more (e.g. 1, 2, or 3) ring atom(s) are heteroatoms selected from nitrogen, oxygen, or S(=O)_{m'} (wherein m' is an integer from 0 to 2), the other ring atoms being carbon. The bridged heterocyclyl groups may be classified into bicyclic, tricyclic, tetracyclic, or polycyclic bridged cycloalkyl groups, depending on the number of rings formed. The term "5- to 20-membered bridged heterocyclyl" refers to a saturated or partially unsaturated polycyclic heterocyclyl group having 5 to 20 ring atoms, wherein any two rings share two ring atoms that are not directly attached. Each monocyclic ring may contain one or more double bond(s), but none of the rings have a completely conjugated π-electron system. Preferred is 6- to 14-membered bridged heterocyclyl. More preferred is 7- to 10-membered bridged heterocyclyl. Specific examples of the bridged heterocyclyl include, but are not limited to:

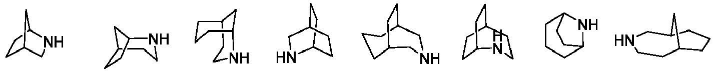

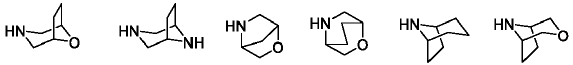

These bridged heterocyclyl groups may be attached to other moieties of a molecule through any one of the suitable ring atoms.

In the present disclosure, each of the above heterocyclyl groups may be optionally substituted. When substituted, the substituent is preferably one or more of the substituent groups recorded in the present application.

As used herein, the terms "aryl", "aryl ring", and "aromatic ring" are used interchangeably, and refer to an all-carbon monocyclic group, an all-carbon non-fused polycyclic (rings are attached by covalent bonds, non-fused) group, or an all-carbon fused polycyclic (i.e. rings that shares adjacent carbon atom pairs) group in which at least one ring is aromatic, i.e. the ring has a conjugated π-electron system. The term "C₆₋₁₄ aryl" refers to an aryl group having 6 to 14 ring atoms. Preferred is C₆₋₁₀ aryl. The C₆₋₁₄ aryl group in the present disclosure includes monocyclic aryl, non-fused polycyclic aryl, and an aromatic fused polycyclic ring, wherein examples of the monocyclic aryl include phenyl, and examples of the non-fused polycyclic aryl include biphenyl and the like.

In the present disclosure, when the C₆₋₁₄ aryl is an aromatic fused polycyclic ring, the aromatic fused polycyclic ring may be a polycyclic group formed by fusing a monoaryl ring with one or more monoaryl rings, non-limiting examples of which include naphthyl, anthryl, and the like.

In some embodiments of the present disclosure, when the C₆₋₁₄ aryl is an aromatic fused polycyclic ring, the aromatic fused polycyclic ring may also be a polycyclic group formed by fusing a monoaryl ring (e.g. phenyl) with one or more non-aromatic ring(s), wherein the ring attached to the parent structure is an aromatic ring or a non-aromatic ring. The non-aromatic rings include, but is not limited to, 3- to 6-membered monocyclic heterocyclyl ring (preferably 5- or 6-membered monocyclic heterocyclyl ring, the ring carbon atoms of which may be substituted with 1 to 2 oxo group(s) to form a cyclic lactam or cyclic lactone structure), 3- to 6-membered monocyclic cycloalkyl ring (preferably 5- or 6-membered monocyclic cycloalkyl ring, the ring carbon atoms of which may be substituted with 1 or 2 oxo group(s) to form a cyclic ketone structure). The polycyclic groups formed by fusing a monoaryl ring with one or more non-aromatic rings as described above may be attached to other groups or the parent structure via a nitrogen or carbon atom, and the ring attached to the parent structure is a monoaryl ring or a non-aromatic ring.

In the present disclosure, each of the above aryl groups may be substituted or unsubstituted. When substituted, the substituent is preferably one or more of the substituent groups recorded in the present application.

As used herein, the terms "heteroaryl", "heteroaryl ring", and "heteroaromatic ring" are used interchangeably, and refer to a monocyclic or fused polycyclic (i.e. sharing adjacent ring atom pairs, which may be C-C or N-C) group in which ring atoms are substituted with at least one heteroatom independently selected from nitrogen, oxygen, or sulfur, wherein the nitrogen and sulfur atoms may be optionally oxidized, and the nitrogen atom may be optionally quaternized. The heteroaryl group has shared 6, 10, or 14 π-electrons, with at least one ring in the group being aromatic. The term "5- to 14-membered heteroaryl" refers to a heteroaryl group having 5 to 14 ring atoms of which 1, 2, 3, or 4 are heteroatoms selected from nitrogen, oxygen, or S(=O)_{m'} (wherein m' is an integer from 0 to 2). Preferred is a 5- to 10-membered heteroaryl group having 5 to 10 ring atoms of which 1, 2, 3, or 4 are heteroatoms. In the present disclosure, the 5- to 14-membered heteroaryl may be monocyclic heteroaryl, fused bicyclic heteroaryl, or fused tricyclic heteroaryl.

As used herein, the term "5- or 6-membered monocyclic heteroaryl" refers to a monocyclic heteroaryl having 5 or 6 ring atoms of which 1, 2, or 3 are heteroatoms selected from nitrogen, oxygen, or S(=O)_{m'} (wherein m' is an integer from 0 to 2). Specific examples of the monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, and the like.

As used herein, the term "8- to 10-membered bicyclic heteroaryl" refers to a fused bicyclic heteroaryl group having 8 to 10 ring atoms of which 1, 2, 3, 4, or 5 are heteroatoms selected from nitrogen, oxygen, or S(=O)_{m'} (wherein m' is an integer from 0 to 2). The fused bicyclic heteroaryl may be either a bicyclic group (preferably a 9- or 10-membered bicyclic heteroaryl ring) formed by fusing a monoaryl ring (e.g. phenyl) with a monocyclic heteroaryl ring (preferably a 5- or 6-membered monocyclic heteroaryl ring), or a bicyclic group formed by fusing a monocyclic heteroaryl ring (preferably a 5- or 6-membered monocyclic heteroaryl ring) with a monocyclic heteroaryl ring (preferably 5- or 6-membered monocyclic heteroaryl ring).

Any two ring atoms, including C-C, N-C, and N-N, attached in the above monocyclic heteroaryl ring may be fused to the cycloalkyl, heterocyclyl, aryl, or heteroaryl as defined in the present disclosure, such as a monocyclic cycloalkyl ring, monocyclic heterocyclyl ring, monoaryl ring, and 5- or 6-membered monocyclic heteroaryl ring, to form a fused polycyclic ring. The two ring atoms attached in the monocyclic heteroaryl ring forming a fused ring with other rings are preferably C-C, including, but not limited to, the following forms:

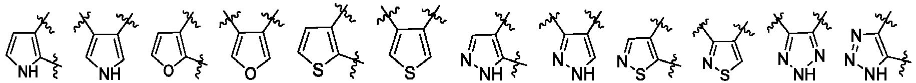

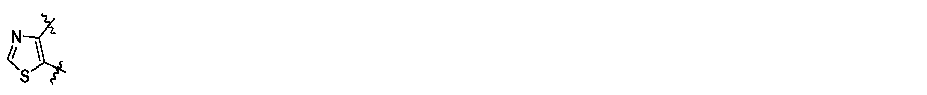

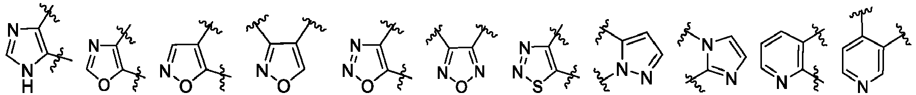

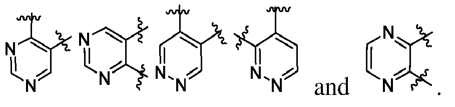

Among the above groups, the ring atom labeled with " 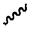 " is attached to other moieties of a molecule.

Non-limiting examples of the 8- to 10-membered bicyclic heteroaryl include benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine, and the like.

Specific examples of the bicyclic heteroaryl include, but are not limited to:

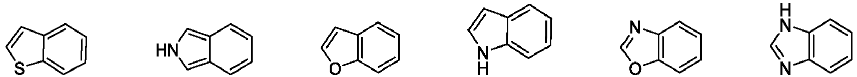

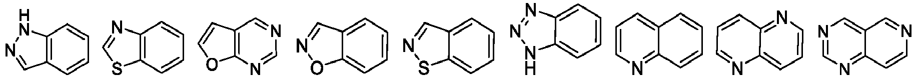

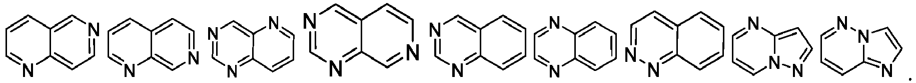

These groups may be attached to other moieties of a molecule through any one of the suitable ring atoms. The ring attached to the parent structure may be a monocyclic heteroaryl ring or a benzene ring.

In some embodiments of the present disclosure, the fused bicyclic heteroaryl or fused tricyclic heteroaryl may be a polycyclic group formed by fusing a monocyclic heteroaryl ring (preferably a 5- or 6-membered monocyclic heteroaryl ring) with one or more non-aromatic ring(s), wherein the ring attached to the parent structure is a monocyclic heteroaryl ring or a non-aromatic ring. The non-aromatic ring includes, but is not limited to, a 3- to 6-membered monocyclic heterocyclyl ring (preferably a 5- or 6-membered monocyclic heterocyclyl ring, the ring carbon atoms of which may be substituted with 1 to 2 oxo group(s) to form a cyclic lactam or cyclic lactone structure), a 3- to 6-membered monocyclic cycloalkyl ring (preferably a 5- or 6-membered monocyclic cycloalkyl ring, the ring carbon atoms of which may be substituted with 1 or 2 oxo group(s) to form a cyclic ketone structure), and the like. The polycyclic groups formed by fusing a monocyclic heteroaryl ring with one or more non-aromatic ring(s) as described above may be attached to other groups or the parent structure via a nitrogen or carbon atom, and the ring attached to the parent structure is a monocyclic heteroaryl ring or a non-aromatic ring. In the present disclosure, each of the above heteroaryl groups may be substituted or unsubstituted. When substituted, the substituent is preferably one or more of the substituent groups recorded in the present application.

As used herein, the term "hydroxy" refers to -OH.

As used herein, the term "hydroxymethyl" refers to -CH₂OH, and "hydroxyethyl" refers to -CH₂CH₂OH or -CH(OH)CH₃.

As used herein, the term "cyanomethyl" refers to -CH₂CN, and "cyanoethyl" refers to -CH₂CH₂CN or -CHCNCH₃.

As used herein, the term "amino" refers to -NH₂.

As used herein, the term "cyano" refers to -CN.

As used herein, the term "nitro" refers to -NO₂.

As used herein, the term "benzyl" refers to -CH₂-benzene.

As used herein, the term "oxo" refers to =O.

As used herein, the term "carboxy" refers to -C(O)OH.

As used herein, the term "carboxylate" refers to -C(O)O(alkyl) or -C(O)O(cycloalkyl).

As used herein, the term "acetyl" refers to -COCH₃.

As used herein, the term "substituted" means that any one or more hydrogen atom(s) attached to the specified atom are substituted with a substituent, and may include deuterium and hydrogen variants, provided that the valency of the specified atom is normal and that the substituted compound is stable. When the substituent is oxo (i.e. =O), it means that two hydrogen atoms are substituted. Oxo substitution does not occur in aromatic groups. The term "optional substitution" or "optionally substituted" means that a group may or may not be substituted. Unless otherwise specified, the type and number of substituents may be arbitrary on a chemically feasible basis.

When any variable (e.g., R) occurs more than once in the composition or structure of a compound, the definition of the variable in each instance is independent. Thus, for example, if a group is substituted with 0-2 R group(s), the group may be optionally substituted with up to two R groups, and there are independent options for R in each instance. Furthermore, combinations of the substituents and/or variants thereof are permissible only if such combinations result in stable compounds.

In any embodiment, any or all of the hydrogens present in a compound, or hydrogens in a particular group or moiety of a compound, may be substituted with deuterium or tritium. One to the maximum number of hydrogens present in the compound may be substituted with deuterium. One to the maximum number of hydrogens present in any group of a general formula compound or a specific compound may be deuterated. For example, when a group is described as an ethyl group, the ethyl may be C₂H₅ or C₂H₅ with x (1 to 5) hydrogen(s) substituted with deuterium, e.g. C₂DₓH₅₋ₓ. When a group is described as a deuterated ethyl group, the deuterated ethyl may be C₂H₅ with x (1 to 5) hydrogen(s) substituted with deuterium, e.g. C₂DₓH₅₋ₓ.

### Detailed Description of the Invention

The compounds of the present disclosure may be prepared by a variety of synthetic methods well known to those skilled in the art, including the specific embodiments set forth below, embodiments formed in combination with other chemical synthetic methods, and equivalents well known to those skilled in the art. Preferred embodiments include, but are not limited to, the examples of the present disclosure.

The following examples describe the present disclosure in detail, but they are not meant to impose any unfavorable limitation on the present disclosure. The present disclosure has been described in detail herein, and its specific embodiments are also disclosed. It will be apparent to those skilled in the art that various changes and modifications can be made to the specific embodiments without departing from the spirit and scope of the present disclosure. Examples, where specific conditions are not specified, are implemented in accordance with general conditions or those recommended by a manufacturer. All of the used agents or instruments, which manufacturers are not specified, are conventional commercially-available products.

Prep-HPLC used in the following examples, unless otherwise specified, may utilize the following conditions:
Prep-HPLC (ammonium bicarbonate method): column type: Waters XBridge C18, 190*250 mm, 5 um; mobile phase system: A: 0.1% aqueous ammonium bicarbonate; B: preparative grade acetonitrile; flow rate: 15 ml/min; B%=20%-100%; column temperature: room temperature.
Prep-HPLC (ammonia water method): column type: Waters XBridge C18, 190*250 mm, 5 um; mobile phase system: A: 0.1% ammonia water solution; B: preparative grade acetonitrile; flow rate: 15 ml/min; B%=20%-100%; column temperature: room temperature.
Prep-HPLC (formic acid method): column type: Waters XBridge C18, 190*250 mm, 5 um; mobile phase system: A: 20 0.1% formic acid; B: preparative grade acetonitrile; flow rate: 15 ml/min; B%=20%-100%; column temperature: room temperature.
Prep-HPLC (trifluoroacetic acid method): column type: Waters XBridge C18, 190*250 mm, 5 um; mobile phase system: A: 0.1% trifluoroacetic acid; B: preparative grade acetonitrile; flow rate: 15 ml/min; B%=20%-100%; column temperature: room temperature.
Prep-HPLC (hydrochloric acid method): column type: Phenomenex luna C18, 80*40 mm*3 um; mobile phase system: [water (hydrochloric acid)-acetonitrile]; B%: 1%-30%; flow rate: 15 ml/min; column temperature: room temperature.

### Preparation Example 1: Preparation of intermediate 1a

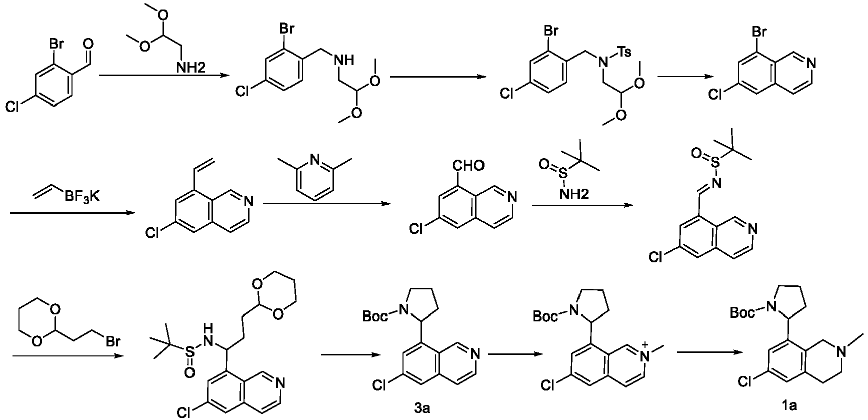

Step 1: 2-Bromo-4-chlorobenzaldehyde (50 g, 227.8 mmol) was dissolved in 200 ml of methanol, and placed in an ice-water bath. Aminoacetaldehyde dimethyl acetal (24 g, 227.8 mmol) and acetic acid (3 ml) were added and stirred for 30 min. NaBH₃CN (28.63 g, 455.6 mmol) was added in batches, and the system reacted at room temperature for 2 h. The reaction mixture was extracted with ethyl acetate, washed with sodium bicarbonate, dried, and concentrated to give a crude product (30 g) which was directly used in the next step. ES-API:[M+H]⁺= 308.0
Step 2: The above crude product N-(2-bromo-4-chlorobenzyl)-2,2-dimethoxyethanamine (30 g) was dissolved in 150 ml of dichloromethane, and placed in an ice-water bath. Triethylamine (29.3 g, 292.5 mmol) and TsCl (27.8 g, 146.5 mmol) were added successively, and the system reacted at room temperature overnight. After standing for 5 h, the mixture was filtered. The filtrate was washed with water and saturated brine successively, dried over anhydrous sodium sulfate, filtered, and purified to give N-(2-bromo-4-chlorobenzyl)-N-(2,2-dimethoxyethyl)-4-methylbenzenesulfonamide (35 g, crude product purity: 76% ). ES-API: [M+H]⁺= 430.0
Step 3: The above N-(2-bromo-4-chlorobenzyl)-N-(2,2-dimethoxyethyl)-4-methylbenzenesulfonamide (35 g, 75.75mmol) was dissolved in dried dichloromethane (300 ml), and placed in an ice-water bath. Aluminum trichloride (80.6 g, 606 mmol) was added, and the system reacted at room temperature overnight. Upon completion of the reaction, the reaction was quenched by being slowly introduced into the ice-water bath. An excess NaOH solution was slowly added and stirred until a clear solution was obtained. The organic layer was separated, washed with water, dried, and purified (30%-40% ethyl acetate/petroleum ether) to give 8-bromo-6-chloroisoquinoline (8 g, total yield of the three steps: 14.5%). ES-API: [M+H]⁺=242.0
Step 4: The 8-bromo-6-chloroisoquinoline (1 g, 4.15 mmol) reacted with potassium vinyltrifluoroborate (1.66 g, 12.45 mmol), [1,1'-bis(diphenylphosphino)ferrocene] dichloropalladium (151 mg, 0.207 mmol), triethylamine (419 mg, 4.15 mmol) and ethanol (50 ml) overnight at 80°C. Ethyl acetate was added. The obtained mixture was washed with water and saturated brine successively, dried over anhydrous sodium sulfate, filtered, and purified to give a product 6-chloro-8-vinylisoquinoline (667 mg, yield: 85%). ES-API: [M+H]⁺= 190.0
Step 5: The 6-chloro-8-vinylisoquinoline (600 mg, 3.17 mmol) was dissolved in 20 ml of tetrahydrofuran. 2,6-dimethypyridine (339 mg, 3.17 mmol) was added at room temperature, followed by K₂O_{S}O₄ 2H₂O (424 mg, 0.951 mmol) and NaIO₄ (5.42 g, 25.36 mmol) in water (10 ml). The system reacted at room temperature for 5 h and was cooled to 0°C. The reaction was quenched by the addition of aqueous sodium thiosulfate solution. The reaction mixture was diluted with ethyl acetate, and filtered. The filtrate was washed with water, saturated aqueous sodium bicarbonate solution and brine successively, dried, concentrated, and purified to give 6-chloroisoquinoline-8-carbaldehyde (400 mg, yield: 66%). ES-API: [M+H]⁺= 192.0.
Step 6: The above 6-chloroisoquinoline-8-carbaldehyde (400 mg, 2.09 mmol) and tert-butylsulfinamide (760 mg, 6.27 mmol) were dissolved in 10 ml of dried dichloromethane. Ti(EtO)₄ (1.9 g, 8.36 mmol) was added, and the system reacted at room temperature for 3 h. Upon completion of the reaction, the reaction mixture was slowly introduced into 20 ml of saturated brine, and 20 ml of dichloromethane was added. The organic layer was separated, filtered, dried, and purified to give (E/Z)-N-(((6-chloroisoquinolin-8-yl)methylene)-2-methylpropane-2-sulfinamide (420 mg, yield: 68%). ES-API: [M+H]⁺= 295.0.
Step 7: Preparation of Grignard reagent: 2-(2-bromoethyl)-1,3-dioxane (849 mg, 4.35 mmol, 8 eq), Mg (104 mg, 4.35 mmol, 8 eq), a catalytic amount of elemental I₂ and 20 ml of dried tetrahydrofuran were added to a 50 ml three-necked flask, and the system reacted at room temperature in the presence of protective nitrogen until a colorless solution with heat and bubbles were generated. The obtained solution was transferred to an oil bath at 75°C, and the system reacted for 2 h until most of the magnesium powder disappeared, and was cooled to room temperature for later use. The (E/Z)-N-(((6-chloroisoquinolin-8-yl)methylene)-2-methylpropane-2-sulfinamide (420 mg, 1.43 mmol) and 20 ml of dried tetrahydrofuran were added to a 100 ml three-necked flask, and placed in a dry ice bath at -78°C in the presence of protective nitrogen. The above Grignard reagent solution (10 ml, 4 eq) was slowly added. After 10 min, the reaction was completed and quenched by the addition of saturated aqueous NH₄Cl solution (20 ml). The organic layer was separated, washed with water, dried, concentrated, and purified to give N-(1-(1-chloroisoquinolin-8-yl)-3-(1,3-dioxan-2-yl)propyl)-2-methylpropane-2-sulfinamid e (469 mg, yield: 80%). ES-API: [M+H]⁺= 411.1.
Step 8: The N-(1-(1-chloroisoquinolin-8-yl)-3-(1,3-dioxan-2-yl)propyl)-2-methylpropane-2-sulfinamid e (469 mg, 1.14 mmol) was added to trifluoroacetic acid/water (6 ml/0.3 ml), and the system reacted at room temperature (<25°C) for 30 min. Et₃SiH (1.32 g, 11.4 mmol) was added, and the system reacted for 2 h. After concentration, the obtained concentrate was dissolved in tetrahydrofuran. TEA (921 mg, 9.12 mmol) and (Boc)₂O (745.5 mg, 3.42 mmol) were added. Upon completion of the reaction, the reaction mixture was extracted with ethyl acetate, washed with saturated brine, and purified to give a product tert-butyl 2-(6-chloroisoquinolin-8-yl)pyrrolidine-1-carboxylate (3a, 189 mg, yield: 50%). ES-API: [M+H]⁺= 333.1.
Step 9: The above tert-butyl 2-(6-chloroisoquinolin-8-yl)pyrrolidine-1-carboxylate (189 mg, 0.569 mmol) and iodomethane (10 eq) were added to 5 ml of acetonitrile, and the system reacted at 90°C in a sealed tube for 6 h. Upon completion of the reaction, the reaction mixture was concentrated to give a product 8-(1-(tert-butoxycarbonyl)pyrrolidin-2-yl)-6-chloro-isoquinolin-2-methyl salt (210 mg, crude product) which was directly used in the next step. ES-API: [M+H]⁺= 348.1.
Step 10: The above 8-(1-(tert-butoxycarbonyl)pyrrolidin-2-yl)-6-chloro-isoquinolin-2-methyl salt (210 mg, crude product) was dissolved in 5 ml of methanol, and placed in an ice-water bath. NaBH₄ (8 eq) was added, and the system reacted in the ice-water bath for 2 h. The reaction was quenched with saturated NH₄Cl. The reaction mixture was extracted with ethyl acetate, washed with brine, dried, and purified to give a product tert-butyl 2-(6-chloro-2-methyl-1,2,3,4-tetrahydroisoquinolin-8-yl)pyrrolidine-1 -carboxylate (intermediate 1a, 160 mg, yield of the two steps: 80%). ES-API: [M+H]⁺= 351.1.

### Preparation Example 2: Preparation of intermediate 2a

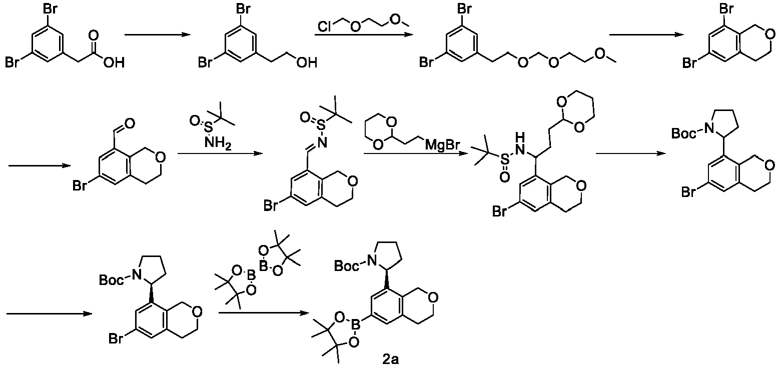

Step 1: 3,5-Dibromophenylacetic acid (7.5 g, 25.5 mmol) and 150 ml of tetrahydrofuran were added to a three-neck reaction flask, and placed in an ice-water bath in the presence of protective nitrogen. Borane in tetrahydrofuran (38.3 mL, 38.3 mmol) was slowly added dropwise and stirred at room temperature for 6 h. Upon completion of the reaction, methanol was slowly added until no more bubbles were generated. The obtained mixture was extracted with ethyl acetate, washed with sodium bicarbonate and saturated brine successively, dried, concentrated, and purified by silica gel column chromatography (ethyl acetate:petroleum ether = 1:20) to give 3,5-dibromophenylethanol (6.3 g, yield: 88.8%). ES-API: [M+H-18]⁺= 260.9.

Step 2: The 3,5-dibromophenylethanol (6.3 g, 22.5 mmol) was dissolved in 120 ml of dichloromethane, and placed in an ice-water bath. Diisopropylethylamine (14.5 g, 112.5 mmol) and 2-methoxyethoxymethyl chloride (8.37 g, 67.5 mmol) were added successively, and the system reacted at room temperature overnight. Upon completion of the reaction, the reaction mixture was washed with water and saturated brine successively, dried over anhydrous sodium sulfate, filtered, and purified by silica gel column chromatography (ethyl acetate:petroleum ether = 1:20) to give 1,3-dibromo-5-(2-((2-methoxyethoxy)methoxy)ethyl)benzene (7.2 g, yield: 87%). ES-API: [M + Na]⁺=391.0.

Step 3: The 1,3-dibromo-5-(2-((2-methoxyethoxy)methoxy)ethyl)benzene (7.2 g, 19.56 mmol) was dissolved in dried dichloromethane (150 ml), and placed in an ice-water bath. Titanium tetrachloride (29.3 mL, 29.3 mmol) was added, and the system reacted in the ice-water bath for 2 h. The reaction was quenched by being slowly poured into saturated brine, and the reaction mixture was extracted with dichloromethane. The organic layer was separated, dried, and purified (ethyl acetate:petroleum ether = 1:15) to give 6,8-dibromoisochroman (5.0 g, yield: 87.7%). ES-API: [M+H]⁺= 294.9.

Step 4: The 6,8-dibromoisochroman (3.5 g, 11.98 mmol) and dried tetrahydrofuran (100 mL) were added to a 250 ml three-necked flask, and the system was cooled in an acetone-dry ice bath at -78°C in the presence of protective nitrogen. n-Butyllithium (5.27 mL, 2.5 M) was slowly added, and the system reacted at -78°C for 0.5 h. Dried N,N-dimethylformamide (5 mL) was slowly added. After 10 min, the reaction was checked by LCMS for completion and quenched by the addition of 1M dilute hydrochloric acid. The reaction mixture was diluted with ethyl acetate, washed with water and saturated brine successively, dried over anhydrous sodium sulfate, filtered, and purified by silica gel column chromatography (ethyl acetate:petroleum ether = 1:10) to give a product 6-bromoisochroman-8-carbaldehyde (1.8 g, yield: 44%). ES-API: [M+H]⁺= 241.0.

Step 5: The 6-bromoisochroman-8-carbaldehyde (1.8 g, 7.5 mmol) and tert-butylsulfinamide (1.81 g, 15 mmol) were dissolved in 50 ml of dichloromethane, and placed in an ice-water bath. Tetraethyl phthalate (3.42 g, 15 mmol) was added, and the system reacted at room temperature for 3 h. Upon completion of the reaction, the reaction mixture was poured into saturated brine, diluted with dichloromethane, and filtered. The organic phase was separated, dried over anhydrous sodium sulfate, filtered, and purified by silica gel column chromatography (ethyl acetate: petroleum ether = 1:2) to give a product N-((6-bromoisochroman-8-yl)methylene)-2-methylpropane-2-sulfinamide (2.36 g, yield: 92%). ES-API: [M+H]⁺=344.1.

Step 6: The N-((6-bromoisochroman-8-yl)methylene)-2-methylpropane-2-sulfinamide (1.86 g, 5.42 mmol) and 20 ml of dried tetrahydrofuran were added to a 100 ml three-necked flask, placed in a -78°C dry ice bath in the presence of protective nitrogen. (2-(1,3-Dioxan-2-yl)ethyl)magnesium bromide (43.4 ml, 0.5M in tetrahydrofuran) was added. After 10 min, the reaction was completed and quenched by the addition of saturated aqueous NH₄Cl solution (20 ml). The organic layer was separated, washed with water, dried, concentrated, and purified by silica gel (ethyl acetate:petroleum ether =1:1) to give N-(1-(6-bromoisochroman-8-yl)-3-(1,3-dioxan-2-yl)propyl)-2-methylpropane-2-sulfinami de (2.1 g, yield: 85%). ES-API: [M+H]⁺=460.2.

Step 7: The N-(1-(6-bromoisochroman-8-yl)-3-(1,3-dioxan-2-yl)propyl)-2-methylpropane-2-sulfinami de (2.5 g, 7.29 mmol) was added to trifluoroacetic acid/water (30 ml/3 ml), and the system reacted at room temperature (<25°C) for 30 min. Et₃SiH(8.45 g, 11.6 mmol) was added, and the system reacted for 5 h. After concentration, trifluoroacetic acid was removed. The residue was dissolved in tetrahydrofuran, and triethylamine (2.21 g, 21.9 mmol) and (Boc)₂O (3.96 g, 18.2 mmol) were added. Upon completion of the reaction, the reaction mixture was extracted with ethyl acetate, washed with saturated brine, and purified by silica gel (ethyl acetate:petroleum ether = 1:4) to give a product tert-butyl 2-(6-bromoisochroman-8-yl)pyrrolidine-1-carboxylate (1.65 g, yield: 59%). ES-API: [M+H]⁺=382.0.

Step 8: The tert-butyl 2-(6-bromoisochroman-8-yl)pyrrolidine-1-carboxylate (750 mg, 1.96 mmol) was chirally resolved by SFC (column type: ChiralpakIC 250 mm*4.6 mm*5 um, mobile phase: HEX-IPA = 85:15, flow rate: 1 ml/min, column temperature: 30°C, 30min) to give tert-butyl (S)-2-(6-bromoisochroman-8-yl)pyrrolidine-1-carboxylate (420 mg, yield: 56%, retention time: 9.619 min, ee value: 100%)). ES-API: [M+H]⁺=382.1.

Step 9: The tert-butyl (S)-2-(6-bromoisochroman-8-yl)pyrrolidine-1-carboxylate (381 mg, 1 mmol), bis(pinacolato)diboron (762 mg, 3 mmol), Pd(dppf)Cl₂ (73 mg, 0.1 mmol) and potassium acetate (294 mg, 3 mmol) were dissolved in 1,4-dioxane (8 ml), and the system reacted under microwave at 100°C for 0.5 h. Upon completion of the reaction, ethyl acetate was added. The obtained mixture was filtered, concentrated, and purified (petroleum ether:ethyl acetate = 50:50) to give tert-butyl (S)-2-(6-(4,4,5,5-tetramethyl-1,3,2-dioxaborolan-2-yl)isochroman-8-yl)pyrrolidine-1-carb oxylate (400 mg, yield: 93%). ES-API: [M+H]⁺=430.3.

### Example 1. Synthesis of compound Z1

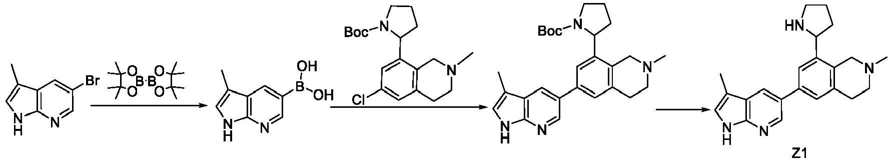

Step 1: 5-Bromo-3-methyl-1H-pyrrolo[2,3-b]pyridine (250 mg, 1.18 mmol), bis(pinacolato)diboron (902 mg, 3.55 mmol), Pd(dppf)Cl₂ (86 mg, 0.0118 mmol) and potassium acetate (173 mg, 1.77 mmol) were dissolved in 1,4-dioxane (20 ml), and the system reacted at 80°C for 5 h. Upon completion of the reaction, ethyl acetate was added. The obtained mixture was washed with water and saturated brine successively, dried over anhydrous sodium sulfate, and purified to give (3-methyl-1H-pyrrolo[2,3-b]pyridin-5-yl)boronic acid (130 mg, yield: 62%). ES-API: [M+H]⁺= 177.0
Step 2: The (3-methyl-1H-pyrrolo[2,3-b]pyridin-5-yl)boronic acid (16 mg, 0.09 mmol), tert-butyl 2-(6-chloro-2-methyl-1,2,3,4-tetrahydroisoquinolin-8-yl)pyrrolidine-1-carboxylate (15 mg, 0.045 mmol), Sphos Pd G2 (3.2 mg, 0.0045 mmol) and K₃PO₄ (28.6 mg, 0.135 mmol) were added to 1,4-dioxane (1 ml) and water (0.2 ml). The system was replaced with nitrogen and reacted under microwave at 100°C for 1 h. Upon completion of the reaction, ethyl acetate was added. The obtained mixture was washed with water and saturated brine successively, dried over anhydrous sodium sulfate, and purified to give tert-butyl 2-(2-methyl-6-(3-methyl-1H-pyrrolo[2,3-b]pyridin-5-yl)-1,2,3,4-tetrahydroisoquinolin-8-y l)pyrrolidine-1-carboxylate (10 mg, yield: 50%). ES-API: [M+H]⁺= 447.2
Step 3: The tert-butyl 2-(2-methyl-6-(3-methyl-1H-pyrrolo[2,3-b]pyridin-5-yl)-1,2,3,4-tetrahydroisoquinolin-8-y l)pyrrolidine-1-carboxylate (10 mg, 0.022 mmol) was added to 4M hydrochloric acid-methanol solution (5 ml), and the system reacted at room temperature for 2 h. Upon completion of the reaction, the reaction mixture was concentrated and purified by prep-HPLC (formic acid method) to give 2-(2-methyl-6-(3-methyl-1H-pyrrolo[2,3-b]pyridin-5-yl)-1,2,3,4-tetrahydroisoquinolin-8-y l)pyrrolidine (Z1, formate, 2.3 mg, yield: 23%). ES-API: [M+H]⁺= 347.2

### Example 2. Synthesis of compound Z2

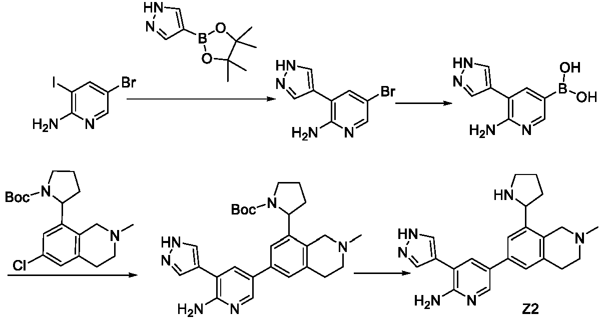

Step 1: 5-Bromo-3-iodopyridin-2-amine (1.49 g, 5 mmol), 4-(4,4,5,5-tetramethyl-1,3,2-dioxaborolan-2-yl)-1H-pyrazole (970 mg, 5 mmol), Pd(dppf)Cl₂ (365 mg, 0.5 mmol) and potassium carbonate (2.07 g, 15 mmol) were dissolved in 1,4-dioxane (30 ml), and the system reacted at 85°C for 20 h. Upon completion of the reaction, ethyl acetate was added. The obtained mixture was washed with water and saturated brine successively, dried over anhydrous sodium sulfate, and purified to give 5-bromo-3-(1H-pyrazol-4-yl)pyridin-2-amine (350 mg, yield: 29%). ES-API: [M+H]⁺= 239.0
Step 2: The 5-bromo-3-(1H-pyrazol-4-yl)pyridin-2-amine (350 mg, 1.47 mmol), bis(pinacolato)diboron (1.12 g, 4.43 mmol), Pd(dppf)Cl₂ (120 mg, 0.147 mmol) and potassium acetate (434 mg, 4.43 mmol) were dissolved in 1,4-dioxane (20 ml), and the system reacted at 80°C for 20 h. Upon completion of the reaction, ethyl acetate was added. The obtained mixture was washed with water and saturated brine successively, dried over anhydrous sodium sulfate, and purified to give (6-amino-5-(1H-pyrazol-4-yl)pyridin-3-yl)boronic acid (100 mg, yield: 33%). ES-API: [M+H]⁺= 205.0
Step 3: The (6-amino-5-(1H-pyrazol-4-yl)pyridin-3-yl)boronic acid (20 mg, 0.098 mmol), tert-butyl 2-(6-chloro-2-methyl-1,2,3,4-tetrahydroisoquinolin-8-yl)pyrrolidine-1-carboxylate (34 mg, 0.098 mmol), Sphos Pd G2 (3.2 mg, 0.0049 mmol) and K₃PO₄ (62 mg, 0.294 mmol) were added to 1,4-dioxane (5 ml) and water (1ml). The system was replaced with nitrogen and reacted under microwave at 100°C for 1 h. Upon completion of the reaction, ethyl acetate was added. The obtained mixture was washed with water and saturated brine successively, dried over anhydrous sodium sulfate, and purified to give tert-butyl 2-(6-(6-amino-5-(1H-pyrazol-4-yl)pyridin-3-yl)-2-methyl-1,2,3,4-tetrahydroisoquinolin-8-yl)pyrrolidine-1-carboxylate (10 mg, yield: 21%). ES-API: [M+H]⁺= 475.2
Step 4: The tert-butyl 2-(6-(6-amino-5-(1H-pyrazol-4-yl)pyridin-3-yl)-2-methyl-1,2,3,4-tetrahydroisoquinolin-8-yl)pyrrolidine-1-carboxylate (10 mg, 0.021 mmol) was added to 4M hydrochloric acid-methanol solution (5 ml), and the system reacted at room temperature for 2 h. Upon completion of the reaction, the reaction mixture was concentrated and purified by prep-HPLC (ammonium bicarbonate method) to give 5-(2-methyl-8-(pyrrolidin-2-yl)-1,2,3,4-tetrahydroisoquinolin-6-yl)-3-(1*H*-pyrazol-4-yl)py ridin-2-amine (Z2, 1.79 mg, yield: 22%). ES-API: [M+H]⁺=375.2.

### Example 3. Synthesis of compound Z3 and isomers thereof

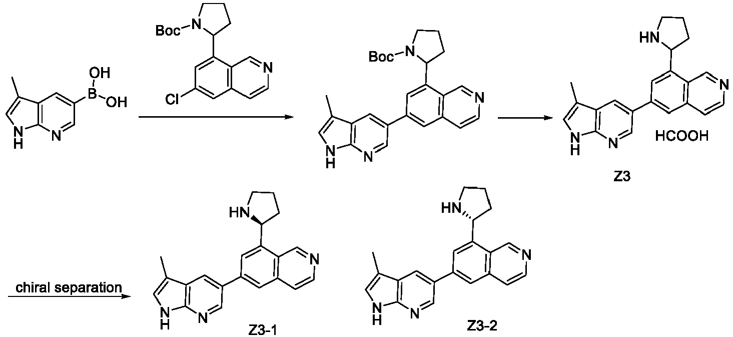

Step 1: (3-Methyl-1H-pyrrolo[2,3-b]pyridin-5-yl)boronic acid (66 mg, 0.2 mmol), tert-butyl 2-(6-chloroisoquinolin-8-yl)pyrrolidine-1-carboxylate (70 mg, 0.4 mmol), Sphos Pd G2 (14.4 mg, 0.02 mmol) and K₃PO₄ (127 mg, 0.6 mmol) were added to 1,4-dioxane (2 ml) and water (0.2 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified to give tert-butyl 2-(6-(3-(methyl-1H-pyrrolo[2,3-b]pyridin-5-yl]isoquinolin-8-yl)pyrrolidine-1-carboxylate (60 mg, yield: 70%). ES-API: [M+H]⁺= 429.2
Step 2: The tert-butyl 2-(6-(3-(methyl-1H-pyrrolo[2,3-b]pyridin-5-yl]isoquinolin-8-yl)pyrrolidine-1-carboxylate (60 mg, 0.14 mmol) was added to 4M hydrochloric acid-methanol solution (5 ml), and the system reacted at room temperature for 2 h. Upon completion of the reaction, the reaction mixture was concentrated and purified by prep-HPLC (ammonium bicarbonate method) to give 6-(3-methyl-1H-pyrrolo[2,3-b]pyridin-5-yl)-8-(pyrrolidin-2-yl)isoquinoline formate (Z3, 19 mg, yield: 41%). ES-API: [M+H]⁺= 329.2
Step 3: The compound Z3 (15 mg) obtained from the above steps was resolved by chiral preparation column (separation column IC 250 mm*4.6 mm*5 um, mobile phase: HEX-ETOH-DEA = 50:50:2, flow rate: 1 ml/min, column temperature: 30°C) to give two isomeric compounds. A structure of one isomeric compound was arbitrarily assigned as (S)-6-(3-methyl-1H-pyrrolo[2,3-b]pyridin-5-yl)-8-(pyrrolidin-2-yl)isoquinoline (Z3-1, 3.5 mg, peak 1, retention time: 15.24 min, yield: 23%, ee: 100%), ES-API: [M+H]⁺= 329.2; ¹H NMR (500 MHz, DMSO-*d*₆) δ 11.46 (s, 1H), 9.62 (s, 1H), 8.66 (d, *J* = 2.2 Hz, 1H), 8.53 (d, *J* = 5.6 Hz, 1H), 8.33 (d, *J* = 2.2 Hz, 1H), 8.18 (d, *J* = 1.8 Hz, 1H), 7.88 (d, *J* = 5.6 Hz, 1H), 7.34 - 7.30 (m, 1H), 5.02 (t, *J* = 7.6 Hz, 1H), 3.18 (dt, *J* = 9.8, 6.3 Hz, 1H), 3.07 (dt, *J* = 9.8, 7.4 Hz, 1H), 2.35 (d, *J* = 1.1 Hz, 3H), 2.05 - 1.91 (m, 1H), 1.90-1.85 (m, 2H), 1.69-1.32 (m, 1H). A structure of the other isomeric compound was arbitrarily assigned as (*R*)-6-(3-methyl-1H-pyrrolo[2,3-b]pyridin-5-yl)-8-(pyrrolidin-2-yl)isoquinoline (Z3-2, 4.5 mg, peak 2, retention time: 24.78 min, yield: 30%, ee: 100%), ES-API: [M+H]⁺=329.2.

### Example 4. Synthesis of compound Z4

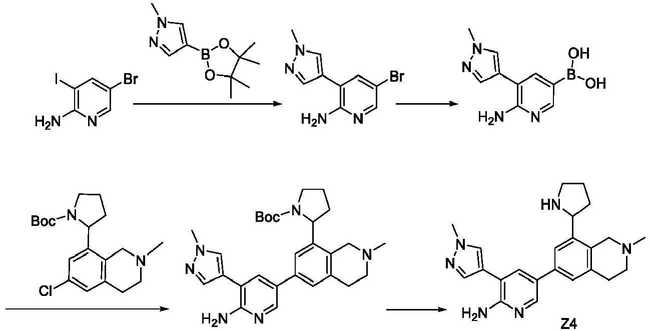

Step 1: 5-Bromo-3-iodopyridin-2-amine (2 g, 6.69 mmol), 1-methyl-4-(4,4,5,5-tetramethyl-1,3,2-dioxaborolan-2-yl)-1H-pyrazole (2.08 mg, 10.0 mmol), Pd(dppf)Cl₂ (273 mg, 0.05 mmol) and potassium carbonate (2.3 g, 16.72 mmol) were dissolved in 1,4-dioxane (30 ml) and water (3 ml), and the system reacted at 85°C for 20 h. Upon completion of the reaction, ethyl acetate was added. The obtained mixture was washed with water and saturated brine successively, dried over anhydrous sodium sulfate, and purified to give 5-bromo-3-(1-methyl-1H-pyrazol-4-yl)pyridin-2-amine (900 mg, yield: 53.6%). ES-API: [M+H]⁺= 253.0
Step 2: The 5-bromo-3-(1-methyl-1H-pyrazol-4-yl)pyridin-2-amine (900 mg, 3.55 mmol), bis(pinacolato)diboron (1.8 g, 7.11 mmol), Pd(dppf)Cl₂ (145 mg, 0.177 mmol) and potassium acetate (697 mg, 7.11 mmol) were dissolved in 1,4-dioxane (20 ml), and the system reacted at 80°C for 20 h. Upon completion of the reaction, ethyl acetate was added. The obtained mixture was washed with water and saturated brine successively, dried over anhydrous sodium sulfate, and purified to give (6-amino-5-(1-methyl-1H-pyrazol-4-yl)pyridin-3-yl)boronic acid (220 mg, yield: 28%). ES-API: [M+H]⁺= 219.0
Step 3: The (6-amino-5-(1-methyl-1H-pyrazol-4-yl)pyridin-3-yl)boronic acid (218 mg, 1.0 mmol), tert-butyl 2-(6-chloro-2-methyl-1,2,3,4-tetrahydroisoquinolin-8-yl)pyrrolidine-1-carboxylate (160 mg, 0.457 mmol), Sphos Pd G2 (72 mg, 0.1 mmol) and K₂CO₃ (414 mg, 3.0 mmol) were added to 1,4-dioxane (6 ml) and water (1 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified to give tert-butyl 2-(6-(6-amino-5-(1-methyl-1H-pyrazol-4-yl)pyridin-3-yl)-2-methyl-1,2,3,4-tetrahydroisoq uinolin-8-yl)pyrrolidine-1-carboxylate (150 mg, yield: 67%). ES-API: [M+H]⁺= 489.2
Step 4: The tert-butyl 2-(6-(6-amino-5-(1-methyl-1H-pyrazol-4-yl)pyridin-3-yl)-2-methyl-1,2,3,4-tetrahydroisoq uinolin-8-yl)pyrrolidine-1-carboxylate (150 mg, 0.307 mmol) was added to 4M hydrochloric acid-methanol solution (8 ml), and the system reacted at room temperature for 2 h. Upon completion of the reaction, the reaction mixture was concentrated and purified by prep-HPLC (ammonium bicarbonate method) to give 3-(1-methyl-1H-pyrazol-4-yl)-5-(2-methyl-8-(pyrrolidin-2-yl)-1,2,3,4-tetrahydroisoquinoli n-6-yl)pyridin-2-amine (60 mg, yield: 50.4%). ES-API: [M+H]⁺=389.2. ¹H NMR (500 MHz, DMSO-*d*₆) δ 8.26 (d, *J* = 2.4 Hz, 1H), 8.10 (s, 1H), 7.83 (d, *J* = 4.3 Hz, 2H), 7.68 (s, 1H), 7.40 (d, *J* = 1.8 Hz, 1H), 5.78 (s, 2H), 4.54 - 4.46 (m, 1H), 3.89 (s, 3H), 3.72 (d, *J* = 15.4 Hz, 1H), 3.50 (d, *J* = 15.2 Hz, 1H), 3.21 (t, *J* = 7.9 Hz, 1H), 2.92 - 2.89 (m, 3H), 2.66 - 2.54 (m, 3H), 2.41 (s, 3H), 2.30 (t, *J* = 10.4 Hz, 1H), 2.08 - 1.84 (m, 3H).

### Example 5. Synthesis of compound Z93 and isomers thereof

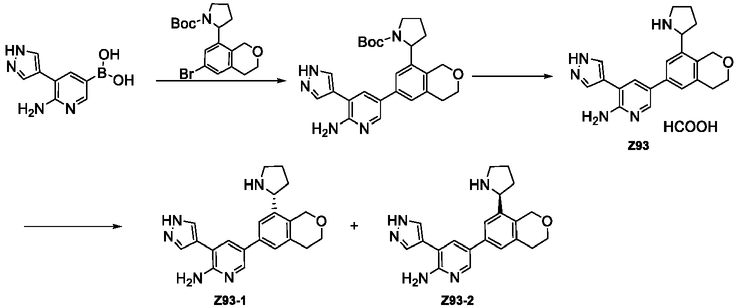

Step 1: (6-Amino-5-(1H-pyrazol-4-yl)pyridin-3-yl)boronic acid (48 mg, 0.236 mmol), tert-butyl 2-(6-bromoisochroman-8-yl)pyrrolidine-1-carboxylate (45 mg, 0.118 mmol), Sphos Pd G2 (34 mg, 0.0472 mmol) and K₂CO₃ (98 mg, 0.708 mmol) were added to 1,4-dioxane (2 ml) and water (0.5 ml). The system was replaced with nitrogen and reacted under microwave at 100°C for 1 h. Upon completion of the reaction, ethyl acetate was added. The obtained mixture was washed with water and saturated brine successively, dried over anhydrous sodium sulfate, and purified (petroleum ether:ethyl acetate = 10:90) to give tert-butyl 2-(6-(6-(6-amino-5-(1H-pyrazol-4-yl)pyridin-3-yl)isochroman-8-yl)pyrrolidine-1-carboxy late (32 mg, yield: 60%). ES-API: [M+H]⁺=462.2.

Step 2: The tert-butyl 2-(6-(6-(6-amino-5-(1H-pyrazol-4-yl)pyridin-3-yl)isochroman-8-yl)pyrrolidine-1-carboxy late (32 mg, 0.069 mmol) was added to 4M hydrochloric acid-methanol solution (5 ml), and the system reacted at room temperature for 3 h. Upon completion of the reaction, the reaction mixture was concentrated. After neutralization with 7M ammonia/methanol solution (1 mL), the obtained mixture was concentrated again and purified by prep-HPLC (formic acid method) to give 3-(1H-pyrazol-4-yl)-5-(8-(pyrrolidin-2-yl)isochroman-6-yl)pyridin-2-amine formate (20 mg, yield: 80%). ES-API: [M+H]⁺=362.2.

Step 3: The 3-(1H-pyrazol-4-yl)-5-(8-(pyrrolidin-2-yl)isochroman-6-yl)pyridin-2-amine formate (Z93, 20 mg) was resolved by chiral preparation column (separation column IC 250 mm*4.6 mm*5 um, mobile phase: HEX-ETOH-DEA = 50:50:2, flow rate: 1 ml/min, column temperature: 30°C, 35 MIN) to give two isomeric compounds. A structure of one isomeric compound was arbitrarily assigned as (R)-3-(1H-pyrazol-4-yl)-5-(8-(pyrrolidin-2-yl)isochroman-6-yl)pyridin-2-amine (Z93-1, 3.8 mg, peak 1, retention time: 9.20 min, yield: 19%, ee: 97%), ES-API: [M+H]⁺= 362.2; ¹H NMR (500 MHz, DMSO-*d*₆) δ 8.17 (d, *J* = 2.4 Hz, 1H), 7.99 (s, 3H), 7.73 (d, *J* = 2.4 Hz, 1H), 7.59 (d, *J* = 1.9 Hz, 1H), 7.27 (d, *J* = 1.9 Hz, 1H), 5.73 (s, 2H), 4.87 - 4.68 (m, 3H), 4.04 (t, *J* = 7.7 Hz, 1H), 3.89 - 3.82 (m, 2H), 3.14-3.04 (m, 1H), 2.93 - 2.88 (m, 1H), 2.87 - 2.83 (m, 2H), 2.17-2.10 (m, 1H), 1.84 - 1.79 (m, 1H), 1.76-1.70 (m, 1H), 1.46 - 1.40 (m, 1H); a structure of the other isomeric compound was arbitrarily assigned as (S)-3-(1H-pyrazol-4-yl)-5-(8-(pyrrolidin-2-yl)isochroman-6-yl)pyridin-2-amine (Z93-2, 3 mg, peak 2, retention time: 15.54 min, yield: 15%, ee: 96.8%), ES-API: [M+H]⁺= 362.2.

### Example 6. Synthesis of compound Z94 and isomers thereof

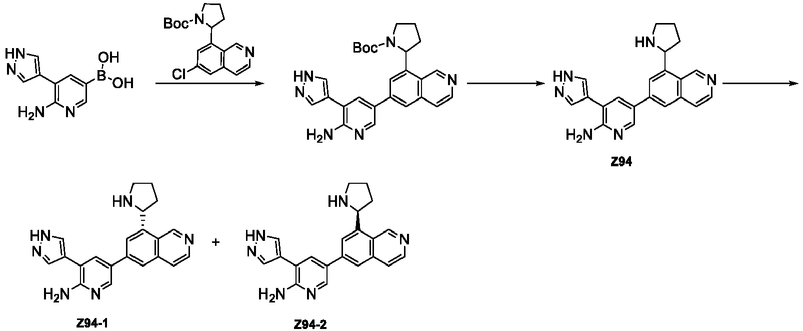

Step 1: (6-Amino-5-(1H-pyrazol-4-yl)pyridin-3-yl)boronic acid (55 mg, 0.271 mmol), tert-butyl 2-(6-chloroisoquinolin-8-yl)pyrrolidine-1-carboxylate (45 mg, 0.135 mmol), Sphos Pd G2 (19.5 mg, 0.0271 mmol) and K₂CO₃ (56 mg, 0.405 mmol) were added to 1,4-dioxane (2 ml) and water (0.5 ml)). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified (dichloromethane:methanol = 90:10) to give tert-butyl 2-(6-(6-(6-amino-5-(1H-pyrazol-4-yl)pyridin-3-yl)isoquinolin-8-yl)pyrrolidine-1-carboxyl ate (24 mg, yield: 39%). ES-API: [M+H]⁺=457.2.

Step 2: The tert-butyl 2-(6-(6-(6-amino-5-(1H-pyrazol-4-yl)pyridin-3-yl)isoquinolin-8-yl)pyrrolidine-1-carboxyl ate (24 mg, 0.069 mmol) was added to 4M hydrochloric acid-methanol solution (5 ml), and the system reacted at room temperature for 3 h. Upon completion of the reaction, the reaction mixture was concentrated and purified by prep-HPLC (ammonium bicarbonate method) to give 3-(1H-pyrazol-4-yl)-5-(8-(pyrrolidin-2-yl)isoquinolin-6-yl)pyridin-2-amine (Z94, 10 mg, yield: 53%). ES-API: [M+H]⁺=357.2.

Step 3: 3-(1H-pyrazol-4-yl)-5-(8-(pyrrolidin-2-yl)isoquinolin-6-yl)pyridin-2-amine (Z94, 10 mg) was resolved by chiral preparation column (separation column IC 250 mm*4.6 mm*5 um, mobile phase: HEX-ETOH-DEA = 50:50:2, flow rate: 1 ml/min, column temperature: 30°C, 35 MIN) to give two isomeric compounds. A structure of one isomeric compound was arbitrarily assigned as (R)-3-(1H-pyrazol-4-yl)-5-(8-(pyrrolidin-2-yl)isoquinolin-6-yl)pyridin-2-amine (Z94-1, 3.9 mg, peak 1, retention time: 11.90 min, yield: 39%, ee value: 97%), ES-API: [M+H]⁺= 357.2; ¹H NMR (500 MHz, DMSO-*d*₆) δ 9.57 (s, 1H), 8.49 (d, *J* = 5.6 Hz, 1H), 8.39 (d, *J* = 2.4 Hz, 1H), 8.14 (d, *J* = 1.8 Hz, 1H), 8.08 (d, *J* = 1.8 Hz, 1H), 8.03 (s, 2H), 7.94 (d, *J* = 2.4 Hz, 1H), 7.81 (d, *J* = 5.6 Hz, 1H), 5.96 (s, 2H), 4.96 (t, *J* = 7.6 Hz, 1H), 3.15 (dt, *J* = 9.9, 6.3 Hz, 1H), 3.07 - 3.02 (m, 1H), 2.46 - 2.41 (m, 1H), 1.94 - 1.79 (m, 3H), 1.65 - 1.58 (m, 1H); a structure of the other isomeric compound was arbitrarily assigned as (S)-3-(1H-pyrazol-4-yl)-5-(8-(pyrrolidin-2-yl)isoquinolin-6-yl)pyridin-2-amine (Z94-2, 3.5 mg, peak 2, retention time: 15.58 min, yield: 35%, ee value: 96.5%), ES-API: [M+H]⁺= 357.2.

### Example 7. Synthesis of compound Z95 and isomers thereof

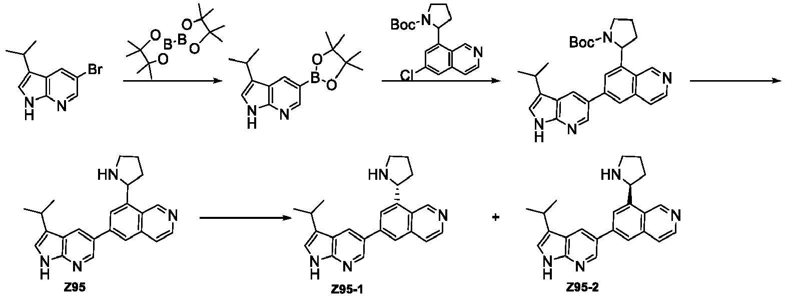

Step 1: 5-Bromo-3-isopropyl-1H-pyrrolo[2,3-b]pyridine (250 mg, 1.05 mmol), bis(pinacolato)diboron (800 mg, 3.15 mmol), Pd(dppf)Cl₂ (77 mg, 0.105 mmol) and potassium acetate (309 mg, 3.15 mmol) were added to dried 1,4-dioxane (8 mL). The system was replaced with nitrogen and reacted under microwave at 100°C for 0.5 h. Upon completion of the reaction, the reaction mixture was diluted with ethyl acetate, filtered, concentrated, and purified by silica gel (petroleum ether:ethyl acetate = 80:20) to give 3-isopropyl-5-(4,4,5,5-tetramethyl-1,3,2-dioxaborolan-2-yl)-1H-pyrrolo[2,3-b]pyridine (230 mg, yield: 76%). ES-API: [M+H]⁺ = 287.2.

Step 2: The 3-isopropyl-5-(4,4,5,5-tetramethyl-1,3,2-dioxaborolan-2-yl)-1H-pyrrolo[2,3-b]pyridine (61 mg, 0.3 mmol), tert-butyl 2-(6-chloroisoquinolin-8-yl)pyrrolidine-1-carboxylate (50 mg, 0.15 mmol), Sphos Pd G2 (21.6 mg, 0.03 mmol) and K₂CO₃ (62 mg, 0.45 mmol) were added to 1,4-dioxane (2 ml) and water (0.5 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified to give tert-butyl 2-(6-(3-(isopropyl-1H-pyrrolo[2,3-b]pyridin-5-yl]isoquinolin-8-yl)pyrrolidine-1-carboxyla te (45 mg, yield: 66%). ES-API: [M+H]⁺ = 457.2.

Step 3: The tert-butyl 2-(6-(3-(isopropyl-1H-pyrrolo[2,3-b]pyridin-5-yl]isoquinolin-8-yl)pyrrolidine-1-carboxyla te (45 mg, 0.099 mmol) was added to 4M hydrochloric acid-methanol solution (5 ml), and the system reacted at room temperature for 2 h. Upon completion of the reaction, the reaction mixture was concentrated and purified by prep-HPLC (ammonium bicarbonate method) to give 6-(3-isopropyl-1H-pyrrolo[2,3-b]pyridin-5-yl)-8-(pyrrolidin-2-yl)isoquinoline (Z95, 18 mg, yield: 51%). ES-API: [M+H]⁺=357.2.

Step 4: The 6-(3-isopropyl-1H-pyrrolo[2,3-b]pyridin-5-yl)-8-(pyrrolidin-2-yl)isoquinoline (Z95, 15 mg) was chirally resolved by SFC (column type: Chiralpak IC 250 mm*4.6 mm*5 um, mobile phase: HEX-ETOH-DEA = 40:60:2, flow rate: 1 ml/min, column temperature: 30°C, 35 MIN) to give two isomeric compounds. A structure of one isomeric compound was arbitrarily assigned as (R)-6-(3-isopropyl-1H-pyrrolo[2,3-b]pyridin-5-yl)-8-(pyrrolidin-2-yl)isoquinoline (Z95-1, 3.8 mg, yield: 25%, retention time: 10.29 min, ee value: 100%). ES-API:[M+H]⁺ =357.2. ¹H NMR (500 MHz, DMSO-*d*₆) δ 11.48 (s, 1H), 9.62 (s, 1H), 8.64 (d, *J* = 2.2 Hz, 1H), 8.52 (d, *J* = 5.6 Hz, 1H), 8.36 (d, *J* = 2.2 Hz, 1H), 8.23 (d, *J* = 1.9 Hz, 1H), 8.17 (d, *J* = 1.8 Hz, 1H), 7.89 (d, *J* = 5.6 Hz, 1H), 7.29 (d, *J* = 2.5 Hz, 1H), 5.00 (t, *J* = 7.6 Hz, 1H), 3.27 - 3.23 (m, 1H), 3.16 (dt, *J* = 9.8, 6.2 Hz, 1H), 3.06 (dt, *J* = 9.8, 7.4 Hz, 1H), 2.46 (d, *J* = 7.1 Hz, 1H), 2.04 - 1.80 (m, 3H), 1.66-1.62 (m, 1H), 1.36 (d, *J* = 6.9 Hz, 6H); a structure of the other isomeric compound was arbitrarily assigned as (S)-6-(3-isopropyl-1H-pyrrolo[2,3-b]pyridin-5-yl)-8-(pyrrolidin-2-yl)isoquinoline (Z95-2, 2.7 mg, yield: 18%, retention time: 16.30 min, ee value: 100%). ES-API:[M+H]⁺ =357.2.

### Example 8. Synthesis of compound Z96

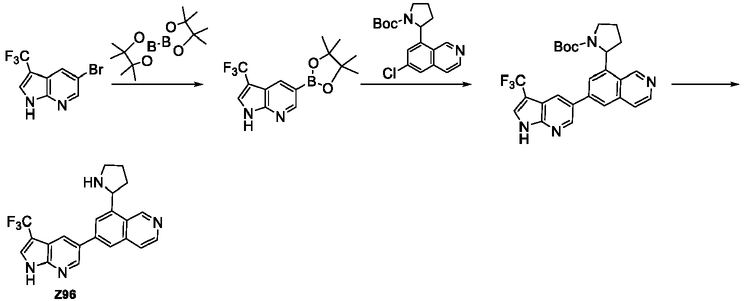

Step 1: 5-Bromo-3-(trifluoromethyl)-1H-pyrrolo[2,3-b]pyridine (250 mg, 0.947 mmol), bis(pinacolato)diboron (721 mg, 2.84 mmol), Pd(dppf)Cl₂ (69 mg, 0.0947 mmol) and potassium acetate (278 mg, 2.84 mmol) were added to dried 1,4-dioxane (8 mL). The system was replaced with nitrogen and reacted under microwave at 110°C for 0.5 h. Upon completion of the reaction, the reaction mixture was diluted with ethyl acetate, filtered, concentrated, and purified by silica gel (petroleum ether:ethyl acetate = 75:25) to give 3-(trifluoromethyl)-5-(4,4,5,5-tetramethyl-1,3,2-dioxaborolan-2-yl)-1H-pyrrolo[2,3-b]pyri dine (240 mg, yield: 82%). ES-API: [M+H]⁺=313.1.

Step 2: The 3-(trifluoromethyl)-5-(4,4,5,5-tetramethyl-1,3,2-dioxaborolan-2-yl)-1H-pyrrolo[2,3-b]pyri dine (94 mg, 0.3 mmol), tert-butyl 2-(6-chloroisoquinolin-8-yl)pyrrolidine-1-carboxylate (50 mg, 0.15 mmol), Sphos Pd G2 (21.6 mg, 0.03 mmol) and K₂CO₃ (62 mg, 0.45 mmol) were added to 1,4-dioxane (2 ml) and water (0.5 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified to give tert-butyl 2-(6-(3-trifluoromethyl-1H-pyrrolo[2,3-b]pyridin-5-yl)isoquinolin-8-yl)pyrrolidine-1-carb oxylate (46 mg, yield: 61%). ES-API: [M+H]⁺=483.2.

Step 3: The tert-butyl 2-(6-(3-trifluoromethyl-1H-pyrrolo[2,3-b]pyridin-5-yl)isoquinolin-8-yl)pyrrolidine-1-carb oxylate (46 mg, 0.093 mmol) was added to 4M hydrochloric acid-methanol solution (5 ml), and the system reacted at room temperature for 2 h. Upon completion of the reaction, the reaction mixture was concentrated and purified by prep-HPLC (ammonium bicarbonate method) to give 6-(3-(trifluoromethyl)-1H-pyrrolo[2,3-b]pyridin-5-yl)-8-(pyrrolidin-2-yl)isoquinoline (Z96, 10 mg, yield: 28%). ES-API: [M+H]⁺=383.2. ¹H NMR (500 MHz, DMSO-*d*₆) δ 9.64 (s, 1H), 8.84 (d, *J* = 2.1 Hz, 1H), 8.55 (d, *J* = 5.6 Hz, 1H), 8.36 (d, *J* = 2.1 Hz, 1H), 8.27 (q, *J* = 1.4 Hz, 1H), 8.24 - 8.22 (m, 2H), 7.92 (d, *J* = 5.6 Hz, 1H), 5.00 (t, *J* = 7.6 Hz, 1H), 3.15 (ddd, *J* = 9.8, 6.8, 5.4 Hz, 1H), 3.06 (dt, *J* = 9.8, 7.4 Hz, 1H), 2.46 (s, 1H), 1.85 (dq, *J* = 10.1, 7.4 Hz, 3H), 1.65 - 1.58 (m, 1H).

### Example 9. Synthesis of compound Z97 and isomers thereof

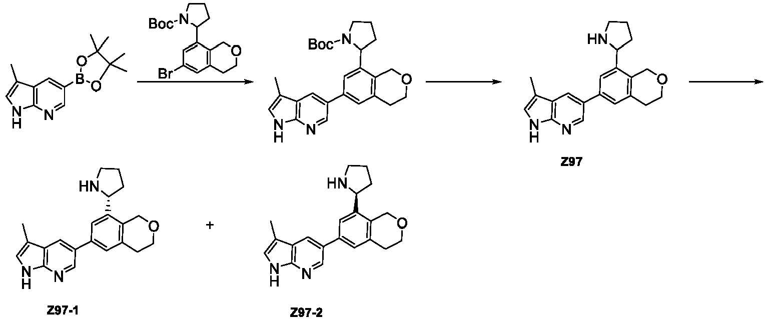

Step 1: Pinacol (3-methyl-1H-pyrrolo[2,3-b]pyridin-5-yl)borate (76 mg, 0.296 mmol), tert-butyl 2-(6-bromoisochroman-8-yl)pyrrolidine-1-carboxylate (50 mg, 0.148 mmol), Sphos Pd G2 (21 mg, 0.0296 mmol) and K₂CO₃ (61 mg, 0.444 mmol) were added to 1,4-dioxane (2 ml) and water (0.5 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified (petroleum ether:ethyl acetate = 20:80) to give tert-butyl 2-(6-(3-(1-methyl-1H-pyrrolo[2,3-b]pyridin-5-yl)isochroman-8-yl)pyrrolidine-1-carboxyla te (45 mg, yield: 70%). ES-API: [M+H]⁺=434.1.

Step 2: The tert-butyl 2-(6-(3-(1-methyl-1H-pyrrolo[2,3-b]pyridin-5-yl)isochroman-8-yl)pyrrolidine-1-carboxyla te (45 mg, 0.108 mmol) was dissolved in dichloromethane (2 mL) and added to 1 mL of trifluoroacetic acid, and the system reacted at room temperature for 0.5 h. Upon completion of the reaction, the reaction mixture was concentrated. After neutralization with 7M ammonia/methanol solution (1 mL), the obtained mixture was concentrated again, and purified by prep-HPLC (ammonia water method) to give 3-methyl-5-(8-(pyrrolidin-2-yl)isochroman-6-yl)-1H-pyrrolo[2,3-b]pyridine (Z97, 10 mg, yield: 27.8%). ES-API: [M+H]⁺=334.1. ¹H NMR (500 MHz, DMSO-*d*₆) δ 11.32 (s, 1H), 8.45 (d, *J* = 2.2 Hz, 1H), 8.09 (d, *J* = 2.2 Hz, 1H), 7.70 (d, *J* = 2.0 Hz, 1H), 7.34 (d, *J* = 2.0 Hz, 1H), 7.25 (dd, *J* = 2.5, 1.3 Hz, 1H), 4.95 - 4.62 (m, 3H), 4.05 (t, *J* = 7.7 Hz, 1H), 3.92 - 3.83 (m, 2H), 3.12-3.07 (m, 1H), 2.94 - 2.85 (m, 3H), 2.31 (t, *J* = 1.9 Hz, 3H), 2.17-2.13 (m, 1H), 1.93 - 1.61 (m, 3H), 1.48-1.42 (m, 1H).

Step 3: The 3-methyl-5-(8-(pyrrolidin-2-yl)isochroman-6-yl)-1H-pyrrolo[2,3-b]pyridine (Z97, 80 mg) was chirally resolved by SFC (column type: Chiralpak IC 250 mm*4.6 mm*5 um, mobile phase: HEX-ETOH-DEA = 50:50:2, flow rate: 1 ml/min, column temperature: 30°C, 35 MIN) to give two isomeric compounds. A structure of one isomeric compound was arbitrarily assigned as (R)-3-methyl-5-(8-(pyrrolidin-2-yl)isochroman-6-yl)-1H-pyrrolo[2,3-b]pyridine (Z97-1, 30 mg, yield: 37.5%, retention time: 6.88 min, purity: 100%, ee value: 100%). ES-API:[M+H]⁺ =334.1. ¹H NMR (500 MHz, DMSO-*d*₆) δ 11.32 (s, 1H), 8.45 (d, *J=* 2.2 Hz, 1H), 8.09 (d, *J* = 2.2 Hz, 1H), 7.70 (d, *J* = 1.9 Hz, 1H), 7.34 (d, *J* = 1.9 Hz, 1H), 7.25 (s, 1H), 4.87 (d, *J* = 15.2 Hz, 1H), 4.75 (d, *J* = 15.2 Hz, 1H), 4.04 (t, *J* = 7.7 Hz, 1H), 3.90-3.86 (m, 2H), 3.50 (d, *J* = 10.0 Hz, 1H), 3.10-1.06 (m, 1H), 2.89 (dt, *J* = 10.3, 7.1 Hz, 3H), 2.31 (s, 3H), 2.15-2.10 (m, 1H), 1.80-1.76 (m, 2H), 1.45-1.40 (, 1H); a structure of the other isomeric compound was arbitrarily assigned as (S)-3-methyl-5-(8-(pyrrolidin-2-yl)isochroman-6-yl)-1H-pyrrolo[2,3-b]pyridine (Z97-2, 30 mg, yield: 37.5%, retention time: 11.09 min, purity: 100%, ee value: 100%). ES-API:[M+H]⁺ =334.1. ¹H NMR (500 MHz, DMSO-*d*₆) δ 11.32 (s, 1H), 8.45 (d, *J* = 2.2 Hz, 1H), 8.09 (d, *J* = 2.2 Hz, 1H), 7.70 (d, *J* = 1.9 Hz, 1H), 7.34 (s, 1H), 7.25 (s, 1H), 4.91 - 4.71 (m, 2H), 4.05 (t, *J* = 7.7 Hz, 1H), 3.90-3.86 (m, 2H), 3.67 - 3.42 (m, 1H), 3.10-1.06 (m, 1H), 2.89 (dt, *J* = 11.3, 6.6 Hz, 3H), 2.31 (s, 3H), 2.20 - 2.11 (m, 1H), 1.88 - 1.71 (m, 2H), 1.48 - 1.39 (m, 1H).

### Example 10. Synthesis of compound Z98 and isomers thereof

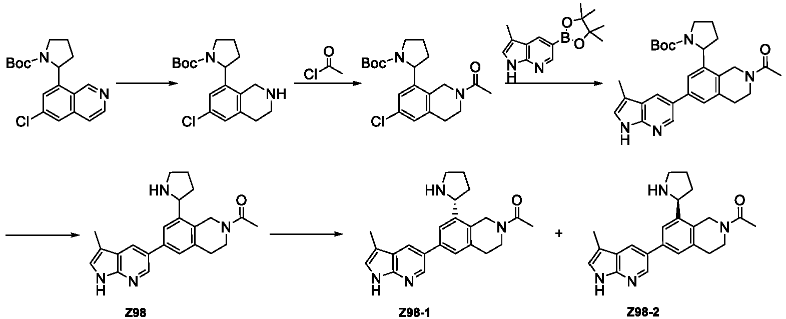

Step 1: Tert-butyl 2-(6-chloroisoquinolin-8-yl)pyrrolidine-1-carboxylate (60 mg, 0.18 mmol) was dissolved in 5 ml of acetic acid. NaBH₄ (55 mg, 1.44 mmol) was added at room temperature, and the system reacted for 0.5 h. The obtained mixture was concentrated, extracted with ethyl acetate, washed with saturated aqueous sodium bicarbonate solution and saturated brine successively, dried, and concentrated to give a product tert-butyl 2-(6-chloro-1,2,3,4-tetrahydroisoquinolin-8-yl)pyrrolidine-1-carboxylate (60 mg, crude product) which was directly used in the next step. ES-API: [M+H]⁺=337.1.

Step 2: The tert-butyl 2-(6-chloro-1,2,3,4-tetrahydroisoquinolin-8-yl)pyrrolidine-1-carboxylate (60 mg, 0.178) was dissolved in dichloromethane. Triethylamine (54 mg, 0.534 mmoL) and acetyl chloride (21 mg, 0.267 mmol) were added successively under an ice-water bath condition, and the system reacted for 1 h. Upon completion of the reaction, the reaction mixture was extracted with ethyl acetate, washed with saturated brine, dried, concentrated, and purified by silica gel (petroleum ether:ethyl acetate = 80:20) to give tert-butyl 2-(2-acetyl-6-chloro-1,2,3,4-tetrahydroisoquinolin-8-yl)pyrrolidine-1-carboxylate (60 mg, yield of the two steps: 88%). ES-API: [M+H]⁺=379.1.

Step 3: The tert-butyl 2-(2-acetyl-6-chloro-1,2,3,4-tetrahydroisoquinolin-8-yl)pyrrolidine-1-carboxylate (60 mg, 0.158 mmol), pinacol (3-methyl-1H-pyrrolo[2,3-b]pyridin-5-yl)borate (56 mg, 0.317 mmol), Sphos Pd G2 (23 mg, 0.0317 mmol) and K₂CO₃ (65.4 mg, 0.474 mmol) were added to 1,4-dioxane (2 ml) and water (0.5 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified to give tert-butyl 2-(2-acetyl-6-(3-methyl-1H-pyrrolo[2,3-b]pyridin-5-yl)-1,2,3,4-tetrahydroisoquinolin-8-yl )pyrrolidine-1-carboxylate (60 mg, yield: 80%). ES-API: [M+H]⁺ = 475.2.

Step 4: The tert-butyl 2-(2-acetyl-6-(3-methyl-1H-pyrrolo[2,3-b]pyridin-5-yl)-1,2,3,4-tetrahydroisoquinolin-8-yl )pyrrolidine-1-carboxylate (60 mg, 0.126 mmol) was dissolved in 3 ml of dichloromethane. 2 ml of trifluoroacetic acid was added, and the system reacted at room temperature for 1 h. Upon completion of the reaction, the reaction mixture was concentrated and purified by prep-HPLC (ammonia water method) to give 1-(6-(3-methyl-1H-pyrrolo[2,3-b]pyridin-5-yl)-8(pyrrolidin-2-yl)-3,4-dihydroisoquinolin-2(1H)-yl)ethanone (Z98, 35 mg, yield: 74%). ES-API: [M+H]⁺= 374.2. ¹H NMR (500 MHz, DMSO-*d*₆) δ 11.32 (s, 1H), 8.45 (d, *J* = 2.2 Hz, 1H), 8.09 (d, *J* = 2.5 Hz, 1H), 7.75 (dd, *J* = 10.4, 2.0 Hz, 1H), 7.39 (t, *J* = 3.0 Hz, 1H), 7.27 - 7.25 (m, 1H), 4.79 (t, *J* = 17.1 Hz, 1H), 4.64-4.68 (m, 1H), 4.23-4.18 (m, 1H), 3.68 (t, *J* = 5.9 Hz, 2H), 3.12-3.08 (m, 1H), 3.01 - 2.82 (m, 4H), 2.31 (t, *J* = 1.5 Hz, 3H), 2.14-2.10 (m, 4H), 1.82-1.78 (m, 2H), 1.53 - 1.38 (m, 1H).

Step 5: The 1-(6-(3-methyl-1H-pyrrolo[2,3-b]pyridin-5-yl)-8(pyrrolidin-2-yl)-3,4-dihydroisoquinolin-2(1H)-yl)ethanone (Z98, 35 mg) was chirally resolved by SFC (column type: ChiralpakIC 250 mm*4.6 mm*5 um, mobile phase: HEX-ETOH-DEA = 50:50:2, flow rate: 1 ml/min, column temperature: 30°C, 30 MIN) to give two isomeric compounds. A structure of one isomeric compound was arbitrarily assigned as (R)-1-(6-(3-methyl-1H-pyrrolo[2,3-b]pyridin-5-yl)-8-(pyrrolidin-2-yl)-3,4-dihydroisoquin olin-2(1H)-yl)ethanone (Z98-1, 10 mg, yield: 28.5%, retention time: 3.65 min, purity: 96%, ee value: 100%). ES-API:[M+H]⁺ = 374.2. ¹H NMR (500 MHz, DMSO-*d*₆) δ 11.33 (s, 1H), 8.46 (d, *J* = 2.2 Hz, 1H), 8.09 (d, *J* = 2.3 Hz, 1H), 7.75 (dd, *J* = 9.8, 2.0 Hz, 1H), 7.40 (dd, *J* = 5.7, 1.8 Hz, 1H), 7.26 (s, 1H), 4.79 (t, *J* = 17.1 Hz, 1H), 4.65-4.60 (m, 1H), 4.24 (dt, *J* = 19.8, 7.7 Hz, 1H), 3.68 (t, *J* = 5.9 Hz, 2H), 3.14-3.10 (m, 1H), 3.00 - 2.95 (m, 2H), 2.86 (t, *J* = 6.1 Hz, 1H), 2.31 (s, 3H), 2.25 - 2.17 (m, 1H), 2.16-2.10 (m, 3H), 1.91 - 1.75 (m, 3H), 1.56 - 1.42 (m, 1H). A structure of the other isomeric compound was arbitrarily assigned as (S)-1-(6-(3-methyl-1H-pyrrolo[2,3-b]pyridin-5-yl)-8-(pyrrolidin-2-yl)-3,4-dihydroisoquin olin-2(1H)-yl)ethanone (Z98-2, 10 mg, yield: 37.5%, retention time: 6.82 min, purity: 97%, ee value: 100%). ES-API:[M+H]⁺ = 374.2.

### Example 11. Synthesis of compound Z99

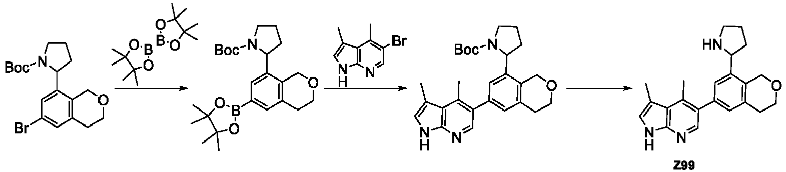

Step 1: Tert-butyl 2-(6-bromoisochroman-8-yl)pyrrolidine-1-carboxylate (381 mg, 1 mmol), bis(pinacolato)diboron (762 mg, 3 mmol), pd(dppf)Cl₂ (73 mg, 0.1 mmol) and potassium acetate (294 mg, 3 mmol) were dissolved in 1,4-dioxane (8 ml), and the system reacted under microwave at 100°C for 0.5 h. Upon completion of the reaction, ethyl acetate was added. The obtained mixture was filtered, concentrated, and purified (petroleum ether:ethyl acetate = 50:50) to give tert-butyl 2-(6-(4,4,5,5-tetramethyl-1,3,2-dioxaborolan-2-yl)isochroman-8-yl)pyrrolidine-1-carboxyl ate (400 mg, yield: 93%). ES-API: [M+H]⁺=430.3.

Step 2: The tert-butyl 2-(6-((4,4,5,5-tetramethyl-1,3,2-dioxaborolan-2-yl)isochroman-8-yl)pyrrolidine-1-carboxy late (123.5 mg, 0.288 mmol), 5-bromo-3,4-dimethyl-1H-pyrrolo[2,3-b]pyridine (50 mg, 0.222 mmol), Sphos Pd G2 (16 mg, 0.0222 mmol) and K₂CO₃ (92 mg, 0.666 mmol) were added to 1,4-dioxane (2 ml) and water (0.5 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified (petroleum ether:ethyl acetate = 20:80) to give tert-butyl 2-(6-(3,4-dimethyl-1H-pyrrolo[2,3-b]pyridin-5-yl)isochroman-8-yl)pyrrolidine-1-carboxyl ate (50 mg, yield: 50.5%). ES-API: [M+H]⁺=448.2.

Step 3: Tert-butyl 2-(6-(3-(1-methyl-1H-pyrrolo[2,3-b]pyridin-5-yl]isochroman-8-yl)pyrrolidine-1-carboxyla te (50 mg, 0.108 mmol) was dissolved in dichloromethane (2 mL) and added to 1 mL of trifluoroacetic acid, and the system reacted at room temperature for 0.5 h. Upon completion of the reaction, the reaction mixture was concentrated. After neutralization with 7M ammonia/methanol solution (1 mL), the obtained mixture was concentrated again and purified by prep-HPLC (ammonia water method) to give 3-methyl-5-(8-(pyrrolidin-2-yl)isochroman-6-yl)-1H-pyrrolo[2,3-b]pyridine (Z99, 34 mg, yield: 87%). ES-API: [M+H]⁺=348.2. ¹H NMR (500 MHz, DMSO-*d*₆) δ 11.24 - 11.17 (m, 1H), 7.94 (s, 1H), 7.35 (d, *J* = 1.9 Hz, 1H), 7.17 (s, 1H), 6.99 (dd, *J* = 14.6, 1.8 Hz, 1H), 4.90 - 4.75 (m, 2H), 4.08 (t, *J* = 7.5 Hz, 1H), 3.94 - 3.84 (m, 2H), 3.57 - 3.40 (m, 2H), 3.03-2.98 (m, 1H), 2.90 - 2.84 (m, 2H), 2.55 (d, *J* = 5.6 Hz, 3H), 2.45 (d, *J* = 1.1 Hz, 3H), 1.84 - 1.73 (m, 2H), 1.68 - 1.60 (m, 1H), 1.46-1.40 (m, 1H).

### Example 12. Synthesis of compound Z100 and isomers thereof

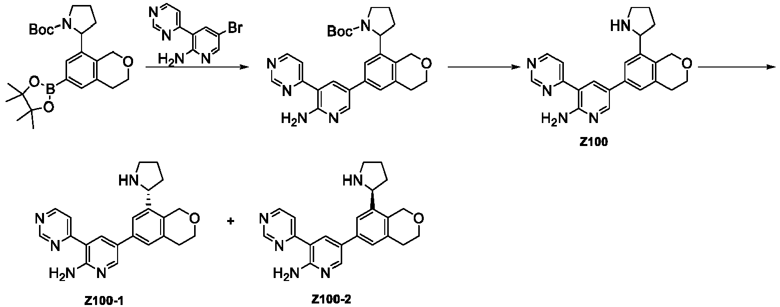

Step 1: Tert-butyl 2-(6-(4,4,5,5-tetramethyl-1,3,2-dioxaborolan-2-yl)isochroman-8-yl)pyrrolidine-1-carboxyl ate (50 mg, 0.2 mmol), 5-bromo-3-(pyrimidin-4-yl)pyridin-2-amine (86 mg, 0.2 mmol), Sphos Pd G2 (28.8 mg, 0.04 mmol) and K₂CO₃ (83 mg, 0.6 mmol) were added to 1,4-dioxane (2 ml) and water (0.5 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified (petroleum ether:ethyl acetate = 10:90) to give tert-butyl 2-(6-(6-amino-5-(pyrimidin-4-yl)pyridin-3-yl)isochroman-8-yl)pyrrolidine-1-carboxylate (57 mg, yield: 60%). ES-API: [M+H]⁺=474.2.

Step 2: The tert-butyl 2-(6-(6-amino-5-(pyrimidin-4-yl)pyridin-3-yl)isochroman-8-yl)pyrrolidine-1-carboxylate (57 mg, 0.120 mmol) was dissolved in dichloromethane (2 mL) and added to 1 mL of trifluoroacetic acid, and the system reacted at room temperature for 0.5 h. Upon completion of the reaction, the reaction mixture was concentrated. After neutralization with 7M ammonia/methanol solution (1 mL), the obtained mixture was concentrated again and purified by prep-HPLC (ammonia water method) to give 3-(pyrimidin-4-yl)-5-(8-(pyrrolidin-2-yl)isochroman-6-yl)pyridin-2-amine (Z100, 28 mg, yield: 63%). ES-API: [M+H]⁺=374.2.

Step 3: The 3-(pyrimidin-4-yl)-5-(8-(pyrrolidin-2-yl)isochroman-6-yl)pyridin-2-amine (Z100, 25 mg) was chirally resolved by SFC (column type: Chiralpak IC 250 mm*4.6 mm*5 um, mobile phase: HEX-ETOH-DEA = 40:60:2, flow rate: 1 ml/min, column temperature: 30°C, 30 MIN) to give two isomers. A structure of one isomer was arbitrarily assigned as (R)-3-(pyrimidin-4-yl)-5-(8-(pyrrolidin-2-yl)isochroman-6-yl)pyridin-2-amine (Z100-1, 8 mg, yield: 32%, retention time: 6.64 min, purity: 96%, ee value: 100%). ES-API:[M+H]⁺ = 374.2. ¹H NMR (500 MHz, DMSO-*d*₆) δ 9.25 (d, *J* = 1.3 Hz, 1H), 8.86 (d, *J* = 5.6 Hz, 1H), 8.44 (d, *J* = 2.3 Hz, 1H), 8.37 (d, *J* = 2.4 Hz, 1H), 8.25 (dd, *J* = 5.7, 1.5 Hz, 1H), 7.75 (s, 2H), 7.64 (d, *J* = 1.9 Hz, 1H), 7.35 (d, *J* = 1.9 Hz, 1H), 4.88 - 4.72 (m, 2H), 4.01 (t, *J* = 7.6 Hz, 1H), 3.93 - 3.83 (m, 2H), 3.70 - 3.47 (m, 1H), 3.10-3.06 (m, 1H), 2.90-2.86 (m, 3H), 2.14-2.10 (m, 1H), 1.87 - 1.70 (m, 2H), 1.44-1.40 (m, 1H); a structure of the other isomer was arbitrarily assigned as (S)-3-(pyrimidin-4-yl)-5-(8-(pyrrolidin-2-yl)isochroman-6-yl)pyridin-2-amine (Z100-2, 8 mg, yield: 32%, retention time: 10.79 min, purity: 97%, ee value: 99%). ES-API:[M+H]⁺ = 374.2. ¹H NMR (500 MHz, DMSO-*d*₆) δ 9.26 (d, *J* = 1.4 Hz, 1H), 8.86 (d, *J* = 5.6 Hz, 1H), 8.45 (d, *J* = 2.3 Hz, 1H), 8.38 (d, *J* = 2.3 Hz, 1H), 8.25 (dd, *J* = 5.7, 1.4 Hz, 1H), 7.81 - 7.71 (m, 2H), 7.65 (d, *J* = 2.0 Hz, 1H), 7.37 (d, *J* = 1.9 Hz, 1H), 4.88 - 4.73 (m, 2H), 4.05 (t, *J* = 7.7 Hz, 1H), 3.91 - 3.83 (m, 2H), 3.71 - 3.46 (m, 1H), 3.10-3.06 (m, 1H), 2.95 - 2.84 (m, 3H), 2.14-2.10 (m, 1H), 1.87 - 1.70 (m, 2H), 1.52 - 1.44 (m, 1H).

### Example 13. Synthesis of compound Z101 and isomers thereof

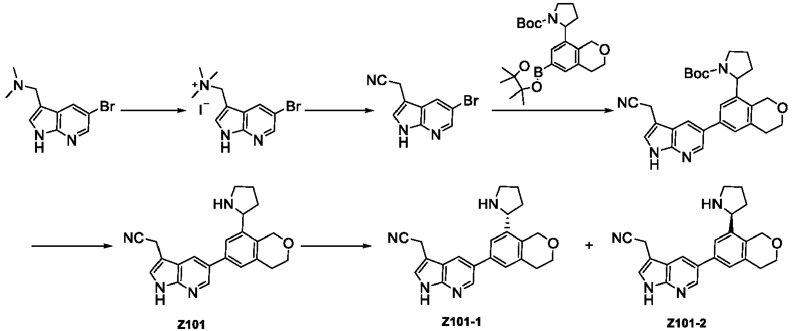

Step 1: 1-(5-Bromo-1H-pyrrolo[2,3-b]pyridin-3-yl)-N,N-dimethylmethanamine (1 g, 3.95 mmol), iodomethane (2.8 g, 19.7 mmol) were added to dichloromethane/toluene (40 ml/80 ml), and the system reacted at room temperature in a sealed tube for 20 h. Upon completion of the reaction, the reaction mixture was concentrated to give 1-(5-bromo-1H-pyrrolo[2,3-b]pyridin-3-yl)-N,N,N-trimethyl methanaminium (1.6 g, crude product). ES-API: [M+H]⁺=269.2.

Step 2: The 1-(5-bromo-1H-pyrrolo[2,3-b]pyridin-3-yl)-N,N,N-trimethyl methanaminium (1.0 g, 2.52 mmol) was dissolved in tetrahydrofuran (100 ml). TMSCN (554 mg, 5.59 mmol) and TBAF (11.2 ml, 11.2 mmol) were added successively, and the system reacted at room temperature for 2 h. Upon completion of the reaction, the reaction mixture was concentrated, diluted with ethyl acetate, washed with aqueous sodium carbonate solution and saturated brine successively, dried over anhydrous sodium sulfate, filtered, concentrated, and purified by silica gel (petroleum ether:ethyl acetate = 50:50) to give 2-(5-bromo-1H-pyrrolo[2,3-b]pyridin-3-yl)acetonitrile (480 mg, yield: 80%). ES-API: [M+H]⁺=236.1/238.1.

Step 3: The 2-(5-bromo-1H-pyrrolo[2,3-b]pyridin-3-yl)acetonitrile (50 mg, 0.211 mmol), tert-butyl 2-(6-(4,4,5,5-tetramethyl-1,3,2-dioxaborolan-2-yl)isochroman-8-yl)pyrrolidine-1-carboxyl ate (90.5 mg, 0.211 mmol), Pd(dppf)Cl₂ dichloromethane adduct (17.2 mg, 0.0211 mmol) and K₂CO₃ (87.3 mg, 0.633 mmol) were added to 1,4-dioxane (2 ml) and water (0.5 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified (petroleum ether:ethyl acetate = 10:90) to give tert-butyl 2-(6-(3-(cyanomethyl)-1H-pyrrolo[2,3-b]pyridin-5-yl)isochroman-8-yl)pyrrolidine-1-carb oxylate (45 mg, yield: 47%). ES-API: [M+H]⁺=459.2.

Step 4: Tert-butyl (6-(3-(cyanomethyl)-1H-pyrrolo[2,3-b]pyridin-5-yl)isochroman-8-yl)pyrrolidine-1-carbox ylate (45 mg, 0.098 mmol) was dissolved in dichloromethane (2 mL) and added to 1 mL of trifluoroacetic acid, and the system reacted at room temperature for 0.5 h. Upon completion of the reaction, the reaction mixture was concentrated. After neutralization with 7M ammonia/methanol solution (1 mL), the obtained mixture was concentrated again and purified by prep-HPLC (ammonia water method) to give 2-(5-(8-(pyrrolidin-2-yl)isochroman-6-yl)-1H-pyrrolo[2,3-b]pyridin-3-yl)acetonitrile (Z101, 22 mg, yield: 63%). ES-API: [M+H]⁺=359.2.

Step 5: The 2-(5-(8-(pyrrolidin-2-yl)isochroman-6-yl)-1H-pyrrolo[2,3-b]pyridin-3-yl)acetonitrile (Z101, 20 mg) was chirally resolved by SFC (column type: Chiralpak IC 250 mm*4.6 mm*5 um, mobile phase: HEX-ETOH-DEA = 50:50:2, flow rate: 1 ml/min, column temperature: 30°C, 30 MIN) to give two isomeric compounds. A structure of one isomeric compound was arbitrarily assigned as (*R*)-2-(5-(8-(pyrrolidin-2-yl)isochroman-6-yl)-1H-pyrrolo[2,3-b]pyridin-3-yl)acetonitrile (Z101-1, 8 mg, yield: 40%, retention time: 9.46 min, ee value: 100%). ES-API:[M+H]⁺ = 359.2. ¹H NMR (500 MHz, DMSO-*d*₆) δ 11.78 (s, 1H), 8.57 (d, *J* = 2.2 Hz, 1H), 8.29 - 8.25 (m, 2H), 7.74 (d, *J* = 1.9 Hz, 1H), 7.53 (d, *J* = 2.2 Hz, 1H), 7.39 (d, *J* = 1.9 Hz, 1H), 4.92 - 4.76 (m, 2H), 4.19 (t, *J* = 7.9 Hz, 1H), 4.13 (s, 2H), 3.93 - 3.85 (m, 2H), 3.18 (d, *J* = 7.2 Hz, 1H), 3.01 (q, *J* = 8.3 Hz, 1H), 2.90 (q, *J* = 6.5, 5.9 Hz, 2H), 2.36 (d, *J* = 1.9 Hz, 1H), 2.22-2.18 (m, 1H), 1.90-1.85 (m, 2H), 1.64-1.60 (m, 1H); the structure pf the other isomeric compound was arbitrarily assigned as (*S*)-2-(5-(8-(pyrrolidin-2-yl)isochroman-6-yl)-1H-pyrrolo[2,3-b]pyridin-3-yl)acetonitrile (Z101-2, 8 mg, yield: 40%, retention time: 13.04 min, ee value: 99.55%). ES-API:[M+H]⁺ = 359.2.

### Example 14. Synthesis of compound Z103

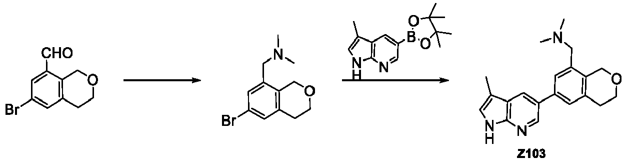

Step 1: 6-Bromoisochroman-8-carbaldehyde (355 mg, 1.47 mmol) was dissolved in 1,2-dichloroethane (10 mL). 2.0M Dimethylamine in tetrahydrofuran (2.2 mL, 4.40 mmol), acetic acid (265 mg, 4.41 mmol) and sodium triacetoxyborohydride (623 mg, 2.94 mmol) were added successively and stirred at room temperature for 4 h. Dichloromethane (50 mL) was added to the reaction mixture. The obtained mixture was washed with saturated sodium bicarbonate (20 mL×2) and saturated brine (20 mL) successively, dried, and concentrated. The crude product was purified by a flash silica gel column (methanol/dichloromethane: 0-5%) to give a desired product 1-(6-bromoisochroman-8-yl)-N,N-dimethylmethanamine (370 mg, yield: 93%) as a colorless liquid. ES-API: [M+H]⁺=270.1/272.1.

Step 2: The 1-(6-bromoisochroman-8-yl)-*N,N-*dimethylmethanamine (50 mg, 0.18 mmol), 3-methyl-5-(4,4,5,5-tetramethyl-1,3,2-dioxaborolan-2-yl)-1H-pyrrolo[2,3-b]pyridine (62 mg, 0.24 mmol), potassium carbonate (75 mg, 0.54 mmol), 2-dicyclohexylphosphine-2',6'-dimethoxy-biphenyl (8 mg, 0.018 mmol), chloro (2-dicyclohexylphosphino-2',6'-dimethoxy-1,1'-biphenylyl)(2'-amino-1,1'-biphenyl-2-yl) palladium (II) (13 mg, 0.018 mmol), 1,4-dioxane (2 mL) and water (0.5 mL) were added to a 5 mL microwave tube. The mixture was purged with nitrogen for 1 min, and the system reacted in a microwave reactor at 110°C for 1 h. Water (10 mL) was added to the reaction mixture, and the obtained mixture was extracted with 40 mL of ethyl acetate. The organic phase was washed with saturated sodium bicarbonate solution (15 mL) and saturated brine (15 mL) successively, dried, and concentrated. The crude product was purified by prep-HPLC (ammonium bicarbonate method) to give a desired product *N*,*N*-dimethyl-1-(6-(3-methyl-1H-pyrrolo[2,3-b]pyridin-5-yl)isochroman-8-yl)methanami ne (Z103, 32 mg, yield: 54%) as a white solid. ¹H NMR (500 MHz, DMSO-*d*₆) δ 11.32 (s, 1H), 8.46 (d, *J* = 1.5 Hz, 1H), 8.12 (d, *J* = 1.5 Hz, 1H), 7.43 - 7.38 (m, 2H), 7.25 (s, 1H), 4.84 (s, 2H), 3.88 (t, *J* = 5.5 Hz, 2H), 3.33 (s, 2H), 2.89 (t, *J* = 5.5 Hz, 2H), 2.30 (s, 3H), 2.16 (s, 6H). ES-API: [M+H]⁺=322.1.

### Example 15. Synthesis of compound Z104 and isomers thereof

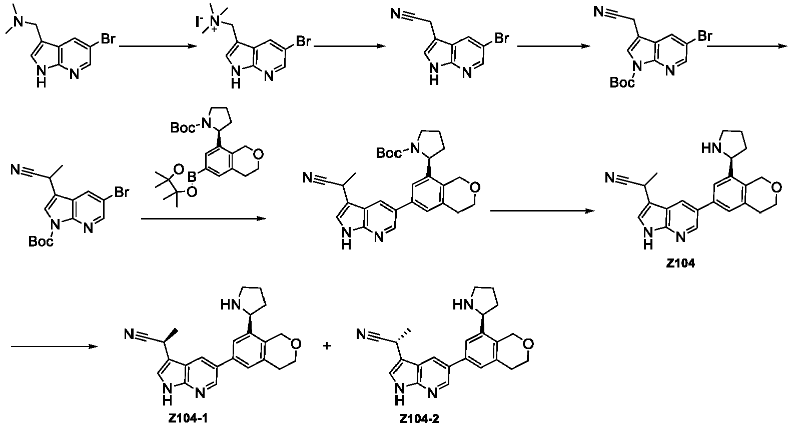

Step 1: 1-(5-Bromo-1H-pyrrolo[2,3-b]pyridin-3-yl)-N,N-dimethylmethanamine (1.0 g, 3.95 mmol) and iodomethane (2.8 g, 19.75 mmol) were added to a mixed solution of 40 ml of dichloromethane and 80 ml of toluene and stirred at room temperature for 20 h. Upon completion of the reaction, the reaction mixture was concentrated to give 1-(5-bromo-1H-pyrrolo[2,3-b]pyridin-3-yl)-N,N,N-trimethyl methanaminium iodide (1.5 g, crude product) which was directly used in the next step. ES-API: [M+H]⁺=268.0/270.0.

Step 2: The 1-(5-bromo-1H-pyrrolo[2,3-b]pyridin-3-yl)-N,N,N-trimethyl methanaminium iodide (1.5 g, 3.93 mmol), trimethylsilyl cyanide (503 mg, 5.08 mmol) and tetrabutylammonium fluoride (11.8 ml, 11.8 mmol, 1M THF solution) were added to 50 ml of tetrahydrofuran and stirred at room temperature for 2 h. Upon completion of the reaction, the reaction mixture was diluted with ethyl acetate, washed with water and saturated brine successively, dried over anhydrous sodium sulfate, filtered, concentrated, and purified by silica gel (petroleum ether:ethyl acetate = 80:20) to give 2-(5-bromo-1H-pyrrolo[2,3-b]pyridin-3-yl)acetonitrile (520 mg, yield: 55%). ES-API: [M+H]⁺=236.0/238.0.

Step 3: The 2-(5-bromo-1H-pyrrolo[2,3-b]pyridin-3-yl)acetonitrile (150mg, 0.638 mmol) was dissolved in dichloromethane (10 ml). (Boc)₂O (209 mg, 0.957 mmol), diisopropylethyl amine (164 mg, 1.276 mmol) and DMAP (7.6 mg, 0.0638 mmol) were added successively, and the system reacted at room temperature for 1 h. Upon completion of the reaction, the reaction mixture was concentrated, diluted with ethyl acetate, washed with water and saturated brine successively, dried over anhydrous sodium sulfate, filtered, concentrated, and purified by silica gel (petroleum ether:ethyl acetate = 80:20) to give tert-butyl 5-bromo-3-(cyanomethyl)-1H-pyrrolo[2,3-b]pyridine-1-carboxylate (205 mg, yield: 95%). ES-API: [M+H]⁺=337.2/339.2.

Step 4: The tert-butyl 5-bromo-3-(cyanomethyl)-1H-pyrrolo[2,3-b]pyridine-1-carboxylate (80 mg, 0.238 mmol) was dissolved in dried N,N-dimethylformamide (5 ml), and the system was cooled in an ice-water bath. 60% NaH (28.5 mg, 0.714 mmol) and iodomethane (34 mg, 0.238 mmol) were added successively, and the system reacted for 0.5 h. Upon completion of the reaction, the reaction was quenched with ice water. The reaction mixture was diluted with ethyl acetate, washed with water and saturated brine successively, dried over anhydrous sodium sulfate, filtered, concentrated, and purified by silica gel (petroleum ether:ethyl acetate = 80:20) to give tert-butyl 5-bromo-3-(1-cyanoethyl)-1H-pyrrolo[2,3-b]pyridine-1-carboxylate (35 mg, yield: 42%). ES-API: [M+H]⁺=350.0/252.0.

Step 5: The tert-butyl 5-bromo-3-(1-cyanoethyl)-1H-pyrrolo[2,3-b]pyridine-1-carboxylate (35 mg, 0.10 mmol), tert-butyl (S)-2-(6-(4,4,5,5-tetramethyl-1,3,2-dioxaborolan-2-yl)isochroman-8-yl)pyrrolidine-1-carb oxylate (43 mg, 0.10 mmol), Sphos Pd G2 (7.2 mg, 0.01 mmol) and K₂CO₃ (41.4 mg, 0.3 mmol) were added to 1,4-dioxane (2 ml) and water (0.5 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified (petroleum ether:ethyl acetate = 10:90) to give (2S) tert-butyl 2-(6-(3-(1-cyanoethyl)-1H-pyrrolo[2,3-b]pyridin-5-yl)isochroman-8-yl)pyrrolidine-1-carb oxylate (35 mg, yield: 74%). ES-API: [M+H]⁺=473.2.

Step 6: The (25) tert-butyl 2-(6--(3-(1-cyanoethyl)-1H-pyrrolo[2,3-b]pyridin-5-yl)isochroman-8-yl)pyrrolidine-1-car boxylate (35 mg, 0.074 mmol) was dissolved in dichloromethane (2 mL) and added to 1 mL of trifluoroacetic acid, and the system reacted at room temperature for 0.5 h. Upon completion of the reaction, the reaction mixture was concentrated. After neutralization with 7M ammonia/methanol solution (1 mL), the obtained mixture was concentrated again and purified by prep-HPLC (ammonia water method) to give 2-(5-(8-((S)-pyrrolidin-2-yl)isochroman-6-yl)-1H-pyrrolo[2,3-b]pyridin-3-yl)propanenitril e (Z104, 11 mg, yield: 48%). ES-API: [M+H]⁺=373.2.

Step 7: The 2-(5-(8-((S)-pyrrolidinyl-2-yl)isochroman-6-yl)-1H-pyrrolo[2,3-b]pyridin-3-yl)propanenit rile (Z104, 8 mg) was chirally resolved by SFC (column type: Chiral pak IC 250 mm*4.6 mm*5 um, mobile phase: ACN-IPA-DEA = 80-20-02, flow rate: 1 ml/min, column temperature: 30°C, 35 min) to give two isomeric compounds. A structure of one isomeric compound was arbitrarily assigned as (*S*)-2-(5-(8-((*S*)-pyrrolidin-2-yl)isochroman-6-yl)-1H-pyrrolo[2,3-b]pyridin-3-yl)propanen itrile (Z104-1, 1.5 mg, yield: 18.7%, retention time: 8.36 min, purity: 75%, ee value: 95%). ES-API:[M+H]⁺ =373.2. A structure of the other isomer was arbitrarily assigned as (*R*)-2-(5-(8-((*S*)-pyrrolidin-2-yl)isochroman-6-yl)-1H-pyrrolo[2,3-b]pyridin-3-yl)propanen itrile (Z104-2, 2.9 mg, yield: 36.2%, retention time: 10.379 min, purity: 86%, ee value: 96%). ES-API:[M+H]⁺ =373.2.

### Example 16. Synthesis of compound Z105

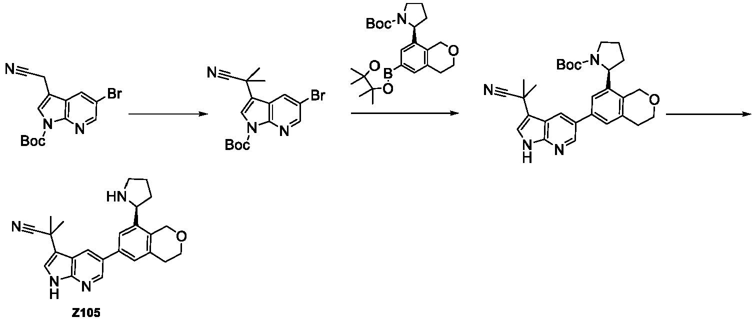

Step 1: Tert-butyl 5-bromo-3-(cyanomethyl)-1H-pyrrolo[2,3-b]pyridine-1-carboxylate (80 mg, 0.238 mmol) was dissolved in dried N,N-dimethylformamide (5 ml), and the system was cooled in an ice-water bath. 60% NaH (28.5 mg, 0.714 mmol) and iodomethane (168 mg, 1.19 mmol) were added successively, and the system reacted under the ice-water bath for 0.5 h. Upon completion of the reaction, the reaction was quenched with ice water. The reaction mixture was diluted with ethyl acetate, washed with water and saturated brine successively, dried over anhydrous sodium sulfate, filtered, concentrated, and purified by silica gel (petroleum ether:ethyl acetate = 80:20) to give tert-butyl 5-bromo-3-(2-cyanopropan-2-yl)-1H-pyrrolo[2,3-b]pyridine-1-carboxylate (70 mg, yield: 81%). ES-API: [M+H]⁺=364.0/366.0.

Step 2: The tert-butyl 5-bromo-3-(2-cyanopropan-2-yl)-1H-pyrrolo[2,3-b]pyridine-1-carboxylate (70 mg, 0.192 mmol), tert-butyl (S)-2-(6-(4,4,5,5-tetramethyl-1,3,2-dioxaborolan-2-yl)isochroman-8-yl)pyrrolidine-1-carb oxylate (90.6 mg, 0.211 mmol), Sphos Pd G2 (13.8 mg, 0.0192 mmol) and K₂CO₃ (0.576 mg, 79.5 mmol) was added to 1,4-dioxane (2 ml) and water (0.5 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified (petroleum ether:ethyl acetate = 10:90) to give tert-butyl (2S)-2-(6-(3-(2-cyanopropan-2-yl)-1H-pyrrolo[2,3-b]pyridin-5-yl)isochroman-8-yl)pyrroli dine-1-carboxylate (35 mg, yield: 37.5%). ES-API: [M+H]⁺=487.2.

Step 3: The tert-butyl (2S)-2-(6-(3-(2-cyanopropan-2-yl)-1H-pyrrolo[2,3-b]pyridin-5-yl)isochroman-8-yl)pyrroli dine-1-carboxylate (35 mg, 0.072 mmol) was dissolved in dichloromethane (2 mL) and added to 1 mL of trifluoroacetic acid, and the system reacted at room temperature for 0.5 h. Upon completion of the reaction, the reaction mixture was concentrated. After neutralization with 7M ammonia/methanol solution (1 mL), the obtained mixture was concentrated again and purified by prep-HPLC (ammonia water method) to give (S)-2-methyl-2-(5-(8-(pyrrolidin-2-yl)isochroman-6-yl)-1H-pyrrolo[2,3-b]pyridin-3-yl)pro panenitrile (Z105, 11 mg, yield: 31%). ES-API: [M+H]⁺=387.2.

### Example 17. Synthesis of compound Z106

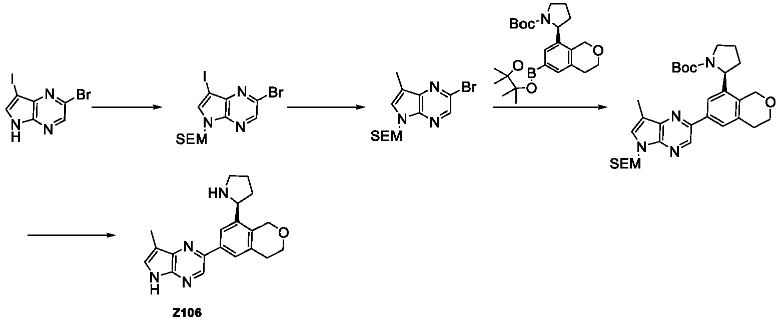

Step 1: 2-Bromo-7-iodo-5H-pyrrolo[2,3-b]pyrazine (1 g, 3.1 mmol) was dissolved in N,N-dimethylformamide (20 ml). 60% NaH (620 mg, 15.5 mmol) and 2-(trimethylsilyl)ethoxymethyl chloride (774 mg, 4.64 mmol) were added successively, and the system reacted at room temperature for 1 h. Upon completion of the reaction, the reaction was quenched with aqueous ammonium chloride solution. The reaction mixture was diluted with ethyl acetate, wash with water and saturated brine successively, dried over anhydrous sodium sulfate, filtered, concentrated, and purified by silica gel (petroleum ether:ethyl acetate = 80:20) to give 2-bromo-7-iodo-5-((2-(trimethylsilyl)ethoxy)methyl)-5H-pyrrolo[2,3-b]pyrazine (1.2 g, yield: 85.7%). ES-API: [M+H]⁺=453.9/455.9.

Step 2: The 2-bromo-7-iodo-5-((2-(trimethylsilyl)ethoxy)methyl)-5H-pyrrolo[2,3-b]pyrazine (900 mg, 1.99 mmol) and bis(tri-tert-butylphosphine)palladium (204 mg, 0.4 mmol) were dissolved in dried tetrahydrofuran (5 ml). Dimethylzinc (2 ml, 2.0 mmol, 1M in n-hexane) was slowly added under an ice-water bath condition, and the system reacted in the ice-water bath for 0.5 h. Upon completion of the reaction, the reaction was quenched with saturated aqueous ammonium chloride solution. The reaction mixture was diluted with ethyl acetate, washed with water and saturated brine successively, dried over anhydrous sodium sulfate, filtered, concentrated, and purified by silica gel (petroleum ether:ethyl acetate = 80:20) to give 2-bromo-7-methyl-5-((2-(trimethylsilyl)ethoxy)methyl)-5H-pyrrolo[2,3-b]pyrazine (70 mg, yield: 10.2%). ES-API: [M+H]⁺=342.0/244.0.

Step 3: The 2-bromo-7-methyl-5-((2-(trimethylsilyl)ethoxy)methyl)-5H-pyrrolo[2,3-b]pyrazine (34 mg, 0.10 mmol), tert-butyl (S)-2-(6-(4,4,5,5-tetramethyl-1,3,2-dioxaborolan-2-yl)isochroman-8-yl)pyrrolidine-1-carb oxylate (43 mg, 0.10 mmol), Sphos Pd G2 (7.2 mg, 0.01 mmol) and K₂CO₃ (41.4 mg, 0.3 mmol) were added to 1,4-dioxane (2 ml) and water (0.5 ml). The system was replaced with nitrogen and reacted under microwave at 110°C for 1 h. Upon completion of the reaction, ethyl acetate was added. The obtained mixture was washed with water and saturated brine successively, dried over anhydrous sodium sulfate, and purified (petroleum ether:ethyl acetate = 10:90) to give tert-butyl (S)-2-(6-(7-methyl-5-((2-(trimethylsilyl)ethoxy)methyl)-5H-pyrrolo[2,3-b]pyrazin-2-yl)is ochroman 8-yl)pyrrolidine-1-carboxylate (20 mg, yield: 36%). ES-API: [M+H]⁺=565.3.

Step 4: The tert-butyl (S)-2-(6-(7-methyl-5-((2-(trimethylsilyl)ethoxy)methyl)-5H-pyrrolo[2,3-b]pyrazin-2-yl)is ochroman 8-yl)pyrrolidine-1-carboxylate (20 mg, 0.035) was dissolved in dichloromethane (2 mL) and added to 1 mL of trifluoroacetic acid, and the system reacted at room temperature for 0.5 h. Upon completion of the reaction, the reaction mixture was concentrated. 7M Ammonia/methanol solution (5 mL) was added and stirred at room temperature for 3 h. The obtained mixture was concentrated again and purified by prep-HPLC (ammonia water method) to give (S)-7-methyl-2-(8-(pyrrolidin-2-yl)isochroman-6-yl)-5H-pyrrolo[2,3-b]pyrazine (Z106, 2.9 mg, yield: 24.7%). ES-API: [M+H]⁺=335.2.

### Example 18. Synthesis of compound Z107

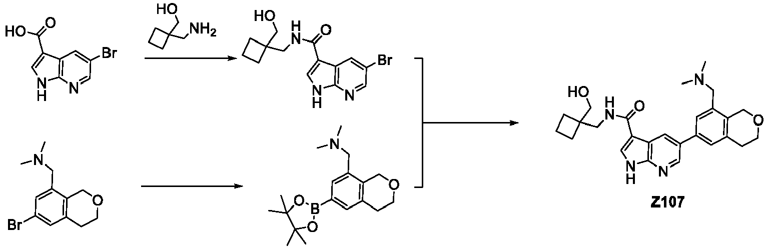

Step 1: 5-Bromo-1*H*-pyrrolo[2,3-b]pyridine-3-carboxylic acid (300 mg, 1.24 mmol), 1-ethyl-3(3-dimethylpropylamine)carbodiimide (476 mg, 2.48 mmol) and 1-hydroxybenzotriazole (335 mg, 2.48 mmol) were dissolved in dichloromethane (10 mL) and dimethylsulfoxide (0.7 mL). *N*,*N*-diisopropylethyl amine (480 mg, 3.72 mmol) and (1-(aminomethyl)cyclobutyl)methanol (157 mg, 1.37 mmol) were added successively and stirred at room temperature for 16 h. Saturated sodium carbonate (8 mL) was added to the reaction mixture, and the obtained mixture was extracted with ethyl acetate (60 mL×2). The organic phase was dried and concentrated. The crude product was purified by a flash silica gel column (methanol/dichloromethane: 2-10%) to give a desired product 5-bromo-*N*-(((1-(hydroxymethyl)cyclobutyl)methyl)-1*H*-pyrrolo[2,3-b]pyridine-3-carboxa mide (265 mg, yield: 63%) as a white solid. ES-API: [M+H]⁺=338.1/340.1.

Step 2: 1-(6-bromoisochroman-8-yl)-N,N-dimethylmethanamine (80 mg, 0.30 mmol), bis(pinacolato)diboron (91 mg, 0.36 mmol), [1,1'-bis(diphenylphosphino)ferrocene]dichloropalladium (22 mg, 0.03 mmol), potassium acetate (88 mg, 0.90 mmol), and 1,4-dioxane (2 mL) were added to a 5 mL microwave tube. The mixture was purged with nitrogen for 1 min, and the system reacted in a microwave reactor at 120°C for 30 min. The reaction was cooled to room temperature. The reaction mixture was dissolved with dichloromethane (5 mL), filtered with diatomaceous earth, and washed with ethyl acetate. The obtained filtrate was concentrated to give crude *N,N*-dimethyl-1-(6-(4,4,5,5-tetramethyl-1,3,2-dioxaborolan-2-yl)isochroman-8-yl)methana mine (165 mg) as a pale brown crude product which was used without further purification. ES-API:[M+H]⁺=318.3.

Step 3: The 5-bromo-N-(((1-(hydroxymethyl)cyclobutyl)methyl)-1*H*-pyrrolo[2,3-b]pyridine-3-carboxa mide (60 mg, 0.18 mmol), the N,N-dimethyl-1-(6-(4,4,5,5-tetramethyl-1,3,2-dioxaborolan-2-yl)isochroman-8-yl)methan amine (165 mg, crude product), potassium carbonate (75 mg, 0.54 mmol), 2-dicyclohexylphosphine-2',6'-dimethoxy-biphenyl (8 mg, 0.018 mmol), chloro (2-dicyclohexylphosphino-2',6'-dimethoxy-1,1'-biphenylyl)(2'-amino-1,1'-biphenyl-2-yl) palladium (II) (13 mg, 0.018 mmol), 1,4-dioxane (2 mL) and water (0.4 mL) were added to a 5 mL microwave tube. The mixture was purged with nitrogen for 1 min, and the system reacted in a microwave reactor at 110°C for 1 h. Water (5 mL) was added to the reaction mixture, and the obtained mixture was extracted with ethyl acetate (60 mL). The organic phase was washed with saturated brine (15 mL), dried, and concentrated. The crude product was purified by prep-HPLC (ammonium bicarbonate method) to give a desired product 5-(8-((dimethylamino)methyl)isochroman-6-yl)-*N*-((1-(hydroxymethyl)cyclobutyl)methyl )-1*H*-pyrrolo[2,3-b]pyridine-3-carboxamide (Z107, 15 mg, yield: 18.5%) as a white solid. ¹H NMR (500 MHz, DMSO-*d*₆) δ 12.19 (s, 1H), 8.61 (d, *J* = 2.0 Hz, 1H), 8.55 (d, *J* = 2.0 Hz, 1H), 8.23 (d, *J* = 3.0 Hz, 1H), 8.07 (t, *J* = 6.0 Hz, 1H), 7.39 (s, 2H), 4.84 (s, 2H), 4.72 (t, *J* = 5.5 Hz, 1H), 3.88 (t, *J* = 5.5 Hz, 2H), 3.41 - 3.34 (m, 6H), 2.90 (t, *J* = 5.5 Hz, 2H), 2.16 (s, 6H), 1.88 - 1.66 (m, 6H). ES-API:[M+H]⁺=449.2.

### Example 19. Synthesis of compound Z136

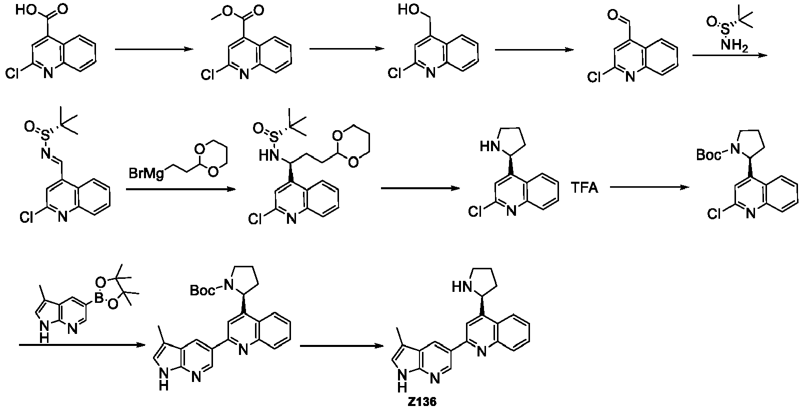

Step 1: 2-Chloroquinoline-4-carboxylic acid (3.0 g, 14.49 mmol) was suspended in dichloromethane (25 mL). Oxalyl chloride (3.68 g, 28.98 mmol) and *N,N*-dimethylformamide (0.1 mL) were added at 0°C and stirred at room temperature for 2 h. After the system was cooled to 0°C, methanol (20 mL) was slowly added dropwise and stirred at room temperature for 1 h. Water (40 mL) was added to the reaction mixture, and the obtained mixture was extracted with dichloromethane (80 mL×2). The organic phases were combined, washed with saturated brine (30 mL), dried over anhydrous sodium sulfate, and concentrated to give methyl 2-chloroquinoline-4-carboxylate (2.95 g, yield: 92%) as a pale brown solid. ES-API: [M+H]⁺=222.1.

Step 2: The methyl 2-chloroquinoline-4-carboxylate (2.95 g, 13.35 mmol) was dissolved in methanol (50 mL). Sodium borohydride (1.52 g, 40.05 mmol) was added in batches at 0°C and stirred at room temperature for 18 h. Saturated ammonium chloride solution (40 mL) and water (20 mL) were added to the reaction mixture, and methanol was removed by rotation. The obtained mixture was extracted with dichloromethane (100 mL×3). The organic phase was dried over anhydrous sodium sulfate, and concentrated. The crude product was purified by a flash silica gel column (methanol/dichloromethane: 1-5%) to give (2-chloroquinolin-4-yl)methanol (1.65 g, yield: 64%) as an off-white solid. ES-API: [M+H]⁺=194.1.

Step 3: The (2-chloroquinolin-4-yl)methanol (1.55 g, 8.03 mmol) was dissolved in dichloromethane (50 mL), and the system was cooled to 0°C. Dess-Martin periodinane (4.08 g, 9.64 mmol) was added and stirred at room temperature for 2 h. A saturated sodium thiosulfate solution (60 mL) was added to the reaction mixture, and the obtained mixture was extracted with dichloromethane (60 mL×2). The organic phase was washed with saturated sodium bicarbonate solution (60 mL), dried over anhydrous sodium sulfate, and concentrated. The crude product was purified by a flash silica gel column (ethyl acetate/petroleum ether: 0-30%) to give 2-chloroquinoline-4-carbaldehyde (1.45 g, yield: 94%) as a white solid. ES-API: [M+H]⁺=192.1.

Step 4: The 2-chloroquinoline-4-carbaldehyde (1.0 g, 5.23 mmol) and (S)-2-methylpropane-2-sulfinamide (1.26 g, 10.46 mmol) were dissolved in dichloromethane (25 mL). Tetraethoxytitanium (2.98 g, 13.08 mmol) was added and stirred at room temperature for 18 h. Saturated brine (100 mL) was added to the reaction mixture, and the obtained mixture was extracted with ethyl acetate (80 mL× 2). The organic phases were combined, washed with saturated brine (50 mL), dried over anhydrous sodium sulfate, and concentrated. The crude product was purified by a flash silica gel column (methanol/dichloromethane: 0-5%) to give (*S,E*)-*N*-((2-chloroquinolin-4-yl)methylene)-2-methylpropane-2-sulfinamide (1.5 g, yield: 97%) as a white solid. ES-API: [M+H]⁺=295.1.

Step 5: The (*S,E*)-*N*-((2-chloroquinolin-4-yl)methylene)-2-methylpropane-2-sulfinamide (1.35 g, 4.59 mmol) was dissolved in tetrahydrofuran (20 mL). In the presence of protective nitrogen, (2-(1,3-dioxan-2-yl)ethyl)magnesium bromide in tetrahydrofuran (27.5 mL, 13.77 mmol, 0.5M) was added dropwise at -78°C and stirred at -78°C for 30 min. The reaction was quenched with saturated ammonium chloride solution (20 mL), and water (20 mL) was added. The obtained mixture was extracted with ethyl acetate (50 mL×2). The organic phases were combined, washed with saturated brine (25 mL), dried over anhydrous sodium sulfate, and concentrated to give (*S*)-*N*-((*S*)-1-(2-chloroquinolin-4-yl)-3-(1,3-dioxan-2-yl)propyl)-2-methylpropane-2-sulfin amide (1.88 g, yield: 100%) as a white solid. ES-API: [M+H]⁺=411.2.

Step 6: Trifluoroacetic acid (20 mL) and water (1 mL) were cooled to 0°C. The (*S*)-*N*-((*S*)-1-(2-chloroquinolin-4-yl)-3-(1,3-dioxan-2-yl)propyl)-2-methylpropane-2-sulfin amide (1.88 g, 4.59 mmol) was added in batches and stirred at room temperature for 45 min. Triethylsilane (5.32 g, 45.90 mmol) was added and stirred at room temperature for 16 h. The reaction mixture was concentrated to give (S)-2-chloro-4-(pyrrolidin-2-yl)quinoline trifluoroacetate (4.1 g, crude product) which was directly used in the next step without further purification. ES-API: [M+H]⁺=233.1 (free base).

Step 7: The (S)-2-chloro-4-(pyrrolidin-2-yl)quinoline trifluoroacetate (4.1 g, crude product) was dissolved in dichloromethane (40 mL). Triethylamine (1.62 g, 16.0 mmol) and di-tert-butyl dicarbonate (1.74 g, 8.0 mmol) were added at 0°C and stirred at room temperature for 1 h. Dichloromethane (40 mL) was added to the reaction mixture. The obtained mixture was washed with water (25 mL) and saturated brine (25 mL) successively, dried over anhydrous sodium sulfate, and concentrated. The crude product was purified by a flash silica gel column (ethyl acetate/petroleum ether: 0-30%) to give tert-butyl (S)-2-(2-chloroquinolin-4-yl)pyrrolidine-1-carboxylate (1.15 g, yield of the 2 steps: 75%) as a viscous liquid. ES-API: [M+H]⁺=333.2.

Step 8: The tert-butyl (S)-2-(2-chloroquinolin-4-yl)pyrrolidine-1-carboxylate (60 mg, 0.23 mmol), 3-methyl-5-(4,4,5,5-tetramethyl-1,3,2-dioxaborolan-2-yl)-1H-pyrrolo[2,3-*b*]pyridine (76 mg, 0.23 mmol), potassium carbonate (95 mg, 0.69 mmol), 2-dicyclohexylphosphine-2',6'-dimethoxy-biphenyl (9 mg, 0.023 mmol), chloro (2-dicyclohexylphosphino-2',6'-dimethoxy-1,1'-biphenylyl)(2'-amino-1,1'-biphenyl-2-yl) palladium (II) (17 mg, 0.023 mmol), 1,4-dioxane (2 mL) and water (0.4 mL) were added to a 5 mL microwave tube. The mixture was purged with nitrogen for 1 min, and the system reacted in a microwave reactor at 110°C for 1 h. Water (5 mL) was added to the reaction mixture, and the obtained mixture was extracted with ethyl acetate (40 mL). The organic phase was washed with saturated brine (10 mL), dried over anhydrous sodium sulfate, and concentrated. The crude product was purified by a flash silica gel column (methanol/dichloromethane: 0-3%) to give tert-butyl (S)-2-(2-(3-methyl-1H-pyrrolo[2,3-b]pyridin-5-yl)quinolin-4-yl)pyrrolidine-1-carboxylate (42 mg, yield: 42%) as a white solid. ES-API: [M+H]⁺=429.3.

Step 9: The tert-butyl (S)-2-(2-(3-methyl-1H-pyrrolo[2,3-b]pyridin-5-yl)quinolin-4-yl)pyrrolidine-1-carboxylate (42 mg, 0.10 mmol) was dissolved in methanol (1 ml). 4M dioxane hydrochloride solution (3 ml) was added and stirred at room temperature for 1 h. The reaction mixture was concentrated. 7.0M Ammonia/methanol solution (4 mL) was added, and a solvent was spun to dryness. The crude product was purified by prep-HPLC (ammonium bicarbonate method) to give (*S*)-2-(3-methyl-1H-pyrrolo[2,3-*b*]pyridin-5-yl)-4-(pyrrolidin-2-yl)quinoline (Z136, 20 mg, yield: 62%) as a white solid. ES-API: [M+H]⁺=329.1. ¹H NMR (500 MHz, DMSO-*d*₆) δ 11.49 (s, 1H), 9.09 (d, *J* = 2.0 Hz, 1H), 8.68 (d, *J* = 1.5 Hz, 1H), 8.34 (s, 1H), 8.19 (d, *J* = 8.5 Hz, 1H), 8.10 (d, *J* = 8.5 Hz, 1H), 7.77 - 7.72 (m, 1H), 7.60 - 7.54 (m, 1H), 7.31 (s, 1H), 4.88 (t, *J* = 7.5 Hz, 1H), 3.22 - 3.14 (m, 1H), 3.10 - 3.04 (m, 1H), 2.50-2.45 (m, 1H), 2.37 (s, 3H), 1.88 - 1.78 (m, 2H), 1.60 - 1.50 (m, 1H).

### Example 20. Synthesis of compound Z109

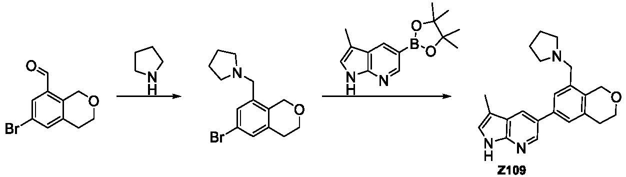

Step 1: Compound tetrahydropyrrole (147 mg, 2.07 mmol) and acetic acid (13 mg, 0.21 mmol) were added to a solution of 6-bromoisochroman-8-carbaldehyde (100 mg, 0.41 mmol) in 1,2-dichloroethane (2 mL), and the mixture was stirred at room temperature for 1 h. Sodium cyanoborohydride (52 mg, 0.83 mmol) was then added and stirred at room temperature overnight. Upon completion of the reaction, the reaction was quenched with aqueous ammonium chloride solution (5 mL), and the reaction mixture was extracted with dichloromethane (5 mLX3). The organic phases were combined, dried over anhydrous sodium sulfate, filtered, and concentrated. The crude product was purified by a flash silica gel column (0-6% methanol (1% ammonia water)/dichloromethane) to give 1-((6-bromoisochroman-8-yl)methyl)pyrrolidine (90 mg, yield: 74%) as a colorless liquid. ES-API: [M+H]⁺ = 296.0, 298.0.

Step 2: In the presence of protective nitrogen, a mixed solution of 3-methyl-5-(4,4,5,5-tetramethyl-1,3,2-dioxaborolan-2-yl)-1H-pyrrolo[2,3-b]pyridine (55 mg, 0.21 mmol), the 1-((6-bromoisochroman-8-yl)methyl)pyrrolidine (80 mg, 0.27 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol), 2-dicyclohexylphosphine-2',6'-dimethoxybiphenyl (8 mg, 0.02 mmol) and potassium carbonate (87 mg, 0.63 mmol) in 1,4-dioxane (2 mL) and water (0.4 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate method) to give 3-methyl-5-(8-(pyrrolidin-1-yl methyl)isochroman-6-yl)-1*H*-pyrrolo[2,3-*b*]pyridine (Z109, 30 mg, yield: 41%) as a white solid. ES-API: [M+H]⁺ = 348.3. ¹H NMR (500 MHz, DMSO-*d*₆) δ 11.33 (s, 1H), 8.46 (d, *J* = 2.0 Hz, 1H), 8.11 (d, *J* = 1.6 Hz, 1H), 7.43 (s, 1H), 7.40 (s, 1H), 7.26 (s, 1H), 4.85 (s, 2H), 3.88 (t, *J* = 5.5 Hz, 2H), 2.88 (t, *J* = 5.5 Hz, 2H), 2.45 (s, 4H), 2.31 (s, 3H), 1.69 (s, 4H).

### Example 21. Synthesis of compound Z114

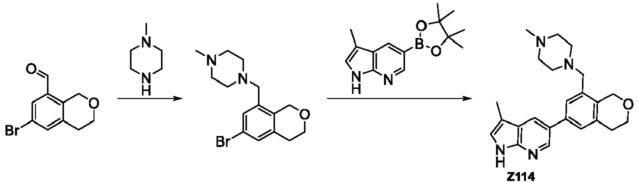

Step 1: Compound N-methylpiperazine (207 mg, 2.07 mmol) and acetic acid (13 mg, 0.21 mmol) were added to a solution of 6-bromoisochroman-8-carbaldehyde (100 mg, 0.41 mmol) in 1,2-dichloroethane (2 mL), and the mixture was stirred at room temperature for 1 h. Sodium cyanoborohydride (52 mg, 0.83 mmol) was then added and stirred at room temperature overnight. Upon completion of the reaction, the reaction was quenched with aqueous ammonium chloride solution (5 mL), and the reaction mixture was extracted with dichloromethane (5 mLX3). The organic phases were combined, dried over anhydrous sodium sulfate, filtered, and concentrated. The crude product was purified by a flash silica gel column (0-6% methanol (1% ammonia water)/dichloromethane) to give 1-((6-bromoisochroman-8-yl)methyl)-4-methylpiperazine (100 mg, yield: 75%) as a colorless liquid. ES-API: [M+H]⁺ = 325.1, 327.1.

Step 2: In the presence of protective nitrogen, a mixed solution of 3-methyl-5-(4,4,5,5-tetramethyl-1,3,2-dioxaborolan-2-yl)-1H-pyrrolo[2,3-b]pyridine (55 mg, 0.21 mmol), the 1-((6-bromoisochroman-8-yl)methyl)-4-methylpiperazine (88 mg, 0.27 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol), 2-dicyclohexylphosphine-2',6'-dimethoxybiphenyl (8 mg, 0.02 mmol) and potassium carbonate (87 mg, 0.63 mmol) in 1,4-dioxane (2 mL) and water (0.4 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate method) to give 3-methyl-5-(8-((4-methylpiperazin-1-yl)methyl)isochroman-6-yl)-1*H*-pyrrolo[2,3-b]pyridi ne (Z114, 31 mg, yield: 39%) as a white solid. ES-API: [M+H]⁺ = 377.4. ¹H NMR (500 MHz, DMSO-d₆) δ 11.33 (s, 1H), 8.46 (s, 1H), 8.12 (s, 1H), 7.40 (s, 2H), 7.26 (s, 1H), 4.85 (s, 2H), 3.88 (t, *J* = 4.5, 2H), 3.42 (s, 2H), 3.34 (s, 4H), 2.89 (t, *J* = 5.5, 2H), 2.37 (s, 4H), 2.31 (s, 3H), 2.14 (s, 3H).

### Example 22. Synthesis of compound Z115

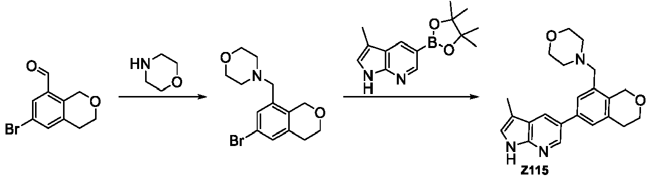

Step 1: Morpholine (180 mg, 2.07 mmol) and acetic acid (13 mg, 0.21 mmol) were added to a solution of 6-bromoisochroman-8-carbaldehyde (100 mg, 0.41 mmol) in 1,2-dichloroethane (2 mL), and the mixture was stirred at room temperature for 1 h. Sodium cyanoborohydride (52 mg, 0.83 mmol) was then added and stirred at room temperature overnight. Upon completion of the reaction, the reaction was quenched with aqueous ammonium chloride solution (5 mL), and the reaction mixture was extracted with dichloromethane (5 mLX3). The organic phases were combined, dried over anhydrous sodium sulfate, filtered, and concentrated. The crude product was purified by a flash silica gel column (0-6% methanol (1% ammonia water)/dichloromethane) to give 4-((6-bromoisochroman-8-yl)methyl)morpholine (90 mg, yield: 70%) as a colorless liquid. ES-API: [M+H]⁺ = 312.0, 314.0.

Step 2: In the presence of protective nitrogen, a mixed solution of 3-methyl-5-(4,4,5,5-tetramethyl-1,3,2-dioxaborolan-2-yl)-1H-pyrrolo[2,3-b]pyridine (55 mg, 0.21 mmol), the 4-((6-bromoisochroman-8-yl)methyl)morpholine (80 mg, 0.27 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol), 2-dicyclohexylphosphine-2',6'-dimethoxybiphenyl (8 mg, 0.02 mmol) and potassium carbonate (87 mg, 0.63 mmol) in 1,4-dioxane (2 mL) and water (0.4 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate method) to give 4-((6-(3-methyl-1*H*-pyrrolo[2,3-*b*]pyridin-5-yl)isochroman-8-yl)methyl)morpholine (Z115, 41 mg, yield: 52%) as a white solid. ES-API: [M+H]⁺ = 364.3. ¹H NMR (500 MHz, DMSO-d₆) δ 11.33 (s, 1H), 8.47 (d, *J* = 2.0 Hz, 1H), 8.12 (d, *J* = 2.0 Hz, 1H), 7.42 (s, 2H), 7.26 (s, 1H), 4.87 (s, 2H), 3.89 (t, *J* = 5.5 Hz, 2H), 3.55 (s, 4H), 3.43 (s, 2H), 2.89 (t, *J* = 5.5 Hz, 2H), 2.37 (s, 4H), 2.31 (s, 3H).

### Example 23. Synthesis of compound Z111

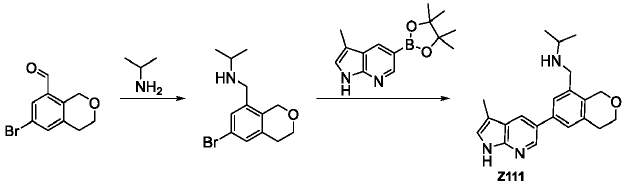

Step 1: Compound isopropylamine (122 mg, 2.07 mmol) and acetic acid (13 mg, 0.21 mmol) were added to a solution of 6-bromoisochroman-8-carbaldehyde (100 mg, 0.41 mmol) in 1,2-dichloroethane (2 mL), and the mixture was stirred at room temperature for 1 h. Sodium cyanoborohydride (52 mg, 0.83 mmol) was then added and stirred at room temperature overnight. Upon completion of the reaction, the reaction was quenched with aqueous ammonium chloride solution (5 mL), and the reaction mixture was extracted with dichloromethane (5 mLX3). The organic phases were combined, dried over anhydrous sodium sulfate, filtered, and concentrated. The crude product was purified by a flash silica gel column (0-6% methanol (1% ammonia water)/dichloromethane) to give *N*-((6-bromoisochroman-8-yl)methyl)propan-2-amine (90 mg, yield: 77%) as a colorless liquid. ES-API: [M+H]⁺ = 284.1, 286.1.

Step 2: In the presence of protective nitrogen, a mixed solution of 3-methyl-5-(4,4,5,5-tetramethyl-1,3,2-dioxaborolan-2-yl)-1H-pyrrolo[2,3-b]pyridine (55 mg, 0.21 mmol), the *N*-((6-bromoisochroman-8-ylmethyl)propan-2-amine (77 mg, 0.27 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol), 2-dicyclohexylphosphine-2',6'-dimethoxybiphenyl (8 mg, 0.02 mmol) and potassium carbonate (87 mg, 0.63 mmol) in 1,4-dioxane (2 mL) and water (0.4 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate method) to give *N*-((6-(3-methyl-1*H*-pyrrolo[2,3-*b*]pyridin-5-yl)isochroman-8-yl)methyl)propan-2-amine (Z111, 40 mg, yield: 57%) as a white solid. ES-API: [M+H]⁺ = 336.3. ¹H NMR (500 MHz, DMSO-d₆) δ 11.33 (s, 1H), 8.48 (d, *J* = 1.5 Hz, 1H), 8.12 (s, 1H), 7.50 (s, 1H), 7.37 (s, 1H), 7.26 (s, 1H), 4.85 (s, 2H), 3.88 (t, *J* = 5.5 Hz, 2H), 3.62 (s, 2H), 2.88 (t, *J* = 5.0 Hz, 2H), 2.77 (dt, *J* = 12.0, 6.0 Hz, 1H), 2.31 (s, 3H), 1.78 (br s, 1H), 1.04 (d, *J* = 6.2 Hz, 6H).

### Example 24. Synthesis of compound Z112

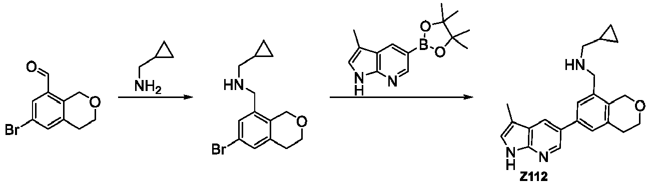

Step 1: Compound cyclopropylmethanamine (147 mg, 2.07 mmol) and acetic acid (13 mg, 0.21 mmol) were added to a solution of 6-bromoisochroman-8-carbaldehyde (100 mg, 0.41 mmol) in 1,2-dichloroethane (2 mL), and the mixture was stirred at room temperature for 1 h. Sodium cyanoborohydride (52 mg, 0.83 mmol) was then added and stirred at room temperature overnight. Upon completion of the reaction, the reaction was quenched with aqueous ammonium chloride solution (5 mL), and the reaction mixture was extracted with dichloromethane (5 mLX3). The organic phases were combined, dried over anhydrous sodium sulfate, filtered, and concentrated. The crude product was purified by a flash silica gel column (0-6% methanol (1% ammonia water)/dichloromethane) to give 1-(6-bromoisochroman-8-yl)-N-(cyclopropylmethyl)methanamine (90 mg, yield: 74%) as a colorless liquid. ES-API: [M+H]⁺ = 296.0, 298.1.

Step 2: In the presence of protective nitrogen, a mixed solution of 3-methyl-5-(4,4,5,5-tetramethyl-1,3,2-dioxaborolan-2-yl)-1*H*-pyrrolo[2,3-b]pyridine (55 mg, 0.21 mmol), the 1-(6-bromoisochroman-8-yl)-*N*-(cyclopropylmethyl)methanamine (80 mg, 0.27 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol), 2-dicyclohexylphosphine-2',6'-dimethoxybiphenyl (8 mg, 0.02 mmol) and potassium carbonate (87 mg, 0.63 mmol) in 1,4-dioxane (2 mL) and water (0.4 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate method) to give 1-cyclopropyl-*N*-((6-(3-methyl-1*H*-pyrrolo[2,3-b]pyridin-5-yl)isochroman-8-yl)methyl)m ethanamine (Z112, 11 mg, yield: 15%) as a white solid. ES-API: [M+H]⁺ = 348.3. ¹H NMR (500 MHz, DMSO-d₆) δ 11.33 (s, 1H), 8.48 (d, *J* = 2.0 Hz, 1H), 8.12 (d, *J* = 2.0 Hz, 1H), 7.51 (s, 1H), 7.38 (s, 1H), 7.26 (s, 1H), 4.83 (s, 2H), 3.88 (t, *J* = 5.5 Hz, 2H), 3.66 (s, 2H), 2.88 (t, *J* = 5.5 Hz, 2H), 2.45 (d, *J* = 7.0 Hz, 2H), 2.31 (s, 3H), 0.93 (m, 1H), 0.50 - 0.31 (m, 2H), 0.12 (m, 2H).

### Example 25. Synthesis of compound Z113

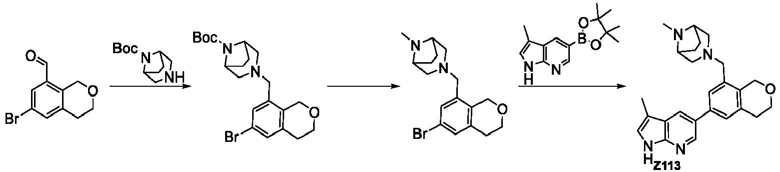

Step 1: Compound tert-butyl 3,8-diazabicyclo[3.2.1]octane-8-carboxylate (264 mg, 1.25 mmol) and acetic acid (19 mg, 0.31 mmol) were added to a solution of 6-bromoisochroman-8-carbaldehyde (150 mg, 0.62 mmol) in 1,2-dichloroethane (2 mL), and the mixture was stirred at room temperature for 1 h. Sodium cyanoborohydride (78 mg, 1.25 mmol) was then added and stirred at room temperature overnight. Upon completion of the reaction, the reaction was quenched with aqueous ammonium chloride solution (5 mL), and the reaction mixture was extracted with dichloromethane (5 mLX3). The organic phases were combined, dried over anhydrous sodium sulfate, filtered, and concentrated. The crude product was purified by a flash silica gel column (0-6% methanol (1% ammonia water)/dichloromethane) to give tert-butyl 3-((6-bromoisochroman-8-yl)methyl)-3,8-diazabicyclo[3.2.1]octane-8-carboxylate (180 mg, yield: 66%) as a colorless liquid. ES-API: [M+H]⁺ = 437.2, 439.2.

Step 2: In the presence of protective nitrogen, lithium aluminum hydride in tetrahydrofuran (0.5 mL, 1.23 mmol, 2.5 M) was slowly added to a solution of the tert-butyl 3-((6-bromoisochroman-8-yl)methyl)-3,8-diazabicyclo[3.2.1]octane-8-carboxylate (180 mg, 0.41 mmol) in tetrahydrofuran (2 mL) under an ice bath condition. The reaction mixture was stirred at 70°C for 1 h and cooled to room temperature. The reaction was quenched with sodium sulfate decahydrate (20 g). The reaction mixture was filtered with diatomaceous earth, and the filtrate was spun to dryness to give 3-((6-bromoisochroman-8-yl)methyl)-8-methyl-3,8-diazabicyclo[3.2.1]octane (150 mg, crude product). API: [M+H]⁺ = 351.1, 353.1.

Step 3: In the presence of protective nitrogen, a mixed solution of 3-methyl-5-(4,4,5,5-tetramethyl-1,3,2-dioxaborolan-2-yl)-1*H*-pyrrolo[2,3-b]pyridine (55 mg, 0.21 mmol), the 3-((6-bromoisochroman-8-yl)methyl)-8-methyl-3,8-diazabicyclo[3.2.1]octane (97 mg, 0.27 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol), 2-dicyclohexylphosphine-2',6'-dimethoxybiphenyl (8 mg, 0.02 mmol) and potassium carbonate (87 mg, 0.63 mmol) in 1,4-dioxane (2 mL) and water (0.4 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate method) to give 3-methyl-5-(8-((8-methyl-3,8-diazabicyclo[3.2.1]octan-3-yl)methyl)isochroman-6-yl)-1*H-*pyrrolo[2,3-b]pyridine (Z113, 19 mg, yield: 22%) as a white solid. ES-API: [M+H]⁺ = 403.3. ¹H NMR (500 MHz, DMSO-d₆) δ 11.32 (s, 1H), 8.45 (d, *J* = 1.5 Hz, 1H), 8.11 (d, *J* = 1.5 Hz, 1H), 7.42 - 7.36 (m, 2H), 7.25 (s, 1H), 4.85 (s, 2H), 3.88 (t, *J* = 5.5 Hz, 2H), 3.39 (s, 2H), 2.97 (s, 2H), 2.89 (t, *J* = 5.0 Hz, 2H), 2.43 (d, *J* = 8.0 Hz, 2H), 2.30 (s, 3H), 2.21 (d, *J* = 10 Hz, 2H), 2.13 (s, 3H), 1.86 - 1.78 (m, 2H), 1.63 - 1.55 (m, 2H).

### Example 26. Synthesis of compound Z186

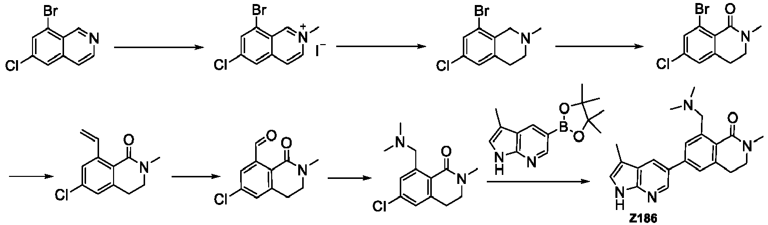

Step 1: 8-Bromo-6-chloroisoquinoline (1 g, 4.12 mmol) was dissolved in acetonitrile (30 mL). Iodomethane (2.92 g, 20.6 mmol) was added, and the system was heated in a sealed tube for 5 h. Upon completion of the reaction, the system was cooled to room temperature, and the obtained mixture was concentrated to give 8-bromo-6-chloro-2-methylisoquinolin-2-ium iodide (1.5 g, crude product). ES-API: [M+H]⁺=256.0, 258.0.

Step 2: The 8-bromo-6-chloro-2-methylisoquinolin-2-ium iodide (1.4 g, crude product) was dissolved in methanol (30 mL). Sodium borohydride (1.12 g, 29.4 mmol) was slowly added under an ice-water bath condition, and the system reacted at room temperature for 2 h. Upon completion of the reaction, ethyl acetate (100 mL) was added. The obtained mixture was washed with water (30 mL) and saturated brine (30 mL) successively, dried over anhydrous sodium sulfate, and filtered. The crude product was purified by a flash silica gel column (ethyl acetate:petroleum ether = 50:50) to give 8-bromo-6-chloro-2-methyl-1,2,3,4-tetrahydroisoquinoline (750 mg, yield of the 2 steps: 78%). ES-API: [M + Na]⁺=260, 262.

Step 3: The 8-bromo-6-chloro-2-methyl-1,2,3,4-tetrahydroisoquinoline (520 mg, 2.0 mmol) was dissolved in dried carbon tetrachloride (20 mL) and acetonitrile (5 mL). Sodium periodate (1.28 g, 6.0 mmol) in water (10 mL) and ruthenium trichloride (124 mg, 0.6 mmoL) was added successively, and the system reacted at room temperature for 4 h. The reaction was quenched by the addition of aqueous sodium thiosulfate solution, and the reaction mixture was extracted with dichloromethane (30 mL × 2 times). The organic layer was separated, dried over anhydrous sodium sulfate, filtered, and concentrated. The crude product was purified by a flash silica gel column (ethyl acetate:petroleum ether = 80:20) to give 8-bromo-6-chloro-2-methyl-3,4-dihydroisoquinolin-1(2H)-one (110 mg, yield: 20%). ES-API: [M+H]⁺= 274, 276.

Step 4: The 8-bromo-6-chloro-2-methyl-3,4-dihydroisoquinolin-1(2H)-one (100 mg, 0.365 mmol) reacted with potassium vinyltrifluoroborate (146 mg, 1.09 mmol), [1,1'-bis(diphenylphosphino)ferrocene]dichloropalladium (29.7 mg, 0.0365 mmol), triethylamine (36.8 mg, 0.365 mmol) and ethanol (8 mL) under nitrogen replacement and microwave at 100°C for 1 h. Ethyl acetate (50 mL) was added. The obtained mixture was washed with water (20 mL) and saturated brine (20 mL) successively, dried over anhydrous sodium sulfate, and purified by column chromatography (ethyl acetate: petroleum ether = 1: 2) to give 6-chloro-2-methyl-8-vinyl-3,4-dihydroisoquinolin-1(2H)-one (50 mg, yield: 62%). ES-API: [M+H]⁺= 222.0.

Step 5: The 6-chloro-2-methyl-8-vinyl-3,4-dihydroisoquinolin-1(2H)-one (50 mg, 0.226 mmol) was dissolved in tetrahydrofuran (5 mL). An aqueous solution (2 mL) of potassium osmate dihydrate (25 mg, 0.0678 mmol) and sodium periodate (290 mg, 1.35 mmol) was added at room temperature. After reacting at room temperature for 2 h, the system was cooled to 0°C, and the reaction was quenched by the addition of aqueous sodium thiosulfate solution. The obtained mixture was diluted with ethyl acetate (20 mL), washed with water (20 mL) and saturated brine (20 mL) successively, dried over anhydrous sodium sulfate, filtered, and concentrated. The crude product was purified by column chromatography (ethyl acetate:petroleum ether = 3:1) to give 6-chloro-2-methyl-1-oxo-1,2,3,4-tetrahydroisoquinoline-8-carbaldehyde (36 mg, yield: 72%). ES-API: [M+H]⁺= 224.0.

Step 6: The 6-chloro-2-methyl-1-oxo-1,2,3,4-tetrahydroisoquinoline-8-carbaldehyde (36 mg, 0.161 mmol) was dissolved in tetrahydrofuran (10 mL). Dimethylamine in tetrahydrofuran (0.24 mL, 0.0483 mmol, 2M), glacial acetic acid (9.66 mg, 0.161 mmol) and sodium cyanoborohydride (20 mg, 0.322 mmoL) were added successively, and the system reacted at room temperature for 2 h. Upon completion of the reaction, ethyl acetate (20 mL) was added. The obtained mixture was washed with saturated aqueous sodium bicarbonate solution (20 mL) and saturated brine (20 mL) successively, dried over anhydrous sodium sulfate, filtered, and concentrated. The crude product was purified by silica gel column (ethyl acetate:petroleum ether = 90:10) to give 6-chloro-8-((dimethylamino)methyl)-2-methyl-3,4-dihydroisoquinolin-1(2H)-one (25 mg, yield: 61.7%). ES-API: [M+H]⁺=253.1.

Step 7: The 6-chloro-8-((dimethylamino)methyl)-2-methyl-3,4-dihydroisoquinolin-1(2H)-one (25 mg, 0.1 mmol), 3-methyl-5-(4,4,5,5-tetramethyl-1,3,2-dioxaborolan-2-yl)-1H-pyrrolo[2,3-b]pyridine (25.8 mg, 0.1 mmol), Sphos G2 Pd (7.2 mg, 0.01 mmol) and potassium carbonate (41.4 mg, 0.3 mmol) were dissolved in 1,4-dioxane (2 mL) and water (0.5 mL), and the system reacted under microwave at 100°C for 0.5 h. Upon completion of the reaction, the reaction mixture was filtered, concentrated, and purified by HPLC (ammonia water method) to give 8-((dimethylamino)methyl)-2-methyl-6-(3-methyl-1H-pyrrolo[2,3-b]pyridin-5-yl)-3,4-dih ydroisoquinolin-1(2H)-one (Z186, 3.5 mg, yield: 10%). ES-API: [M+H]⁺=349.2.

### Example 27. Synthesis of compound Z131

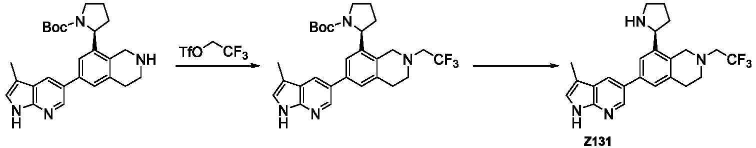

Step 1: tert-butyl (S)-2-(6-(3-methyl-1*H*-pyrrolo[2,3-b]pyridin-5-yl)-1,2,3,4-tetrahydroisoquinolin-8-yl)pyrr olidine-1-carboxylate (70 mg, 0.16 mmol) and triethylamine (48 mg, 0.48 mmol) were dissolved in *N*,*N*-dimethylformamide (2 mL). 2,2,2-Trifluoroethyl triflate (51 mg, 0.24 mmol) was added at room temperature and stirred at room temperature for 16 h. Water (5 mL) was added to the reaction mixture, and the obtained mixture was extracted with ethyl acetate (40 mL). The organic phase was washed with saturated brine (15×3 mL), dried over anhydrous sodium sulfate, and concentrated. The crude product was purified by a flash silica gel column (7M ammonia in methanol/dichloromethane: 0-5%) to give tert-butyl (S)-2-(6-(3-methyl-1*H*-pyrrolo[2,3-*b*]pyridin-5-yl)-2-(2,2,2-trifluoroethyl)-1,2,3,4-tetrahy droisoquinolin-8-yl)pyrrolidine-1-carboxylate (70 mg, yield: 84%) as a pale yellow solid. ES-API: [M+H]⁺=515.3.

Step 2: The tert-butyl (S)-2-(6-(3-methyl-1*H*-pyrrolo[2,3-*b*]pyridin-5-yl)-2-(2,2,2-trifluoroethyl)-1,2,3,4-tetrahy droisoquinolin-8-yl)pyrrolidine-1-carboxylate (65 mg, 0.13 mmol) was added to 3M hydrochloric acid-methanol solution (4 ml) and stirred at room temperature for 1 h. The reaction mixture was concentrated. 7.0M Ammonia/methanol solution (5 mL) was added, and a solvent was spun to dryness. The crude product was purified by prep-HPLC (ammonium bicarbonate method) to give (*S*)-6-(3-methyl-1*H*-pyrrolo[2,3-*b*]pyridin-5-yl)-8-(pyrrolidin-2-yl)-2-(2,2,2-trifluoroethyl) -1,2,3,4-tetrahydroisoquinoline (Z131, 48 mg, yield: 92%) as a pale yellow solid. ES-API: [M+H]⁺415.1. ¹H NMR (500 MHz, CD₃OD) δ 8.51 (d, *J* = 2.0 Hz, 1H), 8.37 (d, *J* = 2.0 Hz, 1H), 7.71 (d, *J* = 1.5 Hz, 1H), 7.57(d, *J* = 1.5 Hz, 1H), 7.26 (q, *J* = 1.0 Hz, 1H), 4.79 (dd, *J* = 10.0, 7.0 Hz, 1H), 4.11 (d, *J* = 15.0 Hz, 1H), 4.00 (d, *J* = 15.0 Hz, 1H), 3.65 - 3.56 (m, 1H), 3.52 - 3.44 (m, 1H), 3.40 (d, *J* = 10.0 Hz, 1H), 3.36 (d, *J* = 10.0 Hz, 1H), 3.10 - 2.98 (m, 4H), 2.58 - 2.48 (m, 1H), 2.39 (d, *J* = 1.0 Hz, 3H), 2.37 - 2.19 (m, 3H).

### Example 28. Synthesis of compound Z190

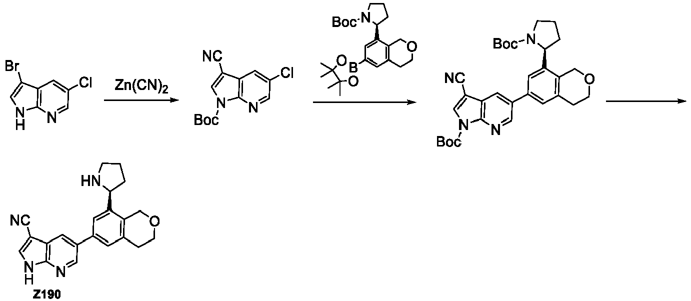

Step 1: 3-Bromo-5-chloro-1H-pyrrolo[2,3-b]pyridine (2.31g, 10.0 mmol) was dissolved in dried N,N-dimethylformamide (5 mL). Zinc cyanide (1.17 mg, 10.0 mmol) and tetrakis(triphenylphosphine)palladium (1.15 g, 1.0 mmol) were added successively, and the system reacted at 100°C for 2 h. Upon completion of the reaction, the reaction mixture was diluted with ethyl acetate (30 mL), washed with saturated brine (30 mL), dried over anhydrous sodium sulfate, filtered, concentrated, and diluted with dichloromethane (30 mL). Di-tert-butyl dicarbonate (3.27 g, 15 mmoL) and triethylamine (2.02 g, 20.0 mmoL) were added successively and stirred at room temperature for 1 h. Upon completion of the reaction, the reaction mixture was concentrated. The crude product was purified by silica gel column (petroleum ether:ethyl acetate = 70:30) to give tert-butyl 5-chloro-3-cyano-1H-pyrrolo[2,3-b]pyridine-1-carboxylate (260 mg, yield: 9.3%). ES-API: [M+H]⁺=278, 222.

Step 2: The tert-butyl 5-chloro-3-cyano-1H-pyrrolo[2,3-b]pyridine-1-carboxylate (30 mg, 0.129 mmol), tert-butyl (S)-2-(6-(4,4,5,5-tetramethyl-1,3,2-dioxaborolan-2-yl)isochroman-8-yl)pyrrolidine-1-carb oxylate (55.7 mg, 0.129 mmol), Sphos Pd G2 (9.3 mg, 0.0129 mmol) and potassium carbonate (53.4 mg, 0.387 mmol) were added to 1,4-dioxane (2 mL) and water (0.5 mL). The system was replaced with nitrogen and reacted under microwave at 110°C for 1 h. Upon completion of the reaction, ethyl acetate (20 mL) was added. The obtained mixture was washed with water (10 mL) and saturated brine (10 mL) successively, dried over anhydrous sodium sulfate, and purified by column chromatography (petroleum ether:ethyl acetate = 10:90) to give tert-butyl (S)-5-(8-(1-(tert-butoxycarbonyl)pyrrolidin-2-yl)isochroman-6-yl)-3-cyano-1H-pyrrolo [2, 3-b]pyridine-1-carboxylate (15 mg, yield: 21%). ES-API: [M+H]⁺=545.2.

Step 3: The tert-butyl (S)-5-(8-(1-(tert-butoxycarbonyl)pyrrolidin-2-yl)isochroman-6-yl)-3-cyano-1H-pyrrolo[2, 3-b]pyridine-1-carboxylate (15 mg, 0.027 mmol) was dissolved in dichloromethane (2 mL) and added to trifluoroacetic acid (1 mL), and the system reacted at room temperature for 0.5 h, Upon completion of the reaction, the reaction mixture was concentrated. After neutralization with ammonia/methanol solution (1 mL), the obtained mixture was concentrated again and purified by HPLC (trifluoroacetic acid method) to give (S)-5-(8-(pyrrolidin-2-yl)isochroman-6-yl)-1H-pyrrolo[2,3-b]pyridine-3-carbonitrile (Z190, trifluoroacetate, 2 mg, yield: 21.7%). ES-API: [M+H]⁺=345.2.

### Example 29. Synthesis of compound Z199

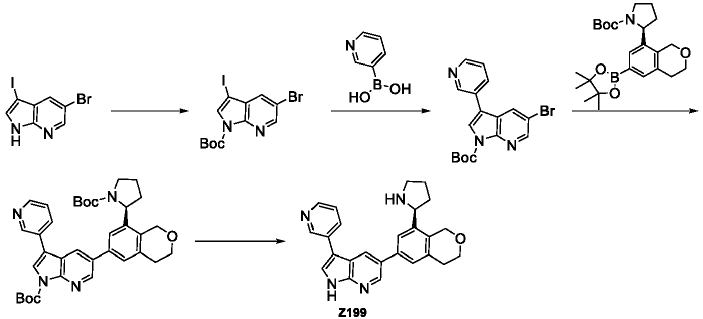

Step 1: 5-Bromo-3-iodo-1H-pyrrolo[2,3-b]pyridine (2 g, 6.21 mmol) was dissolved in dichloromethane (50 mL). Di-tert-butyl dicarbonate (2.03 g, 9.31 mmol), triethylamine (1.25 g, 12.4 mmol) and dimethylamino pyridine (75 mg, 0.621 mmol) were added successively, and the system reacted at room temperature for 1 h. The obtained mixture was concentrated. The crude product was purified by silica gel column (petroleum ether:ethyl acetate = 60:40) to give tert-butyl 5-bromo-3-iodo-1H-pyrrolo[2,3-b]pyridine-1-carboxylate (2.3 g, yield: 87.7%) ES-API: [M+H]⁺=423.0, 424.0.

Step 2: The tert-butyl 5-bromo-3-iodo-1H-pyrrolo[2,3-b]pyridine-1-carboxylate (100 mg, 0.236 mmol) was dissolved in dried dioxane (8 mL). Pyridin-3-ylboronic acid (29 mg, 0.236 mmol) and [1,1'-bis(diphenylphosphino)ferrocene]dichloropalladium-dichloromethane complex (19.2 mg, 0.0236 mmol) were added successively, and the system reacted at 80°C for 0.5 h. Upon completion of the reaction, the reaction mixture was diluted with ethyl acetate (30 mL), washed with saturated brine (30 mL), dried over anhydrous sodium sulfate, filtered, and concentrated. The crude product was purified by silica gel column (petroleum ether:ethyl acetate = 70:30) to give tert-butyl 5-bromo-3-(pyridin-3-yl)-1H-pyrrolo[2,3-b]pyridine-1-carboxylate (40 mg, yield: 45%). ES-API: [M+H]⁺=373, 375.

Step 3: The tert-butyl 5-bromo-3-(pyridin-3-yl)-1H-pyrrolo[2,3-b]pyridine-1-carboxylate (40 mg, 0.11 mmol), tert-butyl (S)-2-(6-(4,4,5,5-tetramethyl-1,3,2-dioxaborolan-2-yl)isochroman-8-yl)pyrrolidine-1-carb oxylate (45 mg, 0.11 mmol), Sphos Pd G2 (7.7 mg, 0.011 mmol) and potassium carbonate (45 mg, 0.387 mmol) were added to 1,4-dioxane (2 mL) and water (0.5 mL). The system was replaced with nitrogen and reacted under microwave at 110°C for 1 h. Upon completion of the reaction, ethyl acetate (20 mL) was added. The obtained mixture was washed with water (10 mL) and saturated brine (10 mL) successively, dried over anhydrous sodium sulfate, and purified by column chromatography (petroleum ether:ethyl acetate = 10:90) to give tert-butyl (S)-5-(8-(1-(tert-butoxycarbonyl)pyrrolidin-2-yl)isochroman-6-yl)-3-(pyridin-3-yl)-1H-py rrolo[2,3-b]pyridine-1-carboxylate (10 mg, yield: 15.3%). ES-API: [M+H]⁺=597.3.

Step 4: The tert-butyl (S)-5-(8-(1-(tert-butoxycarbonyl)pyrrolidin-2-yl)isochroman-6-yl)-3-(pyridin-3-yl)-1H-py rrolo[2,3-b]pyridine-1-carboxylate (10 mg, 0.017 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia/methanol solution (1 mL), the obtained mixture was concentrated again and purified by prep-HPLC (ammonia water method) to give (S)-3-(pyridin-3-yl)-5-(8-(pyrrolidin-2-yl)isochroman-6-yl)-1H-pyrrolo[2,3-b]pyridine (Z199, 3.3 mg, yield: 48%). ES-API: [M+H]⁺=397.2.

### Example 30. Synthesis of compound Z116-1 and compound Z116-2

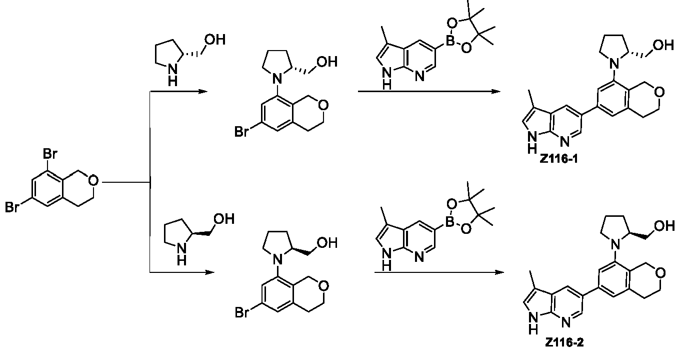

Step 1: In the presence of protective nitrogen, a mixed solution of 6,8-dibromoisochroman (300 mg, 1.03 mmol), D-prolinol (114 mg, 1.13 mmol), cuprous iodide (19 mg, 0.1 mmol), potassium carbonate (285 mg, 2.06 mmol) in *N*,*N*-dimethylformamide (3 mL) was stirred at 100°C overnight. Upon completion of the reaction, the reaction mixture was poured into ethyl acetate (15 mL) and washed with saturated brine (5 mLX3). The organic phase was dried over anhydrous sodium sulfate, filtered, concentrated, and purified by a preparative thin layer chromatography plate (petroleum ether:ethyl acetate = 1:1) to give (R)-(1-(6-bromoisochroman-8-yl)pyrrolidin-2-yl)methanol (30 mg, yield: 9%) as a colorless liquid. ES-API: [M+H]⁺ = 312.0, 314.0.

Step 2: In the presence of protective nitrogen, a mixed solution of 3-methyl-5-(4,4,5,5-tetramethyl-1,3,2-dioxaborolan-2-yl)-1*H*-pyrrolo[2,3-b]pyridine (20 mg, 0.08 mmol), the (R)-(1-(6-bromoisochroman-8-yl)pyrrolidin-2-yl)methanol (30 mg, 0.10 mmol), chloro (2-dicyclohexylphosphino-2',6'-dimethoxy-1,1'-biphenylyl)(2'-amino-1,1'-biphenyl-2-yl) palladium (II) (7 mg, 0.01 mmol), 2-dicyclohexylphosphine-2',6'-dimethoxybiphenyl (4 mg, 0.01 mmol) and potassium carbonate (33 mg, 0.24 mmol) in 1,4-dioxane (2 mL) and water (0.4 mL) was stirred under microwave irradiation at 120°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate method) to give (R)-(1-(6-(3-methyl-1*H*-pyrrolo[2,3-b]pyridin-5-yl)isochroman-8-yl)pyrrolidin-2-yl)metha nol (Z116-1, 3 mg, yield: 10%) as a white solid. ES-API: [M+H]⁺ = 364.3. ¹H NMR (500 MHz, DMSO-d₆) δ 11.34 (s, 1H), 8.08 (d, *J* = 2.0 Hz, 1H), 7.78 (d, *J* = 2.0 Hz, 1H), 7.26 (s, 1H), 6.39 (d, *J* = 2.0 Hz, 1H), 6.36 (d, *J* = 2.0 Hz, 1H), 4.74 (t, *J* = 5.5 Hz, 1H), 4.44 (d, *J* = 2.0 Hz, 2H), 3.89 - 3.81 (m, 2H), 3.69 - 3.64 (m, 1H), 3.55 - 3.46 (m, 1H), 3.42 - 3.34 (m, 1H), 3.17 - 3.10 (m, 1H), 3.03 (dd, J = 16.1, 9.1 Hz, 1H), 2.80 (t, *J* = 5.5 Hz, 2H), 2.26 (d, J = 0.7 Hz, 3H), 2.05 - 1.79 (m, 4H).

Step 3: In the presence of protective nitrogen, a mixed solution of 6,8-dibromoisochroman (300 mg, 1.03 mmol), L-prolinol (114 mg, 1.13 mmol), cuprous iodide (19 mg, 0.1 mmol), potassium carbonate (285 mg, 2.06 mmol) in *N*,*N*-dimethylformamide (3 mL) was stirred at 100°C overnight. Upon completion of the reaction, the reaction mixture was poured into ethyl acetate (15 mL) and washed with saturated brine (5 mLX3). The organic phase was dried over anhydrous sodium sulfate, filtered, and concentrated. The crude product was purified by a preparative chromatography plate (petroleum ether:ethyl acetate = 1:1) to give (S)-(1-(6-bromoisochroman-8-yl)pyrrolidin-2-yl)methanol (30 mg, yield: 9%) as a colorless liquid. ES-API: [M+H]⁺ = 312.0, 314.0.

Step 4: In the presence of protective nitrogen, a mixed solution of 3-methyl-5-(4,4,5,5-tetramethyl-1,3,2-dioxaborolan-2-yl)-1*H*-pyrrolo[2,3-b]pyridine (20 mg, 0.08 mmol), the (S)-(1-(6-bromoisochroman-8-yl)pyrrolidin-2-yl)methanol (30 mg, 0.10 mmol), chloro (2-dicyclohexylphosphino-2',6'-dimethoxy-1,1'-biphenylyl)(2'-amino-1,1'-biphenyl-2-yl) palladium (II) (7 mg, 0.01 mmol), 2-dicyclohexylphosphine-2',6'-dimethoxybiphenyl (4 mg, 0.01 mmol) and potassium carbonate (33 mg, 0.24 mmol) in 1,4-dioxane (2 mL) and water (0.4 mL) was stirred under microwave irradiation at 120°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate method) to give (S)-(1-(6-(3-methyl-1*H*-pyrrolo[2,3-*b*]pyridin-5-yl)isochroman-8-yl)pyrrolidin-2-yl)metha nol (Z116-2, 4 mg, yield: 14%) as a white solid. ES-API: [M+H]⁺ = 364.3. ¹H NMR (500 MHz, DMSO-d₆) δ 11.34 (s, 1H), 8.07 (d, *J* = 2.0 Hz, 1H), 7.78 (d, *J* = 2.0 Hz, 1H), 7.26 (s, 1H), 6.39 (d, *J* = 2.0 Hz, 1H), 6.36 (d, *J* = 2.5 Hz, 1H), 4.77 - 4.70 (m, 1H), 4.44 (s, 2H), 3.87 - 3.82 (m, 2H), 3.70 - 3.64 (m, 1H), 3.55 - 3.46 (m, 1H), 3.42 - 3.35 (m, 1H), 3.19 - 3.10 (m, 1H), 3.07 - 2.98 (m, 1H), 2.80 (t, *J* = 5.6 Hz, 2H), 2.26 (s, 3H), 2.02 - 1.82 (m, 4H).

### Example 31. Synthesis of compound Z110

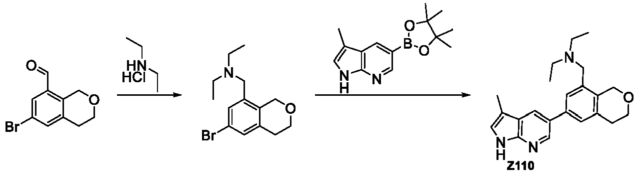

Step 1: Compound diethylamine hydrochloride (227 mg, 2.07 mmol) was added to a solution of 6-bromoisochroman-8-carbaldehyde (100 mg, 0.41 mmol) in 1,2-dichloroethane (2 mL), and the mixture was stirred at room temperature for 1 h. Sodium cyanoborohydride (52 mg, 0.83 mmol) was then added and stirred at room temperature overnight. Upon completion of the reaction, the reaction was quenched with aqueous ammonium chloride solution (5 mL), and the reaction mixture was extracted with dichloromethane (5 mLX3). The organic phases were combined, dried over anhydrous sodium sulfate, filtered, and concentrated. The crude product was purified by a flash silica gel column (0-6% methanol (1% ammonia water)/dichloromethane) to give *N*-((6-bromoisochroman-8-yl)methyl)-*N*-ethylethanamine (90 mg, yield: 74%) as a colorless liquid. ES-API: [M+H]⁺ = 298.1, 300.1.

Step 2: In the presence of protective nitrogen, a mixed solution of 3-methyl-5-(4,4,5,5-tetramethyl-1,3,2-dioxaborolan-2-yl)-1*H*-pyrrolo[2,3-*b*]pyridine (55 mg, 0.21 mmol), the *N*-((6-bromoisochroman-8-yl)methyl)-*N*-ethylethanamine (80 mg, 0.27 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol), 2-dicyclohexylphosphine-2',6'-dimethoxybiphenyl (8 mg, 0.02 mmol) and potassium carbonate (87 mg, 0.63 mmol) in 1,4-dioxane (2 mL) and water (0.4 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate method) to give *N*-ethyl-*N*-((6-(3-methyl-1*H*-pyrrolo[2,3-*b*]pyridin-5-yl)isochroman-8-yl)methyl)ethanami ne (Z110, 14 mg, yield: 19%) as a white solid. ES-API: [M+H]⁺ = 350.3. ¹H NMR (500 MHz, DMSO-*d₆*) δ 11.33 (s, 1H), 8.46 (s, 1H), 8.11 (s, 1H), 7.45 (s, 1H), 7.39 (s, 1H), 7.26 (s, 1H), 4.86 (s, 2H), 3.87 (s, 2H), 3.49 (s, 2H), 2.89 (s, 2H), 2.46 (d, *J=* 6.5 Hz, 4H), 2.31 (s, 3H), 0.98 (t, *J=* 6.5 Hz, 6H).

### Example 32. Synthesis of compound Z226

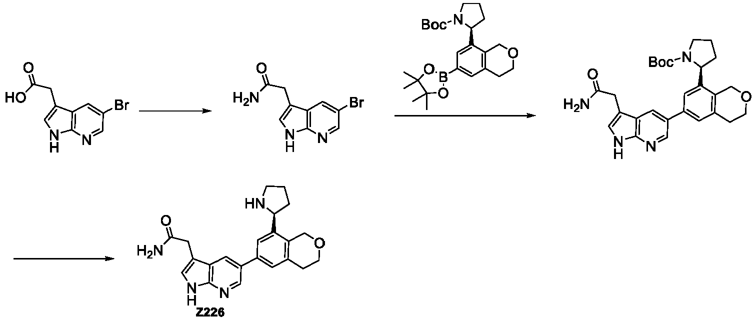

Step 1: Oxalyl chloride (0.49 mL, 0.784 mmol) was added dropwise to a flask containing a solution of 2-(5-bromo-1H-pyrrolo[2,3-b]pyridin-3-yl)acetic acid (100 mg, 0.392 mmol) in dichloromethane (10 mL) at 0°C, followed by N,N-dimethylformamide (0.024 mL, 0.313 mmol). The mixture was stirred at room temperature for 1 h. After concentration, dichloromethane (10 mL) and ammonia water (2 mL) were added and stirred at room temperature for 1 h. The obtained mixture was concentrated. The crude product was purified by column chromatography (petroleum ether:ethyl acetate = 10:90) to give 2-(5-bromo-1H-pyrrolo[2,3-b]pyridin-3-yl)acetamide (40 mg, yield: 40%). ES-API: [M+H]⁺=254.1, 256.1.

Step 2: The 2-(5-bromo-1H-pyrrolo[2,3-b]pyridin-3-yl)acetamide (40 mg, 0.157 mmol), tert-butyl (S)-2-(6-(4,4,5,5-tetramethyl-1,3,2-dioxaborolan-2-yl)isochroman-8-yl)pyrrolidine-1-carb oxylate (67 mg, 0.157 mmol), Sphos Pd G2 (11.3 mg, 0.0157 mmol) and potassium carbonate (65 mg, 0.472 mmol) were added to 1,4-dioxane (2 mL) and water (0.5 mL). The system was replaced with nitrogen and reacted under microwave at 110°C for 1 h. Upon completion of the reaction, ethyl acetate (20 mL) was added. The obtained mixture was washed with water (10 mL) and saturated brine (10 mL) successively, dried over anhydrous sodium sulfate, and purified by column chromatography (petroleum ether:ethyl acetate = 10:90) to give tert-butyl (S)-2-(6-(3-(2-amino-2-oxoethyl)-1H-pyrrolo[2,3-b]pyridin-5-yl)isochroman-8-yl)pyrrolid ine-1-carboxylate (50 mg, crude product). ES-API: [M+H]⁺=477.2.

Step 3: The tert-butyl (S)-2-(6-(3-(2-amino-2-oxoethyl)-1H-pyrrolo[2,3-b]pyridin-5-yl)isochroman-8-yl)pyrrolid ine-1-carboxylate (50 mg, crude product) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia/methanol solution (1 mL), the obtained mixture was concentrated again and purified by prep-HPLC (ammonia water method) to give (S)-2-(5-(8-(pyrrolidin-2-yl)isochroman-6-yl)-1H-pyrrolo[2,3-b]pyridin-3-yl)acetamide (Z226, 15 mg, yield of the 2 steps: 25.4%). ES-API: [M+H]⁺=377.2.

### Example 33. Synthesis of compound Z233

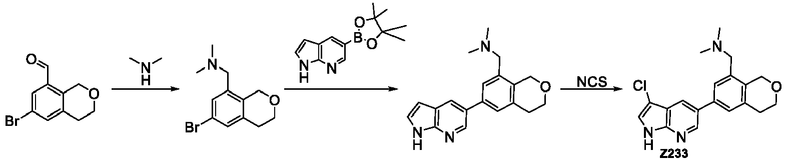

Step 1: 2M Dimethylamine in tetrahydrofuran (1 mL, 2 mmol) and acetic acid (13 mg, 0.21 mmol) were added to a solution of 6-bromoisochroman-8-carbaldehyde (100 mg, 0.41 mmol) in 1,2-dichloroethane (2 mL), and the mixture was stirred at room temperature for 1 h. Sodium cyanoborohydride (52 mg, 0.83 mmol) was then added and stirred at room temperature overnight. Upon completion of the reaction, the reaction was quenched with aqueous ammonium chloride solution (5 mL), and the reaction mixture was extracted with dichloromethane (5 mLX3). The organic phases were combined, dried over anhydrous sodium sulfate, filtered, and concentrated. The crude product was purified by a flash silica gel column (0-6% methanol (1% ammonia water)/dichloromethane) to give 1-(6-bromoisochroman-8-yl)-N,N-dimethylmethanamine (90 mg, yield: 81%) as a colorless liquid. ES-API: [M+H]⁺ = 270.1, 272.1.

Step 2: In the presence of protective nitrogen, a mixed solution of 5-(4,4,5,5-tetramethyl-1,3,2-dioxaborolan-2-yl)-1*H*-pyrrolo[2,3-*b*]pyridine (43 mg, 0.18 mmol), the 1-(6-bromoisochroman-8-yl)-N,N-dimethylmethanamine (40 mg, 0.15 mmol), chloro (2-dicyclohexylphosphino-2',6'-dimethoxy-1,1'-biphenylyl)(2'-amino-1,1'-biphenyl-2-yl) palladium (II) (7 mg, 0.01 mmol), 2-dicyclohexylphosphine-2',6'-dimethoxybiphenyl (4 mg, 0.01 mmol) and potassium carbonate (62 mg, 0.45 mmol) in 1,4-dioxane (2 mL) and water (0.4 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, and concentrated. The crude product was purified by a flash silica gel column (0-6% methanol (1% ammonia water)/dichloromethane) to give 1-(6-(1*H*-pyrrolo[2,3-*b*]pyridin-5-yl)isochroman-8-yl)-*N*,*N*-dimethylmethanamine (40 mg, yield: 88%) as a white solid. ES-API: [M+H]⁺ = 308.2.

Step 3: N-Chlorosuccinimide (9 mg, 65 µmol) was added to a solution of the 1-(6-(1*H*-pyrrolo[2,3-*b*]pyridin-5-yl)isochroman-8-yl)-*N*,*N*-dimethylmethanamine (20 mg, 65 µmol) in acetonitrile (1 mL) and stirred at room temperature for 2 h. The reaction was quenched with sodium thiosulfate solution (1 mL). The reaction mixture was extracted with ethyl acetate (1 mL), spun to dryness, and purified by prep-HPLC (ammonium bicarbonate method) to give 1-(6-(3-chloro-1*H*-pyrrolo[2,3-*b*]pyridin-5-yl)isochroman-8-yl)-*N*,*N*-dimethylmethanamin e (Z233, 8 mg, yield: 36%) as a white solid. ES-API: [M+H]⁺ = 342.2. ¹H NMR (500 MHz, DMSO-*d₆*) δ 12.05 (s, 1H), 8.59 (d, *J =* 2.0 Hz, 1H), 8.10 (d, *J =* 2.0 Hz, 1H), 7.73 (d, *J* = 2.0 Hz, 1H), 7.45 (s, 1H), 7.43 (s, 1H), 4.84 (s, 2H), 3.88 (t, *J* = 5.5 Hz, 2H), 3.34 (s, 2H), 2.90 (t, *J* = 5.5 Hz, 2H), 2.16 (s, 6H).

### Example 34. Synthesis of compound Z108-1

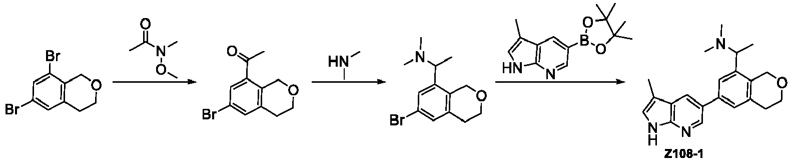

Step 1: In the presence of protective nitrogen, 6,8 dibromoisochroman (2 g, 6.85 mmol) in tetrahydrofuran (30 mL) was cooled to -60°C. n-Butyllithium in tetrahydrofuran (2.74 mL, 6.85 mmol, 2.5 M) was slowly added dropwise and stirred at this temperature for 2 h. N-Methyl-N-methoxyacetamide (1.41 g, 13.70 mmol) was then added, and the system continued to be stirred for 10 min. The reaction was quenched with saturated aqueous ammonium chloride solution (10 mL), and the reaction mixture was extracted with ethyl acetate (10 mLX3). The organic phases were combined, dried over anhydrous sodium sulfate, filtered, and spun to dryness. The crude product was purified by a flash silica gel column (0-20% ethyl acetate/petroleum ether) to give 1-(6-bromoisochroman-8-yl)ethan-1-one (200 mg, yield: 11%). ES-API: [M+H]⁺ = 255.

Step 2: Dimethylamine in tetrahydrofuran (1 mL, 2 mmol, 2 M) and titanium tetraisopropoxide (222 mg, 0.78 mmol) were added to a solution of the 1-(6-bromoisochroman-8-yl)ethan-1-one (200 mg, 0.78 mmol) in 1,2-dichloroethane (2 mL), and the mixture was stirred at room temperature for 1 h. Sodium cyanoborohydride (98 mg, 1.56 mmol) was then added and stirred at room temperature overnight. Upon completion of the reaction, the reaction was quenched with aqueous ammonium chloride solution (5 mL), and the reaction mixture was extracted with dichloromethane (5 mLX3). The organic phases were combined, dried over anhydrous sodium sulfate, filtered, and concentrated. The crude product was purified by a flash silica gel column (0-6% methanol (1% ammonia water)/dichloromethane) to give 1-(6-bromoisochroman-8-yl)-N,N-dimethylethan-1-amine (100 mg, yield: 45%) as a colorless liquid. ES-API: [M+H]⁺ = 284.2, 286.2.

Step 3: In the presence of protective nitrogen, a mixed solution of 3-methyl-5-(4,4,5,5-tetramethyl-1,3,2-dioxaborolan-2-yl)-1H-pyrrolo[2,3-b]pyridine (90 mg, 0.35 mmol), the 1-(6-bromoisochroman-8-yl)-N,N-dimethylethan-1-amine (100 mg, 0.35 mmol), chloro (2-dicyclohexylphosphino-2',6'-dimethoxy-1,1'-biphenylyl)(2'-amino-1,1'-biphenyl-2-yl) palladium (II) (25 mg, 0.03 mmol), 2-dicyclohexylphosphine-2',6'-dimethoxybiphenyl (14 mg, 0.03 mmol) and potassium carbonate (145 mg, 1.05 mmol) in 1,4-dioxane (2 mL) and water (0.4 mL) was stirred under microwave irradiation at 120°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate method) to give *N*,*N*-dimethyl-1-(6-(3-methyl-1*H*-pyrrolo[2,3-*b*]pyridin-5-yl)isochroman-8-yl)ethan-1-ami ne (Z108-1, 40 mg, yield: 34%) as a white solid. ES-API: [M+H]⁺ = 336.3. ¹H NMR (500 MHz, DMSO-*d₆*) δ 11.33 (s, 1H), 8.45 (d, *J =* 2.0 Hz, 1H), 8.10 (d, *J =* 2.0 Hz, 1H), 7.52 (s, 1H), 7.36 (s, 1H), 7.26 (s, 1H), 4.91 (d, *J* = 15.5 Hz, 1H), 4.80 (d, *J* = 15.5 Hz, 1H), 3.87 (t, *J* = 5.5 Hz, 2H), 3.32 - 3.28 (m, 1H), 2.90 (t, *J* = 5.5 Hz, 2H), 2.31 (s, 3H), 2.15 (s, 6H), 1.28 (d, *J* = 6.5 Hz, 3H).

### Example 35. Synthesis of compound Z181

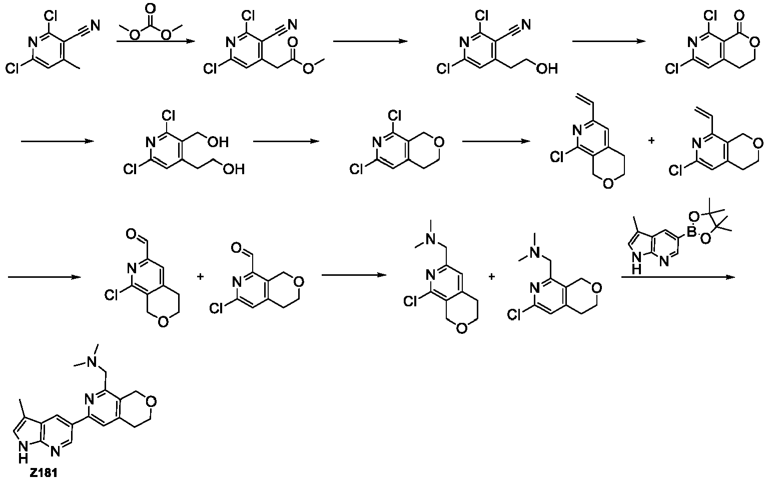

Step 1: In the presence of protective nitrogen, lithium bis(trimethylsilyl)amide in tetrahydrofuran (360 mL, 360 mmol, 1M) was slowly added dropwise to a solution of 2,6-dichloro-4-methylnicotinonitrile (22.4 g, 120 mmol) in tetrahydrofuran (200 mL) at -78°C. After 30 min, dimethyl carbonate (16.2 g, 180 mmol) was slowly added dropwise. The mixture was slowly warmed to 0°C and stirred for 2 h. The obtained mixture was slowly poured into a chilled aqueous ammonium chloride solution (100 mL) and stirred for 10 min. Ethyl acetate (200 mL) was then added. The organic phase was separated, washed with water (100 mL) and saturated brine (100 mL) successively, dried over anhydrous sodium sulfate, and purified by column chromatography (petroleum ether:ethyl acetate = 80:20) to give methyl 2-(2,6-dichloro-3-cyanopyridin-4-yl)acetate (6 g, yield: 20.5%). ES-API: [M+H]⁺=245.0.

Step 2: The methyl 2-(2,6-dichloro-3-cyanopyridin-4-yl)acetate (4 g, 16.39 mmol) was dissolved in anhydrous ethanol (80 mL), and the system was cooled in an ice-water bath. Sodium borohydride (1.86 g, 49.18 mmol) was slowly added in batches, and the system reacted at 0°C for 0.5 h. Upon completion of the reaction, the reaction was quenched with aqueous ammonium chloride solution (20 mL). Ethyl acetate (100 mL) was added. The obtained mixture was washed with water (50 mL) and saturated brine (50 mL) successively, dried over anhydrous sodium sulfate, and purified by column chromatography (petroleum ether:ethyl acetate = 40:60) to give 2,6-dichloro-4-(2-hydroxyethyl)nicotinonitrile (800 mg, yield: 22.5%). ES-API: [M+H]⁺=217.0.

Step 3: Concentrated hydrochloric acid (20 mL) was added to the 2,6-dichloro-4-(2-hydroxyethyl)nicotinonitrile (800 mg, 3.7 mmol), and the system was heated to 100°C for 1 h. Upon completion of the reaction, the reaction mixture was concentrated, and the concentrated hydrochloric acid was removed. Ethyl acetate (50 mL) was added. The obtained mixture was washed with water (30 mL), saturated aqueous sodium bicarbonate solution (50 mL) and saturated brine (30 mL) successively, dried over anhydrous sodium sulfate, and purified by column chromatography (petroleum ether:ethyl acetate = 40:60) to give 6,8-dichloro-3,4-dihydro-1H-pyrano[3,4-c]pyridin-1-one (600 mg, yield: 75%). ES-API: [M+H]⁺=218.0.

Step 4: The 6,8-dichloro-3,4-dihydro-1H-pyrano[3,4-c]pyridin-1-one (600 mg, 2.76 mmol) was dissolved in tetrahydrofuran (10 mL) and anhydrous ethanol (5 mL), and the system was cooled in an ice-water bath. Sodium borohydride (525 mg, 13.8 mmol) was slowly added in batches, and the system was heated to 60°C for 0.5 h. Upon completion of the reaction, the reaction was quenched by the addition of ethyl acetate (20 mL). The reaction mixture was purified by column chromatography (petroleum ether:ethyl acetate = 10:90) to give 2-(2,6-dichloro-3-(hydroxymethyl)pyridin-4-yl)ethan-1-ol (500 mg, yield: 82%). ES-API: [M+H]⁺=222.

Step 5: The 2-(2,6-dichloro-3-(hydroxymethyl)pyridin-4-yl)ethan-1-ol (500 mg, 2.26 mmol) was dissolved in toluene (30 mL). p-Toluenesulfonic acid (778 mg, 4.52 mmol) was added, and the system was heated to 130°C for 18 h. Upon completion of the reaction, the reaction mixture was concentrated. The crude product was purified by column chromatography (petroleum ether:ethyl acetate = 30:70) to give 6,8-dichloro-3,4-dihydro-1H-pyrano[3,4-c]pyridine (360 mg, yield: 78%). ES-API: [M+H]⁺=204.0.

Step 6: The 6,8-dichloro-3,4-dihydro-1H-pyrano[3,4-c]pyridine (360 mg, 1.77 mmol) reacted with potassium vinyltrifluoroborate (237 mg, 1.77 mmol), [1,1'-bis(diphenylphosphino)ferrocene]dichloropalladium (72 mg, 0.088 mmol), triethylamine (536 mg, 5.31 mmol) and ethanol (9 mL) at 80°C for 1 h. Ethyl acetate (30 mL) was added. The obtained mixture was washed with water (50 mL) and saturated brine (50 mL) successively, dried over anhydrous sodium sulfate, and purified by column chromatography (petroleum ether:ethyl acetate = 40:60) to give a mixture of 6-chloro-8-vinyl-3,4-dihydro-1H-pyrano[3,4-c]pyridine and 8-chloro-6-vinyl-3,4-dihydro-1H-pyrano[3,4-c]pyridine (180 mg, yield: 52%). ES-API: [M+H]⁺=196.1.

Step 7: the above mixture (180 mg, 0.92 mmol) was dissolved in tetrahydrofuran (20 mL). An aqueous solution (15 mL) of potassium osmate dihydrate (169 mg, 0.459 mmol) and sodium periodate (197 g, 9.23 mmol) was added at room temperature, and the system reacted at room temperature for 2 h. The obtained mixture was diluted with ethyl acetate (30 mL) and cooled to 0°C. The reaction was quenched by the addition of aqueous sodium thiosulfate solution. The reaction mixture was filtered. The filtrate was washed with water (20 mL) and saturated brine (20 mL) successively, dried over anhydrous sodium sulfate, and purified by column chromatography (petroleum ether:ethyl acetate = 20:80) to give a mixture of 8-chloro-3,4-dihydro-1H-pyrano[3,4-c]pyridine-6-carbaldehyde and 6-chloro-3,4-dihydro-1H-pyrano[3,4-c]pyridine-8-carbaldehyde (100 mg, yield: 55.2%). ES-API: [M+H]⁺ = 198.0.

Step 8: the above mixture (100 mg, 0.51 mmol) was dissolved in acetonitrile (20 mL). 2M Dimethylamine in tetrahydrofuran (2.5 mL, 5.0 mmol), glacial acetic acid (0.2 mL) and sodium triacetoxyborohydride (268 mg, 1.265 mmol) were added successively, and the system reacted at room temperature for 18 h. The obtained mixture was diluted with ethyl acetate (30 mL), washed with saturated aqueous sodium bicarbonate solution (20 mL) and saturated brine (20 mL), dried over anhydrous sodium sulfate, and purified by preparative thin layer plate (ethyl acetate as a developing solvent) to give 1-(8-chloro-3,4-dihydro-1H-pyrano[3,4-c]pyridin-6-yl)-N,N-dimethylmethanamine (35 mg, Rf = 0.3, yield: 30%), ES-API: M+H]⁺= 227.0; 1-(6-chloro-3,4-dihydro-1H-pyrano[3,4-c]pyridin-8-yl)-N,N-dimethylmethanamine (40 mg, Rf = 0.5, yield: 34.7%). ES-API: [M+H]⁺= 227.0.

Step 9: The 1-(6-chloro-3,4-dihydro-1H-pyrano[3,4-c]pyridin-8-yl)-N,N-dimethylmethanamine (40 mg, 0.177 mg), 3-methyl-5-(4,4,5,5-tetramethyl-1,3,2-dioxaborolan-2-yl)-1H-pyrrolo[2,3-b]pyridine (68 mg, 0265 mmol), Sphos Pd G2 (12.7 mg, 0.0177 mmol) and potassium carbonate (73 mg, 0.531 mmol) were added to 1,4-dioxane (2 mL) and water (0.5 mL). The system was replaced with nitrogen and reacted under microwave at 110°C for 1 h. Upon completion of the reaction, ethyl acetate (20 mL) was added. The obtained mixture was washed with water (10 mL) and saturated brine (10 mL) successively, dried over anhydrous sodium sulfate, and purified by HPLC (ammonium bicarbonate method) to give N,N-dimethyl-1-(6-(3-methyl-1H-pyrrolo[2,3-b]pyridin-5-yl)-3,4-dihydro-1H-pyrano[3,4-c]pyridin-8-yl)methanamine (Z181, 6.5 mg, yield: 10.5%). ES-API: [M+H]⁺=323.0. ¹H NMR (500 MHz, DMSO-*d*₆) δ 11.38 (s, 1H), 8.91 (d, *J* = 2.0 Hz, 1H), 8.50 (d, *J* = 2.1 Hz, 1H), 7.75 (s, 1H), 7.26 (dd, *J* = 2.5, 1.3 Hz, 1H), 4.87 (s, 2H), 3.90 (t, *J* = 5.7 Hz, 2H), 3.52 (s, 2H), 2.90 (t, *J* = 5.8 Hz, 2H), 2.32 (d, *J* = 1.1 Hz, 3H), 2.18 (s, 6H).

### Example 36. Synthesis of compound Z229 and compound Z230

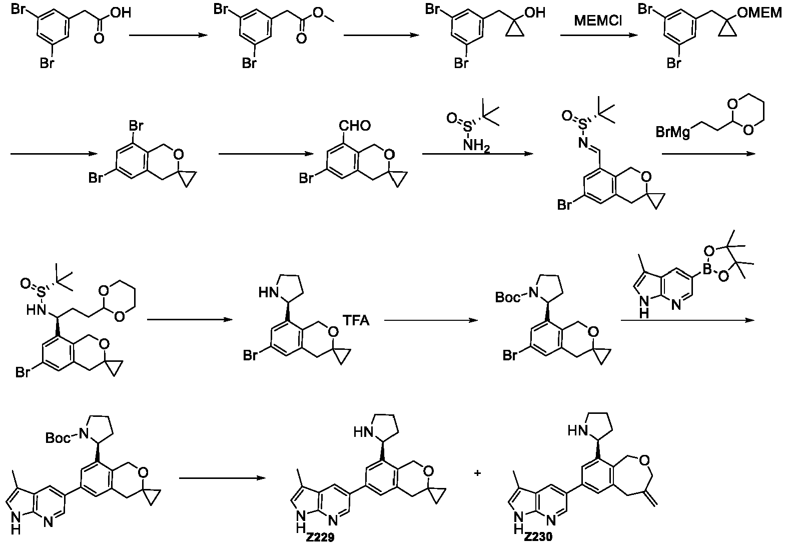

Step 1: 2-(3,5-Dibromophenyl)acetic acid (5.0 g, 17.0 mmol) was dissolved in methanol (50 mL). Thionyl chloride (2.5 mL, 34.0 mmol) was added dropwise at 0°C and the system was heated to reflux and stirred for 5 h. The reaction mixture was concentrated. Ethyl acetate (80 mL) was added. The obtained mixture was washed with saturated sodium bicarbonate solution (30 mL×2) and saturated brine (30 mL), dried over anhydrous sodium sulfate, and concentrated to give methyl 2-(3,5-dibromophenyl)acetate (5.2 g, yield: 99%) as a pale brown liquid. ES-API: [M+H]⁺=309.0.

Step 2: The methyl 2-(3,5-dibromophenyl)acetate (4.6 g, 14.94 mmol) and isopropyl titanate (848 mg, 2.99 mmol) were dissolved in tetrahydrofuran (20 mL). In the presence of protective nitrogen, 1M ethylmagnesium bromide in ethyl ether (41.8 mL, 41.80 mmol) was slowly added dropwise at 0°C, and the system continued to be stirred at 0°C for 1 h. 1M Sulfuric acid solution (40 mL) was added to the reaction mixture, and the obtained mixture was extracted with ethyl acetate (100 mL×2). The organic phases were combined, washed with saturated sodium bicarbonate solution (50 mL) and saturated brine (50 mL) successively, dried over anhydrous sodium sulfate, and concentrated. The crude product was purified by a flash silica gel column (ethyl acetate/petroleum ether: 0-10%) to give 1-(3,5-dibromobenzyl)cyclopropan-1-ol (2.2 g, yield: 48%) as a white solid. ¹H NMR (500 MHz, DMSO-*d₆*) δ 7.66 (t, *J* = 1.5 Hz, 1H), 7.51 (d, *J* = 1.5 Hz, 2H), 5.24 (s, 1H), 2.74 (s, 2H), 0.62 - 0.55 (m, 2H), 0.55- 0.50 (m, 2H). ES-API: [M+H]⁺=306.9.

Step 3: The 1-(3,5-dibromobenzyl)cyclopropan-1-ol (2.05 g, 6.70 mmol) and N,N-diisopropylethyl amine (3.02 g, 23.45 mmol) were dissolved in dichloromethane (15 mL). 1-(Chloromethoxy)-2-methoxyethane (2.49 g, 20.10 mmol) was added dropwise at 0°C and stirred at room temperature for 72 h. Water (15 mL) was added to the reaction mixture, and the obtained mixture was extracted with dichloromethane (50 mL×2). The organic phases were combined, dried over anhydrous sodium sulfate, and concentrated. The crude product was purified by a flash silica gel column (tetrahydrofuran/petroleum ether: 5-10%) to give 1,3-dibromo-5-((1-((2-methoxyethoxy)methoxy)cyclopropyl)methyl)benzene (1.35 g, yield: 51%) as a colorless liquid. ES-API: [M+Na]⁺=417.0.

Step 4: The 1,3-dibromo-5-((1-((2-methoxyethoxy)methoxy)cyclopropyl)methyl)benzene (2.1 g, 5.32 mmol) was dissolved in dichloromethane (4 mL). In the presence of protective nitrogen, titanium tetrachloride in dichloromethane (8.0 mL, 8.0 mmol, 1.0M) was added dropwise at 0°C and stirred at 0°C for 1 h. Methanol (1.5 mL) and 1M saturated sodium bicarbonate solution (20 mL) were added dropwise to the reaction mixture, and the obtained mixture was extracted with dichloromethane (50 mL). The organic phase was washed with saturated brine (25 mL×2), dried over anhydrous sodium sulfate, and concentrated. The crude product was purified by a flash silica gel column (ethyl acetate/petroleum ether: 0-5%) to give 6',8'-dibromospiro[cyclopropane-1,3'-isochroman] (1.5 g, yield: 88%) as a pale yellow liquid. ¹H NMR (500 MHz, CDCl₃) δ 7.55 (d, *J =* 2.0 Hz, 1H), 7.22 (d, *J* = 2.0 Hz, 1H), 4.61 (s, 2H), 2.84 (s, 2H), 0.96 - 0.89 (m, 2H), 0.57 - 0.50 (m, 2H). ES-API: [M+H]⁺=318.9.

Step 5: The 6',8'-dibromospiro[cyclopropane-1,3'-isochroman] (1.0 g, 3.14 mmol) was dissolved in tetrahydrofuran (10 mL). In the presence of protective nitrogen, n-butyllithium in n-hexane (1.25 mL, 3.14 mmol, 2.5M) was slowly added dropwise at -78°C and stirred at -78°C for 2 h. Ethyl formate (377 mg, 6.28 mmol) was then added dropwise and stirred at -78°C for 45 min. A dry ice-acetone bath was removed, and the system continued to be stirred for 15 min. The reaction was quenched by the addition of water (10 mL), and the reaction mixture was extracted with ethyl acetate (50 mL). The organic phase was dried over anhydrous sodium sulfate, and concentrated. The crude product was purified by a flash silica gel column (ethyl acetate/petroleum ether: 0-5%) to give 6'-bromospiro[cyclopropane-1,3'-isochroman]-8'-carbaldehyde (230 mg, yield: 27%) as a colorless liquid. ES-API: [M+H]⁺=267.0.

Step 6: The 6'-bromospiro[cyclopropane-1,3'-isochroman]-8'-carbaldehyde (275 mg, 1.03 mmol) and (S)-2-methylpropane-2-sulfinamide (249 mg, 2.06 mmol) were dissolved in dichloromethane (15 mL). Tetraethoxytitanium (587 mg, 2.58 mmol) was added and stirred at room temperature for 3 h. Saturated brine (30 mL) was added to the reaction mixture, and the obtained mixture was extracted with ethyl acetate (50 mL×2). The organic phases were combined, washed with saturated brine (30 mL), dried over anhydrous sodium sulfate, and concentrated. The crude product was purified by a flash silica gel column (ethyl acetate/petroleum ether: 0-20%) to give (*S*,*E*)-*N*-((6'-bromospiro[cyclopropane-1,3'-isochroman]-8'-yl)methylene)-2-methylpropa ne-2-sulfinamide (380 mg, yield: 100%) as a viscous liquid. ES-API: [M+H]⁺=370.1, 372.0.

Step 7: The (*S*,*E*)-*N*-((6'-bromospiro[cyclopropane-1,3'-isochroman]-8'-yl)methylene)-2-methylpropa ne-2-sulfinamide (350 mg, 0.95 mmol) was dissolved in tetrahydrofuran (7 mL). In the presence of protective nitrogen, (2-(1,3-dioxan-2-yl)ethyl)magnesium bromide in tetrahydrofuran (5.7 mL, 2.85 mmol, 0.5M) was added dropwise at -78°C and stirred at -78°C for 30 min. The reaction was quenched with saturated ammonium chloride solution (10 mL), and water (10 mL) was added. The obtained mixture was extracted with ethyl acetate (50 mL). The organic phase was washed with saturated brine (25 mL), dried over anhydrous sodium sulfate, and concentrated to give (*S*)-*N*-((*S*)-1-(6'-bromospiro[cyclopropane-1,3'-isochroman]-8'-yl)-3-(1,3-dioxan-2-yl)pro pyl)-2-methylpropane-2-sulfinamide (460 mg, yield: 100%) as a viscous liquid. ES-API: [M+H]⁺=486.2, 488.1.

Step 8: Trifluoroacetic acid (20 mL) and water (1 mL) were cooled to 0°C. The above solution was added dropwise to the (*S*)-*N*-((*S*)-1-(6'-bromospiro[cyclopropane-1,3'-isochroman]-8'-yl)-3-(1,3-dioxan-2-yl)pro pyl)-2-methylpropane-2-sulfinamide (460 mg, 0.95 mmol) and stirred at room temperature for 45 min. Triethylsilane (1.1 g, 9.50 mmol) was added and stirred at room temperature for 16 h. The reaction mixture was concentrated to give (S)-2-(6'-bromospiro[cyclopropane-1,3'-isochroman]-8'-yl)pyrrole trifluoroacetate (1.7 g, crude product) which was directly used in the next step without further purification. ES-API: [M+H]⁺=308.0, 310.0 (free base).

Step 9: The (S)-2-(6'-bromospiro[cyclopropane-1,3'-isochroman]-8'-yl)pyrrole trifluoroacetate (1.7 g, crude product) was dissolved in dichloromethane (10 mL). Triethylamine (384 mg, 3.80 mmol) and di-tert-butyl dicarbonate (414 mg, 1.90 mmol) were added at 0°C and stirred at room temperature for 1 h. Dichloromethane (40 mL) was added to the reaction mixture. The obtained mixture was washed with water (15 mL) and saturated brine (15 mL) successively, dried over anhydrous sodium sulfate, and concentrated. The crude product was purified by a flash silica gel column (tetrahydrofuran/petroleum ether: 0-10%) to give tert-butyl (S)-2-(6'-bromospiro[cyclopropane-1,3'-isochroman]-8'-yl)pyrrolidine-1-carboxylate (300 mg, yield of the 2 steps: 73%) as a viscous liquid. ES-API: [M+H]⁺=430.1, 432.0.

Step 10: The tert-butyl (S)-2-(6'-bromospiro[cyclopropane-1,3'-isochroman]-8'-yl)pyrrolidine-1-carboxylate (60 mg, 0.15 mmol), 3-methyl-5-(4,4,5,5-tetramethyl-1,3,2-dioxaborolan-2-yl)-1*H*-pyrrolo[2,3-*b*]pyridine (49 mg, 0.19 mmol), potassium carbonate (62 mg, 0.45 mmol), 2-dicyclohexylphosphine-2',6'-dimethoxy-biphenyl (6 mg, 0.015 mmol), chloro (2-dicyclohexylphosphino-2',6'-dimethoxy-1,1'-biphenylyl)(2'-amino-1,1'-biphenyl-2-yl) palladium (II) (11 mg, 0.015 mmol), 1,4-dioxane (2 mL) and water (0.4 mL) were added to a 5 mL microwave tube. The mixture was purged with nitrogen for 1 min, and the system reacted in a microwave reactor at 110°C for 45 min. Water (5 mL) was added to the reaction mixture, and the obtained mixture was extracted with ethyl acetate (30 mL). The organic phase was washed with saturated brine (10 mL), dried over anhydrous sodium sulfate, and concentrated. The crude product was purified by a flash silica gel column (methanol/dichloromethane: 0-3%) to give tert-butyl (S)-2-(6'-(3-methyl-1H-pyrrolo[2,3-b]pyridin-5-yl)spiro[cyclopropane-1,3'-isochroman]-8'-yl)pyrrolidine-1-carboxylate (68 mg, yield: 100%) as a white solid. ES-API: [M+H]⁺=460.3.

Step 11: The tert-butyl (S)-2-(6'-(3-methyl-1H-pyrrolo[2,3-b]pyridin-5-yl)spiro[cyclopropane-1,3'-isochroman]-8'-yl)pyrrolidine-1-carboxylate (68 mg, 0.15 mmol) was dissolved in methanol (1 mL). 4M Hydrochloric acid/dioxane solution (3 mL) was added and stirred at room temperature for 1 h. The reaction mixture was concentrated. 7.0M Ammonia/methanol solution (4 mL) was added, and a solvent was spun to dryness. The crude product was purified by prep-HPLC (formic acid method) followed by chiral prep-HPLC (separation column: Daicel CHIRALPAK^{®} IC 250*4.6 mm, 5 µm, mobile phase: n-hexane (0.2% DEA):isopropyl alcohol (0.2% DEA) = 50:50, flow rate: 1 ml/min, column temperature: 30°C) to give (S)-3-methyl-5-(8'-(pyrrolidin-2-yl)spiro[cyclopropane-1,3'-isochroman]-6'-yl)-1H-pyrrol o[2,3-*b*]pyridine (Z229, 5 mg, yield: 9%) as a pale pink solid. ¹H NMR (500 MHz, DMSO-*d₆*) δ 11.32 (s, 1H), 8.45 (d, *J* = 2.0 Hz, 1H), 8.09 (d, *J* = 2.0 Hz, 1H), 7.71 (d, *J* = 1.5 Hz, 1H), 7.32 (d, *J=* 1.5 Hz, 1H), 7.22 (q, *J=* 1.0 Hz, 1H), 4.88 - 4.64 (m, 2H), 4.09 (t, *J* = 7.5 Hz, 1H), 3.12 - 3.07 (m, 1H), 3.01 - 2.83 (m, 3H), 2.32 (d, *J=* 1.0 Hz, 3H), 2.21 - 2.11 (m, 1H), 1.93 - 1.71 (m, 2H), 1.51 - 1.41 (m, 1H), 0.88 - 0.76 (m, 2H), 0.60 - 0.47 (m, 2H). ES-API: [M+H]⁺=360.2; (*S*)-3-methyl-5-(4-methylene-9-(pyrrolidin-2-yl)-1,3,4,5-tetrahydrobenzo[*c*]oxepin-7-yl)-1 H-pyrrolo[2,3-b]pyridine (Z230, 7 mg, yield: 13%) as a pale pink solid. ¹H NMR (500 MHz, DMSO-*d₆*) δ 11.34 (s, 1H), 8.47 (d, *J =* 2.0 Hz, 1H), 8.11 (d, *J =* 2.0 Hz, 1H), 7.74 (d, *J* = 2.0 Hz, 1H), 7.47 (d, *J* = 1.6 Hz, 1H), 7.28 - 7.24 (m, 1H), 5.02 - 4.77 (m, 4H), 4.37 - 4.27 (m, 3H), 3.79 - 3.70 (m, 2H), 3.12 - 3.03 (m, 1H), 2.95 - 2.90 (m, 1H), 2.31 (d, *J* = 1.0 Hz, 3H), 2.17 - 2.09 (m, 1H), 1.86 - 1.70 (m, 2H), 1.49 - 1.41 (m, 1H). ES-API: [M+H]⁺=360.2.

### Example 37. Synthesis of compound Z234-1 and compound Z234-2

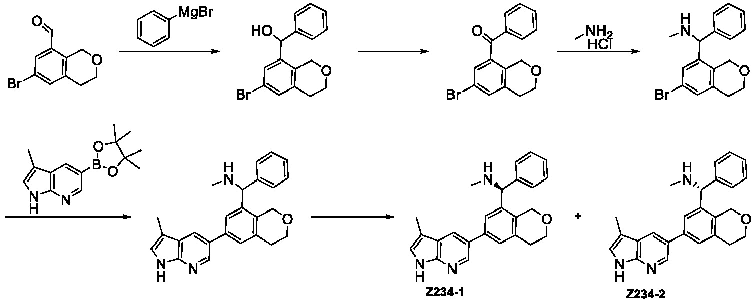

Step 1: In the presence of protective nitrogen, 6-bromoisochroman-8-carbaldehyde (300 mg, 1.24 mmol) in tetrahydrofuran (5 mL) was cooled to 0°C. Phenylmagnesium chloride in tetrahydrofuran (1.86 mL, 1.86 mmol, 1M) was slowly added, and the system was slowly warmed to room temperature and stirred for 2 h. The reaction was quenched with saturated ammonium chloride solution (5 mL), and the reaction mixture was extracted with ethyl acetate (5 mLX3). The organic phases were combined, dried over anhydrous sodium sulfate, filtered, and concentrated. The crude product was purified by a flash silica gel column (0-50% ethyl acetate/petroleum ether) to give (6-bromoisochroman-8-yl)(phenyl)methanol (310 mg, yield: 78%) as a colorless liquid. ES-API: [M+H⁺-H₂O] = 301.0, 303.0.

Step 2: Dess-Martin periodinane (598 mg, 1.41 mmol) was slowly added to a solution of the (6-bromoisochroman-8-yl)(phenyl)methanol (150 mg, 0.47 mmol) in dichloromethane (10 mL) under an ice bath condition and stirred at room temperature for 2 h. The reaction was quenched with sodium thiosulfate solution (10 mL) and sodium bicarbonate solution (10 mL) successively, and the reaction mixture was extracted with dichloromethane (15 mLX3). The organic phases were combined, dried over anhydrous sodium sulfate, filtered, and concentrated. The crude product was purified by a flash silica gel column (0-50% ethyl acetate/petroleum ether) to give (6-bromoisochroman-8-yl)(phenyl)methanone (120 mg, yield: 80%). ES-API: [M+H] ⁺ = 317.1, 319.0.

Step 3: Methylamine hydrochloride (128 mg, 1.89 mmol) and titanium tetraisopropoxide (108 mg, 0.38 mmol) were added to a solution of the (6-bromoisochroman-8-yl)(phenyl)methanone (120 mg, 0.38 mmol) in 1,2-dichloroethane (5 mL), and the mixture was stirred at room temperature overnight. Sodium cyanoborohydride (71 mg, 1.13 mmol) was then added and stirred at room temperature for 2 h. Upon completion of the reaction, the reaction was quenched with aqueous ammonium chloride solution (5 mL), and the reaction mixture was extracted with dichloromethane (5 mLX3). The organic phases were combined, dried over anhydrous sodium sulfate, filtered, and concentrated. The crude product was purified by a flash silica gel column (0-6% methanol (1% ammonia water)/dichloromethane) to give 1-(6-bromoisochroman-8-yl)-N-methyl-1-phenylmethanamine (80 mg, yield: 64%) as a colorless liquid. ES-API: [M+H]⁺ = 332.0, 334.1.

Step 4: In the presence of protective nitrogen, a mixed solution of 3-methyl-5-(4,4,5,5-tetramethyl-1,3,2-dioxaborolan-2-yl)-1H-pyrrolo[2,3-b]pyridine (68 mg, 0.26 mmol), the 1-(6-bromoisochroman-8-yl)-N-methyl-1-phenylmethanamine (80 mg, 0.24 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol), 2-dicyclohexylphosphine-2',6'-dimethoxybiphenyl (8 mg, 0.02 mmol) and potassium carbonate (99 mg, 0.72 mmol) in 1,4-dioxane (2 mL) and water (0.4 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, and concentrated. The crude product was purified by prep-HPLC (ammonium bicarbonate method) to give *N*-methyl-1-(6-(3-methyl-1*H*-pyrrolo[2,3-*b*]pyridin-5-yl)isochroman-8-yl)-1-phenylmetha namine (60 mg, yield: 65%) as a white solid. ES-API: [M+H]⁺ = 384.3.

Step 5: The *N*-methyl-1-(6-(3-methyl-1*H*-pyrrolo[2,3-*b*]pyridin-5-yl)isochroman-8-yl)-1-phenylmetha namine (60 mg, 0.16 mmol) was chirally resolved (column type: Chiralpak IG: 5 µm, 4.6*250 mm, mobile phase: n-hexane (0.2% trifluoroacetic acid):ethanol (0.2% trifluoroacetic acid) = 70:30, flow rate: 1 mL/min, column temperature: room temperature) to give two isomeric compounds. A structure of one isomeric compound was arbitrarily assigned as (*S*)-*N*-methyl-1-(6-(3-methyl-1*H*-pyrrolo[2,3-*b*]pyridin-5-yl)isochroman-8-yl)-1-phenylm ethanamine (Z234-1, trifluoroacetate, 36 mg, yield: 46%, retention time: 8.290 min, ee value: 100%). ES-API: [M+H]⁺ =384.3. ¹H NMR (500 MHz, DMSO-d₆) δ 11.47 (s, 1H), 9.70 (s, 2H), 8.61 (d, *J =* 2.0 Hz, 1H), 8.27 (d, *J =* 2.0 Hz, 1H), 7.97 (s, 1H), 7.62 (s, 1H), 7.58 - 7.53 (m, 2H), 7.51 - 7.46 (m, 2H), 7.45 - 7.40 (m, 1H), 7.33 - 7.31 (m, 1H), 5.53 (t, *J =* 6.0 Hz, 1H), 5.10 (d, *J* = 15.5 Hz, 1H), 4.32 (d, *J* = 15.5 Hz, 1H), 3.96 - 3.87 (m, 1H), 3.80 - 3.68 (m, 1H), 2.99 - 2.90 (m, 1H), 2.88 - 2.79 (m, 1H), 2.61 (t, *J* = 5.0 Hz, 3H), 2.34 (d, *J* = 1.0 Hz, 3H); a structure of the other isomeric compound was arbitrarily assigned as (R)-*N*-methyl-1-(6-(3-methyl-1*H*-pyrrolo[2,3-*b*]pyridin-5-yl)isochroman-8-yl)-1-phenylm ethanamine (Z234-2, trifluoroacetate, 23 mg, yield: 30%, retention time: 9.857 min, ee value: 100%). ES-API: [M+H]⁺ = 384.3. ¹H NMR (500 MHz, DMSO-d₆) δ 11.44 (s, 1H), 9.69 (s, 2H), 8.61 (d, *J =* 2.0 Hz, 1H), 8.25 (d, *J =* 2.0 Hz, 1H), 7.97 (s, 1H), 7.61 (s, 1H), 7.57 - 7.53 (m, 2H), 7.50 - 7.45 (m, 2H), 7.44 - 7.40 (m, 1H), 7.31 (s, 1H), 5.52 (d, *J =* 6.5 Hz, 1H), 5.10 (d, *J* = 15.5 Hz, 1H), 4.32 (d, *J* = 15.5 Hz, 2H), 3.95 - 3.89 (m, 1H), 3.78 - 3.69 (m, 1H), 2.98 - 2.91 (m, 1H), 2.89 - 2.80 (m, 1H), 2.61 (t, *J* = 5.0 Hz, 4H), 2.34 (d, *J* = 1.0 Hz, 3H).

### Example 38. Synthesis of compound Z227

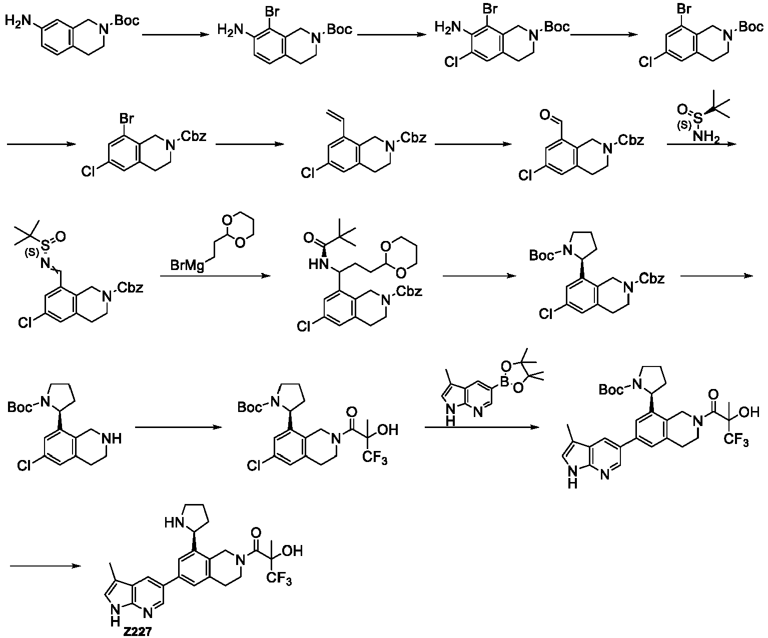

Step 1: Tert-butyl 7-amino-3,4-dihydroisoquinoline-2(1H)-carboxylate (7 g, 28.2 mmol) was dissolved in acetonitrile (150 mL), and the system was cooled to 0°C in an ice-water bath. N-Bromosuccinimide (5.02 g, 28.2 mmol) was slowly added in batches, and the system reacted under the ice-water bath condition for 2 h. Upon completion of the reaction, the reaction mixture was diluted with ethyl acetate (200 mL), washed with saturated aqueous sodium bicarbonate solution (150 mL) and saturated brine (150 mL) successively, dried over anhydrous sodium sulfate, filtered, and concentrated. The crude product was purified by column chromatography (petroleum ether:ethyl acetate = 80:20) to give tert-butyl 7-amino-8-bromo-3,4-dihydroisoquinoline-2(1H)-carboxylate (8.2 g, yield: 88%). ES-API: [M+H]⁺=327.0, 329.0.

Step 2: The tert-butyl 7-amino-8-bromo-3,4-dihydroisoquinoline-2(1H)-carboxylate (7.4 g, 22.6 mmol) was dissolved in N,N-dimethylformamide (150 mL), and the system was cooled to 0°C in an ice-water bath. N-Chlorosuccinimide (3.16 g, 23.7 mmol) was slowly added in batches, and the system was heated to 50°C for 0.5 h. Upon completion of the reaction, the reaction mixture was diluted with ethyl acetate (200 mL), washed with saturated aqueous sodium bicarbonate solution (150 mL) and saturated brine (150 mL) successively, dried over anhydrous sodium sulfate, filtered, and concentrated. The crude product was purified by column chromatography (petroleum ether:ethyl acetate = 80:20) to give tert-butyl 7-amino-6-chloro-8-bromo-3,4-dihydroisoquinoline-2(1H)-carboxylate (5.0 g, yield: 61%). ES-API: [M+H]+=305, 307.

Step 3: The tert-butyl 7-amino-6-chloro-8-bromo-3,4-dihydroisoquinoline-2(1H)-carboxylate (5.0 g, 13.85 mmol) was dissolved in tetrahydrofuran (50 mL). Water (10 mL) and hypophosphorous acid (25 mL, 50% aqueous solution) were added successively, and the system was cooled to 0°C. Sodium nitrite (1.91 g, 27.7 mmol) was slowly added, and the system react at room temperature for 0.5 h. Upon completion of the reaction, the reaction mixture was poured into ice water, and ethyl acetate (50 mL) was added. The obtained mixture was washed with saturated aqueous sodium bicarbonate solution (50 mL) and saturated brine (50 mL) successively, dried over anhydrous sodium sulfate, filtered, and concentrated to give tert-butyl 6-chloro-8-bromo-3,4-dihydroisoquinoline-2(1H)-carboxylate (3.34 g, yield: 70%), ES-API: [M+H]⁺=346.1, 348.1.

Step 4: The tert-butyl 6-chloro-8-bromo-3,4-dihydroisoquinoline-2(1H)-carboxylate (3.34 g, 9.68 mmol) was dissolved in dichloromethane (20 mL). Trifluoroacetic acid (10 mL) was added, and the system reacted at room temperature for 2 h. Upon completion of the reaction, the reaction mixture was concentrated to remove an excess of trifluoroacetic acid. Tetrahydrofuran (50 mL) was added, and the system was cooled to 0°C. Triethylamine (7 g, 29.04 mmoL) and benzyl chloroformate (2.48 g, 14.52 mmoL) were added successively, and the system was reacted at room temperature for 2 h. Upon completion of the reaction, ethyl acetate (50 mL) was added. The obtained mixture was washed with water (50 mL) and saturated brine (50 mL) successively, dried over anhydrous sodium sulfate, filtered, and concentrated. The crude product was purified by column chromatography (petroleum ether:ethyl acetate = 50:50) to give benzyl 8-bromo-6-chloro-3,4-dihydroisoquinoline-2(1H)-carboxylate (3.65 g, yield: 99%), ES-API: [M+H]⁺=380.0, 382.0.

Step 5: The benzyl 8-bromo-6-chloro-3,4-dihydroisoquinoline-2(1H)-carboxylate (3.4 g, 8.97 mmol) reacted with potassium vinyltrifluoroborate (2.38 g, 17.94 mmol), [1,1'-bis(diphenylphosphino)ferrocene]dichloropalladium (366 mg, 0.448 mmol), triethylamine (1.81 g, 17.94 mmol) and ethanol (60 mL) at 90°C for 3 h. Ethyl acetate (100 mL) was added. The obtained mixture was washed with water (50 mL) and saturated brine (50mL) successively, dried over anhydrous sodium sulfate, and purified by column chromatography (petroleum ether:ethyl acetate = 50:50) to give benzyl 6-chloro-8-vinyl-3,4-dihydroisoquinoline-2(1H)-carboxylate (2 g, yield: 68%). ES-API: [M+H]⁺=328.1.

Step 6: The benzyl 6-chloro-8-vinyl-3,4-dihydroisoquinoline-2(1H)-carboxylate (2 g, 6.11 mmol) was dissolved in tetrahydrofuran (40 mL) and acetonitrile (20 mL). An aqueous solution (10 mL) of potassium osmate dihydrate (225 mg, 0.611 mmol) and sodium periodate (7.85 g, 36.7 mmol) was added, and the system reacted at room temperature for 2 h and then was cooled to 0°C. The reaction was quenched by the addition of aqueous sodium thiosulfate solution. Ethyl acetate (100 mL) was added. The obtained mixture was washed with water (50 mL) and saturated brine (50 mL) successively, dried over anhydrous sodium sulfate, and purified by column chromatography (petroleum ether:ethyl acetate = 20:80) to give benzyl 6-chloro-8-formyl-3,4-dihydroisoquinoline-2(1H)-carboxylate (1.1 g, yield: 55%). ES-API: [M+H]⁺ = 330.0.

Step 7: The benzyl 6-chloro-8-formyl-3,4-dihydroisoquinoline-2(1H)-carboxylate (720 mg, 2.18 mmol) and (S)-tert-butylsulfinamide (529 mg, 4.37 mmol) were dissolved in dried dichloromethane (15 mL). Tetraethoxytitanium (1.99 g, 8.75 mmol) was added, and the system reacted at room temperature for 15 h. Upon completion of the reaction, the reaction mixture was slowly poured into saturated brine (20 mL). Dichloromethane (30 mL) was added. The organic layer was separated, filtered, dried over anhydrous sodium sulfate, and purified by column chromatography (ethyl acetate: petroleum ether = 2:1) to give (S)-benzyl 8-(((tert-butylsulfinyl)imino)methyl)-6-chloro-3,4-dihydroisoquinoline-2(1H)-carboxylate (800 mg, yield: 85%). ES-API: [M+H]⁺ = 433.1.

Step 8: The (S)-benzyl 8-(((tert-butylsulfinyl)imino)methyl)-6-chloro-3,4-dihydroisoquinoline-2(1H)-carboxylate (800 mg, 1.85 mmol) and dried tetrahydrofuran (20 mL) were added to a 100 ml three-necked flask. (2-(1,3-Dioxan-2-yl)ethyl)magnesium bromide in tetrahydrofuran (14.5 mL, 0.5M) was slowly added under a dry ice bath condition at -78°C in the presence of protective nitrogen and stirred for 10 min. Upon completion of the reaction, the reaction was quenched by the addition of 1M hydrochloric acid (8 mL). Ethyl acetate (50 mL) was added. The obtained mixture was washed with water (30 mL) and saturated brine (30 mL) successively, dried over anhydrous sodium sulfate, and purified by column chromatography (ethyl acetate:petroleum ether = 9:1) to give benzyl 8-(1-(((S)-tert-butylsulfinyl)amino)-3-(1,3-dioxan-2-yl)propyl)-6-chloro-3,4-dihydroisoqu inoline-2(1H)-carboxylate (950 mg, yield: 94%). ES-API: [M+H]⁺ = 549.1.

Step 9: The benzyl 8-(1-(((S)-tert-butylsulfinyl)amino)-3-(1,3-dioxan-2-yl)propyl)-6-chloro-3,4-dihydroisoqu inoline-2(1H)-carboxylate (950 mg, 1.73 mmol) was added to trifluoroacetic acid/water (10 mL/0.5 mL), and the system reacted at room temperature (<25°C) for 30 min. Triethylsilane (2 g, 17.3 mmol) was added, and the system reacted for 2 h. After concentration, the concentrate was dissolved in tetrahydrofuran. Triethylamine (524 g, 5.19 mmol) and di-tert-butyl dicarbonate (564 mg, 2.59 mmol) were added. Upon completion of the reaction, ethyl acetate (50 mL) was added. The obtained mixture was washed with water (30 mL) and saturated brine (30 mL) successively, dried over anhydrous sodium sulfate, and purified by column chromatography (ethyl acetate: petroleum ether = 3:1) to give (S)-benzyl 8-(1-(tert-butoxycarbonyl)pyrrolidin-2-yl)-6-chloro-3,4-dihydroisoquinoline-2(1H)-carbox ylate (220 mg, yield: 25%). ES-API: [M+H]⁺ = 471.2.

Step 10: The (S)-benzyl 8-(1-(tert-butoxycarbonyl)pyrrolidin-2-yl)-6-chloro-3,4-dihydroisoquinoline-2(1H)-carbox ylate (220 mg, 0.468 mmoL), palladium chloride (9.94 mg, 0.0561 mmoL) and triethylamine (47.3 mg, 0.468 mg) were dissolved in dichloromethane (15 mL). Triethylsilane (217 mg, 1.87 mmoL) was slowly added dropwise. The system reacted at room temperature for 1 h. Upon completion of the reaction, dichloromethane (50 mL) was added. The obtained mixture was washed with water (30 mL) and saturated brine (30 mL) successively, dried over anhydrous sodium sulfate, and purified by column chromatography (dichloromethane:methanol = 10:1) to give tert-butyl (S)-2-(6-chloro-1,2,3,4-tetrahydroisoquinolin-8-yl)pyrrolidine-1-carboxylate (125 mg, yield: 80%). ES-API: [M+H]⁺ = 337.0.

Step 11: The tert-butyl (S)-2-(6-chloro-1,2,3,4-tetrahydroisoquinolin-8-yl)pyrrolidine-1-carboxylate (125 mg, 0.372 mmol) was dissolved in dried dichloromethane (5 mL). 3,3,3-Trifluoro-2-hydroxy-2-methylpropanoic acid (117.5 mg, 0.744 mmol), triethylamine (112 mg, 1.11 mmol) and 2-(7-azobenzotriazol)-N,N,N',N'-tetramethylurea hexafluorophosphate (283 mg, 0.744 mmol) were added successively, and the system reacted at room temperature for 1 h. Upon completion of the reaction, the reaction mixture was diluted with dichloromethane (30 mL), washed with saturated brine (30 mL), dried over anhydrous sodium sulfate, filtered, concentrated, and purified by column chromatography (ethyl acetate:petroleum ether = 3:1) to give (2S)-tert-butyl 2-(6-chloro-2-(3,3,3-trifluoro-2-hydroxy-2-methylpropanoyl)-1,2,3,4-tetrahydroisoquinoli n-8-yl)pyrrolidine-1-carboxylate (80 mg, yield: 45%). ES-API: [M+H]⁺=477.0.

Step 12: The (2S)-tert-butyl 2-(6-chloro-2-(3,3,3-trifluoro-2-hydroxy-2-methylpropanoyl)-1,2,3,4-tetrahydroisoquinoli n-8-yl)pyrrolidine-1-carboxylate (80 mg, 0.168 mmol), 3-methyl-5-(4,4,5,5-tetramethyl-1,3,2-dioxaborolan-2-yl)-1H-pyrrolo[2,3-b]pyridine (65 mg, 0.252 mmol), Sphos Pd G2 (12.0 mg, 0.0168 mmol) and potassium carbonate (69.5 mg, 0.504 mmol) were added to 1,4-dioxane (3 mL) and water (0.5 mL). The system was replaced with nitrogen and reacted under microwave at 110°C for 1 h. Upon completion of the reaction, ethyl acetate (20 mL) was added. The obtained mixture was washed with water (10 mL) and saturated brine (10 mL) successively, dried over anhydrous sodium sulfate, and purified by preparative thin layer chromatography (petroleum ether/ethyl acetate = 1/1) to give (2S)-tert-butyl 2-(6-(3-methyl-1H-pyrrolo[2,3-b]pyridin-5-yl)-2-(3,3,3-trifluoro-2-hydroxy-2-methylprop anoyl)-1,2,3,4-tetrahydroisoquinolin-8-yl)pyrrolidine-1-carboxylate (40 mg, yield: 41%). ES-API: [M+H]+=573.1.

Step 13: The (2S)-tert-butyl 2-(6-(3-methyl-1H-pyrrolo[2,3-b]pyridin-5-yl)-2-(3,3,3-trifluoro-2-hydroxy-2-methylprop anoyl)-1,2,3,4-tetrahydroisoquinolin-8-yl)pyrrolidine-1-carboxylate (40 mg, 0.07 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia/methanol solution (1 mL), the obtained mixture was concentrated again and purified by prep-HPLC (ammonia water method) to give 3,3,3-trifluoro-2-hydroxy-2-methyl-1-(6-(3-methyl-1H-pyrrolo[2,3-b]pyridin-5-yl)-8-((S)-pyrrolidin-2-yl)-3,4-dihydroisoquinolin-2(1H)-yl)propan-1-one (Z227, 15 mg, yield: 45%). ES-API: [M+H]⁺=473.2. ¹H NMR (500 MHz, DMSO-*d*₆) δ 11.34 (s, 1H), 8.47 (d, *J* = 2.2 Hz, 1H), 8.11 (d, *J* = 2.2 Hz, 1H), 7.77 (d, *J=* 2.0 Hz, 1H), 7.41 (d, *J* = 10.1 Hz, 1H), 7.26 (s, 1H), 7.20 (s, 1H), 5.32 (t, *J* = 4.8 Hz, 1H), 4.85 (d, *J* = 17.1 Hz, 1H), 4.65 (d, *J* = 17.6 Hz, 1H), 4.26 (s, 1H), 3.17 - 2.90 (m, 2H), 2.34 - 2.29 (m, 3H), 2.27 - 2.19 (m, 1H), 1.99 (dt, *J* = 17.1, 6.9 Hz, 2H), 1.88 - 1.83 (m, 1H), 1.57 (d, *J* = 24.3 Hz, 3H).

### Example 39. Synthesis of compound Z231

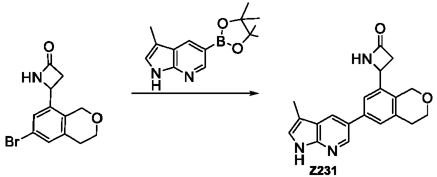

Step 1: 4-(6-bromoisochroman-8-yl)azetidin-2-one (30 mg, 0.11 mmol), 3-methyl-5-(4,4,5,5-tetramethyl-1,3,2-dioxaborolan-2-yl)-1*H*-pyrrolo[2,3-*b*]pyridine (33 mg, 0.13 mmol), sodium carbonate (35 mg, 0.33 mmol), 2-dicyclohexylphosphine-2',6'-dimethoxy-biphenyl (4 mg, 0.01 mmol), chloro (2-dicyclohexylphosphino-2',6'-dimethoxy-1,1'-biphenylyl)(2'-amino-1,1'-biphenyl-2-yl) palladium (II) (7 mg, 0.01 mmol), 1,4-dioxane (1.5 mL) and water (0.3 mL) were added to a 5 mL microwave tube. The mixture was purged with nitrogen for 1 min, and the system reacted in a microwave reactor at 110°C for 45 min. Water (5 mL) was added to the reaction mixture, and the obtained mixture was extracted with ethyl acetate (30 mL). The organic phase was washed with saturated brine (10 mL), dried over anhydrous sodium sulfate, and concentrated. The crude product was purified by prep-HPLC (ammonium bicarbonate method) to give 4-(6-(3-methyl-1H-pyrrolo[2,3-b]pyridin-5-yl)isochroman-8-yl)azetidin-2-one (Z231, 11 mg, yield: 31%) as a white solid, ES-API: [M+H]⁺=334.1. ¹H NMR (500 MHz, DMSO-*d₆*) δ 11.36 (s, 1H), 8.57 - 8.44 (m, 2H), 8.14 (d, *J* = 2.0 Hz, 1H), 7.54 (s, 1H), 7.46 (s, 1H), 7.27 (s, 1H), 4.82 (d, *J* = 15.0 Hz, 1H), 4.69 (dd, *J* = 4.0, 2.0 Hz, 1H), 4.55 (d, *J* = 15.0 Hz, 1H), 3.97 - 3.80 (m, 2H), 3.44 - 3.37 (m, 1H), 2.98 - 2.82 (m, 2H), 2.63 (dd, *J* = 14.5, 2.0 Hz, 1H), 2.31 (d, *J* = 1.0 Hz, 3H).

### Example 40. Synthesis of compound Z138

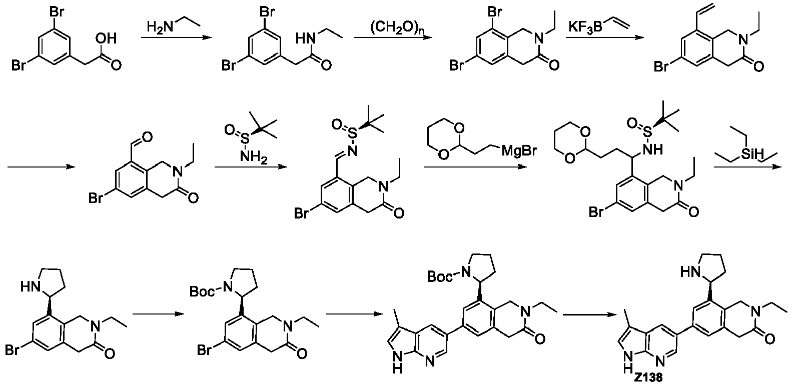

Step 1: 2-(3,5-dibromophenyl)acetic acid (5.8 g, 19.7 mmol), ethylamine hydrochloride (3.2 g, 39.3 mmol) and tetrahydrofuran (100 mL) were added to a 250 mL single-neck round-bottom flask. After stirring at room temperature for 5 min, 2-(7-azobenzotriazole)-N,N,N',N'-tetramethylurea hexafluorophosphate (22.5 g, 59.2 mmol) was added in batches and stirred at room temperature for 30 min. Ethyl acetate (300 mL) was added to the reaction mixture. The obtained mixture was washed with saturated brine (300 mLX3), dried over anhydrous sodium sulfate, and concentrated. The crude product was purified by a flash silica gel column (ethyl acetate:petroleum ether = 10:100) to give 2-(3,5-dibromophenyl)-N-ethylacetamide (5.1 g, yield: 80%). ES-API: [M+H]⁺=319.9.

Step 2: The 2-(3,5-dibromophenyl)-N-ethylacetamide (4.8 g, 14.9 mmol), paraformaldehyde (0.54 g, 18.0 mmol) and Eaton's reagent (15 mL) were added to a 100 mL single-neck flask and stirred at 80°C for 4 h. Ethyl acetate (100 mL) was added to the reaction mixture. The obtained mixture was washed with saturated brine (50 mLX3), dried over anhydrous sodium sulfate, and concentrated. The crude product was purified by a flash silica gel column (ethyl acetate:petroleum ether = 20:100) to give 6,8-dibromo-2-ethyl-1,4-dihydroisoquinolin-3(2H)-one (2.9 g, yield: 59%). ES-API: [M+H]⁺=331.9.

Step 3: The 6,8-dibromo-2-ethyl-1,4-dihydroisoquinolin-3(2H)-one (1.5 g, 4.5 mmol), potassium vinylfluoroborate (0.63 g, 4.7 mmol), tetrakis(triphenylphosphine)palladium (0.5 g, 0.43 mmol), potassium carbonate (1.8 g, 13.0 mmol), dioxane (30 mL) and water (4 mL) were added to a 50 mL three-neck round-bottom flask. The system was replaced with nitrogen three times and reacted at 110°C for 4 h in the presence of protective nitrogen. Ethyl acetate (100 mL) was added to the reaction mixture. The obtained mixture was washed with saturated brine (50 mLX3), dried over anhydrous sodium sulfate, and concentrated. The crude product was purified by a flash silica gel column (ethyl acetate:petroleum ether = 40:100) to give 6-bromo-2-ethyl-8-vinyl-1,4-dihydroisoquinolin-3(2H)-one (2.37 g, crude product). ES-API: [M+H]⁺=280.0.

Step 4: The 6-bromo-2-ethyl-8-vinyl-1,4-dihydroisoquinolin-3(2H)-one (2.37 g, crude product), sodium periodate (10 g, 46.7 mmol), tetrahydrofuran (50 mL) and water (40 mL) were added to a 50 mL single-neck round-bottom flask. Potassium osmate dihydrate (0.6 g, 1.63 mmol) was added in batches under an ice-water bath condition and then stirred at 0°C for 2 h. Ethyl acetate (100 mL) was added to the reaction mixture. The obtained mixture was washed with saturated brine (100 mLX3), dried over anhydrous sodium sulfate, and concentrated. The crude product was purified by a flash silica gel column (ethyl acetate:petroleum ether = 30:100) to give 6-bromo-2-ethyl-3-oxo-1,2,3,4-tetrahydroisoquinoline-8-carbaldehyde (300 mg, yield of the 2 steps: 23.6%). ES-API: [M+H]⁺=282.0.

Step 5: The 6-bromo-2-ethyl-3-oxo-1,2,3,4-tetrahydroisoquinoline-8-carbaldehyde (300 mg, 1.1 mmol) and dichloromethane (10 mL) were added to a 50 mL single-neck round-bottom flask. (S)-2-Methylpropane-2-sulfinamide (260 mg, 2.1 mmol) and tetraethyl titanate (1 mL) were added under an ice-water bath condition and stirred at room temperature for 3 h. Ethyl acetate (30 mL) was added to the reaction mixture. The obtained mixture was washed with saturated brine (30 mLX3), dried over anhydrous sodium sulfate, and concentrated. The crude product was purified by a flash silica gel column (ethyl acetate:petroleum ether = 20:100) to give (S,E)-N-((6-bromo-2-ethyl-3-oxo-1,2,3,4-tetrahydroisoquinolin-8-yl)methylene)-2-methyl propane-2-sulfinamide (350 mg, yield: 83%). ES-API: [M+H]⁺=385.1.

Step 6: The (S,E)-N-((6-bromo-2-ethyl-3-oxo-1,2,3,4-tetrahydroisoquinolin-8-yl)methylene)-2-methyl propane-2-sulfinamide (250 mg, 0.65 mmol) and anhydrous tetrahydrofuran (3 mL) were added to a 5 mL three-neck round-bottom flask, and the system was replaced with nitrogen three times. A 0.5M solution of (2-(1,3-dioxan-2-yl)ethyl)magnesium bromide in tetrahydrofuran (4 mL) was added at -78°C in the presence of protective nitrogen, and the system continued to be stirred for 2 h. The reaction was quenched by the addition of a saturated ammonium chloride solution (5 mL). Ethyl acetate was added (30 mL). The obtained mixture was washed with saturated brine (30 mLX3), dried over anhydrous sodium sulfate, and concentrated. The crude product was purified by a flash silica gel column (ethyl acetate:petroleum ether = 20:100) to give (S)-N-(1-(6-bromo-2-ethyl-3-oxo-1,2,3,4-tetrahydroisoquinolin-8-yl)-3-(1,3-dioxan-2-yl)p ropyl)-2-methylpropane-2-sulfinamide (55 mg, crude product). ES-API: [M+H]⁺=501.1.

Step 7: The (S)-N-(1-(6-bromo-2-ethyl-3-oxo-1,2,3,4-tetrahydroisoquinolin-8-yl)-3-(1,3-dioxan-2-yl)p ropyl)-2-methylpropane-2-sulfinamide (55 mg, crude product), trifluoroacetic acid (3 mL) and water (0.15 mL) were added to a 25 mL-neck round-bottom flask. Trimethylsilane (120 mg, 1.0 mmol) was added at 0°C and stirred for 18 h. The reaction mixture was spun to dryness. Ethyl acetate (30 mL) was added. The obtained mixture was washed with saturated brine (30 mLX3), dried over anhydrous sodium sulfate, and concentrated to give (S)-6-bromo-2-ethyl-8-(pyrrolidin-2-yl)-1,4-dihydroisoquinolin-3(2H)-one (50 mg, crude product). ES-API: [M+H]⁺=323.0.

Step 8: The (S)-6-bromo-2-ethyl-8-(pyrrolidin-2-yl)-1,4-dihydroisoquinolin-3(2H)-one (50 mg, crude product), di-tert-butyl dicarbonate (1 mL), triethylamine (46 mg, 0.45 mmol) and dichloromethane (5 mL) were added to a 25 mL single-neck round-bottom flask and stirred for 1 h. Dichloromethane (20 mL) was added to the reaction mixture. The obtained mixture was washed with saturated brine (20 mLX3), dried over anhydrous sodium sulfate, and concentrated to give tert-butyl tert-butyl (S)-2-(6-bromo-2-ethyl-3-oxo-1,2,3,4-tetrahydroisoquinolin-8-yl)pyrrolidine-1-carboxylat e (58 mg, crude product). ES-API: [M+H]⁺=423.0.

Step 9: The tert-butyl tert-butyl (S)-2-(6-bromo-2-ethyl-3-oxo-1,2,3,4-tetrahydroisoquinolin-8-yl)pyrrolidine-1-carboxylat e (58 mg, crude product), 3-methyl-5-(4,4,5,5-tetramethyl-1,3,2-dioxaborolan-2-yl)-1H-pyrrolo[2,3-b]pyridine (28 mg, 0.11 mmol), sphos-pd-g2 (10 mg, 0.01 mmol), potassium carbonate (40 mg, 0.29 mmol), dioxane (30 mL) and water (4 mL) were added to a 25 mL single-neck round-bottom flask. The system was replaced with nitrogen three times and reacted under microwave at 110°C for 50 min in the presence of protective nitrogen. Ethyl acetate (10 mL) was added to the reaction mixture. The obtained mixture was washed with saturated brine (5 mLX3), dried over anhydrous sodium sulfate, and concentrated. The crude product was purified by a flash silica gel column (methanol:dichloromethane = 10:100) to give tert-butyl (S)-2-(2-ethyl-6-(3-methyl-1H-pyrrolo[2,3-b]pyridin-5-yl)-3-oxo-1,2,3,4-tetrahydroisoqui nolin-8-yl)pyrrolidine-1-carboxylate (27 mg, yield of the 4 steps: 8.7%). ES-API: [M+H]⁺=475.3.

Step 10: The tert-butyl (S)-2-(2-ethyl-6-(3-methyl-1H-pyrrolo[2,3-b]pyridin-5-yl)-3-oxo-1,2,3,4-tetrahydroisoqui nolin-8-yl)pyrrolidine-1-carboxylate (27 mg, 0.05 mmol), trifluoroacetic acid (3 mL) and dichloromethane (6 mL) were added to a 5 mL single-neck round-bottom flask and stirred at room temperature for 30 min. The obtained mixture was spun to dryness. The crude product was purified by prep-HPLC (ammonium bicarbonate method)(column: Waters XBridge C18, 190*250 mm, 5 µm; mobile phase: A: 0.1% aqueous ammonium bicarbonate solution; mobile phase B: acetonitrile; flow rate: 15 ml/min; B%=20%-100%; column temperature: room temperature) to give (S)-2-ethyl-6-(3-methyl-1H-pyrrolo[2,3-b]pyridin-5-yl)-8-(pyrrolidin-2-yl)-1,4-dihydroiso quinolin-3(2H)-one (Z138, 10 mg, yield: 53%). ES-API: [M+H]⁺=375.3.

### Example 41. Synthesis of compound Z235

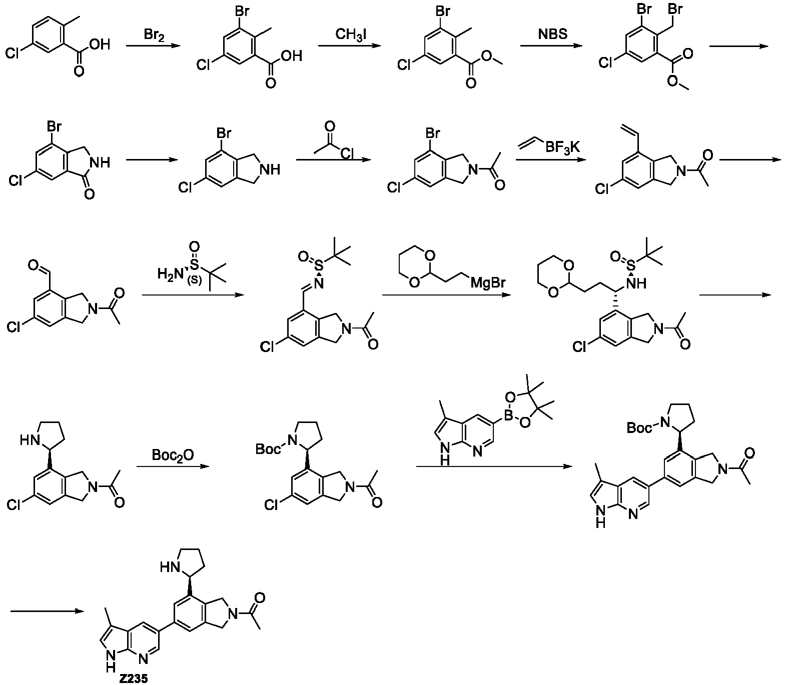

Step 1: Compound 5-chloro-2-methylbenzoic acid (10 g, 58.62 mmol), iron powder (1.64 g, 29.31 mmol) and bromine (40 mL) were placed in a sealed tank and stirred at room temperature for 48 h. The reaction mixture was slowly added dropwise to saturated aqueous sodium bicarbonate solution (400 mL) to quench the reaction, and extracted with ethyl acetate (100 mLX3). The organic phases were combined, dried over anhydrous sodium sulfate, filtered, and concentrated to give 3-bromo-5-chloro-2-methylbenzoic acid (18 g, crude product) as a white solid. ES-API: [M+H]⁺ = 248.9.

Step 2: A mixed solution of the 3-bromo-5-chloro-2-methylbenzoic acid (18 g, 72.15 mmol), iodomethane (20.48 g, 144.30 mmol) and potassium carbonate (29.91 g, 216.44 mmol) in N,N-dimethylformamide (100 mL) was placed in a sealed tube and stirred at 60°C for 2 h. The reaction mixture was dissolved in ethyl acetate (300 mL), and washed with dilute brine (100 mLX3) and water (100 mLX3). The organic phase was dried over anhydrous sodium sulfate, filtered, and concentrated. The crude product was purified by a flash silica gel column (0-10% ethyl acetate/petroleum ether) to give methyl 3-bromo-5-chloro-2-methylbenzoate (14 g, yield of the 2 steps: 91%) as a white solid. ES-API: [M+H]⁺ = 262.9.

Step 3: In the presence of protective nitrogen, a mixed solution of the methyl 3-bromo-5-chloro-2-methylbenzoate (7 g, 26.56 mmol), N-bromosuccinimide (4.73 g, 26.56 mmol) and azobisisobutyronitrile (4.36 g, 26.56 mmol) in carbon tetrachloride (100 mL) was stirred at 90°C for 5 h. The reaction was monitored by TLC for completion. The system was cooled to room temperature, and washed with water (50 mLX3). The organic phase was dried over anhydrous sodium sulfate, filtered, and concentrated. The crude product was purified by a flash silica gel column (0-10% ethyl acetate/petroleum ether) to give methyl 3-bromo-2-(bromomethyl)-5-chlorobenzoate (6 g, yield: 66%) as a colorless liquid. ES-API: [M+H]⁺ = 340.9.

Step 4: 7 M Ammonia/methanol solution (30 mL) was added to the methyl 3-bromo-2-(bromomethyl)-5-chlorobenzoate (6 g, 17.52 mmol), and the system reacted at 70°C in a sealed tube for 2 h. A white solid was precipitated out, cooled to room temperature, filtered, and dried to give 4-bromo-6-chloroisoindolin-1-one (2 g, yield: 46%). ES-API: [M+H]⁺= 246.2, 248.2.

Step 5: 1 M Borane-tetrahydrofuran complex (40 mL) was added dropwise to a cloudy solution of the 4-bromo-6-chloroisoindolin-1-one (1 g, 4.06 mmol) in tetrahydrofuran (20 mL), and the system was heated to reflux overnight. The obtained mixture was cool to room temperature. 1 M Hydrochloric acid (40 mL) was added and continued to be stirred for 1 h. The obtained mixture was adjusted to pH 8 with saturated sodium bicarbonate solution and extracted with ethyl acetate (40 mLX3). The organic phases were combined, dried over anhydrous sodium sulfate, filtered, and concentrated to give 4-bromo-6-chloroisoindoline (1 g, crude product). ES-API: [M+H]⁺ = 232.2, 234.2.

Step 6: N,N-diisopropylethyl amine (1.67 g, 12.90 mmol) and acetyl chloride (675 mg, 8.60 mmol) were added to a solution of the 4-bromo-6-chloroisoindoline (1 g, 4.30 mmol) in dichloromethane (20 mL) under an ice bath condition and stirred for 30 min. The reaction mixture was washed with saturated sodium bicarbonate solution (10 mLX3). The organic phase was dried over anhydrous sodium sulfate, and concentrated to give 1-(4-bromo-6-chloroisoindolin-2-yl)ethan-1-one (1 g, crude product). ES-API: [M+H]⁺ = 274.0, 276.0.

Step 7: In the presence of protective nitrogen, a mixed solution of the compound 1-(4-bromo-6-chloroisoindolin-2-yl)ethan-1-one (900 mg, 3.28 mmol), potassium vinylfluoroborate (1 g, 6.56 mmol), [1,1'-bis(diphenylphosphino)ferrocene]dichloropalladium-dichloromethane complex (267 mg, 0.33 mmol), triethylamine (996 mg, 9.84 mmol) in ethanol (10 mL) was stirred at 80°C for 2 h. The reaction mixture was dissolved in ethyl acetate (30 mL), and washed with saturated brine (20 mL) and water (20 mL) successively. The organic phase was dried over anhydrous sodium sulfate, filtered, and concentrated. The crude product was purified by a flash silica gel column (0-50% ethyl acetate/petroleum ether) to give 1-(6-chloro-4-vinylisoindolin-2-yl)ethan-1-one (360 mg, yield: 50%). [M+H]⁺= 222.1.

Step 8: Potassium osmate dihydrate (100 mg, 0.27 mmol) and sodium periodate (1.73 g, 8.1 mmol) were added to the 1-(6-chloro-4-vinylisoindolin-2-yl)ethan-1-one (300 mg, 1.35 mmol) in tetrahydrofuran (6 mL) and water (6 mL) and stirred at room temperature for 2 h. Upon completion of the reaction, the reaction mixture was poured into water (10 mL) and extracted with ethyl acetate (10 mLX3). The organic phases were combined, dried over anhydrous sodium sulfate, filtered, and concentrated. The crude product was purified by a flash silica gel column (0-50% ethyl acetate/petroleum ether) to give 2-acetyl-6-chloroisoindoline-4-carbaldehyde (165 mg, yield: 55%). [M+H]⁺ = 224.1.

Step 9: In the presence of protective nitrogen, tetraethoxytitanium (899 mg, 3.94 mmol) was added dropwise to a solution of the 2-acetyl-6-chloroisoindoline-4-carbaldehyde (220 mg, 0.98 mmol) and S-tert-butylsulfinamide (238 mg, 1.97 mmol) in dichloromethane (5 mL). The reaction mixture was stirred at room temperature for 1 h. Upon completion of the reaction, dichloromethane (5 mL) and water (1 mL) were added. The obtained mixture was dried over anhydrous sodium sulfate, filtered, and concentrated. The crude product was purified by a flash silica gel column (0-60% ethyl acetate/petroleum ether) to give (S,E)-N-((2-acetyl-6-chloroisoindolin-4-yl)methylene)-2-methylpropane-2-sulfinamide (220 mg, yield: 69%) as a yellow solid. [M+H]⁺ = 327.0.

Step 10: In the presence of protective nitrogen, (1,3-dioxane-2-ethyl)magnesium bromide in tetrahydrofuran (6.24 mL, 3.12 mmol, 0.5 M) was added dropwise to a solution of the (S,E)-*N*-((2-acetyl-6-chloroisoindolin-4-yl)methylene)-2-methylpropane-2-sulfinamide (170 mg, 0.52 mmol) in tetrahydrofuran (10 mL) at -65°C and stirred at this temperature for 1 h. The system was warmed to room temperature and then continued to be stirred overnight. The reaction was quenched with saturated ammonium chloride solution (20 mL), and the reaction mixture was extracted with ethyl acetate (30 mLX3). The organic phases were combined, dried over anhydrous sodium sulfate, filtered, and concentrated to give (S)-*N*-((S)- 1-(2-acetyl-6-chloroisoindolin-4-yl)-3-(1,3-dioxan-2-yl)propyl)-2-methylpropa ne-2-sulfinamide (230 mg, crude product). [M+H]⁺= 443.2.

Step 11: Trifluoroacetic acid (2 mL) and water (0.12 mL) were added to the (S)-*N*-((S)- 1-(2-acetyl-6-chloroisoindolin-4-yl)-3-(1,3-dioxan-2-yl)propyl)-2-methylpropa ne-2-sulfinamide (230 mg, 0.52 mmol) under an ice bath condition and stirred at room temperature for 0.5 h. The obtained mixture was concentrated to give (S)-1-(6-chloro-4-(pyrrolidin-2-yl)isoindolin-2-yl)ethan-1-one (140 mg, crude product). [M+H]⁺ = 265.2.

Step 12: Di-tert-butyl dicarbonate (227 mg, 1.04 mmol) was added to a solution of the (S)-1-(6-chloro-4-(pyrrolidin-2-yl)isoindolin-2-yl)ethan-1-one (140 mg, 0.52 mmol) and N,N-diisopropylamine (202 mg, 1.56 mmol) in dichloromethane (5 mL), and the system reacted at room temperature for 0.5 h. Upon completion of the reaction, the reaction mixture was concentrated. The crude product was purified by a flash silica gel column (0-50% ethyl acetate/petroleum ether) to give tert-butyl (S)-2-(2-acetyl-6-chloroisoindolin-4-yl)pyrrolidine-1-carboxylate (100 mg, yield of the 3 steps: 53%). [M+H]⁺ = 365.2.

Step 13: In the presence of protective nitrogen, a mixed solution of 3-methyl-5-(4,4,5,5-tetramethyl-1,3,2-dioxaborolan-2-yl)-1H-pyrrolo[2,3-b]pyridine (93 mg, 0.36 mmol), the tert-butyl (S)-2-(2-acetyl-6-chloroisoindolin-4-yl)pyrrolidine-1-carboxylate (130 mg, 0.36 mmol), chloro (2-dicyclohexylphosphino-2',6'-dimethoxy-1,1'-biphenylyl)(2'-amino-1,1'-biphenyl-2-yl) palladium (II) (29 mg, 0.04 mmol), 2-dicyclohexylphosphine-2',6'-dimethoxybiphenyl (16 mg, 0.04 mmol) and potassium carbonate (149 mg, 1.08 mmol) in 1,4-dioxane (3 mL) and water (0.6 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, and concentrated. The crude product was purified by a flash silica gel column (0-80% ethyl acetate/petroleum ether) to give tert-butyl (S)-2-(2-acetyl-6-(3-methyl-1*H*-pyrrolo[2,3-*b*]pyridin-5-yl)isoindolin-4-yl)pyrrolidine-1-c arboxylate (100 mg, yield: 60%). [M+H]⁺= 461.2.

Step 14: Trifluoroacetic acid (0.5 mL) was added dropwise to a solution of the tert-butyl (S)-2-(2-acetyl-6-(3-methyl-1*H*-pyrrolo[2,3-*b*]pyridin-5-yl)isoindolin-4-yl)pyrrolidine-1-c arboxylate (100 mg, 0.22 mmol) in dichloromethane (1 mL) under an ice bath condition and stirred at room temperature for 1 h. The obtained mixture was concentrated, and purified by prep-HPLC (formic acid method) to give (S)-1-(6-(3-methyl-1*H*-pyrrolo[2,3-*b*]pyridin-5-yl)-4-(pyrrolidin-2-yl)isoindolin-2-yl)etha n-1-one (Z235, formate, 17 mg, yield: 19%) as a white solid. ES-API: [M+H]⁺ = 361.1. ¹H NMR (500 MHz, DMSO-d₆) δ 11.36 (s, 1H), 8.49 (t, *J=* 2.0 Hz, 1H), 8.27 (s, 1H), 8.14 (t, *J =* 2.5 Hz, 1H), 7.74 (s, 1H), 7.59 (d, *J =* 9.5 Hz, 1H), 7.27 (s, 1H), 4.91 (d, *J =* 18.4 Hz, 2H), 4.70 (d, *J* = 24.1 Hz, 2H), 4.33 - 4.15 (m, 1H), 3.21 - 3.14 (m, 1H), 3.07 - 2.98 (m, 1H), 2.31 (d, *J* = 1.0 Hz, 3H), 2.28 - 2.19 (m, 1H), 2.10 (d, *J* = 7.0 Hz, 3H), 1.96 - 1.88 (m, 1H), 1.88 - 1.80 (m, 1H), 1.74 - 1.64 (m, 1H).

### Example 42. Synthesis of compound Z236

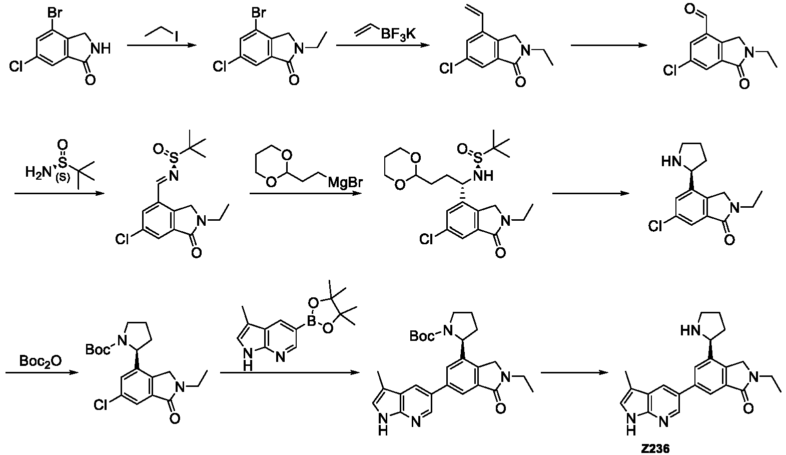

Step 1: A mixed solution of 4-bromo-6-chloroisoindolin-1-one (700 mg, 2.84 mmol), iodoethane (4.43 g, 28.40 mmol) and potassium carbonate (1.18 g, 8.52 mmol) in acetone (10 mL) was stirred at 50°C in a sealed tube for 2 days. The reaction mixture was filtered and concentrated. The crude product was purified by a flash silica gel column (0-50% ethyl acetate/petroleum ether) to give 4-bromo-6-chloro-2-ethylisoindolin-1-one (250 mg, yield: 32%). ES-API: [M+H]⁺ = 274.0, 276.0.

Step 2: In the presence of protective nitrogen, a solution of the compound 4-bromo-6-chloro-2-ethylisoindolin-1-one (200 mg, 0.73 mmol), potassium vinylfluoroborate (223 mg, 1.46 mmol), [1,1'-bis(diphenylphosphino)ferrocene]dichloropalladium-dichloromethane complex (57 mg, 0.07 mmol) and triethylamine (222 mg, 2.19 mmol) in ethanol (4 mL) was stirred at 80°C for 2 h. The reaction mixture was dissolved in ethyl acetate (30 mL), and washed with saturated brine (20 mL) and water (20 mL) successively. The organic phase was dried over anhydrous sodium sulfate, filtered, and concentrated. The crude product was purified by a flash silica gel column (0-50% ethyl acetate/petroleum ether) to give 6-chloro-2-ethyl-4-vinylisoindolin-1-one (160 mg, yield: 99%). [M+H]⁺ = 222.1.

Step 3: Potassium osmate dihydrate (50 mg, 0.68 mmol) and sodium periodate (873 mg, 4.08 mmol) were added to the 6-chloro-2-ethyl-4-vinylisoindolin-1-one (150 mg, 0.68 mmol) in tetrahydrofuran (3 mL) and water (3 mL) and stirred at room temperature for 2 h. Upon completion of the reaction, the reaction mixture was poured into water (10 mL) and extracted with ethyl acetate (10 mLX3). The organic phases were combined, dried over anhydrous sodium sulfate, filtered, and concentrated. The crude product was purified by a flash silica gel column (0-50% ethyl acetate/petroleum ether) to give 6-chloro-2-ethyl-1-oxoisoindoline-4-carbaldehyde (55 mg, yield: 37%). [M+H]⁺= 224.0.

Step 4: In the presence of protective nitrogen, tetraethoxytitanium (286 mg, 1.25 mmol) was added dropwise to a solution of the 6-chloro-2-ethyl-1-oxoisoindoline-4-carbaldehyde (70 mg, 0.31 mmol) and S-tert-butylsulfinamide (76 mg, 0.63 mmol) in dichloromethane (2 mL). The reaction mixture was stirred at room temperature for 1 h. Upon completion of the reaction, dichloromethane (5 mL) and water (1 mL) were added. The obtained mixture was dried over anhydrous sodium sulfate, filtered, and concentrated. The crude product was purified by a flash silica gel column (0-60% ethyl acetate/petroleum ether) to give (S)-*N*-((6-chloro-2-ethyl-1-oxoisoindolin-4-yl)methylene)-2-methylpropane-2-sulfinamide (95 mg, yield: 93%) as a yellow solid. [M+H]⁺ = 327.1.

Step 5: In the presence of protective nitrogen, (1,3-dioxane-2-ethyl)magnesium bromide in tetrahydrofuran (3.49 mL, 1.74 mmol, 0.5 M) was added dropwise to a solution of the (S)-*N*-((6-chloro-2-ethyl-1-oxoisoindolin-4-yl)methylene)-2-methylpropane-2-sulfinamide (95 mg, 0.29 mmol) in tetrahydrofuran (10 mL) at -65°C and stirred at this temperature for 1 h. The system was warmed to room temperature and then continued to be stirred overnight. The reaction was quenched with saturated ammonium chloride solution (20 mL), and the reaction mixture was extracted with ethyl acetate (30 mLX3). The organic phases were combined, dried over anhydrous sodium sulfate, filtered, and concentrated to give (S)-*N*-((R)-1-(6-chloro-2-ethyl-1-oxoisoindolin-4-yl)-3-(1,3-dioxan-2-yl)propyl)-2-methyl propane-2-sulfinamide (120 mg, yield: 93%). [M+H]⁺ = 443.2.

Step 6: Trifluoroacetic acid (2 mL) and water (0.12 mL) were added to the (S)-*N*-((R)-1-(6-chloro-2-ethyl-1-oxoisoindolin-4-yl)-3-(1,3-dioxan-2-yl)propyl)-2-methyl propane-2-sulfinamide (120 mg, 0.27 mmol) under an ice bath condition and stirred at room temperature for 0.5 h. The obtained mixture was concentrated to give (S)-6-chloro-2-ethyl-4-(pyrrolidin-2-yl)isoindolin-1-one (72 mg, yield: 100%). [M+H]⁺ = 265.2.

Step 7: Di-tert-butyl dicarbonate (118 mg, 0.54 mmol) was added to a solution of the (S)-6-chloro-2-ethyl-4-(pyrrolidin-2-yl)isoindolin-1-one (72 mg, 0.27 mmol) and N,N-diisopropylamine (105 mg, 0.81 mmol) in dichloromethane (5 mL), and the system reacted at room temperature for 0.5 h. Upon completion of the reaction, the reaction mixture was concentrated. The crude product was purified by a flash silica gel column (0-50% ethyl acetate/petroleum ether) to give tert-butyl (S)-2-(6-chloro-2-ethyl-1-oxoisoindolin-4-yl)pyrrolidine-1-carboxylate (72 mg, yield: 73%). [M+H]⁺ = 365.2.

Step 8: In the presence of protective nitrogen, a mixed solution of 3-methyl-5-(4,4,5,5-tetramethyl-1,3,2-dioxaborolan-2-yl)-1H-pyrrolo[2,3-b]pyridine (52 mg, 0.20 mmol), the tert-butyl (S)-2-(6-chloro-2-ethyl-1-oxoisoindolin-4-yl)pyrrolidine-1-carboxylate (72 mg, 0.20 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol), 2-dicyclohexylphosphine-2',6'-dimethoxybiphenyl (8 mg, 0.02 mmol) and potassium carbonate (83 mg, 0.60 mmol) in 1,4-dioxane (2 mL) and water (0.2 mL) was stirred under microwave irradiation at 110°C for 0.5 h. Ethyl acetate (10 mL) was poured into the reaction mixture, and the obtained mixture was washed with saturated brine (5 mL) and water (5 mL) successively. The organic phase was dried over anhydrous sodium sulfate, filtered, and concentrated. The crude product was purified by a flash silica gel column (0-80% ethyl acetate/petroleum ether) to give tert-butyl (S)-2-(2-ethyl-6-(3-methyl-1*H*-pyrrolo[2,3-*b*]pyridin-5-yl)-1-oxoisoindolin-4-yl)pyrrolidin e-1-carboxylate (60 mg, yield: 65%). [M+H]⁺ = 461.2.

Step 9: Trifluoroacetic acid (0.5 mL) was added dropwise to a solution of the tert-butyl (S)-2-(2-ethyl-6-(3-methyl-1*H*-pyrrolo[2,3-*b*]pyridin-5-yl)-1-oxoisoindolin-4-yl)pyrrolidin e-1-carboxylate (60 mg, 0.13 mmol) in dichloromethane (1 mL) under an ice bath condition and stirred at room temperature for 1 h. The obtained mixture was concentrated. The crude product was purified by prep-HPLC (ammonium bicarbonate method) to give (S)-2-ethyl-6-(3-methyl-1*H*-pyrrolo[2,3-*b*]pyridin-5-yl)-4-(pyrrolidin-2-yl)isoindolin-1-on e (Z236, 22 mg, yield: 47%) as a white solid. ES-API: [M+H]⁺ = 361.1. ¹H NMR (500 MHz, DMSO-d₆) δ 11.38 (s, 1H), 8.52 (d, *J =* 2.0 Hz, 1H), 8.21 (d, *J =* 2.0 Hz, 1H), 7.96 (d, *J=* 1.5 Hz, 1H), 7.80 (d, *J=* 1.5 Hz, 1H), 7.28 (s, 1H), 4.60 (d, *J=* 8.0 Hz, 2H), 4.29 (t, *J* = 8.0 Hz, 1H), 3.59 (q, *J* = 7.5 Hz, 2H), 3.14 - 3.09 (m, 1H), 2.99 - 2.93 (m, 1H), 2.33 (d, *J* = 1.0 Hz, 3H), 2.29 - 2.19 (m, 1H), 1.99 - 1.74 (m, 2H), 1.69 - 1.61 (m, 1H), 1.21 (t, *J* = 7.5 Hz, 3H).

### Example 43. Synthesis of compound Z232

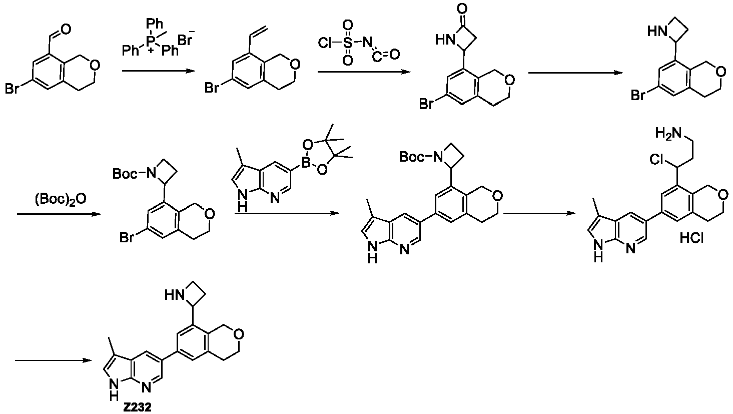

Step 1: 6-Bromoisochroman-8-carbaldehyde (1.0 g, 4.15 mmol) and methyltriphenylphosphonium bromide (4.44 g, 12.45 mmol) were dissolved in tetrahydrofuran (40 mL). In the presence of protective nitrogen, 60% sodium hydride (622 mg, 15.56 mmol) was added at 0°C and stirred at 0°C for 30 min. The system was then warmed to room temperature and continued to be stirred for 16 h. Water (40 mL) was added to the reaction mixture, and the obtained mixture was extracted with ethyl acetate (100 mL). The organic phase was washed with saturated brine (50 mL), dried over anhydrous sodium sulfate, and concentrated. The crude product was purified by a flash silica gel column (ethyl acetate/petroleum ether: 0-15%) to give 6-bromo-8-vinylisochroman (950 mg, yield: 96%) as a colorless liquid. ¹H NMR (500 MHz, CDCl₃) δ 7.43 (d, *J* = 1.5 Hz, 1H), 7.19 (s, 1H), 6.58 (dd, *J* = 17.5, 11.0 Hz, 1H), 5.63 (dd, *J* = 17.5, 1.0 Hz, 1H), 5.36 (dd, *J* = 11.0, 1.0 Hz, 1H), 4.75 (s, 2H), 3.91 (t, *J* = 5.5 Hz, 2H), 2.84 (t, *J* = 5.5 Hz, 2H).

Step 2: The 6-bromo-8-vinylisochroman (1.7 g, 7.11 mmol) was dissolved in dichloromethane (8 mL). In the presence of protective nitrogen, thioisocyanate chloride (1.52 g, 40.05 mmol) in dichloromethane was slowly added dropwise at room temperature and stirred at room temperature for 16 h. The reaction mixture was cooled to 0°C. An aqueous solution (15 mL) of sodium sulfite (1.34 g, 10.66 mmol) and sodium carbonate (2.26 g, 21.33 mmol) was added dropwise and stirred at room temperature for 1 h. Dichloromethane (80 mL) was added to the reaction mixture. The obtained mixture was filtered with diatomaceous earth. The filtrate was separated, and the obtained aqueous layer was extracted with dichloromethane (50 mL×2). The organic phases were combined, dried over anhydrous sodium sulfate, and concentrated. The crude product was purified by a flash silica gel column (tetrahydrofuran/petroleum ether: 0-50%) to give 4-(6-bromoisochroman-8-yl)azetidin-2-one (350 mg, yield: 17%) as a white solid. ES-API: [M+H]⁺=282.1,284.1.

Step 3: The 4-(6-bromoisochroman-8-yl)azetidin-2-one (280 mg, 0.99 mmol) was dissolved in tetrahydrofuran (10 mL), and the system was cooled to 0°C. 1M Borane in tetrahydrofuran (6.93 mL, 6.93 mmol) was added dropwise and stirred at room temperature for 30 min, and then the system was heated to reflux for 16 h. The reaction mixture was cooled to 0°C. 6M hydrochloric acid (10 mL) was slowly added dropwise, and then heated to reflux for 30 min. The reaction mixture was cooled to room temperature. 6M Sodium hydroxide solution was added to adjust pH=10, and the obtained mixture was extracted with ethyl acetate (50 mL×2). The organic phases were combined, washed with saturated sodium chloride solution (3 mL), dried over anhydrous sodium sulfate, and concentrated to give 4-(6-bromoisochroman-8-yl)azetidine (950 mg, crude product) which was directly used in the next reaction without further purification.

Step 4: The 4-(6-bromoisochroman-8-yl)azetidine (950 mg, crude product) was dissolved in dichloromethane (80 mL). Triethylamine (505 mg, 5.0 mmol) and di-tert-butyl dicarbonate (1.09 g, 5.0 mmol) were added at 0°C and stirred at 0°C for 1 h. The reaction mixture was concentrated. Ethyl acetate (50 mL) was added. The obtained mixture was washed with water (25 mL×2) and saturated brine (25 mL) successively, dried over anhydrous sodium sulfate, and concentrated. The crude product was purified by a flash silica gel column (tetrahydrofuran/petroleum ether: 0-18%) to give tert-butyl 2-(6-bromoisochroman-8-yl)azetidine-1-carboxylate (175 mg, yield of the 2 steps: 48%) as a colorless liquid. ES-API: [M+Na]⁺=390.1, 392.1.

Step 5: The tert-butyl 2-(6-bromoisochroman-8-yl)azetidine-1-carboxylate (70 mg, 0.19 mmol), 3-methyl-5-(4,4,5,5-tetramethyl-1,3,2-dioxaborolan-2-yl)-1*H*-pyrrolo[2,3-*b*]pyridine (59 mg, 0.23 mmol), sodium carbonate (60 mg, 0.57 mmol), 2-dicyclohexylphosphine-2',6'-dimethoxy-biphenyl (4 mg, 0.01 mmol), chloro (2-dicyclohexylphosphino-2',6'-dimethoxy-1,1'-biphenylyl)(2'-amino-1,1'-biphenyl-2-yl) palladium (II) (14 mg, 0.019 mmol), 1,4-dioxane (2.0 mL) and water (0.5 mL) were added to a 5 mL microwave tube. The mixture was purged with nitrogen for 1 min, and the system reacted in a microwave reactor at 110°C for 45 min. Water (5 mL) was added to the reaction mixture, and the obtained mixture was extracted with ethyl acetate (40 mL). The organic phase was washed with saturated brine (15 mL), dried over anhydrous sodium sulfate, and concentrated. The crude product was purified by a flash silica gel column (methanol/dichloromethane: 0-2%) to give tert-butyl 2-(6-(3-methyl-1*H*-pyrrolo[2,3-b]pyridin-5-yl)isochroman-8-yl)azetidine-1-carboxylate (75 mg, yield: 94%) as a pale yellow solid. ES-API:[M+H]⁺=420.3.

Step 6: The tert-butyl 2-(6-(3-methyl-1*H*-pyrrolo[2,3-*b*]pyridin-5-yl)isochroman-8-yl)azetidine-1-carboxylate (65 mg, 0.15 mmol) was dissolved in 4M hydrogen chloride/dioxane solution (4 mL) and stirred at room temperature for 2 h. The reaction mixture was concentrated to give 3-chloro-3-(6-(3-methyl-1*H*-pyrrolo[2,3-*b*]pyridin-5-yl)isochroman-8-yl)propan-1-amine hydrochloride (60 mg, yield: 100%) as a pale yellow solid. ES-API: [M+H]⁺=356.2 (free base).

Step 7: The 3-chloro-3-(6-(3-methyl-1*H*-pyrrolo[2,3-*b*]pyridin-5-yl)isochroman-8-yl)propan-1-amine hydrochloride (50 mg, 0.13 mmol) was dissolved in methanol (30 mL). 4M Sodium hydroxide solution (2 mL, 8.0 mmol) was added at 0°C and stirred at room temperature for 16 h. The reaction mixture was extracted with dichloromethane (40 mL). The organic phase was washed with saturated brine (10 mL), dried over anhydrous sodium sulfate, and concentrated. The crude product was purified by thin layer chromatography preparative plate (dichloromethane/7M ammonia in methanol = 15:1) to give crude product, which was then purified by prep-HPLC (formic acid method) to give 5-(8-(azetidin-2-yl)isochroman-6-yl)-3-methyl-1*H*-pyrrolo[2,3-*b*]pyridine (Z232, 1 mg, yield: 2.6%) as a white solid. ES-API: [M+H]⁺=320.2 (free base).

### Example 44. Synthesis of compound Z228-1 and compound Z228-2

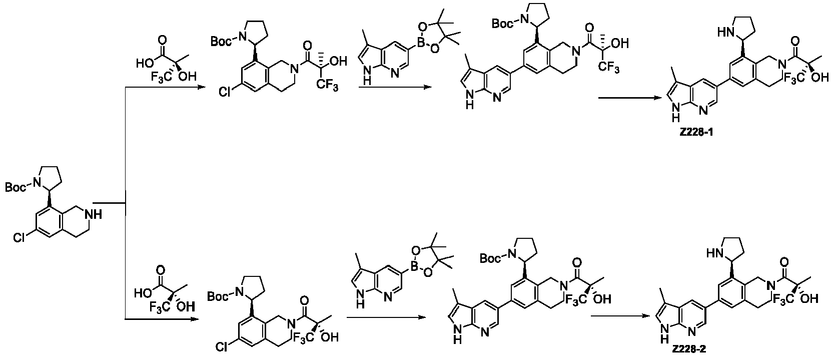

Step 1: Tert-butyl 2-(6-chloro-1,2,3,4-tetrahydroisoquinolin-8-yl)pyrrolidine-1-carboxylate (75 mg, 0.222 mmol) was dissolved in dried dichloromethane (5 mL). (R)-3,3,3-Trifluoro-2-hydroxy-2-methylpropanoic acid (70.3 mg, 0.444 mmol), triethylamine (67.2 mg, 0.666 mmol) and 2-(7-azobenzotriazol)-N,N,N',N'-tetramethylurea hexafluorophosphate (169 mg, 0.444 mmol) were added successively, and the system reacted at room temperature for 1 h. Upon completion of the reaction, the reaction mixture was diluted with dichloromethane (30 mL), washed with saturated brine (30 mL), dried over anhydrous sodium sulfate, filtered, and concentrated. The crude product was purified by column chromatography (petroleum ether/ethyl acetate = 1/1) to give tert-butyl (S)-2-(6-chloro-2-((R)-3,3,3-trifluoro-2-hydroxy-2-methylpropanoyl)-1,2,3,4-tetrahydrois oquinolin-8-yl)pyrrolidine-1-carboxylate (105 mg, yield: 99%). ES-API: [M+H]⁺=477.0.

Step 2: The tert-butyl (S)-2-(6-chloro-2-((R)-3,3,3-trifluoro-2-hydroxy-2-methylpropanoyl)-1,2,3,4-tetrahydrois oquinolin-8-yl)pyrrolidine-1-carboxylate (105 mg, 0.22 mmol), 3-methyl-5-(4,4,5,5-tetramethyl-1,3,2-dioxaborolan-2-yl)-1H-pyrrolo[2,3-b]pyridine (113 mg, 0.44 mmol), Sphos Pd G2 (15.8 mg, 0.022 mmol) and potassium carbonate (91 mg, 0.66 mmol) were added to 1,4-dioxane (3 mL) and water (0.5 mL). The system was replaced with nitrogen and reacted under microwave at 110°C for 1 h. Upon completion of the reaction, ethyl acetate (20 mL) was added. The obtained mixture was washed with water (10 mL) and saturated brine (10 mL) successively, dried over anhydrous sodium sulfate, and purified by preparative thin layer chromatography (petroleum ether/ethyl acetate = 1/1) to give tert-butyl (S)-2-(6-(3-methyl-1H-pyrrolo[2,3-b]pyridin-5-yl)-2-((R)-3,3,3-trifluoro-2-hydroxy-2-met hylpropanoyl)-1,2,3,4-tetrahydroisoquinolin-8-yl)pyrrolidine-1-carboxylate (40 mg, yield: 32%). ES-API: [M+H]⁺=573.1.

Step 3: The tert-butyl (S)-2-(6-(3-methyl-1H-pyrrolo[2,3-b]pyridin-5-yl)-2-((R)-3,3,3-trifluoro-2-hydroxy-2-met hylpropanoyl)-1,2,3,4-tetrahydroisoquinolin-8-yl)pyrrolidine-1-carboxylate (40 mg, 0.07 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia/methanol solution (1 mL), the obtained mixture was concentrated again and purified by prep-HPLC (ammonia water method) to give (R)-3,3,3-trifluoro-2-hydroxy-2-methyl-1-(6-(3-methyl-1H-pyrrolo[2,3-b]pyridin-5-yl)-8-( (S)-pyrrolidin-2-yl)-3,4-dihydroisoquinolin-2(1H)-yl)propan-1-one (Z228-1, 22 mg, yield: 66.6%). ES-API: [M+H]⁺=473.2. ¹H NMR (500 MHz, DMSO-*d*₆) δ 11.33 (d, *J =* 2.4 Hz, 1H), 8.45 (d, *J* = 2.2 Hz, 1H), 8.09 (d, *J* = 2.2 Hz, 1H), 7.76 (d, *J* = 2.0 Hz, 1H), 7.38 (s, 1H), 7.28 - 7.24 (m, 1H), 7.19 (s, 1H), 5.25-5.22 (m, 1H), 4.99 - 4.78 (m, 1H), 4.64 (d, *J* = 17.4 Hz, 1H), 4.20 (t, *J* = 7.7 Hz, 1H), 4.12 (s, 1H), 3.76 - 3.66 (m, 1H), 3.13-3.08 (m, 1H), 2.98-2.93 (m, 3H), 2.31 (d, *J =* 1.2 Hz, 3H), 2.23-2.20 (m, 1H), 1.85-1.81 (m, 2H), 1.60 (d, *J* = 21.9 Hz, 3H), 1.48-1.43 (m, 1H).

Step 4: The tert-butyl (S)-2-(6-chloro-1,2,3,4-tetrahydroisoquinolin-8-yl)pyrrolidine-1-carboxylate (75 mg, 0.222 mmol) was dissolved in dried dichloromethane (5 mL). (S)-3,3,3-Trifluoro-2-hydroxy-2-methylpropanoic acid (70.3 mg, 0.444 mmol), triethylamine (67.2 mg, 0.666 mmol) and 2-(7-azobenzotriazol)-N,N,N',N'-tetramethylurea hexafluorophosphate (169 mg, 0.444 mmol) were added successively, and the system reacted at room temperature for 1 h. Upon completion of the reaction, the reaction mixture was diluted with dichloromethane (30 mL), washed with saturated brine (30 mL), dried over anhydrous sodium sulfate, filtered, and concentrated. The crude product was purified by column chromatography (petroleum ether/ethyl acetate = 1/1) to tert-butyl (S)-2-(6-chloro-2-((S)-3,3,3-trifluoro-2-hydroxy-2-methylpropanoyl)-1,2,3,4-tetrahydroiso quinolin-8-yl)pyrrolidine-1-carboxylate (85 mg, yield: 81%). ES-API: [M+H]⁺=477.0.

Step 5: The tert-butyl (S)-2-(6-chloro-2-((S)-3,3,3-trifluoro-2-hydroxy-2-methylpropanoyl)-1,2,3,4-tetrahydroiso quinolin-8-yl)pyrrolidine-1-carboxylate (85 mg, 0.178 mmol), 3-methyl-5-(4,4,5,5-tetramethyl-1,3,2-dioxaborolan-2-yl)-1H-pyrrolo[2,3-b]pyridine (92 mg, 0.357 mmol), Sphos Pd G2 (13 mg, 0.0178 mmol) and potassium carbonate (74 mg, 0.534 mmol) were added to 1,4-dioxane (3 mL) and water (0.5 mL. The system was replaced with nitrogen and reacted under microwave at 110°C for 1 h. Upon completion of the reaction, ethyl acetate (20 mL) was added. The obtained mixture was washed with water (10 mL) and saturated brine (10 mL) successively, dried over anhydrous sodium sulfate, and purified by preparative thin layer chromatography (petroleum ether/ethyl acetate = 1/1) to give tert-butyl (S)-2-(6-(3-methyl-1H-pyrrolo[2,3-b]pyridin-5-yl)-2-((S)-3,3,3-trifluoro-2-hydroxy-2-met hylpropanoyl)-1,2,3,4-tetrahydroisoquinolin-8-yl)pyrrolidine-1-carboxylate (40 mg, yield: 39%). ES-API: [M+H]⁺=573.1.

Step 6: The tert-butyl (S)-2-(6-(3-methyl-1H-pyrrolo[2,3-b]pyridin-5-yl)-2-((S)-3,3,3-trifluoro-2-hydroxy-2-met hylpropanoyl)-1,2,3,4-tetrahydroisoquinolin-8-yl)pyrrolidine-1-carboxylate (40 mg, 0.07 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia water/methanol solution (1 mL), the obtained mixture was concentrated again and purified by prep-HPLC (ammonia water method) to give (S)-3,3,3-trifluoro-2-hydroxy-2-methyl-1-(6-(3-methyl-1H-pyrrolo[2,3-b]pyridin-5-yl)-8-( (S)-pyrrolidin-2-yl)-3,4-dihydroisoquinolin-2(1H)-yl)propan-1-one (Z228-2, 25 mg, yield: 75.7%). ES-API: [M+H]⁺=473.2. ¹H NMR (500 MHz, DMSO-*d*₆) δ 11.33 (d, *J =* 2.4 Hz, 1H), 8.45 (d, *J* = 2.2 Hz, 1H), 8.09 (d, *J* = 2.2 Hz, 1H), 7.76 (d, *J* = 2.0 Hz, 1H), 7.38 (s, 1H), 7.26 (s, 1H), 7.19 (d, *J* = 6.7 Hz, 1H), 5.23-5.18 (m, 1H), 4.96 - 4.78 (m, 1H), 4.64 (d, *J* = 17.1 Hz, 1H), 4.19 (t, *J* = 7.7 Hz, 1H), 4.09 (s, 1H), 3.56 (d, *J* = 28.4 Hz, 1H), 3.13 - 3.06 (m, 1H), 3.01 - 2.89 (m, 3H), 2.31 (s, 3H), 2.21 (s, 1H), 1.85 - 1.72 (m, 2H), 1.57 (d, *J* = 21.9 Hz, 3H), 1.46 (m, 1H).

### Example 45. Synthesis of compound Z137

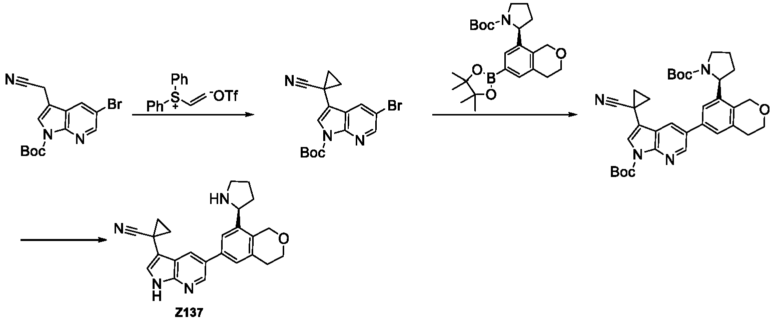

Step 1: Tert-butyl 5-bromo-3-(cyanomethyl)-1H-pyrrolo[2,3-b]pyridine-1-carboxylate (100 mg, 0.3 mmol) was dissolved in dried N,N-dimethylformamide (5 mL), and the system was cooled in an ice-water bath. 1,8-Diazabicyclo[5.4.0]undec-7-ene (136.8 mg, 0.9 mmol) and diphenyl(vinyl)sulfonium triflate (130 mg, 0.36 mmol) were added successively, and the system reacted for 1 h. Upon completion of the reaction, the reaction was quenched by the addition of a saturated aqueous ammonium chloride solution (10 mL). Ethyl acetate (30 mL) was added. The obtained mixture was washed with water (200 mLX1) and saturated brine (20 mLX1) successively, dried over anhydrous sodium sulfate, filtered, and concentrated. The crude product was purified by silica gel column chromatography (petroleum ether:ethyl acetate = 80:20) to give tert-butyl 5-bromo-3-(1-cyanocyclopropyl)-1H-pyrrolo[2,3-b]pyridine-1-carboxylate (75 mg, yield: 69.4%). ES-API: [M+H]⁺=362.0.

Step 2: The tert-butyl 5-bromo-3-(1-cyanocyclopropyl)-1H-pyrrolo[2,3-b]pyridine-1-carboxylate (36 mg, 0.1 mmol), tert-butyl (S)-2-(6-(4,4,5,5-tetramethyl-1,3,2-dioxaborolan-2-yl)isochroman-8-yl)pyrrolidine-1-carb oxylate (43 mg, 0.10 mmol), Sphos Pd G2 (7.2 mg, 0.01 mmol) and potassium carbonate (41.4 mg, 0.3 mmol) were added to 1,4-dioxane (2 mL) and water (0.5 mL). The system was replaced with nitrogen and reacted under microwave at 110°C for 1 h. Upon completion of the reaction, ethyl acetate (20 mL) was added. The obtained mixture was washed with water and saturated brine successively, dried over anhydrous sodium sulfate, and purified by column chromatography (petroleum ether:ethyl acetate = 10:90) to give tert-butyl (S)-5-(8-(1-(tert-butoxycarbonyl)pyrrolidin-2-yl)isochroman-6-yl)-3-(1-cyanocyclopropyl )-1H-pyrrolo[2,3-b]pyridine-1-carboxylate (58 mg, yield: 98%). ES-API: [M+H]⁺=585.3.

Step 3: The tert-butyl (S)-5-(8-(1-(tert-butoxycarbonyl)pyrrolidin-2-yl)isochroman-6-yl)-3-(1-cyanocyclopropyl )-1H-pyrrolo[2,3-b]pyridine-1-carboxylate (58 mg, 0.10 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia/methanol solution (1 mL), the obtained mixture was concentrated again and purified by prep-HPLC (ammonia water method) to give (S)-1-(5-(8-(pyrrolidin-2-yl)isochroman-6-yl)-1H-pyrrolo[2,3-b]pyridin-3-yl)cyclopropan e-1-carbonitrile (Z137, 12 mg, yield: 31%). ES-API: [M+H]⁺=385.2. ¹H NMR (500 MHz, DMSO-*d*₆) δ 11.84 (s, 1H), 8.53 (d, *J =* 2.1 Hz, 1H), 8.22 (d, *J =* 2.2 Hz, 1H), 7.71 (d, *J =* 2.0 Hz, 1H), 7.59 (d, *J=* 2.0 Hz, 1H), 7.34 (d, *J=* 1.9 Hz, 1H), 4.92 - 4.69 (m, 3H), 4.08 (t, *J* = 7.7 Hz, 1H), 3.98 - 3.82 (m, 3H), 3.66 - 3.46 (m, 1H), 3.11-3.06 (m, 1H), 2.98 - 2.82 (m, 4H), 2.19 - 2.11 (m, 1H), 1.92 - 1.72 (m, 3H), 1.71 - 1.62 (m, 3H), 1.54 - 1.41 (m, 4H).

### Example 46. Synthesis of compound Z237

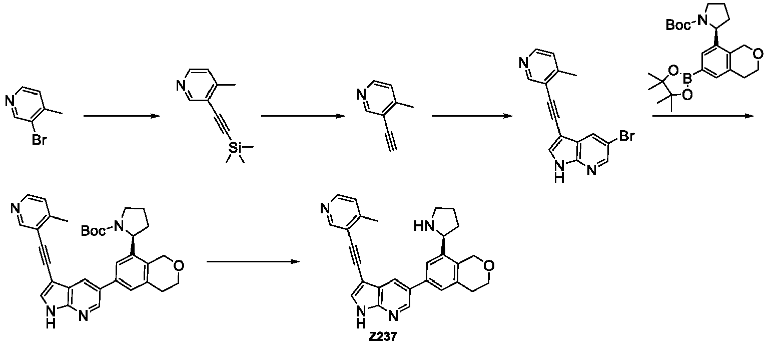

Step 1: 3-Bromo-4-methylpyridine (500 mg, 2.89 mmol) was dissolved in anhydrous N,N-dimethylformamide (5 mL). Trimethylsilylacetylene (424.8 mg, 4.34 mmol), bis(triphenylphosphine)palladium chloride (203.3 mg, 0.29 mmol), cuprous iodide (110.2 mL, 0.58 mmol) and triethylamine (875.7 mg, 8.67 mmol) were added and stirred at room temperature for 2 min in the presence of protective nitrogen. Then the system was heated to 115°C in an oil bath and stirred for 16 h. The reaction was monitored by LC-MS for completion. The reaction mixture was poured into water (20 mL), extracted with ethyl acetate (200 mL×2), dried over anhydrous sodium sulfate, filtered, concentrated, and purified by column chromatography (tetrahydrofuran:petroleum ether = 20%-40%) to give 4-methyl-3-((trimethylsilyl)ethynyl)pyridine (450 mg, yield: 82.4%). ES-API: [M+H]⁺=190.2.

Step 2: The 4-methyl-3-((trimethylsilyl)ethynyl)pyridine (450 mg, 2.38 mmol) was dissolved in methanol (10 mL). Potassium carbonate (492.7 mg, 3.57 mmol) was added at room temperature and stirred for 2 h. The reaction was monitored by LC-MS for completion. The reaction mixture was filtered, concentrated, and purified by column chromatography (tetrahydrofuran:petroleum ether = 20%-50%) to give 3-ethynyl-4-methylpyridine (250 mg, yield: 89.9%). ES-API: [M+H]⁺=118.1.

Step 3: The 3-ethynyl-4-methylpyridine (250 mg, 2.13 mg) was dissolved in anhydrous triethylamine (10 mL). 5-Bromo-3-iodo-1H-pyrrolo[2,3-b]pyridine (754.4 mg, 2.34 mmol), bis(triphenylphosphine)palladium chloride (147.2 mg, 0.21 mmol) and cuprous iodide (110.2 mL, 0.58 mmol) were added and stirred at room temperature for 2 min in the presence of protective nitrogen. Then the system was heated to 80°C in an oil bath and stirred for 16 h. The reaction was monitored by LC-MS for completion, and water (20 mL) was added to the reaction mixture. The obtained mixture was extracted with ethyl acetate (50 mL×2), dried over anhydrous sodium sulfate, filtered, concentrated, and purified by column chromatography (tetrahydrofuran:petroleum ether = 50%-60%) to give 5-bromo-3-((4-methylpyridin-3-yl)ethynyl)-1H-pyrrolo[2,3-b]pyridine (312 mg, yield: 47.1%). ES-API: [M+H]⁺=312.2, 314.2.

Step 4: The 5-bromo-3-((4-methylpyridin-3-yl)ethynyl)-1H-pyrrolo[2,3-b]pyridine (60 mg, 0.19 mmol) was dissolved in dioxane (5 mL) and water (1 mL). tert-butyl (S)-2-(6-(4,4,5,5-tetramethyl-1,3,2-dioxaborolan-2-yl)isochroman-8-yl)pyrrolidine-1-carb oxylate (97.8 mg, 0.23 mmol), chloro (2-dicyclohexylphosphino-2',6'-dimethoxy-1,1'-biphenylyl)(2'-amino-1,1'-biphenyl-2-yl) palladium (II) (14.4 mg, 0.02 mmol) and potassium phosphate (127.2 mg, 0.60 mmol) were added at room temperature and stirred at 100°C for 2 h in the presence of protective nitrogen. Dichloromethane (40 mL) was added to the reaction mixture. The obtained mixture was washed with water (10 mL) and saturated brine (20 mL), dried over anhydrous sodium sulfate, filtered, concentrated, and purified by column chromatography (tetrahydrofuran:petroleum ether = 40%-60%) to give tert-butyl (S)-2-(6-(3-((4-methylpyridin-3-yl)ethynyl)-1H-pyrrolo[2,3-b]pyridin-5-yl)isochroman-8-yl)pyrrolidine-1-carboxylate (31.2 mg, yield: 30.9%). ES-API: [M+H]⁺=535.2.

Step 5: The tert-butyl (S)-2-(6-(3-((4-methylpyridin-3-yl)ethynyl)-1H-pyrrolo[2,3-b]pyridin-5-yl)isochroman-8-yl)pyrrolidine-1-carboxylate (31.2 mg, 0.0584 mmol) was dissolved in dichloromethane (2 mL). Trifluoroacetic acid (3 mL) was added, and the system reacted at room temperature for 1 h. The reaction mixture was concentrated. 7.0M Ammonia/methanol solution (5 mL) was added, and a solvent was spun to dryness. The crude product was purified by prep-HPLC (trifluoroacetic acid method) to give (S)-3-((4-methylpyridin-3-yl)ethynyl)-5-(8-(pyrrolidin-2-yl)isochroman-6-yl)-1H-pyrrolo[ 2,3-b]pyridine (Z237, 2.5 mg, yield: 10%). ES-API: [M+H]⁺=435.2.

### Example 47. Synthesis of compound Z119 and isomers thereof

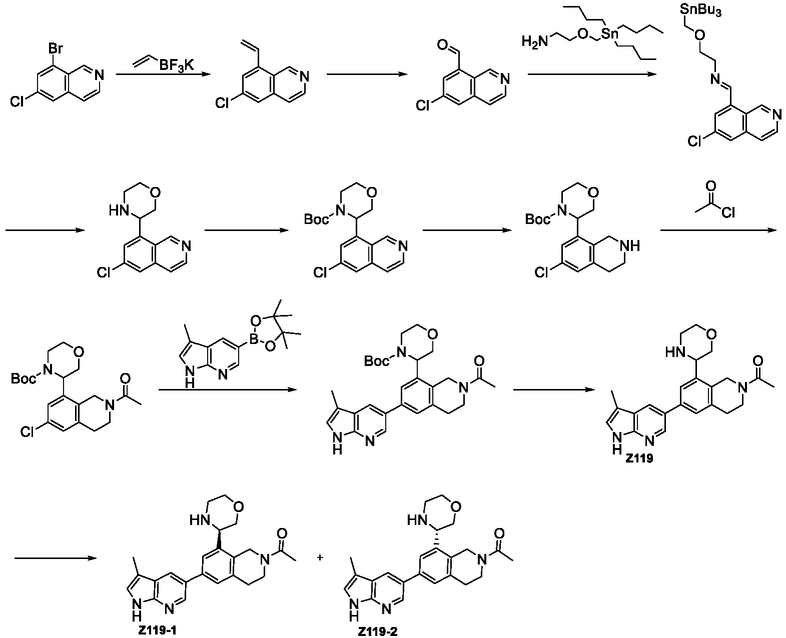

Step 1: 8-Bromo-6-chloro-isoquinoline (1 g, 4.12 mmol) was dissolved in ethanol (20 mL). Potassium trifluoro(vinyl)borate (1.66 g, 12.4 mmol), [1,1'-bis(diphenylphosphino)ferrocene]dichloropalladium (302 mg, 0.41 mmol) and triethylamine (1.25 g, 12.4 mmol) were added, and the system was warmed to 80°C for 3 h. The reaction mixture was spun to dryness, and purified by column chromatography (petroleum ether/ethyl acetate = 4/1) to give 6-chloro-8-vinylisoquinoline (750 mg, yield: 95.9%). ES-API:[M+1]⁺=190.1.

Step 2: The 6-chloro-8-vinylisoquinoline (750 mg, 3.95 mmol) was dissolved in tetrahydrofuran/water (10 mL/5 mL). 2,6-Lutidine (424 mg, 3.95 mmol), sodium periodate (6.77 g, 31.64 mmol) and potassium osmate dihydrate (437 mg, 1.19 mmol) were added, and the system reacted at room temperature for 2 h. The reaction was quenched with aqueous sodium thiosulfate solution, and the reaction mixture was extracted with ethyl acetate. The organic phase was dried over anhydrous sodium sulfate, spun to dryness, and purified by column chromatography (petroleum ether/ethyl acetate = 2/1) to give 6-chloroisoquinoline-8-carbaldehyde (390 mg, yield: 51%). ES-API:[M+1]⁺=191.9.

Step 3: The 6-chloroisoquinoline-8-carbaldehyde (270 mg, 1.41 mmol) was dissolved in dichloromethane (10 mL). 2-(Tributylstannylmethoxy)ethanamine (513 mg, 1.41 mmol) and 4A molecular sieves (300 mg) were added, and the system react at room temperature overnight. The reaction mixture was filtered and spun to dryness to give 1-(6-chloro-8-isoquinolyl)-N-[2-(tributylstannylmethoxy)ethyl]methanamine as a crude product which was directly used in the next step without purification.

Step 4: (R,R)-2,2'-Isopropylidenebis(4-phenyl-2-oxazoline) (94 mg, 0.28 mmol) was added in one portion to a suspension of copper triflate (510 mg, 1.41 mmol) in hexafluoroisopropanol (2.5 mL) followed by a solution of the 1-(6-chloro-8-isoquinolyl)-N-[2-(tributylstannylmethoxy)ethyl]methanamine (crude product) in hexafluoroisopropanol (2.5 mL), and the system reacted at room temperature overnight. The reaction was quenched with 1M sodium hydroxide solution, and the reaction mixture was extracted with dichloromethane. The organic phase was dried over anhydrous sodium sulfate and spun to dryness to give 3-(6-chloro-8-isoquinolyl)morpholine as a crude product which was directly used in the next step without purification. ES-API:[M+1]⁺=249.1.

Step 5: The 3-(6-chloro-8-isoquinolyl)morpholine (crude product) was dissolved in tetrahydrofuran (5 mL). Triethylamine (428 mg, 4.23 mmol) and di-tert-butyl dicarbonate (615 mg, 2.82 mmol) were added, and the system reacted at room temperature for 2 h. Upon completion of the reaction, the reaction was quenched by the addition of water. The reaction mixture was extracted with ethyl acetate, dried over anhydrous sodium sulfate, spun to dryness, and purified by column chromatography (petroleum ether/ethyl acetate = 4/1) to give tert-butyl 3-(6-chloro-8-isoquinolyl)morpholine-4-carboxylate (200 mg, yield: 40.1%). ES-API:[M+1]⁺=349.1.

Step 6: The tert-butyl 3-(6-chloro-8-isoquinolyl)morpholine-4-carboxylate (100 mg, 0.29 mmol) was dissolved in acetic acid (1.5 mL), and the system was cooled to 0°C. Sodium borohydride (43 mg, 1.15 mmol) was added, and the system reacted at 0°C for 1 h. Upon completion of the reaction, the reaction was quenched by the addition of water. The obtained mixture was basified with sodium carbonate, extracted with dichloromethane, dried over anhydrous sodium sulfate, and spun to dryness to give tert-butyl 3-(6-chloro-1,2,3,4-tetrahydroisoquinolin-8-yl)morpholine-4-carboxylate as a crude product which was directly used in the next step without purification. ES-API:[M+1]⁺=353.1.

Step 7: The tert-butyl 3-(6-chloro-1,2,3,4-tetrahydroisoquinolin-8-yl)morpholine-4-carboxylate (crude product, 0.28 mmol) was dissolved in dichloromethane (1 mL). Triethylamine (85 mg, 0.84 mmol) was added, and the system was cooled to 0°C. Acetyl chloride (22 mg, 0.28 mmol) was then added, and the system reacted at 0°C for 0.5 h. Upon completion of the reaction, the reaction was quenched by the addition of water. The reaction mixture was extracted with dichloromethane, dried over anhydrous sodium sulfate, spun to dryness, and purified by column chromatography (petroleum ether/ethyl acetate = 3/1) to give tert-butyl 3-(2-acetyl-6-chloro-3,4-dihydro-1H-isoquinolin-8-yl)morpholine-4-carboxylate (80 mg, yield: 72%). ES-API:[M+1-100]⁺=295.1.

Step 8: The tert-butyl 3-(2-acetyl-6-chloro-3,4-dihydro-1H-isoquinolin-8-yl)morpholine-4-carboxylate (80 mg, 0.2 mmol) was dissolved in dioxane/water (1 mL/0.2 mL). 3-Methyl-5-(4,4,5,5-tetramethyl-1,3,2-dioxaborolan-2-yl)-1H-pyrrolo[2,3-b]pyridine (63 mg, 0.24 mmol), Sphos-Pd-G2 (14 mg, 0.02 mmol) and potassium carbonate (56 mg, 0.4 mmol) were added. The system was heated to 100°C for 1.5 h. Upon completion of the reaction, the reaction mixture was spun to dryness and purified by a preparative thin layer chromatographic column (petroleum ether/ethyl acetate = 2/1) to tert-butyl 3-[2-acetyl-6-(3-methyl-1H-pyrrolo[2,3-b]pyridin-5-yl)-3,4-dihydro-1H-isoquinolin-8-yl] morpholine-4-carboxylate (62 mg, yield: 62%). ES-API:[M+1]⁺=491.3.

Step 9: The tert-butyl 3-[2-acetyl-6-(3-methyl-1H-pyrrolo[2,3-b]pyridin-5-yl)-3,4-dihydro-1H-isoquinolin-8-yl] morpholine-4-carboxylate (62 mg, 0.13 mmol) was dissolved in dichloromethane (1.3 mL). Trifluoroacetic acid (1.3 mL) was added, and the system react at room temperature for 1 h. Upon completion of the reaction, the reaction mixture was spun to dryness and purified by a preparative thin layer chromatographic column (dichloromethane/methanol = 10/1) to give 1-(6-(3-methyl-1H-pyrrolo[2,3-b]pyridin-5-yl)-8-(morpholin-3-yl)-3,4-dihydroisoquinolin -2(1H)ethan-1-one (Z119, 25 mg, yield: 51%). ES-API:[M+1]⁺=391.2. ¹H NMR (400 MHz, CDCl₃) δ 8.53 (s, 1H), 8.05 - 7.96 (m, 1H), 7.95 - 7.85 (m, 1H), 7.30 (s, 1H), 7.11 - 7.04 (m, 1H), 5.26 - 4.65 (m, 1H), 4.64 - 4.28 (m, 1H), 4.01 - 3.77 (m, 4H), 3.76 - 3.50 (m, 3H), 3.38 - 3.05 (m, 2H), 3.03 - 2.83 (m, 2H), 2.33 (s, 3H), 2.29 (s, 1H), 2.18 (s, 2H).

Step 10: The 1-(6-(3-methyl-1H-pyrrolo[2,3-b]pyridin-5-yl)-8-(morpholin-3-yl)-3,4-dihydroisoquinolin -2(1H)ethan-1-one (Z119, 21 mg) was chirally resolved (mobile phase: HEX:ETOH=70:30); separation column: AB 250 mm*4.6 mm*5 um); flow rate: 1.0 ml/min; T: 30.0°C) to give two isomeric compounds. A structure of one isomeric compound was arbitrarily assigned as (R)-1-(6-(3-methyl-1H-pyrrolo[2,3-b]pyridin-5-yl)-8-(morpholin-3-yl)-3,4-dihydroisoquin olin-2(1H)-yl)ethan-1-one: (Z119-1, retention time: 12.533 min, 9 mg, purity: 99%, de value: 100%, yield: 42.9%). ES-API:[M+H]⁺= 391.1; ¹H NMR (400 MHz, DMSO-*d₆*) δ 11.35 (s, 1H), 8.49 - 8.43 (m, 1H), 8.14 - 8.07 (m, 1H), 7.82 - 7.75 (m, 1H), 7.50 - 7.45 (m, 1H), 7.27 (s, 1H), 4.92 - 4.55 (m, 2H), 4.09 - 3.94 (m, 1H), 3.84 - 3.49 (m, 5H), 3.24 (t, *J=* 10.4 Hz, 1H), 3.05 - 2.90 (m, 3H), 2.87 (t, *J=* 6.0 Hz, 1H), 2.32 (s, 3H), 2.21 - 2.07 (m, 3H).

A structure of the other isomeric compound was arbitrarily assigned as (S)-1-(6-(3-methyl-1H-pyrrolo[2,3-b]pyridin-5-yl)-8-(morpholin-3-yl)-3,4-dihydroisoquin olin-2(1H)-yl)ethan-1-one (Z119-2, retention time: 13.819 min, 10 mg, purity: 99%, de value: 100%, yield: 47.6%). ES-API:[M+H]⁺= 391.1. ¹H NMR (400 MHz, DMSO-*d₆*) δ 11.36 (s, 1H), 8.51 - 8.45 (m, 1H), 8.15 - 8.08 (m, 1H), 7.84 - 7.76 (m, 1H), 7.50 (s, 1H), 7.31 - 7.24 (m, 1H), 4.92 - 4.57 (m, 2H), 4.15 - 4.03 (m, 1H), 3.87 - 3.54 (m, 5H), 3.45 - 3.35 (m, 1H), 3.11 - 2.82 (m, 4H), 2.37 - 2.29 (m, 3H), 2.22 - 2.08 (m, 3H).

### Example 48. Synthesis of compound Z132

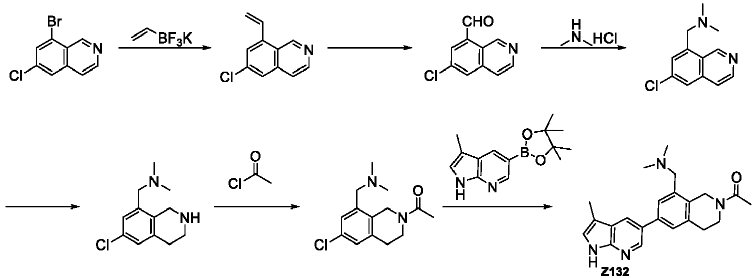

Step 1: 8-Bromo-6-chloroisoquinoline (1.8 g, 7.469 mmol), potassium vinyltrifluoroborate (3 g, 22.41 mmol) and triethylamine (2.3 g, 22.41 mmol) were dissolved in ethanol (15 mL). 1,1-Bis(diphenylphosphino)ferrocene dichloropalladium (546 mg, 0.747 mmol) was then added, and the system was heated to 80°C and stirred for 2 h in the presence of protective nitrogen. Upon completion of the reaction, the reaction mixture was filtered, spun to dryness, extracted with ethyl acetate/water, spun to dryness, and purified by column chromatography (0-20% ethyl acetate/petroleum ether, petroleum ether/ethyl acetate = 5/1, Rf = 0.4) to give 6-chloro-8-vinylisoquinoline (850 mg, yield: 61%)ES-API:[M+H]⁺=190.00.

Step 2: The 6-chloro-8-vinylisoquinoline (850 mg, 4.45 mmol) was dissolved in tetrahydrofuran (10 mL). 2,6-Lutidine (476 mg, 4.45 mmol) was added and stirred at room temperature. Sodium periodate (7.6 g, 35.6 mmol) and potassium osmate dihydrate (492 mg, 1.335 mmol) were added to water (5 mL). The aqueous solution was added to a reaction flask under an ice bath condition and stirred at room temperature for 1 h. Upon completion of the reaction, the reaction was quenched by the addition of aqueous sodium thiosulfate solution. The obtained mixture was filtered, extracted with ethyl acetate, spun to dryness, and purified by column chromatography (0-30% ethyl acetate/petroleum ether, petroleum ether/ethyl acetate = 2/1, Rf = 0.5) to give 6-chloroisoquinoline-8-carbaldehyde (320 mg, yield: 37%). ES-API:[M+H]⁺=192.0.

Step 3: The 6-chloroisoquinoline-8-carbaldehyde (260 mg, 1.36 mmol) was added to dichloromethane (5 mL). Dimethylamine hydrochloride (443 mg, 5.44 mmol) was added and stirred at room temperature for 2 h. Sodium borohydride acetate (865 mg, 4.08 mmol) was added. Upon completion of the reaction, a saturated sodium chloride solution was added. The obtained mixture was extracted with dichloromethane, spun to dryness, and purified by column chromatography (0-10% methanol/dichloromethane, dichloromethane/methanol = 20/1, Rf = 0.5) to give 1-(6-chloroisoquinolin-8-yl)-N,N-dimethylmethanamine (85 mg, yield: 28%)ES-API:[M+H]⁺=221.1.

Step 4: Sodium borohydride (33 mg, 0.864 mmol) was added to a solution of the 1-(6-chloroisoquinolin-8-yl)-N,N-dimethylmethanamine (95 mg, 0.432 mmol) in acetic acid (3 mL) and stirred at 0°C for 0.5 h. The reaction was monitored by LC-MS for completion, and quenched by the addition of aqueous sodium bicarbonate solution. The reaction mixture was extracted with dichloromethane, dried over anhydrous sodium sulfate, concentrated, and purified by column chromatography (0-10%, methanol in dichloromethane, dichloromethane/methanol = 10/1, Rf = 0.4) to give 1-(6-chloro-1,2,3,4-tetrahydroisoquinolin-8-yl)-N,N-dimethylmethanamine (90 mg, yield: 95%). ES-API:[M+H]⁺= 225.1.

Step 5: Triethylamine (203 mg, 2.01 mmol) and acetyl chloride (63mg, 0.804 mmol) were added to a solution of the 1-(6-chloro-1,2,3,4-tetrahydroisoquinolin-8-yl)-N,N-dimethylmethanamine (90 mg, 0.402 mmol) in tetrahydrofuran (1 mL) and stirred at 0°C for 15 min. The reaction was monitored by LC-MS for completion, and quenched by the addition of water. The reaction mixture was extracted with dichloromethane, dried over anhydrous sodium sulfate, and concentrated to give 1-(6-chloro-8-((dimethylamino)methyl)-3,4-dihydroisoquinolin-2(1H)-yl)ethanone (80 mg, yield: 75%). ES-API:[M+H]⁺= 267.1.

Step 6: 3-Methyl-5-(4,4,5,5-tetramethyl-1,3,2-dioxaborolan-2-yl)-1H-pyrrolo[2,3-b]pyridine (116 mg, 0.45 mmol), S-phos Pd G2 (22 mg, 0.03 mmmol) and potassium carbonate (124 mg, 0.9 mmmol) were added to the 1-(6-chloro-8-((dimethylamino)methyl)-3,4-dihydroisoquinolin-2(1H)-yl)ethanone (80 mg, 0.3 mmol), and the system was heated to 110°C and stirred in the presence of protective nitrogen. The reaction was monitored by LC-MS for completion. The reaction mixture was extracted with ethyl acetate/water, dried over anhydrous sodium sulfate, concentrated, and purified by prep-HPLC (Ultimate XB-C18, 50*250 mm, 10 um, mobile phase system: A: purified water (0.05% ammonia water) B: pure acetonitrile, flow rate: 80 ml/min, gradient: within 50 min, B/A = 20%-90%, wavelength: 214 nm, column temperature: room temperature) to give 1-(8-((dimethylamino)methyl)-6-(3-methyl-1H-pyrrolo[2,3-b]pyridin-5-yl)-3,4-dihydroiso quinolin-2(1H)-yl)ethanone (Z132, 24.4 mg, yield: 23%) as a white powder. ES-API:[M+H]⁺= 363.2. ¹H NMR (400 MHz, DMSO-d₆) δ 11.33 (s, 1H), 8.46 (s, 1H), 8.12 (s, 1H), 7.47 (s, 2H), 7.23 (s, 1H), 4.73 (d, *J* = 21.6 Hz, 2H), 3.64 (t, *J* = 5.2 Hz, 2H), 3.31 (s, 2H), 2.96-2.91 (m, 1H), 2.85-2.80 (m, 1H), 2.35-2.05 (m, 12H).

### Example 49. Synthesis of compound Z121

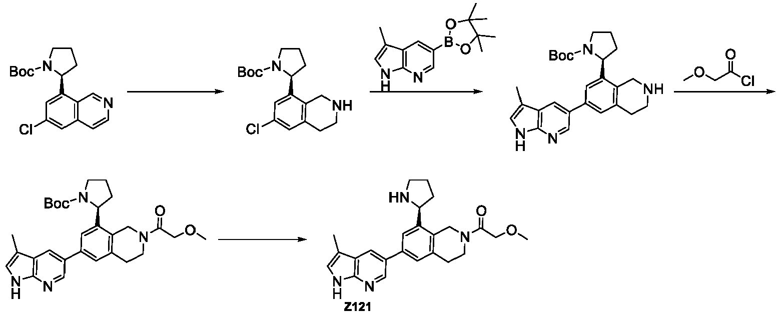

Step 1: Sodium borohydride (360 mg, 9.6 mmol) was added to a solution of tert-butyl (S)-2-(6-chloroisoquinolin-8-yl)pyrrolidine-1-carboxylate (800 mg, 2.4 mmol) in acetic acid (30 mL) at 0°C and stirred for 2 h. The reaction was monitored by LC-MS for completion, and quenched by the addition of sodium bicarbonate solution. The reaction mixture was extracted with dichloromethane, dried over anhydrous sodium sulfate, concentrated, and purified by column chromatography (0-10% methanol/dichloromethane, dichloromethane/methanol = 10/1, Rf = 0.6) to give tert-butyl (S)-2-(6-chloro-1,2,3,4-tetrahydroisoquinolin-8-yl)pyrrolidine-1-carboxylate (900 mg, crude product). ES-API:[M+H]⁺= 337.2.

Step 2: In the presence of protective nitrogen, 3-methyl-5-(4,4,5,5-tetramethyl-1,3,2-dioxaborolan-2-yl)-1*H*-pyrrolo[2,3-*b*]pyridine (921 mg, 3.57 mmol), chloro (2-dicyclohexylphosphino-2',6'-dimethoxy-1,1'-biphenylyl)(2'-amino-1,1'-biphenyl-2-yl) palladium (II) (173 mg, 0.24 mmmol) and potassium carbonate (985 mg, 7.14 mmmol) were added to a solution of the (S)-2-(6-chloro-1,2,3,4-tetrahydroisoquinolin-8-yl)pyrrolidine-1-carboxylate (800 mg, 2.38 mmol) in 1,4-dioxane (20 mL) and water (4 mL), and the mixture was stirred at 110°C for 2 h. The obtained mixture was extracted with ethyl acetate/water, dried over anhydrous sodium sulfate, concentrated, and purified by column chromatography (0-10% methanol/dichloromethane, dichloromethane/methanol = 10/1, Rf = 0.4) to give tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (495 mg, yield: 48%) as a yellow powder. ES-API:[M+H]⁺= 433.3.

Step 3: Triethylamine (21 mg, 0.21 mol) and 2-methoxyacetyl chloride (17 mg, 0.16 mmol) were added to a solution of the tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (50 mg, 0.12 mmol) in tetrahydrofuran (1 mL) at 0°C and stirred for 15 min. The reaction was monitored by LC-MS for completion, and quenched by the addition of water. The reaction mixture was extracted with dichloromethane, dried over anhydrous sodium sulfate, and concentrated to give tert-butyl (S)-2-(2-2-methoxyacetyl-6-(3-methyl-1H-pyrrolo[2,3-b]pyridin-5-yl)-1,2,3,4-tetrahydrois oquinolin-8-yl)pyrrolidine-1-carboxylate (60 mg, yield: 95%). ES-API:[M+H]⁺= 501.3.

Step 4: Hydrochloric acid-methanol solution (4M, 1 mL) was added to the tert-butyl (S)-2-(2-2-methoxyacetyl-6-(3-methyl-1H-pyrrolo[2,3-b]pyridin-5-yl)-1,2,3,4-tetrahydrois oquinolin-8-yl)pyrrolidine-1-carboxylate (60 mg, 0.11 mmol) and stirred at room temperature for 1 h. The reaction was monitored by LC-MS for completion. The reaction mixture was adjusted to pH 7 with aqueous sodium bicarbonate solution, extracted with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-1-(6-(3-methyl-1*H*-pyrrolo[2,3-*b*]pyridin-5-yl)-8-(pyrrolidin-2-yl)-3,4-dihydroisoquin olin-2(1H)-yl)methoxyethyl ketone (Z121, 18.2 mg, yield: 38%) as a white powder. ES-API:[M+H]⁺= 405.2. ¹H NMR (400 MHz, CDCl₃) δ 9.60 (s, 1H), 8.52 (s, 1H), 7.95 (s, 1H), 7.93 - 7.82 (m, 1H), 7.23 - 7.14 (m, 1H), 7.13 - 7.04 (m, 1H), 4.94 (d, *J* = 18.2 Hz, 1H), 4.81 - 4.65 (m, 1H), 4.62 - 4.46 (m, 1H), 4.34 (s, 1H), 4.18 (s, 1H), 3.87 - 3.58 (m, 2H), 3.45 (s, 3H), 3.30 - 3.12 (m, 1H), 3.06 - 2.75 (m, 3H), 2.44 - 2.25 (m, 4H), 2.16 - 1.76 (m, 4H).

### Example 50. Synthesis of compound Z122

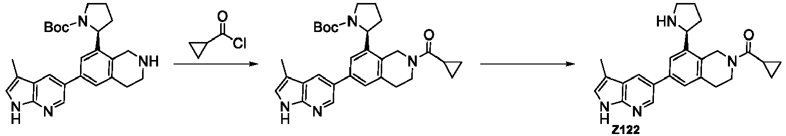

Step 1: Triethylamine (21 mg, 0.21 mmol) and cyclopropylcarbonyl chloride (17 mg, 0.14 mmol) to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (50 mg, 0.12 mmol) in dichloromethane (1 mL) were added under an ice bath condition and stirred for 15 min. The reaction was monitored by LC-MS for completion, and quenched by the addition of water. The reaction mixture was extracted with dichloromethane, dried over anhydrous sodium sulfate, concentrated, and purified by column chromatography (0-5% methanol/dichloromethane, dichloromethane/methanol = 20/1, Rf = 0.6) to give tert-butyl (S)-2-(2-cyclopropylcarbamoyl-6-(3-methyl-1H-pyrrolo[2,3-b]pyridin-5-yl)-1,2,3,4-tetrah ydroisoquinolin-8-yl)pyrrolidine-1-carboxylate (60 mg, yield: 95%). ES-API:[M+H]⁺= 501.3.

Step 2: 4M Hydrochloric acid/methanol solution (1 mL) was added to the tert-butyl (S)-2-(2-cyclopropylcarbamoyl-6-(3-methyl-1H-pyrrolo[2,3-b]pyridin-5-yl)-1,2,3,4-tetrah ydroisoquinolin-8-yl)pyrrolidine-1-carboxylate (60 mg, 0.11 mmol) and stirred at room temperature for 1 h. The reaction was monitored by LC-MS for completion. The reaction mixture was adjusted to pH 7 with sodium bicarbonate solution, extracted with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-1-(6-(3-methyl-1*H*-pyrrolo[2,3-*b*]pyridin-5-yl)-8-(pyrrolidin-2-yl)-3,4-dihydroisoquin olin-2(*1H*)-yl)cyclopropyl methyl ketone (Z122, 13.1 mg, yield: 25%) as a white powder. ES-API:[M+H]⁺= 401.2. ¹H NMR (400 MHz, CDCl₃) δ 9.42 (s, 1H), 8.52 (s, 1H), 8.08 - 7.88 (m, 1H), 7.83 (s, 1H), 7.25 - 7.17 (m, 1H), 7.16 - 6.98 (m, 1H), 4.93 (d, *J* = 25.9 Hz, 1H), 4.80 - 4.37 (m, 2H), 4.02 - 3.83 (m, 2H), 3.81 - 3.66 (m, 2H), 3.23 - 2.78 (m, 2H), 2.32 (s, 3H), 2.15 - 1.72 (m, 4H), 1.29 - 1.18 (m, 1H), 1.11 - 0.98 (m, 2H), 0.91 - 0.78 (m, 2H).

### Example 51. Synthesis of compound Z126

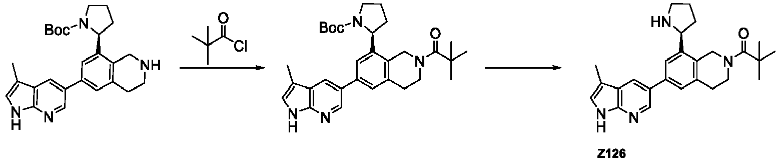

Step 1: Triethylamine (21.01 mg, 208.07 µmol) and tert-butylcarbonyl chloride (17 mg, 144.27 µmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (50 mg, 120.12 µmol) in dichloromethane (1 mL) and stirred at 0°C for 15 min. The reaction was monitored by LC-MS for completion, and quenched by the addition of water. The reaction mixture was extracted with dichloromethane, dried over anhydrous sodium sulfate, concentrated, and purified by column chromatography (0-5%, methanol in dichloromethane, dichloromethane/methanol = 20/1, Rf = 0.6) to give tert-butyl (S)-2-(2-tert-butylcarbamoyl-6-(3-methyl-1H-pyrrolo[2,3-b]pyridin-5-yl)-1,2,3,4-tetrahyd roisoquinolin-8-yl)pyrrolidine-1-carboxylate (60 mg, yield: 95%). ES-API:[M+H]+= 517.3.

Step 2: Hydrochloric acid-methanol solution was added to the tert-butyl (S)-2-(2-tert-butylcarbamoyl-6-(3-methyl-1H-pyrrolo[2,3-b]pyridin-5-yl)-1,2,3,4-tetrahyd roisoquinolin-8-yl)pyrrolidine-1-carboxylate (60 mg, 111.94 µmol) and stirred at room temperature for 1 h. The reaction was monitored by LC-MS for completion. The reaction mixture was adjusted to pH 7 with aqueous sodium bicarbonate solution, extracted with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-1-(6-(3-methyl-1H-pyrrolo[2,3-b]pyridin-5-yl)-8-pyrrolidin-2-yl)-3,4-dihydroisoquino lin-2(1H)-yl)tert-butyl methyl ketone (Z126, 16.4 mg, yield: 33%) as a white powder. ES-API:[M+H]⁺= 417.2. ¹H NMR (400 MHz, CDCl₃) δ 8.55 (s, 1H), 7.94 (s, 1H), 7.85 (s, 1H), 7.11 (s, 1H), 7.02 (s, 1H), 4.83 (s, 2H), 4.68 - 4.57 (m, 1H), 3.97 - 3.87 (m, 1H), 3.66 - 3.52 (m, 2H), 3.37 - 3.26 (m, 1H), 2.97 - 2.83 (m, 1H), 2.82 - 2.69 (m, 1H), 2.43 - 2.30 (m, 1H), 2.29 (s, 3H), 2.22 - 2.10 (m, 1H), 2.02 - 1.90 (m, 2H), 1.31 (s, 9H).

### Example 52. Synthesis of compound Z125

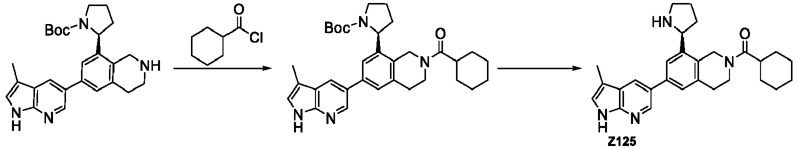

Step 1: Triethylamine (21.01 mg, 208.07 µmol) and cyclohexylcarbonyl chloride (21 mg, 144.27 µmol) were added to a solution of tert-butyl (S)-2-(6-(3-methyl-1*H*-pyrrolo[2,3-*b*]pyridin-5-yl)-1,2,3,4-tetrahydroisoquinolin-8-yl)pyrr olidine-1-carboxylate (50 mg, 120.12 µmol) in dichloromethane (1 mL) and stirred at 0°C for 15 min. The reaction was monitored by LC-MS for completion, and quenched by the addition of water. The reaction mixture was extracted with dichloromethane, dried over anhydrous sodium sulfate, concentrated, and purified by column chromatography (0-5%, methanol in dichloromethane, dichloromethane/methanol = 20/1, Rf = 0.6) to give tert-butyl (S)-2-(2-cyclohexylformyl-6-(3-methyl-1H-pyrrolo[2,3-b]pyridin-5-yl)-1,2,3,4-tetrahydroi soquinolin-8-yl)pyrrolidine-1-carboxylate (60 mg, yield: 95%). ES-API:[M+H]⁺= 543.4.

Step 2: Hydrochloric acid-methanol solution was added to the tert-butyl (S)-2-(2-cyclohexylformyl-6-(3-methyl-1H-pyrrolo[2,3-b]pyridin-5-yl)-1,2,3,4-tetrahydroi soquinolin-8-yl)pyrrolidine-1-carboxylate (60 mg, 111.94 µmol) and stirred at room temperature for 1 h. The reaction was monitored by LC-MS for completion. The reaction mixture was adjusted to pH 7 with sodium bicarbonate solution, extracted with dichloromethane/water, dried over anhydrous sodium sulfate, concentrated, and purified by prep-HPLC (column information: Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-1-(6-(3-methyl-1H-pyrrolo[2,3-b]pyridin-5-yl)-8-(pyrrolidin-2-yl)-3,4-dihydroisoquin olin-2(1H)-yl)cyclohexyl methyl ketone (Z125, 15.3 mg, yield: 31%) as a white powder. ES-API:[M+H]⁺= 443.3. ¹H NMR (400 MHz, CDCl₃) δ 9.86 (s, 1H), 8.53 (s, 1H), 8.01 - 7.78 (m, 2H), 7.24 - 6.89 (m, 2H), 5.14 - 4.70 (m, 2H), 4.72 - 4.43 (m, 1H), 3.80 - 3.47 (m, 3H), 3.32 - 3.14 (m, 1H), 2.90 - 2.50 (m, 3H), 2.29 (s, 3H), 2.18 - 2.00 (m, 2H), 1.96 - 1.65 (m, 6H), 1.64 - 1.46 (m, 2H), 1.45 - 1.14 (m, 4H).

### Example 53. Synthesis of compound Z123

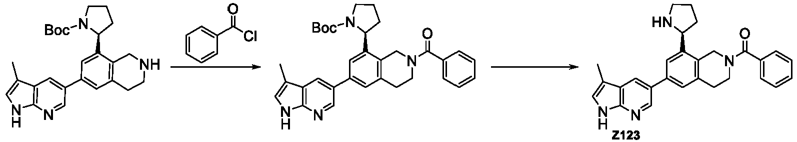

Step 1: Triethylamine (21 mg, 0.21 mmol) and benzoyl chloride (20 mg, 0.14 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (50 mg, 0.12 mmol) in dichloromethane (1 mL) under an ice bath condition and stirred for 15 min. The reaction was monitored by LC-MS for completion, and quenched by the addition of water. The reaction mixture was extracted with dichloromethane, dried over anhydrous sodium sulfate, concentrated, and purified by column chromatography (0-5% methanol/dichloromethane, dichloromethane/methanol = 20/1, Rf = 0.6) to give tert-butyl (S)-2-(2-benzoyl-6-(3-methyl-1H-pyrrolo[2,3-b]pyridin-5-yl)-1,2,3,4-tetrahydroisoquinoli n-8-yl)pyrrolidine-1-carboxylate (60 mg, yield: 95%). ES-API:[M+H]⁺= 537.30.

Step 2: 4M Hydrochloric acid/methanol solution (1 mL) was added to the tert-butyl (S)-2-(2-benzoyl-6-(3-methyl-1*H*-pyrrolo[2,3-b]pyridin-5-yl)-1,2,3,4-tetrahydroisoquinoli n-8-yl)pyrrolidine-1-carboxylate (60 mg, 0.11 mmol) and stirred at room temperature for 1 h. The reaction was monitored by LC-MS for completion. The reaction mixture was adjusted to pH 7 with sodium bicarbonate solution, extracted with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give a product (S)-1-(6-(3-methyl-1*H*-pyrrolo[2,3-*b*]pyridin-5-yl)-8-pyrrolidin-2-yl)-3,4-dihydroisoquino lin-2(1*H*)-yl)benzyl ketone (Z123, 11.3 mg, yield: 22%) as a white powder. ES-API:[M+H]⁺= 437.3. ¹H NMR (400 MHz, CDCl₃)δ8.59 (s, 1H), 8.12-7.73 (m, 2H), 7.50-7.38 (m, 5H), 7.19 - 6.94 (m, 2H),5.10-4.64 (m, 2H), 3.80-3.14 (m, 5H), 3.05-2.65 (m, 2H), 2.28 (s, 3H),2.20-1.73 (m, 4H).

### Example 54. Synthesis of compound Z124

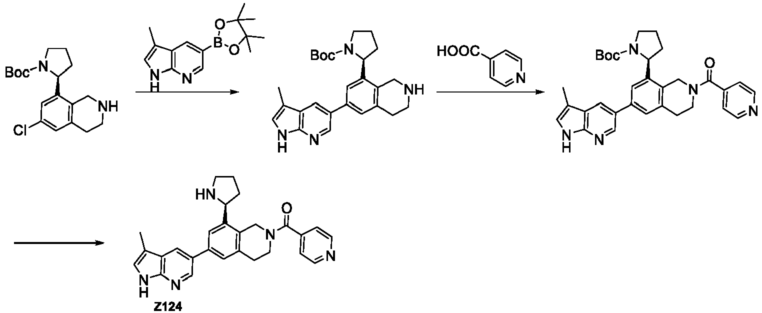

Step 1: In the presence of protective nitrogen, 3-methyl-5-(4,4,5,5-tetramethyl-1,3,2-dioxaborolan-2-yl)-1*H*-pyrrolo[2,3-*b*]pyridine (230 mg, 0.891 mmol), chloro (2-dicyclohexylphosphino-2',6'-dimethoxy-1,1'-biphenylyl)(2'-amino-1,1'-biphenyl-2-yl) palladium (II) (53 mg, 0.0742 mmmol) and potassium carbonate (307 mg, 2.23 mmmol) were added to tert-butyl (S)-2-(6-chloro-1,2,3,4-tetrahydroisoquinolin-8-yl)pyrrolidine-1-carboxylate (250 mg, 0.742 mmol), and the mixture was stirred at 110°C for 3 h. The obtained mixture was treated with ethyl acetate/water, dried over anhydrous sodium sulfate, concentrated, and purified by column chromatography (0-10% methanol (1% ammonia water):dichloromethane) to give tert-butyl (S)-2-(6-(3-methyl-1*H*-pyrrolo[2,3-b]pyridin-5-yl)-1,2,3,4-tetrahydroisoquinolin-8-yl)pyrr olidine-1-carboxylate (220 mg, yield: 68%) as a yellow powder. ES-API:[M+H]⁺= 433.3.

Step 2: Picolinic acid (17 mg, 0.138 mmol), triethylamine (35 mg, 0.347 mmol) and 2-(7-azobenzotriazole)-N,N,N',N'-tetramethylurea hexafluorophosphate (65 mg, 0.173 mmol) were added to a solution of the tert-butyl (S)-2-(6-(3-methyl-1*H*-pyrrolo[2,3-b]pyridin-5-yl)-1,2,3,4-tetrahydroisoquinolin-8-yl)pyrr olidine-1-carboxylate (50 mg, 0.115 mmol) in N,N-dimethylformamide (1 ml) and stirred at 25°C for 2 h. The reaction was monitored by LCMS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane) to give tert-butyl (S)-2-(6-(3-methyl-1*H*-pyrrolo[2,3-*b*]pyridin-5-yl)-2-picolyl-1,2,3,4-tetrahydroisoquinolin -8-yl)pyrrolidine-1-carboxylate (55 mg, 89%). ES-API:[M+H]⁺= 538.3.

Step 3: 4M Hydrochloric acid/methanol solution (1 mL) was added to the tert-butyl (S)-2-(6-(3-methyl-1*H*-pyrrolo[2,3-*b*]pyridin-5-yl)-2-picolyl-1,2,3,4-tetrahydroisoquinolin -8-yl)pyrrolidine-1-carboxylate (55 mg, 0.102 mmol) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, concentrated, and purified by preparative thin layer chromatography (methanol:dichloromethane = 10:1) to give (S)-(6-(3-methyl-1*H*-pyrrolo[2,3-*b*]pyridin-5-yl)-8-(pyrrolidin-2-yl)-3,4-dihydroisoquinoli n-2(1*H*)-yl)(pyridin-4-yl)methanone (Z124, 10.2 mg, yield: 23%) as a white powder. ES-API:[M+H]⁺= 438.2. ¹H NMR (400 MHz, CDCl₃) δ 8.74 - 8.59 (m, 2H), 8.43 (d, *J =* 8.8 Hz, 1H), 7.82 (d, *J* = 11.6 Hz, 1H), 7.78 - 7.53 (m, 1H), 7.34 - 7.20 (m, 2H), 7.20 - 7.05 (m, 1H), 6.96 (s, 1H), 5.09 - 4.53 (m, 2H), 4.52 - 3.86 (m, 1H), 3.51 - 2.38 (m, 6H), 2.21 (s, 3H), 2.15 - 1.48 (m, 4H).

### Example 55. Synthesis of compound Z127

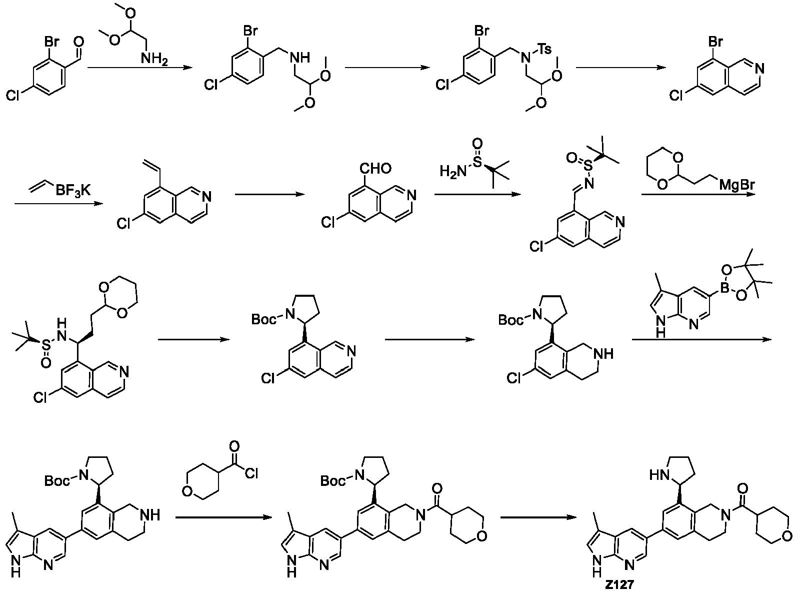

Step 1: 2-Bromo-4-chlorobenzaldehyde (50 g, 0.22 mol) was dissolved in toluene. 2,2-Dimethoxyethanamine (34 g, 0.33 mmol) and acetic acid (4 mL) were added, and the system was heated to 110°C and stirred for 3 h in the presence of protective nitrogen. The obtained mixture was spun to dryness. Methanol (200 mL) was added, and the system was cooled to 0°C. Sodium borohydride (15 g, 0.4 mol) was slowly added, and the system reacted at room temperature for 1 h. Upon completion of the reaction, the solution was poured into ice water, extracted with dichloromethane, dried over anhydrous sodium sulfate, and spun to dryness to give N-(2-bromo-4-chlorobenzyl)-2,2-dimethoxyethanamine (61 g, crude product). ES-API:[M+H]⁺= 310.0.

Step 2: The N-(2-bromo-4-chlorobenzyl)-2,2-dimethoxyethanamine (61g, 0.23 mmol) was added to dichloromethane (150 mL). Triethylamine (34.8 g, 0.344 mol) was added. p-Toluenesulfonyl chloride (65.6 g, 0.344 mol) was added under an ice-water bath condition, and the system reacted at room temperature overnight. Upon completion of the reaction, water was added. The obtained mixture was extracted with dichloromethane and spun to dryness. The crude product was beaten with petroleum ether to give N-(2-bromo-4-chlorobenzyl)-N-(2,2-dimethoxyethyl)-4-methylbenzenesulfonamide (35 g, yield: 38%). ES-API:[M+Na]⁺= 486.0.

Step 3: The N-(2-bromo-4-chlorobenzyl)-N-(2,2-dimethoxyethyl)-4-methylbenzenesulfonamide (35 g, 76 mmol) was added to anhydrous dichloromethane (150 mL). Aluminum trichloride (80 g, 0.6 mol) was added under an ice-water bath condition and stirred at room temperature overnight. Upon completion of the reaction, the reaction mixture was added to ice water. Sodium hydroxide solution was added until a clear solution was obtained. The obtained mixture was extracted with dichloromethane, spun to dryness, and purified by column chromatography (0-20% ethyl acetate/petroleum ether, petroleum ether/ethyl acetate = 5/1, Rf = 0.5 mobile phase) to give 8-bromo-6-chloroisoquinoline (3.5 g, yield: 19%). ES-API:[M+H]⁺= 243.9.

Step 4: The 8-bromo-6-chloroisoquinoline (3.5 g, 14.4 mmol), potassium vinyltrifluoroborate (5.912 g, 43.2 mmol) and triethylamine (4.37 g, 43.2 mmol) were dissolved in ethanol (35 mL). 1,1-Bis(diphenylphosphino)ferrocene dichloropalladium (1.05 g, 1.44 mmol) was then added, and the system was heated to 80°C and stirred for 2 h in the presence of protective nitrogen. Upon completion of the reaction, the reaction mixture was filtered, spun to dryness, extracted with ethyl acetate/water, spun to dryness, and purified by column chromatography (0-20% ethyl acetate/petroleum ether, petroleum ether/ethyl acetate = 511, Rf = 0.4 mobile phase) to give 6-chloro-8-vinylisoquinoline (1.6 g, yield: 57%). ES-API:[M+H]⁺= 190.0.

Step 5: The 6-chloro-8-vinylisoquinoline (1.6 g, 8.46 mmol) was dissolved in tetrahydrofuran (32 mL). 2,6-Lutidine (0.9 g, 8.46 mmol) was added and stirred at room temperature. Sodium periodate (14.47 g, 67.68 mmol) and potassium osmate dihydrate (0.93 g, 2.538 mmol) were added to water (16 mL). The aqueous solution was added to a reaction flask under an ice bath condition and stirred at room temperature for 1 h. Upon completion of the reaction, the reaction was quenched by the addition of aqueous sodium thiosulfate solution. The reaction mixture was filtered, extracted with ethyl acetate, spun to dryness, and purified by column chromatography (0-30% ethyl acetate/petroleum ether, petroleum ether/ethyl acetate = 2/1, Rf = 0.5 mobile phase) to give 6-chloroisoquinoline-8-carbaldehyde (0.8 g, yield: 50%). ES-API:[M+H]⁺= 192.1.

Step 6: The 6-chloroisoquinoline-8-carbaldehyde (0.8 g, 4.18 mmol) and (S)-2-methylpropane-2-sulfinamide (1.0 g, 8.37 mmol) were added to dichloromethane (20 mL) followed by tetraethyl titanate (2.74 g, 16.72 mmol), and the system reacted at room temperature overnight. Upon completion of the reaction, saturated sodium chloride solution was added followed by dichloromethane. The obtained mixture was extracted and spun to dryness to give (S,E)-N-((6-chloroisoquinolin-8-yl)methylene)-2-methylpropane-2-sulfinamide (1.15 g, yield: 93%). ES-API:[M+H]⁺= 295.1.

Step 7: The (S,E)-N-((6-chloroisoquinolin-8-yl)methylene)-2-methylpropane-2-sulfinamide (1.15 g, 3.92 mmol) was added to tetrahydrofuran (20 mL), and the system was cooled to -78°C in a dry ice bath. (2-(1,3-dioxan-2-yl)ethyl)magnesium bromide (3.4 g, 3.92 mmol) was added and stirred for 10 min. The dry ice bath was removed, and a saturated ammonium chloride solution was added. The obtained mixture was extracted with ethyl acetate, spun to dryness, and purified by column chromatography (0-30% ethyl acetate/petroleum ether, petroleum ether/ethyl acetate = 2/1, Rf = 0.5) to give (S)-N-((S)-1-(6-chloroisoquinolin-8-yl)-3-(1,3-dioxan-2-yl)propyl)-2-methylpropane-2-sul finamide (2.0 g, crude product). ES-API:[M+H]⁺= 411.1.

Step 8: The (S)-N-((S)-1-(6-chloroisoquinolin-8-yl)-3-(1,3-dioxan-2-yl)propyl)-2-methylpropane-2-sul finamide (2.0 g, 4.87 mmol) was added to trifluoroacetic acid (50 mL) and water (2.5 mL) and stirred at room temperature for 30 min. Triethylsilane (5.66 g, 48.7 mmol) was added and stirred at room temperature for 2 h. Upon completion of the reaction, the reaction mixture was spun to dryness. Tetrahydrofuran (40 mL), triethylamine (4.9 g, 48.7 mmol) and di-tert-butyl dicarbonate (3.18 g, 14.61 mmol) were added and stirred at room temperature for 30 min. Upon completion of the reaction, the reaction mixture was extracted with ethyl acetate, spun to dryness, and purified by column chromatography (0-30% ethyl acetate/petroleum ether, petroleum ether/ethyl acetate = 4/1, Rf = 0.3 mobile phase) to give tert-butyl (S)-2-(6-chloroisoquinolin-8-yl)pyrrolidine-1-carboxylate (0.9 g, yield: 56%). ES-API:[M+H]⁺= 333.1.

Step 9: Sodium borohydride (18 mg, 0.48 mmol) was added to a solution of the tert-butyl (S)-2-(6-chloroisoquinolin-8-yl)pyrrolidine-1-carboxylate (40 mg, 0.12 mmol) in acetic acid (1.5 mL) and stirred at 0°C for 2 h. The reaction was monitored by LC-MS for completion, and quenched by the addition of sodium bicarbonate solution. The reaction mixture was extracted with dichloromethane, dried over anhydrous sodium sulfate, concentrated, and purified by column chromatography (0-10%, methanol in dichloromethane, dichloromethane/methanol = 4/1, Rf = 0.6) to give tert-butyl (S)-2-(6-chloro-1,2,3,4-tetrahydroisoquinolin-8-yl)pyrrolidine-1-carboxylate (18 mg, yield: 45%). ES-API:[M+H]⁺= 337.2.

Step 10: 3-Methyl-5-(4,4,5,5-tetramethyl-1,3,2-dioxaborolan-2-yl)-1H-pyrrolo[2,3-b]pyridine (21 mg, 0.081 mmol), S-phosPdG2 (4 mg, 0.0054 mmmol) and potassium carbonate (22 mg, 0.162 mmmol) were added to the tert-butyl (S)-2-(6-chloro-1,2,3,4-tetrahydroisoquinolin-8-yl)pyrrolidine-1-carboxylate (18 mg, 0.054 mmol), and the mixture was heated to 110°C and stirred in the presence of protective nitrogen. The reaction was monitored by LC-MS for completion. The reaction mixture was treated with ethyl acetate/water, dried over anhydrous sodium sulfate, concentrated, and purified by column chromatography (mobile phase 0-10% methanol/dichloromethane, dichloromethane/methanol = 10/1, Rf = 0.4) to give tert-butyl (S)-2-(6-(3-methyl-1H-pyrrolo[2,3-b]pyridin 5-yl)-1,2,3,4-tetrahydroisoquinolin-8-yl)pyrrolidine-1-carboxylate (8 mg, yield: 35%) as a yellow powder. ES-API:[M+H]⁺= 433.3.

Step 11: Triethylamine (5.8 mg, 0.057 mmol) and tetrahydro-2H-pyran-4-carbonyl chloride (3.4 mg, 0.023 mmol) were added to a solution of the tert-butyl (S)-2-(6-(3-methyl-1H-pyrrolo[2,3-b]pyridin 5-yl)-1,2,3,4-tetrahydroisoquinolin-8-yl)pyrrolidine-1-carboxylate (8 mg, 0.019 mmol) in tetrahydrofuran (1 mL) and stirred at 0°C for 15 min. The reaction was monitored by LC-MS for completion, and quenched by the addition of water. The reaction mixture was extracted with dichloromethane, dried over anhydrous sodium sulfate, and concentrated to give tert-butyl (S)-2-(2-tetrahydro-2H-pyran-4-formyl-6-(3-methyl-1H-pyrrolo[2,3-b]pyridin-5-yl)-1,2,3, 4-tetrahydroisoquinolin-8-yl)pyrrolidine-1-carboxylate (9 mg, yield: 87%). ES-API:[M+H]⁺= 545.4.

Step 12: Hydrochloric acid-methanol solution was added to the tert-butyl (S)-2-(2-tetrahydro-2H-pyran-4-formyl-6-(3-methyl-1H-pyrrolo[2,3-b]pyridin-5-yl)-1,2,3, 4-tetrahydroisoquinolin-8-yl)pyrrolidine-1-carboxylate (9 mg, 0.016 mmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-(6-(3-methyl-1H-pyrrolo[2,3-b]pyridin-5-yl)-8-(pyrrolidin-2-yl)-3,4-dihydroisoquinoli n-2(1H)-yl)(tetrahydro-2H-pyran-4-yl)methyl ketone (Z127, 1.5 mg, yield: 21%) as a white powder. ES-API:[M+H]⁺= 445.2.

### Example 56. Synthesis of compound Z128

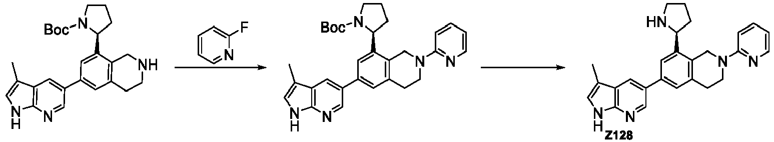

Step 1: 2-Fluoropyridine (10.77 mg, 0.11 mmol) and cesium carbonate (90.44 mg, 277.42 µmol) was added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (40 mg, 92.47 µmol) in N,N-dimethylacetamide (0.4 mL), and the system reacted under microwave at 160°C for 2 h. The reaction was monitored by LC-MS for completion. The reaction mixture was post-treated with ethyl acetate and water, dried over anhydrous sodium sulfate, concentrated, and purified by column chromatography (0-5% methanol in dichloromethane, dichloromethane/methanol = 10/1, Rf = 0.7) to give tert-butyl (S)-2-(6-(3-methyl-1H-pyrrolo[2,3-b]pyridin-5-yl)-2-(pyridin-2-yl)-1,2,3,4-tetrahydroisoq uinolin-8-yl)pyridine-1-carboxylate (28 mg, yield: 59.41%). ES-API:[M+H]⁺= 510.3.

Step 2: Hydrochloric acid-methanol solution was added to the tert-butyl (S)-2-(6-(3-methyl-1H-pyrrolo[2,3-b]pyridin-5-yl)-2-(pyridin-2-yl)-1,2,3,4-tetrahydroisoq uinolin-8-yl)pyridine-1-carboxylate (30 mg, 72.65 µmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-6-(3-methyl-1H-pyrrolo[2,3-b]pyridin-5-yl)-2-(pyridin-2-yl)-8-(pyrrolidin-2-yl)-1,2,3, 4-tetrahydroisoquinoline (Z128, 3.5 mg, yield: 12.44%) as a white powder. ES-API:[M+H]⁺= 410.2.

### Example 57. Synthesis of compound Z130

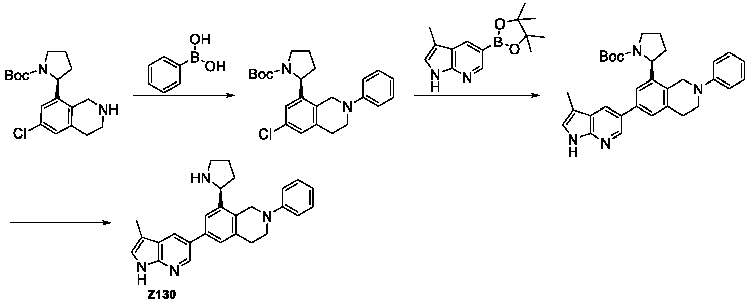

Step 1: Phenylboronic acid (34.75 mg, 284.99 µmol), pyridine (37.57 mg, 474.98 µmol, 38.26 µL) and copper acetate (64.70 mg, 356.24 µmol) were added to a solution of the tert-butyl 2-(6-chloro-1,2,3,4-tetrahydroisoquinolin-8-yl)pyrrolidine-1-carboxylate (80 mg, 237.49 µmol) in dichloromethane (1 mL), and the mixture was stirred at 25°C for 16 h. The reaction was monitored by LC-MS for completion. The reaction mixture was post-treated with dichloromethane and water, dried over anhydrous sodium sulfate, concentrated, and purified by column chromatography (0-5%, methanol in dichloromethane) to give tert-butyl (S)-2-(6-chloro-2-phenyl-3,4-dihydro-1H-isoquinolin-8-yl)pyrrolidine-1-carboxylate (30 mg, yield: 30.59%). ES-API:[M+H]⁺= 413.2.

Step 2: 3-Methyl-5-(4,4,5,5-tetramethyl-1,3,2-dioxaborolan-2-yl)-1H-pyrrolo[2,3-b]pyridine (28.13 mg, 108.97 µmol), chloro (2-dicyclohexylphosphino-2', 6' -dimethoxy-1,1' -biphenyl) [2-(2' -amino-1,1' -biphenyl)]pal ladium (II) (5.24 mg, 7.26 µmol) and potassium carbonate (30.12 mg, 217.94 µmol) were added to a solution of the tert-butyl (S)-2-(6-chloro-2-phenyl-3,4-dihydro-1H-isoquinolin-8-yl)pyrrolidine-1-carboxylate (30 mg, 72.65 µmol) in dioxane (0.5 mL), and the mixture was stirred at 110°C for 2 h. The reaction was monitored by LC-MS for completion. The reaction mixture was treated with dichloromethane/water, dried over anhydrous sodium sulfate, concentrated, and purified by column chromatography (0-10% = methanol/dichloromethane) to give tert-butyl (S)-2-(6-3-methyl-1H-pyrrolo[2,3-b]pyridin-5-yl-2-phenyl-3,4-dihydro-1H-isoquinolin-8-yl)pyrrolidine-1-carboxylate (20 mg, yield: 54.02%). ES-API:[M+H]+= 442.4.

Step 3: Hydrochloric acid-methanol solution was added to the tert-butyl (S)-2-(6-3-methyl-1H-pyrrolo[2,3-b]pyridin-5-yl-2-phenyl-3,4-dihydro-1H-isoquinolin-8-yl)pyrrolidine-1-carboxylate (20 mg, 39.24 µmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (column information: Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-6-(3-methyl-1H-pyrrolo[2,3-b]pyridin-5-yl)-2-phenyl-8-(pyrrolidin-2-yl)-1,2,3,4-tetra hydroisoquinoline (Z130, 2.4 mg, yield: 15%) as a white powder. ES-API:[M+H]⁺= 442.4.

### Example 58. Synthesis of compound Z210

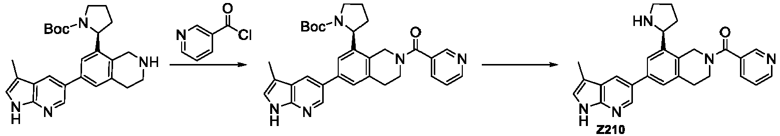

Step 1: Triethylamine (21.01 mg, 208.07 µmol) and nicotinoyl chloride (11.78 mg, 83.23 µmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 69.36 µmol) in dichloromethane (1 ml) and stirred at 0°C for 15 min. The reaction was monitored by LC-MS for completion, and quenched by the addition of water. The reaction mixture was extracted with dichloromethane, dried over anhydrous sodium sulfate, and concentrated to give tert-butyl (S)-2-(2-nicotinoyl-6-(3-methyl-1H-pyrrolo[2,3-b]pyridin-5-yl)-1,2,3,4-tetrahydroisoquin olin-8-yl)pyrrolidine-1-carboxylate (35 mg, yield: 93.86%). ES-API:[M+H]⁺= 538.3.

Step 2: Hydrochloric acid-methanol solution was added to the tert-butyl (S)-2-(2-nicotinoyl-6-(3-methyl-1H-pyrrolo[2,3-b]pyridin-5-yl)-1,2,3,4-tetrahydroisoquin olin-8-yl)pyrrolidine-1-carboxylate (35 mg, 65.10 µmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (column information: Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-1-(6-(3-methyl-1H-pyrrolo[2,3-b]pyridin-5-yl)-8-(pyrrolidin-2-yl)-3,4-dihydroisoquin olin-2(1H)-yl)(pyridin-3-yl)methanone (Z210, 1.2 mg, yield: 4.2%) as a white powder. ES-API:[M+H]⁺=438.2.

### Example 59. Synthesis of compound Z211

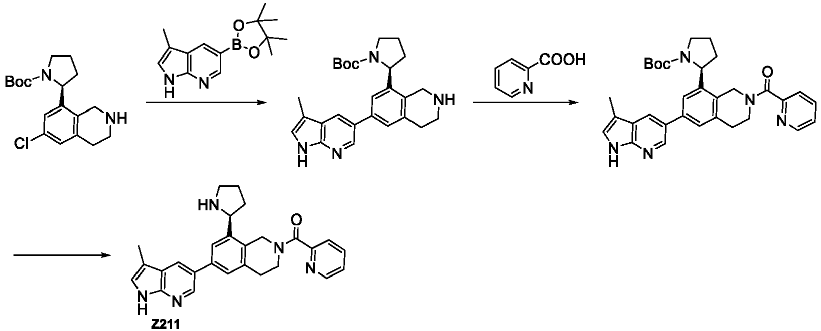

Step 1: 3-Methyl-5-(4,4,5,5-tetramethyl-1,3,2-dioxaborolan-2-yl)-1H-pyrrolo[2,3-b]pyridine (230 mg, 0.891 mmol), S-phosPdG2 (53 mg, 0.0742 mmmol) and potassium carbonate (307mg, 2.23 mmmol) were added to tert-butyl (S)-2-(6-chloro-1,2,3,4-tetrahydroisoquinolin-8-yl)pyrrolidine-1-carboxylate (250 mg, 0.742 mmol), and the mixture was heated to 110°C and stirred in the presence of protective nitrogen. The reaction was monitored by LC-MS for completion. The reaction mixture was treated with ethyl acetate/water, dried over anhydrous sodium sulfate, concentrated, and purified by column chromatography (0-10% methanol/dichloromethane, dichloromethane/methanol = 10/1, Rf = 0.4 mobile phase) to give tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (220 mg, yield: 68%) as a yellow powder. ES-API:[M+H]⁺= 433.3.

Step 2: Picolinic acid (10.2 mg, 0.083 mmol), triethylamine (21 mg, 0.208 mmol) and N,N,N',N'-tetramethyl-O-(7-azabenzotriazol-1-yl)urea hexafluorophosphate (39.2 mg, 0.104 mmol) were added to a solution of the tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in N,N-dimethylformamide (1 mL) was stirred at 25°C for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol = 10/1, Rf = 0.6) to give tert-butyl (S)-2-(6-(3-methyl-1H-pyrrolo[2,3-b]pyridin-5-yl)-2-picolyl-1,2,3,4-tetrahydroisoquinolin -8-yl)pyrrolidine-1-carboxylate (24 mg, yield: 64 %). ES-API:[M+H]⁺= 538.3.

Step 3: Hydrochloric acid-methanol solution was added to the tert-butyl (S)-2-(6-(3-methyl-1H-pyrrolo[2,3-b]pyridin-5-yl)-2-picolyl-1,2,3,4-tetrahydroisoquinolin -8-yl)pyrrolidine-1-carboxylate (24 mg, 0.045 mmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-(6-(3-methyl-1H-pyrrolo[2,3-b]pyridin-5-yl)-8-(pyrrolidin-2-yl)-3,4-dihydroisoquinoli n-2(1H)-yl)(pyridin-2-yl)methanone (Z211, 5.9 mg, yield: 30%) as a white powder. ES-API:[M+H]⁺= 438.2.

### Example 60. Synthesis of compound Z212

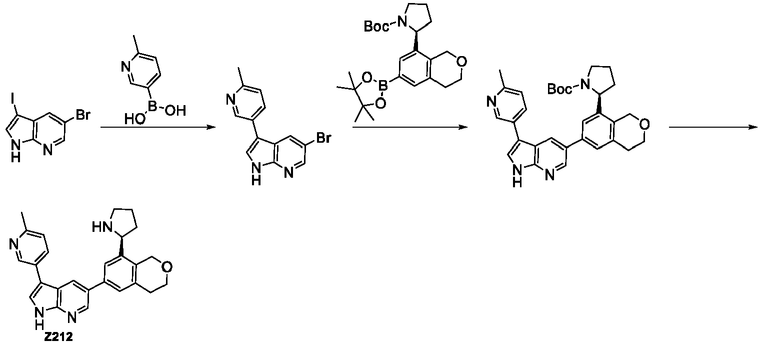

Step 1: (6-Methylpyridin-3-yl)boronic acid (305 mg, 2.23 mmol), 1,1'-bis(diphenylphosphino)ferrocene dichloropalladium (II) (136 mg, 0.186mmol) and potassium carbonate (513 mg, 3.72mmol) were added to a solution of 5-bromo-3-iodo-1*H*-pyrrolo[2,3-*b*]pyridine (600 mg, 1.86 mmol) in dioxane/water (5 mL/1 mL) and stirred at 80°C overnight. The reaction was monitored by LC-MS for completion, and quenched by the addition of water. The reaction mixture was extracted with dichloromethane, dried over anhydrous sodium sulfate, concentrated, and purified by column chromatography (0-10%, methanol/dichloromethane, dichloromethane/methanol = 10/1, Rf = 0.5) to give 5-bromo-3-(6-methylpyridin-3-yl)-1H-pyrrolo[2,3-b]pyridine (24 mg, yield: 4.5%). ES-API:[M+H]⁺= 288.0.

Step 2: tert-butyl (S)-2-(6-(4,4,5,5-tetramethyl-1,3,2-dioxaborolan-2-yl)isochroman-8-yl)pyrrolidine-1-carb oxylate (37 mg, 0.087 mmol), chloro (2-dicyclohexylphosphino-2',6'-dimethoxy-1,1'-biphenylyl)(2'-amino-1,1'-biphenyl-2-yl) palladium (II) (6 mg, 0.0087 mmol) and potassium carbonate (24 mg, 0.173 mmol) were added to a solution of the 5-bromo-3-(6-methylpyridin-3-yl)-1H-pyrrolo[2,3-b]pyridine (35 mg, 0.087 mmol) in dioxane/water (1 mL/0.2 mL), and the system reacted under microwave at 100°C for 2 h. The reaction was monitored by LC-MS for completion, and quenched by the addition of water. The reaction mixture was extracted with dichloromethane, dried over anhydrous sodium sulfate, concentrated, and purified by column chromatography (0-10%, methanol/dichloromethane) to give tert-butyl (S)-2-(6-(3-(6-methylpyridin-3-yl)-1*H*-pyrrolo[2,3-*b*]pyridin-5-yl)isochroman-8-yl)pyrroli dine-1-carboxylate (8 mg, yield: 18 %). ES-API:[M+H]⁺=511.3.

Step 3: Trifluoroacetic acid (0.2 mL) in dichloromethane (0.4 mL) was added to the tert-butyl (S)-2-(6-(3-(6-methylpyridin-3-yl)-1*H*-pyrrolo[2,3-*b*]pyridin-5-yl)isochroman-8-yl)pyrroli dine-1-carboxylate (8 mg, 0.016 mmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 mL/min, gradient: B/A = 20%-90% over 50 min, wavelength: 214 nm, column temperature: room temperature) to give (S)-3-(6-methylpyridin-3-yl)-5-(8-(pyrrolidin-2-yl)isochroman-6-yl)-1*H*-pyrrolo[2,3-*b*]pyr idine (Z212, 0.7 mg, yield: 11%). ES-API:[M+H]⁺=411.2.

### Example 61. Synthesis of compound Z213

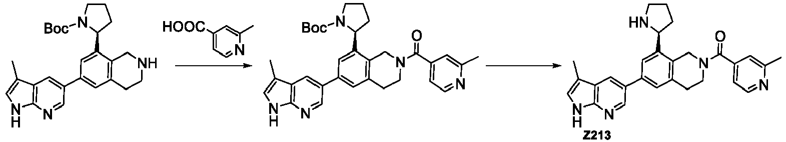

Step 1: 2-Methylisonicotinic acid (9.5 mg, 0.069 mmol), triethylamine (21 mg, 0.208 mmol) and N,N,N',N'-tetramethyl-O-(7-azabenzotriazol-1-yl)urea hexafluorophosphate (39.2 mg, 0.104 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in N,N-dimethylformamide (1 mL) and stirred at 25°C for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol = 10/1, Rf = 0.6) to give tert-butyl (S)-2-(6-(3-methyl-1H-pyrrolo[2,3-b]pyridin-5-yl)-2-(2-methylisonicotinoyl)-1,2,3,4-tetra hydroisoquinolin-8-yl)pyrrolidine-1-carboxylate (30 mg, yield: 78 %). ES-API:[M+H]⁺= 552.4.

Step 2: Hydrochloric acid/methanol solution was added to the tert-butyl (S)-2-(6-(3-methyl-1H-pyrrolo[2,3-b]pyridin-5-yl)-2-(2-methylisonicotinoyl)-1,2,3,4-tetra hydroisoquinolin-8-yl)pyrrolidine-1-carboxylate (30 mg, 0.054 mmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-(6-(3-methyl-1H-pyrrolo[2,3-b]pyridin-5-yl)-8-(pyrrolidin-2-yl)-3,4-dihydroisoquinoli n-2(1H)-yl)(2-methylpyridin-4-yl)methanone (Z213, 3.9 mg, yield: 15%) as a white powder. ES-API:[M+H]⁺= 452.3. ¹H NMR (400 MHz, CDCl₃) δ 8.63 - 8.50 (m, 2H), 7.99 - 7.79 (m, 2H), 7.20 - 6.96 (m, 4H), 4.97 (s, 1H), 4.82 (s, 1H), 4.63 - 4.29 (m, 1H), 3.85 - 3.66 (m, 1H), 3.58 - 3.44 (m, 2H), 3.40 - 3.25 (m, 1H), 3.06 - 2.70 (m, 1H), 2.61 - 2.57 (m, 3H), 2.50 - 2.33 (m, 1H), 2.28 (s, 3H), 2.24 - 1.90 (m, 4H).

### Example 62. Synthesis of compound Z214

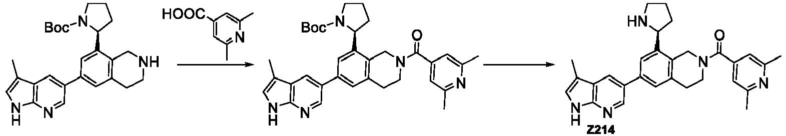

Step 1: 2,6-Dimethylisonicotinic acid (10.5 mg, 0.069 mmol), triethylamine (21 mg, 0.208 mmol) and N,N,N',N'-tetramethyl-O-(7-azabenzotriazol-1-yl)urea hexafluorophosphate (39.2 mg, 0.104 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in N,N-dimethylformamide (1 mL) and stirred at 25°C for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol/ 10/1, Rf = 0.6) to give tert-butyl (S)-2-(6-(3-methyl-1H-pyrrolo[2,3-b]pyridin-5-yl)-2-(2,6-dimethylisonicotinoyl)-1,2,3,4-t etrahydroisoquinolin-8-yl)pyrrolidine-1-carboxylate (31 mg, yield: 79%). ES-API:[M+H]⁺= 566.4.

Step 2: Hydrochloric acid/methanol solution was added to the tert-butyl (S)-2-(6-(3-methyl-1H-pyrrolo[2,3-b]pyridin-5-yl)-2-(2,6-dimethylisonicotinoyl)-1,2,3,4-t etrahydroisoquinolin-8-yl)pyrrolidine-1-carboxylate (31 mg, 0.055 mmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (column information: Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-(6-(3-methyl-1H-pyrrolo[2,3-b]pyridin-5-yl)-8-(pyrrolidin-2-yl)-3,4-dihydroisoquinoli n-2(1H)-yl)(2,6-dimethylpyridin-4-yl)methanone (Z214, 5.61 mg, yield: 22%) as a white powder. ES-API:[M+H]⁺= 466.3. ¹H NMR (400 MHz, CDCl₃) δ 8.52 (d, *J* = 14.2 Hz, 1H), 7.92 (d, *J* = 8.4 Hz, 2H), 7.18 - 7.08 (m, 1H), 7.08 - 6.98 (m, 2H), 6.93 (s, 1H), 5.04 - 4.88 (m, 1H), 4.81 - 4.56 (m, 1H), 4.38 - 4.12 (m, 1H), 3.68 - 3.49 (m, 1H), 3.48 - 3.28 (m, 2H), 3.13 - 2.63 (m, 3H), 2.55 (s, 6H), 2.31 (s, 3H), 2.24 - 2.12 (m, 1H), 2.09 - 1.95 (m, 3H).

### Example 63. Synthesis of compound Z215

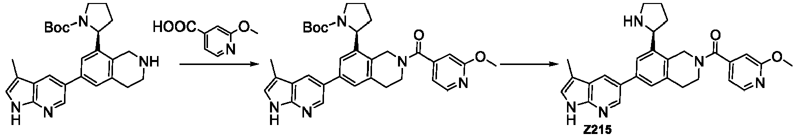

Step 1: 2-Methoxyisonicotinic acid (10.6 mg, 0.069 mmol), triethylamine (21 mg, 0.208 mmol) and N,N,N',N'-tetramethyl-O-(7-azabenzotriazol-1-yl)urea hexafluorophosphate (39.2 mg, 0.104 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in N,N-dimethylformamide (1 mL) and stirred at 25°C for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol= 10/1, Rf = 0.6) to give tert-butyl (S)-2-(6-(3-methyl-1H-pyrrolo[2,3-b]pyridin-5-yl)-2-(2-methoxyisonicotinoyl)-1,2,3,4-tet rahydroisoquinolin-8-yl)pyrrolidine-1-carboxylate (31 mg, yield: 79%). ES-API:[M+H]⁺= 568.4.

Step 2: Hydrochloric acid-methanol solution was added to the tert-butyl (S)-2-(6-(3-methyl-1H-pyrrolo[2,3-b]pyridin-5-yl)-2-(2-methoxyisonicotinoyl)-1,2,3,4-tet rahydroisoquinolin-8-yl)pyrrolidine-1-carboxylate (31 mg, 0.055 mmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-(6-(3-methyl-1H-pyrrolo[2,3-b]pyridin-5-yl)-8-(pyrrolidin-2-yl)-3,4-dihydroisoquinoli n-2(1H)-yl)(2-methoxypyridin-4-yl)methanone (Z215, 2.6 mg, yield: 10%) as a white powder. ES-API:[M+H]⁺= 468.3.

### Example 64. Synthesis of compound Z216

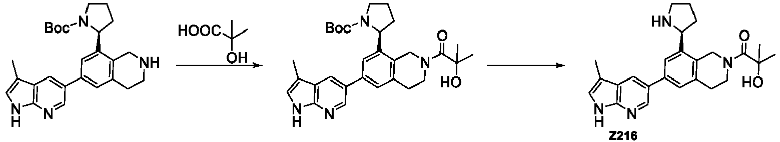

Step 1: 2-Hydroxy-2-methylpropanoic acid (7.2 mg, 0.069 mmol), triethylamine (21 mg, 0.208 mmol) and 2-(7-azobenzotriazole)-N,N,N',N'-tetramethyluronium hexafluorophosphate (39.2 mg, 0.104 mmol) were added to a solution of tert-butyl (S)-2-(6-(3-methyl-1*H*-pyrrolo[2,3-*b*]pyridin-5-yl)-1,2,3,4-tetrahydroisoquinolin-8-yl)pyrr olidine-1-carboxylate (30 mg, 0.069 mmol) in N,N-dimethylformamide (1 mL) and stirred at room temperature for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol = 10/1, Rf = 0.6) to give tert-butyl (S)-2-(2-(2-hydroxy-2-methylpropanoyl)-6-(3-methyl-1*H*-pyrrolo[2,3-*b*]pyridin-5-yl)-1,2, 3,4-tetrahydroisoquinolin-8-yl)pyrrolidine-1-carboxylate (30 mg, yield: 83%). ES-API:[M+H]⁺= 519.4.

Step 2: 4M Hydrochloric acid/methanol solution (1 mL) was added to the tert-butyl (S)-2-(2-(2-hydroxy-2-methylpropanoyl)-6-(3-methyl-1*H*-pyrrolo[2,3-*b*]pyridin-5-yl)-1,2, 3,4-tetrahydroisoquinolin-8-yl)pyrrolidine-1-carboxylate (30 mg, 0.058 mmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-2-hydroxy-2-methyl-1-(6-(3-methyl-1*H*-pyrrolo[2,3-*b*]pyridin-5-yl)-8-(pyrrolidin-2-yl )-3,4-dihydroisoquinolin-2(1*H*)-yl)propan-1-one (Z216, 3.1 mg, yield: 14 %) as a white powder. ES-API:[M+H]⁺=419.3.

### Example 65. Synthesis of compound Z34-1

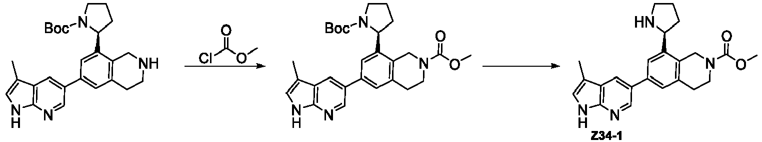

Step 1: Methyl chloroformate (7.86 mg, 0.069 mmol) and triethylamine (21 mg, 0.208 mmol) were added to a solution of tert-butyl (S)-2-(6-(3-methyl-1*H*-pyrrolo[2,3-b]pyridin-5-yl)-1,2,3,4-tetrahydroisoquinolin-8-yl)pyrr olidine-1-carboxylate (30 mg, 0.069 mmol) in tetrahydrofuran (1 mL) and stirred at room temperature for 2 h. The reaction was monitored by LC-MS for completion, and quenched by the addition of water. The reaction mixture was extracted with ethyl acetate, washed with saturated brine, dried over anhydrous sodium sulfate, and concentrated to give (S)-methyl 8-(1-(tert-butoxycarbonyl)pyrrolidin-2-yl)-6-(3-methyl-1*H*-pyrrolo[2,3-*b*]pyridin-5-yl)-3,4 -dihydroisoquinoline-2(1*H*)-carboxylate (30 mg, yield: 88%). ES-API:[M+H]⁺= 491.3.

Step 2: 4M Hydrochloric acid/methanol solution (1 mL) was added to the (S)-methyl 8-(1-(tert-butoxycarbonyl)pyrrolidin-2-yl)-6-(3-methyl-1*H*-pyrrolo[2,3-*b*]pyridin-5-yl)-3,4 -dihydroisoquinoline-2(1*H*)-carboxylate (30 mg, 0.058 mmol) and stirred at room temperature for 1 h. The reaction was monitored by LC-MS for completion. The reaction mixture was adjusted to pH 7 with saturated sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-methyl (6-(3-methyl-1*H*-pyrrolo[2,3-*b*]pyridin-5-yl)-8-(pyrrolidin-2-yl)-3,4-dihydroisoquinoline-2(1*H*)carboxylate (Z34-1, 4.9 mg, yield: 22%) as a white powder. ES-API:[M+H]⁺= 391.2.

### Example 66. Synthesis of compound Z54-1

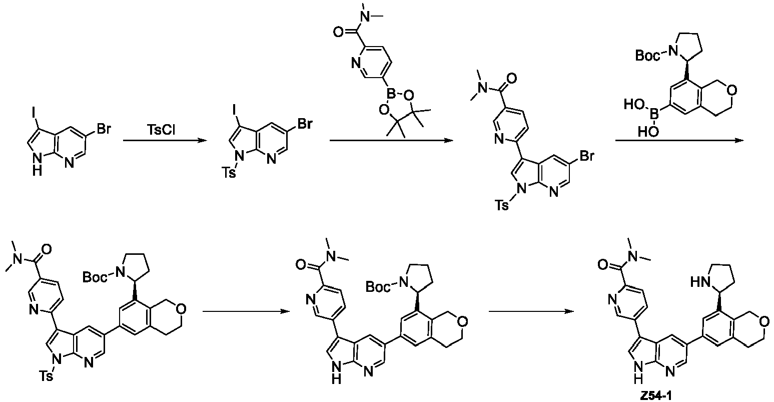

Step 1: 5-Bromo-3-iodo-1*H*-pyrrolo[2,3-b]pyridine (1 g, 3.11 mmol) was dissolved in tetrahydrofuran (10 mL). Sodium hydride (0.25 g, 6.22 mmol) was added and stirred at room temperature for 0.5 h. p-Toluenesulfonyl chloride (0.62 g, 3.26 mmol) was added in batches, and the system reacted at room temperature for 2 h. The reaction was quenched by the addition of water, and the reaction mixture was extracted with ethyl acetate. The organic phase was washed with brine, dried over anhydrous sodium sulfate, and spun to dryness to give 5-bromo-3-iodo-1-tosyl-1*H*-pyrrolo[2,3-*b*]pyridine (1.4 g, yield: 94.6%). ES-API:[M+H]⁺= 476.9.

Step 2: The 5-bromo-3-iodo-1-tosyl-1*H*-pyrrolo[2,3-*b*]pyridine (0.7 g, 1.47 mmol) was dissolved in dioxane/water (7.5/1.5 mL). *N*,*N*-dimethyl-5-(4,4,5,5-tetramethyl-1,3,2-dioxaborolan-2-yl)pyridine carboxamide (0.34 g, 1.77 mmol), potassium carbonate (0.41 g, 2.94 mmol) and 1,1'-bis(diphenylphosphino)ferrocene dichloropalladium (II) (0.107 g, 0.147 mmol) were added, and the system reacted at 80°C for 3 h in the presence of protective nitrogen. The reaction mixture was cooled, and water was added. The obtained mixture was extracted with ethyl acetate. The organic phase was washed with brine, dried over anhydrous sodium sulfate, concentrated, purified by column chromatography (ethyl acetate/petroleum ether = 1/5, Rf = 0.6) to give 6-(5-bromo-1-tosyl-1*H*-pyrrolo[2,3-b]pyridin-3-yl)-*N,N-*dimethylnicotinamide (0.53 g, yield: 72%). ES-API:[M+H]⁺= 499.1.

Step 3: The 6-(5-bromo-1-tosyl-1*H*-pyrrolo[2,3-b]pyridin-3-yl)-*N,N-*dimethylnicotinamide (0.53 g, 1.07 mmol) was dissolved in dioxane/water (7.5/1.5 mL). (S)-(8-(1-(tert-butoxycarbonyl)pyrrolidin-2-yl)isochroman-6-yl)boronic acid (0.34 g, 1.07 mmol), potassium carbonate (0.41 g, 2.94 mmol) and chloro (2-dicyclohexylphosphino-2',6'-dimethoxy-1,1'-biphenylyl)(2'-amino-1,1'-biphenyl-2-yl) palladium (II) (0.107 g, 0.147 mmol) were added, and the system reacted under microwave at 100°C for 3 h in the presence of protective nitrogen. The reaction mixture was cooled, and water was added. The obtained mixture was extracted with ethyl acetate. The organic phase was washed with brine, dried over anhydrous sodium sulfate, concentrated, and purified by column chromatography (ethyl acetate/petroleum ether = 1/1, Rf = 0.5) to give tert-butyl (S)-2-(6-(3-(5-(dimethylcarbamoyl)pyridin-2-yl)-1-tosyl-1*H*-pyrrolo[2,3-*b*]pyridin-5-yl)is ochroman-8-yl)pyrrolidine-1-carboxylate (366 mg, yield: 50%). ES-API:[M+H]⁺= 722.40.

Step 4: The tert-butyl (S)-2-(6-(3-(5-(dimethylcarbamoyl)pyridin-2-yl)-1-tosyl-1*H*-pyrrolo[2,3-*b*]pyridin-5-yl)is ochroman-8-yl)pyrrolidine-1-carboxylate (366 mg, 0.5 mmol) was dissolved in methanol/water (5/0.5 mL). Sodium hydroxide (0.242 g, 6.1 mmol) was added, and the system reacted at 65°C for 1 h. The reaction mixture was concentrated. Water was added. The obtained mixture was separated with dichloromethane. The organic phase was washed with brine, dried over anhydrous sodium sulfate, concentrated, and purified by prep-HPLC (column information: Ultimate XB-C18, 50*250 mm, 10 um, mobile phase system: A: purified water B: pure acetonitrile, flow rate: 80 ml/min, gradient: B/A = 20%-90% over 50 min, wavelength: 214 nm, column temperature: room temperature) to give tert-butyl (S)-2-(6-(3-(6-(dimethylcarbamoyl)pyridin-3-yl)-1*H*-pyrrolo[2,3-*b*]pyridin-5-yl)isochrom an-8-yl)pyrrolidine-1-carboxylate (183.6 mg, yield: 65%). ES-API: ES-API:[M+H]⁺= 568.40.

Step 5: The tert-butyl (S)-2-(6-(3-(6-(dimethylcarbamoyl)pyridin-3-yl)-1*H*-pyrrolo[2,3-*b*]pyridin-5-yl)isochrom an-8-yl)pyrrolidine-1-carboxylate (183.6 mg, 0.25 mmol) was dissolved in dichloromethane (5 mL). Trifluoroacetic acid (2 mL) was added, and the system reacted at room temperature for 2 h. The reaction mixture was concentrated, and water was added. The obtained mixture was extracted with dichloromethane. The organic phase was washed with brine, dried over anhydrous sodium sulfate, concentrated, purified by prep-HPLC(column information: Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-*N,N*-dimethyl-5-(5-(8-(pyrrolidin-2-yl)isochroman-6-yl)-1*H*-pyrrolo[2,3-b]pyridin-3-y 1)pyridine carboxamide (Z54-1, 16 mg, yield: 10.5%). ES-API:[M+H]⁺= 468.40. ¹H NMR (400 MHz, CDCl₃) δ 10.32 (s, 1H), 9.38 (s, 1H), 8.93 (s, 1H), 8.72 (s, 1H), 8.56 (s, 1H), 8.06 (dd, *J* = 8.0, 1.6 Hz, 1H), 7.84 (s, 1H), 7.78 (s, 1H), 7.67 (d, *J* = 8.0 Hz, 1H), 7.53 - 7.28 (m, 1H), 7.16 (s, 1H), 4.92 (d, *J* = 15.2 Hz, 1H), 4.70 (d, *J* = 15.2 Hz, 1H), 4.50 (s, 1H), 3.83 (t, *J* = 5.6 Hz, 2H), 3.71 (d, J = 10.7 Hz, 1H), 3.16 (s, 3H), 3.12 (s, 3H),, 2.89 - 2.76 (m, 1H), 2.73 - 2.61 (m, 1H), 2.45 - 2.28 (m, 2H), 2.25 - 2.06 (m, 2H). ES-API:[M+H]⁺= 468.40.

### Example 67. Synthesis of compound Z217

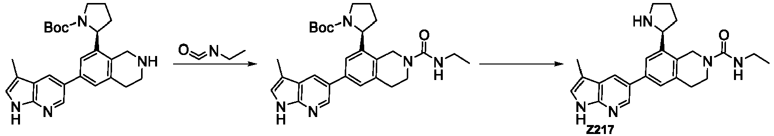

Step 1: Ethyl isocyanate (5 mg, 0.069 mmol) and triethylamine (21 mg, 0.208 mmol) were added to a solution of tert-butyl (S)-2-(6-(3-methyl-1*H*-pyrrolo[2,3-*b*]pyridin-5-yl)-1,2,3,4-tetrahydroisoquinolin-8-yl)pyrr olidine-1-carboxylate (30 mg, 0.069 mmol) in tetrahydrofuran (1 mL) and stirred at room temperature for 2 h. The reaction was monitored by LC-MS for completion, and quenched by the addition of water. The reaction mixture was extracted with ethyl acetate, washed with saturated brine, dried over anhydrous sodium sulfate, and concentrated to give tert-butyl (S)-2-(2-(ethylcarbamoyl)-6-(3-methyl-1*H*-pyrrolo[2,3-*b*]pyridin-5-yl)-1,2,3,4-tetrahydroi soquinolin-8-yl)pyrrolidine-1-carboxylate (30 mg, yield: 86%). ES-API:[M+H]⁺= 504.3.

Step 2: 4M Hydrochloric acid/methanol solution (1 mL) was added to the tert-butyl (S)-2-(2-(ethylcarbamoyl)-6-(3-methyl-1*H*-pyrrolo[2,3-*b*]pyridin-5-yl)-1,2,3,4-tetrahydroi soquinolin-8-yl)pyrrolidine-1-carboxylate (30 mg, 0.059 mmol) and stirred at room temperature for 1 h. The reaction was monitored by LC-MS for completion. The reaction mixture was adjusted to pH 7 with sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-*N*-ethyl-6-(3-methyl-1*H*-pyrrolo[2,3-*b*]pyridin-5-yl)-8-(pyrrolidin-2-yl)-3,4-dihydrois oquinoline-2(1*H*)-carboxamide (Z217, 4.9 mg, yield: 20%) as a white powder. ES-API:[M+H]⁺= 404.2.

### Example 68. Synthesis of compound Z37-1

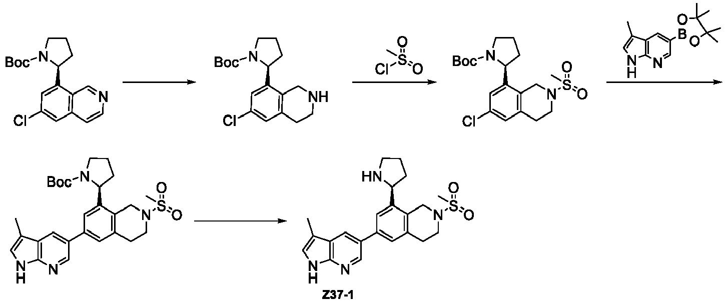

Step 1: Sodium borohydride (360 mg, 9.6 mmol) was added to a solution of tert-butyl (S)-2-(6-chloroisoquinolin-8-yl)pyrrolidine-1-carboxylate (800 mg, 2.4 mmol) in acetic acid (30 mL) under an ice bath condition and stirred for 2 h. The reaction was monitored by LC-MS for completion, and quenched by the addition of sodium bicarbonate. The reaction mixture was extracted with dichloromethane, dried over anhydrous sodium sulfate, concentrated, and purified by column chromatography (0-10% methanol/dichloromethane, dichloromethane/methanol = 10/1, Rf = 0.4) to give tert-butyl (S)-2-(6-chloro-1,2,3,4-tetrahydroisoquinolin-8-yl)pyrrolidine-1-carboxylate (900 mg, crude product). ES-API:[M+H]⁺= 337.2.

Step 2: Triethylamine (90 mg, 0.89 mmol) and methanesulfonyl chloride (40 mg, 0.356 mmol) were added to a solution of the tert-butyl (S)-2-(6-chloro-1,2,3,4-tetrahydroisoquinolin-8-yl)pyrrolidine-1-carboxylate (100 mg, 0.297 mmol) in dichloromethane (1 mL) under an ice bath condition and stirred for 15 min. The reaction was monitored by LC-MS for completion, and quenched by the addition of water. The reaction mixture was extracted with dichloromethane, dried over anhydrous sodium sulfate, and concentrated to give tert-butyl (S)-2-(6-chloro-2-(methylsulfonyl)-1,2,3,4-tetrahydroisoquinolin-8-yl)pyrrolidine-1-carbo xylate (100 mg, yield: 81%). ES-API:[M+Na]⁺= 437.2.

Step 3: In the presence of protective nitrogen, 3-methyl-5-(4,4,5,5-tetramethyl-1,3,2-dioxaborolan-2-yl)-1*H*-pyrrolo[2,3-*b*]pyridine (74.65 mg, 0.289 mmol), chloro (2-dicyclohexylphosphino-2',6'-dimethoxy-1,1'-biphenylyl)(2'-amino-1,1'-biphenyl-2-yl) palladium (17.34 mg, 0.024 mmmol) and potassium carbonate (99.77 mg, 0.723 mmmol) were added to the tert-butyl (S)-2-(6-chloro-2-(methylsulfonyl)-1,2,3,4-tetrahydroisoquinolin-8-yl)pyrrolidine-1-carbo xylate (100 mg, 0.24 mmol), and the mixture was stirred at 110°C for 3 h. The reaction was monitored by LC-MS for completion. The reaction mixture was treated with ethyl acetate/water, dried over anhydrous sodium sulfate, concentrated, and purified by column chromatography (0-6% methanol/dichloromethane, dichloromethane/methanol = 10/1, Rf = 0.6) to give tert-butyl (S)-2-(6-(3-methyl-1*H*-pyrrolo[2,3-*b*]pyridin-5-yl)-2-(methylsulfonyl)-1,2,3,4-tetrahydrois oquinolin-8-yl)pyrrolidine-1-carboxylate (80 mg, yield: 65%) as a yellow powder. ES-API:[M+H-100]⁺= 411.3.

Step 4: 4M Hydrochloric acid/methanol solution (1 mL) was added to the tert-butyl (S)-2-(6-(3-methyl-1*H*-pyrrolo[2,3-*b*]pyridin-5-yl)-2-(methylsulfonyl)-1,2,3,4-tetrahydrois oquinolin-8-yl)pyrrolidine-1-carboxylate (80 mg, 0.157 mmol) and stirred at room temperature for 1 h. The reaction was monitored by LC-MS for completion. The reaction mixture was adjusted to pH 7 with saturated sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-6-(3-methyl-1*H*-pyrrolo[2,3-*b*]pyridin-5-yl)-2-(methylsulfonyl)-8-(pyrrolidin-2-yl)-1,2 ,3,4-tetrahydroisoquinoline (Z37-1, 23.6 mg, yield: 37%) as a white powder. ES-API:[M+H]⁺= 411.2. ¹H NMR (400 MHz, CDCl₃) δ 8.46 (s, 1H), 7.99 (s, 1H), 7.71 (s, 1H), 7.27 - 7.25 (m, 1H), 7.09 (s, 1H),4.64 (d, *J* = 16.4 Hz, 1H), 4.51 (d, *J* = 15.6 Hz, 1H), 4.28 (t, *J* = 8.0 Hz, 1H), 3.64 - 3.55 (m, 1H), 3.54 - 3.44 (m, 1H),3.33 - 3.23 (m, 1H), 3.14 - 3.01 (m, 3H), 2.89 (s, 3H), 2.34 (s, 3H), 2.33 - 2.16 (m, 1H), 2.05 - 1.94 (m, 1H), 1.94 - 1.67 (m, 2H).

### Example 69. Synthesis of compound Z218

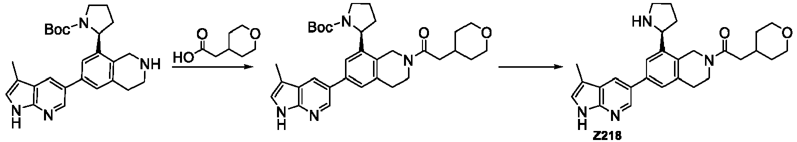

Step 1: 2-(Tetrahydro-2*H*-pyran-4-yl)acetic acid (8 mg, 0.055 mmol), triethylamine (21 mg, 0.208 mmol) and 1-propylphosphonic anhydride (44 mg, 0.14 mmol) were added to a solution of tert-butyl (S)-2-(6-(3-methyl-1*H*-pyrrolo[2,3-*b*]pyridin-5-yl)-1,2,3,4-tetrahydroisoquinolin-8-yl)pyrr olidine-1-carboxylate (20 mg, 0.046 mmol) in dichloromethane (0.6 mL) and stirred at room temperature for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol = 10/1, Rf = 0.6) to give tert-butyl (S)-2-(6-(3-methyl-1*H*-pyrrolo[2,3-*b*]pyridin-5-yl)-2-(2-(tetrahydro-2*H*-pyran-4-yl)acetyl) -1,2,3,4-tetrahydroisoquinolin-8-yl)pyrrolidine-1-carboxylate (20 mg, yield: 77%). ES-API:[M+H]⁺= 559.3.

Step 2: 4M Hydrochloric acid/methanol solution (1 mL) was added to the tert-butyl (S)-2-(6-(3-methyl-1*H*-pyrrolo[2,3-*b*]pyridin-5-yl)-2-(2-(tetrahydro-2*H*-pyran-4-yl)acetyl) -1,2,3,4-tetrahydroisoquinolin-8-yl)pyrrolidine-1-carboxylate (30 mg, 0.055 mmol) and stirred at room temperature for 1 h. The reaction was monitored by LC-MS for completion. The reaction mixture was adjusted to pH 7 with saturated aqueous sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-1-(6-(3-methyl-1*H*-pyrrolo[2,3-*b*]pyridin-5-yl)-8-(pyrrolidin-2-yl)-3,4-dihydroisoquin olin-2(1*H*)-yl)-2-(tetrahydro-2H-pyran-4-yl)ethanone (Z218, 3.5 mg, yield: 14 %) as a white powder. ES-API:[M+H]⁺=459.2.

### Example 70. Synthesis of compound Z219

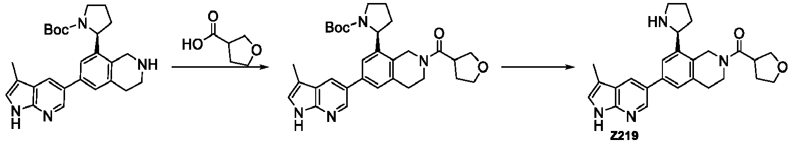

Step 1: Tetrahydrofuran-3-carboxylic acid (8 mg, 0.069 mmol), triethylamine (21 mg, 0.208 mmol) and 1-propylphosphonic anhydride (66 mg, 0.21 mmol) were added to a solution of tert-butyl (S)-2-(6-(3-methyl-1*H*-pyrrolo[2,3-*b*]pyridin-5-yl)-1,2,3,4-tetrahydroisoquinolin-8-yl)pyrr olidine-1-carboxylate (30 mg, 0.069 mmol) in dichloromethane (0.6 mL) and stirred at room temperature for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol = 10/1, Rf = 0.6) to give (2S)-tert-butyl 2-(6-(3-methyl-1*H*-pyrrolo[2,3-*b*]pyridin-5-yl)-2-(tetrahydrofuran-3-carbonyl)-1,2,3,4-tetr ahydroisoquinolin-8-yl)pyrrolidine-1-carboxylate (30 mg, yield: 81%). ES-API:[M+H]⁺= 531.3.

Step 2: 4M hydrochloric acid/methanol solution (1 mL) was added to the (2S)-tert-butyl 2-(6-(3-methyl-1*H*-pyrrolo[2,3-*b*]pyridin-5-yl)-2-(tetrahydrofuran-3-carbonyl)-1,2,3,4-tetr ahydroisoquinolin-8-yl)pyrrolidine-1-carboxylate (30 mg, 0.056 mmol) and stirred at room temperature for 1 h. The reaction was monitored by LC-MS for completion. The reaction mixture was adjusted to pH 7 with saturated sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (6-(3-methyl-1*H*-pyrrolo[2,3-*b*]pyridin-5-yl)-8-(S)-pyrrolidin-2-yl)-3,4-dihydroisoquinoli n-2(1H)-yl)(tetrahydrofuran-3-yl)methanone (Z219, 9.1 mg, yield: 37 %) as a white powder. ES-API:[M+H]⁺=431.2. ¹H NMR (400 MHz, CDCl₃) δ 8.60 - 8.50 (m, 1H), 7.99 - 7.88 (m, 1H), 7.87 - 7.74 (m, 1H), 7.12 - 7.03 (m, 1H),7.02 - 6.86 (m, 1H), 4.92 - 4.80 (m, 1H), 4.76 - 4.60 (m, 3H), 4.15 - 3.93 (m, 1H), 3.92 - 3.79 (m, 3H),3.77 - 3.53 (m, 3H), 3.39 - 3.29 (m, 1H), 2.86 - 2.67 (m, 2H), 2.42 - 2.30 (m, 1H), 2.29 - 2.24 (m, 3H),2.23 - 2.17 (m, 2H), 2.15 - 2.10 (m, 1H), 2.07 - 1.85 (m, 2H).

### Example 71. Synthesis of compound Z220

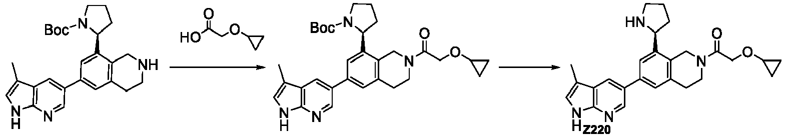

Step 1: 2-Cyclopropoxyacetic acid (8 mg, 0.069 mmol), triethylamine (21 mg, 0.208 mmol) and 1-propylphosphonic anhydride (66 mg, 0.21 mmol) were added to a solution of tert-butyl (S)-2-(6-(3-methyl-1*H*-pyrrolo[2,3-*b*]pyridin-5-yl)-1,2,3,4-tetrahydroisoquinolin-8-yl)pyrr olidine-1-carboxylate (30 mg, 0.069 mmol) in dichloromethane (0.6 mL) and stirred at room temperature for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol = 10/1, Rf = 0.6) to give tert-butyl (S)-2-(2-(2-cyclopropoxyacetyl)-6-(3-methyl-1*H*-pyrrolo[2,3-*b*]pyridin-5-yl)-1,2,3,4-tetra hydroisoquinolin-8-yl)pyrrolidine-1-carboxylate (15 mg, yield: 41%). ES-API:[M+H]⁺= 539.3.

Step 2: 4M Hydrochloric acid/methanol solution (1 mL) was added to the tert-butyl (S)-2-(2-(2-cyclopropoxyacetyl)-6-(3-methyl-1*H*-pyrrolo[2,3-*b*]pyridin-5-yl)-1,2,3,4-tetra hydroisoquinolin-8-yl)pyrrolidine-1-carboxylate (15 mg, 0.028 mmol) and stirred at room temperature for 1 h. The reaction was monitored by LC-MS for completion. The reaction mixture was adjusted to pH 7 with saturated sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-2-cyclopropyl-1-(6-(3-methyl-1*H*-pyrrolo[2,3-*b*]pyridin-5-yl)-8-(pyrrolidin-2-yl)-3,4-dihydroisoquinolin-2(1*H*)-yl)ethanone (Z220, 2.8 mg, yield: 23%) as a white powder. ES-API:[M+H]⁺=439.2.

### Example 72. Synthesis of compound Z221

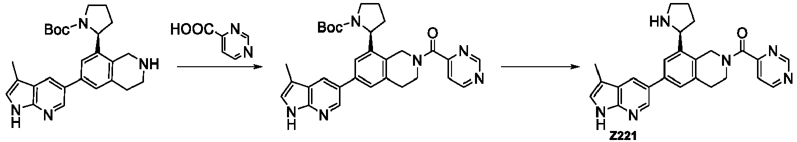

Step 1: Pyrimidine-4-carboxylic acid (10.33 mg, 0.083 mmol), triethylamine (21 mg, 0.208 mmol) and 1-propylphosphonic anhydride (66 mg, 0.21 mmol) were added to a solution of tert-butyl (S)-2-(6-(3-methyl-1*H*-pyrrolo[2,3-*b*]pyridin-5-yl)-1,2,3,4-tetrahydroisoquinolin-8-yl)pyrr olidine-1-carboxylate (30 mg, 0.069 mmol) in dichloromethane (0.6 mL) and stirred at room temperature for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol = 10/1, Rf = 0.6) to give tert-butyl (S)-2-(6-(3-methyl-1*H*-pyrrolo[2,3-*b*]pyridin-5-yl)-2-(pyrimidine-4-carbonyl)-1,2,3,4-tetra hydroisoquinolin-8-yl)pyrrolidine-1-carboxylate (30 mg, yield: 80%). ES-API:[M+H]⁺= 539.4.

Step 2: 4 M Hydrochloric acid/methanol solution (1 mL) was added to the tert-butyl (S)-2-(6-(3-methyl-1*H*-pyrrolo[2,3-*b*]pyridin-5-yl)-2-(pyrimidine-4-carbonyl)-1,2,3,4-tetra hydroisoquinolin-8-yl)pyrrolidine-1-carboxylate (30 mg, 0.056 mmol) and stirred at room temperature for 1 h. The reaction was monitored by LC-MS for completion. The reaction mixture was adjusted to pH 7 with saturated sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-(6-(3-methyl-1*H*-pyrrolo[2,3-*b*]pyridin-5-yl)-8-(pyrrolidin-2-yl)-3,4-dihydroisoquinoli n-2(1H)-yl)(pyrimidin-4-yl)methanone (Z221, 5.2 mg, yield: 21%) as a white powder. ES-API:[M+H]⁺=439.3.

### Example 73. Synthesis of compound Z222

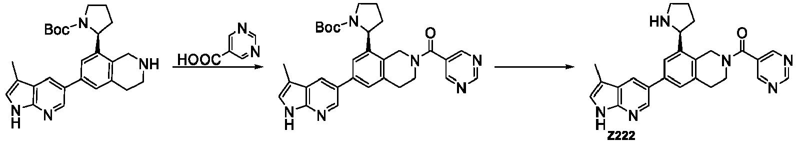

Step 1: Pyrimidine-5-carboxylic acid (10.33 mg, 0.083 mmol), triethylamine (21 mg, 0.208 mmol) and 1-propylphosphonic anhydride (66 mg, 0.21 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in dichloromethane (0.6 mL) and stirred at room temperature for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol = 10/1, Rf = 0.6) to give tert-butyl (S)-2-(6-(3-methyl-1H-pyrrolo[2,3-b]pyridin-5-yl)-2-(pyrimidine-5-carbonyl)-1,2,3,4-tetra hydroisoquinolin-8-yl)pyrrolidine-1-carboxylate (30 mg, yield: 80%). ES-API:[M+H]⁺= 539.4.

Step 2: 4 M Hydrochloric acid/methanol solution was added to the tert-butyl (S)-2-(6-(3-methyl-1H-pyrrolo[2,3-b]pyridin-5-yl)-2-(pyrimidine-5-carbonyl)-1,2,3,4-tetra hydroisoquinolin-8-yl)pyrrolidine-1-carboxylate (30 mg, 0.056 mmol) and stirred at room temperature for 1 h. The reaction was monitored by LC-MS for completion. The reaction mixture was adjusted to pH 7 with saturated sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-(6-(3-methyl-1H-pyrrolo[2,3-b]pyridin-5-yl)-8-(pyrrolidin-2-yl)-3,4-dihydroisoquinoli n-2(1H)-yl)(pyrimidin-5-yl)methanone (Z222, 4.7 mg, yield: 19%) as a white powder. ES-API:[M+H]⁺=439.3. ¹H NMR (400 MHz, CDCl₃) δ 9.28 (s, 1H), 8.94 - 8.84 (m, 1H), 8.80 (s, 1H), 8.54 (s, 1H),8.00 - 7.80 (m, 2H), 7.10 - 6.95 (m, 2H), 4.97 (d, *J* = 8.4 Hz, 2H), 4.80 -4.68 (m, 1H),3.80 - 3.66 (m, 1H), 3.63 - 3.34 (m, 3H), 2.91 - 2.75 (m, 1H), 2.70 - 2.55 (m, 1H),2.29 (s, 3H), 2.25 - 2.15 (m, 1H), 2.15 - 1.80 (m, 3H).

### Example 74. Synthesis of compound Z223

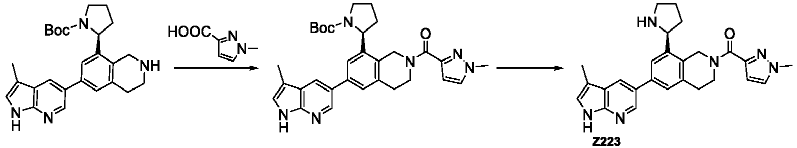

Step 1: 1-Methyl-1*H*-pyrazole-3-carboxylic acid (10.5 mg, 0.083 mmol), triethylamine (21 mg, 0.208 mmol) and 1-propylphosphonic anhydride (66 mg, 0.21 mmol) were added to a solution of tert-butyl (S)-2-(6-(3-methyl-1*H*-pyrrolo[2,3-*b*]pyridin-5-yl)-1,2,3,4-tetrahydroisoquinolin-8-yl)pyrr olidine-1-carboxylate (30 mg, 0.069 mmol) in dichloromethane (0.6 mL) and stirred at room temperature for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol = 10/1, Rf = 0.6) to give tert-butyl (S)-2-(2-(1-methyl-1*H*-pyrazole-3-carbonyl)-6-(3-methyl-1*H*-pyrrolo[2,3-b]pyridin-5-yl)-1,2,3,4-tetrahydroisoquinolin-8-yl)pyrrolidine-1-carboxylate (30 mg, yield: 80%). ES-API:[M+H]⁺= 541.3
Step 2: 4M Hydrochloric acid/methanol solution (1 mL) was added to the tert-butyl (S)-2-(2-(1-methyl-1*H*-pyrazole-3-carbonyl)-6-(3-methyl-1*H*-pyrrolo[2,3-b]pyridin-5-yl)-1,2,3,4-tetrahydroisoquinolin-8-yl)pyrrolidine-1-carboxylate (30 mg, 0.055 mmol) and stirred at room temperature for 1 h. The reaction was monitored by LC-MS for completion. The reaction mixture was adjusted to pH 7 with saturated sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-(1-methyl-1*H*-pyrazol-3-yl)(6-(3-methyl-1*H*-pyrrolo[2,3-*b*]pyridin-5-yl)-8-(pyrrolidin-2-yl)-3,4-dihydroisoquinolin-2(1*H*)-yl)methanone (Z223, 5.7 mg, yield: 23%) as a white powder. ES-API:[M+H]⁺=441.2. ¹H NMR (400 MHz, CDCl₃) δ 8.54 (s, 1H), 7.98 - 7.68 (m, 2H), 7.41 - 7.35 (m, 1H), 7.03 - 6.82 (m, 2H), 6.79 - 6.65 (m, 1H), 5.27 - 4.96 (m, 1H), 4.92 - 4.67 (m, 2H), 4.12 - 3.74 (m, 5H), 3.69 - 3.36 (m, 2H), 2.95 - 2.59 (m, 2H), 2.26 (s, 3H), 2.22 - 1.80 (m, 4H).

### Example 75. Synthesis of compound Z224

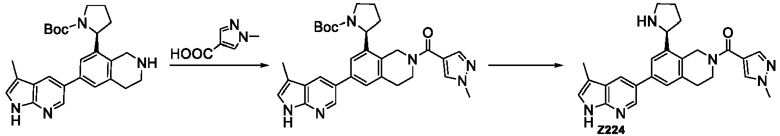

Step 1: 1-Methyl-1H-pyrazole-4-carboxylic acid (10.5 mg, 0.083 mmol), triethylamine (21 mg, 0.208 mmol) and 1-propylphosphonic anhydride (66 mg, 0.21 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in dichloromethane (0.6 mL) and stirred at room temperature for 2 h. The reaction was monitored by LC-MS for completion, and quenched by the addition of water. The reaction mixture was extracted with ethyl acetate, washed with saturated brine, dried over anhydrous sodium sulfate, concentrated, and purified by column chromatography (0-6% methanol/dichloromethane, dichloromethane/methanol = 10/1, Rf = 0.6) to give tert-butyl (S)-2-(2-(1-methyl-1H-pyrazole-4-carbonyl)-6-(3-methyl-1H-pyrrolo[2,3-b]pyridin-5-yl)-1,2,3,4-tetrahydroisoquinolin-8-yl)pyrrolidine-1-carboxylate (30 mg, yield: 80%). ES-API:[M+H]⁺= 541.3.

Step 2: 4M Hydrochloric acid/methanol solution (1 mL) was added to the tert-butyl (S)-2-(2-(1-methyl-1H-pyrazole-4-carbonyl)-6-(3-methyl-1H-pyrrolo[2,3-b]pyridin-5-yl)-1,2,3,4-tetrahydroisoquinolin-8-yl)pyrrolidine-1-carboxylate (30 mg, 0.055 mmol) and stirred at room temperature for 1 h. The reaction was monitored by LC-MS for completion. The reaction mixture was adjusted to pH 7 with saturated sodium bicarbonate solution, treated with dichloromethane/water, dried over anhydrous sodium sulfate, concentrated, and purified by prep-HPLC (Ultimate XB-C18, 50*250 mm, 10 um, mobile phase system: A: purified water (0.05% ammonia water), B: pure acetonitrile, flow rate: 80 ml/min, gradient: B/A = 20%-90% over 50 min, wavelength: 214 nm, column temperature: room temperature) to give (S)-(1 -methyl-1H-pyrazol-4-yl)(6-(3-methyl- 1H-pyrrolo[2,3-b]pyridin-5-yl)-8-(pyrrolidin-2-yl)-3,4-dihydroisoquinolin-2(1H)-yl)methanone (Z224, 3.1 mg, yield: 13%) as a white powder. ES-API:[M+H]⁺=441.2.

### Example 76. Synthesis of compound Z225

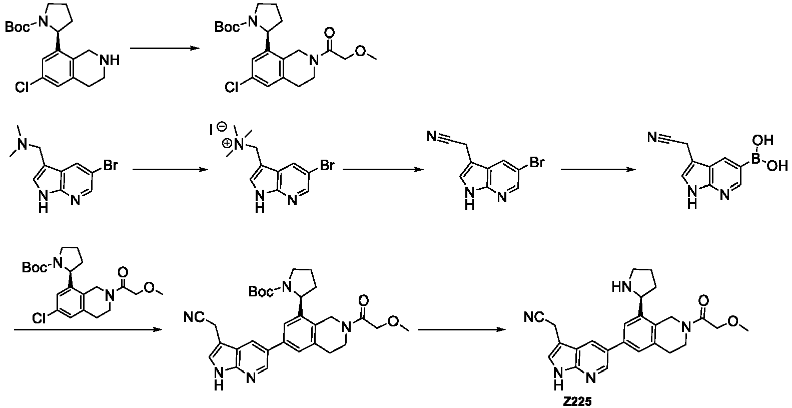

Step 1: 1-(5-Bromo-1H-pyrrolo[2,3-*b*]pyridin-3-yl)-*N,N-*dimethyl-methanamine (0.2 g, 0.79 mmol) was dissolved in tetrahydrofuran (1 mL), and the system was cooled in an ice bath. Iodomethane (279 mg, 1.97 mmol) was added dropwise, and the system reacted at room temperature overnight. The reaction mixture was spun to dryness to give (5-bromo-3a,7a-dihydro-1*H*-pyrrolo[2,3-*b*]pyridin-3-yl)methyl-trimethylammonium iodide (300 mg, crude product). ES-API:[M+1]⁺=268.0.

Step 2: The (5-bromo-3a,7a-dihydro-1*H*-pyrrolo[2,3-*b*]pyridin-3-yl)methyl-trimethylammonium iodide (300 mg, 0.75 mmol) was dissolved in tetrahydrofuran (5 mL). Tetrabutylammonium fluoride (394 mg, 1.51 mmol) and trimethylsilyl cyanide (187 mg, 1.88 mmol) were added, and the system reacted at room temperature for 4 h. The reaction was quenched with water, and the reaction mixture was extracted with ethyl acetate. The organic phase was dried over anhydrous sodium sulfate, spun to dryness, and purified by column chromatography (dichloromethane/methanol = 10/1, Rf = 0.5) to give 2-(5-bromo-1H-pyrrolo[2,3-b]pyridin-3-yl)acetonitrile (100 mg, yield: 56%). ES-API:[M+1]⁺=236.0.

Step 3: The 2-(5-bromo-1H-pyrrolo[2,3-b]pyridin-3-yl)acetonitrile (80 mg, 0.34 mmol) was dissolved in dioxane (2 mL). Bis(pinacolato)diboron (129 mg, 0.51 mmol), [1,1'-bis(diphenylphosphino)ferrocene]dichloropalladium (25 mg, 0.034 mmol) and potassium acetate (99 mg, 1.02 mmol) were then added, and the system was heated to 100°C overnight. The reaction mixture was extracted with ethyl acetate, dried over anhydrous sodium sulfate, and spun to dryness to give [3-(cyanomethyl)-1H-pyrrolo[2,3-b]pyridin-5-yl]boronic acid as a crude product which was directly used in the next step without purification. ES-API:[M+1]⁺=202.1.

Step 4: Tert-butyl 2-(6-chloro-1,2,3,4-tetrahydroisoquinolin-8-yl)pyrrolidine-1-carboxylate (95 mg, 0.79 mmol) was dissolved in dichloromethane (3 mL). Triethylamine (86 mg, 1.97 mmol) and 2-methoxyacetyl chloride (31 mg, 0.285 mmol) were added, and the system reacted at room temperature for 2 h. The reaction was quenched with water. The reaction mixture was extracted with dichloromethane, dried over anhydrous sodium sulfate, and spun to dryness to give tert-butyl 2-[6-chloro-2-(2-methoxyacetyl)-3,4-dihydro-1H-isoquinolin-8-yl]pyrrolidine-1-carboxyla te (100 mg, crude product). ES-API:[M+1-100]⁺=309.2.

Step 5: The [3-(cyanomethyl)-1H-pyrrolo[2,3-b]pyridin-5-yl]boronic acid (crude product, 0.32 mmol) was dissolved in dioxane/water (1.5 mL/0.3 mL)). The tert-butyl 2-[6-chloro-2-(2-methoxyacetyl)-3,4-dihydro-1H-isoquinolin-8-yl]pyrrolidine-1-carboxyla te (105 mg, 0.26 mmol), chloro (2-dicyclohexylphosphino-2',6'-dimethoxy-1,1'-biphenylyl)(2'-amino-1,1'-biphenyl-2-yl) palladium (II) (23 mg, 0.032 mmol) and potassium carbonate (132 mg, 0.96 mmol) were then added. The system was heated to 100°C for 2 h. Upon completion of the reaction, the reaction mixture was spun to dryness and purified by a preparative thin layer chromatographic column (dichloromethane/methanol = 10/1, Rf = 0.7) to give tert-butyl 2-[6-[3-(cyanomethyl)-1*H*-pyrrolo[2,3-*b*]pyridin-5-yl]-2-(2-methoxyacetyl)-3,4-dihydro-1 *H*-isoquinolin-8-yl]pyrrolidine-1-carboxylate (33 mg, yield: 19%). ES-API:[M+1-100]⁺=430.3.

Step 6: The tert-butyl 2-[6-[3-(cyanomethyl)-1*H*-pyrrolo[2,3-*b*]pyridin-5-yl]-2-(2-methoxyacetyl)-3,4-dihydro-1 *H*-isoquinolin-8-yl]pyrrolidine-1-carboxylate (50 mg, 0.094 mmol) was dissolved in dichloromethane (1 mL). Trifluoroacetic acid (1 mL) was added. The system reacted at room temperature for 1 h. Upon completion of the reaction, the reaction mixture was spun to dryness, extracted with dichloromethane and saturated sodium bicarbonate, dried over anhydrous sodium sulfate, spun to dryness, and purified by a preparative thin layer chromatographic column (dichloromethane/methanol = 10/1, Rf = 0.4) to give (S)-2-(5-(2-(2-methoxyacetyl)-8-(pyrrolidin-2-yl)-1,2,3,4-tetrahydroisoquinolin-6-yl)-1H-pyrrolo[2,3-b]pyridin-3-yl)acetonitrile (Z225, 5.1 mg, yield: 12.6%). ES-API:[M+1]⁺=430.2.

### Example 77. Synthesis of compound Z286

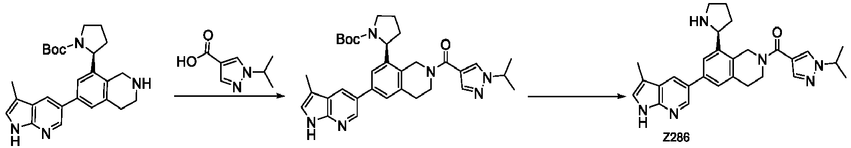

Step 1: 1-Isopropyl-1H-pyrazole-4-carboxylic acid (12.83 mg, 0.083 mmol), 1-propylphosphonic anhydride (66.16 mg, 0.21 mmol) and triethylamine (21.01 mg, 0.21 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in dichloromethane (1 mL). The obtained mixture was stirred at 25°C for 2 h. The reaction was monitored by LCMS for completion, and quenched by the addition of water. The reaction mixture was extracted with ethyl acetate, washed with saturated brine, dried over anhydrous sodium sulfate, and concentrated to give tert-butyl (S)-2-(2-(1-isopropyl-1H-pyrazole-4-carbonyl)-6-(3-methyl-1H-pyrrolo[2,3-b]pyridin-5-yl )-1,2,3,4-tetrahydroisoquinolin-8-yl)pyrrolidine-1-carboxylate (40 mg, crude product). ES-API:[M+H]⁺= 569.4.

Step 2: Trifluoroacetic acid (0.5 mL) was added to a solution of tert-butyl (S)-2-(2-(1-isopropyl-1H-pyrazole-4-carbonyl)-6-(3-methyl-1H-pyrrolo[2,3-b]pyridin-5-yl )-1,2,3,4-tetrahydroisoquinolin-8-yl)pyrrolidine-1-carboxylate (40 mg, 0.07 mmol, ) in dichloromethane (1 mL) and stirred at room temperature for 1 h. The reaction was monitored by LCMS for completion. The reaction mixture was adjusted to pH 7 with saturated sodium bicarbonate, treated with dichloromethane/water, dried over anhydrous sodium sulfate, and concentrated. The crude product was prepared and purified (column: Ultimate XB-C18, 50*250 mm, 10 um; mobile phase: A: purified water (0.05% ammonia water) B: pure acetonitrile; flow rate: 80 ml/min; gradient: B/A = 20%-90% over 50 min; wavelength: 214 nm; column temperature: room temperature) to give (S)-(1-isopropyl-1H-pyrazol-4-yl)(6-(3-methyl-1H-pyrrolo[2,3-b]pyridin-5-yl)-8-(pyrrolid in-2-yl)-3,4-dihydroisoquinolin-2(1H)-yl)methanone (Z286, 1.8 mg, yield: 5.46%) as a white powder. ES-API:[M+H]⁺=469.3. ¹H NMR (400 MHz, CDCl₃) δ 9.69 (s, 1H), 8.45 (s, 1H), 7.99 (s, 1H), 7.89 (s, 1H), 7.74 (s, 2H), 7.28 (s, 1H), 7.09 (s, 1H), 5.08-4.76 (m, 2H), 4.60-4.25 (m, 2H), 4.08-3.69 (m, 2H), 3.37-3.28 (m, 1H), 3.17-3.08 (m, 1H), 3.07-2.88 (m, 3H), 2.33 (s, 3H), 2.05-1.88 (m, 2H), 1.81-1.67 (m, 1H), 1.52 (d, *J* = 3.3 Hz, 3H).

### Example 78. Synthesis of compound Z287 and compound Z288

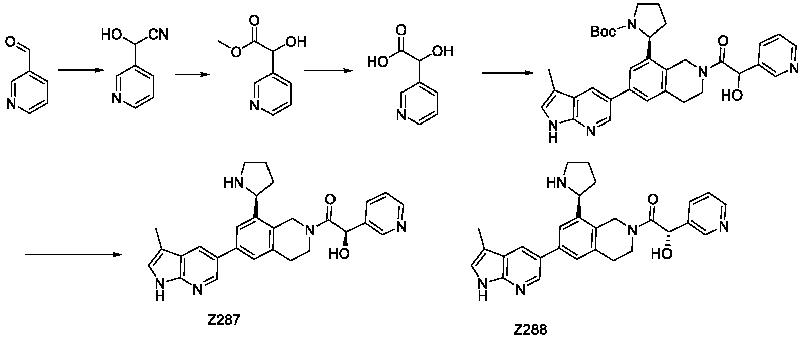

Step 1: Nicotinaldehyde (1 g, 9.34 mmol) and acetic acid (10 mL) were dissolved in trimethylsilyl cyanide (326 mg, 9.34 mmol) under an ice bath condition, and the system was warmed to room temperature for 16 h. The reaction was quenched by the addition of water, and the reaction mixture was extracted with ethyl acetate. The organic phase was washed with brine, dried over anhydrous sodium sulfate, and concentrated to give 2-hydroxy-2-(pyridin-3-yl)acetonitrile (1 g, yield: 80%) as a crude product. ES-API: [M+H]⁺ = 135.1.

Step 2: The 2-hydroxy-2-(pyridin-3-yl)acetonitrile (2 g, 14.91 mmol) was dissolved in hydrochloric acid-methanol solution (20 mL), and the system reacted at room temperature for 16 h. Upon completion of the reaction, the reaction mixture was quenched by the addition of aqueous sodium bicarbonate solution, adjusted to pH 7, and extracted with ethyl acetate. The organic phase was washed with brine, dried over anhydrous sodium sulfate, and concentrated to give a crude product. The crude product was purified by a thin layer chromatographic column (dichloromethane/methanol = 10/1) to give a product methyl 2-hydroxy-2-(pyridin-3-yl)acetate (219 mg, yield: 8.79%). ES-API: [M+H]⁺ = 168.1.

Step 3: The methyl 2-hydroxy-2-(pyridin-3-yl)acetate (219 mg, 1.31 mmol) and lithium hydroxide (31.37 mg, 1.31 mmol) were dissolved in water (0.5 mL) and tetrahydrofuran (0.5 mL) and stirred at room temperature for 1 h. The obtained mixture was then spun to dryness to give 2-hydroxy-2-(3-pyridyl)acetic acid (200 mg, yield: 100%) as a crude product. ES-API: [M+H]⁺ = 154.1.

Step 4: The 2-hydroxy-2-(3-pyridyl)acetic acid (12.74 mg, 0.083 mmol), tert-butyl (S)-2-[6-(3-methyl-1H-pyrrolo[2,3-b]pyridin-5-yl)-1,2,3,4-tetrahydroisoquinolin-8-yl]pyrr olidine-1-carboxylate (30 mg, 0.069 mmol), 1-(3-dimethylaminopropyl)-3-ethylcarbodiimide hydrochloride (39.89 mg, 0.208 mmol) and 1-hydroxybenzotriazole (28.11 mg, 0.208 mmol) were dissolved in N,N-dimethylformamide (0.5 mL), and the system reacted at room temperature for 1 h. Upon completion of the reaction, the reaction mixture was washed with water, extracted with ethyl acetate, dried over anhydrous sodium sulfate, and concentrated to give tert-butyl (2S)-2-(2-(2-hydroxy-2-(pyridin-3-yl)acetyl)-6-(3-methyl-1H-pyrrolo[2,3-b]pyridin-5-yl)-1,2,3,4-tetrahydroisoquinolin-8-yl)pyrrolidine-1-carboxylate (39 mg, yield: 100%) as a crude product. ES-API: [M+H]⁺ = 568.8.

Step 5: The tert-butyl (2S)-2-(2-(2-hydroxy-2-(pyridin-3-yl)acetyl)-6-(3-methyl-1H-pyrrolo[2,3-b]pyridin-5-yl)-1,2,3,4-tetrahydroisoquinolin-8-yl)pyrrolidine-1-carboxylate (39 mg, 0.068 mmol) was dissolved in dichloromethane (1 mL) and trifluoroacetic acid (0.5 mL), and the system reacted at room temperature for 1 h. After concentration, the concentrate was basified with ammonia water, and concentrated again. The crude product was prepared by HPLC (column: Ultimate XB-C18, 19 mm*150 mm, 10 um; mobile phase: A: purified water (0.05% ammonia water) B: pure acetonitrile; flow rate: 5 mL/min; gradient: B/A = 30%-40% over 9 min; wavelength: 214 nm; column temperature: room temperature) to give two isomeric compounds. A structure of one isomeric compound was arbitrarily assigned as (R)-2-hydroxy-1-(6-(3-methyl-1H-pyrrolo[2,3-b]pyridin-5-yl)-8-((S)-pyrrolidin-2-yl)-3,4-dihydroisoquinolin-2(1H)-yl)-2-(pyridin-3-yl)ethan-1-one (Z287, 1 mg, yield: 3.2%, retention time: 1.296 min) ES-API: [M+H]⁺= 468.8; a structure of the other isomeric compound was arbitrarily assigned as (S)-2-hydroxy-1-(6-(3-methyl-1H-pyrrolo[2,3-b]pyridin-5-yl)-8-((S)-pyrrolidin-2-yl)-3,4-dihydroisoquinolin-2(1H)-yl)-2-(pyridin-3-yl)ethan-1-one (Z288, 3.1 mg, yield: 9.8%, retention time: 1.307 min). ES-API: [M+H]⁺ = 468.8.

### Example 79. Synthesis of compound Z289

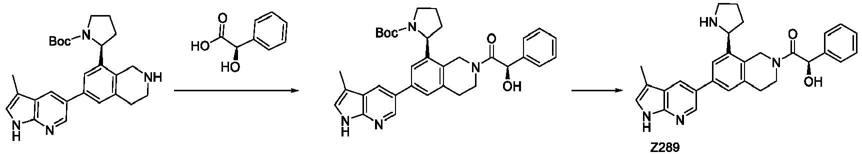

Step 1: (R)-2-Hydroxy-2-phenylacetic acid (12.66 mg, 0.083 mmol), 1-(3-dimethylaminopropyl)-3-ethylcarbodiimide hydrochloride (39.89 mg, 0.21 mmol), triethylamine (21.01 mg, 0.21 mmol) and 1-hydroxybenzotriazole (14.06 mg, 0.11 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in N,N-dimethylformamide (1 mL), and the mixture was stirred at 25°C for 2 h. The reaction was monitored by LCMS for completion, and quenched by the addition of water. The reaction mixture was extracted with ethyl acetate, washed with saturated brine, dried over anhydrous sodium sulfate, and concentrated to give tert-butyl (S)-2-(2-((R)-2-hydroxy-2-phenylacetyl)-6-(3-methyl-1H-pyrrolo[2,3-b]pyridin-5-yl)-1,2, 3,4-tetrahydroisoquinolin-8-yl)pyrrolidine-1-carboxylate (30 mg, crude product). ES-API:[M+H]⁺= 567.4.

Step 2: Trifluoroacetic acid (0.5 mL) was added to a solution of the tert-butyl (S)-2-(2-((R)-2-hydroxy-2-phenylacetyl)-6-(3-methyl-1H-pyrrolo[2,3-b]pyridin-5-yl)-1,2, 3,4-tetrahydroisoquinolin-8-yl)pyrrolidine-1-carboxylate (30 mg, 0.053 mmol) in dichloromethane (1 mL) and stirred at room temperature for 1 h. The reaction was checked by LCMS for completion. The reaction mixture was adjusted to pH 7 with saturated sodium bicarbonate, treated with dichloromethane/water, dried over anhydrous sodium sulfate, and concentrated. The crude product was prepared and purified (column: Ultimate XB-C18, 50*250 mm, 10 um; mobile phase: A: purified water (0.05% ammonia water) B: pure acetonitrile; flow rate: 80 ml/min; gradient: B/A = 20%-90% over 50 min; wavelength: 214 nm; column temperature: room temperature) to give (R)-2-hydroxy-1-(6-(3-methyl-1H-pyrrolo[2,3-b]pyridin-5-yl)-8-((S)-pyrrolidin-2-yl)-3,4-dihydroisoquinolin-2(1H)-yl)-2-acetophenone (Z289, 2.2 mg, yield: 8.9 %) as a white powder. ES-API:[M+H]⁺=467.3.

### Example 80. Synthesis of compound Z290

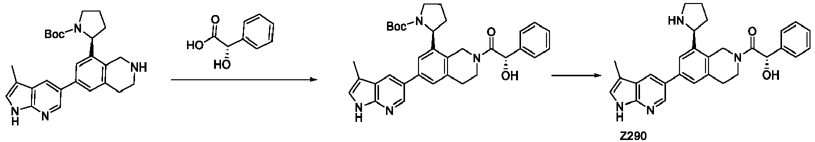

Step 1: (S)-2-Hydroxy-2-phenylacetic acid (12.66 mg, 0.083 mmol), 1-(3-dimethylaminopropyl)-3-ethylcarbodiimide hydrochloride (39.89 mg, 0.21 mmol), triethylamine (21.01, 0.21 mmol) and 1-hydroxybenzotriazole (14.06 mg, 0.11 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in N,N-dimethylformamide (1 mL), and the mixture was stirred at 25°C for 2 h. The reaction was monitored by LCMS for completion, and quenched by the addition of water. The reaction mixture was extracted with ethyl acetate, washed with saturated brine, dried over anhydrous sodium sulfate, and concentrated to give a product tert-butyl (S)-2-(2-((S)-2-hydroxy-2-phenylacetyl)-6-(3-methyl-1H-pyrrolo[2,3-b]pyridin-5-yl)-1,2, 3,4-tetrahydroisoquinolin-8-yl)pyrrolidine-1-carboxylate (30 mg, crude product). ES-API:[M+H]⁺= 567.4.

Step 2: Trifluoroacetic acid (0.5 mL was added to a solution of the tert-butyl (S)-2-(2-((S)-2-hydroxy-2-phenylacetyl)-6-(3-methyl-1H-pyrrolo[2,3-b]pyridin-5-yl)-1,2, 3,4-tetrahydroisoquinolin-8-yl)pyrrolidine-1-carboxylate (30 mg, 0.053 mmol) in dichloromethane (1 mL) and stirred at room temperature for 1 h. The reaction was monitored by LCMS for completion. The reaction mixture was adjusted to pH 7 with saturated sodium bicarbonate, treated with dichloromethane/water, dried over anhydrous sodium sulfate, and concentrated. The crude product was prepared and purified (column: Ultimate XB-C18, 50*250 mm, 10 um; mobile phase: A: purified water (0.05% ammonia water) B: pure acetonitrile; flow rate: 80 ml/min; gradient: B/A = 20%-90% over 50 min; wavelength: 214 nm; column temperature: room temperature) to give (S)-2-hydroxy-1-(6-(3-methyl-1H-pyrrolo[2,3-b]pyridin-5-yl)-8-((S)-pyrrolidin-2-yl)-3,4-dihydroisoquinolin-2(1H)-yl)-2-acetophenone (Z290, 3.3 mg, yield: 13.4%) as a white powder. ES-API:[M+H]⁺=467.3.

### Example 81. Synthesis of compound Z291

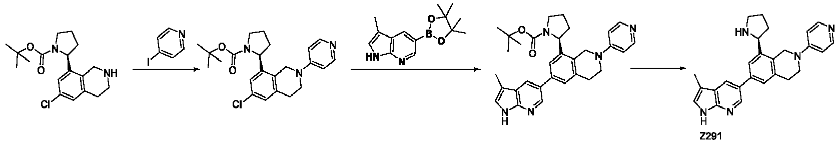

Step 1: In the presence of protective nitrogen, a mixture of tert-butyl (S)-2-(6-chloro-1,2,3,4-tetrahydroisoquinolin-8-yl)pyrrolidine-1-carboxylate (10 mg, 30 µmol), 4-iodopyridine (12 mg, 59 µmol), chloro (2-dicyclohexylphosphino-2',6'-dimethoxy-1,1'-biphenylyl)(2'-amino-1,1'-biphenyl-2-yl) palladium (II) (3 mg, 4 µmol), 2-dicyclohexylphosphine-2',6'-dimethoxybiphenyl (2 mg, 5 µmol) and cesium carbonate (30 mg, 92 µmol) in toluene (1 mL) was stirred at 100°C for 3 h. Upon completion of the reaction, the reaction mixture was concentrated. The crude product was purified by column chromatography (0-100% ethyl acetate/petroleum ether) to give tert-butyl (S)-2-(6-chloro-2-(pyridin-4-yl)-1,2,3,4-tetrahydroisoquinolin-8-yl)pyrrolidine-1-carboxyl ate (5 mg, yield: 40%). ES-API: [M+H]⁺ = 414.2.

Step 2: In the presence of protective nitrogen, a mixed solution of the tert-butyl (S)-2-(6-chloro-2-(pyridin-4-yl)-1,2,3,4-tetrahydroisoquinolin-8-yl)pyrrolidine-1-carboxyl ate (25 mg, 60 µmol), 3-methyl-5-(4,4,5,5-tetramethyl-dioxaborolan-2-yl)-1*H*-pyrrolo[2,3-*b*]pyridine (31 mg, 120 µmol), chloro (2-dicyclohexylphosphino-2',6'-dimethoxy-1,1'-biphenylyl)(2'-amino-1,1'-biphenyl-2-yl) palladium (II) (5 mg, 7 µmol), 2-dicyclohexylphosphine-2',6'-dimethoxybiphenyl (3 mg, 7 µmol) and potassium carbonate (25 mg, 181 µmol) in 1,4-dioxane (2 mL) and water (0.4 mL) was stirred at 120°C for 2 h. Ethyl acetate (10 mL) was poured into the reaction mixture, and the obtained mixture was washed with saturated brine (5 mL) and water (5 mL) successively. The organic phase was dried over anhydrous sodium sulfate, filtered, and concentrated. The crude product was purified by column chromatography (methanol/dichloromethane = 0-10%) to give tert-butyl (S)-2-(6-(3-methyl-1*H*-pyrrolo[2,3-*b*]pyridin-5-yl)-2-(pyridin-4-yl)-1,2,3,4-tetrahydroisoq uinolin-8-yl)pyrrolidine-1-carboxylate (25 mg, yield: 81.7%) as a clear oily liquid. ES-API: [M+H]⁺ = 510.2.

Step 3: Trifluoroacetic acid (0.5 mL) was added dropwise to a solution of the tert-butyl (S)-2-(6-(3-methyl-1*H*-pyrrolo[2,3-*b*]pyridin-5-yl)-2-(pyridin-4-yl)-1,2,3,4-tetrahydroisoq uinolin-8-yl)pyrrolidine-1-carboxylate (30 mg, 59 µmol) in dichloromethane (1 mL) under an ice bath condition and stirred at room temperature for 1 h. The obtained mixture was concentrated, and the crude product was purified by preparative chromatographic column (ammonium bicarbonate method) to give (S)-6-(3-methyl-1*H*-pyrrolo[2,3-*b*]pyridin-5-yl)-2-(pyridin-4-yl)-8-(pyrrolidin-2-yl)-1,2,3, 4-tetrahydroisoquinoline (Z291, 14.25 mg, purity: 100%, yield: 59%) as a white solid. ES-API: [M+H]⁺ = 410.2. ¹H NMR (500 MHz, DMSO-d6) δ 11.38 (s, 1H), 8.56 (d, *J* = 2.0 Hz, 1H), 8.23 - 8.19 (m, 3H), 7.82 (s, 1H), 7.60 (s, 1H), 7.28 (s, 1H), 7.01 (d, *J* = 6.0 Hz, 2H), 4.81 - 4.75 (m, 1H), 4.71 (d, *J* = 16.5 Hz, 1H), 4.58 (d, *J* = 16.0 Hz, 1H), 3.75 - 3.69 (m, 1H), 3.67 - 3.60 (m, 1H), 3.43 - 3.36 (m, 1H), 3.31 - 3.22 (m, 1H), 3.07 - 3.00 (m, 2H), 2.44 - 2.35 (m, 1H), 2.32 (s, 3H), 2.18 - 1.92 (m, 3H).

### Example 82. Synthesis of compound Z292

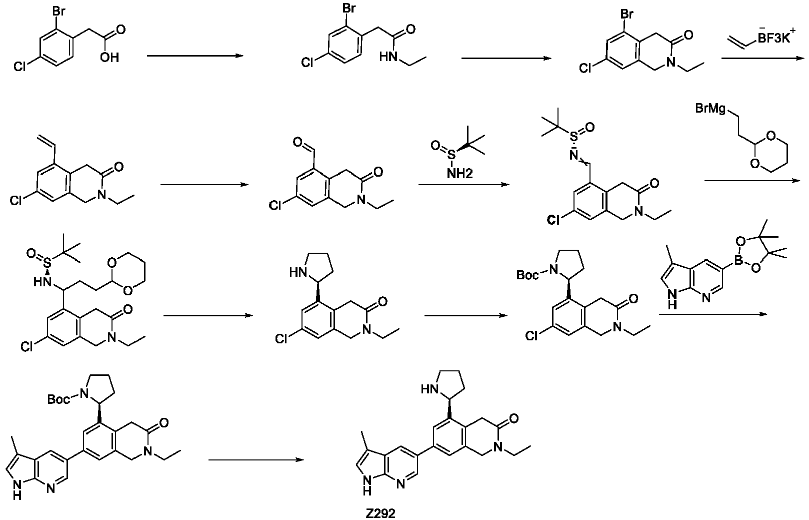

Step 1: 2-Bromo-4-chlorophenylacetic acid (26 g, 104.213 mmol) and ethylamine hydrochloride (10.1 g, 125.055 mmol) were dissolved in dichloromethane (200 mL). Ethyl diisopropylamine (51.051 mL, 312.638 mmol) and 1-propylphosphonic anhydride (97.699 mL, 156.319 mmol, 50% in ethyl acetate) were added under an ice-water bath condition. The mixture was stirred at room temperature for 1 h. The mixture was washed with water (100 mL x 2) and concentrated. Ethyl acetate was added to the residue, and the obtained mixture was filtered. The filter cake was dried to give 2-(2-bromo-4-chlorophenyl)-N-ethylacetamide (18 g, 65.085 mmol, yield: 62.45%). ES-API: [M+H]⁺=275.9, 277.9.

Step 2: The 2-(2-bromo-4-chlorophenyl)-N-ethylacetamide (12 g, 43.390 mmol) was dissolved in Eaton's reagent (100 mL) solution. Paraformaldehyde (6.51 g, 216.951 mmol) was added. The mixture was stirred at 100°C for 3 h. The obtained mixture was cooled, poured into ice water (100 mL), and extracted with ethyl acetate (100 mL x 3). The ethyl acetate layers were combined, washed with saturated sodium chloride solution (100 mL x 1), dried over anhydrous sodium sulfate, filtered, and concentrated. The crude product was purified by column chromatography (petroleum ether/tetrahydrofuran = 70/30) to give 5-bromo-7-chloro-2-ethyl-1,4-dihydroisoquinolin-3(2H)-one (14 g, crude product). ES-API: [M+H]⁺=287.9, 289.9.

Step 3: Potassium vinyltrifluoroborate (13.00 g, 97.030 mmol), triethylamine (13.487 mL, 97.030 mmol) and 1,1-bis(diphenylphosphino)ferrocene dichloropalladium-dichloromethane complex (1.98 g, 2.426 mmol) were added to a solution of the 5-bromo-7-chloro-2-ethyl-1,4-dihydroisoquinolin-3(2H)-one (14 g, crude product) in ethanol (420 mL). The mixture was degassed with nitrogen and stirred at 100°C for 4 h. The obtained mixture was poured into water (250 mL), and extracted with ethyl acetate (250 mL x 3). The ethyl acetate layers were combined, washed with saturated sodium chloride solution (200 mL x 1), dried over anhydrous sodium sulfate, filtered, and concentrated. The crude product was purified by column chromatography (petroleum ether/ethyl acetate = 40/60) to give 7-chloro-2-ethyl-5-vinyl-1,4-dihydroisoquinolin-3(2H)-one (5 g, 19.516 mmol, yield: 44.97%). ES-API: [M+H]⁺=236.0.

Step 4: The 7-chloro-2-ethyl-5-vinyl-1,4-dihydroisoquinolin-3(2H)-one (5 g, 21.213 mmol) was dissolved in tetrahydrofuran (120 mL) solution under an ice-water bath condition, and slowly added to a solution of sodium periodate (27.22 g, 127.275 mmol) and potassium osmate dihydrate (0.23 g, 0.636 mmol) in water (60 mL). The mixture was stirred at room temperature for 1 h. The obtained mixture was poured into ethyl acetate (100 mL), and filtered. The filtrate was dried over anhydrous sulfuric acid, filtered, and concentrated. The crude product was purified by column chromatography (petroleum ether/ethyl acetate = 20/80) to give 7-chloro-2-ethyl-3-oxo-1,2,3,4-tetrahydroisoquinoline-5-carbaldehyde (2.8 g, 11.780 mmol, yield: 55.53%). ES-API: [M+H]⁺=238.0.

Step 5: The 7-chloro-2-ethyl-3-oxo-1,2,3,4-tetrahydroisoquinoline-5-carbaldehyde (2.8 g, 11.780 mmol) and (S)-2-methylpropane-2-sulfinamide (2.86 g, 23.560 mmol) were dissolved in dried dichloromethane (30 mL). Tetraethyl titanate (10.75 g, 47.120 mmol) was added at room temperature. The mixture was stirred at room temperature for 18 h. The obtained mixture was poured into saturated brine (50 mL) and extracted with dichloromethane (100 mL x 3). The organic phases were combined, filtered, washed with saturated brine solution (100 mL x 1), dried over anhydrous sodium sulfate, filtered, and concentrated. The crude product was purified by column chromatography (petroleum ether/tetrahydrofuran = 30/70) to give (S)-N-((7-chloro-2-ethyl-3-oxo-1,2,3,4-tetrahydroisoquinolin-5-yl)methylene)-2-methylpr opane-2-sulfinamide (3.34 g, 9.798 mmol, yield: 83.18%). ES-API: [M+H]⁺=341.1.

Step 6: The (S)-N-((7-chloro-2-ethyl-3-oxo-1,2,3,4-tetrahydroisoquinolin-5-yl)methylene)-2-methylpr opane-2-sulfinamide (3.34 g, 9.798 mmol) was dissolved in dried tetrahydrofuran (30 mL). (2-(1,3-Dioxan-2-yl)ethyl)magnesium bromide (78.4 mL, 39.2 mmol) was slowly added under a dry ice bath condition at -78°C. The mixture was stirred at room temperature for 1 h. The reaction was quenched by the addition of saturated aqueous ammonium chloride solution (5 mL), and the reaction mixture was extracted with ethyl acetate (100 mL x 3). The organic phases were combined, washed with saturated brine solution (100 mL x 1), dried over anhydrous sodium sulfate, filtered, and concentrated. The crude product was purified by column chromatography (petroleum ether/tetrahydrofuran = 30/70) to give (S)-N-(1-(7-chloro-2-ethyl-3-oxo-1,2,3,4-tetrahydroisoquinolin-5-yl)-3-(1,3-dioxan-2-yl)p ropyl)-2-methylpropane-2-sulfinamide (700 mg, 1.56 mmol, yield: 15.9%). ES-API: [M+H]⁺=457.1.

Step 7: The (S)-N-(1-(7-chloro-2-ethyl-3-oxo-1,2,3,4-tetrahydroisoquinolin-5-yl)-3-(1,3-dioxan-2-yl)p ropyl)-2-methylpropane-2-sulfinamide (0.70 g, 1.56 mmol) was dissolved in trifluoroacetic acid (5 mL), and water (0.25 mL) was added. The mixture was stirred at room temperature for 0.5 h. Triethylsilane (1.78 g, 15.316 mmol) was then added. The mixture was stirred at room temperature for 2 h. The reaction mixture was concentrated directly to give (S)-7-chloro-2-ethyl-5-[pyrrol-2-yl]-1,4-dihydroisoquinolin-3(2H)-one (700 mg, crude product). ES-API: [M+H]⁺=279.1.

Step 8: The (S)-7-chloro-2-ethyl-5-[pyrrol-2-yl]-1,4-dihydroisoquinolin-3(2H)-one (700 mg, crude product) was dissolved in dichloromethane (20 mL). Triethylamine (0.178 mL, 1.281 mmol) and di-tert-butyl dicarbonate (0.147 mL, 0.640 mmol) were added. The mixture was stirred at room temperature for 0.5 h. The reaction mixture was concentrated. The crude product was purified by column chromatography (ethyl acetate/petroleum ether = 40/60) to give tert-butyl (S)-2-(7-chloro-2-ethyl-3-oxo-1,2,3,4-tetrahydroisoquinolin-5-yl)pyrrolidine-1-carboxylat e (110 mg, 0.226 mmol, yield of the 2 steps: 14.48%). ES-API: [M+H]⁺=379.1.

Step 9: The tert-butyl (S)-2-(7-chloro-2-ethyl-3-oxo-1,2,3,4-tetrahydroisoquinolin-5-yl)pyrrolidine-1-carboxylat e (110 mg, 0.290 mmol), 3-methyl-5-(4,4,5,5-tetramethyl-1,3,2-dioxaborolan-2-yl)-1H-pyrrolo[2,3-b]pyridine (112.41 mg, 0.435 mmol), chloro (2-dicyclohexylphosphino-2',6'-dimethoxy-1,1'-biphenylyl)(2'-amino-1,1'-biphenyl-2-yl) palladium (II) (20.90 mg, 0.029 mmol) and potassium carbonate (120.37 mg, 0.871 mmol) were added to 1,4-dioxane (20 mL) and water (2 mL). The system was replaced with nitrogen and heated in an oil bath at 120°C for 1 h. Upon completion of the reaction, ethyl acetate (100 mL) was added. The obtained mixture was washed with water (100 mL) and saturated brine (100 mL), dried over anhydrous sodium sulfate, and concentrated. The crude product was purified by column chromatography (ethyl acetate/petroleum ether = 80/20) to give tert-butyl (S)-2-(2-ethyl-7-(3-methyl-1H-pyrrolo[2,3-b]pyridin-5-yl)-3-oxo-1,2,3,4-tetrahydroisoqui nolin-5-yl)pyrrolidine-1-carboxylate (40 mg, 0.064 mmol, yield: 22.06%). ES-API: [M+H]⁺=475.2.

Step 10: The tert-butyl (S)-2-(2-ethyl-7-(3-methyl-1H-pyrrolo[2,3-b]pyridin-5-yl)-3-oxo-1,2,3,4-tetrahydroisoqui nolin-5-yl)pyrrolidine-1-carboxylate (40 mg, 0.064 mmol) was dissolved in dichloromethane (5 mL) and added to trifluoroacetic acid (3 mL), and the system reacted at room temperature for 0.5 h. Upon completion of the reaction, the reaction mixture was concentrated. After neutralization with ammonia-methanol solution (1 mL), the obtained mixture was concentrated again. The crude product was purified by prep-HPLC (ammonia water method) to give (S)-2-ethyl-7-(3-methyl-1H-pyrrolo[2,3-b]pyridin-5-yl)-5-(pyrrolidin-2-yl)-1,4-dihydroiso quinolin-3(2H)-one (Z292, 12 mg, yield: 38.00%). ES-API: [M+H]⁺=475.2. ¹H NMR (500 MHz, DMSO-*d*₆) δ 11.37 (d, *J* = 2.4 Hz, 1H), 8.49 (d, *J* = 2.2 Hz, 1H), 8.14 (d, *J* = 2.2 Hz, 1H), 7.81 (d, *J* = 1.9 Hz, 1H), 7.55 (d, *J* = 1.9 Hz, 1H), 7.28 - 7.25 (m, 1H), 4.58 (d, *J* = 6.8 Hz, 2H), 4.34 (t, *J* = 7.8 Hz, 1H), 3.64 - 3.57 (m, 2H), 3.48 (q, *J* = 7.1 Hz, 3H), 3.20-3.13 (m, 1H), 3.05-2.98 (m, 1H), 2.32 - 2.31 (m, 3H), 2.23-2.19 (m, 1H), 1.87 - 1.81 (m, 2H), 1.59 - 1.52 (m, 1H), 1.13 - 1.10 (m, 3H).

### Example 83. Synthesis of compound Z293

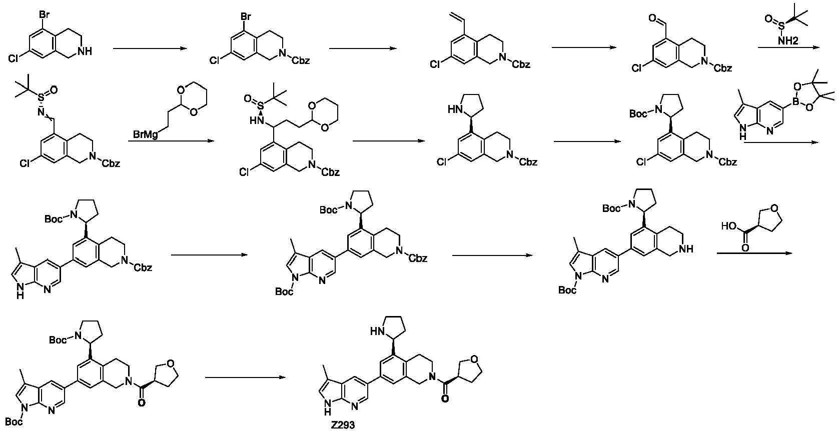

Step 1: 5-Bromo-7-chloro-1,2,3,4-tetrahydroisoquinoline (11.37 g, 46.120 mmol) was dissolved in tetrahydrofuran (100 mL) and water (30 mL). Potassium carbonate (19.12 g, 138.360 mmol) was added, and the system was cooled to 0°C. Benzyl chloroformate (16.334 mL, 115.300 mmol) was slowly added, and the system reacted at room temperature for 2 h. Ethyl acetate (100 mL) was added. The obtained mixture was washed with water (50 mL) and saturated brine (50 mL) successively, dried over anhydrous sodium sulfate, and concentrated to give a crude solid. The crude product was beaten with dichloromethane (50 mL), filtered, and rinsed with petroleum ether. The filter cake was dried to give a product benzyl 5-bromo-7-chloro-1,2,3,4-tetrahydroisoquinoline-2-carboxylate (15 g, yield: 83.73%). ES-API: [M+H]⁺=380.1, 382.1.

Step 2: The benzyl 5-bromo-7-chloro-1,2,3,4-tetrahydroisoquinoline-2-carboxylate (15 g, 38.616 mmol) and potassium vinyltrifluoroborate (10.56 g, 78.808 mmol) were dissolved in anhydrous ethanol (300 mL). Triethylamine (5.477 mL, 39.404 mmol) and [1,1'-bis(diphenylphosphino)ferrocene]dichloropalladium-dichloromethane complex (1.61 g, 1.970 mmol) were added. In the presence of protective nitrogen, the system was heated to 90°C in an oil bath for 18 h. The obtained mixture was poured into ice water (100 mL), extracted with ethyl acetate (100 mL x 3), washed with saturated brine (50 mLx1), dried over anhydrous sodium sulfate, and concentrated to give a crude product. The crude product was purified by column chromatography (petroleum ether/ethyl acetate = 80/20) to give benzyl 7-chloro-5-vinyl-1,2,3,4-tetrahydroisoquinoline-2-carboxylate (12 g, yield: 92.90%). ES-API: [M+H]⁺=328.1.

Step 3: The benzyl 7-chloro-5-vinyl-1,2,3,4-tetrahydroisoquinoline-2-carboxylate (12 g, 36.607 mmol) was dissolved in tetrahydrofuran (400 mL) and water (200 mL). Sodium periodate (46.98 g, 219.639 mmol) and potassium osmate dihydrate (0.30 g, 0.805 mmol) were slowly added successively, and the system reacted at room temperature for 2 h. Ethyl acetate (200 mL) was added, and the obtained mixture was stirred and filtered. The filtrate was washed with water (100 mL) and saturated brine (200 mLx1) successively, dried over anhydrous sodium sulfate, and concentrated to give a crude product. The crude product was purified by silica gel column chromatography (petroleum ether/ethyl acetate = 80/20) to give a product benzyl 7-chloro-5-formyl-1,2,3,4-tetrahydroisoquinoline-2-carboxylate (9 g, yield: 74.55%). ES-API: [M+H]⁺=330.1.

Step 4: The benzyl 7-chloro-5-formyl-1,2,3,4-tetrahydroisoquinoline-2-carboxylate (9 g, 27.291 mmol) and (S)-2-methylpropane-2-sulfinamide (8.27 g, 68.227 mmol) were dissolved in dried dichloromethane (180 mL). Tetraethyl titanate (24.90 g, 109.164 mmol) was added at room temperature and stirred at room temperature for 18 h. The obtained mixture was poured into brine (200 mL) and extracted with dichloromethane (100 mL x 3). The organic phases were combined, filtered, washed with saturated aqueous sodium chloride solution (200 mL x 1), dried over anhydrous sodium sulfate, filtered, and concentrated. The crude product was purified by column chromatography (petroleum ether/tetrahydrofuran = 30/70) to give (S)-benzyl 5-(((tert-butylsulfinyl)imino)methyl)-7-chloro-3,4-dihydroisoquinoline-2(1H)-carboxylate (10.5 g, yield: 88.83%). ES-API: [M+H]⁺=433.1.

Step 5: The (S)-benzyl 5-(((tert-butylsulfinyl)imino)methyl)-7-chloro-3,4-dihydroisoquinoline-2(1H)-carboxylate (10 g, 23.096 mmol) was dissolved in dried tetrahydrofuran (100 mL). (2-(1,3-Dioxan-2-yl)ethyl)magnesium bromide (184.770 mL, 92.385 mmol, 0.5M in tetrahydrofuran) was slowly added under a dry ice bath condition at -78°C. The mixture was stirred at room temperature for 0.5 h. The reaction was quenched by the addition of aqueous ammonium chloride solution (50 mL), and the reaction mixture was extracted with ethyl acetate (100 mLx2). The organic phases were combined, washed with saturated aqueous sodium chloride solution (200 mL x 1), dried over anhydrous sodium sulfate, filtered, and concentrated. The crude product was purified by column chromatography (petroleum ether/tetrahydrofuran = 30/70) to give benzyl 5-(1-(((S)-tert-butylsulfinyl)amino)-3-(1,3-dioxan-2-yl)propyl)-7-chloro-3,4-dihydroisoqu inoline-2(1H)-carboxylate (11.67 g, yield: 92%). ES-API: [M+H]⁺=549.2.

Step 6: The benzyl 5-(1-(((S)-tert-butylsulfinyl)amino)-3-(1,3-dioxan-2-yl)propyl)-7-chloro-3,4-dihydroisoqu inoline-2(1H)-carboxylate (11.67 g, 21.249 mmol) was dissolved in trifluoroacetic acid (100 mL), and water (5 mL) was added. The mixture was stirred at room temperature for 0.5 h. Triethylsilyl hydride (33.851 mL, 212.518 mmol) was added and stirred at room temperature for 2 h. The reaction mixture was concentrated to give (S)-benzyl 7-chloro-5-(pyrrolidin-2-yl)-3,4-dihydroisoquinoline-2(1H)-carboxylate (15 g, crude product). ES-API: [M+H]⁺=371.1.

Step 7: The (S)-benzyl 7-chloro-5-(pyrrolidin-2-yl)-3,4-dihydroisoquinoline-2(1H)-carboxylate (15 g, crude product) was dissolved in tetrahydrofuran (150 mL). N,N-diisopropylethyl amine (26.737 mL, 161.777 mmol) and di-tert-butyl dicarbonate (11.150 mL, 48.533 mmol) were added. The mixture was stirred at room temperature for 1 h. Ethyl acetate (100 mL) was added. The obtained mixture was washed with water (100 mLx1) and saturated brine (100 mL×1) successively and concentrated. The crude product was purified by silica gel column chromatography (tetrahydrofuran/petroleum ether = 40/60) to give (S)-benzyl 5-(1-(tert-butoxycarbonyl)pyrrolidin-2-yl)-7-chloro-3,4-dihydroisoquinoline-2(1H)-carbox ylate (10 g, yield of the 2 steps: 65.62%). ES-API: [M+H]⁺=471.2.

Step 8: The (S)-benzyl 5-(1-(tert-butoxycarbonyl)pyrrolidin-2-yl)-7-chloro-3,4-dihydroisoquinoline-2(1H)-carbox ylate (1.00 g, 2.123 mmol), 3-methyl-5-(4,4,5,5-tetramethyl-1,3,2-dioxaborolan-2-yl)-1H-pyrrolo[2,3-b]pyridine (660 mg, 2.548 mmol), chloro (2-dicyclohexylphosphino-2',6'-dimethoxy-1,1'-biphenylyl)(2'-amino-1,1'-biphenyl-2-yl) palladium (II) (0.08 g, 0.106 mmol) and potassium carbonate (0.88 g, 6.369 mmol) were added to 1,4-dioxane (20 mL) and water (2 mL). The system was replaced with nitrogen and heated in an oil bath at 120°C for 1 h. Upon completion of the reaction, ethyl acetate (100 mL) was added. The obtained mixture was washed with water (100 mL) and saturated brine (100 mL) successively, dried over anhydrous sodium sulfate, and concentrated to give (S)-benzyl 5-(1-(tert-butoxycarbonyl)pyrrolidin-2-yl)-7-(3-methyl-1H-pyrrolo[2,3-b]pyridin-5-yl)-3,4 -dihydroisoquinoline-2(1H)-carboxylate (1.8 g, crude product). ES-API: [M+H]⁺=567.3.

Step 9: The (S)-benzyl 5-(1-(tert-butoxycarbonyl)pyrrolidin-2-yl)-7-(3-methyl-1H-pyrrolo[2,3-b]pyridin-5-yl)-3,4 -dihydroisoquinoline-2(1H)-carboxylate (1.8 g, crude product) was dissolved in dichloromethane (30 mL) solution. Di-tert-butyl dicarbonate (0.471 mL, 2.049 mmol) and 4-dimethylamino pyridine (0.03 g, 0.273 mmol) were added successively. The mixture was stirred at room temperature for 0.5 h. The reaction mixture was concentrated. The crude product was purified by column chromatography (petroleum ether/ethyl acetate = 1/1) to give (S)-benzyl 7-(1-(tert-butoxycarbonyl)-3-methyl-1H-pyrrolo[2,3-b]pyridin-5-yl)-5-(1-(tert-butoxycarb onyl)pyrrolidin-2-yl)-3,4-dihydroisoquinoline-2(1H)-carboxylate (600 mg, yield of the 2 steps: 59.29%). ES-API: [M+H]⁺=667.2.

Step 10: The (S)-benzyl 7-(1-(tert-butoxycarbonyl)-3-methyl-1H-pyrrolo[2,3-b]pyridin-5-yl)-5-(1-(tert-butoxycarb onyl)pyrrolidin-2-yl)-3,4-dihydroisoquinoline-2(1H)-carboxylate (600 mg, 0.810 mmol) was dissolved in dichloromethane (10 mL). Palladium chloride (159.56 mg, 0.900 mmol), triethylamine (0.250 mL, 1.800 mmol) and triethylsilane (418.51 mg, 3.599 mmol) were added successively at room temperature, and the system reacted for 0.5 h. Dichloromethane (20 mL) was added. The obtained mixture was washed with water (10 mL) and saturated brine (10 mL) successively, dried over anhydrous sodium sulfate, and concentrated to give tert-butyl (S)-5-(5-(1-(tert-butoxycarbonyl)pyrrolidin-2-yl)-1,2,3,4-tetrahydroisoquinolin-7-yl)-3-me thyl-1H-pyrrolo[2,3-b]pyridine-1-carboxylate (500 mg, crude product). ES-API: [M+H]⁺=533.3.

Step 11: The tert-butyl (S)-5-(5-(1-(tert-butoxycarbonyl)pyrrolidin-2-yl)-1,2,3,4-tetrahydroisoquinolin-7-yl)-3-me thyl-1H-pyrrolo[2,3-b]pyridine-1-carboxylate (100 mg, 0.188 mmol) was dissolved in dichloromethane (5 mL). (R)-Tetrahydrofuran-3-carboxylic acid (32.70 mg, 0.282 mmol), triethylamine (0.078 mL, 0.563 mmol) and 2-(7-azobenzotriazole)-N,N,N',N'-tetramethylurea hexafluorophosphate (142.76 mg, 0.375 mmol) were added, and the system reacted at room temperature for 0.5 h. Upon completion of the reaction, dichloromethane (20 mL) was added. The obtained mixture was washed with water (10 mL) and saturated brine (10 mL) successively, dried over anhydrous sodium sulfate, and concentrated. The crude product was purified by column chromatography (ethyl acetate/petroleum ether = 30%-80%) to give tert-butyl 5-(5-((S)-1-(tert-butoxycarbonyl)pyrrolidin-2-yl)-2-((R)-tetrahydrofuran-3-carbonyl)-1,2,3 ,4-tetrahydroisoquinolin)-7-yl)-3-methyl-1H-pyrrolo[2,3-b]pyridine-1-carboxylate (45 mg, yield: 36.00%). ES-API: [M+H]⁺=631.3.

Step 12: The tert-butyl 5-(5-((S)-1-(tert-butoxycarbonyl)pyrrolidin-2-yl)-2-((R)-tetrahydrofuran-3-carbonyl)-1,2,3 ,4-tetrahydroisoquinolin)-7-yl)-3-methyl-1H-pyrrolo[2,3-b]pyridine-1-carboxylate (45 mg, 0.068 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia-methanol solution (1 mL), the obtained mixture was concentrated again and purified by prep-HPLC (ammonia water method) to give (7-(3-methyl-1H-pyrrolo[2,3-b]pyridin-5-yl)-5-((S)-pyrrolidin-2-yl)-3,4-dihydroisoquinoli n-2(1H)-yl)((R)-tetrahydrofuran-3-yl)methanone (Z293, 18 mg, yield: 57.00%). ES-API: [M+H]⁺=431.2. ¹H NMR (500 MHz, DMSO-*d*₆) δ 11.34 (d, *J* = 6.5 Hz, 1H), 8.47 (t, *J* = 2.6 Hz, 1H), 8.11 (dd, *J* = 7.5, 2.2 Hz, 1H), 7.76 (dd, *J* = 12.1, 2.0 Hz, 1H), 7.44 (dd, *J* = 11.1, 1.9 Hz, 1H), 7.26 (d, *J* = 5.6 Hz, 1H), 4.91 - 4.61 (m, 3H), 4.24 (t, *J* = 7.7 Hz, 1H), 3.94 (t, *J* = 8.1 Hz, 1H), 3.83 (dt, *J* = 11.4, 5.7 Hz, 1H), 3.77 - 3.67 (m, 5H), 3.66 - 3.59 (m, 1H), 3.48 (p, *J* = 7.4 Hz, 2H), 3.13 - 3.08 (m, 1H), 2.98 - 2.82 (m, 3H), 2.31 (s, 3H), 2.20-2.16 (m, 1H), 2.14 - 1.94 (m, 3H), 1.83 - 1.75 (m, 2H), 1.50-1.40 (, 1H).

### Example 84. Synthesis of compound Z294

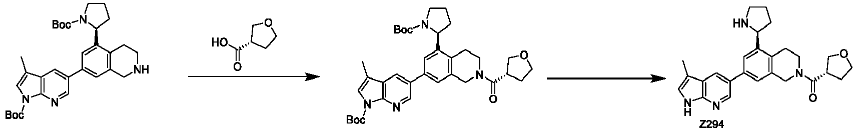

Step 1: tert-butyl (S)-5-(5-(1-(tert-butoxycarbonyl)pyrrolidin-2-yl)-1,2,3,4-tetrahydroisoquinolin-7-yl)-3-me thyl-1H-pyrrolo[2,3-b]pyridine-1-carboxylate (100 mg, 0.188 mmol) was dissolved in dichloromethane (5 mL). (S)-Tetrahydrofuran-3-carboxylic acid (32.70 mg, 0.282 mmol), triethylamine (0.078 mL, 0.563 mmol) and 2-(7-azobenzotriazole)-N,N,N',N'-tetramethylurea hexafluorophosphate (142.76 mg, 0.375 mmol) were added, and the system reacted at room temperature for 0.5 h. Upon completion of the reaction, dichloromethane (20 mL) was added. The obtained mixture was washed with water (10 mL) and saturated brine (10 mL) successively, dried over anhydrous sodium sulfate, and concentrated. The crude product was purified by column chromatography (ethyl acetate/petroleum ether = 30%-80%) to give tert-butyl 5-(5-((*S*)-1-(tert-butoxycarbonyl)pyrrolidin-2-yl)-2-((*S*)-tetrahydrofuran-3-carbonyl)-1,2,3, 4-tetrahydroisoquinolin-7-yl)-3-methyl-1H-pyrrolo[2,3-b]pyridine-1-carboxylate (80 mg, yield: 66.67%). ES-API: [M+H]⁺=631.3.

Step 2: The tert-butyl 5-(5-((*S*)-1-(tert-butoxycarbonyl)pyrrolidin-2-yl)-2-((*S*)-tetrahydrofuran-3-carbonyl)-1,2,3, 4-tetrahydroisoquinolin-7-yl)-3-methyl-1H-pyrrolo[2,3-b]pyridine-1-carboxylate (80 mg, 0.127 mmol) was dissolved in dichloromethane (5 mL) and added to trifluoroacetic acid (3 mL), and the system reacted at room temperature for 0.5 h. Upon completion of the reaction, the reaction mixture was concentrated. After neutralization with ammonia-methanol solution (1 mL), the obtained mixture was concentrated again and purified by prep-HPLC (ammonia water method) to give (7-(3-methyl-1H-pyrrolo[2,3-b]pyridin-5-yl)-5-((S)-pyrrolidin-2-yl)-3,4-dihydroisoquinoli n-2(1H)-yl)((S)-tetrahydrofuran-3-yl)methanone (Z294, 20 mg, yield: 38.00%). ES-API: [M+H]⁺=431.2. ¹H NMR (500 MHz, DMSO-*d*₆) δ 11.34 (d, *J* = 6.8 Hz, 1H), 8.47 (t, *J* = 2.6 Hz, 1H), 8.11 (dd, *J* = 7.5, 2.2 Hz, 1H), 7.76 (dd, *J* = 11.3, 1.9 Hz, 1H), 7.45 (d, *J* = 10.6 Hz, 1H), 7.27 (d, *J* = 5.3 Hz, 1H), 4.82-4.89 (m, 2H), 4.70-4.65 (m, 1H), 4.26 (t, *J* = 7.7 Hz, 1H), 3.94 (d, *J* = 7.9 Hz, 1H), 3.76 - 3.67 (m, 5H), 3.48 (t, *J* = 7.9 Hz, 1H), 3.13 - 3.09 (m, 1H), 2.99 - 2.84 (m, 3H), 2.31 (t, *J* = 1.4 Hz, 3H), 2.22 - 2.16 (m, 1H), 2.13 - 1.98 (m, 3H), 1.82-1.78 (m, 2H).

### Example 85. Synthesis of compound Z295

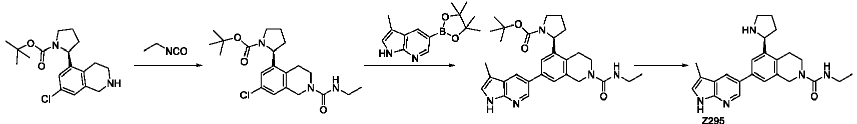

Step 1: Ethyl isocyanate (42 mg, 0.59 mmol) was added to a solution of tert-butyl (S)-2-(7-chloro-1,2,3,4-tetrahydroisoquinolin-5-yl)pyrrolidine-1-carboxylate (200 mg, 0.59 mmol) in dichloromethane (2 mL) and stirred at room temperature for 2 h. Upon completion of the reaction, the reaction mixture was concentrated, and purified by silica gel column chromatography (0-80% tetrahydrofuran/petroleum ether) to give tert-butyl (S)-2-(7-chloro-2-(ethylcarbamoyl)-1,2,3,4-tetrahydroisoquinolin-5-yl)pyrrolidine-1-carbo xylate (55 mg, yield: 22%). ES-API: [M+H]⁺ = 430.1.

Step 2: In the presence of protective nitrogen, a mixed solution of the tert-butyl (S)-2-(7-chloro-2-(ethylcarbamoyl)-1,2,3,4-tetrahydroisoquinolin-5-yl)pyrrolidine-1-carbo xylate (50 mg, 0.12 mmol), 3-methyl-5-(4,4,5,5-tetramethyl-dioxaborolan-2-yl)-1*H*-pyrrolo[2,3-b]pyridine (38 mg, 0.15 mmol), chloro (2-dicyclohexylphosphino-2',6'-dimethoxy-1,1'-biphenylyl)(2'-amino-1,1'-biphenyl-2-yl) palladium (II) (9 mg, 12 µmol), 2-dicyclohexylphosphine-2',6'-dimethoxybiphenyl (5 mg, 12 µmol) and potassium carbonate (50 mg, 0.36 mmol) in 1,4-dioxane (2 mL) and water (0.4 mL) was stirred at 120°C for 2 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by silica gel column chromatography (methanol/dichloromethane = 0-10%) to give tert-butyl (S)-2-(2-formyl-7-(3-methyl-1*H*-pyrrolo[2,3-b]pyridin-5-yl)-1,2,3,4-tetrahydroisoquinolin -5-yl) pyrrolidine-1-carboxylate (40 mg, yield: 65%) as a clear oily liquid. ES-API: [M+H]⁺ = 504.3.

Step 3: Trifluoroacetic acid (0.5 mL) was added dropwise to a solution of the tert-butyl (S)-2-(2-formyl-7-(3-methyl-1*H*-pyrrolo[2,3-b]pyridin-5-yl)-1,2,3,4-tetrahydroisoquinolin -5-yl) pyrrolidine-1-carboxylate (40 mg, 79 µmol) in dichloromethane (1 mL) under an ice bath condition and stirred at room temperature for 1 h. The obtained mixture was concentrated, and purified by preparative chromatographic column (ammonium bicarbonate method) to give (S)-*N*-ethyl-7-(3-methyl-1*H*-pyrrolo[2,3-*b*]pyridin-5-yl)-5-(pyrrolidin-2-yl)-3,4-dihydrois oquinoline-2(1*H*)-carboxamide (Z295, 10 mg, purity: 100%, yield: 31%). ES-API: [M+H]⁺ = 404.2.

### Example 86. Synthesis of compound Z296

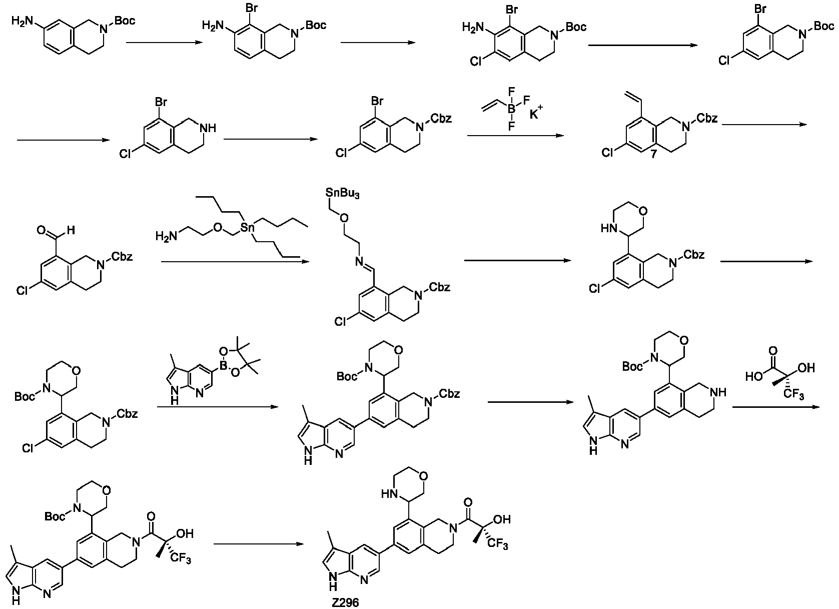

Step 1: Tert-butyl 7-amino-3,4-dihydro-1H-isoquinolin-2-carboxylate (25 g, 60.41 mmol) was dissolved in acetonitrile (500 mL). N-Bromosuccinimide (17.92 g, 100.68 mmol) was added, and the reaction mixture was stirred at -5°C-0°C for 4 h. The reaction mixture was quenched by the addition of aqueous sodium thiosulfate solution, and extracted with ethyl acetate. The organic phase was dried over anhydrous sodium sulfate, and concentrated. The crude product was purified by column chromatography (petroleum ether/ethyl acetate = 10/1) to give tert-butyl 7-amino-8-bromo-3,4-dihydro-1H-isoquinoline-2-carboxylate (30 g, yield: 76.1%). ES-API:[M+1]⁺=271.

Step 2: The tert-butyl 7-amino-8-bromo-3,4-dihydro-1H-isoquinoline-2-carboxylate (30 g, 91.68 mmol) was dissolved in N,N-dimethylformamide (130 mL). N-Chlorosuccinimide (12.86 g, 96.26 mmol) was added, and the system reacted at 50°C for 4 h. The reaction mixture was quenched with aqueous sodium thiosulfate solution, and extracted with ethyl acetate. The organic phase was dried over anhydrous sodium sulfate, and concentrated. The crude product was purified by column chromatography (petroleum ether/ethyl acetate = 10/1) to give tert-butyl 7-amino-8-bromo-6-chloro-3,4-dihydro-1H-isoquinoline-2-carboxylate (22 g, yield: 66.4%). ES-API:[M+1]⁺=305.0.

Step 3: The tert-butyl 7-amino-8-bromo-6-chloro-3,4-dihydro-1H-isoquinoline-2-carboxylate (22 g, 60.84 mmol) was dissolved in tetrahydrofuran (54 mL), water (86 mL) and hypophosphorous acid (214 mL). Sodium nitrite (6.3 g, 91.24 mmol) was added, and the system reacted at room temperature for 4 h. The reaction mixture was quenched with aqueous sodium thiosulfate solution, extracted with ethyl acetate, and washed with a sodium bicarbonate solution. The organic phase was dried over anhydrous sodium sulfate and spun to dryness to give tert-butyl 8-bromo-6-chloro-3,4-dihydro-1H-isoquinoline-2-carboxylate as a crude product which was directly used in the next step. ES-API:[M+1]⁺=290.0.

Step 4: The tert-butyl 8-bromo-6-chloro-3,4-dihydro-1H-isoquinoline-2-carboxylate (21 g, 60.58 mmol) was dissolved in a solution of dichloromethane (50 mL) and trifluoroacetic acid (50 mL), and the system reacted at room temperature for 4 h. The reaction mixture was concentrated to give 8-bromo-6-chloro-1,2,3,4-tetrahydroisoquinoline as a crude product which was directly used in the next step. ES-API:[M+1]⁺=245.90.

Step 5: The 8-bromo-6-chloro-1,2,3,4-tetrahydroisoquinoline (15 g, 60.84 mmol) was dissolved in dichloromethane (50 mL). Benzylcarbonyl chloride (15.57 g, 91.27 mmol) and triethylamine (18.47 g, 182.53 mmol) were added, and the system reacted at room temperature for 4 h. Upon completion of the reaction, the reaction was quenched by the addition of water. The reaction mixture was extracted with ethyl acetate, dried over anhydrous sodium sulfate, and concentrated. The crude product was purified by column chromatography (petroleum ether/ethyl acetate = 10/1) to give a product benzyl 8-bromo-6-chloro-3,4-dihydro-1H-isoquinoline-2-carboxylate (15 g, yield: 60.4%). ES-API:[M+1]⁺=380.0.

Step 6: The benzyl 8-bromo-6-chloro-3,4-dihydro-1H-isoquinoline-2-carboxylate (15 g, 39.4 mmol) was dissolved in ethanol (110 mL). Potassium trifluorovinyl borate (15.84 g, 118.21 mmol), triethylamine (11.96 g, 118.21 mmol) and 1,1-bis(diphenylphosphino)ferrocene dichloropalladium (2.88 g, 3.94 mmol) were added. The system reacted at 80°C for 4 h. Upon completion of the reaction, the reaction mixture was concentrated. The crude product was purified by column chromatography (petroleum ether/ethyl acetate = 10/1) to give a product benzyl 6-chloro-8-vinyl-3,4-dihydro-1H-isoquinoline-2-carboxylate (12 g, yield: 92.7%). ES-API:[M+1]⁺=328.0.

Step 7: 2,6-Lutidine (1.96 g, 36.6 mmol), sodium periodate (62.64 g, 292.86 mmol) and potassium osmate dihydrate (3.42 g, 10.98 mmol) were added to a solution of the benzyl 6-chloro-8-vinyl-3,4-dihydro-1H-isoquinoline-2-carboxylate (12 g, 36.6 mmol) in tetrahydrofuran/water (200 mL/100 mL), and the system reacted at room temperature for 2 h. The reaction mixture was quenched with aqueous sodium thiosulfate solution, and extracted with ethyl acetate. The organic phase was dried over anhydrous sodium sulfate, and concentrated. The crude product was purified by column chromatography (petroleum ether/ethyl acetate = 10/1) to give benzyl 6-chloro-8-formyl-3,4-dihydro-1H-isoquinoline-2-carboxylate (4.5 g, yield: 37.3%). ES-API:[M+1]⁺=330.1.

Step 8: 2-((Tributylstannyl)methoxy)ethanamine (220.85 mg, 0.61 mmol) and 4A molecular sieves were added to a solution of the benzyl 6-chloro-8-formyl-3,4-dihydro-1H-isoquinoline-2-carboxylate (200 mg, 0.61mmol) in dichloromethane (6 mL) and stirred at 30°C for 16 h. Upon completion of the reaction, the reaction mixture was filtered and spun to dryness to give benzyl 6-chloro-8-[(E)-2(tributylstannomethoxy)ethyliminomethyl]-3,4-dihydro-1H-isoquinoline-2-carboxylate (410 mg, crude product) as a crude product which was directly used in the next step.

Step 9: (R,R)-2,2'-Isopropylidene bis(4-phenyl-2-oxazoline) (40.57 mg, 0.12 mmol) was added to a suspension of copper (II) triflate (219.39 mg, 0.6mmol) in hexafluoroisopropanol (2.5 mL) followed by a solution of the benzyl 6-chloro-8-[(E)-2(tributylstannomethoxy)ethyliminomethyl]-3,4-dihydro-1H-isoquinoline-2-carboxylate (410 mg, 0.6 mmol) in hexafluoroisopropanol (2.5 mL), and the reaction mixture was stirred at 30°C for 16 h. The reaction was monitored by LCMS for completion. 1N sodium hydroxide was added. The mixture was stirred, and extracted with dichloromethane. The organic layer was dried over anhydrous sodium sulfate, and concentrated to give a product benzyl 6-chloro-8-(morpholin-3-yl)-3,4-dihydro-1H-isoquinoline-2-carboxylate (235.00 mg, crude product) as a yellow oil which was directly used in the next step. ES-API:[M+H]⁺= 387.2.

Step 10:Triethylamine (184.4 mg, 1.82 mmol) and di-tert-butyl dicarbonate (264.84 g, 1.21 mmol) were added to a solution of the benzyl 6-chloro-8-(morpholin-3-yl)-3,4-dihydro-1H-isoquinoline-2-carboxylate (235 mg, 0.6 mmol, ) in tetrahydrofuran (5 mL) and stirred at room temperature for 2 h. Upon completion of the reaction, the reaction mixture was extracted with ethyl acetate and concentrated. The crude product was purified by column chromatography (ethyl acetate/petroleum ether = 1/5) to give tert-butyl 3-(2-((benzyloxy)carbonyl)-6-chloro-1,2,3,4-tetrahydroisoquinolin-8-yl)morpholine-4-car boxylate (90 mg, yield: 30.42%)ES-API:[M+H-100]⁺= 387.1.

Step 11: 3-Methyl-5-(4,4,5,5-tetramethyl-1,3,2-dioxaborolan-2-yl)-1H-pyrrolo[2,3-b]pyridine (72.06 mg, 0.28 mmol), chloro (2-dicyclohexylphosphino-2',6'-dimethoxy-1,1'-biphenylyl)(2'-amino-1,1'-biphenyl-2-yl) palladium (II) (16.74 mg, 0.023 mmol) and potassium carbonate (96.31 mg, 0.69 mmol) were added to a solution of the tert-butyl 3-(2-((benzyloxy)carbonyl)-6-chloro-1,2,3,4-tetrahydroisoquinolin-8-yl)morpholine-4-car boxylate (90 mg, 0.23 mmol) in dioxane/water (2 mL/0.4 mL). The mixture was heated to 110°C and stirred for 2 h in the presence of protective nitrogen, and the reaction was monitored by LCMS for completion. The obtained mixture was treated with ethyl acetate/water, dried over anhydrous sodium sulfate, and concentrated. The crude product was purified by column chromatography (dichloromethane/methanol = 10/1) to give a product tert-butyl 3-(2-((benzyloxy)carbonyl)-6-(3-methyl-1H-pyrrolo[2,3-b]pyridin-5-yl)-1,2,3,4-tetrahydr oisoquinolin-8-yl)morpholine-4-carboxylate (40 mg, yield: 29.51%). ES-API:[M+H]⁺= 583.4.

Step 12: Palladium on carbon (10 mg) was added to a solution of 3-(2-((benzyloxy)carbonyl)-6-(3-methyl-1H-pyrrolo[2,3-b]pyridin-5-yl)-1,2,3,4-tetrahydr oisoquinolin-8-yl)morpholine-4-carboxylate (40 mg, 0.069 mmol) in methanol (1 mL), and the mixture was stirred under hydrogen atmosphere at 25°C for 1 h. The reaction was monitored by LCMS for completion, the reaction mixture was filtered. The filtrate was dried over anhydrous sodium sulfate, and concentrated to give a product tert-butyl 3-[6-(3-methyl-1H-pyrrolo[2,3-b]pyridin-5-yl)-1,2,3,4-tetrahydroisoquinolin-8-yl]morpho line-4-carboxylate (25 mg, yield: 81.19%). ES-API:[M+H]⁺= 449.3.

Step 13: (S)-3,3,3-Trifluoro-2-hydroxy-2-methylpropanoic acid (114.18 mg, 0.722mmol), 1-(3-dimethylaminopropyl)-3-ethylcarbodiimide hydrochloride (44.87 mg, 0.23 mmol), 1-hydroxybenzotriazole (15.81 mg, 0.12 mmol) and triethylamine (23.64 mg, 0.23 mmol) were added to a solution of the tert-butyl 3-[6-(3-methyl-1H-pyrrolo[2,3-b]pyridin-5-yl)-1,2,3,4-tetrahydroisoquinolin-8-yl]morpho line-4-carboxylate (35 mg, 0.078 mmol) in N,N-dimethylformamide (1 mL), and the mixture was stirred at 25°C for 2 h. The reaction was monitored by LCMS for completion, and quenched by the addition of water. The reaction mixture was extracted with ethyl acetate, dried over anhydrous sodium sulfate, and concentrated. The crude product was purified by column chromatography (dichloromethane/methanol = 10/1) to give a product tert-butyl 3-(6-(3-methyl-1H-pyrrolo[2,3-b]pyridin-5-yl)-2-((S)-3,3,3-trifluoro-2-hydroxy-2-methylp ropanoyl)-1,2,3,4-tetrahydroisoquinolin-8-yl)morpholine-4-carboxylate (10 mg, yield: 21.77%). ES-API:[M+H]⁺= 589.4.

Step 14: Trifluoroacetic acid (0.5 mL) was added to a solution of the tert-butyl 3-(6-(3-methyl-1H-pyrrolo[2,3-b]pyridin-5-yl)-2-((S)-3,3,3-trifluoro-2-hydroxy-2-methylp ropanoyl)-1,2,3,4-tetrahydroisoquinolin-8-yl)morpholine-4-carboxylate (10 mg, 0.017 mmol) in dichloromethane (1 mL) and stirred at room temperature for 1 h. The reaction was monitored by LCMS for completion. The reaction mixture was adjusted to pH 7 with saturated sodium bicarbonate, treated with dichloromethane/water, dried over anhydrous sodium sulfate, and concentrated. The crude product was prepared and purified (column: Ultimate XB-C18, 50*250 mm, 10 um; mobile phase: A: purified water (0.05% ammonia water) B: pure acetonitrile; flow rate: 80 ml/min; gradient: B/A = 20%-90% over 50 min; wavelength: 214 nm; column temperature: room temperature) to give (2S)-3,3,3-trifluoro-2-hydroxy-2-methyl-1-(6-(3-methyl-1H-pyrrolo[2,3-b]pyridin-5-yl)-8-(morpholin-3-yl)-3,4-dihydroisoquinolin-2(1H)-yl)propan-1-one (Z296, 3.8 mg, yield: 45.79%) as a white powder. ES-API:[M+H]⁺=489.3. ¹H NMR (400 MHz, CDCl₃) δ 9.11 (s, 1H), 7.91-7.53 (m, 3H), 7.08 (s, 1H), 4.87-4.44 (m, 2H), 4.39-4.06 (m, 2H), 4.04-3.49 (m, 4H), 3.45-2.72 (m, 4H), 2.62-2.36 (m, 1H), 2.30 (s, 3H), 1.89-1.86 (m, 3H).

### Example 87. Synthesis of compound Z297

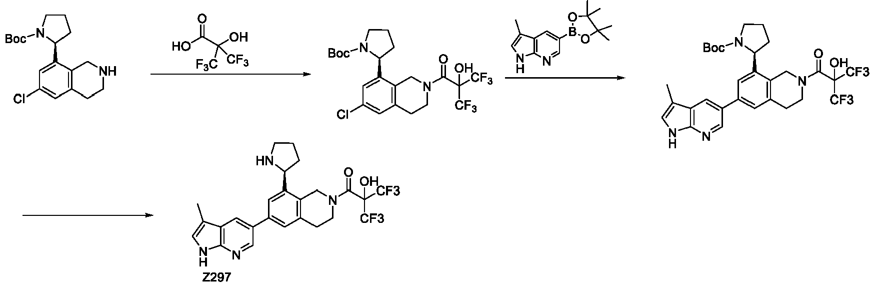

Step 1: 3,3,3-trifluoro-2-hydroxy-2-(trifluoromethyl)propanoic acid (188.85 mg, 0.9 mmol), 1-(3-dimethylaminopropyl)-3-ethylcarbodiimide hydrochloride (170.73 mg, 0.9 mmol), 1-hydroxybenzotriazole (120.34 mg, 0.9 mmol) and triethylamine (90.12 mg, 0.9 mmol) were added to a solution of tert-butyl (S)-2-(6-chloro-1,2,3,4-tetrahydroisoquinolin-8-yl)pyrrolidine-1-carboxylate (100 mg, 0.3 mmol) in N,N-dimethylformamide (3 mL), and the mixture was stirred at 25°C for 2 h. The reaction was monitored by LCMS for completion, and quenched by the addition of water. The reaction mixture was extracted with ethyl acetate, dried over anhydrous sodium sulfate, and concentrated. The crude product was purified by column chromatography (dichloromethane/methanol = 10/1) to give a product tert-butyl (S)-2-(6-chloro-2-(3,3,3-trifluoro-2-hydroxy-2-(trifluoromethyl)propanoyl)-1,2,3,4-tetrahy droisoquinolin-8-yl)pyrrolidine-1-carboxylate (18 mg, yield: 11.42%). ES-API:[M+H-100]⁺= 431.1.

Step 2: 3-Methyl-5-(4,4,5,5-tetramethyl-1,3,2-dioxaborolan-2-yl)-1H-pyrrolo[2,3-b]pyridine (10.5 mg, 0.04 mmol), chloro (2-dicyclohexylphosphino-2',6'-dimethoxy-1,1'-biphenylyl)(2'-amino-1,1'-biphenyl-2-yl) palladium (II) (2.44 mg, 0.003 mmol) and potassium carbonate (14.04 mg, 0.102 mmol) were added to a solution of the tert-butyl (S)-2-(6-chloro-2-(3,3,3-trifluoro-2-hydroxy-2-(trifluoromethyl)propanoyl)-1,2,3,4-tetrahy droisoquinolin-8-yl)pyrrolidine-1-carboxylate (18 mg, 0.034 mmol) in dioxane/water (2 mL/0.4 mL), and the mixture was heated to 110°C and stirred for 2 h in the presence of protective nitrogen. The reaction was monitored by LCMS for completion. The reaction mixture was treated with ethyl acetate/water, dried over anhydrous sodium sulfate, and concentrated. The crude product was purified by column chromatography (dichloromethane/methanol = 10/1) to give a product tert-butyl (S)-2-(6-(3-methyl-1H-pyrrolo[2,3-b]pyridin-5-yl)-2-(3,3,3-trifluoro-2-hydroxy-2-(trifluor omethyl)propanoyl)-1,2,3,4-tetrahydroisoquinolin-8-yl)pyrrolidine-1-carboxylate (12 mg, yield: 56.48%). ES-API:[M+H]⁺=627.3.

Step 3: Trifluoroacetic acid (0.5 mL) was added to a solution of the tert-butyl (S)-2-(6-(3-methyl-1H-pyrrolo[2,3-b]pyridin-5-yl)-2-(3,3,3-trifluoro-2-hydroxy-2-(trifluor omethyl)propanoyl)-1,2,3,4-tetrahydroisoquinolin-8-yl)pyrrolidine-1-carboxylate (12 mg, 0.02 mmol) in dichloromethane (1 mL) and stirred at room temperature for 1 h. The reaction was monitored by LCMS for completion. The reaction mixture was adjusted to pH 7 with saturated sodium bicarbonate, treated with dichloromethane/water, dried over anhydrous sodium sulfate, and concentrated. The crude product was prepared and purified (column: Ultimate XB-C18, 50*250 mm, 10 um; mobile phase: A: purified water (0.05% ammonia water) B: pure acetonitrile; flow rate: 80 ml/min; gradient: B/A = 20%-90% over 50 min; wavelength: 214 nm; column temperature: room temperature) to give (S)-3,3,3-trifluoro-2-hydroxy- 1-(6-(3-methyl-1H-pyrrolo[2,3-b]pyridin-5-yl)-8-(pyrrolidin -2-yl)-3,4-dihydroisoquinolin-2(1H)-yl)-2-(trifluoromethyl)propan-1-one (Z297, 1.3 mg, yield: 12.89%) as a white powder. ES-API:[M+H]⁺=527.2.

### Example 88. Synthesis of compound Z298

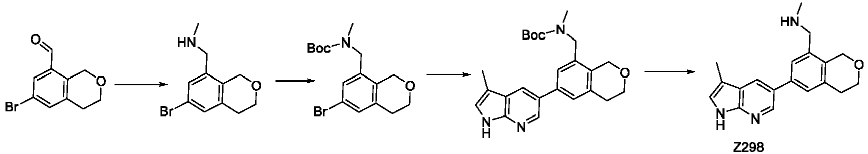

Step 1: 6-Bromoisochroman-8-carbaldehyde (100 mg, 0.415 mmol), methylamine hydrochloride (84 mg, 1.244 mmol) and dichloromethane (10 mL) were added to a 25 mL single-neck round-bottom flask. The obtained mixture was stirred at room temperature for 6 h. Sodium cyanoborohydride (78.2 mg, 1.244 mmol) was added in batches and then stirred at room temperature overnight. Ethyl acetate (30 mL) was added to the reaction mixture. The obtained mixture was washed with saturated brine (30 mLX3), dried over anhydrous sodium sulfate, and concentrated. The crude product was purified by silica gel column chromatography (ethyl acetate:petroleum ether = 10:100) to give 1-(6-bromoisochroman-8-carbaldehyde)-N-methylmethanamine (20 mg, yield: 19%). ES-API: [M+H]⁺=256.1.

Step 2: The 1-(6-bromoisochroman-8-carbaldehyde)-N-methylmethanamine (20 mg, 0.078 mmol), di-tert-butyl dicarbonate (51 mg, 0.23 mmol), triethylamine (24 mg, 0.23 mmol) and dichloromethane (10 mL) were added to a 25 mL single-neck round-bottom flask and stirred for 1 h. Dichloromethane (20 mL) was added. The obtained mixture was washed with saturated brine (20 mLX3), dried over anhydrous sodium sulfate, and concentrated to give tert-butyl ((6-bromoisochroman-8-yl)methyl)(methyl)carbamate (18 mg, yield: 64%) as a crude product. ES-API: [M+H]⁺=356.1.

Step 3: The tert-butyl ((6-bromoisochroman-8-yl)methyl)(methyl)carbamate (18 mg, 0.051 mmol), 3-methyl-5-(4,4,5,5-tetramethyl-1,3,2-dioxaborolan-2-yl)-1H-pyrrolo[2,3-b]pyridine (26 mg, 0.10 mmol), chloro (2-dicyclohexylphosphino-2',6'-dimethoxy-1,1'-biphenylyl)(2'-amino-1,1'-biphenyl-2-yl) palladium (II) (3.6 mg, 0.005 mmol), potassium carbonate (21 mg, 0.15 mmol), dioxane (3 mL) and water (0.3 mL) were added to a 25 mL single-neck round-bottom flask. The system was replaced with nitrogen three times and then protected with a nitrogen balloon. The system reacted under microwave at 110°C for 50 min, and ethyl acetate (10 mL) was added to the reaction mixture. The obtained mixture was washed with saturated brine (5 mLX3), dried over anhydrous sodium sulfate, and concentrated to give tert-butyl methyl ((6-(3-methyl-1H-pyrrolo[2,3-b]pyridin-5-yl)isochroman-8-yl)methyl)carbamate (20 mg, crude product). ES-API: [M+H]⁺=408.2.

Step 4: The tert-butyl methyl ((6-(3-methyl-1H-pyrrolo[2,3-b]pyridin-5-yl)isochroman-8-yl)methyl)carbamate (20 mg, crude product), trifluoroacetic acid (3 mL) and dichloromethane (6 mL) were added to a 5 mL single-neck round-bottom flask and stirred at room temperature for 30 min. The reaction mixture was spun to dryness. The crude product was purified by prep-HPLC (column: Waters XBridge C18, 190*250 mm, 5 µm; mobile phase: A: 0.1% aqueous ammonium bicarbonate solution; mobile phase B: acetonitrile; flow rate: 15 ml/min; column temperature: room temperature) to give N-methyl-1-(6-(3-methyl-1H-pyrrolo[2,3-b]pyridin-5-yl)isochroman-8-yl)methanamine (Z298, 7 mg, yield: 44.5%). ES-API: [M+H]⁺=308.2.

### Example 89. Synthesis of compound Z299

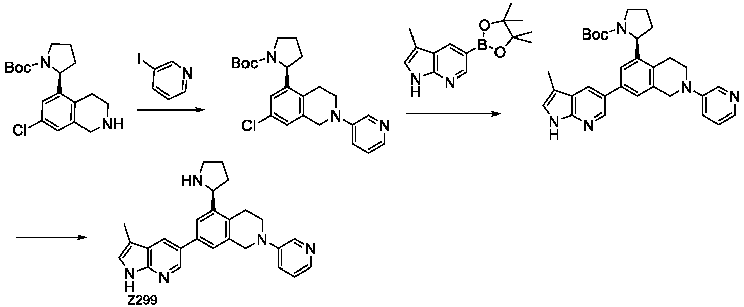

Step 1: tert-butyl (S)-2-(7-chloro-1,2,3,4-tetrahydroisoquinolin-5-yl)pyrrolidine-1-carboxylate (100 mg, 0.297 mmol) was dissolved in dried 1,4-dioxane (3 mL). 3-Iodopyridine (91.28 mg, 0.445 mmol), chloro (2-dicyclohexylphosphino-2',6'-dimethoxy-1,1'-biphenylyl)(2'-amino-1,1'-biphenyl-2-yl) palladium (II) (10.69 mg, 0.015 mmol) and cesium carbonate (193.45 mg, 0.594 mmol) were added successively, and the system was heated to 100°C under microwave for 3 h. The obtained mixture was diluted with ethyl acetate (30 mL), washed with saturated brine (30 mL), dried over anhydrous sodium sulfate, filtered, and concentrated. The crude product was purified by silica gel column chromatography (methanol/dichloromethane = 1/20) to give tert-butyl (S)-2-(7-chloro-2-(pyridin-3-yl)-1,2,3,4-tetrahydroisoquinolin-5-yl)pyrrolidine-1-carboxyl ate (22 mg, yield: 17.90%). ES-API: [M+H]⁺=414.2.

Step 2: The tert-butyl (S)-2-(7-chloro-2-(pyridin-3-yl)-1,2,3,4-tetrahydroisoquinolin-5-yl)pyrrolidine-1-carboxyl ate (22 mg, 0.053 mmol), 3-methyl-5-(4,4,5,5-tetramethyl-1,3,2-dioxaborolan-2-yl)-1H-pyrrolo[2,3-b]pyridine (20.58 mg, 0.08 mmol), chloro (2-dicyclohexylphosphino-2',6'-dimethoxy-1,1'-biphenylyl)(2'-amino-1,1'-biphenyl-2-yl) palladium (II) (3.83 mg, 0.005 mmol) and potassium carbonate (22.04 mg, 0.159 mmol) were added to 1,4-dioxane (3 mL) and water (0.5 mL). The system was replaced with nitrogen three times and reacted under microwave at 115°C for 1 h. Upon completion of the reaction, ethyl acetate (20 mL) was added. The obtained mixture was washed with water (10 mL) and saturated brine (10 mL) successively, and dried over anhydrous sodium sulfate. The crude product was purified by preparative thin layer chromatography (petroleum ether/ethyl acetate = 1/1) to give tert-butyl (S)-2-(7-(3-methyl-1H-pyrrolo[2,3-b]pyridin-5-yl)-2-(pyridin-3-yl)-1,2,3,4-tetrahydroisoq uinolin-5-yl)pyrrolidine-1-carboxylate (15 mg, yield: 55.37%). ES-API: [M+H]⁺=510.3.

Step 3: The tert-butyl (S)-2-(7-(3-methyl-1H-pyrrolo[2,3-b]pyridin-5-yl)-2-(pyridin-3-yl)-1,2,3,4-tetrahydroisoq uinolin-5-yl)pyrrolidine-1-carboxylate (15 mg, 0.029 mmol) was dissolved in dichloromethane (2mL) and added to trifluoroacetic acid (1 mL), and the system reacted at room temperature for 0.5 h. Upon completion of the reaction, the reaction mixture was concentrated. After neutralization with ammonia-methanol solution (1 mL), the obtained mixture was concentrated again and purified by prep-HPLC (formic acid method) to give (S)-7-(3-methyl-1H-pyrrolo[2,3-b]pyridin-5-yl)-2-(pyridin-3-yl)-5-(pyrrolidin-2-yl)-1,2,3, 4-tetrahydroisoquinoline (Z299, formate, 3.5 mg, yield: 27.59%). ES-API: [M+H]⁺=410.2.

### Example 90. Synthesis of compound Z300

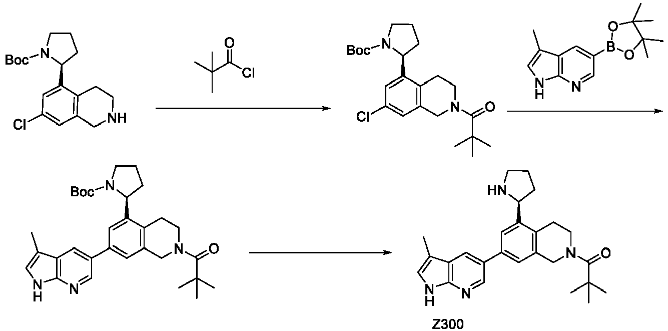

Step 1: Triethylamine (90.12 mg, 890.59 µmol) and pivaloyl chloride (42.95 mg, 356.24 µmol) were added to a solution of tert-butyl (S)-2-(7-chloro-1,2,3,4-tetrahydroisoquinolin-5-yl)pyrrolidine-1-carboxylate (100 mg, 296.86 µmol) in dichloromethane (1 mL) and stirred at 0°C for 15 min. The reaction was monitored by LCMS for completion, and quenched by the addition of water. The reaction mixture was extracted with dichloromethane, dried over anhydrous sodium sulfate, and concentrated. The crude product was purified by column chromatography (methanol/dichloromethane = 0-5%) to give a product tert-butyl (S)-2-(7-chloro-2-pivaloyl-1,2,3,4-tetrahydroisoquinolin-5-yl)pyrrolidine-1-carboxylate (100 mg, yield: 77.9%). ES-API:[M+H-56]⁺=376.1.

Step 2: The compound tert-butyl (S)-2-(7-chloro-2-pivaloyl-1,2,3,4-tetrahydroisoquinolin-5-yl)pyrrolidine-1-carboxylate (53 mg, 125.90 µmol, ) was dissolved in dioxane/water (2 mL/0.4 mL). 3-Methyl-5-(4,4,5,5-tetramethyl-1,3,2-dioxaborolan-2-yl)-1H-pyrrolo[2,3-b]pyridine (39 mg, 151.6 mmol), chloro (2-dicyclohexylphosphino-2',6'-dimethoxy-1,1'-biphenylyl)(2'-amino-1,1'-biphenyl-2-yl) palladium (II) (9 mg, 12.59 µmol) and potassium carbonate (52.12 mg, 377.7 µmol) were added. The system was heated to 100°C for 2 h. Hydrochloric acid-methanol solution was added and stirred at room temperature for 1 h. The reaction was monitored by LCMS for completion. The reaction mixture was adjusted to pH 7 with sodium bicarbonate solution, treated with dichloromethane/water, dried over anhydrous sodium sulfate, and concentrated. The crude product was purified by a preparative thin layer chromatographic column (dichloromethane/methanol = 10/1) to give a product tert-butyl (S)-2-(2-pivaloyl-7-(3-methyl-1H-pyrrolo [2,3-b]pyridin-5-yl)-1,2,3,4-tetrahydroisoquinol-5-yl)pyrrolidine-1-carboxylate (40 mg, yield: 62%). ES-API:[M+H]⁺=517.3.

Step 3: The tert-butyl (S)-2-(2-pivaloyl-7-(3-methyl-1H-pyrrolo [2,3-b]pyridin-5-yl)-1,2,3,4-tetrahydroisoquinol-5-yl)pyrrolidine-1-carboxylate (40 mg, 77.52 µmol) was dissolved in dichloromethane (1 mL). Trifluoroacetic acid (1 mL) was added. The system reacted at room temperature for 1 h. Upon completion of the reaction monitored by LCMS, the reaction mixture was spun to dryness. The crude product was purified by a preparative thin layer chromatographic column (dichloromethane/methanol = 10/1) to give a product (S)-2,2-dimethyl-1-(7-(3-methyl-1H-pyrrolo[2,3-b]pyridin-5-yl)-5-(pyrrolidin-2-yl)-3,4-di hydroisoquinolin-2(1H)-yl)propan-1-one (Z300, 6.63 mg, yield: 20.6%). ES-API:[M+1]⁺=417.2.

### Example 91. Synthesis of compound Z301

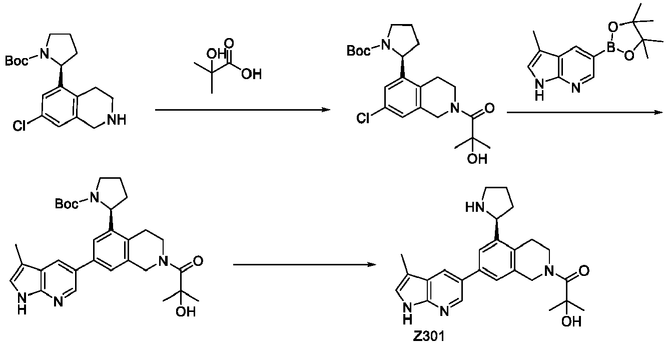

Step 1: Triethylamine (90.12 mg, 890.59 µmol), 1-(3-dimethylaminopropyl)-3-ethylcarbodiimide hydrochloride (113 mg, 593.7 µmol) and 1-hydroxybenzotriazole (80.22 mg, 593.7 µmol) were added to a solution of tert-butyl (S)-2-(7-chloro-1,2,3,4-tetrahydroisoquinolin-5-yl)pyrrolidine-1-carboxylate (100 mg, 286.86 µmol) and 2-hydroxy-2-methylpropanoic acid (37.29 mg, 356.24 µmol) in N,N-dimethylformamide (3 mL) and stirred at 25°C for 1 h. The reaction was monitored by LCMS for completion, and quenched by the addition of water. The reaction mixture was extracted with ethyl acetate, dried over anhydrous sodium sulfate, and concentrated. The crude product was purified by column chromatography (methanol/dichloromethane = 0-5%) to give a product tert-butyl (S)-2-(7-chloro-2-(2-hydroxy-2-methylpropanoyl)-1,2,3,4-tetrahydroisoquinolin-5-yl)pyrr olidine-1-carboxylate (100 mg, yield: 82.4%). ES-API:[M+H-56]⁺=367.2.

Step 2: The compound tert-butyl (S)-2-(7-chloro-2-(2-hydroxy-2-methylpropanoyl)-1,2,3,4-tetrahydroisoquinolin-5-yl)pyrr olidine-1-carboxylate (100 mg, 236.44 µmol) was dissolved in dioxane/water (2 mL/0.4 mL). 3-Methyl-5-(4,4,5,5-tetramethyl-1,3,2-dioxaborolan-2-yl)-1H-pyrrolo[2,3-b]pyridine (74 mg, 286 mmol), chloro (2-dicyclohexylphosphino-2',6'-dimethoxy-1,1'-biphenylyl)(2'-amino-1,1'-biphenyl-2-yl) palladium (II) (18 mg, 24 µmol) and potassium carbonate (98 mg, 712 µmol) were added. The system was heated to 100°C for 2 h. Hydrochloric acid/methanol solution was added and stirred at room temperature for 1 h. The reaction was monitored by LCMS for completion. The reaction mixture was adjusted to pH 7 with sodium bicarbonate solution, treated with dichloromethane/water, dried over anhydrous sodium sulfate, and concentrated. The crude product was purified by column chromatography (tetrahydrofuran/petroleum ether = 30/70) to give a product tert-butyl (S)-2-(2-(1-hydroxycyclopropanecarbonyl)-7-(3-methyl-1H-pyrrolo[2,3-b]pyridin-5-yl)-1, 2,3,4-tetrahydroisoquinolin-5-yl)pyrrolidine-1-carboxylate (100 mg, yield: 81.4%). ES-API:[M+H]⁺=519.3.

Step 3: The tert-butyl (S)-2-(2-(1-hydroxycyclopropanecarbonyl)-7-(3-methyl-1H-pyrrolo[2,3-b]pyridin-5-yl)-1, 2,3,4-tetrahydroisoquinolin-5-yl)pyrrolidine-1-carboxylate (100 mg, 192.81 µmol) was dissolved in dichloromethane (1 mL). Trifluoroacetic acid (1 mL) was added. The system reacted at room temperature for 1 h. Upon completion of the reaction monitored by LCMS, the reaction mixture was spun to dryness. The crude product was purified by a preparative thin layer chromatographic column (dichloromethane/methanol = 10/1) to give a product (S)-2-hydroxy-2-methyl-1-(7-(3-methyl-1H-pyrrolo[2,3-b]pyridin-5-yl)-5-(pyrrolidin-2-yl )-3,4-dihydroisoquinolin-2(1H)-yl)propan-1-one (Z301, 20 mg, yield: 25%). ES-API:[M+1]⁺=419.3. ¹H NMR (400 MHz, CDCl₃) δ 10.61-9.97 (m, 1H), 8.34-7.98 (m, 1H), 7.89-7.51 (m, 2H), 6.97 (s, 1H), 6.88 (s, 1H), 5.20-4.05 (m, 4H), 3.75-3.59 (m, 2H), 3.48-3.36 (m, 1H), 2.85-2.38 (m, 2H), 2.24 (s, 5H), 2.14-1.97 (m, 2H), 1.53 (s, 6H).

### Example 92. Synthesis of compound Z302

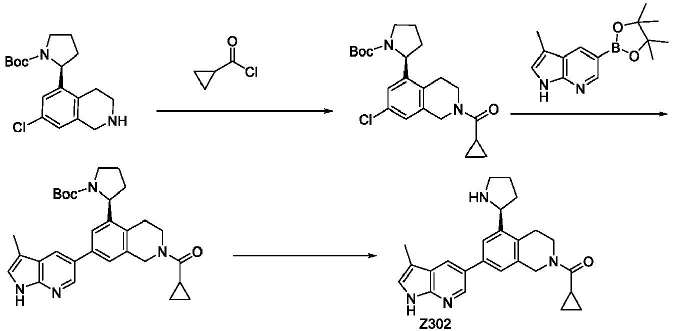

Step 1: Triethylamine (90.12 mg, 890.59 µmol) and cyclopropylcarbonyl chloride (37.24 mg, 356 µmol) were added to a solution of tert-butyl (S)-2-(7-chloro-1,2,3,4-tetrahydroisoquinolin-5-yl)pyrrolidine-1-carboxylate (100 mg, 296.86 µmol) in dichloromethane (1 mL) and stirred at 0°C for 15 min. The reaction was monitored by LCMS for completion, and quenched by the addition of water. The reaction mixture was extracted with dichloromethane, dried over anhydrous sodium sulfate, and concentrated. The crude product was purified by column chromatography (methanol/dichloromethane = 0-5%) to give a product tert-butyl (S)-2-(7-chloro-2-(cyclopropanecarbonyl)-1,2,3,4-tetrahydroisoquinolin-5-yl)pyrrolidine-1-carboxylate (100 mg, yield: 83.6%). ES-API:[M+H-56]⁺=349.1.

Step 2: The compound tert-butyl (S)-2-(7-chloro-2-(cyclopropanecarbonyl)-1,2,3,4-tetrahydroisoquinolin-5-yl)pyrrolidine-1-carboxylate (100 mg, 247.5 µmol) was dissolved in dioxane/water (2 mL/0.4 mL). 3-Methyl-5-(4,4,5,5-tetramethyl-1,3,2-dioxaborolan-2-yl)-1H-pyrrolo[2,3-b]pyridine (91.79 mg, 355.6 mmol), chloro (2-dicyclohexylphosphino-2',6'-dimethoxy-1,1'-biphenylyl)(2'-amino-1,1'-biphenyl-2-yl) palladium (II) (21 mg, 29 µmol) and potassium carbonate (122.7 mg, 889 µmol) were added. The system was heated to 100°C for 2 h. Hydrochloric acid-methanol solution was added and stirred at room temperature for 1 h. The reaction was monitored by LCMS for completion. The reaction mixture was adjusted to pH 7 with sodium bicarbonate solution, treated with dichloromethane/water, dried over anhydrous sodium sulfate, and concentrated. The crude product was purified by a preparative thin layer chromatographic column (dichloromethane/methanol = 10/1) to give a product tert-butyl (S)-2-(2-(cyclopropanecarbonyl)-7-(3-methyl-1H-pyrrolo[2,3-b]pyridin-5-yl)-1,2,3,4-tetra hydroisoquinolin-5-yl)pyrrolidine-1-carboxylate (100 mg, yield: 80.8%). ES-API:[M+H]⁺=501.3.

Step 3: The tert-butyl (S)-2-(2-(cyclopropanecarbonyl)-7-(3-methyl-1H-pyrrolo[2,3-b]pyridin-5-yl)-1,2,3,4-tetra hydroisoquinolin-5-yl)pyrrolidine-1-carboxylate (100 mg, 200 µmol) was dissolved in dichloromethane (1 mL). Trifluoroacetic acid (1 mL) was added. The system reacted at room temperature for 1 h. Upon completion of the reaction, the reaction mixture was spun to dryness. The crude product was purified by a preparative thin layer chromatographic column (dichloromethane/methanol = 10/1) to give a product (S)-cyclopropyl (7-(3-methyl-1H-pyrrolo[2,3-b]pyridin-5-yl)-5-(pyrrolidin-2-yl)-3,4-dihydroisoquinolin-2( 1H)-yl)methanone (Z302, 30 mg, yield: 37.5%). ES-API:[M+1]⁺=401.2. ¹H NMR (400 MHz, CDCl₃) δ 10.84-10.47 (m, 1H), 8.55-8.41 (m, 1H), 8.14-7.64 (m, 2H), 7.07-6.86 (m, 2H), 5.15-4.69 (m, 2H), 4.59-4.44 (m, 1H), 3.95-3.71 (m, 2H), 3.63-3.25 (m, 3H), 3.00-2.67 (m, 1H), 2.31-1.95 (m, 7H), 1.79 (s, 1H), 1.09-0.94 (m, 2H), 0.84-0.75 (m, 2H).

### Example 93. Synthesis of compound Z303

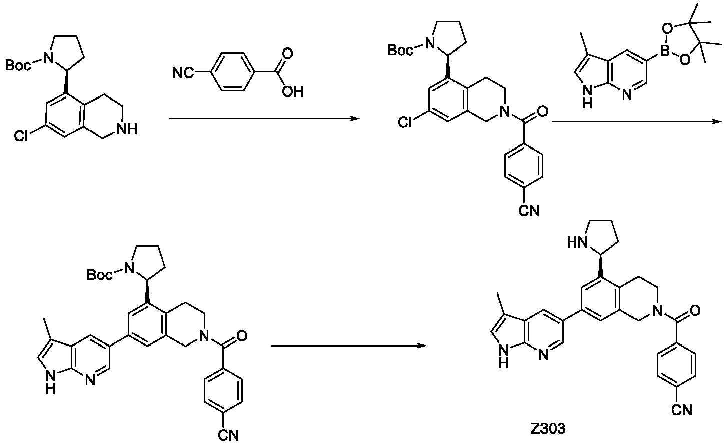

Step 1: 4-Cyanobenzoic acid (52.41 mg, 356.24 µmol), 1-propylphosphonic anhydride (283.21 mg, 890.59 µmol) and triethylamine (89.95 mg, 890.59 µmol) were added to a solution of tert-butyl (S)-2-(7-chloro-1,2,3,4-tetrahydroisoquinolin-5-yl)pyrrolidine-1-carboxylate (100 mg, 286.86 µmol) in dichloromethane (1 mL) and stirred at 25°C for 2 h. The reaction was monitored by LCMS for completion, and quenched by the addition of water. The reaction mixture was extracted with ethyl acetate, washed with saturated brine, dried over anhydrous sodium sulfate, and concentrated. The crude product was purified by column chromatography (0-6% methanol/dichloromethane) to give a product tert-butyl (S)-2-(7-chloro-2-(4-cyanobenzoyl)-1,2,3,4-tetrahydroisoquinolin-5-yl)pyrrolidine-1-carb oxylate (70 mg, yield: 52.6%). ES-API:[M+H-56]⁺=410.2.

Step 2: The compound tert-butyl (S)-2-(7-chloro-2-(4-cyanobenzoyl)-1,2,3,4-tetrahydroisoquinolin-5-yl)pyrrolidine-1-carb oxylate (70 mg, 151.17 µmol) was dissolved in dioxane/water (1 mL/0.2 mL). 3-Methyl-5-(4,4,5,5-tetramethyl-1,3,2-dioxaborolan-2-yl)-1H-pyrrolo[2,3-b]pyridine (73.58 mg, 285.05 µmol), chloro (2-dicyclohexylphosphino-2',6'-dimethoxy-1,1'-biphenylyl)(2'-amino-1,1'-biphenyl-2-yl) palladium (II) (17.09 mg, 23.75 µmol) and potassium carbonate (98.34 mg, 712.64 µmol) were added. The system was heated to 100°C for 2 h. Hydrochloric acid-methanol solution was added and stirred at room temperature for 1 h. The reaction was monitored by LCMS for completion. The reaction mixture was adjusted to pH 7 with sodium bicarbonate solution, treated with dichloromethane/water, dried over anhydrous sodium sulfate, and concentrated. The crude product was purified by column chromatography (methanol/dichloromethane = 0-6%) to give a product tert-butyl (S)-2-(2-(4-cyanobenzoyl)-7-(3-methyl-1H-pyrrolo[2,3-b]pyridin-5-yl)-1,2,3,4-tetrahydroi soquinolin-5-yl)pyrrolidine-1-carboxylate (68 mg, yield: 76.5%). ES-API:[M+H]⁺=562.3.

Step 3: The tert-butyl (S)-2-(2-(4-cyanobenzoyl)-7-(3-methyl-1H-pyrrolo[2,3-b]pyridin-5-yl)-1,2,3,4-tetrahydroi soquinolin-5-yl)pyrrolidine-1-carboxylate (68 mg, 115.6 µmol) was dissolved in dichloromethane (1 mL). Trifluoroacetic acid (1 mL) was added, and the system reacted at room temperature for 1 h. Upon completion of the reaction monitored by LCMS, the reaction mixture was spun to dryness. The crude product was purified by a preparative thin layer chromatographic column (dichloromethane/methanol = 10/1) to give a product (S)-4-(7-(3-methyl-1H-pyrrolo[2,3-b]pyridin-5-yl)-5-(pyrrolidin-2-yl)-1,2,3,4-tetrahydrois oquinoline-2-carbonyl)benzonitrile (Z303, 17 mg, yield: 31.8%). ES-API:[M+1]⁺=462.2. ¹H NMR (400 MHz, CDCl₃) δ 10.82 (s, 1H), 8.63-8.2 (m, 1H), 7.87-7.61 (m, 4H), 7.33-7.25 (m, 2H), 7.20-6.70 (m, 2H), 5.55-3.75 (m, 3H), 3.72-3.19 (m, 4H), 2.86-2.66 (m, 1H), 2.47-1.91 (m, 8H).

### Example 94. Synthesis of compound Z304

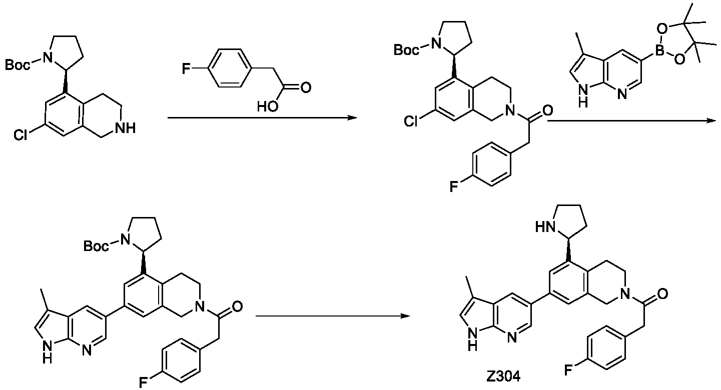

Step 1: 2-(4-Fluorophenyl)acetic acid (54.91 mg, 356.24 µmol), 1-propylphosphonic anhydride (283.21 mg, 890.59 µmol) and triethylamine (89.95 mg, 890.59 µmol) were added to a solution of tert-butyl (S)-2-(7-chloro-1,2,3,4-tetrahydroisoquinolin-5-yl)pyrrolidine-1-carboxylate (100 mg, 286.86 µmol) in dichloromethane (1 ml) and stirred at 25°C for 2 h. The reaction was monitored by LCMS for completion, and quenched by the addition of water. The reaction mixture was extracted with ethyl acetate, washed with saturated brine, dried over anhydrous sodium sulfate, and concentrated. The crude product was purified by column chromatography (methanol/dichloromethane = 0-6%) to give a product tert-butyl (S)-2-(7-chloro-2-(2-(4-fluorophenyl)acetyl)-1,2,3,4-tetrahydroisoquinolin-5-yl)pyrrolidin e-1-carboxylate (80 mg, yield: 60%). ES-API:[M+H-56]⁺=417.1.

Step 2: The compound tert-butyl (S)-2-(7-chloro-2-(2-(4-fluorophenyl)acetyl)-1,2,3,4-tetrahydroisoquinolin-5-yl)pyrrolidin e-1-carboxylate (80 mg, 169.14 µmol) was dissolved in dioxane/water (1 mL/0.2 mL). 3-Methyl-5-(4,4,5,5-tetramethyl-1,3,2-dioxaborolan-2-yl)-1H-pyrrolo[2,3-b]pyridine (52.39 mg, 202.97 µmol), chloro (2-dicyclohexylphosphino-2',6'-dimethoxy-1,1'-biphenylyl)(2'-amino-1,1'-biphenyl-2-yl) palladium (II) (12.17 mg, 16.93 µmol) and potassium carbonate (70.02 mg, 507.42 µmol) were added. The system was heated to 100°C for 2 h. Hydrochloric acid-methanol solution was added and stirred at room temperature for 1 h. The reaction was monitored by LCMS for completion. The reaction mixture was adjusted to pH 7 with sodium bicarbonate solution, treated with dichloromethane/water, dried over anhydrous sodium sulfate, and concentrated. The crude product was purified by column chromatography (methanol/dichloromethane = 0-6%) to give a product tert-butyl (S)-2-(2-(2-(4-fluorophenyl)acetyl)-7-(3-methyl-1H-pyrrolo[2,3-b]pyridin-5-yl)-1,2,3,4-te trahydroisoquinolin-5-yl)pyrrolidine-1-carboxylate (78 mg, yield: 81%). ES-API:[M+H]⁺=569.4.

Step 3: The tert-butyl (S)-2-(2-(2-(4-fluorophenyl)acetyl)-7-(3-methyl-1H-pyrrolo[2,3-b]pyridin-5-yl)-1,2,3,4-te trahydroisoquinolin-5-yl)pyrrolidine-1-carboxylate (78 mg, 137.3 µmol) was dissolved in dichloromethane (1 mL). Trifluoroacetic acid (1 mL) was added. The system reacted at room temperature for 1 h. Upon completion of the reaction, the reaction mixture was spun to dryness. The crude product was purified by a preparative thin layer chromatographic column (dichloromethane/methanol = 10/1) to give a product (S)-2-(4-fluorophenyl)-1-(7-(3-methyl-1H-pyrrolo[2,3-b]pyridin-5-yl)-5-(pyrrolidin-2-yl)-3,4-dihydroisoquinolin-2(1H)-yl)ethan-1-one (Z304, 38.92 mg, yield: 60%). ES-API:[M+1]⁺=469.3. ¹H NMR (400 MHz, CDCl₃) δ 10.78-10.09 (m, 1H), 8.55-8.31 (m, 1H), 8.12-7.64 (m, 2H), 7.32-7.25 (m, 2H), 7.18-7.00 (m, 3H), 6.96-6.84 (m, 1H), 5.06-4.81 (m, 1H), 4.81-4.30 (m, 2H), 4.01-3.54 (m, 4H), 3.51-2.96 (m, 3H), 2.76-2.61 (m, 1H), 2.37-2.06 (m, 5H), 2.04-1.76 (m, 2H).

### Example 95. Synthesis of compound Z305

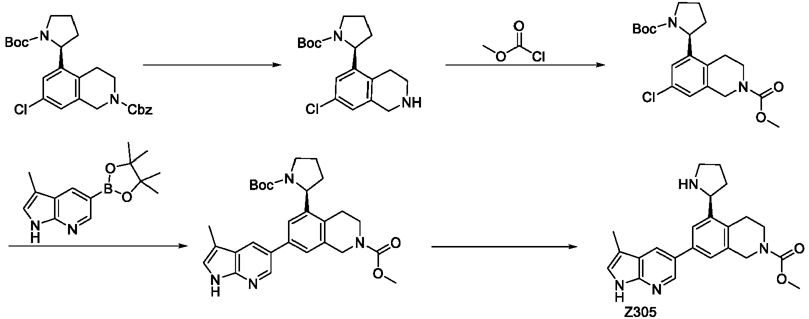

Step 1: Palladium chloride (241 mg, 1.4 mmol), triethylamine (1.03 g, 10.9 mmol) and triethylsilane (3.95 g, 34 mmol) were added to a solution of benzyl (S)-benzyl-(5-(1-(tert-butoxycarbonyl)pyrrolidin-2-yl)-7-chloro-3,4-dihydroisoquinoline-2 (1H)-carboxylate (1.6 g, 3.4 mmol) in dichloromethane (20 ml) and stirred at room temperature for 30 min. The reaction was monitored by LCMS for completion, and quenched by the addition of water. The reaction mixture was extracted with dichloromethane, dried over anhydrous sodium sulfate, and concentrated. The crude product was purified by column chromatography (methanol/dichloromethane = 0-5%) to give a product tert-butyl (S)-2-(7-chloro-1,2,3,4-tetrahydroisoquinolin-5-yl)pyrrolidine-1-carboxylate (700 mg, yield: 61.7%). ES-API:[M+H]⁺=337.1.

Step 2: Triethylamine (90.12 mg, 890.59 µmol) and methylcarbonyl chloride (37.24 mg, 356 µmol) were added to a solution of tert-butyl (S)-2-(7-chloro-1,2,3,4-tetrahydroisoquinolin-5-yl)pyrrolidine-1-carboxylate (100 mg, 296.86 µmol) in dichloromethane (1 mL) and stirred at 0°C for 15 min. The reaction was monitored by LCMS for completion, and quenched by the addition of water. The reaction mixture was extracted with dichloromethane, dried over anhydrous sodium sulfate, and concentrated. The crude product was purified by column chromatography (methanol/dichloromethane = 0-5%) to give a product (S)-methyl 5-(1-(tert-butoxycarbonyl)pyrrolidin-2-yl)-7-chloro-3,4-dihydroisoquinoline-2(1H)-carbox ylate (100 mg, yield: 83.6%). ES-API:[M+H-100]⁺=295.1.

Step 3: The compound (S)-methyl 5-(1-(tert-butoxycarbonyl)pyrrolidin-2-yl)-7-chloro-3,4-dihydroisoquinoline-2(1H)-carbox ylate (100 mg, 247.5 µmol) was dissolved in dioxane/water (2 mL/0.4 mL). 3-Methyl-5-(4,4,5,5-tetramethyl-1,3,2-dioxaborolan-2-yl)-1H-pyrrolo[2,3-b]pyridine (91.79 mg, 355.6 mmol), chloro (2-dicyclohexylphosphino-2',6'-dimethoxy-1,1'-biphenylyl)(2'-amino-1,1'-biphenyl-2-yl) palladium (II) (21 mg, 29 µmol) and potassium carbonate (122.7 mg, 889 µmol) were added. The system was heated to 100°C for 2 h. Hydrochloric acid-methanol solution was added and stirred at room temperature for 1 h. The reaction was monitored by LCMS for completion. The reaction mixture was adjusted to pH 7 with sodium bicarbonate solution, treated with dichloromethane/water, dried over anhydrous sodium sulfate, and concentrated. The crude product was prepared and purified (column: Waters XBridge C18, 190*250 mm, 5 µm; mobile phase: A: 0.1% aqueous ammonium bicarbonate solution; mobile phase B: acetonitrile; flow rate: 15 ml/min; column temperature: room temperature) to give a product (S)-methyl 5-(1-(tert-butoxycarbonyl)pyrrolidin-2-yl)-7-(3-methyl-1H-pyrrolo[2,3-b]pyridin-5-yl)-3,4 -dihydroisoquinoline-2(1H)-carboxylate (100 mg, yield: 80.8%). ES-API:[M+H]⁺=491.2.

Step 4: The (S)-methyl 5-(1-(tert-butoxycarbonyl)pyrrolidin-2-yl)-7-(3-methyl-1H-pyrrolo[2,3-b]pyridin-5-yl)-3,4 -dihydroisoquinoline-2(1H)-carboxylate (100 mg, 200 µmol) was dissolved in dichloromethane (1 mL). Trifluoroacetic acid (1 mL) was added. The system reacted at room temperature for 1 h. Upon completion of the reaction, the reaction mixture was spun to dryness. The crude product was purified by a preparative thin layer chromatographic column (dichloromethane/methanol = 10/1) to give a product methyl (S)-7-(3-methyl-1H-pyrrolo[2,3-b]pyridin-5-yl)-5-(pyrrolidin-2-yl)-3,4-dihydroisoquinoli ne-2(1H)-carboxylate (Z305, 30 mg, yield: 37.5%). ES-API:[M+1]⁺=391.2. ¹H NMR (400 MHz, CDCl₃) δ 10.74-10.40 (m, 1H), 8.60-8.43 (m, 1H), 8.09-7.64 (m, 2H), 7.00-6.81 (m, 2H), 5.07-4.80 (m, 1H), 4.65-4.11 (m, 2H), 3.72 (s, 3H), 3.69 -3.34 (m, 4H), 3.25-2.85 (m, 1H), 2.64 (d, *J* = 15.7 Hz, 1H), 2.31-1.98 (m, 7H).

### Example 96. Synthesis of compound Z306

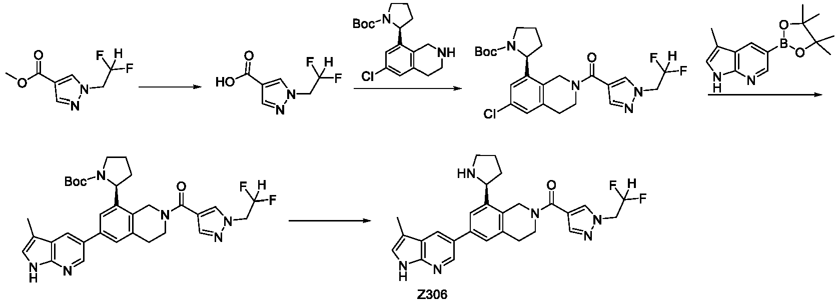

Step 1: Lithium hydroxide hydrate (275.84 mg, 6.57 mmol) was added to a solution of methyl 1-(2,2-difluoroethyl)pyrazole-4-carboxylate (250 mg, 1.31 mmol) in tetrahydrofuran (1 mL), and the mixture was stirred at 25°C for 1 h. The reaction was monitored by LCMS for completion. The reaction mixture was extracted with ethyl acetate/water. The aqueous layer was adjusted to pH 1, extracted with ethyl acetate/water, dried over anhydrous sodium sulfate, and concentrated to give a product 1-(2,2-difluoroethyl)pyrazole-4-carboxylic acid (140.00 mg, crude product) as a yellow solid. ES-API:[M+H]⁺=177.1.

Step 2: 1-Propylphosphonic anhydride (141.68 mg, 445.30 µmol), 1-(2,2-difluoroethyl)pyrazole-4-carboxylic acid (62.74 mg, 0.36 mmol) and triethylamine (90.12 mg, 0.89 mmol) were added to a solution of tert-butyl (S)-2-(6-chloro-1,2,3,4-tetrahydroisoquinolin-8-yl)pyrrolidine-1-carboxylate (100 mg, 296.86 µmol) in dichloromethane (2 mL), and the mixture was stirred at 25°C for 16 h. The reaction was monitored by LCMS for completion. The reaction mixture was extracted with dichloromethane/water, washed with saturated aqueous sodium chloride solution, dried over anhydrous sodium sulfate, and concentrated. The crude product was purified by column chromatography (methanol/dichloromethane = 0-6%) to give a product tert-butyl (S)-2-[6-chloro-2-[1-(2,2-difluoroethyl)pyrazole-4-carbonyl]-3,4-dihydro-1H-isoquinolin-8-yl]pyrrolidine-1-carboxylate (78.00 mg, yield: 53.08%) as a yellow oil. ES-API:[M+H-100]⁺=395.2.

Step 3: The tert-butyl (S)-2-[6-chloro-2-[1-(2,2-difluoroethyl)pyrazole-4-carbonyl]-3,4-dihydro-1H-isoquinolin-8-yl]pyrrolidine-1-carboxylate (78 mg, 0.157 mmol), 3-methyl-5-(4,4,5,5-tetramethyl-1,3,2-dioxaborolan-2-yl)-1H-pyrrolo[2,3-b]pyridine (61.02 mg, 0.236 mmol), chloro (2-dicyclohexylphosphino-2',6'-dimethoxy-1,1'-biphenylyl)(2'-amino-1,1'-biphenyl-2-yl) palladium (II) (22.68 mg, 0.031 mmol) and potassium carbonate (65.34 mg, 0.472 mmol) were dissolved in 1,4-dioxane solution (1 mL) and water (0.1 mL), and the system was warmed to 100°C for 2 h. Upon completion of the reaction monitored by LCMS, the reaction mixture was quenched by the addition of water, and extracted with ethyl acetate. The organic phase was washed with saturated brine, dried over anhydrous sodium sulfate, and concentrated. The crude product was purified by thin layer chromatographic column (dichloromethane/methanol = 10/1) to give a product tert-butyl (S)-2-[2-[1-(2,2-difluoroethyl)pyrazole-4-carbonyl]-6-(3-methyl-1H-pyrrolo[2,3-b]pyridin -5-yl)-3,4-dihydro-1H-isoquinolin-8-yl]pyrrolidine-1-carboxylate (90 mg, yield: 96.69%). ES-API: [M+H]⁺ = 591.6.

Step 4: The tert-butyl (S)-2-[2-[1-(2,2-difluoroethyl)pyrazole-4-carbonyl]-6-(3-methyl-1H-pyrrolo[2,3-b]pyridin -5-yl)-3,4-dihydro-1H-isoquinolin-8-yl]pyrrolidine-1-carboxylate (90 mg, 0.152 mmol) was dissolved in dichloromethane (2 mL) and trifluoroacetic acid solution (2 mL) and stirred at room temperature for 1 h. The reaction mixture was spun to dryness. The crude product was purified by a preparative thin layer chromatographic column (dichloromethane/methanol = 10/1) to give a product (S)-[1-(2,2-difluoroethyl)pyrazol-4-yl]-[6-(3-methyl-1H-pyrrolo[2,3-b]pyridin-5-yl)-8-[py rrolidin-2-yl]-3,4-dihydro-1H-isoquinolin-2-yl]methanone (Z306, 10.9 mg, yield: 14.58%). ES-API: [M+H]⁺ = 491.5.

### Example 97. Synthesis of compound Z307

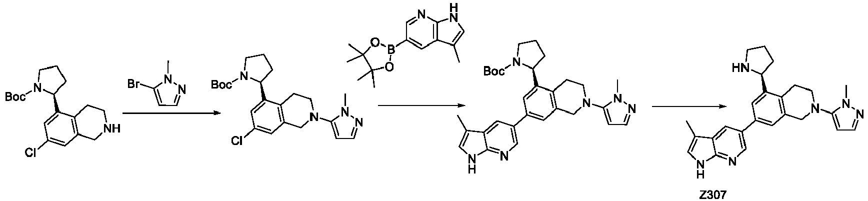

Step 1: tert-butyl (S)-2-(7-chloro-1,2,3,4-tetrahydroisoquinolin-5-yl)pyrrolidine-1-carboxylate (100 mg, 0.297 mmol), 5-bromo-1-methyl-1H-pyrazole (47.8 mg, 0.297 mmol), tris(dibenzylideneacetone)dipalladium (54.4 mg, 0.059 mmol), 1,1'-binaphthyl-2,2'-bis(diphenyl) (81.4 mg, 0.13 mmol), sodium tert-butoxide (85.5 mg, 0.89 mmol) and tert-butanol (3 mL) were added to a 10 mL microwave reactor. The system was replaced with nitrogen three times and then protected with a nitrogen balloon. The system reacted under microwave at 110°C for 50 min. Ethyl acetate (10 mL) was added to the reaction mixture. The obtained mixture was washed with saturated brine (10 mLX3), dried over anhydrous sodium sulfate, and concentrated. The crude product was purified by silica gel column chromatography (ethyl acetate:petroleum ether = 10:100) to give tert-butyl (S)-2-(7-chloro-2-(1-methyl-1H-pyrazol-5-yl)-1,2,3,4-tetrahydroisoquinolin-5-yl)pyrrolidi ne-1-carboxylate (15 mg, yield: 12%). ES-API: [M+H]⁺=417.2.

Step 2: The tert-butyl (S)-2-(7-chloro-2-(1-methyl-1H-pyrazol-5-yl)-1,2,3,4-tetrahydroisoquinolin-5-yl)pyrrolidi ne-1-carboxylate (15 mg, 0.036 mmol), 3-methyl-5-(4,4,5,5-tetramethyl-1,3,2-dioxaborolan-2-yl)-1H-pyrrolo[2,3-b]pyridine (19 mg, 0.072 mmol), chloro (2-dicyclohexylphosphino-2',6'-dimethoxy-1,1'-biphenylyl)(2'-amino-1,1'-biphenyl-2-yl) palladium (II) (2.59 mg, 0.004 mmol), potassium carbonate (14.9 mg, 0.108 mmol), dioxane (2 mL) and water (0.3 mL) were added to a 10 mL microwave reactor. The system was replaced with nitrogen three times and then protected with a nitrogen balloon. The system reacted under microwave at 110°C for 50 min. Ethyl acetate (10 mL) was added to the reaction mixture. The obtained mixture was washed with saturated brine (5 mLX3), dried over anhydrous sodium sulfate, and concentrated. The crude product was purified by silica gel column chromatography (methanol:dichloromethane = 10:100) to give a desired product tert-butyl (S)-2-(2-(1-methyl-1H-pyrazol-5-yl)-7-(3-methyl-1H-pyrrolo[2,3-b]pyridin-5-yl)-1,2,3,4-t etrahydroisoquinolin-5-yl)pyrrolidine-1-carboxylate (8 mg, yield: 43%). ES-API: [M+H]⁺=513.2.

Step 3: The tert-butyl (S)-2-(2-(1-methyl-1H-pyrazol-5-yl)-7-(3-methyl-1H-pyrrolo[2,3-b]pyridin-5-yl)-1,2,3,4-t etrahydroisoquinolin-5-yl)pyrrolidine-1-carboxylate (8 mg 0.016 mmol), trifluoroacetic acid (3 mL) and dichloromethane (6 mL) were added to a 25 mL single-neck round-bottom flask and stirred at room temperature for 30 min. The reaction mixture was spun to dryness. The crude product was purified by prep-HPLC (formic acid method) to give (S)-2-(1-methyl-1H-pyrazol-5-yl)-7-(3-methyl-1H-pyrrolo[2,3-b]pyridin-5-yl)-5-(pyrrolidi n-2-yl)-1,2,3,4-tetrahydroisoquinoline (Z307, formate, 1.1 mg, yield: 17.0%). ES-API: [M+H]⁺=413.3.

### Example 98. Synthesis of compound Z308

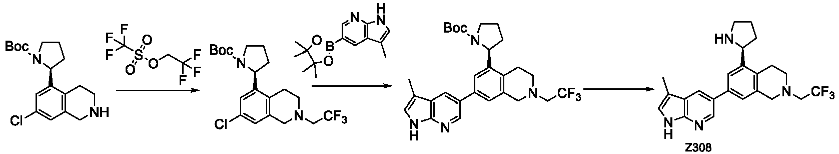

Step 1: Tert-butyl (S)-2-(7-chloro-1,2,3,4-tetrahydroisoquinolin-5-yl)pyrrolidine-1-carboxylate (100 mg, 0.297 mmol), 2,2,2-trifluoroethyl triflate (103 mg, 0.445 mmol), triethylamine (90 mg, 0.891 mmol) and N,N-dimethylformamide (10 ml) were added to a 50 mL single-neck round-bottom flask and stirred at room temperature overnight. Ethyl acetate (30 mL) was added to the reaction mixture. The obtained mixture was washed with saturated brine (30 mLX3), dried over anhydrous sodium sulfate, and concentrated. The crude product was purified by silica gel column chromatography (ethyl acetate:petroleum ether = 10:100) to give a desired product tert-butyl (S)-2-(7-chloro-2-(2,2,2-trifluoroethyl)-1,2,3,4-tetrahydroisoquinolin-5-yl)pyrrolidine-1-c arboxylate (30 mg, yield: 24%). ES-API: [M+H]⁺=419.2.

Step 2: The tert-butyl (S)-2-(7-chloro-2-(2,2,2-trifluoroethyl)-1,2,3,4-tetrahydroisoquinolin-5-yl)pyrrolidine-1-c arboxylate (30 mg, 0.072 mmol), 3-methyl-5-(4,4,5,5-tetramethyl-1,3,2-dioxaborolan-2-yl)-1H-pyrrolo[2,3-b]pyridine (37 mg, 0.143 mmol), chloro (2-dicyclohexylphosphino-2',6'-dimethoxy-1,1'-biphenylyl)(2'-amino-1,1'-biphenyl-2-yl) palladium (II) (5.16 mg, 0.007 mmol), potassium carbonate (29.7 mg, 0.215 mmol), dioxane (2 mL) and water (0.3 mL) were added to a 10 mL microwave reactor. The system was replaced with nitrogen three times and then protected with a nitrogen balloon. The system reacted under microwave at 110°C for 50 min. Ethyl acetate (10 mL) was added to the reaction mixture. The obtained mixture was washed with saturated brine (5 mLX3), dried over anhydrous sodium sulfate, and concentrated. The crude product was purified by silica gel column chromatography (methanol:dichloromethane = 10:100) to give a desired product tert-butyl (S)-2-(7-(3-methyl-1H-pyrrolo[2,3-b]pyridin-5-yl)-2-(2,2,2-trifluoroethyl)-1,2,3,4-tetrahy droisoquinolin-5-yl)pyrrolidine-1-carboxylate (20 mg, yield: 54%). ES-API: [M+H]⁺=515.3.

Step 3: The tert-butyl (S)-2-(7-(3-methyl-1H-pyrrolo[2,3-b]pyridin-5-yl)-2-(2,2,2-trifluoroethyl)-1,2,3,4-tetrahy droisoquinolin-5-yl)pyrrolidine-1-carboxylate (20 mg, 0.035 mmol), trifluoroacetic acid (3 mL) and dichloromethane (6 mL) were added to a 25 mL single-neck round-bottom flask and stirred at room temperature for 30 min. The reaction mixture was spun to dryness. The crude product was purified by prep-HPLC (formic acid method) to give (S)-7-(3-methyl-1H-pyrrolo[2,3-b]pyridin-5-yl)-5-(pyrrolidin-2-yl)-2-(2,2,2-trifluoroethyl) -1,2,3,4-tetrahydroisoquinoline (Z308, formate, 7 mg, yield: 48.1%). ES-API: [M+H]⁺=415.3.

### Example 99. Synthesis of compound Z309-1 and compound Z309-2

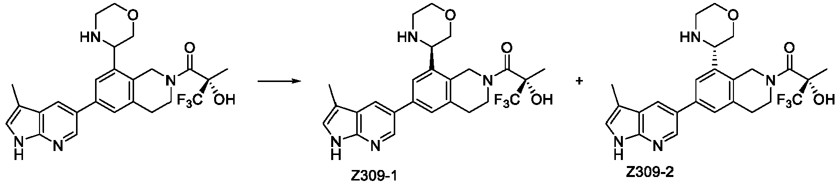

Step 1: Compound (2S)-3,3,3-trifluoro-2-hydroxy-2-methyl-1-(6-(3-methyl-1*H*-pyrrolo[2,3-*b*]pyridin-5-yl)-8-(morpholin-3-yl)-3,4-dihydroisoquinolin-2(1*H*)-yl)propan-1-one (50 mg, 0.102 mmol) was chirally resolved (column type: Chiralpak AB: 5 µm, 4.6*250 mm, mobile phase: n-hexane:ethanol:diethylamine = 40:60:0.2, flow rate: 1 mL/min, column temperature: room temperature) to give two isomeric compounds. A structure of one isomeric compound was arbitrarily assigned as (S)-3,3,3-trifluoro-2-hydroxy-2-methyl-1-(6-(3-methyl-1*H*-pyrrolo[2,3-*b*]pyridin-5-yl)-8-( (R)-morpholin-3-yl)-3,4-dihydroisoquinolin-2(1*H*)-yl)propan-1-one (Z309-1, 19 mg, yield: 38%, retention time: 5.656 min, purity: 100%, ee value: 100%). ES-API: [M+H]⁺ = 489.3. ¹H NMR (400 MHz, DMSO-*d*₆) δ 11.34 (s, 1H), 8.46 (d, *J* = 2.4 Hz, 1H), 8.10 (d, *J* = 2.0 Hz, 1H), 7.79 (d, *J* = 1.6 Hz, 1H), 7.46 (s, 1H), 7.40 - 7.14 (m, 2H), 5.42 - 5.16 (m, 1H), 4.99 - 4.59 (m, 1H), 4.31 - 4.02 (m, 1H), 3.97 (d, *J* = 8.8 Hz, 1H), 3.92 - 3.65 (m, 3H), 3.60 - 3.50 (m, 1H), 3.28 - 3.21 (m, 1H), 3.08 - 2.82 (m, 4H), 2.31 (s, 3H), 1.59 (s, 3H). A structure of the other isomeric compound was arbitrarily assigned as (S)-3,3,3-trifluoro-2-hydroxy-2-methyl-1-(6-(3-methyl-1*H*-pyrrolo[2,3-*b*]pyridin-5-yl)-8-( (S)-morpholin-3-yl)-3,4-dihydroisoquinolin-2(1*H*)-yl)propan-1-one (Z309-2, 15 mg, yield: 30%, retention time: 9.572 min, purity: 100%, ee value: 100%). ES-API: [M+H]⁺ = 489.3. ¹H NMR (400 MHz, DMSO-*d*₆) δ 11.35 (s, 1H), 8.46 (d, *J* = 2.0 Hz, 1H), 8.11 (d, *J* = 2.0 Hz, 1H), 7.79 (d, *J* = 1.6 Hz, 1H), 7.45 (s, 1H), 7.39 - 7.13 (m, 2H), 5.42 - 5.06 (m, 1H), 5.00 - 4.57 (m, 1H), 4.23 - 4.02 (m, 1H), 4.02 - 3.92 (m, 1H), 3.85 - 3.61 (m, 3H), 3.54 (td, *J* = 10.8, 3.2 Hz, 1H), 3.29 - 3.24 (m, 1H), 3.10 - 2.83 (m, 4H), 2.32 (s, 3H), 1.66 - 1.51 (m, 3H).

### Example 100. Synthesis of compound Z310

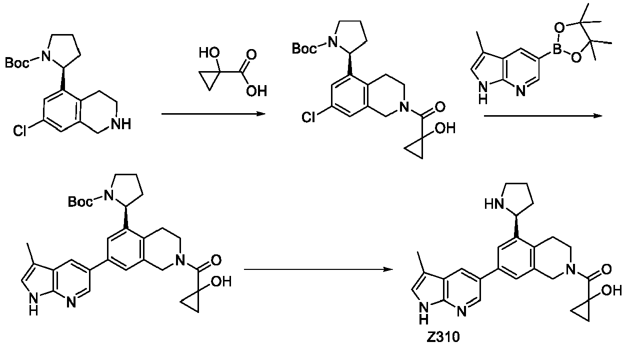

Step 1: 1-Hydroxycyclopropane carboxylic acid (58.5 mg, 573.7 µmol), triethylamine (90.12 mg, 890.59 µmol), 1-(3-dimethylaminopropyl)-3-ethylcarbodiimide hydrochloride (113 mg, 593.7 µmol) and 1-hydroxybenzotriazole (80.22 mg, 593.7 µmol) were added to a solution of tert-butyl (S)-2-(7-chloro-1,2,3,4-tetrahydroisoquinolin-5-yl)pyrrolidine-1-carboxylate (100 mg, 286.86 µmol) in N,N-dimethylformamide (3 mL) and stirred at 25°C for 1 h. The reaction was monitored by LCMS for completion, and quenched by the addition of water. The reaction mixture was extracted with ethyl acetate, dried over anhydrous sodium sulfate, and concentrated. The crude product was purified by column chromatography (methanol/dichloromethane = 0-5%) to give a product tert-butyl (S)-2-(7-chloro-2-(1-hydroxycyclopropanecarbonyl)-1,2,3,4-tetrahydroisoquinolin-5-yl)py rrolidine-1-carboxylate (50 mg, yield: 40.2%). ES-API:[M+H]⁺=421.0.

Step 2: The compound tert-butyl (S)-2-(7-chloro-2-(1-hydroxycyclopropanecarbonyl)-1,2,3,4-tetrahydroisoquinolin-5-yl)py rrolidine-1-carboxylate (50 mg, 119 µmol) was dissolved in dioxane/water (1 mL/0.2 mL). 3-Methyl-5-(4,4,5,5-tetramethyl-1,3,2-dioxaborolan-2-yl)-1H-pyrrolo[2,3-b]pyridine (37 mg, 143 mmol), chloro (2-dicyclohexylphosphino-2',6'-dimethoxy-1,1'-biphenylyl)(2'-amino-1,1'-biphenyl-2-yl) palladium (II) (9 mg, 12 µmol) and potassium carbonate (49 mg, 356 µmol) were added, and the system was heated to 100°C for 2 h. Hydrochloric acid-methanol solution was added and stirred at room temperature for 1 h. The reaction was monitored by LCMS for completion. The reaction mixture was adjusted to pH 7 with sodium bicarbonate solution, treated with dichloromethane/water, dried over anhydrous sodium sulfate, and concentrated. The crude product was purified by column chromatography (methanol/dichloromethane = 0-5%) to give a product tert-butyl (S)-2-(2-(1-hydroxycyclopropanecarbonyl)-7-(3-methyl-1H-pyrrolo[2,3-b]pyridin-5-yl)-1, 2,3,4-tetrahydroisoquinolin-5-yl)pyrrolidine-1-carboxylate (16.8 mg, yield: 27.4%). ES-API:[M+H]⁺=517.3.

Step 3: The tert-butyl (S)-2-(2-(1-hydroxycyclopropanecarbonyl)-7-(3-methyl-1H-pyrrolo[2,3-b]pyridin-5-yl)-1, 2,3,4-tetrahydroisoquinolin-5-yl)pyrrolidine-1-carboxylate (16.8 mg, 32 µmol) was dissolved in dichloromethane (1 mL). Trifluoroacetic acid (1 mL) was added. The system reacted at room temperature for 1 h. Upon completion of the reaction monitored by LCMS, the reaction mixture was spun to dryness. The crude product was purified by a preparative thin layer chromatographic column (dichloromethane/methanol = 10/1) to give a product (S)-(1-hydroxycyclopropyl)(7-(3-methyl-1H-pyrrolo[2,3-b]pyridin-5-yl)-5-(pyrrolidin-2-y l)-3,4-dihydroisoquinolin-2(1H)-yl)methanone (Z310, 4.5 mg, yield: 34%). ES-API:[M+1]⁺=417.3.

### Example 101. Synthesis of compound Z311

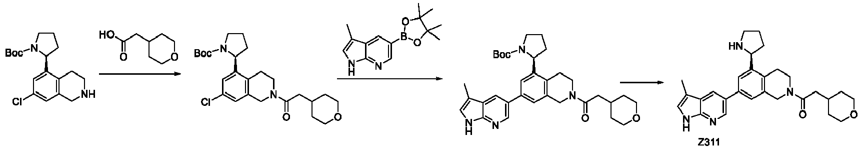

Step 1: 1-Propylphosphonic anhydride (284 mg, 0.45 mmol) was added to a solution of tert-butyl (S)-2-(7-chloro-1,2,3,4-tetrahydroisoquinolin-5-yl)pyrrolidine-1-carboxylate (100 mg, 0.3 mmol) in dichloromethane (10 mL) under an ice-water bath condition and stirred at 0°C for 10 min. Tetrahydropyran-4-acetic acid (65 mg, 0.45 mmol) was slowly added and stirred at room temperature for 1 h. The obtained mixture was poured into water (10 mL), extracted with dichloromethane (10 mL x 3), washed with saturated sodium chloride solution (10 mL x 1), dried over anhydrous sodium sulfate, filtered, and concentrated. The crude product was purified by column chromatography (petroleum ether/ethyl acetate = 50/50) to give tert-butyl (S)-2-(7-chloro-2-(2-(tetrahydro-2H-pyran-4-yl)acetyl)-1,2,3,4-tetrahydroisoquinolin-5-yl) pyrrolidine-1-carboxylate (70 mg, yield: 51%). ES-API: [M+H]⁺=463.

Step 2: Chloro (2-dicyclohexylphosphino-2',6'-dimethoxy-1,1'-biphenylyl)(2'-amino-1,1'-biphenyl-2-yl) palladium (II) (10 mg, 0.005 mmol) and potassium carbonate (64.64 mg, 0.468 mmol) were added to a solution of the tert-butyl (S)-2-(7-chloro-2-(2-(tetrahydro-2H-pyran-4-yl)acetyl)-1,2,3,4-tetrahydroisoquinolin-5-yl) pyrrolidine-1-carboxylate (70 mg, 0.156 mmol) and 3-methyl-5-(4,4,5,5-tetramethyl-1,3,2-dioxaborolan-2-yl)-1H-pyrrolo[2,3-b]pyridine (80 mg, 0.31 mmol) in dioxane (2 ml) and water (0.5 ml). The mixture was degassed with nitrogen and stirred at 110°C for 2 h. Water (3 mL) was added, and the reaction mixture was extracted with ethyl acetate (3 mL x 3). The organic phases were combined, washed with sodium chloride solution (3 mL×1), dried over anhydrous sodium sulfate, filtered, and concentrated. The crude product was purified by column chromatography (petroleum ether/ethyl acetate = 50/50) to give tert-butyl (S)-2-(7-(3-methyl-1H-pyrrolo[2,3-b]pyridin-5-yl)-2-(2-(tetrahydro-2H-pyran-4-yl)acetyl) -1,2,3,4-tetrahydroisoquinolin-5-yl)pyrrolidine-1-carboxylate (60 mg, yield: 71%) ES-API: [M+H]⁺=559.2.

Step 3: The tert-butyl (S)-2-(7-(3-methyl-1H-pyrrolo[2,3-b]pyridin-5-yl)-2-(2-(tetrahydro-2H-pyran-4-yl)acetyl) -1,2,3,4-tetrahydroisoquinolin-5-yl)pyrrolidine-1-carboxylate (60 mg, 0.055 mmol) was dissolved in anhydrous dichloromethane (0.5 mL). Trifluoroacetic acid (0.2 mL) was added, and the system reacted at room temperature for 2 h. The reaction was monitored by LCMS for completion, and the reaction mixture was concentrated to dryness under reduced pressure. After neutralization with ammonia-methanol solution (1 mL), the obtained mixture was concentrated again, and purified by prep-HPLC (formic acid method) to give 1-[7-(3-methyl-1H-pyrrolo[2,3-b]pyridin-5-yl)-5-[(2S)-tetrahydro-1H-pyrrol-2-yl]-1,2,3,4 -tetrahydroisoquinolin-2-yl]-2-(3,4,5,6-tetrahydro-2H-pyran-4-yl)ethan-1-one (Z311, 21 mg, yield: 43%). ES-API: [M+H]+=459.2.

### Example 102. Synthesis of compound Z312

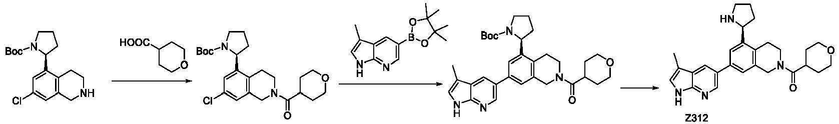

Step 1: 1-Propylphosphonic anhydride (284 mg, 0.45 mmol) was added to a solution of tert-butyl (S)-2-(7-chloro-1,2,3,4-tetrahydroisoquinolin-5-yl)pyrrolidine-1-carboxylate (100 mg, 0.3 mmol) in dichloromethane (5 mL) under an ice-water bath condition and stirred at 0°C for 10 min. Tetrahydro-2H-pyran-4-carboxylic acid (65 mg, 0.45 mmol) was slowly added and stirred at room temperature for 1 h. The obtained mixture was poured into water (10 mL), extracted with dichloromethane (10 mL x 3), washed with saturated sodium chloride solution (10 mL x 1), dried over anhydrous sodium sulfate, filtered, and concentrated. The crude product was purified by column chromatography (petroleum ether/ethyl acetate = 50/50) to give tert-butyl (S)-2-(7-chloro-2-(tetrahydro-2H-pyran-4-formyl)-1,2,3,4-tetrahydroisoquinolin-5-yl)pyrr olidine-1-carboxylate (70 mg, yield: 51%). ES-API: [M+H]⁺=449.

Step 2: Chloro (2-dicyclohexylphosphino-2',6'-dimethoxy-1,1'-biphenylyl)(2'-amino-1,1'-biphenyl-2-yl) palladium (II) (10 mg, 0.005 mmol) and potassium carbonate (64.64 mg, 0.468 mmol) were added to a solution of the tert-butyl (S)-2-(7-chloro-2-(tetrahydro-2H-pyran-4-formyl)-1,2,3,4-tetrahydroisoquinolin-5-yl)pyrr olidine-1-carboxylate (70 mg, 0.156 mmol) and 3-methyl-5-(4,4,5,5-tetramethyl-1,3,2-dioxaborolan-2-yl)-1H-pyrrolo[2,3-b]pyridine (80 mg, 0.31 mmol) in dioxane (2 mL) and water (0.5 mL). The mixture was degassed with nitrogen and stirred at 110°C for 2 h. Water (3 mL) was added, and the reaction mixture was extracted with ethyl acetate (3 mL x 3). The combined organic phase was washed with sodium chloride solution (3 mLx1), dried over anhydrous sodium sulfate, filtered, and concentrated. The crude product was purified by column chromatography (petroleum ether/ethyl acetate = 50/50) to give tert-butyl (S)-2-(7-(3-methyl-1H-pyrrolo[2,3-b]pyridin-5-yl)-2-(tetrahydro-2H-pyran-4-formyl)-1,2, 3,4-tetrahydroisoquinolin-5-yl)pyrrolidine-1-carboxylate (60 mg, yield: 71%) ES-API: [M+H]⁺=545.2.

Step 3: The tert-butyl (S)-2-(7-(3-methyl-1H-pyrrolo[2,3-b]pyridin-5-yl)-2-(tetrahydro-2H-pyran-4-formyl)-1,2, 3,4-tetrahydroisoquinolin-5-yl)pyrrolidine-1-carboxylate (60 mg, 0.055 mmol) was dissolved in anhydrous dichloromethane (0.5 mL). Trifluoroacetic acid (0.2 mL) was added, and the system reacted at room temperature for 2 h. The reaction was monitored by LCMS for completion, and the reaction mixture was concentrated to dryness under reduced pressure. After neutralization with ammonia-methanol solution (1 mL), the obtained mixture was concentrated again, and purified by prep-HPLC (ammonium bicarbonate method) to give (S)-1-[7-(3-methyl-1H-pyrrolo[2,3-b]pyridin-5-yl)-5-[pyrrolidin-2-yl]-3,4-dihydroisoquin olin-2(1H)-yl](tetrahydro-2H-pyran-4-yl)methanone (Z312, 8.7 mg, yield: 23%). ES-API: [M+H]⁺=445.

### Example 103. Synthesis of compound Z313 and compound Z38-1

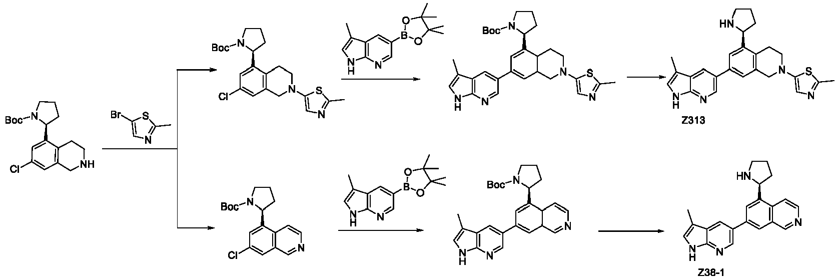

Step 1: tert-butyl (S)-2-(7-chloro-1,2,3,4-tetrahydroisoquinolin-5-yl)pyrrolidine-1-carboxylate (100 mg, 0.297 mmol) was dissolved in dried 1,4-dioxane (3 mL). 5-Bromo-2-methylthiazole (105.71 mg, 0.594 mmol), tris(dibenzylideneacetone)dipalladium (27.18 mg, 0.030 mmol), 1,1'-binaphthyl-2,2'-bis(diphenylphosphine) (36.97 mg, 0.059 mmol) and sodium tert-butoxide (71.33 mg, 0.742 mmol) were added successively, and the system was heated to 100°C under microwave for 3 h. Upon completion of the reaction, the reaction mixture was diluted with ethyl acetate (30 mL), washed with saturated brine (30 mL), dried over anhydrous sodium sulfate, filtered, and concentrated. The crude product was purified by column chromatography (methanol/dichloromethane = 1/20) to give tert-butyl (S)-2-(7-chloro-2-(2-methylthiazol-5-yl)-1,2,3,4-tetrahydroisoquinolin-5-yl)pyrrolidine-1-carboxylate (10 mg, yield: 7.76%). ES-API: [M+H]⁺=434.2; tert-butyl (S)-2-(7-chloroisoquinolin-5-yl)pyrrolidine-1-carboxylate (30 mg, yield: 30.3%). ES-API: [M+H]⁺=333.1.

Step 2: The tert-butyl (S)-2-(7-chloro-2-(2-methylthiazol-5-yl)-1,2,3,4-tetrahydroisoquinolin-5-yl)pyrrolidine-1-carboxylate (10 mg, 0.023 mmol), 3-methyl-5-(4,4,5,5-tetramethyl-1,3,2-dioxaborolan-2-yl)-1H-pyrrolo[2,3-b]pyridine (8.92 mg, 0.035 mmol), chloro (2-dicyclohexylphosphino-2',6'-dimethoxy-1,1'-biphenylyl)(2'-amino-1,1'-biphenyl-2-yl) palladium (II) (8.30 mg, 0.012 mmol) and potassium carbonate (3.18 mg, 0.023 mmol) were added to 1,4-dioxane (3 mL) and water (0.5 mL). The system was replaced with nitrogen and reacted under microwave at 115°C for 1 h. The reaction was monitored by LCMS for completion, and ethyl acetate (20 mL) was added. The obtained mixture was washed with water (10 mL) and saturated brine (10 mL) successively, dried over anhydrous sodium sulfate, and concentrated. The crude product was purified by preparative thin layer chromatography (petroleum ether/ethyl acetate = 1/1) to give (2S)-tert-butyl 2-(7-(3-methyl-1H-pyrrolo[2,3-b]pyridin-5-yl)-2-(2-methylthiazol-5-yl)-1,2,3,4,4a,8a-hex ahydroisoquinolin-5-yl)pyrrolidine-1-carboxylate (8 mg, yield: 65.68%). ES-API: [M+H]⁺=532.3.

Step 3: The (2S)-tert-butyl 2-(7-(3-methyl-1H-pyrrolo[2,3-b]pyridin-5-yl)-2-(2-methylthiazol-5-yl)-1,2,3,4,4a,8a-hex ahydroisoquinolin-5-yl)pyrrolidine-1-carboxylate (8 mg, 0.019 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia-methanol solution (1 mL), the obtained mixture was concentrated again, and purified by prep-HPLC (formic acid method) to give (S)-2-methyl-5-(7-(3-methyl-1H-pyrrolo[2,3-b]pyridin-5-yl)-5-(pyrrol-2-yl)-3,4-dihydrois oquinolin-2(1H)-yl)thiazole (Z313, formate, 1.1 mg, yield: 12.1%). ES-API: [M+H]⁺=432.2.

Step 4: The tert-butyl (S)-2-(7-chloroisoquinolin-5-yl)pyrrolidine-1-carboxylate (30 mg, 0.090 mmol), 3-methyl-5-(4,4,5,5-tetramethyl-1,3,2-dioxaborolan-2-yl)-1H-pyrrolo[2,3-b]pyridine (23.22 mg, 0.090 mmol), chloro (2-dicyclohexylphosphino-2',6'-dimethoxy-1,1'-biphenylyl)(2'-amino-1,1'-biphenyl-2-yl) palladium (II) (6.48 mg, 0.009 mmol) and potassium carbonate (37.26 mg, 0.27 mmol) were added to 1,4-dioxane (3 mL) and water (0.5 mL). The system was replaced with nitrogen and reacted under microwave at 115°C for 1 h. Upon completion of the reaction, ethyl acetate (20 mL) was added. The obtained mixture was washed with water (10 mL ×1) and saturated brine (10 mLx 1), and dried over anhydrous sodium sulfate. The crude product was purified by preparative thin layer chromatography (petroleum ether/ethyl acetate = 1/1) to give tert-butyl (S)-2-(7-(3-methyl-1H-pyrrolo[2,3-b]pyridin-5-yl)-4a,8a-dihydroisoquinolin-5-yl)pyrrolid ine-1-carboxylate (8 mg, yield: 21.1%). ES-API: [M+H]⁺=429.1.

Step 5: The tert-butyl (S)-2-(7-(3-methyl-1H-pyrrolo[2,3-b]pyridin-5-yl)-4a,8a-dihydroisoquinolin-5-yl)pyrrolid ine-1-carboxylate (8 mg, 0.019 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia-methanol solution (1 mL), the obtained mixture was concentrated again, and purified by prep-HPLC (formic acid method) to give (S)-7-(3-methyl-1H-pyrrolo[2,3-b]pyridin-5-yl)-2-(2-methylthiazol-5-yl)-5-(pyrrolidin-2-y l)-1,2,3,4-tetrahydroisoquinoline (Z38-1, formate, 3.5 mg, yield: 56.1%). ES-API: [M+H]⁺=329.1. ¹H NMR (500 MHz, DMSO-*d*₆) δ 11.46 (s, 1H), 9.39 (s, 1H), 8.67 (d, *J* = 2.2 Hz, 1H), 8.52 (d, *J* = 5.9 Hz, 1H), 8.38 (q, *J* = 2.0 Hz, 2H), 8.34 (d, *J* = 2.2 Hz, 1H), 8.28 (s, 1H), 8.05 (d, *J* = 6.0 Hz, 1H), 7.32 (s, 1H), 4.92 (t, *J* = 7.7 Hz, 1H), 3.2-3.08 (m, 3H), 2.47 - 2.43 (m, 1H), 2.36 - 2.35 (m, 3H), 1.93-1.87 (m, 2H), 1.72 - 1.63 (m, 1H).

### Example 104. Synthesis of compound Z314

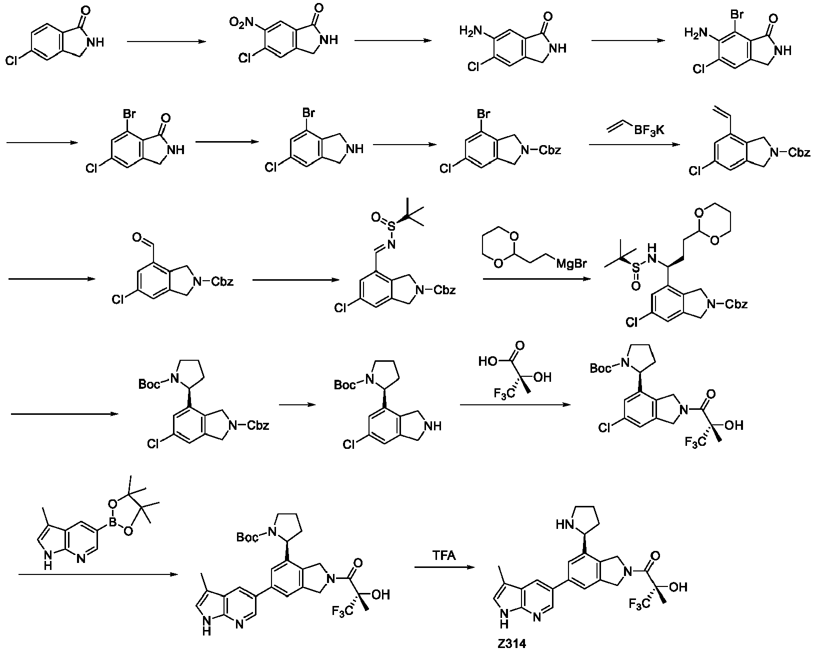

Step 1: 5-Chloroisoindolin-1-one (5 g, 29.83 mmol) was dissolved in concentrated sulfuric acid (60 mL). Concentrated nitric acid (2.3 mL) was added dropwise under ice bath cooling, and the system reacted in an ice bath for 1 h. Upon completion of the reaction, the reaction mixture was poured into ice water, and extracted with dichloromethane. The organic phase was dried over anhydrous sodium sulfate and spun to dryness to give a product 5-chloro-6-nitroisoindolin-1-one (6 g, yield: 95%). ES-API:[M+H]⁺=213.0.

Step 2: 5-Chloro-6-nitroisoindolin-1-one (4.5 g, 21.2 mmol) was dissolved in ethanol/water (100 mL/10 mL). Reduced iron powder (7.1 g, 127 mmol) and ammonium chloride (6.8 g, 127 mmol) were added, and the system was heated to 90°C for 2 h. Upon completion of the reaction, the reaction mixture was filtered, washed with methanol, and spun to dryness to give a crude product. The crude product was purified by column chromatography (dichloromethane/methanol = 3/1) to give a product 6-amino-5-chloroisoindolin-1-one (2.3 g, yield: 55%). ES-API:[M+H]⁺=183.0.

Step 3: The 6-amino-5-chloroisoindolin-1-one (2.2 g, 12.1 mmol) was dissolved in dichloromethane/methanol (50 mL/10 mL). N-bromosuccinimide (2.6 g, 14.5 mmol) was added, and the system reacted at room temperature for 1 h. The reaction mixture was spun to dryness to give a crude product. The crude product was purified by column chromatography (dichloromethane/methanol = 10/1) to give a product 6-amino-7-bromo-5-chloro-isoindolin-1-one (3 g, crude product). ES-API:[M+H]⁺=261.0.

Step 4: The 6-amino-7-bromo-5-chloro-isoindolin-1-one (3 g, 11.5 mmol) was dissolved in tetrahydrofuran/water (10 mL/10 mL) and hypophosphorous acid (20 mL), and the system was cooled in an ice bath. Sodium nitrite (1.6 g, 22.9 mmol) was added, and the system was warmed to room temperature for 1 h. Upon completion of the reaction, the reaction mixture was extracted with water and dichloromethane, washed with saturated sodium bicarbonate, dried over anhydrous sodium sulfate, and spun to dryness to give a crude product. The crude product was beaten with petroleum ether/ethyl acetate (5/1) to give a product 7-bromo-5-chloro-isoindolin-1-one (900 mg, crude product). ES-API:[M+H]⁺=247.9.

Step 5: The 7-bromo-5-chloro-isoindolin-1-one (900 mg, 3.6 mmol) was dissolved in tetrahydrofuran (20 mL). Borane tetrahydrofuran (36.5 mL, 36.5 mmol) was added, and the system was heated to 70°C overnight. The reaction mixture was quenched with 2M hydrochloric acid, heated to 70°C and stirred for 30 min, and extracted with dichloromethane. The aqueous phase was neutralized with sodium bicarbonate, extracted with dichloromethane, dried over anhydrous sodium sulfate, and spun to dryness to give a product 4-bromo-6-chloro-isoindoline (500 mg, crude product). ES-API:[M+H]⁺=231.9.

Step 6: The 4-bromo-6-chloro-isoindoline (500 mg, 2.2 mmol) was dissolved in dichloromethane (10 mL). Benzyl chloroformate (734 mg, 4.3 mmol) and triethylamine (653 mg, 6.4 mmol) were added, and the system reacted at room temperature for 1 h. Upon completion of the reaction, the reaction mixture was extracted with water and dichloromethane, dried over anhydrous sodium sulfate, and spun to dryness to give a crude product. The crude product was purified by silica gel column chromatography (petroleum ether/ethyl acetate = 10/1) to give a product benzyl 4-bromo-6-chloro-isoindoline-2-carboxylate (367 mg, yield: 43%). ES-API:[M+23]⁺=390.0.

Step 7: The benzyl 4-bromo-6-chloro-isoindoline-2-carboxylate (367 mg, 1.0 mmol) was dissolved in ethanol (8 mL). Potassium vinyl trifluoroborate (402 mg, 3.0 mmol), 1,1-bis(diphenylphosphino)ferrocene dichloropalladium (73 mg, 0.1 mmol) and triethylamine (304 mg, 3.0 mmol) were added, and the system was heated to 80°C for 3 h. Upon completion of the reaction, the reaction mixture was spun to dryness to give a crude product. The crude product was purified by column chromatography (petroleum ether/ethyl acetate = 4/1) to give a product benzyl 6-chloro-4-vinyl-isoindoline-2-carboxylate (300 mg, yield: 90%). ES-API:[M+H]⁺=314.1.

Step 8: The benzyl 6-chloro-4-vinyl-isoindoline-2-carboxylate (300 mg, 0.96 mmol) was dissolved in tetrahydrofuran/water (6 mL/3 mL). Sodium periodate (1.64 g, 7.6 mmol) and potassium osmate dihydrate (106 mg, 0.27 mmol) were added, and the system reacted at room temperature for 2 h. The reaction mixture was quenched with aqueous sodium thiosulfate solution, and extracted with ethyl acetate. The organic phase was dried over anhydrous sodium sulfate and spun to dryness to give a crude product. The crude product was purified by a preparative thin layer chromatographic column (petroleum ether/ethyl acetate = 2/1) to give a product benzyl 6-chloro-4-formyl-isoindoline-2-carboxylate (120 mg, yield: 33%). ES-API:[M+23]⁺=338.1.

Step 9: The benzyl 6-chloro-4-formyl-isoindoline-2-carboxylate (120 mg, 0.38 mmol) was dissolved in dichloromethane (3 mL). (*S*)-2-Methylpropane-2-sulfinamide (92 mg, 0.76 mmol) and tetraethyl titanate (347 mg, 1.5 mmol) were added, and the system reacted at room temperature overnight. Upon completion of the reaction, the reaction was quenched by the addition of water. The reaction mixture was extracted with dichloromethane, dried over anhydrous sodium sulfate, and spun to dryness to give a product benzyl 4-[(*E*)-[(*S*)-tert-butylsulfinylliminomethyl]-6-chloro-isoindoline-2-carboxylate (120 mg, crude product). ES-API:[M+H]⁺=419.2.

Step 10: The benzyl 4-[(*E*)-[(*S*)-tert-butylsulfinylliminomethyl]-6-chloro-isoindoline-2-carboxylate (120 mg, 0.29 mmol) was dissolved in tetrahydrofuran (2 mL), and the system was cooled under a dry ice/ethanol bath condition. [2-(1,3-dioxan-2-yl)ethyl]magnesium bromide (2.3 mL, 1.2 mmol) was added dropwise, and the system reacted for 2 h with cooling. The reaction mixture was quenched by being poured into water, extracted with ethyl acetate, dried over anhydrous sodium sulfate, and spun to dryness to give a crude product. The crude product was purified by column chromatography (petroleum ether/ethyl acetate = 1/1) to give a product benzyl 6-chloro-4-((*S*)-1-((*S*)-1,1-dimethylethylsulfonamido)-3-(1,3-dioxan-2-yl)propyl)isoindoli ne-2-carboxylate (145 mg, yield: 95%). ES-API:[M+H]⁺=535.2.

Step 11: The benzyl 6-chloro-4-((*S*)-1-((*S*)-1,1-dimethylethylsulfonamido)-3-(1,3-dioxan-2-yl)propyl)isoindoli ne-2-carboxylate (145 mg, 0.27 mmol) was dissolved in trifluoroacetic acid (1 mL). Water (0.1 mL) was added, and the system reacted at room temperature for 1 h. Upon completion of the reaction, triethylsilane (315 mg, 2.7 mmol) was added, and the system reacted at room temperature overnight. The reaction mixture was spun to dryness. Tetrahydrofuran/water (1 mL/1 mL) was added, and the obtained mixture was basified with sodium bicarbonate. Di-tert-butyl dicarbonate (89 mg, 0.4 mmol) was then added, and the system reacted at room temperature for 2 h. Upon completion of the reaction, the reaction mixture was extracted with ethyl acetate/water, dried over anhydrous sodium sulfate, and spun to dryness to give a crude product. The crude product was purified by a preparative thin layer chromatographic column (petroleum ether/ethyl acetate = 3/1) to give a product (S)-benzyl 4-(1-(tert-butoxycarbonyl)pyrrolidin-2-yl)-6-chloroisoindoline-2-carboxylate (70 mg, yield: 56%). ES-API:[M+H-100]⁺=357.1.

Step 12: Palladium chloride (14 mg, 0.08 mmol), triethylamine (0.04 mL, 0.31 mmol) and triethylsilane (0.1 mL, 0.61 mmol) were added successively to a solution of the (S)-benzyl 4-(1-(tert-butoxycarbonyl)pyrrolidin-2-yl)-6-chloroisoindoline-2-carboxylate (70 mg, 0.15 mmol) in dichloromethane (2 mL) and stirred at room temperature for 1 h. The reaction mixture was quenched with methanol (2 mL), filtered, and concentrated to give tert-butyl (S)-2-(6-chloroisoindolin-4-yl)pyrrolidine-1-carboxylate (50 mg, crude product). ES-API: [M+H]⁺ = 323.4.

Step 13: *N,N*-Diisopropylethyl amine (180 mg, 1.39 mmol) and *N*,*N*,*N'*,*N'*-tetramethyl-*O*-(7-azabenzotriazol-1-yl)urea hexafluorophosphate (106 mg, 0.28 mmol) were added successively to a solution of the tert-butyl (S)-2-(6-chloroisoindolin-4-yl)pyrrolidine-1-carboxylate (45 mg, 0.14 mmol) and (S)-3,3,3-trifluoro-2-hydroxy-2-methylpropanoic acid (44 mg, 0.28 mmol) in dichloromethane (2 mL) and stirred at room temperature for 1 h. Dichloromethane (10 mL) was added to the reaction mixture, and the obtained mixture was washed with water (5 mL) and saturated brine (5 mL) successively. The organic phase was dried over anhydrous sodium sulfate, filtered, and concentrated. The crude product was purified by silica gel column chromatography (tetrahydrofuran/petroleum ether = 0-50%) to give tert-butyl (S)-2-(6-chloro-2-((S)-3,3,3-trifluoro-2-hydroxy-2-methylpropanoyl)isoindolin-4-yl)pyrro lidine-1-carboxylate (40 mg, yield: 62%). ES-API: [M+H]⁺ = 407.2.

Step 14: In the presence of protective nitrogen, a mixed solution of the tert-butyl (S)-2-(6-chloro-2-((S)-3,3,3-trifluoro-2-hydroxy-2-methylpropanoyl)isoindolin-4-yl)pyrro lidine-1-carboxylate (40 mg, 0.09 mmol), 3-methyl-5-(4,4,5,5-tetramethyl-dioxaborolan-2-yl)-1*H*-pyrrolo[2,3-*b*]pyridine (29 mg, 0.11 mmol), chloro (2-dicyclohexylphosphino-2',6'-dimethoxy-1,1'-biphenylyl)(2'-amino-1,1'-biphenyl-2-yl) palladium (II) (6 mg, 0.01 mmol), 2-dicyclohexylphosphine-2',6'-dimethoxybiphenyl (4 mg, 0.01 mmol) and potassium carbonate (24 mg, 0.17 mmol) in 1,4-dioxane (2 mL) and water (0.4 mL) was stirred at 120°C for 2 h. Ethyl acetate (10 mL) was poured into the reaction mixture, and the obtained mixture was washed with saturated brine (5 mL) and water (5 mL) successively. The organic phase was dried over anhydrous sodium sulfate, filtered, and concentrated. The crude product was purified by silica gel column chromatography (methanol/dichloromethane = 0-10%) to give tert-butyl (S)-2-(6-(3-methyl-1*H*-pyrrolo[2,3-*b*]pyridin-5-yl)-2-((S)-3,3,3-trifluoro-2-hydroxy-2-met hylpropanoyl)isoindolin-4-yl)pyrrolidine-1-carboxylate (25 mg, yield: 52%). ES-API: [M+H]⁺ = 559.3.

Step 15: Trifluoroacetic acid (0.5 mL) was added dropwise to a solution of the tert-butyl (S)-2-(6-(3-methyl-1*H*-pyrrolo[2,3-*b*]pyridin-5-yl)-2-((S)-3,3,3-trifluoro-2-hydroxy-2-met hylpropanoyl)isoindolin-4-yl)pyrrolidine-1-carboxylate (25 mg, 45 µmol) in dichloromethane (1 mL) under an ice bath condition and stirred at room temperature for 1 h. The reaction mixture was concentrated, and purified by prep-HPLC (ammonium bicarbonate method) to give (S)-3,3,3-trifluoro-2-hydroxy-2-methyl-1-(6-(3-methyl-1H-pyrrolo[2,3-b]pyridin-5-yl)-4-( (S)-pyrrolidin-2-yl)isoindolin-2-yl)propan-1-one (Z314, 9.5 mg, purity: 100%, yield: 46%) as a white solid. ES-API: [M+H]⁺ = 459.2. ¹H NMR (400 MHz, DMSO-*d*₆) δ 11.35 (s, 1H), 8.53 - 8.41 (m, 1H), 8.18 - 8.06 (m, 1H), 7.70 (d, *J* = 5.2 Hz, 1H), 7.59 (d, *J* = 6.4 Hz, 1H), 7.35 - 7.06 (m, 2H), 5.31 - 5.11 (m, 2H), 4.85 (s, 1H), 4.80 (s, 1H), 4.27 - 4.07 (m, 1H), 3.19 - 3.07 (m, 1H), 3.03 - 2.91 (m, 1H), 2.31 (s, 3H), 2.26 - 2.13 (m, 1H), 1.97 - 1.74 (m, 2H), 1.69 - 1.53 (m, 4H).

### Example 105. Synthesis of compound Z315

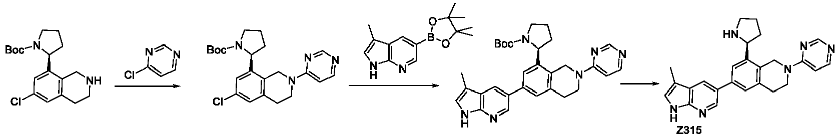

Step 1: tert-butyl (S)-2-(6-chloro-1,2,3,4-tetrahydroisoquinolin-8-yl)pyrrolidine-1-carboxylate (100 mg, 0.297 mmol) was dissolved in dried N,N-dimethylformamide (5 mL). 4-Chloropyrimidine (102.00 mg, 0.891 mmol) and potassium carbonate (204.83 mg, 1.484 mmol) were added, and the system was heated to 50°C for 18 h. Upon completion of the reaction, the reaction mixture was diluted with ethyl acetate (30 mL), washed with saturated brine (30 mL), dried over anhydrous sodium sulfate, filtered, and concentrated. The crude product was purified by silica gel column chromatography (methanol/dichloromethane = 1/20) to give tert-butyl (S)-2-(6-chloro-2-(pyrimidin-4-yl)-1,2,3,4-tetrahydroisoquinolin-8-yl)pyrrolidine-1-carbo xylate (80 mg, yield: 64.95%). ES-API: [M+H]⁺=415.1.

Step 2: The tert-butyl (S)-2-(6-chloro-2-(pyrimidin-4-yl)-1,2,3,4-tetrahydroisoquinolin-8-yl)pyrrolidine-1-carbo xylate (80 mg, 0.193 mmol), 3-methyl-5-(4,4,5,5-tetramethyl-1,3,2-dioxaborolan-2-yl)-1H-pyrrolo[2,3-b]pyridine (74.65 mg, 0.289 mmol), chloro (2-dicyclohexylphosphino-2',6'-dimethoxy-1,1'-biphenylyl)(2'-amino-1,1'-biphenyl-2-yl) palladium (II) (13.89 mg, 0.019 mmol) and potassium carbonate (79.94 mg, 0.578 mmol) were added to 1,4-dioxane (3 mL) and water (0.5 mL). The system was replaced with nitrogen and reacted under microwave at 115°C for 1 h. Upon completion of the reaction, ethyl acetate (20 mL) was added. The obtained mixture was washed with water (10 mL) and saturated brine (10 mL) successively, and dried over anhydrous sodium sulfate. The crude product was purified by preparative thin layer chromatography (petroleum ether/ethyl acetate = 1/1) to give tert-butyl (S)-2-(6-(3-methyl-1H-pyrrolo[2,3-b]pyridin-5-yl)-2-(pyrimidin-4-yl))-1,2,3,4-tetrahydroi soquinolin-8-yl)pyrrolidine-1-carboxylate (60 mg, yield: 60.94%). ES-API: [M+H]⁺=511.3.

Step 3: The tert-butyl (S)-2-(6-(3-methyl-1H-pyrrolo[2,3-b]pyridin-5-yl)-2-(pyrimidin-4-yl))-1,2,3,4-tetrahydroi soquinolin-8-yl)pyrrolidine-1-carboxylate (60 mg, 0.117 mmol) was dissolved in dichloromethane (2 mL). Trifluoroacetic acid (1 mL) was added, and the system reacted at room temperature for 0.5 h. Upon completion of the reaction, the reaction mixture was concentrated. After neutralization with ammonia-methanol solution (1 mL), the obtained mixture was concentrated again and purified by prep-HPLC (ammonia water method) to give (S)-6-(3-methyl-1H-pyrrolo[2,3-b]pyridin-5-yl)-2-(pyrimidin-4-yl)-8-(pyrrolidin-2-yl)-1,2 ,3,4-tetrahydroisoquinoline (Z315, 30 mg, yield: 62.5%). ES-API: [M+H]⁺=411.2. ¹H NMR (500 MHz, DMSO-*d*₆) δ 11.35 (d, *J* = 2.4 Hz, 1H), 8.56 (d, *J* = 1.2 Hz, 1H), 8.47 (d, *J* = 2.2 Hz, 1H), 8.23 (d, *J* = 6.1 Hz, 1H), 8.11 (d, *J* = 2.1 Hz, 1H), 7.78 (d, *J* = 2.0 Hz, 1H), 7.43 (d, *J* = 2.0 Hz, 1H), 7.27 - 7.26 (m, 1H), 6.92 (dd, *J* = 6.3, 1.3 Hz, 1H), 5.00 (d, *J* = 16.7 Hz, 1H), 4.75 (d, *J* = 16.5 Hz, 1H), 4.33 (t, *J* = 7.7 Hz, 1H), 3.83 (s, 2H), 3.16-3.10 (m, 1H), 3.01 (t, *J* = 6.1 Hz, 2H).

### Example 106. Synthesis of compound Z316

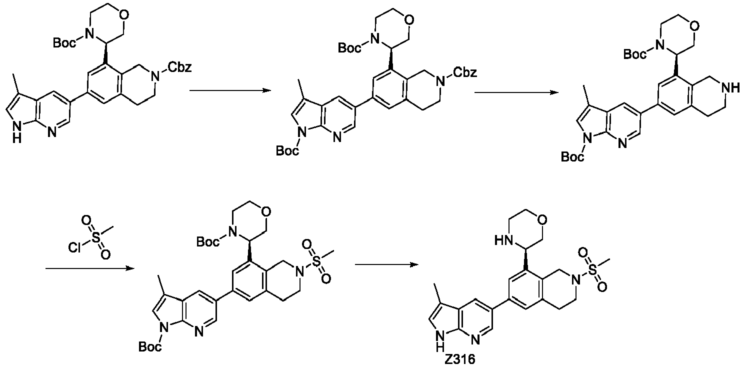

Step 1: Triethylamine (7.64 mg, 0.076 mmol) and di-tert-butyl dicarbonate (16.64 mg, 0.076 mmol) were added to tert-butyl (R)-3-(2-((benzyloxy)carbonyl)-6-(3-methyl-1H-pyrrolo[2,3-b]pyridin-5-yl)-1,2,3,4-tetrah ydroisoquinolin-8-yl)morpholine-4-carboxylate (40 mg, 0.069 mmol) and stirred at room temperature overnight. The mixture was washed with water (2 mL X 2) and brine (2 mL), dried over anhydrous sodium sulfate, and concentrated to give tert-butyl (R)-3-(2-((benzyloxy)carbonyl)-6-(1-(tert-butoxycarbonyl)-3-methyl-1H-pyrrolo[2,3-b]py ridin-5-yl)-1,2,3,4-tetrahydroisoquinolin-8-yl)morpholine-4-carboxylate (45 mg, yield: 95%). ES-API: [M+H]⁺=683.

Step 2: Palladium on carbon (10 mg) was added to a solution (3 mL) of the tert-butyl (R)-3-(2-((benzyloxy)carbonyl)-6-(1-(tert-butoxycarbonyl)-3-methyl-1H-pyrrolo[2,3-b]py ridin-5-yl)-1,2,3,4-tetrahydroisoquinolin-8-yl)morpholine-4-carboxylate (45 mg, 0.06 mmol) in isopropanol, and the system reacted at room temperature overnight. The reaction mixture was concentrated to dryness under reduced pressure to give tert-butyl (R)-3-(6-(1-(tert-butoxycarbonyl)-3-methyl-1H-pyrrolo[2,3-b]pyridin-5-yl)-1,2,3,4-tetrahy droisoquinolin-8-yl)morpholine-4-carboxylate (30 mg, yield: 91%) ES-API: [M+H]⁺=549.2.

Step 3: The tert-butyl (R)-3-(6-(1-(tert-butoxycarbonyl)-3-methyl-1H-pyrrolo[2,3-b]pyridin-5-yl)-1,2,3,4-tetrahy droisoquinolin-8-yl)morpholine-4-carboxylate (30 mg, 0.05 mmol) was dissolved in dichloromethane (1 mL). 2-(7-azobenzotriazole)-N,N,N',N'-tetramethylurea hexafluorophosphate (50 mg, 0.1 mmol), methanesulfonyl chloride (34 mg, 0.1 mmol), and N,N-diisopropyl ethylenediamine (40 mg, 0.15 mmol) were added successively, and the system reacted at room temperature for 2 h. Upon completion of the reaction, the reaction mixture was diluted with dichloromethane (10 mL). The organic phase was washed with water (5 mL) and sodium chloride solution (5 mL), dried over anhydrous sodium sulfate, and concentrated to dryness under reduced pressure. The crude product was purified by column chromatography (ethyl acetate:petroleum ether = 50:50) to give tert-butyl (R)-3-(6-(1-(tert-butoxycarbonyl)-3-methyl-1H-pyrrolo[2,3-b]pyridin-5-yl)-2-(methylsulf onyl)-1,2,3,4-tetrahydroisoquinolin-8-yl)morpholine-4-carboxylate (25 mg, yield: 81%) ES-API: [M+H]⁺=627.2.

Step 4: The tert-butyl (R)-3-(6-(1-(tert-butoxycarbonyl)-3-methyl-1H-pyrrolo[2,3-b]pyridin-5-yl)-2-(methylsulf onyl)-1,2,3,4-tetrahydroisoquinolin-8-yl)morpholine-4-carboxylate (25 mg, 0.045 mmol) was dissolved in anhydrous dichloromethane (0.5 mL). Trifluoroacetic acid (0.2 mL) was added, and the system reacted at room temperature for 2 h. The reaction was monitored by LC-MS for completion, and the reaction mixture was concentrated to dryness under reduced pressure. After neutralization with ammonia-methanol solution (1 mL), the obtained mixture was concentrated again, and purified by prep-HPLC (ammonium bicarbonate method) to give 2-(methyldioxo-λ6-sulfanyl)-6-(3-methyl-1H-pyrrolo[2,3-b]pyridin-5-yl)-8-[(3R)-1,4-oxa zecyclohex-3-yl]-1,2,3,4-tetrahydroisoquinoline (Z316, 1.07 mg, yield: 8%). ES-API: [M+H]⁺=427.

### Example 107. Synthesis of compound Z317

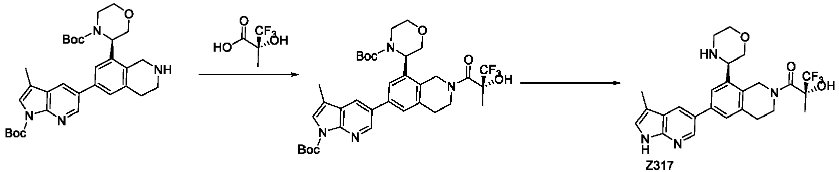

Step 1: tert-butyl (R)-3-(6-(1-(tert-butoxycarbonyl)-3-methyl-1H-pyrrolo[2,3-b]pyridin-5-yl)-1,2,3,4-tetrahy droisoquinolin-8-yl)morpholine-4-carboxylate (40 mg, 0.07 mmol) was dissolved in dichloromethane (1 mL). 2-(7-azobenzotriazole)-N,N,N',N'-tetramethylurea hexafluorophosphate (50 mg, 0.1 mmol), (R)-2-trifluoromethyl-2-hydroxypropanoic acid (18 mg, 0.1 mmol), and N,N-diisopropyl ethylenediamine (40 mg, 0.15 mmol) were added successively, and the system reacted at room temperature for 2 h. Upon completion of the reaction, the reaction mixture was diluted with dichloromethane (10 mL). The organic phase was washed with water (5 mL) and sodium chloride solution (5 mL), dried over anhydrous sodium sulfate, and concentrated to dryness under reduced pressure. The crude product was purified by column chromatography (ethyl acetate:petroleum ether = 50:50) to give tert-butyl (R)-3-(6-(1-(tert-butoxycarbonyl)-3-methyl-1H-pyrrolo[2,3-b]pyridin-5-yl)-2-((R)-3,3,3-tr ifluoro-2-hydroxy-2-methylpropanoyl)-1,2,3,4-tetrahydroisoquinolin-8-yl)morpholine-4-c arboxylate (30 mg, yield: 862.3%) ES-API: [M+H]⁺=689.2.

Step 2: The tert-butyl (R)-3-(6-(1-(tert-butoxycarbonyl)-3-methyl-1H-pyrrolo[2,3-b]pyridin-5-yl)-2-((R)-3,3,3-tr ifluoro-2-hydroxy-2-methylpropanoyl)-1,2,3,4-tetrahydroisoquinolin-8-yl)morpholine-4-c arboxylate (30 mg, 0.045 mmol) was dissolved in anhydrous dichloromethane (0.5 mL). Trifluoroacetic acid (0.2 mL) was added, and the system reacted at room temperature for 2 h. The reaction was monitored by LCMS for completion, and the reaction mixture was concentrated to dryness under reduced pressure. After neutralization with ammonia-methanol solution (1 mL), the obtained mixture was concentrated again, and purified by prep-HPLC (ammonium bicarbonate method) to give (R)-3,3,3-trifluoro-2-hydroxy-2-methyl-1-[6-(3-methyl-1H-pyrrolo[2,3-b]pyridin-5-yl)-8-[ (R)-morpholin-3-yl]-3,4-dihydroisoquinolin-2(1H)-yl]propan-1-one (Z317, 1.2 mg, yield: 8%). ES-API: [M+H]⁺=588.

### Example 108. Synthesis of compound Z318

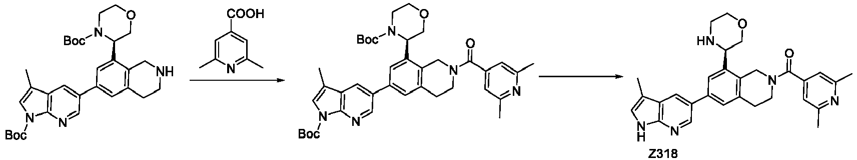

Step 1: tert-butyl (R)-3-(6-(1-(tert-butoxycarbonyl)-3-methyl-1H-pyrrolo[2,3-b]pyridin-5-yl)-1,2,3,4-tetrahy droisoquinolin-8-yl)morpholine-4-carboxylate (40 mg, 0.07 mmol) was dissolved in dichloromethane (1 mL). 2-(7-azobenzotriazole)-N,N,N',N'-tetramethylurea hexafluorophosphate (50 mg, 0.1 mmol), 2,6-dimethyl-4-pyridinecarboxylic acid (16 mg, 0.1 mmol), and N,N-diisopropyl ethylenediamine (40 mg, 0.15 mmol) were added successively, and the system reacted at room temperature for 2 h. Upon completion of the reaction, the reaction mixture was diluted with dichloromethane (10 mL). The organic phase was washed with water (5 mL) and sodium chloride solution (5 mL), dried over anhydrous sodium sulfate, and concentrated to dryness under reduced pressure. The crude product was purified by silica gel column chromatography (ethyl acetate:petroleum ether = 50:50) to give tert-butyl (R)-3-(6-(1-(tert-butoxycarbonyl)-3-methyl-1H-pyrrolo[2,3-b]pyridin-5-yl)-2-(2,6-dimeth ylisonicotinoyl)-1,2,3,4-tetrahydroisoquinolin-8-yl)morpholine-4-carboxylate (30 mg, yield: 62%) ES-API: [M+H]⁺=682.

Step 2: The tert-butyl (R)-3-(6-(1-(tert-butoxycarbonyl)-3-methyl-1H-pyrrolo[2,3-b]pyridin-5-yl)-2-(2,6-dimeth ylisonicotinoyl)-1,2,3,4-tetrahydroisoquinolin-8-yl)morpholine-4-carboxylate (30 mg, 0.045 mmol) was dissolved in anhydrous dichloromethane (0.5 mL). Trifluoroacetic acid (0.2 mL) was added, and the system reacted at room temperature for 2 h. The reaction was monitored by LCMS for completion, and the reaction mixture was concentrated to dryness under reduced pressure. After neutralization with ammonia-methanol solution (1 mL), the obtained mixture was concentrated again, and purified by prep-HPLC (ammonium bicarbonate method) to give (R)-(2,6-dimethylpyridin-4-yl)[6-(3-methyl-1H-pyrrolo[2,3-b]pyridin-5-yl)-8-[morpholin-3-yl]-3,4-dihydroisoquinolin-2(1H)-yl]methanone (Z318, 17.76 mg, yield: 77%). ES-API: [M+H]⁺=482.

### Example 109. Compound Z319

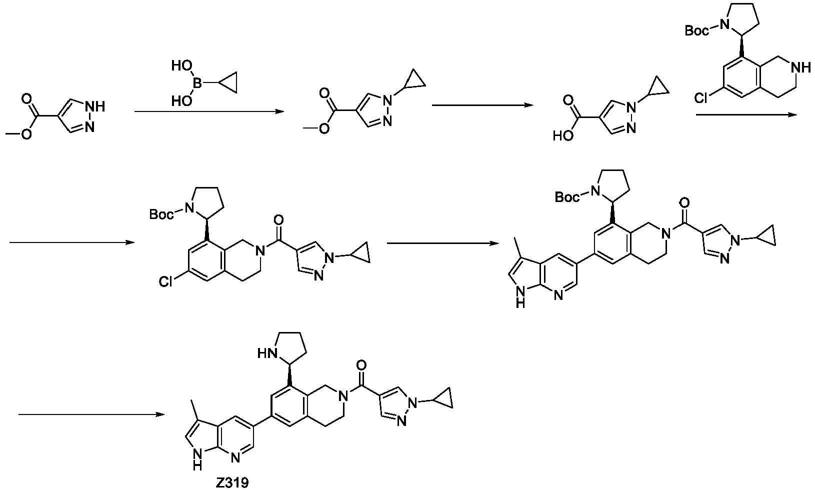

Step 1: Methyl 1*H*-pyrazole-4-carboxylate (500 mg, 3.97 mmol) was dissolved in 1,2-dichloroethane (25 mL). Cyclopropylboronic acid (683 mg, 7.94 mmol), sodium carbonate (842 mg, 7.94 mmol), 2,2'-bipyridine (619 mg, 3.97 mmol) and copper acetate (718 mg, 3.97 mmol) were added, and the system was heated to 70°C overnight in the presence of protective nitrogen. The reaction was quenched by the addition of water, and the reaction product was extracted with ethyl acetate. The organic phase was dried over anhydrous sodium sulfate and spun to dryness. The crude product was purified by column chromatography (petroleum ether/ethyl acetate = 2/1) to give methyl 1-cyclopropyl-1*H*-pyrazole-4-carboxylate (430 mg, yield: 65%). ES-API:[M+H]⁺= 167.1.

Step 2: The methyl 1-cyclopropyl-1*H*-pyrazole-4-carboxylate (200 mg, 1.2 mmol) was dissolved in tetrahydrofuran/methanol/water (3 mL/3 mL/1 mL). Lithium hydroxide monohydrate (253 mg, 6.02 mmol) was added, and the system was heated to 50°C for 1 h. Upon completion of the reaction, the reaction mixture was extracted with water and ethyl acetate. The aqueous phase was adjusted to pH 2 with 1M hydrochloric acid, extracted with ethyl acetate, dried over anhydrous sodium sulfate, and spun to dryness to give a product 1-cyclopropyl-1*H*-pyrazole-4-carboxylic acid (160 mg, yield: 87%). ES-API:[M+H]+= 153.1.

Step 3: tert-butyl (S)-2-(6-chloro-1,2,3,4-tetrahydroisoquinolin-8-yl)pyrrolidine-1-carboxylate (120 mg, 0.36 mmol) was dissolved in dichloromethane (5 mL). The 1-cyclopropyl-1*H*-pyrazole-4-carboxylic acid (160 mg, 1.05 mmol), 2-(7-azobenzotriazol)-N,N,N',N'-tetramethylurea hexafluorophosphate (271 mg, 0.71 mmol) and triethylamine (108 mg, 1.07 mmol) were added and stirred at room temperature for 2 h. Upon completion of the reaction, the reaction was quenched by the addition of water. The reaction mixture was extracted with dichloromethane, washed with saturated brine, dried over anhydrous sodium sulfate, and spun to dryness. The crude product was purified by a preparative thin layer chromatographic column (petroleum ether/ethyl acetate = 1/1) to give a product tert-butyl (S)-2-(6-chloro-2-(1-cyclopropyl-1*H*-pyrazole-4-carbonyl)-1,2,3,4-tetrahydroisoquinolin-8-yl)pyrrolidine-1-carboxylate (200 mg, crude product). (Rf = 0.4, petroleum ether/ethyl acetate = 1/1). ES-API:[M+H]+= 471.2.

Step 4: The tert-butyl (S)-2-(6-chloro-2-(1-cyclopropyl-1*H*-pyrazole-4-carbonyl)-1,2,3,4-tetrahydroisoquinolin-8-yl)pyrrolidine-1-carboxylate (200 mg, 0.42 mmol) was dissolved in dioxane/water (2 mL/0.4 mL). 3-Methyl-5-(4,4,5,5-tetramethyl-1,3,2-dioxaborolan-2-yl)-1H-pyrrolo[2,3-b]pyridine (131 mg, 0.51 mmol), chloro (2-dicyclohexylphosphino-2',6'-dimethoxy-1,1'-biphenylyl)(2'-amino-1,1'-biphenyl-2-yl) palladium (II) (30 mg, 0.042 mmol) and potassium carbonate (117 mg, 0.85 mmol) were added. The system was heated to 100°C for 2 h. Upon completion of the reaction, the reaction mixture was spun to dryness. The crude product was purified by a preparative thin layer chromatographic column (dichloromethane/methanol = 20/1) to give a product tert-butyl (S)-2-(2-(1-cyclopropyl-1*H*-pyrazole-4-carbonyl)-6-(3-methyl-1*H*-pyrrolo[2,3-b]pyridin-5 -yl)-1,2,3,4-tetrahydroisoquinolin-8-yl)pyrrolidine-1-carboxylate (100 mg, yield: 41%). ES-API:[M+1]⁺=567.3.

Step 5: The tert-butyl (S)-2-(2-(1-cyclopropyl-1*H*-pyrazole-4-carbonyl)-6-(3-methyl-1*H*-pyrrolo[2,3-b]pyridin-5 -yl)-1,2,3,4-tetrahydroisoquinolin-8-yl)pyrrolidine-1-carboxylate (100 mg, 0.18 mmol) was dissolved in dichloromethane (2 mL). Trifluoroacetic acid (1.5 mL) was added. The system reacted at room temperature for 1 h. Upon completion of the reaction, the reaction mixture was spun to dryness, extracted with dichloromethane and saturated aqueous sodium carbonate solution, dried over anhydrous sodium sulfate, and spun to dryness. The crude product was purified by a preparative thin layer chromatographic column (dichloromethane/methanol = 10/1) to give a product (S)-(1-cyclopropyl-1H-pyrazol-4-yl)(6-(3-methyl-1H-pyrrolo[2,3-b]pyridin-5-yl)-8-(pyrro lidin-2-yl)-3,4-dihydroisoquinolin-2(1H)-yl)methanone (Z319, 26.8 mg, yield: 32%). ES-API:[M+1]⁺=467.3. ¹H NMR (400 MHz, CDCl₃) δ 8.85 (s, 1H), 8.48 (s, 1H), 8.01 (s, 1H), 7.89 (s, 1H), 7.80-7.67 (m, 2H), 7.29 (s, 1H), 7.08 (s, 1H), 5.11-4.99 (m, 1H),4.93-4.71 (m, 1H),4.55-4.17 (m, 1H), 3.91 (s, 2H), 3.67-3.59 (m, 1H), 3.36-3.27 (m, 1H), 3.14-2.98 (m, 3H), 2.34 (s, 3H), 2.26-2.14 (m, 1H), 2.06-1.85 (m, 4H), 1.18-1.12 (m, 2H), 1.08-1.03 (m, 2H).

### Example 110. Synthesis of compound Z320

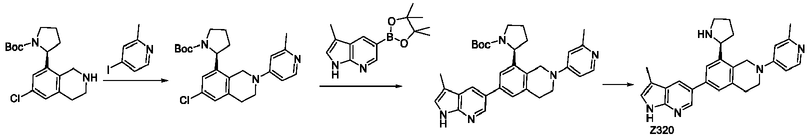

Step 1: tert-butyl (S)-2-(6-chloro-1,2,3,4-tetrahydroisoquinolin-8-yl)pyrrolidine-1-carboxylate (100 mg, 0.297 mmol) was dissolved in dried 1,4-dioxane (2 mL). 4-Iodo-2-methylpyridine (130.04 mg, 0.594 mmol), (2-dicyclohexylphosphino-2',6'-diisopropoxy-1,1'-biphenylyl)(2-amino-1,1'-biphenyl-2-y 1)palladium (II) mesylate (14.91 mg, 0.018 mmol), 2-dicyclohexylphosphine-2',6'-diisopropoxy-1,1'-biphenyl (27.70 mg, 0.059 mmol) and potassium carbonate (123.09 mg, 0.891 mmol) were added successively, and the system was heated to 140°C under microwave for 5 h. Upon completion of the reaction, the reaction mixture was diluted with ethyl acetate (30 mL), washed with saturated brine (30 mL), dried over anhydrous sodium sulfate, filtered, and concentrated. The crude product was purified by silica gel column chromatography (methanol/dichloromethane = 1/20) to give tert-butyl (S)-2-(6-chloro-2-(2-methylpyridin-4-yl)-1,2,3,4-tetrahydroisoquinolin-8-yl)pyrrolidine-1-carboxylate (80 mg, yield: 63%). ES-API: [M+H]⁺=428.1.

Step 2: The tert-butyl (S)-2-(6-chloro-2-(2-methylpyridin-4-yl)-1,2,3,4-tetrahydroisoquinolin-8-yl)pyrrolidine-1-carboxylate (80 mg, 0.193 mmol), 3-methyl-5-(4,4,5,5-tetramethyl-1,3,2-dioxaborolan-2-yl)-1H-pyrrolo[2,3-b]pyridine (72.38 mg, 0.280 mmol), chloro (2-dicyclohexylphosphino-2',6'-dimethoxy-1,1'-biphenylyl)(2'-amino-1,1'-biphenyl-2-yl) palladium (II) (13.47 mg, 0.019 mmol) and potassium carbonate (77.51 mg, 0.561 mmol) were added to 1,4-dioxane (3 mL) and water (0.5 mL). The system was replaced with nitrogen and reacted under microwave at 115°C for 1 h. Upon completion of the reaction, ethyl acetate (20 mL) was added. The obtained mixture was washed with water (10 mL) and saturated brine (10 mL) successively, and dried over anhydrous sodium sulfate. The crude product was purified by preparative thin layer chromatography (petroleum ether/ethyl acetate = 1/1) to give tert-butyl (S)-2-(6-(3-methyl-1H-pyrrolo[2,3-b]pyridin-5-yl)-2-(2-methylpyridin-4-yl))-1,2,3,4-tetra hydroisoquinolin-8-yl)pyrrolidine-1-carboxylate (60 mg, yield: 61.29%). ES-API: [M+H]⁺=524.3.

Step 3: The tert-butyl (S)-2-(6-(3-methyl-1H-pyrrolo[2,3-b]pyridin-5-yl)-2-(2-methylpyridin-4-yl))-1,2,3,4-tetra hydroisoquinolin-8-yl)pyrrolidine-1-carboxylate (60 mg, 0.115 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia-methanol solution (1 mL), the obtained mixture was concentrated again, and purified by prep-HPLC (formic acid method) to give (S)-6-(3-methyl-1H-pyrrolo[2,3-b]pyridin-5-yl)-2-(2-methylpyridin-4-yl)-8-(pyrrolidin-2-yl)-1,2,3,4-tetrahydroisoquinoline formate (Z320, 45 mg, yield: 92%). ES-API: [M+H]⁺=424.2. ¹H NMR (500 MHz, DMSO-*d*₆) δ 11.41 (d, *J* = 2.3 Hz, 1H), 8.59 (d, *J* = 2.2 Hz, 1H), 8.29 (s, 1H), 8.23 (d, *J* = 2.2 Hz, 1H), 8.13 (d, *J* = 6.2 Hz, 1H), 7.85 (d, *J* = 1.9 Hz, 1H), 7.64 (d, *J* = 1.8 Hz, 1H), 7.29 (dd, *J* = 2.3, 1.3 Hz, 1H), 7.00 (d, *J* = 2.5 Hz, 1H), 6.97 (dd, *J* = 6.4, 2.6 Hz, 1H), 4.88 (t, *J* = 8.2 Hz, 1H), 4.78 (d, *J* = 16.4 Hz, 1H), 4.63 (d, *J* = 16.4 Hz, 1H), 3.77 (dd, *J* = 12.3, 6.0 Hz, 1H), 3.65 (dt, *J* = 12.6, 6.2 Hz, 1H), 3.45 (q, *J* = 7.6, 6.0 Hz, 1H), 3.33 (s, 1H), 3.04 (d, *J=* 6.0 Hz, 2H), 2.42 (s, 3H), 2.32 (d, *J* = 1.0 Hz, 3H), 2.18 - 2.02 (m, 4H).

### Example 111. Synthesis of compound Z246

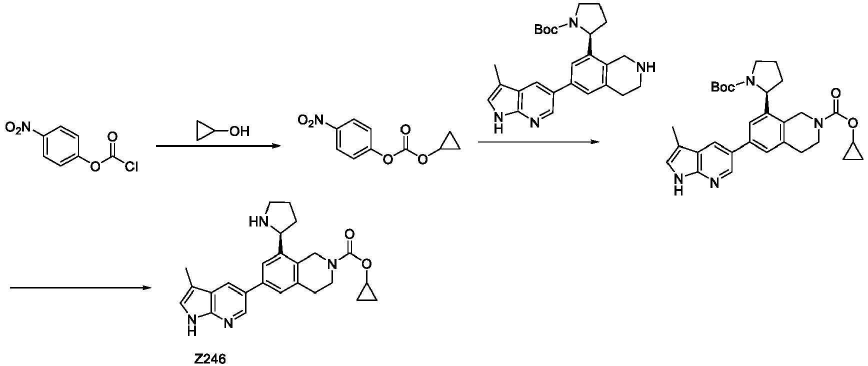

Step 1: Cyclopropanol (207.69 mg, 3.58 mmol) followed by pyridine (259.29 mg, 3.28 mmol) was added to (4-nitrophenyl)carbonyl chloride (600 mg, 2.98 mmol) at 0°C, and the mixture was stirred at 0°C for 1 h. The reaction was monitored by LCMS for completion. The reaction mixture was diluted with dichloromethane (5 mL) and 0.1M aqueous sulfuric acid solution (10 mL), and washed with saturated sodium bicarbonate solution (10 mL) and saturated brine (10 mL). The organic phase was dried over anhydrous sodium sulfate, filtered, and concentrated to a volume of about 5 mL. Cyclohexane (10 mL) was added. The obtained mixture was filtrated and concentrated. The crude product was purified by column chromatography (petroleum ether/ethyl acetate = 50/50) to give a product cyclopropyl(4-nitrophenyl) carbonate (450 mg, yield: 67.66%). ES-API:[M+H-100]⁺=224.1.

Step 2: The cyclopropyl(4-nitrophenyl) carbonate (15.48 mg, 0.069 mmol) and N,N-diisopropyl ethylenediamine (26.89 mg, 0.21 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in dichloromethane (1 mL), and the mixture was stirred at 25°C for 2 h. The reaction was monitored by LCMS for completion, and quenched by the addition of water. The reaction mixture was extracted with dichloromethane, dried over anhydrous sodium sulfate, and concentrated. The crude product was purified by column chromatography (petroleum ether/ethyl acetate = 50/50) to give a product (S)-cyclopropyl 8-(1-(tert-butoxycarbonyl)pyrrolidin-2-yl)-6-(3-methyl-1H-pyrrolo[2,3-b]pyridin-5-yl)-3,4 -dihydroisoquinoline-2(1H)-carboxylate (30 mg, yield: 83.73%). ES-API:[M+H]⁺= 517.3. Step 3: Trifluoroacetic acid (1 mL) was added to a solution of the (S)-cyclopropyl 8-(1-(tert-butoxycarbonyl)pyrrolidin-2-yl)-6-(3-methyl-1H-pyrrolo[2,3-b]pyridin-5-yl)-3,4 -dihydroisoquinoline-2(1H)-carboxylate (30 mg, 0.058 mmol) in dichloromethane (2 mL) and stirred at room temperature for 1 h. The reaction was monitored by LCMS for completion. The reaction mixture was adjusted to pH 7 with saturated sodium bicarbonate, treated with dichloromethane/water, dried over anhydrous sodium sulfate, and concentrated. The crude product was purified by prep-HPLC (column: Ultimate XB-C18, 50*250 mm, 10 um; mobile phase: A: purified water (0.05% ammonia water) B: pure acetonitrile; flow rate: 80 ml/min; gradient: B/A = 20%-90% over 50 min; wavelength: 214 nm; column temperature: room temperature) to give (S)-cyclopropyl 6-(3-methyl-1H-pyrrolo[2,3-b]pyridin-5-yl)-8-(pyrrolidin-2-yl)-3,4-dihydroisoquinoline-2 (1H)-carboxylate (Z246, 1.8 mg, yield: 7.44%) as a white powder. ES-API:[M+H]⁺=417.2.

### Example 112. Synthesis of compound Z285-1

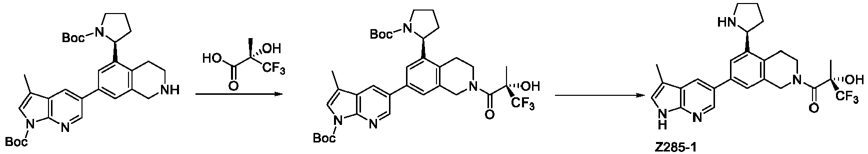

Step 1: tert-butyl (S)-5-(5-(1-(tert-butoxycarbonyl)pyrrolidin-2-yl)-1,2,3,4-tetrahydroisoquinolin-7-yl)-3-me thyl-1H-pyrrolo[2,3-b]pyridine-1-carboxylate (100 mg, 0.188 mmol) was dissolved in dichloromethane (5 mL). (S)-3,3,3-trifluoro-2-hydroxy-2-methylpropanoic acid (22.26 mg, 0.141 mmol), triethylamine (0.013 mL, 0.094 mmol) and 2-(7-azobenzotriazole)-N,N,N',N'-tetramethylurea hexafluorophosphate (35.69 mg, 0.094 mmol) were added successively, and the system reacted at room temperature for 1 h. Upon completion of the reaction, the reaction mixture was diluted with dichloromethane (30 mL), washed with saturated brine (30 mL), dried over anhydrous sodium sulfate, filtered, and concentrated. The crude product was purified by silica gel (petroleum ether/ethyl acetate = 1/1) to give tert-butyl 5-(5-((S)-1-(tert-butoxycarbonyl)pyrrolidin-2-yl)-2-((S)-3,3,3-trifluoro-2-hydroxy-2-meth ylpropanoyl)-1,2,3,4-tetrahydroisoquinolin-7-yl)-3-methyl-1H-pyrrolo[2,3-b]pyridine-1-c arboxylate (30 mg, yield: 47.51%). ES-API: [M+H]⁺=673.3.

Step 2: The tert-butyl 5-(5-((S)-1-(tert-butoxycarbonyl)pyrrolidin-2-yl)-2-((S)-3,3,3-trifluoro-2-hydroxy-2-meth ylpropanoyl)-1,2,3,4-tetrahydroisoquinolin-7-yl)-3-methyl-1H-pyrrolo[2,3-b]pyridine-1-c arboxylate (30 mg, 0.045 mmol) was dissolved in dichloromethane (3 mL) and added to trifluoroacetic acid (2 mL), and the system reacted at room temperature for 0.5 h. Upon completion of the reaction, the reaction mixture was concentrated. After neutralization with ammonia-methanol solution (1 mL), the obtained mixture was concentrated again and purified by prep-HPLC (ammonia water method) to give (S)-3,3,3-trifluoro-2-hydroxy-2-methyl-1-(7-(3-methyl-1H-pyrrolo[2,3-b]pyridin-5-yl)-5-( (S)-pyrrolidin-2-yl)-3,4-dihydroisoquinolin-2(1H)-yl)propan-1-one (Z285-1, 9 mg, yield: 42.75%). ES-API: [M+H]⁺=473.2. ¹H NMR (500 MHz, DMSO-*d*₆) δ 11.34 (s, 1H), 8.47 (d, *J* = 2.1 Hz, 1H), 8.12 (d, *J* = 2.2 Hz, 1H), 7.77 (s, 1H), 7.44 (s, 1H), 7.26 (d, *J* = 2.1 Hz, 1H), 5.17 (s, 1H), 4.85 - 4.62 (m, 2H), 4.26 (t, *J* = 7.7 Hz, 2H), 4.01 (s, 1H), 3.75 (s, 1H), 3.13 - 3.09 (m, 1H), 2.94 (q, *J* = 8.1 Hz, 2H), 2.31 (d, *J =* 1.1 Hz, 3H), 2.20 (dt, *J* = 12.6, 6.3 Hz, 1H), 2.05-1.97 (m, 1H), 1.85-1.75 (, 2H).

### Example 123. Synthesis of compound Z151-1

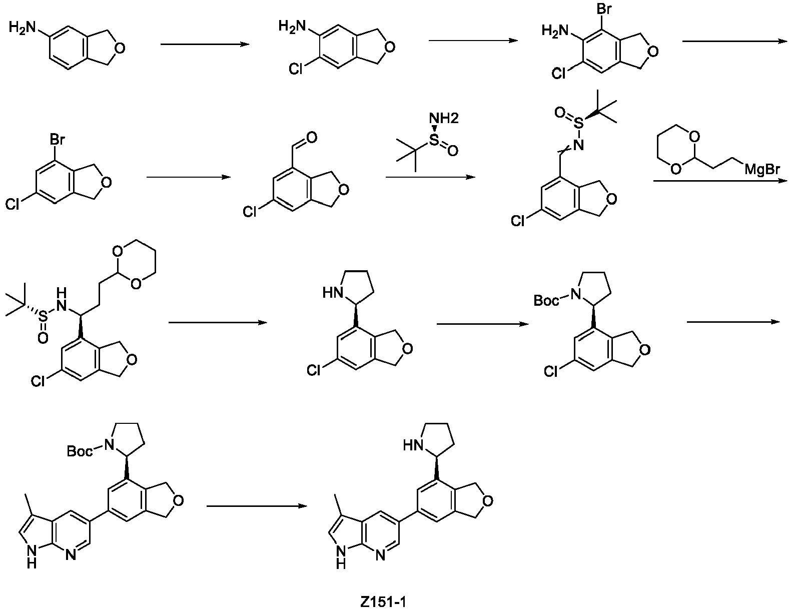

Step 1: 1,3-Dihydroisobenzofuran-5-amine (1.8 g, 13.317 mmol) and N-chlorosuccinimide (2.66 g, 19.975 mmol) were dissolved in N,N-dimethylformamide (20 mL) and stirred at room temperature for 1 h. Ethyl acetate (100 mL) was added. The obtained mixture was washed with water (100 mL x 3) and concentrated. The crude product was purified by silica gel column chromatography (tetrahydrofuran/petroleum ether = 10/90) to give 6-chloro-1,3-dihydroisobenzofuran-5-amine (550 mg, yield: 24.35%). ES-API: [M+H]⁺=170.0.

Step 2: The 6-chloro-1,3-dihydroisobenzofuran-5-amine (550 mg, 3.243 mmol) was dissolved in acetonitrile (30 mL) solution. N-bromosuccinimide (634.86 mg, 3.567 mmol) was added. The mixture was stirred at room temperature for 1 h. Ethyl acetate (100 mL) was added. The obtained mixture was washed with saturated sodium chloride solution (100 mL x 1), dried over anhydrous sodium sulfate, filtered, and concentrated. The crude product was purified by column chromatography (tetrahydrofuran/petroleum ether = 10/90) to give 4-bromo-6-chloro-1,3-dihydroisobenzofuran-5-amine (480 mg, yield: 59.57%). ES-API: [M+H]⁺=248.0, 250.0.

Step 3: The 4-bromo-6-chloro-1,3-dihydroisobenzofuran-5-amine (480 mg, 1.932 mmol) was dissolved in a mixed solvent of tetrahydrofuran (5 mL) and water (2 mL). Hypophosphorous acid (5 mL) and sodium nitrite (213.25 mg, 3.091 mmol) were added successively, and the system reacted at room temperature for 2 h. The obtained mixture was poured into water (10 mL), and extracted with ethyl acetate (20 mL x 3). The ethyl acetate layers were combined, washed with saturated aqueous sodium bicarbonate solution (30 mLx1) and saturated sodium chloride solution (30 mL x 1) successively, dried over anhydrous sodium sulfate, filtered, and concentrated. The crude product was purified by column chromatography (tetrahydrofuran/petroleum ether = 10/90) to give 4-bromo-6-chloro-1,3-dihydroisobenzofuran (210 mg, yield: 46.7%). ES-API: [M+H]⁺=233.0, 235.0.

Step 4: The 4-bromo-6-chloro-1,3-dihydroisobenzofuran (210 mg, 0.90 mmol) was dissolved in dried tetrahydrofuran (15 mL), and the system was cooled to -65°C in a dry ice bath. n-Butyllithium (0.514 mL, 1.285 mmol) was slowly added and stirred at -65°C for 10 min. The reaction was quenched by dropwise addition of saturated aqueous ammonium chloride solution (1 mL). Ethyl acetate (30 mL) was added. The obtained mixture was washed with water (30 mLx1) and saturated sodium chloride solution (30 mL x 1), dried over anhydrous sodium sulfate, filtered, and concentrated. The crude product was purified by column chromatography (petroleum ether/ethyl acetate = 20/80) to give 6-chloro-1,3-dihydroisobenzofuran-4-carbaldehyde (120 mg, yield: 73.2%). ES-API: [M+H]⁺=183.0.

Step 5: The 6-chloro-1,3-dihydroisobenzofuran-4-carbaldehyde (120 mg, 0.659 mmol) and (S)-2-methylpropane-2-sulfinamide (159.5 mg, 1.318 mmol) were dissolved in dried dichloromethane (10 mL). Tetraethyl titanate (0.599 mL, 2.629 mmol) was added at room temperature and stirred for 1 h. The obtained mixture was poured into brine (30 mL) and extracted with dichloromethane (50 mL x 3). The organic phases were combined, filtered, washed with saturated sodium chloride solution (100 mL x 1), dried over anhydrous sodium sulfate, filtered, and concentrated. The crude product was purified by column chromatography (petroleum ether/tetrahydrofuran = 30/70) to give (S)-N-((6-chloro-1,3-dihydroisobenzofuran-4-yl)methylene)-2-methylpropane-2-sulfinami de (63 mg, yield: 33.54%). ES-API: [M+H]⁺=286.1.

Step 6: The (S)-N-((6-chloro-1,3-dihydroisobenzofuran-4-yl)methylene)-2-methylpropane-2-sulfinami de (63 mg, 0.220 mmol) was dissolved in dried tetrahydrofuran (10 mL). (2-(1,3-Dioxan-2-yl)ethyl)magnesium bromide (1.76 mL, 0.88 mmol) was slowly added under a dry ice bath condition at -78°C. The mixture was stirred at room temperature for 0.5 h. The reaction was quenched by the addition of aqueous ammonium chloride solution (5 mL), and the reaction mixture was extracted with ethyl acetate (10 mLx2). The organic phases were combined, washed with saturated sodium chloride solution (100 mL x 1), dried over anhydrous sodium sulfate, filtered, and concentrated. The crude product was purified by column chromatography (petroleum ether/tetrahydrofuran = 30/70) to give (*S*)-N-((*S*)-1-(6-chloro-1,3-dihydroisobenzofuran-4-yl)-3-(1,3-dioxan-2-yl)propyl)-2-meth ylpropane-2-sulfinamide (70 mg, yield: 79.00%). ES-API: [M+H]⁺=402.1.

Step 7: The (*S*)-N-((*S*)-1-(6-chloro-1,3-dihydroisobenzofuran-4-yl)-3-(1,3-dioxan-2-yl)propyl)-2-meth ylpropane-2-sulfinamide (70 mg, 0.174 mmol) was dissolved in trifluoroacetic acid (3 mL), and water (0.15 mL) was added. The mixture was stirred at room temperature for 0.5 h. Triethylsilane (202.3 mg, 1.74 mmol) was added. The mixture was stirred at room temperature for 3 h. The reaction mixture was concentrated to give (S)-2-(6-chloro-1,3-dihydroisobenzofuran-4-yl)pyrrolidine (40 mg, crude product). ES-API: [M+H]⁺=224.0.

Step 8: The (S)-2-(6-chloro-1,3-dihydroisobenzofuran-4-yl)pyrrolidine (40 mg, crude product) was dissolved in dichloromethane (20 mL). Triethylamine (87.8 mg, 0.87 mmol) and di-tert-butyl dicarbonate (75 mg, 0.348 mmol) were added. The mixture was stirred at room temperature for 0.5 h. The reaction mixture was concentrated. The crude product was purified by silica gel column chromatography (ethyl acetate/petroleum ether = 40/60) to give tert-butyl (S)-2-(6-chloro-1,3-dihydroisobenzofuran-4-yl)pyrrolidine-1-carboxylate (46 mg, yield of the 2 steps: 81.6%). ES-API: [M+H]⁺=324.1.

Step 9: The tert-butyl (S)-2-(6-chloro-1,3-dihydroisobenzofuran-4-yl)pyrrolidine-1-carboxylate (46 mg, 0.142 mmol), 3-methyl-5-(4,4,5,5-tetramethyl-1,3,2-dioxaborolan-2-yl)-1H-pyrrolo[2,3-b]pyridine (55.00 mg, 0.213 mmol), chloro (2-dicyclohexylphosphino-2',6'-dimethoxy-1,1'-biphenylyl)(2'-amino-1,1'-biphenyl-2-yl) palladium (II) (10.24 mg, 0.014 mmol) and potassium carbonate (58.90 mg, 0.426 mmol) were added to 1,4-dioxane (2 mL) and water (0.5 mL). The system was replaced with nitrogen and heated to 115°C under microwave for 1.5 h. Upon completion of the reaction, ethyl acetate (10 mL) was added. The obtained mixture was washed with water (10 mL x 1) and saturated brine (10 mL x 1), dried over anhydrous sodium sulfate, and concentrated. The crude product was purified by silica gel column chromatography (ethyl acetate/petroleum ether = 80/20) to give tert-butyl (S)-2-(6-(3-methyl-1H-pyrrolo[2,3-b]pyridin-5-yl)-1,3-dihydroisobenzofuran-4-yl)pyrroli dine-1-carboxylate (55 mg, yield: 92.2%). ES-API: [M+H]⁺=420.2.

Step 10: The tert-butyl (S)-2-(6-(3-methyl-1H-pyrrolo[2,3-b]pyridin-5-yl)-1,3-dihydroisobenzofuran-4-yl)pyrroli dine-1-carboxylate (55 mg, 0.131 mmol) was dissolved in dichloromethane (3 mL) and added to trifluoro (2 mL), and the system reacted at room temperature for 0.5 h. Upon completion of the reaction, the reaction mixture was concentrated. After neutralization with ammonia-methanol solution (1 mL), the obtained mixture was concentrated again, and purified by prep-HPLC (formic acid method) to give (S)-3-methyl-5-(7-(pyrrolidin-2-yl)-1,3-dihydroisobenzofuran-5-yl)-1H-pyrrolo[2,3-b]pyri dine (Z151-1, 25 mg, yield: 59.8%). ES-API: [M+H]⁺=320.2. ¹H NMR (500 MHz, DMSO-*d*₆) δ 11.41 - 11.36 (m, 1H), 8.51 (d, *J* = 2.1 Hz, 1H), 8.33 (s, 1H), 8.17 (d, *J* = 2.1 Hz, 1H), 7.72 (s, 1H), 7.58 (s, 1H), 7.28 (s, 1H), 5.14 (s, 2H), 5.07 (s, 2H), 4.25 (dd, *J =* 9.4, 7.0 Hz, 1H), 3.24-3.19 (m, 1H), 3.10-3.05 (m, 1H), 2.31 (s, 3H), 2.23 (dtd, *J* = 11.8, 7.4, 3.7 Hz, 1H), 1.97-1.92 (m, 1H), 1.90-1.82 (m, 1H), 1.78 (dt, *J=* 12.1, 8.8 Hz, 1H).

### Example 124. Synthesis of compound Z247

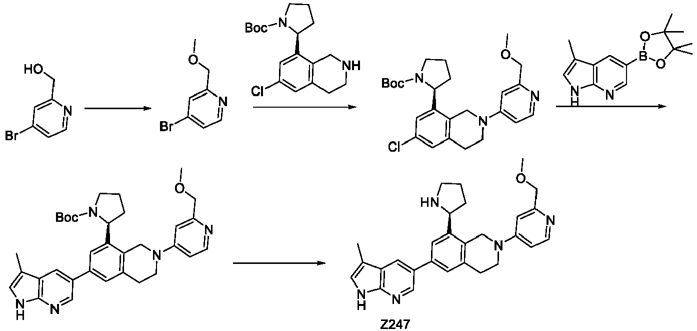

Step 1: Sodium hydride (319.11 mg, 7.978 mmol)was added to a solution of (4-bromopyridin-2-yl)methanol (500 mg, 2.659 mmol) in N,N-dimethylformamide (10 mL) under an ice-water bath condition. The mixture was stirred at 0°C for 10 min. Iodomethane (0.248 mL, 3.989 mmol) was slowly added and stirred at room temperature for 1 h. The obtained mixture was poured into water (100 mL), extracted with ethyl acetate (100 mL x 3), washed with saturated sodium chloride solution (100 mL x 1), dried over anhydrous sodium sulfate, filtered, and concentrated. The crude product was purified by column chromatography (petroleum ether/ethyl acetate = 80/20) to give 4-bromo-2-(methoxymethyl)pyridine (350 mg, yield: 65.14%). ES-API: [M+H]⁺=201.9, 203.9.

Step 2: tert-butyl (S)-2-(6-chloro-1,2,3,4-tetrahydroisoquinolin-8-yl)pyrrolidine-1-carboxylate (30 mg, 0.089 mmol) was dissolved in dried 1,4-dioxane (2 mL). The 4-bromo-2-(methoxymethyl) pyridine (53.98 mg, 0.267 mmol), (2-dicyclohexylphosphino-2',6'-diisopropoxy-1,1'-biphenylyl)(2-amino-1,1'-biphenyl-2-y 1)palladium (II) mesylate (14.91 mg, 0.018 mmol), 2-dicyclohexylphosphine-2',6'-diisopropoxy-1,1'-biphenyl (8.31 mg, 0.018 mmol) and potassium carbonate (36.93 mg, 0.267 mmol) were added successively, and the system was heated to 140°C under microwave for 5 h. Upon completion of the reaction, the reaction mixture was diluted with ethyl acetate (30 mL), washed with saturated brine (30 mL), dried over anhydrous sodium sulfate, filtered, and concentrated. The crude product was purified by silica gel column chromatography (methanol/dichloromethane = 1/20) to give tert-butyl (S)-2-(6-chloro-2-(2-(methoxymethyl)pyridin-4-yl)-1,2,3,4-tetrahydroisoquinolin-8-yl)pyr rolidine-1-carboxylate (25 mg, yield: 61.29%). ES-API: [M+H]⁺=458.2.

Step 3: The tert-butyl (S)-2-(6-chloro-2-(2-(methoxymethyl)pyridin-4-yl)-1,2,3,4-tetrahydroisoquinolin-8-yl)pyr rolidine-1-carboxylate (25 mg, 0.055 mmol), 3-methyl-5-(4,4,5,5-tetramethyl-1,3,2-dioxaborolan-2-yl)-1H-pyrrolo[2,3-b]pyridine (28.18 mg, 0.109 mmol), chloro (2-dicyclohexylphosphino-2',6'-dimethoxy-1,1'-biphenylyl)(2'-amino-1,1'-biphenyl-2-yl) palladium (II) (7.87 mg, 0.011 mmol) and potassium carbonate (22.63 mg, 0.164 mmol) were added to 1,4-dioxane (3 mL) and water (0.5 mL). The system was replaced with nitrogen and reacted under microwave at 115°C for 1 h. Upon completion of the reaction, ethyl acetate (20 mL) was added. The obtained mixture was washed with water (10 mL) and saturated brine (10 mL) successively, and dried over anhydrous sodium sulfate. The crude product was purified by preparative thin layer chromatography (petroleum ether/ethyl acetate = 1/1) to give tert-butyl (S)-2-(6-(3-methyl-1H-pyrrolo[2,3-b]pyridin-5-yl)-2-(2-(methoxymethyl)pyridin-4-yl)-1,2 ,3,4-tetrahydroisoquinolin-8-yl)pyrrolidine-1-carboxylate (22 mg, yield: 72.16%). ES-API: [M+H]⁺=554.3.

Step 4: The tert-butyl (S)-2-(6-(3-methyl-1H-pyrrolo[2,3-b]pyridin-5-yl)-2-(2-(methoxymethyl)pyridin-4-yl)-1,2 ,3,4-tetrahydroisoquinolin-8-yl)pyrrolidine-1-carboxylate (22 mg, 0.036 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia/methanol solution (1 mL), the obtained mixture was concentrated again, and purified by prep-HPLC (ammonium bicarbonate method) to give (S)-2-(2-(methoxymethyl)pyridin-4-yl)-6-(3-methyl-1H-pyrrolo[2,3-b]pyridin-5-yl)-8-(pyr rolidin-2-yl)-1,2,3,4-tetrahydroisoquinoline (Z247, 9 mg, yield: 44.4%). ES-API: [M+H]⁺=454.2. ¹H NMR (500 MHz, DMSO-*d*₆) δ 11.35 (s, 1H), 8.48 (d, *J* = 2.1 Hz, 1H), 8.16 - 8.10 (m, 2H), 7.76 (d, *J* = 2.0 Hz, 1H), 7.46 (d, *J* = 1.8 Hz, 1H), 7.27 (s, 1H), 6.92 (d, *J* = 2.6 Hz, 1H), 6.84 (dd, *J* = 6.1, 2.6 Hz, 1H), 4.73 - 4.53 (m, 3H), 4.43 (d, *J* = 8.6 Hz, 1H), 4.38 (s, 2H), 3.66 (q, *J* = 6.0 Hz, 2H), 3.36 (d, *J* = 1.6 Hz, 3H), 3.17 (s, 1H), 3.03 (d, *J* = 6.4 Hz, 3H), 2.31 (d, *J* = 1.1 Hz, 3H), 2.03 - 1.97 (m, 1H), 1.95 - 1.79 (m, 3H).

### Example 125. Synthesis of compound Z88-1

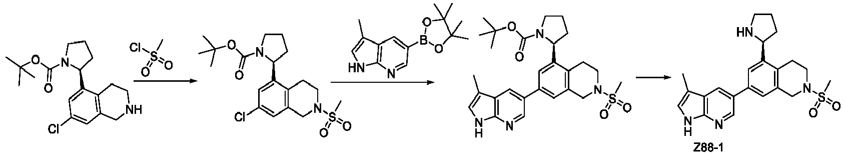

Step 1: N,N-Diisopropylethyl amine (230 mg, 1.78 mmol) and methanesulfonyl chloride (68 mg, 0.59 mmol) were added to a solution of tert-butyl (S)-2-(7-chloro-1,2,3,4-tetrahydroisoquinolin-5-yl)pyrrolidine-1-carboxylate (200 mg, 0.59 mmol) in dichloromethane (2 mL) and stirred at room temperature for 1 h. Upon completion of the reaction, the reaction mixture was concentrated to give a crude product. The crude product was purified by silica gel column chromatography (tetrahydrofuran/petroleum ether = 0-80%) to give tert-butyl (S)-2-(7-chloro-2-(methylsulfonyl)-1,2,3,4-tetrahydroisoquinolin-5-yl)pyrrolidine-1-carbo xylate (55 mg, yield: 22%). ES-API: [M+H]⁺ = 437.1.

Step 2: In the presence of protective nitrogen, a mixed solution of the tert-butyl (S)-2-(7-chloro-2-(methylsulfonyl)-1,2,3,4-tetrahydroisoquinolin-5-yl)pyrrolidine-1-carbo xylate (50 mg, 0.12 mmol), 3-methyl-5-(4,4,5,5-tetramethyl-dioxaborolan-2-yl)-1*H*-pyrrolo[2,3-*b*]pyridine (37 mg, 0.14 mmol), chloro (2-dicyclohexylphosphino-2',6'-dimethoxy-1,1'-biphenylyl)(2'-amino-1,1'-biphenyl-2-yl) palladium (II) (9 mg, 12 µmol), 2-dicyclohexylphosphine-2',6'-dimethoxybiphenyl (5 mg, 12 µmol) and potassium carbonate (50 mg, 0.36 mmol) in 1,4-dioxane (2 mL) and water (0.4 mL) was stirred at 120°C for 2 h. Ethyl acetate (10 mL) was poured into the reaction mixture, and the obtained mixture was washed with saturated brine (5 mL) and water (5 mL) successively. The organic phase was dried over anhydrous sodium sulfate, filtered, and concentrated to give a crude product. The crude product was purified by silica gel column chromatography (0-10% methanol/dichloromethane) to give tert-butyl (S)-2-(7-(3-methyl-1H-pyrrolo[2,3-b]pyridin-5-yl)-2-(methylsulfonyl)-1,2,3,4-tetrahydrois oquinolin-5-yl)pyrrolidine-1-carboxylate (40 mg, yield: 65%) as a clear oily liquid. ES-API: [M+H]⁺ = 511.3.

Step 3: Trifluoroacetic acid (0.5 mL) was added dropwise to a solution of the tert-butyl (S)-2-(7-(3-methyl-1H-pyrrolo[2,3-b]pyridin-5-yl)-2-(methylsulfonyl)-1,2,3,4-tetrahydrois oquinolin-5-yl)pyrrolidine-1-carboxylate (40 mg, 78 µmol) in dichloromethane (1 mL) under an ice bath condition and stirred at room temperature for 1 h. The reaction mixture was concentrated to give a crude product. The product was purified by preparative chromatographic column (ammonium bicarbonate method) to give (S)-7-(3-methyl-1H-pyrrolo[2,3-b]pyridin-5-yl)-2-(methylsulfonyl)-5-(pyrrolidin-2-yl)-1,2 ,3,4-tetrahydroisoquinoline (Z88-1, 17 mg, purity: 100%, yield: 53%) as a white solid. ES-API: [M+H]⁺ = 411.1. ¹H NMR (500 MHz, DMSO-*d₆*) δ 11.39 (s, 1H), 8.59 (d, *J=* 2.0 Hz, 1H), 8.23 (d, *J* = 2.0 Hz, 1H), 7.82 (s, 1H), 7.67 (s, 1H), 7.29 (d, *J* = 1.0 Hz, 1H), 4.78 - 4.71 (m, 1H), 4.49 (s, 2H), 3.51 (t, *J* = 6.0 Hz, 2H), 3.49 - 3.42 (m, 1H), 3.38 - 3.33 (m, 1H), 3.11 - 3.00 (m, 2H), 2.99 (s, 3H), 2.45 - 2.36 (m, 1H), 2.32 (s, 3H), 2.21 - 2.01 (m, 3H).

### Example 126. Synthesis of compound Z340

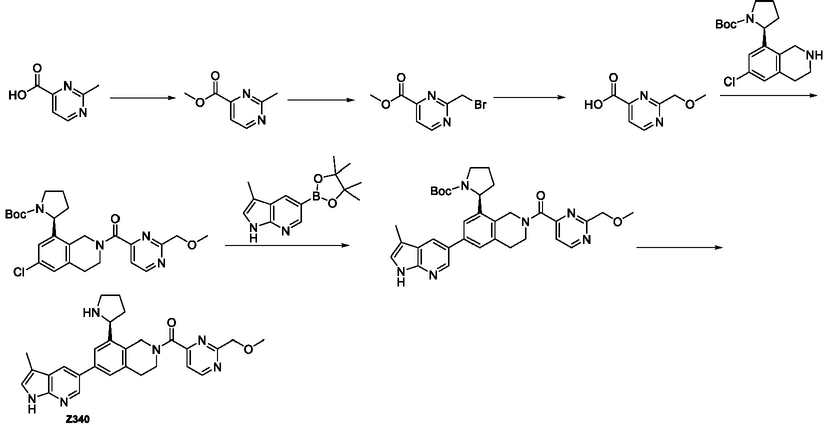

Step 1: Thionyl chloride (0.8 mL, 10.9 mmol) was added to a solution of 2-methylpyrimidine-4-carboxylic acid (1.0 g, 7.2 mmol) in methanol (20 mL) and stirred at 70°C for 16 h. The reaction mixture was concentrated. A saturated aqueous sodium bicarbonate solution was added, and the obtained mixture was extracted with ethyl acetate. The organic layer was dried over anhydrous sodium sulfate, filtered, and concentrated in vacuum to give methyl 2-methylpyrimidine-4-carboxylate (0.6 g, crude product) as a yellow solid. ES-API:[M+H]⁺=153.10.

Step 2: N-Bromosuccinimide (701.9 mg, 3.9 mmol) and 2,2'-azobis(2-methylpropionitrile) (64.8 mg, 0.4 mmol)were added to a solution of the methyl 2-methylpyrimidine-4-carboxylate (0.6 g, 3.9 mmol) in carbon tetrachloride (12 mL) and stirred at 80°C for 40 h. The reaction mixture was concentrated, and purified by silica gel column chromatography (petroleum ether:ethyl acetate = 1:1) to give methyl 2-(bromomethyl)pyrimidine-4-carboxylate (130.0 mg, yield: 14.27%) as a yellow oil. ES-API:[M+H]⁺=230.9/233.0.

Step 3: In the presence of protective nitrogen, sodium methoxide (60.8 mg, 1.2 mmol) was added to a solution of methyl 2-(bromomethyl)pyrimidine-4-carboxylate (0.13 g, 0.6 mmol) in methanol (8 mL) and stirred at 25°C for 16 h. The mixture was quenched with hydrochloric acid (1N), and extracted with dichloromethane:methanol (10:1). The combined organic layer was washed with brine, dried over anhydrous sodium sulfate, filtered, and concentrated to give a product 2-(methoxymethyl)pyrimidine-4-carboxylic acid (0.1 g, crude product) as a yellow solid. ES-API:[M+H]⁺=169.1.

Step 4: The 2-(methoxymethyl)pyrimidine-4-carboxylic acid (69.9 mg, 0.4 mmol), 1-propylphosphonic anhydride (254.4 mg, 0.4 mmol, 50% in ethyl acetate) and triethylamine (72.1 mg, 0.7 mmol) were added to a solution of tert-butyl (S)-2-(6-chloro-1,2,3,4-tetrahydroisoquinolin-8-yl)pyrrolidine-1-carboxylate (0.08 g, 0.2 mmol) in dichloromethane (8 mL), and stirred at 25°C for 2 h. Water was added to the mixture, and the reaction mixture was extracted with ethyl acetate. The organic layer was dried over anhydrous sodium sulfate, filtered, concentrated, and purified by a preparative thin layer chromatographic column (petroleum ether:ethyl acetate = 1:2) to give tert-butyl (*S*)-2-[6-chloro-2-[2-(methoxymethyl)pyrimidine-4-carbonyl]-1,2,3,4-tetrahydroisoquinoli n-8-yl]pyrrolidine-1-carboxylate (85.0 mg, yield: 73.5%) as a yellow oil. ES-API:[M+H-100]⁺=387.30.

Step 5: 3-Methyl-5-(4,4,5,5-tetramethyl-1,3,2-dioxaborolan-2-yl)-1H-pyrrolo[2,3-b]pyridine (54.1 mg, 0.2 mmol), potassium carbonate (72.3 mg, 0.5 mmol) and chloro (2-dicyclohexylphosphino-2',6'-dimethoxy-1,1'-biphenylyl)(2'-amino-1,1'-biphenyl-2-yl) palladium (II) (12.6 mg, 17.4 µmol) were added to a solution of the tert-butyl (*S*)-2-[6-chloro-2-[2-(methoxymethyl)pyrimidine-4-carbonyl]-1,2,3,4-tetrahydroisoquinoli n-8-yl]pyrrolidine-1-carboxylate (0.085 g, 0.2 mmol) in 1,4-dioxane (4 mL) and water (0.8 mL). The system was heated to 100°C for 1 h. Upon completion of the reaction, the reaction mixture was spun to dryness and purified by a preparative thin layer chromatographic column (ethyl acetate) to give a product tert-butyl (*S*)-2-[2-[2-(methoxymethyl)pyrimidine-4-carbonyl]-6-(3-methyl-1*H*-pyrrolo[2,3-b]pyridi n-5-yl)-1,2,3,4-tetrahydroisoquinolin-8-yl]pyrrolidine-1-carboxylate (65.0 mg, yield: 63.91%) as a yellow oil. ES-API:[M+H-100]⁺=583.4.

Step 6: The tert-butyl (*S*)-2-[2-[2-(methoxymethyl)pyrimidine-4-carbonyl]-6-(3-methyl-1*H*-pyrrolo[2,3-*b*]pyridi n-5-yl)-1,2,3,4-tetrahydroisoquinolin-8-yl]pyrrolidine-1-carboxylate (65 mg, 0.1 mmol) was dissolved in dichloromethane (2 mL). Trifluoroacetic acid (2 mL) was added. The system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, concentrated, spun to dryness, and purified by a preparative thin layer chromatographic column (dichloromethane/methanol = 10/1) to give a product (*S*)-[2-(methoxymethyl)pyrimidin-4-yl]-[6-(3-methyl-1*H*-pyrrolo[2,3-*b*]pyridin-5-yl)-8-[p yrrolidin-2-yl]-3,4-dihydroisoquinolin-2(1H)-yl]methanone (Z340, 30.3 mg, yield: 56.29%). ES-API:[M+1]⁺=483.3. ¹H NMR (400 MHz, CDCl₃) δ 9.15 (s, 1H), 8.94-8.92 (m, 1H), 8.51-8.46 (m, 1H), 8.03-7.96 (m, 1H), 7.80 (d, *J* = 1.6 Hz, 1H), 7.58-7.51 (m, 1H), 7.32-7.26 (m, 1H), 7.09 (s, 1H), 5.15-4.86 (m, 2H),4.78-4.75 (m, 2H), 4.42 (t, *J* = 7.6 Hz, 1H), 4.10-3.99 (m, 1H), 3.80-3.72 (m, 1H), 3.57 (s, 3H), 3.34-3.25 (m, 1H), 3.17-3.01 (m, 3H), 2.37-2.30 (m, 3H), 2.04-1.77 (m, 4H).

### Example 127. Synthesis of compound Z355

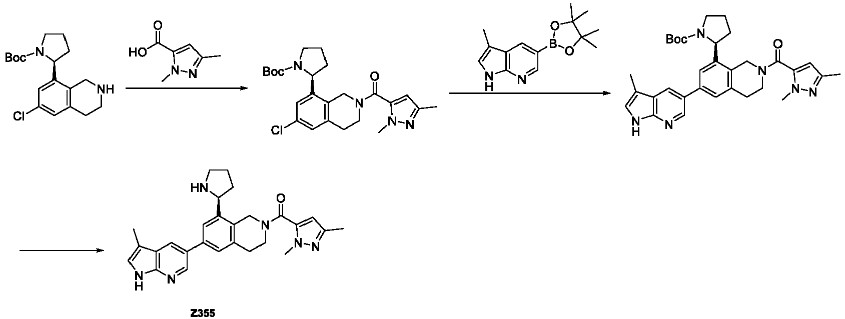

Step 1: 2,5-Dimethylpyrazole-3-carboxylic acid (58.8 mg, 0.42 mmol), 1-propylphosphonic anhydride (197.2 mg, 0.31 mmol, 50% in ethyl acetate) and triethylamine (63.08 mg, 0.62 mmol) were added to a solution of tert-butyl (S)-2-(6-chloro-1,2,3,4-tetrahydroisoquinolin-8-yl)pyrrolidine-1-carboxylate (70 mg, 0.21 mmol) in dichloromethane (6 mL), and the mixture was stirred at 25°C for 2 h. The reaction was monitored by LCMS for completion, and quenched by the addition of water. The reaction mixture was extracted with dichloromethane, dried over anhydrous sodium sulfate, concentrated, and purified by a preparative thin layer chromatographic column (petroleum ether/ethyl acetate = 1/1) to give a product tert-butyl (*S*)-2-[6-chloro-2-(2,5-dimethylpyrazole-3-carbonyl)-1,2,3,4-tetrahydroisoquinolin-8-yl]p yrrolidine-1-carboxylate (80.00 mg, yield: 83.88%) as a yellow oil. ES-API:[M+1]⁺=459.2.

Step 2: The tert-butyl (S)-2-[6-chloro-2-(2,5-dimethylpyrazole-3-carbonyl)-1,2,3,4-tetrahydroisoquinolin-8-yl]p yrrolidine-1-carboxylate (80 mg, 0.17 mmol) was dissolved in dioxane/water (4 mL/0.0.8 mL). 3-Methyl-5-(4,4,5,5-tetramethyl-1,3,2-dioxaborolan-2-yl)-1*H*-pyrrolo[2,3-*b*]pyridine (53.99 mg, 0.21 mmol), chloro (2-dicyclohexylphosphino-2',6'-dimethoxy-1,1'-biphenylyl)(2'-amino-1,1'-biphenyl-2-yl) palladium (II) (12.55 mg, 17.43 µmol) and potassium carbonate (72.16 mg, 0.52 mmol) were added. The system was heated to 100°C for 1 h. Upon completion of the reaction, the reaction mixture was spun to dryness and purified by a preparative thin layer chromatographic column (dichloromethane/methanol = 10/1) to give a product tert-butyl (*S*)-2-[2-(2,5-dimethylpyrazole-3-carbonyl)-6-(3-methyl-1*H*-pyrrolo[2,3-*b*]pyridin-5-yl)-1, 2,3,4-tetrahydroisoquinolin-8-yl]pyrrolidine-1-carboxylate (80.00 mg, yield: 82.75%). ES-API:[M+1]⁺=555.3.

Step 3: The (*S*)-2-[2-(2,5-dimethylpyrazole-3-carbonyl)-6-(3-methyl-1*H*-pyrrolo[2,3-*b*]pyridin-5-yl)-1, 2,3,4-tetrahydroisoquinolin-8-yl]pyrrolidine-1-carboxylate (80 mg, 0.14 mmol) was dissolved in dichloromethane (2.0 mL). Trifluoroacetic acid (2.0 mL) was added. The system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated. The crude product was purified by a preparative thin layer chromatographic column (dichloromethane/methanol = 10/1) to give a product (S)-(1,3-dimethyl-1H-pyrazol-5-yl)(6-(3-methyl-1H-pyrrolo[2,3-b]pyridin-5-yl)-8-(pyrroli din-2-yl)-3,4-dihydroisoquinolin-2(1H)-yl)methanone (Z355, 27.00 mg, yield: 41.18%) as a yellow solid. ES-API:[M+1]⁺= 455.3. ¹H NMR (400 MHz, CDCl₃) δ 9.40 (s, 1H), 8.50 (s, 1H), 8.03 (s, 1H), 7.84-7.66 (m, 1H),7.30 (s, 1H), 7.11 (s, 1H), 6.18 (s, 1H), 5.13-4.99 (m, 0.5H), 4.91-4.83 (m, 1H), 4.45-4.35 (m, 0.5H),3.97-3.80 (m, 5H), 3.36 -2.92 (m, 4H), 2.35 (s, 3H), 2.29 (s, 3H), 2.21-1.78 (m, 4H), 1.73-1.55 (m, 1H).

### Example 128. Synthesis of compound Z346

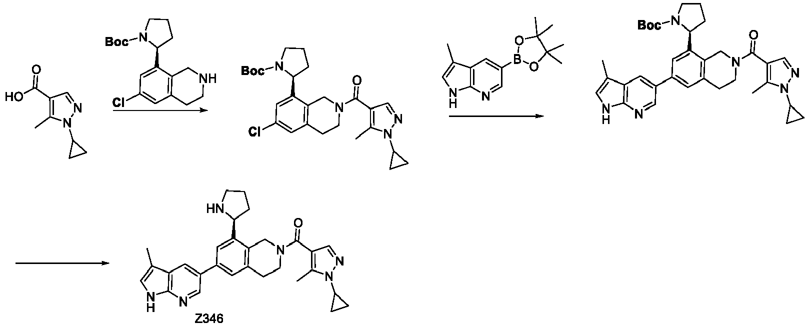

Step 1: 1-Cyclopropyl-3-methyl-pyrazole-4-carboxylic acid (35.52 mg, 213.74 µmol), 1-propylphosphonic anhydride (169.8 mg, 267.18 µmol, 50% in ethyl acetate) and triethylamine (0.05 mL) were added to a solution of tert-butyl (S)-2-(6-chloro-1,2,3,4-tetrahydroisoquinolin-8-yl)pyrrolidine-1-carboxylate (60 mg, 178.12 µmol) in dichloromethane (2 mL), and the mixture was stirred at 20°C for 2 h. The reaction mixture was spun to dryness to remove a solvent and obtain a crude product. The crude product was purified by silica gel column chromatography (petroleum ether/ethyl acetate = 1/1) to give tert-butyl (S)-2-[6-chloro-2-(1-cyclopropyl-5-methyl-1H-pyrazole-4-carbonyl)-1,2,3,4-tetrahydroiso quinolin-8-yl]pyrrolidine-1-carboxylate (65.00 mg, yield: 71.48%) as a yellow oil. ES-API: [M+H]⁺ = 485.23.

Step 2: The tert-butyl (S)-2-[6-chloro-2-(1-cyclopropyl-5-methyl-1H-pyrazole-4-carbonyl)-1,2,3,4-tetrahydroiso quinolin-8-yl]pyrrolidine-1-carboxylate (65.00 mg, 134.02 µmol), 3-methyl-5-(4,4,5,5-tetramethyl-1,3,2-dioxaborolan-2-yl)-1*H*-pyrrolo[2,3-*b*]pyridine (44.06 mg, 0.17 mmol), chloro (2-dicyclohexylphosphino-2',6'-dimethoxy-1,1'-biphenylyl)(2'-amino-1,1'-biphenyl-2-yl) palladium (II) (10.24 mg, 0.014 mmol) and potassium carbonate (39.32 mg, 0.28 mmol) were dissolved in 1,4-dioxane (2 mL) and water (0.1 mL), and the system was warmed to 100°C for 2 h. Upon completion of the reaction, the reaction mixture was quenched by the addition of water, and extracted with ethyl acetate. The organic phase was washed with brine, dried over anhydrous sodium sulfate, filtered, and concentrated to give a crude product. The crude product was purified by a thin layer chromatographic column (dichloromethane/methanol = 10/1) to give tert-butyl (*S*)-2-[2-(1-cyclopropyl-5-methyl-1H-pyrazole-4-carbonyl)-6-(3-methyl-1*H*-pyrrolo[2,3-*b* ]pyridin-5-yl)-1,2,3,4-tetrahydroisoquinolin-8-yl]pyrrolidine-1-carboxylate (50.00 mg, yield: 57.50%) as a white solid. ES-API: [M+H]⁺ = 581.32.

Step 3: The tert-butyl (*S*)-2-[2-(1-cyclopropyl-5-methyl-1H-pyrazole-4-carbonyl)-6-(3-methyl-1*H*-pyrrolo[2,3-*b* ]pyridin-5-yl)-1,2,3,4-tetrahydroisoquinolin-8-yl]pyrrolidine-1-carboxylate (45 mg, 77.49 µmol) was dissolved in dichloromethane (2 mL) and trifluoroacetic acid solution (2 mL) and stirred at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a thin layer chromatographic column (dichloromethane/methanol = 5/1) to give a product to give (*S*)-(1-cyclopropyl-5-methyl-1H-pyrazol-4-yl)-[6-(3-methyl-1*H*-pyrrolo[2,3-*b*]pyridin-5-y 1)-8-[pyrrolidin-2-yl]-3,4-dihydroisoquinolin-2(1H)-yl]methanone (Z346, 18.00 mg, yield: 39.33%) as a pale yellow solid. ES-API: [M+H]⁺ = 481.27. ¹H NMR (400 MHz, DMSO-d6) δ 11.38 (s, 1H), 8.59 (d, *J* = 1.9 Hz, 1H), 8.33 -8.28 (m, 1H), 7.90 (s, 1H), 7.64-7.56 (m, 2H), 7.28-7.23 (m, 1H), 4.95 (d, *J=* 17.1 Hz, 1H), 4.80 (d, *J=* 17.2 Hz, 1H), 4.73-4.59 (m, 1H), 3.80-3.72 (m, 2H), 3.61-3.42 (m, 2H), 2.99-2.95 (m, 2H), 2.41 (s, 3H), 2.30 (s, 3H), 2.21-1.93 (m, 3H), 1.25-1.20 (m, 2H), 1.09-0.97 (m, 4H).

### Example 129. Synthesis of compound Z341

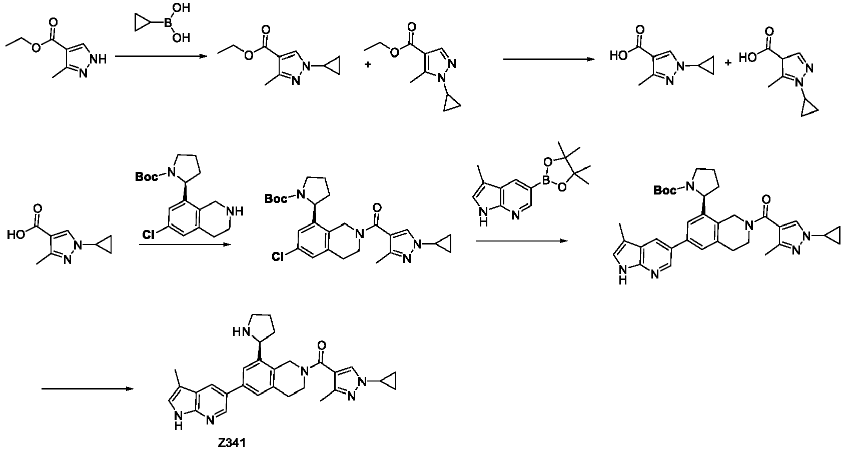

Step 1: Cyclopropylboronic acid (1.11 g, 13 mmol) and sodium carbonate (1.38 g, 13 mmol) were added to a solution of ethyl 3-methyl-1*H*-pyrazole-4-carboxylate (1 g, 6.49 mmol) in 1,2-dichloroethane (50 mL), and the mixture was warmed to 70°C. 2,2'-Bipyridine (1.01 g, 6.49 mmol) and copper (II) acetate (1.18 g, 6.49 mmol) were added, and the reaction was continued at this temperature for 16 h. The mixture was poured into water (20 mL), extracted with dichloromethane, and concentrated to give a crude product as a yellow liquid. The crude product was purified by silica gel column chromatography (petroleum ether:ethyl acetate = 5:1) to give ethyl 1-cyclopropyl-3-methyl-pyrazole-4-carboxylate and ethyl 1-cyclopropyl-5-methyl-1H-pyrazole-4-carboxylate (610.00 mg, crude product) as a yellow liquid. ES-API: [M+H]⁺ = 195.11.

Step 2: Sodium hydroxide (314 mg, 7.85 mmol) was added to a solution of a mixture (610 mg, 3.14 mmol) of the ethyl 1-cyclopropyl-3-methyl-pyrazole-4-carboxylate and the ethyl 1-cyclopropyl-5-methyl-1H-pyrazole-4-carboxylate in 1,4-dioxane (5 mL) and water (5 mL), and the system was then warmed to 70°C for 3 h. Upon completion of the reaction, 1N hydrochloric acid was added to the solution to adjust the pH to 4. The reaction mixture was then extracted with ethyl acetate 3 times. The organic phases were combined and concentrated to give a crude product. The crude product was purified by prep-HPLC (column: Xbridge C18 19 mm*250 mm; mobile phase: acetonitrile: water: trifluoroacetic acid = 30:70:0.04; flow rate: 1 ml/min, 9 min; column temperature: 30°C) to give two monomers: 1-cyclopropyl-3-methyl-pyrazole-4-carboxylic acid (relative retention time was supplemented) (180.00 mg, peak 1, retention time: 6.55 min, yield: 34.49%). ES-API: [M+H]⁺ = 167.08. ¹H NMR (400 MHz, DMSO-d6) δ 8.11 (s, 1H), 3.67-3.62 (m, 1H), 2.25 (s, 3H), 1.17-0.78 (m, 4H). cyclopropyl-5-methyl-1H-pyrazole-4-carboxylic acid (relative retention time was supplemented) (120.00 mg, retention time: 7.40 min, yield: 22.99%). ES-API: [M+H]⁺ = 167.08. ¹H NMR (400 MHz, DMSO-d6) δ7.61 (s, 1H), 3.54-3.48 (m, 1H), 2.52 (s, 3H), 1.04-0.96 (m, 4H).

Step 3: The ethyl 1-cyclopropyl-5-methyl-1*H*-pyrazole-4-carboxylate (35.52 mg, 213.74 µmol), 1-propylphosphonic anhydride (169.8 mg, 267.18 µmol, 50% in ethyl acetate) and triethylamine (0.05 mL) were added to a solution of tert-butyl (S)-2-(6-chloro-1,2,3,4-tetrahydroisoquinolin-8-yl)pyrrolidine-1-carboxylate (60 mg, 178.12 µmol) in dichloromethane (2 mL), and the obtained mixture was stirred at 20°C for 2 h. The reaction mixture was spun to dryness to give a crude product. The crude product was purified by silica gel column chromatography (petroleum ether/ethyl acetate = 1/1) to give tert-butyl (S)-2-[6-chloro-2-(1-cyclopropyl-3-methyl-1H-pyrazole-4-carbonyl)-1,2,3,4-tetrahydroiso quinolin-8-yl]pyrrolidine-1-carboxylate (70.00 mg, yield: 76.98%) as a yellow oil. ES-API: [M+H]⁺ = 485.23.

Step 4: The tert-butyl (S)-2-[6-chloro-2-(1-cyclopropyl-3-methyl-1H-pyrazole-4-carbonyl)-1,2,3,4-tetrahydroiso quinolin-8-yl]pyrrolidine-1-carboxylate (70 mg, 144.32 µmol), 3-methyl-5-(4,4,5,5-tetramethyl-1,3,2-dioxaborolan-2-yl)-1H-pyrrolo[2,3-b]pyridine (44.06 mg, 0.17 mmol), chloro (2-dicyclohexylphosphino-2',6'-dimethoxy-1,1'-biphenylyl)(2'-amino-1,1'-biphenyl-2-yl) palladium (II) (10.24 mg, 0.014 mmol) and potassium carbonate (39.32 mg, 0.28 mmol) were dissolved in 1,4-dioxane (2 mL) and water (0.1 mL), and the system was warmed to 100°C for 2 h. Upon completion of the reaction, the reaction mixture was quenched by the addition of water, and extracted with ethyl acetate. The organic phase was washed with brine, dried over anhydrous sodium sulfate, filtered, and concentrated to give a crude product. The crude product was purified by a thin layer chromatographic column (dichloromethane/methanol = 10/1) to give tert-butyl (*S*)-2-[2-(1-cyclopropyl-3-methyl-1H-pyrazole-4-carbonyl)-6-(3-methyl-1*H*-pyrrolo[2,3-*b* ]pyridin-5-yl)-1,2,3,4-tetrahydroisoquinolin-8-yl]pyrrolidine-1-carboxylate (45.00 mg, yield: 51.75%) as a white solid. ES-API: [M+H]⁺ = 581.32.

Step 5: The tert-butyl (*S*)-2-[2-(1-cyclopropyl-3-methyl-1H-pyrazole-4-carbonyl)-6-(3-methyl-1*H*-pyrrolo[2,3-*b* ]pyridin-5-yl)-1,2,3,4-tetrahydroisoquinolin-8-yl]pyrrolidine-1-carboxylate (45 mg, 77.49 µmol) was dissolved in dichloromethane (2 mL) and trifluoroacetic acid solution (2 mL) and stirred at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a thin layer chromatographic column (dichloromethane/methanol = 5/1) to give a product (*S*)-(1-cyclopropyl-3-methyl-1H-pyrazol-4-yl(6-(3-methyl-1*H*-pyrrolo[2,3-*b*]pyridin-5-yl) -8-(pyrrolidin-2-yl]-3,4-dihydroisoquinolin-2(1H)-yl)methanone (Z341, 18.80 mg, yield: 44.13%) as a pale yellow solid.

### Example 314. Synthesis of compound Z314-1

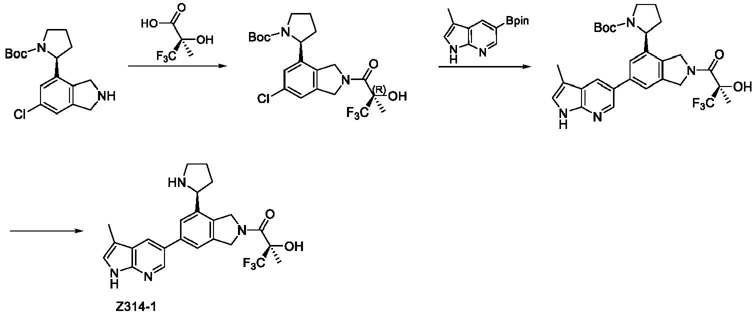

Step 1: N,N-Diisopropylethyl amine (400 mg, 3.10 mmol) and *N*,*N*,*N*',*N*'-tetramethyl-O-(7-azabenzotriazol-1-yl)urea hexafluorophosphate (175 mg, 0.46 mmol) were added to a mixture of tert-butyl (S)-2-(6-chloroisoindolin-4-yl)pyrrolidine-1-carboxylate (100 mg, 0.31 mmol) and (R)-3,3,3-trifluoro-2-hydroxy-2-methylpropanoic acid (98 mg, 0.62 mmol) in dichloromethane (5 mL) successively and stirred at room temperature for 1 h. The reaction mixture was dissolved in dichloromethane (10 mL), and washed with water (5 mL) and saturated brine (5 mL) successively. The organic phase was dried over anhydrous sodium sulfate, filtered, concentrated, and purified by a flash silica gel column (0-50% tetrahydrofuran/petroleum ether) to give tert-butyl (S)-2-(6-chloro-2-((R)-3,3,3-trifluoro-2-hydroxy-2-methylpropanoyl)isoindolin-4-yl)pyrro lidine-1-carboxylate (70 mg, yield: 49%). ES-API: [M+H]⁺ = 463.1.

Step 2: In the presence of protective nitrogen, a mixed solution of the tert-butyl (S)-2-(6-chloro-2-((R)-3,3,3-trifluoro-2-hydroxy-2-methylpropanoyl)isoindolin-4-yl)pyrro lidine-1-carboxylate (70 mg, 0.15 mmol), 3-methyl-5-(4,4,5,5-tetramethyl-dioxaborolan-2-yl)-1*H*-pyrrolo[2,3-*b*]pyridine (58 mg, 0.23 mmol), chloro (2-dicyclohexylphosphino-2',6'-dimethoxy-1,1'-biphenylyl)(2'-amino-1,1'-biphenyl-2-yl) palladium (II) (11 mg, 0.02 mmol) and potassium carbonate (63 mg, 0.46 mmol) in 1,4-dioxane (2 mL) and water (0.4 mL) was stirred at 120°C for 2 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by a flash silica gel column (0-10% methanol/dichloromethane) to give tert-butyl (S)-2-(6-(3-methyl-1*H*-pyrrolo[2,3-*b*]pyridin-5-yl)-2-((R)-3,3,3-trifluoro-2-hydroxy-2-met hylpropanoyl)isoindolin-4-yl)pyrrolidine-1-carboxylate (50 mg, yield: 59%). ES-API: [M+H]⁺ = 559.3.

Step 3: Trifluoroacetic acid (0.5 mL) was added dropwise to a solution of the tert-butyl (S)-2-(6-(3-methyl-1*H*-pyrrolo[2,3-*b*]pyridin-5-yl)-2-((R)-3,3,3-trifluoro-2-hydroxy-2-met hylpropanoyl)isoindolin-4-yl)pyrrolidine-1-carboxylate (50 mg, 90 µmol) in dichloromethane (1 mL) under an ice bath condition and stirred at room temperature for 1 h. The reaction mixture was concentrated, and purified by prep-HPLC (formic acid method) to give (R)-3,3,3-trifluoro-2-hydroxy-2-methyl-1-(6-(3-methyl-1*H*-pyrrolo[2,3-b]pyridin-5-yl)-4-( (S)-pyrrolidin-2-yl)isoindolin-2-yl)propan-1-one formate (Z314-1, 36 mg, purity: 100%, yield: 79%) as a white solid. ES-API: [M+H]⁺ = 459.2. ¹H NMR (400 MHz, DMSO-*d*₆) δ 11.36 (s, 1H), 8.54 - 8.47 (m, 1H), 8.31 (s, 1H), 8.21 - 8.13 (m, 1H), 7.76 (s, 1H), 7.64 (s, 1H), 7.27 (s, 1H), 5.31 - 5.17 (m, 2H), 4.84 (d, *J* = 3.6 Hz, 1H), 4.81 (d, *J* = 3.6 Hz, 1H), 4.38 - 4.21 (m, 1H), 3.29 - 3.17 (m, 1H), 3.15 - 3.01 (m, 1H), 2.33 (s, 3H), 2.29 - 2.17 (m, 1H), 2.08 - 1.83 (m, 2H), 1.84 - 1.72 (m, 1H), 1.66 - 1.57 (m, 3H).

### Example 130. Synthesis of compound Z347

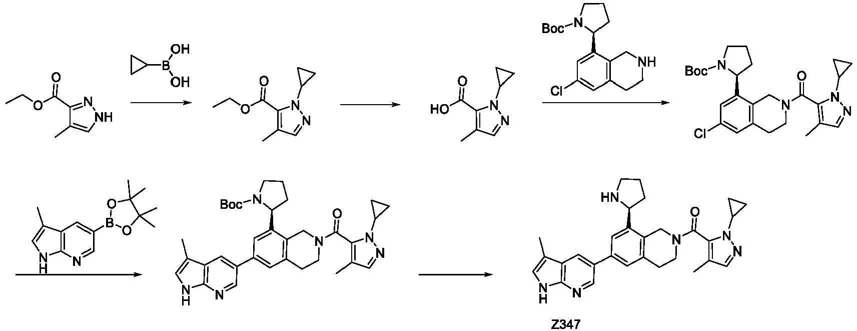

Step 1: Cyclopropylboronic acid (1.1 g, 13.0 mmol), sodium carbonate (1.3 g, 13.0 mmol), 2,2'-bipyridine (1.0 g, 6.5 mmol) and copper acetate (1.3 g, 6.5 mmol) were added to a solution of ethyl 4-methyl-1H-pyrazole-3-carboxylate (1.0 g, 6.5 mmol) in 1,2-dichloroethane (20 mL) and stirred at 70°C for 16 h. The reaction mixture was cooled to room temperature and then filtered out, poured into water, and extracted with ethyl acetate. The organic layers were combined, and dried over anhydrous sodium sulfate. The reaction mixture was spun to dryness, and purified by silica gel column chromatography (petroleum ether/ethyl acetate = 1/1) to give ethyl 1-cyclopropyl-4-methyl-1H-pyrazole-3-carboxylate (0.3 g, yield: 23.8%) as a yellow oil. ES-API:[M+H]⁺=195.1. ¹H NMR (400 MHz, CDCl₃) δ 7.23 (s, 1H), 4.35 (q, *J* = 7.2 Hz, 2H), 3.62-3.55 (m, 1H), 2.23 (s, 3H), 1.36 (t, *J* = 7.2 Hz, 3H), 1.13-1.07 (m, 2H), 1.04-0.96 (m, 2H).

Step 2: Sodium hydroxide (123.6 mg, 3.09 mmol) was added to a solution of the ethyl 1-cyclopropyl-4-methyl-1H-pyrazole-3-carboxylate (0.3 g, 1.54 mmol) in methanol (5 mL) and water (15 mL) and stirred at 25°C for 16 h. The mixture was concentrated. 1M Hydrochloric acid was added, and the obtained mixture was extracted with ethyl acetate. The organic layer was dried over anhydrous sodium sulfate, filtered, and concentrated in vacuum to give 1-cyclopropyl-4-methyl-1H-pyrazole-3-carboxylic acid (200.00 mg, crude product) as a white solid. ES-API:[M+H]⁺=167.1.

Step 3: The 1-cyclopropyl-4-methyl-1H-pyrazole-3-carboxylic acid (69 mg, 0.4 mmol), 1-propylphosphonic anhydride (190.8 mg, 0.3 mmol, 50% in ethyl acetate) and triethylamine (63.1 mg, 0.6 mmol) were added to a solution of tert-butyl (S)-2-(6-chloro-1,2,3,4-tetrahydroisoquinolin-8-yl)pyrrolidine-1-carboxylate (70 mg, 0.2 mmol) in dichloromethane (2 mL) and stirred at 25°C for 4 h. Water was added to the mixture, and the reaction mixture was extracted with ethyl acetate. The organic layer was dried over anhydrous sodium chloride, filtered, concentrated, and purified by a preparative thin layer chromatographic column (petroleum ether:ethyl acetate = 1:1) to give tert-butyl (S)-2-[6-chloro-2-(1-cyclopropyl-4-methyl-1H-pyrazole-3-carbonyl)-1,2,3,4-tetrahydroiso quinolin-8-yl]pyrrolidine-1-carboxylate (70.0 mg, yield: 69.45%) as a yellow oil. ES-API:[M+H]⁺=485.3.

Step 4: The tert-butyl (S)-2-[6-chloro-2-(1-cyclopropyl-4-methyl-1H-pyrazole-3-carbonyl)-1,2,3,4-tetrahydroiso quinolin-8-yl]pyrrolidine-1-carboxylate (0.07 g, 0.14 mmol) was dissolved in dioxane/water (4 mL/0.8 mL). 3-Methyl-5-(4,4,5,5-tetramethyl-1,3,2-dioxaborolan-2-yl)-1H-pyrrolo[2,3-b]pyridine (44.7 mg, 0.17 mmol), potassium carbonate (59.75 mg, 0.43 mmol) and chloro (2-dicyclohexylphosphino-2',6'-dimethoxy-1,1'-biphenylyl)(2'-amino-1,1'-biphenyl-2-yl) palladium (II) (10.4 mg, 14.4 µmol) were added. The system was heated to 100°C for 1 h. Upon completion of the reaction, the reaction mixture was spun to dryness. The crude product was purified by a preparative thin layer chromatographic column (dichloromethane:methanol = 10:1) to give a product tert-butyl (S)-2-[2-(1-cyclopropyl-4-methyl-1H-pyrazole-3-carbonyl)-6-(3-methyl-1H-pyrrolo[2,3-b ]pyridin-5-yl)-1,2,3,4-tetrahydroisoquinolin-8-yl]pyrrolidine-1-carboxylate (70.0 mg, yield: 83.52%) as a yellow oil. ES-API:[M+H]⁺=581.5.

Step 5: The tert-butyl (S)-2-[2-(1-cyclopropyl-4-methyl-1H-pyrazole-3-carbonyl)-6-(3-methyl-1H-pyrrolo[2,3-b ]pyridin-5-yl)-1,2,3,4-tetrahydroisoquinolin-8-yl]pyrrolidine-1-carboxylate (70.0 mg, 0.12 mmol) was dissolved in dichloromethane (2 mL). Trifluoroacetic acid (2 mL) was added. The system reacted at room temperature for 1 h. Upon completion of the reaction, the reaction mixture was spun to dryness. The crude product was purified by a preparative thin layer chromatographic column (dichloromethane:methanol = 8:1) to give (S)-(1-cyclopropyl-4-methyl-1H-pyrazol-3-yl)-[6-(3-methyl-1H-pyrrolo[2,3-b]pyridin-5-y l)-8-[pyrrolidin-2-yl]-3,4-dihydro-1H-isoquinolin-2-yl]methanone (Z347, 14.8 mg, yield: 25.55%) as a yellow solid. ES-API:[M+H]⁺=481.3. ¹H NMR (400 MHz, CDCl₃) *δ* 9.75 (s, 1H), 8.51 (s, 1H), 8.10-7.94 (m, 1H), 7.88-7.68 (m, 1H), 7.38-7.27 (m, 2H), 7.09 (s, 1H), 5.23-5.05 (m, 1H), 4.94-4.67 (m, 1H), 4.55-4.25 (m, 1H), 4.12-3.85 (m, 2H), 3.67-3.52 (m, 1H), 3.43-3.28 (m, 1H), 3.18-2.94 (m, 3H), 2.34 (s, 3H), 2.17 (s, 3H), 2.12-1.96 (m, 2H), 1.96-1.82 (m, 1H), 1.80-1.64 (m, 1H), 1.21-1.09 (m, 2H), 1.07-0.96 (m, 2H).

### Example 131. Synthesis of compound Z342

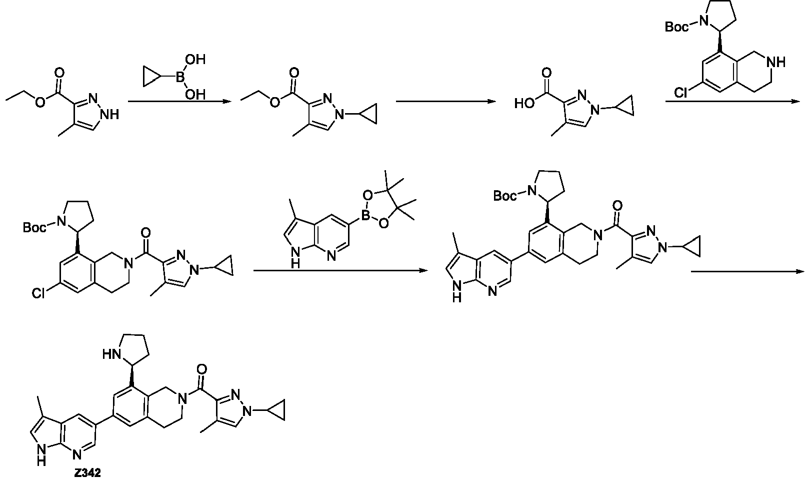

Step 1: Cyclopropylboronic acid (1.1 g, 13.0 mmol), sodium carbonate (1.3 g, 13.0 mmol), 2,2'-bipyridine (1.0 g, 6.5 mmol) and copper acetate (1.3 g, 6.5 mmol) were added to a solution of ethyl 4-methyl-1H-pyrazole-3-carboxylate (1.0 g, 6.5 mmol) in 1,2-dichloroethane (20 mL) and stirred at 70°C for 16 h. The reaction mixture was cooled to room temperature and then filtered out, poured into water, and extracted with ethyl acetate. The organic layers were combined, and dried over anhydrous sodium sulfate. The reaction mixture was spun to dryness, and purified by silica gel column chromatography (petroleum ether/ethyl acetate = 1/1) to give ethyl 1-cyclopropyl-4-methyl-1H-pyrazole-3-carboxylate (0.5 g, yield: 39.7%) as a yellow oil. ES-API:[M+H]⁺=195.1. ¹H NMR (400 MHz, CDCl₃) δ 7.21 (s, 1H), 4.34 (q, *J* = 7.2 Hz, 2H), 4.16-4.09 (m, 1H), 2.22 (s, 3H), 1.38 (t, *J* = 7.2 Hz, 3H), 1.21-1.16 (m, 2H), 1.02-0.96 (m, 2H).

Step 2: Sodium hydroxide (185.3 mg, 4.63 mmol) was added to a solution of the ethyl 1-cyclopropyl-4-methyl-1H-pyrazole-3-carboxylate (0.5 g, 2058 mmol) in methanol (5 mL) and water (15 mL) and stirred at 25°C for 16 h. The mixture was concentrated. 1M Hydrochloric acid was added, and the obtained mixture was extracted with ethyl acetate. The organic layer was dried over anhydrous sodium sulfate, filtered, and concentrated in vacuum to give 1-cyclopropyl-4-methyl-1H-pyrazole-3-carboxylic acid (300.00 mg, crude product) as a white solid. ES-API:[M+H]⁺=167.1.

Step 3: The 1-cyclopropyl-4-methyl-1H-pyrazole-3-carboxylic acid (69 mg, 0.4 mmol), 1-propylphosphonic anhydride (190.8 mg, 0.3 mmol, 50% in ethyl acetate) and triethylamine (63.1 mg, 0.6 mmol) were added to a solution of tert-butyl (S)-2-(6-chloro-1,2,3,4-tetrahydroisoquinolin-8-yl)pyrrolidine-1-carboxylate (70 mg, 0.2 mmol) in dichloromethane (2 mL) and stirred at 25°C for 4 h. Water was added to the mixture, and the reaction mixture was extracted with ethyl acetate. The organic layer was dried over anhydrous sodium sulfate, filtered, concentrated, and purified by a preparative thin layer chromatographic column (petroleum ether:ethyl acetate = 1:1) to give tert-butyl (S)-2-[6-chloro-2-(1-cyclopropyl-4-methyl-1H-pyrazole-3-carbonyl)-1,2,3,4-tetrahydroiso quinolin-8-yl]pyrrolidine-1-carboxylate (60.00 mg, yield: 59.53%) as a yellow oil. ES-API:[M+H]⁺=485.3.

Step 4: The tert-butyl (S)-2-[6-chloro-2-(1-cyclopropyl-4-methyl-1H-pyrazole-3-carbonyl)-1,2,3,4-tetrahydroiso quinolin-8-yl]pyrrolidine-1-carboxylate (60 mg, 0.12 mmol) was dissolved in dioxane/water (4 mL/0.8 mL). 3-Methyl-5-(4,4,5,5-tetramethyl-1,3,2-dioxaborolan-2-yl)-1H-pyrrolo[2,3-b]pyridine (38.3 mg, 0.15 mmol), potassium carbonate (51.21 mg, 0.37 mmol) and chloro (2-dicyclohexylphosphino-2',6'-dimethoxy-1,1'-biphenylyl)(2'-amino-1,1'-biphenyl-2-yl) palladium (II) (8.91 mg, 12.37 µmol) were added. The system was heated to 100°C for 1 h. Upon completion of the reaction, the reaction mixture was spun to dryness and purified by a preparative thin layer chromatographic column (dichloromethane:methanol = 10:1) to give a product tert-butyl (S)-2-[2-(1-cyclopropyl-4-methyl-1H-pyrazole-3-carbonyl)-6-(3-methyl-1H-pyrrolo[2,3-b ]pyridin-5-yl)-1,2,3,4-tetrahydroisoquinolin-8-yl]pyrrolidine-1-carboxylate (60.00 mg, yield: 83.52%) as a yellow oil. ES-API:[M+H]⁺=581.4.

Step 5: The tert-butyl (S)-2-[2-(1-cyclopropyl-4-methyl-1H-pyrazole-3-carbonyl)-6-(3-methyl-1H-pyrrolo[2,3-b ]pyridin-5-yl)-1,2,3,4-tetrahydroisoquinolin-8-yl]pyrrolidine-1-carboxylate (60.0 mg, 0.1 mmol) was dissolved in dichloromethane (2 mL). Trifluoroacetic acid (2 mL) was added. The system reacted at room temperature for 1 h. Upon completion of the reaction, the reaction mixture was spun to dryness. The crude product was purified by a preparative thin layer chromatographic column (dichloromethane:methanol = 8:1) to give (S)-(1-cyclopropyl-4-methyl-1H-pyrazol-3-yl)-[6-(3-methyl-1H-pyrrolo[2,3-b]pyridin-5-y l)-8-[pyrrolidin-2-yl]-3,4-dihydro-2(1H)-isoquinolin-yl]methanone (Z342, 30.00 mg, yield: 60.42%) as a yellow solid. ES-API:[M+H]⁺=481.3. ¹H NMR (400 MHz, CDCl₃) *δ* 10.19 (s, 1H), 8.57 (s, 1H), 8.01-7.79 (m, 2H), 7.21-7.19 (m, 1H), 7.12-6.96 (m, 2H), 5.21-4.85 (m, 1H), 4.81-4.66 (m, 1H), 4.62-4.27 (m, 1H), 3.73-3.23 (m, 5H), 3.06-2.60 (m, 2H), 2.46-2.36 (m, 1H), 2.25 (s, 3H), 2.20-1.89 (m, 6H), 1.35-1.17 (m, 2H), 0.96-0.75 (m, 2H).

### Example 132. Synthesis of compound Z327

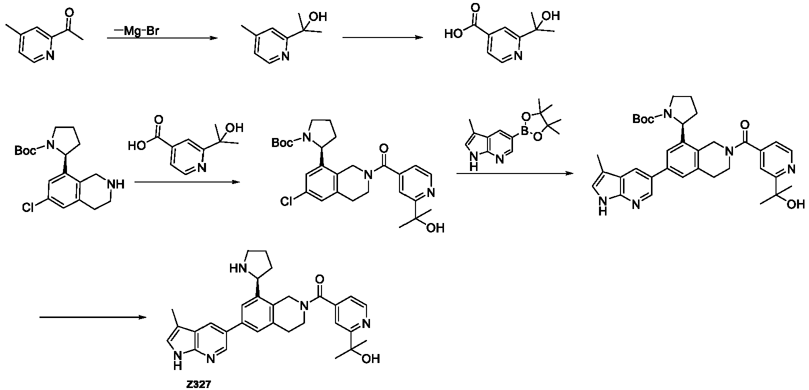

Step 1: 1-(4-Methyl-2-pyridyl)ethanone (1 g, 7.40 mmol) was dissolved in tetrahydrofuran (20 mL) in a three-neck flask under a dry nitrogen atmosphere. The flask was cooled in an ice bath, and methylmagnesium bromide (2.65 g, 22.20 mmol, 20 mL) was added dropwise. The solution was warmed to 40°C and stirred for 3 h. The reaction was monitored by LCMS for completion. The reaction mixture was then cooled in an ice bath, quenched by the addition of saturated aqueous ammonium chloride solution (50 ml), and then extracted with ethyl acetate (40 mlX5). The organic was washed with saturated brine solution, dried over anhydrous sodium sulfate, and concentrated under reduced pressure. The residue was purified by silica gel column chromatography (0-35% ethyl acetate in petroleum ether) to give a product 2-(4-methyl-2-pyridyl)propan-2-ol (375.00 mg, yield: 33.52%) as a yellow liquid. ES-API: [M+H-100]+= 152.2.

Step 2: Potassium permanganate (1.57 g, 9.92 mmol) was added to a solution of the 2-(4-methyl-2-pyridyl)propan-2-ol (375 mg, 2.48 mmol) in water (15 mL) at 60°C. The reaction was heated to 100°C and stirred for 20 h. The reaction was monitored by LCMS for completion. The suspension was cooled to 25°C and then filtered with diatomaceous earth. The aqueous phase was washed with ethyl acetate. Water was then removed, and the obtained mixture was freeze-dried to give a product 2-(1-hydroxy-1-methyl-ethyl)pyridine-4-carboxylic acid (450 mg, crude product) as a white solid. ES-API: [M+H]⁺= 182.0.

Step 3: The 2-(1-hydroxy-1-methyl-ethyl)pyridine-4-carboxylic acid (64.55 mg, 0.36 mmol), 2-(7-azobenzotriazole)-N,N,N',N'-tetramethylurea hexafluorophosphate (101.53 mg, 0.27 mmol) and triethylamine (180.24 mg, 1.78 mmol) were added to a solution of tert-butyl (S)-2-(6-chloro-1, 2, 3, 4-tetrahydroisoquinolin-8-yl) pyrrolidine-1-carboxylate (60 mg, 0.18 mmol) in dichloromethane (2 mL), and the mixture was stirred at 25°C for 2 h. The reaction was monitored by LCMS for completion, and quenched by the addition of water. The reaction mixture was extracted with dichloromethane, dried over anhydrous sodium sulfate, concentrated, and purified by silica gel column chromatography (mobile phase 0-50% ethyl acetate/petroleum ether, ethyl acetate/petroleum ether = 1/1, Rf = 0.5) to give a product tert-butyl (S)-2-(6-chloro-2-(2-(2-hydroxypropan-2-yl)isonicotinoyl)-1,2,3,4-tetrahydroisoquinol-8-yl)pyrrolidine-1-carboxylate (34 mg, yield: 38.17%). ES-API:[M+H]⁺= 500.3.

Step 4: 3-Methyl-5-(4,4,5,5-tetramethyl-1,3,2-dioxaborolan-2-yl)-1H-pyrrolo[2, 3-b]pyridine (29.73 mg, 0.12 mmol), chloro (2-dicyclohexylphosphino-2',6'-dimethoxy-1,1'-biphenylyl)(2'-amino-1,1'-biphenyl-2-yl) palladium (II (6.91 mg, 0.0096 mmmol) and potassium carbonate (39.74 mg, 0.29 mmmol) were added to a solution of the compound tert-butyl (S)-2-(6-chloro-2-(2-(2-hydroxypropan-2-yl)isonicotinoyl)-1,2,3,4-tetrahydroisoquinol-8-yl)pyrrolidine-1-carboxylate (48 mg, 0.096 mmol) in dioxane/water (2 ml/0.4 ml), and the mixture was heated to 110°C and stirred in the presence of protective nitrogen. The reaction was monitored by LCMS for completion. The reaction mixture was treated with ethyl acetate/water, dried over anhydrous sodium sulfate, concentrated, and purified by silica gel column chromatography (mobile phase 0-10% methanol/dichloromethane, dichloromethane/methanol = 10/1, Rf = 0.6) to give a product tert-butyl (S)-2-(2-(2-(2-hydroxypropan-2-yl)isonicotinoyl)-6-(3-methyl-1H-pyrrolo[2,3-b]pyridin-5 -yl)-1,2,3,4-tetrahydroisoquinolin-8-yl)pyrrolidine-1-carboxylate (45 mg, yield: 78.69%). ES-API:[M+H]⁺= 596.4.

Step 5: Trifluoroacetic acid (1 mL) was added to a solution of the compound tert-butyl (S)-2-(2-(2-(2-hydroxypropan-2-yl)isonicotinoyl)-6-(3-methyl-1H-pyrrolo[2,3-b]pyridin-5 -yl)-1,2,3,4-tetrahydroisoquinolin-8-yl)pyrrolidine-1-carboxylate (45 mg, 0.076 mmol) in dichloromethane (2 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, concentrated, and purified by prep-HPLC (column: Ultimate XB-C18, 50*250 mm, 10 um, mobile phase system: A: purified water (0.05% ammonia water) B: pure acetonitrile, flow rate: 80 ml/min, gradient: B/A = 20%-90% over 50 min, wavelength: 214 nm, column temperature: room temperature) to give (S)-(2-(2-hydroxypropan-2-yl)pyridin-4-yl)(6-(3-methyl-1H-pyrrolo[2,3-b]pyridin-5-yl)-8 -(pyrrolidin-2-yl)-3,4-dihydroisoquinolin-2(1H)-yl)methanone (Z327, 13.6 mg, yield: 36.33%) as a white powder. ES-API:[M+H]⁺=496.3. ¹H NMR (400 MHz, CDCl₃) δ 9.55-9.34 (m, 1H), 8.67-8.60 (m, 1H), 8.49 (d, *J* = 15.0 Hz, 1H), 7.97 (d, *J* = 12.6 Hz, 1H), 7.89-7.62 (m, 1H), 7.58-7.46 (m, 1H), 7.31-7.27 (m, 1H), 7.24-7.18 (m, 1H), 7.09 (s, 1H), 5.14-4.79 (m, 2H), 4.63-4.50 (m, 1H), 4.11-3.78 (m, 1H), 3.63-3.39 (m, 2H), 3.31-3.16 (m, 1H), 3.11-2.79 (m, 3H), 2.47-2.36 (m, 1H), 2.34 (s, 3H), 2.15-1.89 (m, 2H), 1.87-1.76 (m, 1H), 1.64-1.52 (m, 6H).

### Example 133. Synthesis of compound Z333

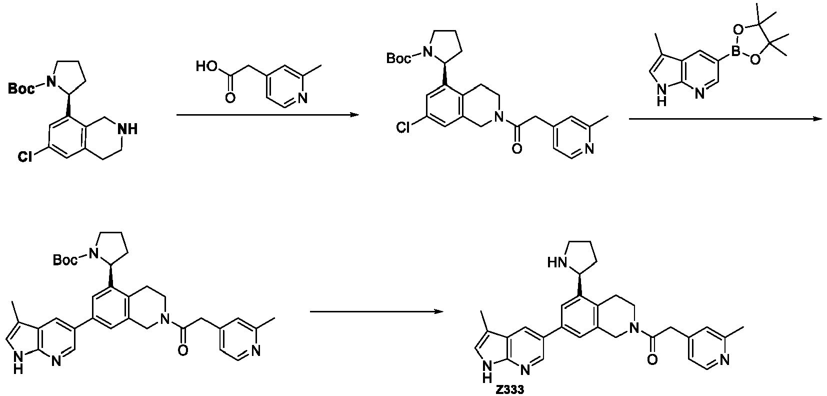

Step 1: 2-(2-Methylpyridin-4-yl)acetic acid (179.7 mg, 1.19 mmol), 1-propylphosphonic anhydride (756.8 mg, 1.19 mmol, 50% in ethyl acetate) and triethylamine (0.05 mL) were added to a solution of tert-butyl (S)-2-(6-chloro-1,2,3,4-tetrahydroisoquinolin-8-yl)pyrrolidine-1-carboxylate (200 mg, 0.595 mmol) in dichloromethane (2 mL), and the obtained mixture was stirred at 20°C for 2 h. The reaction mixture was spun to dryness to remove a solvent and obtain a crude product. The crude product was purified by silica gel column chromatography (petroleum ether/ethyl acetate = 1/1) to give tert-butyl (S)-2-[7-chloro-2-[2-(2-methyl-4-pyridyl)acetyl]-1,2,3,4-tetrahydroisoquinolin-5-yl]pyrrol idine-1-carboxylate as a yellow oil (160 mg, yield: 57.1%). ES-API: [M+H]⁺= 470.20

Step 2: 3-Methyl-5-(4,4,5,5-tetramethyl-1,3,2-dioxaborolan-2-yl)-1H-pyrrolo[2,3-b]pyridine (105.45 mg, 408.51 µmol), potassium carbonate (140.94 mg, 1.02 mmol) and chloro (2-dicyclohexylphosphino-2',6'-dimethoxy-1,1'-biphenylyl)(2'-amino-1,1'-biphenyl-2-yl) palladium (II) (24.51 mg, 34.04 µmol) were added to a solution of the compound tert-butyl (S)-2-[7-chloro-2-[2-(2-methyl-4-pyridyl)acetyl]-1,2,3,4-tetrahydroisoquinolin-5-yl]pyrrol idine-1-carboxylate (0.16 g, 340.42 µmol) in dioxane/water (4 mL/0.8 mL), and the mixture was heated to 100°C and stirred in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by a preparative thin layer chromatographic column (dichloromethane/methanol = 10/1) to give a product tert-butyl (S)-2-(7-(3-methyl-1H-pyrrolo[2,3-b]pyridin-5-yl)-2-(2-(2-methylpyridin-4-yl) acetyl)-1,2,3,4-tetrahydroisoquinolin-5-yl)pyrrolidine-1-carboxylate (100.00 mg, yield: 51.93%) as a yellow oil. ES-API:[M+H]⁺=566.40.

Step 3: Trifluoroacetic acid (2 ml) was added to a solution of the compound tert-butyl (S)-2-(7-(3-methyl-1H-pyrrolo[2,3-b]pyridin-5-yl)-2-(2-(2-methylpyridin-4-yl) acetyl)-1,2,3,4-tetrahydroisoquinolin-5-yl)pyrrolidine-1-carboxylate (100.00 mg, 176.77 µmol) in dichloromethane (2 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by a preparative thin layer chromatographic column (dichloromethane/methanol = 8/1) to give (S)-2-(2-methyl-4-pyridyl)-1-[7-(3-methyl-1H-pyrrolo[2,3-b]pyridin-5-yl)-5-pyrrolidin-2-yl-3,4-dihydro-1H-isoquinolin-2-yl]ethanone (Z333, 20.00 mg, yield: 24.30%) as a yellow solid. ES-API:[M+H]⁺=467.3.

### Example 134. Synthesis of compound Z365

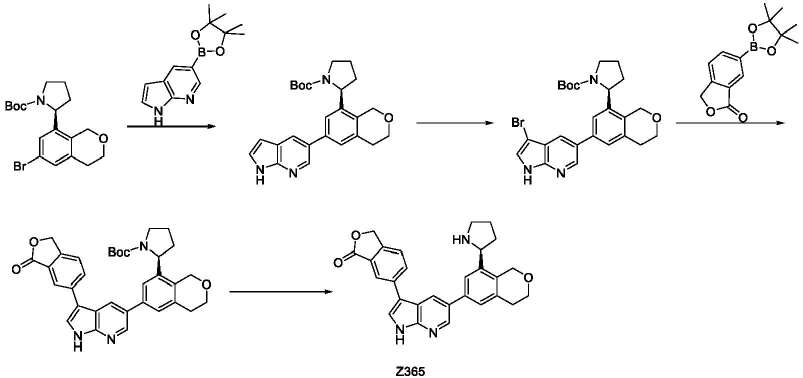

Step 1: tert-butyl (S)-2-(6-bromoisochroman-8-yl)pyrrolidine-1-carboxylate (660 mg, 1.726 mmol), 5-(4,4,5,5-tetramethyl-1,3,2-dioxaborolan-2-yl)-1H-pyrrolo[2,3-b]pyridine (632.12 mg, 2.590 mmol), chloro (2-dicyclohexylphosphino-2',6'-dimethoxy-1,1'-biphenylyl)(2'-amino-1,1'-biphenyl-2-yl) palladium (II) (124.30 mg, 0.173 mmol), potassium carbonate (714.73mg, 5.18mmol), dioxane (2 mL) and water (0.3 mL) were added to a 10 mL microwave reactor. The system was replaced with nitrogen three times and then protected with a nitrogen balloon. The system reacted under microwave at 110°C for 50 min. Ethyl acetate (10 mL) was added to the reaction mixture. The obtained mixture was washed with saturated brine (5 mLX3), dried over anhydrous sodium sulfate, and concentrated. The crude product was purified by a flash silica gel column (methanol:dichloromethane = 10:100) to give a desired product tert-butyl (S)-2-(6-(1H-pyrrolo[2,3-b]pyridin-5-yl)isochroman-8-yl)pyrrolidine-1-carboxylate (500 mg, yield: 69%). ES-API: [M+H]⁺=420.3.

Step 2: The tert-butyl (S)-2-(6-(1H-pyrrolo[2,3-b]pyridin-5-yl)isochroman-8-yl)pyrrolidine-1-carboxylate (500 mg, 1.2 mmol), N-bromosuccinimide (254.5 mg, 1.43 mmol) and acetonitrile (20 mL) were added to a 25 mL single-neck round-bottom flask and stirred at room temperature for 1 h. Ethyl acetate (30 mL) was added to the reaction mixture. The obtained mixture was washed with saturated brine (30 mL X3), dried over anhydrous sodium sulfate, and concentrated. The crude product was purified by a flash silica gel column (methanol:dichloromethane = 10:100) to give a desired product tert-butyl (S)-2-(6-(3-bromo-1H-pyrrolo[2,3-b]pyridin-5-yl)isochroman-8-yl)pyrrolidine-1-carboxyl ate (550 mg, yield: 92.6%). ES-API: [M+H]⁺=498.1.

Step 3: The tert-butyl (S)-2-(6-(3-bromo-1H-pyrrolo[2,3-b]pyridin-5-yl)isochroman-8-yl)pyrrolidine-1-carboxyl ate (100 mg, 0.2 mmol), 6-(4,4,5,5-tetramethyl-1,3,2-dioxaborolan-2-yl)isobenzofuran-1(3H)-one (208.7 mg, 0.8 mmol), chloro (2-dicyclohexylphosphino-2',6'-dimethoxy-1,1'-biphenylyl)(2'-amino-1,1'-biphenyl-2-yl) palladium (II) (14.5 mg, 0.02 mmol), sodium bicarbonate (50.6 mg, 0.6 mmol), dioxane (2 mL) and water (0.3 mL) were added to a 25 mL single-neck round-bottom flask. The system was replaced with nitrogen three times and then protected with a nitrogen balloon. The system reacted under microwave at 100°C for 20 min. Ethyl acetate (10 mL) was added to the reaction mixture. The obtained mixture was washed with 5 mL of saturated brine (5 mLX3), dried over anhydrous sodium sulfate, and concentrated. The crude product was purified by a flash silica gel column (methanol:dichloromethane = 10:100) to give a desired product tert-butyl (S)-2-(6-(3-(3-oxo-1,3-dihydroisobenzofuran-5-yl)-1H-pyrrolo[2,3-b]pyridin-5-yl)isochro man-8-yl)pyrrolidine-1-carboxylate (60 mg, yield: 54%). ES-API: [M+H]⁺=552.3.

Step 4: The tert-butyl (S)-2-(6-(3-(3-oxo-1,3-dihydroisobenzofuran-5-yl)-1H-pyrrolo[2,3-b]pyridin-5-yl)isochro man-8-yl)pyrrolidine-1-carboxylate (60 mg, 0.11 mmol), trifluoroacetic acid (3 mL) and dichloromethane (6 mL) were added to a 5 mL single-neck round-bottom flask and stirred at room temperature for 30 min. The reaction mixture was spun to dryness. The crude product was purified by prep-HPLC (formic acid method) to give (S)-6-(5-(8-(pyrrolidin-2-yl)isochroman-6-yl)-1H-pyrrolo[2,3-b]pyridin-3-yl)isobenzofura n-1(3H)-one (Z365, 30 mg, yield: 55.4%). ES-API: [M+H]⁺=452.3.

### Example 135. Synthesis of compound Z364

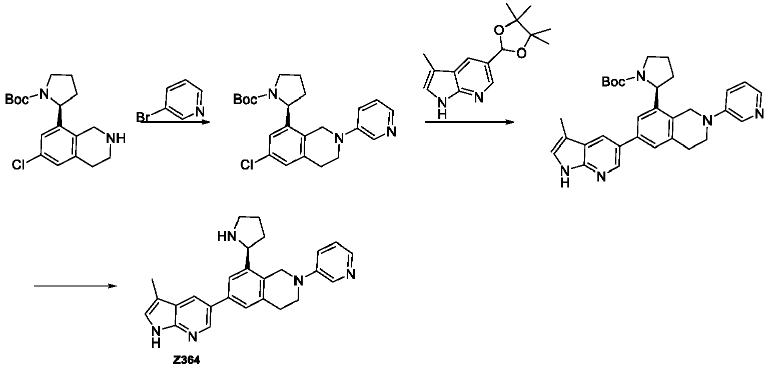

Step 1: tert-butyl (S)-2-(6-chloro-1,2,3,4-tetrahydroisoquinolin-8-yl)pyrrolidine-1-carboxylate (200 mg, 0.59 mmol), 3-bromopyridine (281.4 mg, 1.78 mmol), chloro (2-dicyclohexylphosphino-2',6'-dimethoxy-1,1'-biphenylyl)(2'-amino-1,1'-biphenyl-2-yl) palladium (II) (108.7 mg, 0.12 mmol), cesium carbonate (580.7 mg, 1.78 mmol), dioxane (2 mL) and water (0.3 mL) were added to a 10 mL microwave reactor. The system was replaced with nitrogen three times and then protected with a nitrogen balloon. The system reacted under microwave at 110°C for 3 h. Ethyl acetate (10 mL) was added to the reaction mixture. The obtained mixture was washed with saturated brine (5 mLX3), dried over anhydrous sodium sulfate, and concentrated. The crude product was purified by a flash silica gel column (methanol:dichloromethane = 10:100) to give a desired product tert-butyl (S)-2-(6-chloro-2-(pyridin-3-yl)-1,2,3,4-tetrahydroisoquinolin-8-yl)pyrrolidine-1-carboxyl ate (130 mg, yield: 52.9%). ES-API: [M+H]⁺=414.2.

Step 2: The tert-butyl (S)-2-(6-chloro-2-(pyridin-3-yl)-1,2,3,4-tetrahydroisoquinolin-8-yl)pyrrolidine-1-carboxyl ate (20 mg, 0.05 mmol), 3-methyl-5-(4,4,5,5-tetramethyl-1,3-dioxolan-2-yl)-1H-pyrrolo[2,3-b]pyridine (18.7 mg, 0.07 mmol), chloro (2-dicyclohexylphosphino-2',6'-dimethoxy-1,1'-biphenylyl)(2'-amino-1,1'-biphenyl-2-yl) palladium (II) (3.48 mg, 0.005 mmol), potassium carbonate (20 mg, 0.145 mmol), dioxane (2 mL) and water (0.3 mL) were added to a 25 mL single-neck round-bottom flask. The system was replaced with nitrogen three times and then protected with a nitrogen balloon. The system reacted under microwave at 110°C for 1 h. Ethyl acetate (10 mL) was added to the reaction mixture. The obtained mixture was washed with saturated brine (5 mLX3), dried over anhydrous sodium sulfate, and concentrated. The crude product was purified by a flash silica gel column (methanol:dichloromethane = 10:100) to give a desired product tert-butyl (S)-2-(6-(3-methyl-1H-pyrrolo[2,3-b]pyridin-5-yl)-2-(pyridin-3-yl)-1,2,3,4-tetrahydroisoq uinolin-8-yl)pyrrolidine-1-carboxylate (20 mg, yield: 81%). ES-API: [M+H]⁺=510.3.

Step 3: The tert-butyl (S)-2-(6-(3-methyl-1H-pyrrolo[2,3-b]pyridin-5-yl)-2-(pyridin-3-yl)-1,2,3,4-tetrahydroisoq uinolin-8-yl)pyrrolidine-1-carboxylate (20 mg, 0.04 mmol), trifluoroacetic acid (1 mL) and dichloromethane (2 mL) were added to a 5 mL single-neck round-bottom flask and stirred at room temperature for 30 min. The reaction mixture was spun to dryness. The crude product was purified by prep-HPLC (formic acid method) to give (S)-6-(3-methyl-1H-pyrrolo[2,3-b]pyridin-5-yl)-2-(pyridin-3-yl)-8-(pyrrolidin-2-yl)-1,2,3, 4-tetrahydroisoquinoline (Z364, 4 mg, yield: 24.9%). ES-API: [M+H]⁺=410.2.

### Example 136. Synthesis of compound Z323

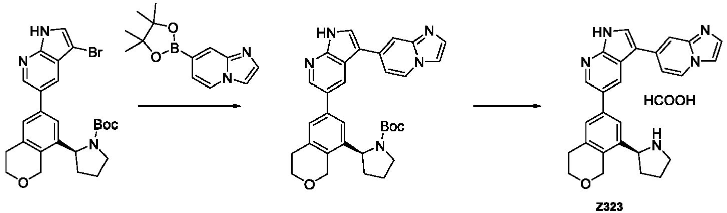

Step 1: In the presence of protective nitrogen, a mixed solution of tert-butyl (S)-2-(6-(3-bromo-1*H*-pyrrolo[2,3-*b*]pyridin-5-yl)isochroman-8-yl)pyrrolidine-1-carboxyl ate (74 mg, 0.15 mmol), 7-(4,4,5,5-tetramethyl-1,3,2-dioxaborolan-2-yl)imidazo[1,2-*a*]pyridine (44 mg, 0.18 mmol), 1,1'-bis(diphenylphosphino)ferrocene dichloropalladium (II) (11 mg, 0.02 mmol) and potassium carbonate (62 mg, 0.45 mmol) in 1,4-dioxane (2 mL) and water (0.4 mL) was stirred at 85°C for 1 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by a flash silica gel column (0-10% methanol/dichloromethane) to give tert-butyl (S)-2-(6-(3-(imidazo[1,2-*a*]pyridin-7-yl)-1*H*-pyrrolo[2,3-*b*]pyridin-5-yl)isochroman-8-yl) pyrrolidine-1-carboxylate (40 mg, yield: 50%) as a clear oily liquid. ES-API: [M+H]⁺ = 536.3.

Step 2: Trifluoroacetic acid (0.5 mL) was added dropwise to a solution of the tert-butyl (S)-2-(6-(3-(imidazo[1,2-*a*]pyridin-7-yl)-1*H*-pyrrolo[2,3-*b*]pyridin-5-yl)isochroman-8-yl) pyrrolidine-1-carboxylate (40 mg, 75 µmol) in dichloromethane (1 mL) under an ice bath condition and stirred at room temperature for 1 h. The reaction mixture was concentrated, neutralized with 7M amine-methanol (5 mL), concentrated, and then purified by prep-HPLC (formic acid) to give (S)-7-(5-(8-(pyrrolidin-2-yl)isochroman-6-yl)-1*H*-pyrrolo[2,3-*b*]pyridin-3-yl)imidazo[1,2-*a*]pyridine carboxylate (Z323, 11 mg, purity: 100%, yield: 31%) as a white solid. ES-API: [M+H]⁺ = 436.3. ¹H NMR (400 MHz, DMSO-*d₆*) δ 12.21 (s, 1H), 8.67 - 8.44 (m, 3H), 8.34 (s, 1H), 8.15 (s, 1H), 8.09 - 7.85 (m, 2H), 7.79 (s, 1H), 7.69 - 7.44 (m, 2H), 7.40 (d, *J* = 7.1 Hz, 1H), 5.03 - 4.86 (m, 1H), 4.88 - 4.73 (m, 1H), 4.35 (t, *J* = 8.8 Hz, 1H), 4.00 - 3.82 (m, 3H), 3.36 - 3.24 (m, 1H), 3.20 - 3.09 (m, 1H), 3.00 - 2.83 (m, 1H), 2.36 - 2.21 (m, 1H), 2.07 - 1.71 (m, 3H).

### Example 137. Synthesis of compound Z330

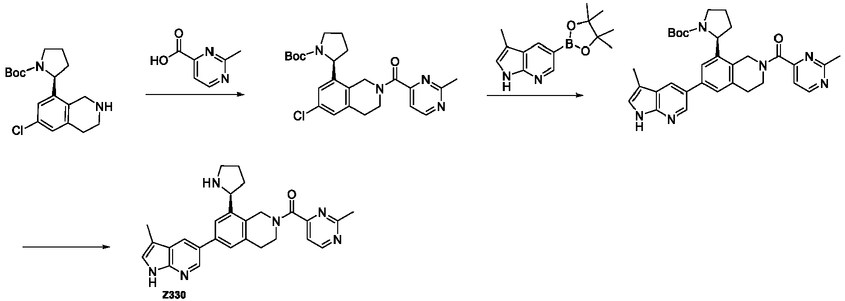

Step 1: 2-Methylpyrimidine-4-carboxylic acid (29.5 mg, 213.74 µmol), 1-propylphosphonic anhydride (169.8 mg, 267.18 µmol, 50% in ethyl acetate) and triethylamine (0.05 mL) were added to a solution of tert-butyl (S)-2-(6-chloro-1,2,3,4-tetrahydroisoquinolin-8-yl)pyrrolidine-1-carboxylate (60 mg, 178.12 µmol) in dichloromethane (2 mL), and the obtained mixture was stirred at 20°C for 2 h. The reaction mixture was spun to dryness to remove a solvent and obtain a crude product. The crude product was purified by silica gel column chromatography (petroleum ether/ethyl acetate = 1/1) to give tert-butyl (S)-2-[6-chloro-2-(2-methylpyrimidine-4-carbonyl)-1,2,3,4-tetrahydroisoquinolin-8-yl]pyr rolidine-1-carboxylate as a yellow oil (70.00 mg, yield: 86.00%). ES-API: [M+H]⁺ = 457.20.

Step 2: The tert-butyl (S)-2-[6-chloro-2-(2-methylpyrimidine-4-carbonyl)-1,2,3,4-tetrahydroisoquinolin-8-yl]pyr rolidine-1-carboxylate (70.00 mg, 153.18 µmol), 3-methyl-5-(4,4,5,5-tetramethyl-1,3,2-dioxaborolan-2-yl)-1H-pyrrolo[2,3-b]pyridine (47.45 mg, 0.18 mmol), chloro (2-dicyclohexylphosphino-2',6'-dimethoxy-1,1'-biphenylyl)(2'-amino-1,1'-biphenyl-2-yl) palladium (II) (10.24 mg, 0.014 mmol) and potassium carbonate (42.34 mg, 0.31 mmol) were dissolved in 1,4-dioxane solution (2 mL) and water (0.1 mL), and the system was warmed to 100°C for 2 h. Upon completion of the reaction, the reaction mixture was quenched by the addition of water, and extracted with ethyl acetate. The organic phase was washed with brine, dried over anhydrous sodium sulfate, filtered, and concentrated to give a crude product. The crude product was purified by a thin layer chromatographic column (dichloromethane/methanol = 10/1) to give tert-butyl (S)-2-[2-(2-methylpyrimidine-4-carbonyl)-6-(3-methyl-1H-pyrrolo[2,3-b]pyridin-5-yl)-1,2 ,3,4-tetrahydroisoquinolin-8-yl]pyrrolidine-1-carboxylate (50.00 mg, yield: 60.42%) as a white solid. ES-API: [M+H]⁺ = 553.29.

Step 3: The tert-butyl (S)-2-[2-(2-methylpyrimidine-4-carbonyl)-6-(3-methyl-1H-pyrrolo[2,3-b]pyridin-5-yl)-1,2 ,3,4-tetrahydroisoquinolin-8-yl]pyrrolidine-1-carboxylate (50 mg, 90.47 µmol) was dissolved in dichloromethane (2 mL) and trifluoroacetic acid solution (2 mL) and stirred at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a thin layer chromatographic column (dichloromethane:methanol = 5/1) to give a product (S)-[6-(3-methyl-1H-pyrrolo[2,3-b]pyridin-5-yl)-8-[pyrrolidin-2-yl]-3,4-dihydroisoquinoli n-2(1H)-yl](2-methylpyrimidin-4-yl)methanone (Z330, 14.0 mg, yield: 33.51%) as a pale yellow solid. ES-API: [M+H]⁺ = 453.24.

### Example 138. Synthesis of compound Z331

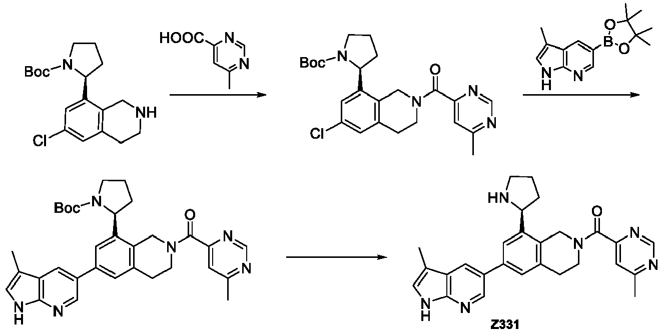

Step 1: 6-Methylpyrimidine-4-carboxylic acid (49.20 mg, 0.36 mmol), 1-propylphosphonic anhydride (337.1 mg, 0.53 mmol, 50% in ethyl acetate) and triethylamine (54.07 mg, 0.53 mmol) were added to a solution of tert-butyl (S)-2-(6-chloro-1,2,3,4-tetrahydroisoquinolin-8-yl)pyrrolidine-1-carboxylate (60 mg, 0.18 mmol) in dichloromethane (2 mL), and the mixture was stirred at 25°C for 2 h. The reaction was monitored by LCMS for completion, and quenched by the addition of water. The reaction mixture was extracted with dichloromethane, dried over anhydrous sodium sulfate, and concentrated. The crude product was purified by silica gel column chromatography (mobile phase 0-50% ethyl acetate/petroleum ether, ethyl acetate/petroleum ether = 1/1, Rf = 0.5) to give a product tert-butyl (S)-2-(6-chloro-2-(6-methylpyrimidine-4-carbonyl)-1,2,3,4-tetrahydroisoquinolin-8-yl)pyr rolidine-1-carboxylate (58 mg, yield: 71.26%). ES-API:[M+H-100]⁺= 357.2.

Step 2: 3-Methyl-5-(4,4,5,5-tetramethyl-1,3,2-dioxaborolan-2-yl)-1H-pyrrolo[2,3-b]pyridine (39.31 mg, 0.15 mmol), hloro (2-dicyclohexylphosphino-2',6'-dimethoxy-1,1'-biphenylyl)(2'-amino-1,1'-biphenyl-2-yl) palladium (II) (9.13 mg, 0.013 mmmol) and potassium carbonate (52.55 mg, 0.38 mmmol) were added to a solution of the compound tert-butyl (S)-2-(6-chloro-2-(6-methylpyrimidine-4-carbonyl)-1,2,3,4-tetrahydroisoquinolin-8-yl)pyr rolidine-1-carboxylate (58 mg, 0.13 mmol) in dioxane/water (2 mL/0.4 mL), and the mixture was heated to 110°C and stirred in the presence of protective nitrogen. The reaction was monitored by LCMS for completion. The reaction mixture was treated with ethyl acetate/water, dried over anhydrous sodium sulfate, and concentrated. The crude product was purified by silica gel column chromatography (mobile phase 0-10% methanol/dichloromethane, dichloromethane/methanol = 10/1, Rf = 0.6) to give a product tert-butyl (S)-2-(6-(3-methyl-1H-pyrrolo[2,3-b]pyridin-5-yl)-2-(6-methylpyrimidine-4-carbonyl)-1,2 ,3,4-tetrahydroisoquinolin-8-yl)pyrrolidine-1-carboxylate (50 mg, yield: 71.28%). ES-API:[M+H]⁺= 553.4.

Step 3: Trifluoroacetic acid (1 mL) was added to a solution of the compound tert-butyl (S)-2-(6-(3-methyl-1H-pyrrolo[2,3-b]pyridin-5-yl)-2-(6-methylpyrimidine-4-carbonyl)-1,2 ,3,4-tetrahydroisoquinolin-8-yl)pyrrolidine-1-carboxylate (50 mg, 0.09 mmol) in dichloromethane (2 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, concentrated, and purified by prep-HPLC (column: Ultimate XB-C18, 50*250 mm, 10 um, mobile phase system: A: purified water (0.05% ammonia water), B: pure acetonitrile, flow rate: 80 mL/min, gradient: B/A = 20%-90% over 50 min, wavelength: 214 nm, column temperature: room temperature) to give (S)-(6-(3-methyl-1H-pyrrolo[2,3-b]pyridin-5-yl)-8-(pyrrolidin-2-yl)-3,4-dihydroisoquinoli n-2(1H)-yl)(6-methylpyrimidin-4-yl)methanone (Z331, 14.2 mg, yield: 34.6%) as a white powder. ES-API:[M+H]⁺=453.3. ¹HNMR (400 MHz, CDCl₃) δ 9.53-9.27 (m, 1H), 9.14 (s, 1H), 8.54-8.48 (m, 1H), 7.95 (d, *J* =11.6 Hz, 1H), 7.88-7.69 (m, 1H), 7.53 (d, *J=* 19.3 Hz, 1H), 7.22 (d, *J* = 19.0 Hz, 1H), 7.07 (s,1H), 5.16-4.88 (m, 2H), 4.67-4.06 (m, 1H), 3.91-3.59 (m, 2H), 3.51-3.27 (m, 1H), 3.29-2.95 (m, 3H), 2.62 (s, 3H), 2.42-2.34 (m, 1H), 2.33 (s, 3H), 2.15-1.79 (m, 3H).

### Example 139. Synthesis of compound Z328

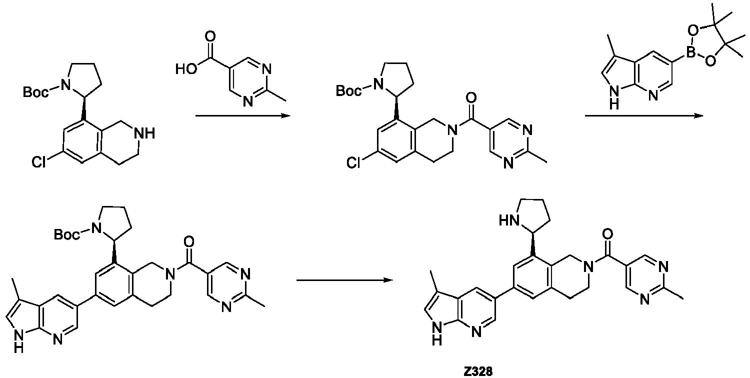

Step 1: 2-Methylpyrimidine-5-carboxylic acid (29.5 mg, 213.74 µmol), 1-propylphosphonic anhydride (170 mg, 267.18 µmol, 50% in ethyl acetate) and triethylamine (0.05 mL) were added to a solution of tert-butyl (S)-2-(6-chloro-1,2,3,4-tetrahydroisoquinolin-8-yl)pyrrolidine-1-carboxylate (60 mg, 178.12 µmol) in dichloromethane (2 mL). The mixture was stirred at 20°C for 2 h. The reaction mixture was spun to dryness to give a crude product. The crude product was purified by silica gel column chromatography (petroleum ether/ethyl acetate = 1/1) to give tert-butyl (S)-2-[6-chloro-2-(2-methylpyrimidine-5-carbonyl)-1,2,3,4-tetrahydroisoquinolin-8-yl]pyr rolidine-1-carboxylate (70.00 mg, yield: 86.00%) as a yellow oil. ES-API: [M+H]⁺ = 457.20.

Step 2: The tert-butyl (S)-2-[6-chloro-2-(2-methylpyrimidine-5-carbonyl)-1,2,3,4-tetrahydroisoquinolin-8-yl]pyr rolidine-1-carboxylate (70.00 mg, 153.18 µmol), 3-methyl-5-(4,4,5,5-tetramethyl-1,3,2-dioxaborolan-2-yl)-1H-pyrrolo[2,3-b]pyridine (47.45 mg, 0.18 mmol), chloro (2-dicyclohexylphosphino-2',6'-dimethoxy-1,1'-biphenylyl)(2'-amino-1,1'-biphenyl-2-yl) palladium (II) (10.24 mg, 0.014 mmol) and potassium carbonate (42.34 mg, 0.31 mmol) were dissolved in 1,4-dioxane solution (2 mL) and water (0.1 mL), and the system was warmed to 100°C for 2 h. The reaction was monitored by LCMS for completion. The reaction mixture was quenched by the addition of water, and extracted with ethyl acetate. The organic phase was washed with brine, dried over anhydrous sodium sulfate, concentrated, and then purified by a thin layer chromatographic column (dichloromethane/methanol = 10/1) to give tert-butyl (S)-2-[2-(2-methylpyrimidine-5-carbonyl)-6-(3-methyl-1H-pyrrolo[2,3-b]pyridin-5-yl)-1,2 ,3,4-tetrahydroisoquinolin-8-yl]pyrrolidine-1-carboxylate (60.00 mg, yield: 70.87%) as a white solid. ES-API: [M+H]⁺ = 553.29.

Step 3: The tert-butyl (S)-2-[2-(2-methylpyrimidine-5-carbonyl)-6-(3-methyl-1H-pyrrolo[2,3-b]pyridin-5-yl)-1,2 ,3,4-tetrahydroisoquinolin-8-yl]pyrrolidine-1-carboxylate (50 mg, 90.47 µmol) was dissolved in dichloromethane (2 mL) and trifluoroacetic acid solution (2 mL) and stirred at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a thin layer chromatographic column (dichloromethane/methanol = 5/1) to give (S)-[6-(3-methyl-1H-pyrrolo[2,3-b]pyridin-5-yl)-8-[pyrrolidin-2-yl]-3,4-dihydroisoquinoli n-2(1H)-yl](2-methylpyrimidin-5-yl)methanone (Z328, 13.7 mg, yield: 33.13%) as a pale yellow solid. ES-API: [M+H]⁺ = 453.24. ¹H NMR (400 MHz, CDCl₃) δ 9.49-9.11 (m, 1H), 8.87-8.73 (m, 2H), 8.46 (s, 1H), 7.99 (s, 1H), 7.89-7.62 (m, 1H), 7.25-7.19 (m, 1H), 7.08 (s, 1H), 5.07-4.79 (m, 2H), 4.61-4.02 (m, 1H), 3.69-3.38 (m, 2H), 3.30-3.17 (m, 1H), 3.14-2.85 (m, 3H), 2.81 (s, 3H), 2.46-2.38 (m, 1H), 2.34 (s, 3H), 2.04-1.85 (m, 3H).

### Example 140. Synthesis of compound Z336

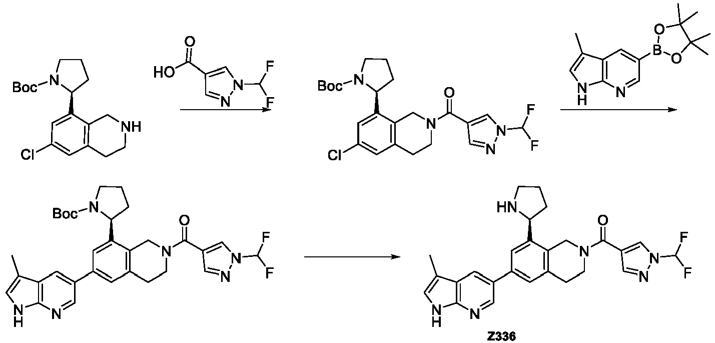

Step 1: 1-(Difluoromethyl)-1H-pyrazole-4-carboxylic acid (60.0 mg, 0.42 mmol), 1-propylphosphonic anhydride (337.1 mg, 0.53 mmol, 50% in ethyl acetate) and triethylamine (0.1 mL) were added to a solution of tert-butyl (S)-2-(6-chloro-1,2,3,4-tetrahydroisoquinolin-8-yl)pyrrolidine-1-carboxylate (120 mg, 0.18 mmol) in dichloromethane (2 mL), and the mixture was stirred at 20°C for 2 h. The reaction mixture was spun to dryness to remove a solvent and obtain a crude product. The crude product was purified by silica gel column chromatography (petroleum ether/ethyl acetate = 1/1) to give tert-butyl (S)-2-[6-chloro-2-(1-(difluoromethyl)-1H-pyrazole-4-carbonyl)-1,2,3,4-tetrahydroisoquin olin-8-yl]pyrrolidine-1-carboxylate as a yellow oil (160.00 mg, yield: 55.02%). ES-API: 3[M+H]⁺ = 481.18.

Step 2: The tert-butyl (S)-2-[6-chloro-2-(1-(difluoromethyl)-1H-pyrazole-4-carbonyl)-1,2,3,4-tetrahydroisoquin olin-8-yl]pyrrolidine-1-carboxylate (160 mg, 332.69 µmol), 3-methyl-5-(4,4,5,5-tetramethyl-1,3,2-dioxaborolan-2-yl)-1H-pyrrolo[2,3-b]pyridine (103.06 mg, 0.40 mmol), chloro (2-dicyclohexylphosphino-2',6'-dimethoxy-1,1'-biphenylyl)(2'-amino-1,1'-biphenyl-2-yl) palladium (II) (23.94 mg, 0.033 mmol) and potassium carbonate (91.96 mg, 0.67 mmol) were dissolved in 1,4-dioxane solution (2 mL) and water (0.1 mL), and the system was warmed to 100°C for 2 h. Upon completion of the reaction, the reaction mixture was quenched by the addition of water, and extracted with ethyl acetate. The organic phase was washed with brine, dried over anhydrous sodium sulfate, concentrated, and then purified by a thin layer chromatographic column (dichloromethane/methanol = 10/1) to give tert-butyl (S)-2-[2-(1-(difluoromethyl)-1H-pyrazole-4-carbonyl)-6-(3-methyl-1H-pyrrolo[2,3-b]pyri din-5-yl)-1,2,3,4-tetrahydroisoquinolin-8-yl]pyrrolidine-1-carboxylate (120.00 mg, yield: 62.55%) as a white solid. ES-API: 3[M+H]⁺ = 577.27.

Step 3: The tert-butyl (S)-2-[2-(1-(difluoromethyl)-1H-pyrazole-4-carbonyl)-6-(3-methyl-1H-pyrrolo[2,3-b]pyri din-5-yl)-1,2,3,4-tetrahydroisoquinolin-8-yl]pyrrolidine-1-carboxylate (120 mg, 208.10 µmol) was dissolved in dichloromethane (2 mL) and trifluoroacetic acid solution (2 mL) and stirred at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a thin layer chromatographic column (dichloromethane/methanol = 5/1) to give a product (S)-(1-(difluoromethyl)-1H-pyrazol-4-yl)-[6-(3-methyl-1H-pyrrolo[2,3-b]pyridin-5-yl)-8-[ pyrrolidin-2-yl]-3,4-dihydroisoquinolin-2(1H)-yl]methanone (Z336, 35.0 mg, yield: 31.80%) as a pale yellow solid. ES-API: 3[M+H]⁺ = 477.22.

### Example 141. Synthesis of compound Z335

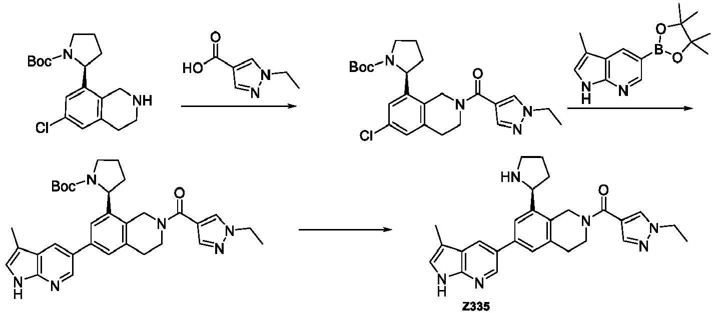

Step 1: 1-Ethylpyrazole-4-carboxylic acid (99.85 mg, 0.71 mmol), 1-propylphosphonic anhydride (337 mg, 0.53 mmol, 50% in ethyl acetate) and triethylamine (108 mg, 1.07 mmol) were added to a solution of tert-butyl (S)-2-(6-chloro-1,2,3,4-tetrahydroisoquinolin-8-yl)pyrrolidine-1-carboxylate (0.12 g, 0.36 mmol) in dichloromethane (5 mL) and stirred at 25°C for 4 h. Water was added to the mixture, and the reaction mixture was extracted with ethyl acetate. The organic layer was dried over anhydrous sodium chloride, filtered, concentrated, and purified by a preparative thin layer chromatographic column (petroleum ether:ethyl acetate = 1:2) to give tert-butyl (S)-2-[6-chloro-2-(1-ethyl-1H-pyrazole-4-carbonyl)-1,2,3,4-tetrahydro-isoquinolin-8-yl]p yrrolidine-1-carboxylate (120.00 mg, yield: 73.39%) as a yellow oil. ES-API:[M+H]⁺=459.3.

Step 2: The tert-butyl (S)-2-[6-chloro-2-(1-ethyl-1H-pyrazole-4-carbonyl)-1,2,3,4-tetrahydro-isoquinolin-8-yl]p yrrolidine-1-carboxylate (120 mg, 0.26 mmol) was dissolved in dioxane/water (4 mL/0.8 mL). 3-Methyl-5-(4,4,5,5-tetramethyl-1,3,2-dioxaborolan-2-yl)-1H-pyrrolo[2,3-b]pyridine (80.98 mg, 0.31 mmol), potassium carbonate (108 mg, 0.78 mmol) and chloro (2-dicyclohexylphosphino-2',6'-dimethoxy-1,1'-biphenylyl)(2'-amino-1,1'-biphenyl-2-yl) palladium (II) (18.8 mg, 26.14 µmol) were added, and the system was heated to 100°C for 1 h. Upon completion of the reaction, the reaction mixture was spun to dryness. The crude product was purified by a preparative thin layer chromatographic column (dichloromethane:methanol = 20:1) to give a product tert-butyl (S)-2-[2-(1-ethyl-1H-pyrazole-4-carbonyl)-6-(3-methyl-1H-pyrrolo[2,3-b]pyridin-5-yl)-1, 2,3,4-tetrahydro-isoquinolin-8-yl]pyrrolidine-1-carboxylate (100.00 mg, yield: 62.06%) as a yellow oil. ES-API:[M+H]⁺=555.4.

Step 3: The tert-butyl (S)-2-[2-(1-ethyl-1H-pyrazole-4-carbonyl)-6-(3-methyl-1H-pyrrolo[2,3-b]pyridin-5-yl)-1, 2,3,4-tetrahydro-isoquinolin-8-yl]pyrrolidine-1-carboxylate (100.0 mg, 0.18 mmol) was dissolved in dichloromethane (2 mL). Trifluoroacetic acid (2 mL) was added. The system reacted at room temperature for 1 h. Upon completion of the reaction, the reaction mixture was spun to dryness. The crude product was purified by a preparative thin layer chromatographic column (dichloromethane:methanol = 10:1) to give (S)-(1-ethyl-1H-pyrazol-4-yl)-[6-(3-methyl-1H-pyrrolo[2,3-b]pyridin-5-yl)-8-[pyrrolidin-2-yl]-3,4-dihydroisoquinolin-2(1H)-yl]methanone (Z335, 23.90 mg, yield: 29.16%) as a red solid. ES-API:[M+H]⁺=455.3. ¹H NMR (400 MHz, CDCl₃) δ 10.00 (s, 1H), 8.49 (s, 1H), 8.01 (s, 1H), 7.87 (s, 1H), 7.75 (s, 2H), 7.29 (s, 1H), 7.11 (s, 1H), 5.12-4.98 (m, 1H), 4.96-4.75 (m, 1H), 4.51-4.29 (m, 1H), 4.20 (q, *J* = 7.2 Hz, 2H), 4.04-3.81 (m, 2H), 3.36-3.24 (m, 1H), 3.16-2.96 (m, 3H), 2.34 (s, 3H), 2.30-2.14 (m, 1H), 2.05-1.85 (m, 2H), 1.73-1.61 (m, 1H), 1.51 (t, *J* = 7.2 Hz, 3H).

### Example 142. Synthesis of compound Z356

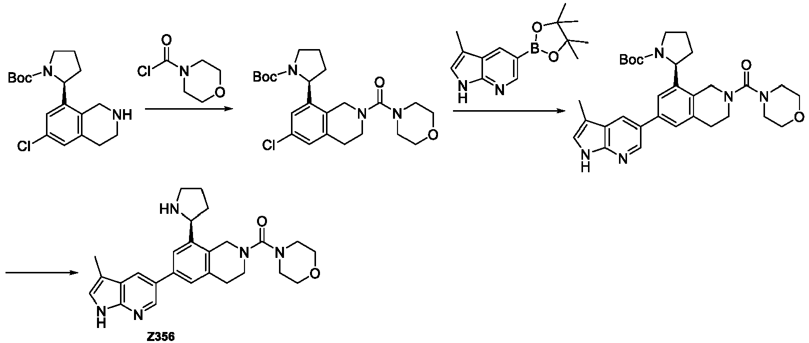

Step 1: tert-butyl (S)-2-(6-chloro-1,2,3,4-tetrahydroisoquinolin-8-yl)pyrrolidine-1-carboxylate (60 mg, 0.18 mmol) and triethylamine (54 mg, 0.53 mmol) were dissolved in dichloroethane (5 mL). 4-Morpholinecarbonyl chloride (53 mg, 0.36 mmol) was added under an ice bath condition and stirred at room temperature for 2 h. The obtained mixture was diluted with dichloromethane (15 mL), washed with saturated sodium bicarbonate solution (5 mL) and saturated brine (5 mL), dried over anhydrous sodium sulfate, and concentrated. The crude product was purified by silica gel column chromatography (methanol/dichloromethane: 0-5%) to give a desired product tert-butyl (S)-2-(6-chloro-2-(morpholine-4-carbonyl)-1,2,3,4-tetrahydroisoquinolin-8-yl)pyrrolidine-1-carboxylate (65 mg, yield: 81.1%) as a pale pink solid. ES-API: [M+Na]⁺=473.2.

Step 2: The tert-butyl (S)-2-(6-chloro-2-(morpholine-4-carbonyl)-1,2,3,4-tetrahydroisoquinolin-8-yl)pyrrolidine-1-carboxylate (65 mg, 0.14 mmol), 3-methyl-5-(4,4,5,5-tetramethyl-1,3,2-dioxaborolan-2-yl)-1H-pyrrolo[2,3-*b*]pyridine (56 mg, 0.22 mmol), 2-dicyclohexylphosphine-2',6'-dimethoxy-biphenyl (6 mg, 0.014 mmol), chloro (2-dicyclohexylphosphino-2', 6'-dimethoxy-1,1' -biphenylyl)(2'-amino-1,1' -biphenyl-2-yl) palladium (II) (11 mg, 0.014 mmol), potassium carbonate (60 mg, 0.43 mmol), 1,4-dioxane (5 mL) and water (1 mL) were added to a 25 mL round-bottom flask. The system was replaced with nitrogen three times and reacted at 110°C for 2 h. The reaction was cooled to room temperature, and ethyl acetate (50 mL) was added. The obtained mixture was washed with saturated brine (15 mL), dried over anhydrous sodium sulfate, and concentrated. The crude product was purified by silica gel column chromatography (methanol/dichloromethane: 0-5%) to give a desired product tert-butyl (S)-2-(6-(3-methyl-1*H*-pyrrolo[2,3-*b*]pyridin-5-yl)-2-(morpholine-4-carbonyl)-1,2,3,4-tetr ahydroisoquinolin-8-yl)pyrrolidine-1-carboxylate (65 mg, yield: 82.5%) as a pale yellow solid. ES-API: [M+H]⁺=546.4.

Step 3: The tert-butyl (S)-2-(6-(3-methyl-1*H-*pyrrolo[2,3-*b*]pyridin-5-yl)-2-(morpholine-4-carbonyl)-1,2,3,4-tetr ahydroisoquinolin-8-yl)pyrrolidine-1-carboxylate (65 mg, 0.12 mmol) was dissolved in methanol (1 mL). 4.0M Hydrogen chloride-dioxane solution (4 mL, 16.0 mmol) was added, and the system reacted at room temperature for 2 h. The reaction mixture was concentrated. 7.0M Ammonia-methanol solution (5 mL) was added, and a solvent was spun to dryness. The crude product was purified by prep-HPLC (formic acid method) to give a desired product (*S*)-(6-(3-methyl-1*H*-pyrrolo[2,3-*b*]pyridin-5-yl)-8-(pyrrolidin-2-yl)-3,4-dihydroisoquinoli n-2(1*H*)-yl)(morpholino)methanone (Z356, formate, 36 mg, yield: 61.5%) as a white solid. ES-API: [M+H]⁺=446.3 (free base). ¹H NMR (500 MHz, DMSO-*d₆*) δ 11.37 (s, 1H), 8.51 (d, *J* = 2.0 Hz, 1H), 8.33 (s, 1H), 8.16 (d, *J* = 2.0 Hz, 1H), 7.77 (s, 1H), 7.47 (s, 1H), 7.27 (s, 1H), 4.54 (d, *J* = 16.5 Hz, 1H), 4.48 - 4.36 (m, 2H), 3.68 - 3.54 (m, 4H), 3.50 - 3.39 (m, 2H), 3.34 - 3.08 (m, 6H), 3.02 - 2.89 (m, 2H), 2.36 - 2.23 (m, 4H), 2.04 - 1.87 (m, 2H), 1.81 - 1.68 (m, 1H).

### Example 143. Synthesis of compound Z256

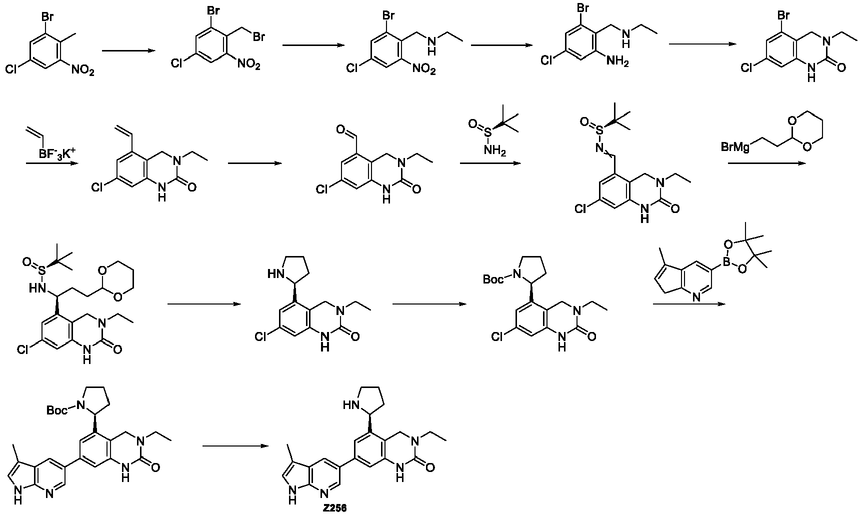

Step 1: 1-Bromo-5-chloro-2-methyl-3-nitrobenzene (4.64 g, 18.524 mmol), N-bromosuccinimide (3.63 g, 20.377 mmol) and benzoyl peroxide (0.45 g, 1.852 mmol) were added to carbon tetrachloride (50 mL), and the system was heated to reflux in an oil bath overnight. After the reaction mixture was spun to dryness to remove a solvent, the obtained mixture was diluted with ethyl acetate (30 mL), washed with saturated brine (30 mLx1), dried over anhydrous sodium sulfate, filtered, and concentrated to give 1-bromo-2-(bromomethyl)-5-chloro-3-nitrobenzene (6.1 g, 18.520 mmol, crude product) which was directly used in the next step without purification. ES-API: [M+H]+=329.8.

Step 2: The 1-bromo-2-(bromomethyl)-5-chloro-3-nitrobenzene (6.1 g, 18.520 mmol) in tetrahydrofuran (30 mL) was added to a 70% ethylamine in water (11.5 mL, 185.202 mmol) and stirred at room temperature for 1 h. The obtained mixture was poured into water (50 mL), and extracted with ethyl acetate (50 mLx3). The organic phase layers were combined, washed with saturated brine solution (100 mL x 1), dried over anhydrous sodium sulfate, filtered, concentrated, and purified by silica gel column chromatography (ethyl acetate/petroleum ether = 20/80) to give N-(2-bromo-4-chloro-6-nitrophenyl)ethanamine (2.5 g, yield: 45.98%.) ES-API: [M+H]+=292.9.

Step 3: The N-(2-bromo-4-chloro-6-nitrophenyl)ethanamine (2.5 g, 8.516 mmol) was dissolved in a solution of ethanol (100 mL) and water (20 mL). Iron (2.38 g, 42.582 mmol) and ammonium chloride (4.56 g, 85.164 mmol) were added. The mixture was stirred at 90°C for 1 h. The reaction mixture was filtered with diatomaceous earth, and concentrated to give 3-bromo-5-chloro-2-[(ethylamino)methyl]aniline (2.3 g, crude product) which was directly used in the next step without purification. ES-API: [M+H]+=263.1.

Step 4: The 3-bromo-5-chloro-2-[(ethylamino)methyl]aniline (2.1 g, 7.968 mmol) was dissolved in tetrahydrofuran solution (30 mL). Lithium bis-trimethylsilylamide (39.839 mL, 39.839 mmol) was added under an ice-water bath condition and stirred at 0°C for 10 min. N,N'-Carbonyldiimidazole (1.68 g, 10.358 mmol) was slowly added, and the mixture was stirred at 0°C for 1 h. The reaction was quenched with 1M hydrochloric acid (50 mL). The reaction mixture was poured into water (100 mL), and extracted with ethyl acetate (100 mL x 3). The organic layers were combined, washed with saturated brine solution (100 mL x 1), dried over anhydrous sodium sulfate, filtered, concentrated, and purified by silica gel column chromatography (ethyl acetate/petroleum ether = 40/60) to give 5-bromo-7-chloro-3-ethyl-2-oxo-1,2,3,4-tetrahydroquinazoline (850 mg, yield: 36.84%). ES-API: M+H]+=288.9.

Step 5: The 5-bromo-7-chloro-3-ethyl-2-oxo-1,2,3,4-tetrahydroquinazoline (850 mg, 2.935 mmol) and potassium vinyltrifluoroborate (786.58 mg, 5.87mmol) were dissolved in ethanol (20 mL). [1,1'-Bis(diphenylphosphino)ferrocene]dichloropalladium dichloromethane complex (239 mg, 0.293 mmol) and triethylamine (592.8 mg, 5.87 mmol) were added. The mixture was degassed with nitrogen and stirred at 85°C for 3 h. The obtained mixture was poured into water (30 mL), and extracted with ethyl acetate (30 mL x 3). The organic layers were combined, washed with saturated brine solution (30 mL x 1), dried over anhydrous sodium sulfate, filtered, concentrated, and purified by silica gel column chromatography (ethyl acetate/petroleum ether = 50/50) to give 7-chloro-3-ethyl-2-oxo-5-vinyl-1,2,3,4-tetrahydroquinazoline (556 mg, yield: 80%). ES-API: M+H]+=237.0.

Step 6: The 7-chloro-3-ethyl-2-oxo-5-vinyl-1,2,3,4-tetrahydroquinazoline (450 mg, 1.901 mmol) was dissolved in tetrahydrofuran (30 mL) and water (15 mL). Sodium periodate (2439.81 mg, 11.407 mmol) and potassium osmate dihydrate (70.05 mg, 0.190 mmol) were added and stirred at room temperature for 1 h. The obtained mixture was poured into water (30 mL), and extracted with ethyl acetate (30 mL x 3). The organic layers were combined, washed with saturated sodium chloride solution (30 mL x 1), dried over anhydrous sodium sulfate, filtered, concentrated, and purified by silica gel column chromatography (PE/EA = 50/50) to give 7-chloro-3-ethyl-2-oxo-1,2,3,4-tetrahydroquinazoline-5-carbaldehyde (250 mg, yield: 55.10%). ES-API: [M+H]⁺=239.0.

Step 7: The 7-chloro-3-ethyl-2-oxo-1,2,3,4-tetrahydroquinazoline-5-carbaldehyde (250 mg, 1.047 mmol) and (S)-2-methylpropane-2-sulfinamide (253.91 mg, 2.095 mmol) were dissolved in dichloromethane (20 mL). Tetraethyl titanate (955.75 mg, 4.190 mmol) was added. The mixture was stirred at room temperature for 2 h. Upon completion of the reaction, the reaction mixture was poured into saturated brine (10 mL) and washed. The organic phase was separated, dried over anhydrous sodium sulfate, concentrated, and purified by silica gel column chromatography (ethyl acetate/petroleum ether = 70/30) to give (S)-N-((7-chloro-3-ethyl-2-oxo-1,2,3,4-tetrahydroquinazolin-5-yl)methylene)-2-methylpro pane-2-sulfinamide (350 mg, yield: 97.8%). ES-API: [M+H]⁺=342.1.

Step 8: (1,3-Dioxane-2-ethyl)magnesium bromide (16.381 mL, 8.191 mmol) was added to a solution of the (S)-N-((7-chloro-3-ethyl-2-oxo-l,2,3,4-tetrahydroquinazolin-5-yl)methylene)-2-methylpro pane-2-sulfinamide (350 mg, 1.024 mmol) in tetrahydrofuran at -78°C. The mixture was stirred at -78°C for 10 min. The reaction was quenched with aqueous ammonium chloride solution (20 mL), and the reaction mixture was extracted with ethyl acetate (50 mL x 3). The organic layers were combined, washed with saturated sodium chloride solution (100 mL x 1), dried over anhydrous sodium sulfate, filtered, and concentrated. The crude product was purified by silica gel column chromatography (petroleum ether/tetrahydrofuran = 10/90) to give (S)-N-((S)-1-(7-chloro-3-ethyl-2-oxo-1,2,3,4-tetrahydroquinazolin-5-yl)-3-(1,3-dioxo-2-yl )propyl)-2-methylpropane-2-sulfinamide (320 mg, yield: 68.24%). ES-API: [M+H]⁺=342.1.

Step 9: A solution of trifluoroacetic acid (8 mL) and water (0.5 mL) was added to the (S)-N-((S)-1-(7-chloro-3-ethyl-2-oxo-1,2,3,4-tetrahydroquinazolin-5-yl)-3-(1,3-dioxo-2-yl )propyl)-2-methylpropane-2-sulfinamide (300 mg, 0.655 mmol) and stirred at room temperature for 0.5 h. Triethylsilane (2.092 mL, 13.100 mmol) was then added, and the mixture was stirred at room temperature for 18 h. The reaction mixture was concentrated to give (S)-7-chloro-3-ethyl-5-(pyrrolidin-2-yl)-3,4-dihydroquinazolin-2(1H)-one (400 mg, crude product) which was directly used in the next reaction without purification. ES-API: [M+H]⁺=280.1.

Step 10: The (S)-7-chloro-3-ethyl-5-(pyrrolidin-2-yl)-3,4-dihydroquinazolin-2(1H)-one (400 mg, crude product) was dissolved in dichloromethane (10 mL). N,N-Diisopropylethyl amine (923.90 mg, 7.149 mmol) and di-tert-butyl dicarbonate (624.08 mg, 2.859 mmol) were added and stirred at room temperature for 1 h. The obtained mixture was poured into water (10 mL) and extracted with dichloromethane (10 mL). The organic layers were combined, washed with saturated sodium chloride solution (20 mL x 1), dried over anhydrous sodium sulfate, filtered, and concentrated to give a crude product. The crude product was purified by silica gel column chromatography (ethyl acetate/petroleum ether = 50/50) to give (2S)-tert-butyl 2-(7-chloro-3-ethyl-2-oxo-1,2,3,4-ttetrahydroquinazolin-5-yl)-tetrahydropyran-1-carboxyl ate (220 mg, yield of the 2 steps: 88.39%). ES-API: [M+H]⁺=380.2.

Step 11: The (2S)-tert-butyl 2-(7-chloro-3-ethyl-2-oxo-1,2,3,4-ttetrahydroquinazolin-5-yl)-tetrahydropyran-1-carboxyl ate (50 mg, 0.132 mmol), 3-methyl-5-(4,4,5,5-tetramethyl-1,3,2-dioxaborolan-2-yl)-1H-pyrrolo[2,3-b]pyridine (50.96 mg, 0.197 mmol), chloro (2-dicyclohexylphosphino-2',6'-dimethoxy-1,1'-biphenylyl)(2'-amino-1,1'-biphenyl-2-yl) palladium (II) (9.48 mg, 0.013 mmol) and potassium carbonate (54.57 mg, 0.395 mmol) were added to 1,4-dioxane (3 mL) and water (0.5 mL). the system was replaced with nitrogen and reacted under microwave at 115°C for 0.5 h. Upon completion of the reaction, ethyl acetate (20 mL) was added. The obtained mixture was washed with water (10 mL) and saturated brine (10 mL) successively, dried over anhydrous sodium sulfate, concentrated, and purified by preparative thin layer chromatography (petroleum ether/ethyl acetate = 1/1) to give tert-butyl (S)-2-(3-ethyl-7-(3-methyl-1H-pyrrolo[2,3-b]pyridin-5-yl)-2-oxo-1,2,3,4-tetrahydroquinaz olin-5-yl) pyrrolidine-1-carboxylate (30 mg, yield: 47.92%). ES-API: [M+H]⁺=476.3.

Step 12: The tert-butyl (S)-2-(3-ethyl-7-(3-methyl-1H-pyrrolo[2,3-b]pyridin-5-yl)-2-oxo-1,2,3,4-tetrahydroquinaz olin-5-yl) pyrrolidine-1-carboxylate (30 mg, 0.063 mmol) was dissolved in dichloromethane (2 mL) and added to trifluoroacetic acid (1 mL), and the system reacted at room temperature for 0.5 h. Upon completion of the reaction, the reaction mixture was concentrated. After neutralization with ammonia-methanol solution (1 mL), the obtained mixture was concentrated again to give a crude product. The crude product was purified by prep-HPLC alkaline process (ammonia water) to give (S)-3-ethyl-7-(3-methyl-1H-pyrrolo[2,3-b]pyridin-5-yl)-5-(pyrrolidin-2-yl)-3,4-dihydroqui nazolin-2(1H)-one (Z256, 3 mg, yield: 12.7%). ES-API: [M+H]⁺=376.2. ¹H NMR (400 MHz, DMSO-*d*₆) δ 11.41 - 11.28 (m, 1H), 9.20-9.13 (m, 1H), 8.39-8.30 (m, 1H), 8.03 - 7.91 (m, 1H), 7.39 (s, 1H), 7.31 - 7.22 (m, 1H), 6.95 (d, *J* = 7.9 Hz, 1H), 4.51 (q, *J* = 15.0 Hz, 2H), 4.17 (s, 1H), 3.40 (q, *J* = 6.9 Hz, 2H), 3.10-3.01 (m, 2H), 2.30 (t, *J* = 1.8 Hz, 3H), 2.18 (s, 2H), 1.80-1.71(m, 2H), 1.50 (s, 1H), 1.13 (td, *J* = 7.1, 3.2 Hz, 3H).

### Example 144. Synthesis of compound Z402

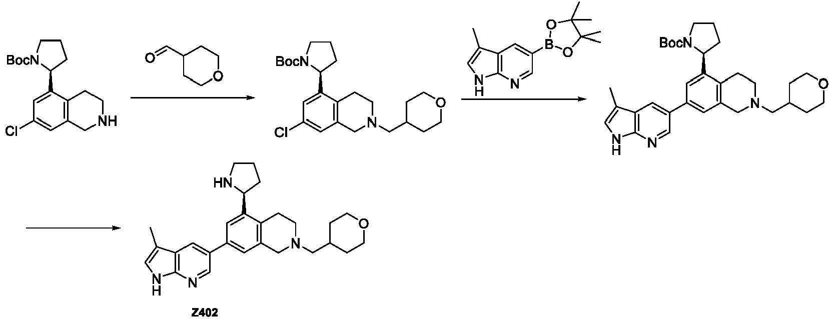

Step 1: tert-butyl (S)-2-(7-chloro-1,2,3,4-tetrahydroisoquinolin-5-yl)pyrrolidine-1-carboxylate (210 mg, 0.623 mmol) was dissolved in 1,2-dichloroethane (2 mL). Tetrahydropyran-4-carbaldehyde (142.31 mg, 1.247 mmol) was added with stirring. After the reaction mixture was stirred at room temperature for 0.5 h, sodium triacetylborohydride (525.98 mg, 2.494 mmol) was added and stirred at room temperature overnight. The reaction was quenched by the addition of water (5 mL), and the aqueous phase was adjusted to pH 9-10. The reaction mixture was extracted with dichloromethane (10 mLX2). The organic phases were combined, washed with saturated brine, dried over anhydrous sodium sulfate, filtered, and concentrated to give a crude product. The crude product was purified by silica gel column chromatography (0-100% ethyl acetate/petroleum ether followed by 0-10% methanol/dichloromethane + 0.1% ammonia water) to give tert-butyl (S)-2-[7-chloro-2-(3,4,5,6-tetrahydro-2H-pyran-4-ylmethyl)-1,2,3,4-tetrahydroisoquinolin -5-yl]pyrrolidine-1-carboxylate (290 mg, crude product) which was directly used in the next reaction. LCMS: ES-API [M+H]⁺=435.3.

Step 2: 3-Methyl-5-(4,4,5,5-tetramethyl-1,3,2-dioxaborolan-2-yl)-1H-pyrrolo[2,3-b]pyridine (166.15 mg, 0.644 mmol) and the tert-butyl (S)-2-[7-chloro-2-(3,4,5,6-tetrahydro-2H-pyran-4-ylmethyl)-1,2,3,4-tetrahydroisoquinolin -5-yl]pyrrolidine-1-carboxylate (140 mg, 0.322 mmol) were dissolved in 1,4-dioxane (3 mL). Potassium carbonate (133.44 mg, 0.966 mmol) and water (0.75 mL) followed by chloro (2-dicyclohexylphosphino-2',6'-dimethoxy-1,1'-biphenylyl)(2'-amino-1,1'-biphenyl-2-yl) palladium (II) (46.38 mg, 0.064 mmol) were added. The system was heated at 120°C for 3 h in the presence of protective nitrogen. Ethyl acetate (10 mL) and saturated brine were added to the reaction mixture. The aqueous phase was extracted with ethyl acetate (10 mLX2). The organic phases were combined, washed with saturated brine, dried over anhydrous sodium sulfate, and concentrated under reduced pressure. The crude product was purified by silica gel column chromatography (0-100% ethyl acetate/petroleum ether followed by 0-10% methanol/dichloromethane + 0.1% ammonia water) to give tert-butyl (S)-2-[7-(3-methyl-1H-pyrrolo[2,3-*b*]pyridin-5-yl)-2-(3,4,5,6-tetrahydro)-2H-pyran-4-ylm ethyl)-1,2,3,4-tetrahydroisoquinolin-5-yl]pyrrolidine-1-carboxylate (100 mg, yield: 58.55%). LCMS: ES-API [M+H]⁺= 531.4.

Step 3: The tert-butyl (S)-2-[7-(3-methyl-1H-pyrrolo[2,3-*b*]pyridin-5-yl)-2-(3,4,5,6-tetrahydro)-2H-pyran-4-ylm ethyl)-1,2,3,4-tetrahydroisoquinolin-5-yl]pyrrolidine-1-carboxylate (100 mg, 0.188 mmol) was dissolved in dichloromethane (4 mL). Trifluoroacetic acid (2 mL) was added with stirring. The system reacted at room temperature for 2 h. The reaction mixture was concentrated, and then purified by prep-HPLC (formic acid method) to give 7-(3-methyl-1H-pyrrolo[2,3-*b*]pyridin-5-yl)-2-(3,4,5,6-tetrahydro-2H-pyran-4-ylmethyl)-5 -[(2S)-tetrahydro-1H-pyrrol-2-yl]-1,2,3,4-tetrahydroisoquinoline (Z402, formate, 54 mg, yield: 59.40%) as a white solid. ¹H NMR (400 MHz, DMSO-*d*₆) δ 11.37 (s, 1H), 8.56 (d, *J* = 2.0 Hz, 1H), 8.24 - 8.15 (m, 2H), 7.75 (s, 1H), 7.50 (s, 1H), 7.27 (s, 1H), 4.81 - 4.66 (m, 1H), 3.91 - 3.79 (m, 2H), 3.71 - 3.60 (m, 2H), 3.52 - 3.41 (m, 1H), 3.33 (q, *J* = 11.2, 9.8 Hz, 3H), 3.00 - 2.79 (m, 2H), 2.79 - 2.66 (m, 2H), 2.42 - 2.33 (m, 3H), 2.32 (s, 3H), 2.19 - 2.00 (m, 3H), 1.97 - 1.82 (m, 1H), 1.65 (d, *J* = 12.0 Hz, 2H), 1.22 - 1.10 (m, 2H). LCMS: ES-API [M+H]⁺= 431.3.

### Example 145. Synthesis of compound Z337

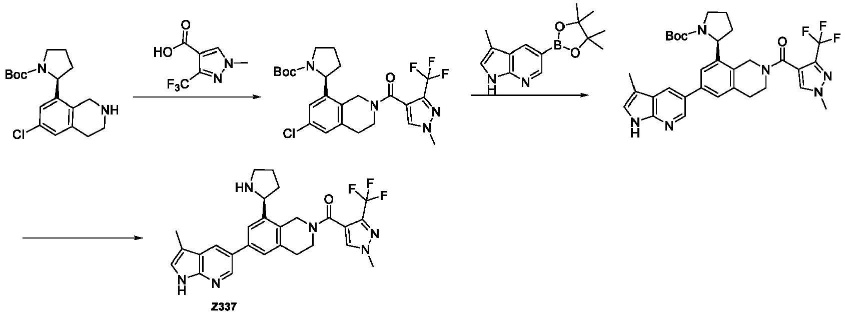

Step 1: tert-butyl (S)-2-(6-chloro-1,2,3,4-tetrahydroisoquinolin-8-yl)pyrrolidine-1-carboxylate (120 mg, 0.356 mmol), 1-methyl-3-(trifluoromethyl)pyrazole-4-carboxylic acid (82.98 mg, 0.427 mmol) and 1-propylphosphonic anhydride (452.8 mg, 0.712 mmol, 50% in ethyl acetate) were dissolved in triethylamine (108.14 mg, 1.07 mmol) and dichloromethane (5 mL), and the system reacted at room temperature for 2 h. The reaction was quenched by the addition of water, and the reaction mixture was extracted with ethyl acetate. The organic phase was washed with brine, dried over anhydrous sodium sulfate, filtered, and concentrated. The crude product was purified by a preparative thin layer chromatographic column (ethyl acetate/petroleum ether = 2/1) to give a product tert-butyl (S)-2-[6-chloro-2-[1-methyl-3-(trifluoromethyl)pyrazole-4-carbonyl]-3,4-dihydro-1H-isoq uinolin-8-yl]pyrrolidine-1-carboxylate (160 mg, yield: 87.56%). ES-API: [M+H]⁺ = 513.9.

Step 2: The (S)-2-[6-chloro-2-[1-methyl-3-(trifluoromethyl)pyrazole-4-carbonyl]-3,4-dihydro-1H-isoq uinolin-8-yl]pyrrolidine-1-carboxylate (160 mg, 0.311 mmol), 3-methyl-5-(4,4,5,5-tetramethyl-1,3,2-dioxaborolan-2-yl)-1H-pyrrolo[2,3-b]pyridine (85.13 mg, 0.329 mmol), chloro (2-dicyclohexylphosphino-2',6'-dimethoxy-1,1'-biphenylyl)(2'-amino-1,1'-biphenyl-2-yl) palladium (II) (19.78 mg, 0.027 mmol) and potassium carbonate (75.97 mg, 0.549 mmol) were dissolved in 1,4-dioxane solution (1 mL) and water (0.1 mL), and the system was warmed to 100°C for 2 h. Upon completion of the reaction, the reaction mixture was quenched by the addition of water, and extracted with ethyl acetate. The organic phase was washed with brine, dried over anhydrous sodium sulfate, filtered, and concentrated to give a crude product. The crude product was purified by a thin layer chromatographic column (dichloromethane/methanol = 10/1) to give a product tert-butyl (S)-2-[6-(3-methyl-1H-pyrrolo[2,3-b]pyridin-5-yl)-2-[1-methyl-3-(trifluoromethyl)pyrazol e-4-carbonyl]-3,4-dihydro-1H-isoquinolin-8-yl]pyrrolidine-1-carboxylate (120 mg, yield: 63.21%). ES-API: [M+H]⁺ = 609.7.

Step 3: The tert-butyl (S)-2-[6-(3-methyl-1H-pyrrolo[2,3-b]pyridin-5-yl)-2-[1-methyl-3-(trifluoromethyl)pyrazol e-4-carbonyl]-3,4-dihydro-1H-isoquinolin-8-yl]pyrrolidine-1-carboxylate (120 mg, 0.197 mmol) was dissolved in dichloromethane (2 mL) and trifluoroacetic acid solution (2 mL) and stirred at room temperature for 1 h. The reaction mixture was spun to dryness to give a crude product. The crude product was purified by prep-HPLC (column: Ultimate XB-C18, 50*250 mm, 10 um, mobile phase: A: purified water (0.05% ammonia water); B: pure acetonitrile, flow rate: 80 ml/min, gradient: B/A = 20%-90% over 50 min, wavelength: 214 nm, column temperature: room temperature) to give a product [6-(3-methyl-1H-pyrrolo[2,3-b]pyridin-5-yl)-8-[(2S)-pyrrolidin-2-yl]-3,4-dihydro-1H-isoq uinolin-2-yl]-[1-methyl-3-(trifluoromethyl)pyrazol-4-yl]methanone (Z337, 22.7 mg, yield: 22.64%). ES-API: [M+H]⁺ = 509.3. ¹H NMR (400 MHz, CDCl₃) δ 8.58-8.32 (m, 1H), 7.99-7.72 (m, 2H), 7.63-7.42 (m, 1H), 7.06-6.88 (m, 2H), 5.08-4.53 (m, 3H), 4.11-3.90 (m, 4H), 3.84-3.72 (m, 1H), 3.64-3.27 (m, 3H), 2.81-2.65 (m, 1H), 2.44-2.35 (m, 1H), 2.27 (s, 3H), 2.22-2.02 (m, 3H).

### Example 146. Synthesis of compound Z401

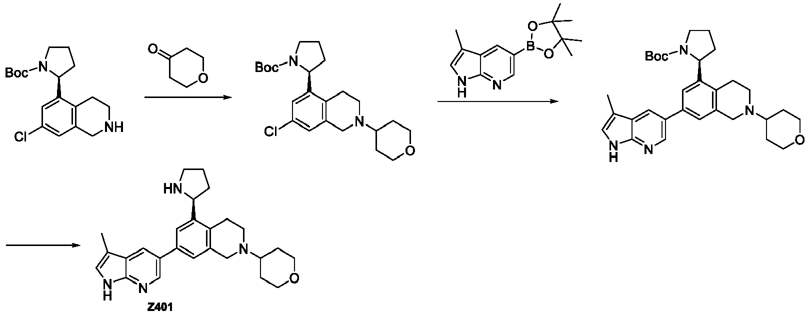

Step 1: tert-butyl (S)-2-(6-chloro-1,2,3,4-tetrahydroisoquinolin-8-yl)pyrrolidine-1-carboxylate (100 mg, 0.30 mmol) and tetrahydropyran-4-one (89 mg, 0.89 mmol) were dissolved in 1,2-dichloroethane (3 mL). Sodium triacetyl borohydride (188 mg, 0.89 mmol) was added and stirred at room temperature for 4 h. The obtained mixture was diluted with dichloromethane (30 mL), washed with saturated sodium bicarbonate solution (15 mL) and saturated brine (10 mL), dried over anhydrous sodium sulfate, and concentrated. The crude product was purified by a flash silica gel column (7M ammonia-methanol/dichloromethane: 0-5%) to give a desired product tert-butyl (S)-2-(7-chloro-2-(tetrahydro-2H-pyran-4-yl)-1,2,3,4-tetrahydroisoquinolin-5-yl)pyrrolidi ne-1-carboxylate (90 mg, yield: 72.0%) as a colorless liquid. ES-API: [M+H]⁺=421.3.

Step 2: The tert-butyl (S)-2-(7-chloro-2-(tetrahydro-2H-pyran-4-yl)-1,2,3,4-tetrahydroisoquinolin-5-yl)pyrrolidi ne-1-carboxylate (90 mg, 0.21 mmol), 3-methyl-5-(4,4,5,5-tetramethyl-1,3,2-dioxaborolan-2-yl)-1H-pyrrolo[2,3-b]pyridine (83 mg, 0.32 mmol), 2-dicyclohexylphosphine-2',6'-dimethoxy-biphenyl (9 mg, 0.02 mmol), chloro (2-dicyclohexylphosphino-2',6'-dimethoxy-1,1'-biphenylyl)(2'-amino-1,1'-biphenyl-2-yl) palladium (II) (15 mg, 0.02 mmol), potassium carbonate (89 mg, 0.64 mmol), 1,4-dioxane (5 mL) and water (1 mL) were added to a 25 mL round-bottom flask. The system was replaced with nitrogen three times and reacted at 110°C for 2 h. The reaction was cooled to room temperature. Ethyl acetate (50 mL) was added. The obtained mixture was washed with saturated brine (15 mL), dried over anhydrous sodium sulfate, and concentrated. The crude product was purified by a flash silica gel column (7M ammonia-methanol/dichloromethane: 0-3%) to give a desired product tert-butyl (S)-2-(7-(3-methyl-1*H*-pyrrolo[2,3-*b*]pyridin-5-yl)-2-(tetrahydro-2*H*-pyran-4-yl)-1,2,3,4-t etrahydroisoquinolin-5-yl)pyrrolidine-1-carboxylate (90 mg, yield: 81.5%) as a pale yellow solid. ES-API: [M+H]⁺=517.3.

Step 3: The tert-butyl (S)-2-(7-(3-methyl-1*H*-pyrrolo[2,3-*b*]pyridin-5-yl)-2-(tetrahydro-2*H*-pyran-4-yl)-1,2,3,4-t etrahydroisoquinolin-5-yl)pyrrolidine-1-carboxylate (90 mg, 0.17 mmol) was dissolved in methanol (1 mL). 4.0M Hydrogen chloride-dioxane solution (4 mL, 16.0 mmol) was added, and the system reacted at room temperature for 18 h. The reaction mixture was concentrated. 7.0M Ammonia-methanol solution (5 mL) was added, and a solvent was spun to dryness. The crude product was purified by prep-HPLC (formic acid) to give a desired product (*S*)-7-(3-methyl-1*H*-pyrrolo[2,3-*b*]pyridin-5-yl)-5-(pyrrolidin-2-yl)-2-(tetrahydro-2*H*-pyra n-4-yl)-1,2,3,4-tetrahydroisoquinoline (Z401, formate, 51 mg, yield: 63.4%) as a white solid. ES-API: [M+H]⁺=417.3 (free base). ¹H NMR (500 MHz, DMSO-*d₆*) δ 11.35 (s, 1H), 8.52 (d, *J* = 2.0 Hz, 1H), 8.29 (s, 2H), 8.17 (d, *J* = 2.0 Hz, 1H), 7.73 (s, 1H), 7.43 (s, 1H), 7.26 (s, 1H), 4.62 - 4.51 (m, 1H), 3.93 (d, *J* = 9.5 Hz, 2H), 3.85 - 3.72 (m, 2H), 3.41 - 3.28 (m, 3H), 3.23 - 3.16 (m, 1H), 2.98 - 2.74 (m, 4H), 2.66 - 2.56 (m, 1H), 2.35 - 2.26 (m, 4H), 2.09 - 1.73 (m, 5H), 1.58 - 1.44 (m, 2H).

### Example 147. Synthesis of compound Z239

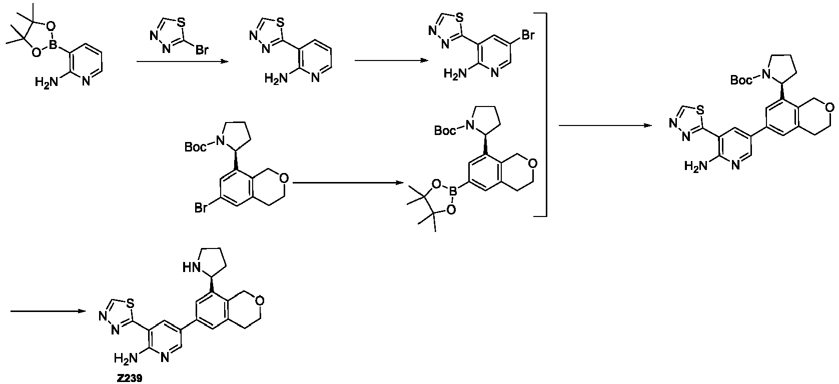

Step 1: In the presence of protective nitrogen, sodium carbonate (72.24 mg, 681.59 µmol), 2-bromo-1,3,4-thiadiazole (89.98 mg, 545.27 µmol) and 1,1-bis(diphenylphosphino)ferrocene dichloride (133.05 mg, 181.76 µmol) were added to a solution of (3-(4,4,5,5-tetramethyl-1,3,2-dioxaborolan-2-yl)pyridin-2-amine (600 mg, 2.72 mmol) in 1,4-dioxane (20 mL) and water (2 mL). The reaction mixture was stirred at 100°C under microwave for 1 h. The reaction was monitored by LCMS for completion, and the reaction mixture was concentrated to give a crude product. The crude product was purified by silica gel column chromatography (petroleum ether:ethyl acetate = 1:1) to give a product 3-(1,3,4-thiadiazol-2-yl)pyridin-2-amine (250.00 mg, yield: 51.64%) as a yellow solid. ES-API:[M+H]⁺=179.10.

Step 2: In the presence of protective nitrogen, N-bromosuccinimide (299.61 mg, 1.68 mmol) was added to a solution of the 3-(1,3,4-thiadiazol-2-yl)pyridin-2-amine (0.25 g, 1.40 mmol) in tetrahydrofuran (20 mL) at 0°C. The reaction mixture was stirred at 0°C for 1 h. The reaction was monitored by LCMS for completion, and quenched by the addition of sodium thiosulfate solution. The reaction mixture was extracted with ethyl acetate, dried over anhydrous sodium sulfate, and concentrated to give a crude product. The crude product was purified by silica gel column chromatography (petroleum ether:ethyl acetate = 10:1) to give a product 5-bromo-3-(1,3,4-thiadiazol-2-yl)pyridin-2-amine (250.00 mg, yield: 69.31%) as a yellow solid. ES-API:[M+H]⁺=256.90/258.90.

Step 3: Potassium acetate (154.03 mg, 1.57 µmol), 4,4,5,5-tetramethyl-2-(4,4,5,5-tetramethyl-1,3,2-dioxaborolan-2-yl)-1,3,2-dioxaborolane (199.28 mg, 784.74 µmol) and 1,1-bis(diphenylphosphino)ferrocene dichloropalladium (38.30 mg, 52.32 µmol) were added to a solution of tert-butyl (S)-2-(6-bromoisochroman-8-yl)pyrrolidine-1-carboxylate (0.2 g, 523.16 µmol) in 1,4-dioxane (3.0 mL). The reaction mixture was stirred at 100°C for 16 h. The reaction was monitored by LCMS for completion and quenched with water. The reaction mixture was extracted with ethyl acetate, dried over anhydrous sodium sulfate, filtered, and concentrated to give tert-butyl (S)-2-[6-(4,4,5,5-tetramethyl-1,3,2-dioxaborolan-2-yl)isochroman-8-yl]pyrrolidine-1-carb oxylate as a crude product which was directly used in the next step without purification. ES-API:[M+H]⁺=374.20

Step 4: The tert-butyl (S)-2-[6-(4,4,5,5-tetramethyl-1,3,2-dioxaborolan-2-yl)isochroman-8-yl]pyrrolidine-1-carb oxylate (200.39 mg, 466.73 µmol), 1,1-bis(diphenylphosphino)ferrocene dichloropalladium (113.88 mg, 155.58 µmol) and cesium carbonate (380.17 mg, 1.17 mmol) were added to a solution of the 5-bromo-3-(1,3,4-thiadiazol-2-yl)pyridin-2-amine (0.1 g, 388.94 µmol) in 1,4-dioxane (3 mL) and water (0.3 mL). The reaction mixture was stirred at 100°C under microwave for 1 h. The reaction was monitored by LCMS for completion, and the reaction mixture was concentrated. The crude product was purified by silica gel column chromatography (petroleum ether:ethyl acetate = 1:1) to give a product tert-butyl (S)-2-[6-[6-amino-5-(1,3,4-thiadiazol-2-yl)-3-pyridyl]isochroman-8-yl]pyrrolidine-1-carb oxylate (100.00 mg, yield: 42.89%) as a yellow oil. ES-API:[M+H]⁺=480.30.

Step 5: Trifluoroacetic acid (1.0 mL) was added to a solution of the tert-butyl (S)-2-[6-[6-amino-5-(1,3,4-thiadiazol-2-yl)-3-pyridyl]isochroman-8-yl]pyrrolidine-1-carb oxylate (0.1 g, 208.51 µmol) in dichloromethane (1.0 mL) and stirred at 30°C for 1 h. A saturated aqueous sodium bicarbonate solution was added to the reaction, and the obtained mixture was then extracted with ethyl acetate (150 mL). The organic layer was separated, dried over anhydrous sodium sulfate, filtered, and concentrated to give a crude product. The crude product was purified by a preparative thin layer chromatographic column (dichloromethane:methanol = 10:1) and beating (ethyl acetate) to give 5-[8-[(S)-pyrrolidin-2-yl]isochroman-6-yl]-3-(1,3,4-thiadiazol-2-yl)pyridin-2-amine (Z239, 20.00 mg, yield: 24.01%) as a yellow solid. ES-API:[M+H]⁺=380.1.

### Example 148. Synthesis of compound Z285

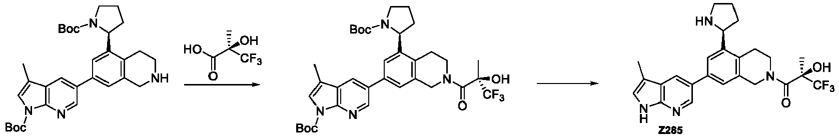

Step 1: tert-butyl (S)-5-(5-(1-(tert-butoxycarbonyl)pyrrolidin-2-yl)-1,2,3,4-tetrahydroisoquinolin-7-yl)-3-me thyl-1H-pyrrolo[2,3-b]pyridine-1-carboxylate (15 mg, 0.028 mmol) was dissolved in dichloromethane (3 mL). (R)-3,3,3-Trifluoro-2-hydroxy-2-methylpropanoic acid (8.90 mg, 0.056 mmol), N,N-diisopropylethyl amine (0.014 mL, 0.084 mmol) and 2-(7-azobenzotriazole)-N,N,N',N'-tetramethylurea hexafluorophosphate (10.92 mg, 0.084 mmol) were added successively, and the system reacted at room temperature for 1 h. Upon completion of the reaction, the reaction mixture was diluted with dichloromethane (30 mL), washed with saturated brine (30 mL), dried over anhydrous sodium sulfate, filtered, and concentrated to give a crude product. The crude product was purified by silica gel column chromatography (petroleum ether/ethyl acetate = 1/1) to give tert-butyl 5-(5-((S)-1-(tert-butoxycarbonyl)pyrrolidin-2-yl)-2-((R)-3,3,3-trifluoro-2-hydroxy-2-meth ylpropanoyl)-1,2,3,4-tetrahydroisoquinolin-7-yl)-3-methyl-1H-pyrrolo[2,3-b]pyridine-1-c arboxylate (6 mg, yield: 32%). ES-API: [M+H]⁺=673.3.

Step 2: The tert-butyl 5-(5-((S)-1-(tert-butoxycarbonyl)pyrrolidin-2-yl)-2-((R)-3,3,3-trifluoro-2-hydroxy-2-meth ylpropanoyl)-1,2,3,4-tetrahydroisoquinolin-7-yl)-3-methyl-1H-pyrrolo[2,3-b]pyridine-1-c arboxylate (6 mg, 0.009 mmol) was dissolved in dichloromethane (3 mL) and added to trifluoroacetic acid (2 mL), and the system reacted at room temperature for 0.5 h. Upon completion of the reaction, the reaction mixture was concentrated. After neutralization with ammonia water-methanol solution (1 mL), the obtained mixture was concentrated again and purified by prep-HPLC alkaline process (ammonia water) to give (R)-3,3,3-trifluoro-2-hydroxy-2-methyl-1-(7-(3-methyl-1H-pyrrolo[2,3-b]pyridin-5-yl)-5-( (S)-pyrrolidin-2-yl)-3,4-dihydroisoquinolin-2(1H)-yl)propan-1-one (Z285, 1.8 mg, yield: 44%). ES-API: [M+H]⁺=473.2.

### Example 149. Synthesis of compound Z284

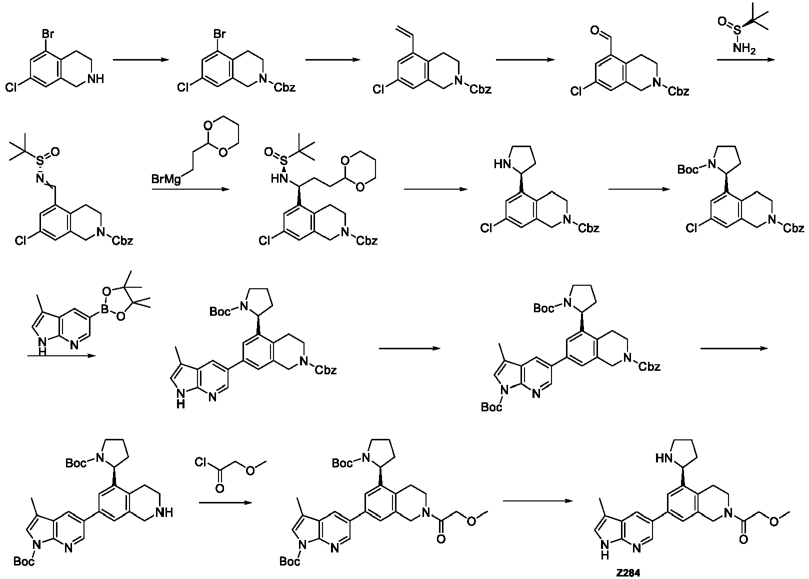

Step 1: 5-Bromo-7-chloro-1,2,3,4-tetrahydroisoquinoline (11.37 g, 46.120 mmol) was dissolved in tetrahydrofuran (100 mL) and water (30 mL). Potassium carbonate (19.12 g, 138.360 mmol) was added, and the system was cooled to 0°C. Benzyl chloroformate (16.334 mL, 115.300 mmol) was then added. The mixture was stirred at room temperature for 2 h. The obtained mixture was diluted with water, and extracted with ethyl acetate (100 mL). The ethyl acetate layers were combined, washed with saturated sodium chloride solution (100 mL x 1), dried anhydrous sodium sulfate, filtered, and concentrated. Dichloromethane (50 ml) was added. The obtained mixture was stirred and beaten, filtered, washed with petroleum ether (100 mL), and filtered. The filter cake was dried to give benzyl 5-bromo-7-chloro-1,2,3,4-tetrahydroisoquinoline-2-carboxylate (15 g, yield: 85.4%) as a white solid. ES-API: [M+H]⁺=380.0.

Step 2: A solution of the benzyl 5-bromo-7-chloro-1,2,3,4-tetrahydroisoquinoline-2-carboxylate (15 g, 39.404 mmol) and potassium vinyltrifluoroborate (10.56 g, 78.808 mmol) was added to anhydrous ethanol (300 mL). Triethylamine (5.477 mL, 39.404 mmol) and [1,1'-bis(diphenylphosphino)ferrocene]dichloropalladium dichloromethane complex (1.61 g, 1.970 mmol) were added. The mixture was degassed with nitrogen, heated to 100°C in an oil bath and stirred for 3 h, and then stirred at 90°C for 15 h. The obtained mixture was poured into water (100 mL), and extracted with ethyl acetate (100 mL x 3). The organic layers were combined, washed with saturated sodium chloride solution (100 mL x 1), dried over anhydrous sulfuric acid, filtered, and concentrated to give a crude product. The crude product was purified by silica gel column chromatography (ethyl acetate/petroleum ether = 20/80) to give benzyl 7-chloro-5-vinyl-1,2,3,4-tetrahydroisoquinoline-2-carboxylate (12 g, yield: 92.90%). ES-API: [M+H]⁺=328.1.

Step 3: Sodium periodate (46.98 g, 219.639 mmol), potassium osmate dihydrate (0.30g, 0.805mmol) and water (200 mL) were added to a solution of the benzyl 7-chloro-5-vinyl-1,2,3,4-tetrahydroisoquinoline-2-carboxylate (12 g, 36.607 mmol) in tetrahydrofuran (400 mL) and stirred at room temperature for 2 h. The obtained mixture was diluted with ethyl acetate (100 mL) and filtered. The organic layer was separated, washed with water, dried over anhydrous sodium sulfate, filtered, and concentrated to give a crude product. The crude product was purified by silica gel column chromatography (ethyl acetate/petroleum ether = 20/80) to give a compound benzyl 7-chloro-5-formyl-1,2,3,4-tetrahydroisoquinoline-2-carboxylate (9 g, yield: 74.55%). ES-API: [M+H]⁺=330.1.

Step 4: The benzyl 7-chloro-5-formyl-1,2,3,4-tetrahydroisoquinoline-2-carboxylate (9 g, 27.291 mmol) and (S)-2-methylpropyl-2-sulfinamide were dissolved in dichloromethane (180 mL). Tetraethyl titanate (24.90 g, 109.164 mmol) was slowly added and stirred at room temperature for 18 h. The reaction mixture was diluted with dichloromethane and saturated sodium chloride solution. The organic layer was separated, washed with saturated sodium chloride solution, and concentrated to give a crude product. The crude product was purified by silica gel column chromatography (ethyl acetate/petroleum ether = 80/20) to give a compound (S)-benzyl 5-(((tert-butylsulfinyl)amino)methyl)-7-chloro-3,4-tetrahydroisoquinoline-2(1H)-carboxyl ate (10.5 g, yield: 88.83%). ES-API: [M+H]⁺=433.1.

Step 5: The (S)-benzyl 5-(((tert-butylsulfinyl)amino)methyl)-7-chloro-3,4-tetrahydroisoquinoline-2(1H)-carboxyl ate (10 g, 23.096 mmol) was dissolved in tetrahydrofuran (100 mL), and the system was cooled to -78°C. (1,3-Dioxane-2-ethyl)magnesium bromide (184.770 mL, 92.385 mmol) was added, and the mixture was stirred at -78°C for 1 h. The reaction was quenched by the addition of saturated ammonium chloride (50 mL). The reaction mixture was poured into water (100 mL), and extracted with ethyl acetate (100 mL x 3). The organic layers were combined, washed with saturated sodium chloride solution (100 mL x 1), dried over anhydrous sodium sulfate, filtered, and concentrated to give a crude product. The crude product was purified by silica gel column chromatography (ethyl acetate/petroleum ether = 60/40) to give benzyl 5-((S)-1-(((S)-tert-butylsulfinyl)amino)-3-(1,3-dioxan-2-yl)propyl)-7-chloro-3,4-dihydrois oquinoline-2(1H)-carboxylate (11.67 g, yield: 92%). ES-API: [M+H]⁺=549.2.

Step 6: A solution of trifluoroacetic acid (100 mL) and water (5 mL) was added to the benzyl 5-((S)-1-(((S)-tert-butylsulfinyl)amino)-3-(1,3-dioxan-2-yl)propyl)-7-chloro-3,4-dihydrois oquinoline-2(1H)-carboxylate (11.67 g, 21.252 mmol) and stirred at room temperature for 0.5 h. Triethylsilane (33.851 mL, 212.518 mmol) was then added, and the mixture was stirred at room temperature for 2 h. The reaction mixture was concentrated to give (S)-benzyl 7-chloro-5-(pyrrolidin-2-yl)-3,4-dihydroisoquinoline-2(1H)-carboxylate (15 g, crude product) which was directly used in the next reaction without purification. ES-API: [M+H]⁺=371.1.

Step 7: The (S)-benzyl 7-chloro-5-(pyrrolidin-2-yl)-3,4-dihydroisoquinoline-2(1H)-carboxylate (15 g, crude product) was dissolved in dichloromethane (150 mL). N,N-Diisopropylethyl amine (26.737 mL, 161.777 mmol) and di-tert-butyl dicarbonate (11.150 mL, 48.533 mmol) were added and stirred at room temperature for 1 h. The obtained mixture was poured into water (100 mL). The organic layer was separated, washed with saturated sodium chloride solution (20 mL x 1), dried over anhydrous sodium sulfate, filtered, and concentrated to give a crude product. The crude product was purified by silica gel column chromatography (ethyl acetate/petroleum ether = 50/50) to give (S)-benzyl 5-(1-(tert-butoxycarbonyl)pyrrolidin-2-yl)-7-chloro-3,4-dihydroisoquinoline-2(1H)-carbox ylate (10 g, yield of the 2 steps: 100%). ES-API: [M+H]⁺=471.1.

Step 8: The (S)-benzyl 5-(1-(tert-butoxycarbonyl)pyrrolidin-2-yl)-7-chloro-3,4-dihydroisoquinoline-2(1H)-carbox ylate (1.00 g, 2.123 mmol), 3-methyl-5-(4,4,5,5-tetramethyl-1,3,2-dioxaborolan-2-yl)-1H-pyrrolo[2,3-b]pyridine (0.66 g, 2.548 mmol), chloro (2-dicyclohexylphosphino-2',6'-dimethoxy-1,1'-biphenylyl)(2'-amino-1,1'-biphenyl-2-yl) palladium (II) (0.08 g, 0.106 mmol) and potassium carbonate (0.88 g, 6.369 mmol) were added to 1,4-dioxane (10 ml) and water (2 ml). The system was replaced with nitrogen and reacted at 120°C for 1 h. Upon completion of the reaction, ethyl acetate (100 mL) was added. The obtained mixture was washed with water (50 mL) and saturated brine (50 mL), dried over anhydrous sodium sulfate, filtered, and concentrated to give a crude product. The crude product was purified by silica gel column chromatography (petroleum ether/ethyl acetate = 5/95) to give (S)-benzyl 5-(1-(tert-butoxycarbonyl)pyrrolidin-2-yl)-7-(3-methyl-1H-pyrrolo[2,3-b]pyridin-5-yl)-3,4 -dihydroisoquinoline-2(1H)-carboxylate (774 mg, yield: 64.50%). ES-API: [M+H]⁺=567.3.

Step 9: The (S)-benzyl 5-(1-(tert-butoxycarbonyl)pyrrolidin-2-yl)-7-(3-methyl-1H-pyrrolo[2,3-b]pyridin-5-yl)-3,4 -dihydroisoquinoline-2(1H)-carboxylate (774 mg, 1.366 mmol) was dissolved in dichloromethane (50 mL). N,N-Diisopropylethyl amine (528.6 mg, 4.098 mmol), di-tert-butyl dicarbonate (0.471 mL, 2.049 mmol) and 4-dimethylamino pyridine (16.45 mg, 0.136 mmol) were added. The mixture was stirred at room temperature for 0.5 h. The reaction mixture was concentrated to give a crude product. The crude product was purified by silica gel column chromatography (petroleum ether/ethyl acetate = 5/95) to give (S)-benzyl 7-(1-(tert-butoxycarbonyl)-3-methyl-1H-pyrrolo[2,3-b]pyridin-5-yl)-5-(1-(tert-butoxycarb onyl)pyrrolidin-2-yl)-3,4-dihydroisoquinoline-2(1H)-carboxylate (540 mg, yield: 59.3%). ES-API: [M+H]⁺=667.3.

Step 10: The (S)-benzyl 7-(1-(tert-butoxycarbonyl)-3-methyl-1H-pyrrolo[2,3-b]pyridin-5-yl)-5-(1-(tert-butoxycarb onyl)pyrrolidin-2-yl)-3,4-dihydroisoquinoline-2(1H)-carboxylate (540 mg, 0.810 mmol) was dissolved in dichloromethane (15 mL). Palladium chloride (159.56 mg, 0.090 mmol), triethylamine (0.250 mL, 1.800 mmol) and triethylsilane (418.51 mg, 3.599 mmol) were then added, and the mixture was stirred at room temperature for 0.5 h. The obtained mixture was diluted with water (30 mL) and extracted with dichloromethane (30 mL x 3). The organic layers were combined, washed with saturated sodium chloride solution (30 mL), dried over anhydrous sodium sulfate, filtered, and concentrated to give a crude product. The crude product was purified by silica gel column chromatography (methanol/dichloromethane = 0%∼10%) to give tert-butyl (S)-5-(5-(1-(tert-butoxycarbonyl)pyrrolidin-2-yl)-1,2,3,4-tetrahydroisoquinolin-7-yl)-3-me thyl-1H-pyrrolidin[2,3-b]pyridine-1-carboxylate (400 mg, yield: 92.6%). ES-API: [M+H]⁺=533.3.

Step 11: The tert-butyl (S)-5-(5-(1-(tert-butoxycarbonyl)pyrrolidin-2-yl)-1,2,3,4-tetrahydroisoquinolin-7-yl)-3-me thyl-1H-pyrrolidin[2,3-b]pyridine-1-carboxylate (150 mg, 0.282 mmol) was dissolved in dried dichloromethane (10 mL). Triethylamine (67.2 mg, 0.666 mmol) and methoxyacetyl chloride (45.84 mg, 0.422 mmol) were added successively under an ice-water bath condition and stirred for 1 h. Upon completion of the reaction, the reaction mixture was diluted with dichloromethane (30 mL), washed with saturated brine (30 mL), dried over anhydrous sodium sulfate, filtered, and concentrated to give a crude product. The crude product was purified by silica gel column chromatography (petroleum ether/ethyl acetate = 1/1) to give tert-butyl (S)-5-(5-(1-(tert-butoxycarbonyl)pyrrolidin-2-yl)-2-(2-methoxyacetyl)-1,2,3,4-tetrahydrois oquinolin-7-yl)-3-methyl-1H-pyrrolo[2,3-b]pyridine-1-carboxylate (110 mg, yield: 64.71%). ES-API: [M+H]⁺=605.3.

Step 12: The tert-butyl (S)-5-(5-(1-(tert-butoxycarbonyl)pyrrolidin-2-yl)-2-(2-methoxyacetyl)-1,2,3,4-tetrahydrois oquinolin-7-yl)-3-methyl-1H-pyrrolo[2,3-b]pyridine-1-carboxylate (110 mg, 0.182 mmol) was dissolved in dichloromethane (5 mL) and added to trifluoroacetic acid (3 mL), and the system reacted at room temperature for 0.5 h. Upon completion of the reaction, the reaction mixture was concentrated. After neutralization with ammonia-methanol solution (1 mL), the obtained mixture was concentrated again and purified by prep-HPLC alkaline process (ammonia water) to give (S)-2-methoxy-1-(7-(3-methyl-1H-pyrrolo[2,3-b]pyridin-5-yl)-5-(pyrrolidin-2-yl)-3,4-dih ydroisoquinolin-2(1H)-yl)ethan-1-one (Z284, 32 mg, yield: 43.43%). ES-API: [M+H]⁺=405.2. ¹H NMR (400 MHz, DMSO-*d*₆) δ 11.34 (d, *J* = 14.0 Hz, 1H), 8.49-8.40 (m, 1H), 8.13 - 8.02 (m, 1H), 7.81 - 7.71 (m, 1H), 7.41 (d, *J* = 14.2 Hz, 1H), 7.26 (s, 1H), 4.76-4.63 (m, 2H), 4.21 (d, *J* = 5.5 Hz, 3H), 3.90 - 3.52 (m, 3H), 3.35-3.25 (m, 3H), 3.15-3.05 (m, 1H), 3.00 - 2.72 (m, 3H), 2.31 (d, *J* = 1.1 Hz, 3H), 2.25-2.10 (m, 1H), 1.88 - 1.70 (m, 2H), 1.48-1.38 (m, 1H).

### Example 150. Synthesis of compound Z283

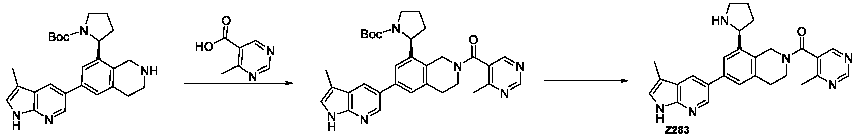

Step 1: 4-Methylpyrimidine-5-carboxylic acid (11.5 mg, 0.083 mmol), 1-propylphosphonic anhydride (132.3 mg, 0.21 mmol, 50% in ethyl acetate) and triethylamine (21.01 mg, 0.21 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in dichloromethane (1 mL) and stirred at 25°C for 2 h. The reaction was monitored by LCMS for completion, and quenched by the addition of water. The reaction mixture was extracted with ethyl acetate, washed with saturated brine, dried over anhydrous sodium sulfate, filtered, and concentrated to give a product tert-butyl (S)-2-(6-(3-methyl-1H-pyrrolo[2,3-b]pyridin-5-yl)-2-(4-methylpyrimidine-5-carbonyl)-1,2 ,3,4-tetrahydroisoquinolin-8-yl)pyrrolidine-1-carboxylate (35 mg, crude product). ES-API:[M+H]⁺= 553.4.

Step 2: Trifluoroacetic acid (0.5 mL) was added to a solution of the compound tert-butyl (S)-2-(6-(3-methyl-1H-pyrrolo[2,3-b]pyridin-5-yl)-2-(4-methylpyrimidine-5-carbonyl)-1,2 ,3,4-tetrahydroisoquinolin-8-yl)pyrrolidine-1-carboxylate (35 mg, 0.063 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 ml/min, gradient: B/A = 20%-90% over 50 min, wavelength: 214 nm, column temperature: room temperature) to give a product (S)-(6-(3-methyl-1H-pyrrolo[2,3-b]pyridin-5-yl)-8-(pyrrolidin-2-yl)-3,4-dihydroisoquinoli n-2(1H)-yl)(4-methylpyrimidin-5-yl)methanone (Z283, 7.3 mg, yield: 25%) as a white powder. ES-API:[M+H]⁺=453.3. ¹H NMR (400 MHz, CDCl₃) δ 9.16-9.12 (m, 1H), 8.99-8.80 (m, 1H), 8.56 (s, 1H),8.50-8.45 (m, 1H), 8.03-7.98 (m, 1H), 7.85-7.57 (m, 1H), 7.32 (s, 1H), 7.08 (s, 1H),5.41-4.90 (m, 1H), 4.82-4.36 (m, 1H), 4.19-3.85 (m, 1H),3.57-3.31 (m, 2H), 3.19-2.98 (m, 2H), 2.95-2.82 (m, 2H),2.60-2.45 (m, 3H), 2.43-2.34 (m, 1H), 2.34 (s, 3H), 2.13-1.90 (m, 3H).

### Example 151. Synthesis of compound Z282

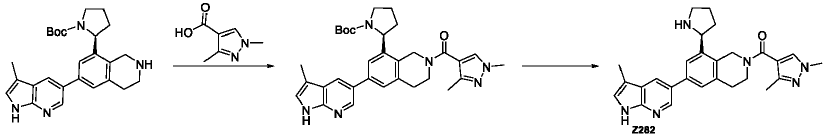

Step 1: 1,3-Dimethyl-1H-pyrazole-4-carboxylic acid (11.7 mg, 0.083 mmol), 1-propylphosphonic anhydride (132.3 mg, 0.21 mmol, 50% in ethyl acetate) and triethylamine (21.01 mg, 0.21 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in dichloromethane (1 mL) and stirred at 25°C for 2 h. The reaction was monitored by LCMS for completion, and quenched by the addition of water. The reaction mixture was extracted with ethyl acetate, washed with saturated brine, dried over anhydrous sodium sulfate, filtered, and concentrated to give a product tert-butyl (S)-2-(2-(1,3-dimethyl-1H-pyrazole-4-carbonyl)-6-(3-methyl-1H-pyrrolo[2,3-b]pyridin-5-yl)-1,2,3,4-tetrahydroisoquinolin-8-yl)pyrrolidine-1-carboxylate (35 mg, crude product). ES-API:[M+H]⁺= 555.4.

Step 2: Trifluoroacetic acid (0.5 mL) was added to a solution of the compound tert-butyl (S)-2-(2-(1,3-dimethyl-1H-pyrazole-4-carbonyl)-6-(3-methyl-1H-pyrrolo[2,3-b]pyridin-5-yl)-1,2,3,4-tetrahydroisoquinolin-8-yl)pyrrolidine-1-carboxylate (35 mg, 0.063 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 mL/min, gradient: B/A = 20%-90% over 50 min, wavelength: 214 nm, column temperature: room temperature) to give (S)-(1,3-dimethyl-1H-pyrazol-4-yl)(6-(3-methyl-1H-pyrrolo[2,3-b]pyridin-5-yl)-8-(pyrroli din-2-yl)-3,4-dihydroisoquinolin-2(1H)-yl)methanone (Z282, 1.7 mg, yield: 6%) as a white powder. ES-API:[M+H]⁺=455.3.

### Example 152. Synthesis of compound Z281

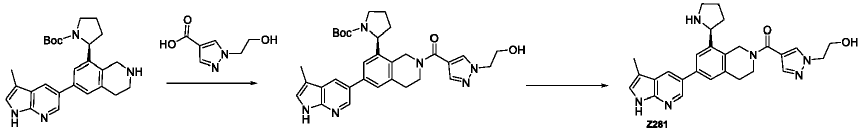

Step 1: 1-(2-Hydroxyethyl)-1H-pyrazole-4-carboxylic acid (11.7 mg, 0.083 mmol), 1-(3-dimethylaminopropyl)-3-ethylcarbodiimide hydrochloride (39.89 mg, 0.21 mmol), triethylamine (21.01, 0.21 mmol) and 1-hydroxybenzotriazole (14.06 mg, 0.11mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in N,N-dimethylformamide (1 ml), and the mixture was stirred at 25°C for 2 h. The reaction was monitored by LCMS for completion, and quenched by the addition of water. The reaction mixture was extracted with ethyl acetate, washed with saturated brine, dried over anhydrous sodium sulfate, filtered, and concentrated to give a product tert-butyl (S)-2-(2-(1-(2-hydroxyethyl)-1H-pyrazole-4-carbonyl)-6-(3-methyl-1H-pyrrolo[2,3-b]pyri din-5-yl)-1,2,3,4-tetrahydroisoquinolin-8-yl)pyrrolidine-1-carboxylate (40 mg, crude product). ES-API:[M+H]⁺= 571.4.

Step 2: Trifluoroacetic acid (0.5 mL) was added to a solution of the compound tert-butyl (S)-2-(2-(1-(2-hydroxyethyl)-1H-pyrazole-4-carbonyl)-6-(3-methyl-1H-pyrrolo[2,3-b]pyri din-5-yl)-1,2,3,4-tetrahydroisoquinolin-8-yl)pyrrolidine-1-carboxylate (40 mg, 0.07 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 mL/min, gradient: B/A = 20%-90% over 50 min, wavelength: 214 nm, column temperature: room temperature) to give (S)-(1-(2-hydroxyethyl)-1H-pyrazol-4-yl)(6-(3-methyl-1H-pyrrolo[2,3-b]pyridin-5-yl)-8-( pyrrolidin-2-yl)-3,4-dihydroisoquinolin-2(1H)-yl)methanone (Z281, 4.8 mg, yield: 14.6%) as a white powder. ES-API:[M+H]⁺=471.3.

### Example 153. Synthesis of compound Z280

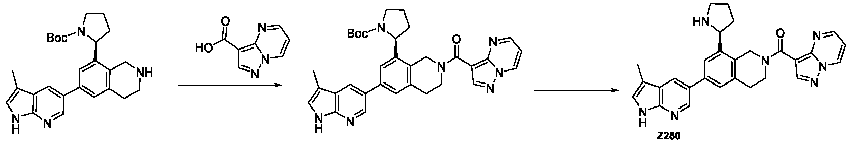

Step 1: Pyrazolo[1,5-a]pyrimidine-3-carboxylic acid (13.58 mg, 0.083 mmol), 1-propylphosphonic anhydride (132.3 mg, 0.21 mmol, 50% in ethyl acetate) and triethylamine (21.01 mg, 0.21 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in dichloromethane (1 mL), and the reaction mixture was stirred at 25°C for 2 h. The reaction was monitored by LCMS for completion, and quenched by the addition of water. The reaction mixture was extracted with ethyl acetate, washed with saturated brine, dried over anhydrous sodium sulfate, filtered, and concentrated to give a product tert-butyl (S)-2-(6-(3-methyl-1H-pyrrolo[2,3-b]pyridin-5-yl)-2-(pyrazolo[1,5-a]pyrimidine-3-carbon yl)-1,2,3,4-tetrahydroisoquinolin-8-yl)pyrrolidine-1-carboxylate (30 mg, crude products). ES-API:[M+H]⁺= 578.3.

Step 2: Trifluoroacetic acid (0.5 mL) was added to a solution of the compound tert-butyl (S)-2-(6-(3-methyl-1H-pyrrolo[2,3-b]pyridin-5-yl)-2-(pyrazolo[1,5-a]pyrimidine-3-carbon yl)-1,2,3,4-tetrahydroisoquinolin-8-yl)pyrrolidine-1-carboxylate (40 mg, 0.069 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 ml/min, gradient: B/A = 20%-90% over 50 min, wavelength: 214 nm, column temperature: room temperature) to give (S)-(6-(3-methyl-1H-pyrrolo[2,3-b]pyridin-5-yl)-8-(pyrrolidin-2-yl)-3,4-dihydroisoquinoli n-2(1H)-yl)(pyrazolo[1,5-a]pyrimidin-3-yl)methanone (Z280, 5.4 mg, yield: 16.33%) as a white powder. ES-API:[M+H]⁺=478.2.

### Example 154. Synthesis of compound Z271-1 and compound Z271-2

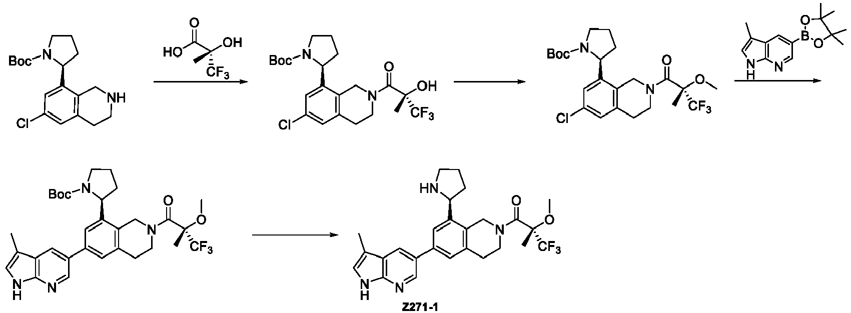

Step 1: (S)-3,3,3-Trifluoro-2-hydroxy-2-methylpropanoic acid (46.93 mg, 0.30 mmol), 1-(3-dimethylaminopropyl)-3-ethylcarbodiimide hydrochloride (85.36 mg, 0.45 mmol), triethylamine (44.97 mg, 0.45 mmol) and 1-hydroxy-benzotriazole (60.17 mg, 0.45 mmol) were added to a solution of tert-butyl (S)-2-(6-chloro-1,2,3,4-tetrahydroisoquinolin-8-yl)pyrrolidine-1-carboxylate (50 mg, 0.15 mmol) in N,N-dimethylformamide (1 mL), and the mixture was stirred at 25°C for 2 h. The reaction was monitored by LCMS for completion, and quenched by the addition of water. The reaction mixture was extracted with ethyl acetate, washed with saturated brine, dried over anhydrous sodium sulfate, and concentrated to give a product tert-butyl (S)-2-(6-chloro-2-((S)-3,3,3-trifluoro-2-hydroxy-2-methylpropanoyl)-1,2,3,4-tetrahydroisoqui nolin-8-yl)pyrrolidine-1-carboxylate (70 mg, crude product). ES-API:[M+H-100]⁺= 377.1.

Step 2: Sodium hydride (14.09 mg, 0.59 mmol) was added to a solution of the compound tert-butyl (S)-2-(6-chloro-2-((S)-3,3,3-trifluoro-2-hydroxy-2-methylpropanoyl)-1,2,3,4-tetrahydroisoqui nolin-8-yl)pyrrolidine-1-carboxylate (70 mg, 0.15 mmol) in N,N-dimethylformamide (1.5 mL) and stirred at 0°C for 0.5 h. Iodomethane (84.54 mg, 0.59 mmmol) was then added to the mixture, and the mixture was stirred at 25°C for 1 h. The reaction was monitored by LCMS for completion, and quenched by the addition of water. The reaction mixture was extracted with ethyl acetate, washed with saturated brine, dried over anhydrous sodium sulfate, filtered, and concentrated to give a crude product. The crude product was purified by silica gel column chromatography ((mobile phase 0-25% ethyl acetate/petroleum ether, ethyl acetate/petroleum ether = 1/5, Rf = 0.6) to give a product tert-butyl (S)-2-(6-chloro-2-((S)-3,3,3-trifluoro-2-methoxy-2-methylpropanoyl)-1,2,3,4-tetrahydroisoqui nolin-8-yl)pyrrolidine-1-carboxylate (50 mg, yield: 69.39%). ES-API:[M+H-100]⁺= 391.1.

Step 3: 3-Methyl-5-(4,4,5,5-tetramethyl-1,3,2-dioxaborolan-2-yl)-1H-pyrrolo[2,3-b]pyridine (31.55 mg, 0.12 mmol), chloro (2-dicyclohexylphosphino-2',6'-dimethoxy-1,1'-biphenylyl)(2'-amino-1,1'-biphenyl-2-yl) palladium (II) (7.33 mg, 0.01 mmmol) and potassium carbonate (42.16 mg, 0.31 mmmol) were added to a solution of the compound tert-butyl (S)-2-(6-chloro-2-((S)-3,3,3-trifluoro-2-methoxy-2-methylpropanoyl)-1,2,3,4-tetrahydroisoqui nolin-8-yl)pyrrolidine-1-carboxylate (50 mg, 0.10 mmol) in dioxane/water (2 mL/0.5 mL), and the mixture was heated to 110°C and stirred for 2 h in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by silica gel column chromatography (0-10% methanol/dichloromethane, dichloromethane/methanol = 10/1, Rf = 0.6) to give a product tert-butyl (S)-2-(6-(3-methyl-1H-pyrrolo[2,3-b]pyridin-5-yl)-2-((S)-3,3,3-trifluoro-2-methoxy-2-met hylpropanoyl)-1,2,3,4-tetrahydroisoquinolin-8-yl)pyrrolidine-1-carboxylate (37 mg, yield: 61.93%). ES-API:[M+H-100]⁺= 587.4.

Step 4: Trifluoroacetic acid (0.5 mL) was added to a solution of the compound tert-butyl (S)-2-(6-(3-methyl-1H-pyrrolo[2,3-b]pyridin-5-yl)-2-((S)-3,3,3-trifluoro-2-methoxy-2-met hylpropanoyl)-1,2,3,4-tetrahydroisoquinolin-8-yl)pyrrolidine-1-carboxylate (37 mg, 0.06 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 mL/min, gradient: B/A = 20%-90% over 50 min, wavelength: 214 nm, column temperature: room temperature) to give (S)-3,3,3-trifluoro-2-methoxy-2-methyl-1-(6-(3-methyl-1H-pyrrolo[2,3-b]pyridin-5-yl)-8-( (S)-pyrrolidin-2-yl)-3,4-dihydroisoquinolin-2(1H)-yl)propan-1-one (Z271-1, 6.3 mg, 20.53%) as a white powder. ES-API:[M+H]⁺=487.3. ¹H NMR (400 MHz, CDCl₃) δ 8.73 (s, 1H), 8.49 (d, *J* = 2.0 Hz, 1H), 8.03 (d, *J* = 2.0 Hz, 1H), 7.74 (d, *J* = 12.1 Hz, 1H), 7.28 (s, 1H), 7.08 (s, 1H), 5.41-4.69 (m, 2H),4.48-3.57 (m, 3H), 3.49-3.32 (m, 3H), 3.30-3.21 (m, 1H), 3.10-2.97 (m, 3H), 2.35 (s, 3H), 2.33-2.19 (m, 1H), 2.06-1.75 (m, 3H), 1.70 (s, 3H).

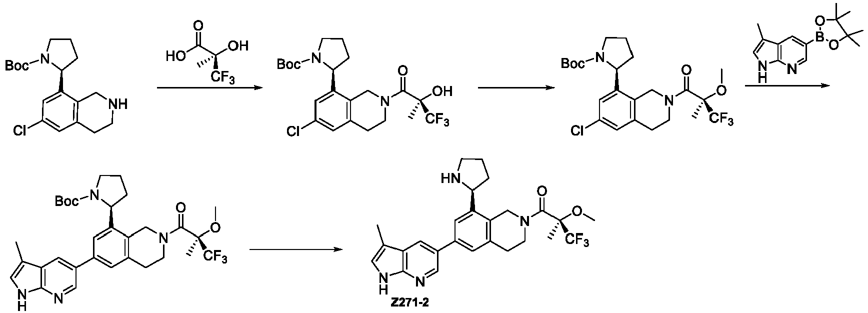

Step 1: (R)-3,3,3-Trifluoro-2-hydroxy-2-methylpropanoic acid (37.54 mg, 0.24 mmol), 1-(3-dimethylaminopropyl)-3-ethylcarbodiimide hydrochloride (68.29 mg, 0.36 mmol), triethylamine 35.98 mg, 0.36 mmol) and 1-hydroxy-benzotriazole (48.13 mg, 0.36 mmol) were added to a solution of tert-butyl (S)-2-(6-chloro-1,2,3,4-tetrahydroisoquinolin-8-yl)pyrrolidine-1-carboxylate (40 mg, 0.12 mmol) in N,N-dimethylformamide (1 mL), and the mixture was stirred at 25°C for 2 h. The reaction was monitored by LCMS for completion, and quenched by the addition of water. The reaction mixture was extracted with ethyl acetate, washed with saturated brine, dried over anhydrous sodium sulfate, filtered, and concentrated to a product tert-butyl (S)-2-(6-chloro-2-((R)-3,3,3-trifluoro-2-hydroxy-2-methylpropanoyl)-1,2,3,4-tetrahydroisoqui nolin-8-yl)pyrrolidine-1-carboxylate (60 mg, crude product). ES-API:[M+H-100]⁺= 377.1.

Step 2: Sodium hydride (12.08 mg, 0.50 mmol) was added to a solution of the compound tert-butyl (S)-2-(6-chloro-2-((R)-3,3,3-trifluoro-2-hydroxy-2-methylpropanoyl)-1,2,3,4-tetrahydroisoqui nolin-8-yl)pyrrolidine-1-carboxylate (60 mg, 0.13 mmol) in N,N-dimethylformamide (1.5 mL) and stirred at 0°C for 0.5 h. Iodomethane (72.74 mg, 0.50 mmmol) was then added to the mixture, and the mixture was stirred at 25°C for 1 h. The reaction was monitored by LCMS for completion, and quenched by the addition of water. The reaction mixture was extracted with ethyl acetate, washed with saturated brine, dried over anhydrous sodium sulfate, filtered, and concentrated to a product tert-butyl (S)-2-(6-chloro-2-((R)-3,3,3-trifluoro-2-methoxy-2-methylpropanoyl)-1,2,3,4-tetrahydrois oquinolin-8-yl)pyrrolidine-1-carboxylate (60 mg, crude product). ES-API:[M+H-100]⁺= 391.1.

Step 3: 3-Methyl-5-(4,4,5,5-tetramethyl-1,3,2-dioxaborolan-2-yl)-1H-pyrrolo[2,3-b]pyridine (37.86mg, 0.15 mmol), chloro (2-dicyclohexylphosphino-2',6'-dimethoxy-1,1'-biphenylyl)(2'-amino-1,1'-biphenyl-2-yl) palladium (II) (8.79mg, 0.012 mmmol) and potassium carbonate (50.60 mg, 0.37 mmmol) were added to a solution of the compound tert-butyl (S)-2-(6-chloro-2-((R)-3,3,3-trifluoro-2-methoxy-2-methylpropanoyl)-1,2,3,4-tetrahydrois oquinolin-8-yl)pyrrolidine-1-carboxylate (60 mg, 0.12 mmol) in dioxane/water (2 mL/0.5 mL), and the mixture was heated to 110°C and stirred for 2 h in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by silica gel column chromatography (0-10% methanol/dichloromethane, dichloromethane/methanol = 10/1, Rf = 0.6) to give a product tert-butyl (S)-2-(6-(3-methyl-1H-pyrrolo[2,3-b]pyridin-5-yl)-2-((R)-3,3,3-trifluoro-2-methoxy-2-me thylpropanoyl)-1,2,3,4-tetrahydroisoquinolin-8-yl)pyrrolidine-1-carboxylate (18 mg, yield: 25.11%). ES-API:[M+H-100]⁺= 587.4.

Step 4: Trifluoroacetic acid (0.5 mL) was added to a solution of the compound tert-butyl (S)-2-(6-(3-methyl-1H-pyrrolo[2,3-b]pyridin-5-yl)-2-((R)-3,3,3-trifluoro-2-methoxy-2-me thylpropanoyl)-1,2,3,4-tetrahydroisoquinolin-8-yl)pyrrolidine-1-carboxylate (18 mg, 0.03 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 mL/min, gradient: B/A = 20%-90% over 50 min, wavelength: 214 nm, column temperature: room temperature) to give (R)-3,3,3-trifluoro-2-methoxy-2-methyl-1-(6-(3-methyl-1H-pyrrolo[2,3-b]pyridin-5-yl)-8-((S)-pyrrolidin-2-yl)-3,4-dihydroisoquinolin-2(1H)-yl)propan-1-one (Z271-2, 1.1 mg, 7.37%) as a white powder. ES-API:[M+H]⁺=487.3.

### Example 155. Synthesis of compound Z279

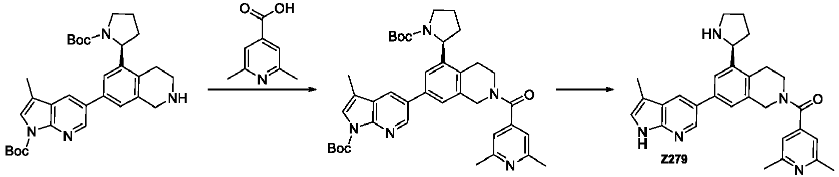

Step 1: tert-butyl (S)-5-(5-(1-(tert-butoxycarbonyl)pyrrolidin-2-yl)-1,2,3,4-tetrahydroisoquinolin-7-yl)-3-me thyl-1*H*-pyrrolidin[2,3-*b*]pyridine-1-carboxylate (30 mg) was dissolved in dichloromethane (1 mL). 2,6-Dimethylisonicotinic acid (13 mg, 0.08 mmol), triethylamine (0.04 mL, 0.28 mmol) and 50% 1-propylphosphonic anhydride in ethyl acetate (107 mg, 0.17 mmol) were added successively and stirred at room temperature for 2 h. The mixture was quenched with saturated sodium bicarbonate solution (10 mL) and extracted with dichloromethane (10 mLX3). The organic phases were combined, dried over anhydrous sodium sulfate, filtered, concentrated, and purified by a flash silica gel column (0-7% methanol/dichloromethane) to give tert-butyl (S)-5-(5-(1-(tert-butoxycarbonyl)pyrrolidin-2-yl)-2-(2,6-dimethylisonicotinoyl)-1,2,3,4-tet rahydroisoquinolin-7-yl)-3-methyl-1H-pyrrolo[2,3-b]pyridine-1-carboxylate (15 mg, yield: 40%). ES-API: [M+H]⁺ = 666.3.

Step 2: Trifluoroacetic acid (0.1 mL) was added dropwise to a solution of the tert-butyl (S)-5-(5-(1-(tert-butoxycarbonyl)pyrrolidin-2-yl)-2-(2,6-dimethylisonicotinoyl)-1,2,3,4-tet rahydroisoquinolin-7-yl)-3-methyl-1H-pyrrolo[2,3-b]pyridine-1-carboxylate (15 mg, 23 µmol) in dichloromethane (0.2 mL) under an ice bath condition and stirred at room temperature for 1 h. The reaction mixture was concentrated, and purified by prep-HPLC (ammonium bicarbonate) to give (S)-(2,6-dimethylpyridin-4-yl)(7-(3-methyl-1H-pyrrolo[2,3-*b*]pyridin-5-yl)-5-(pyrrolidin-2-yl)-3,4-dihydroisoquinolin-2(1*H*)-yl)methanone (Z279, 2.5 mg, purity: 100%, yield: 24%) as a white solid. ES-API: [M+H]⁺ = 466.3.

### Example 156. Synthesis of compound Z240-1

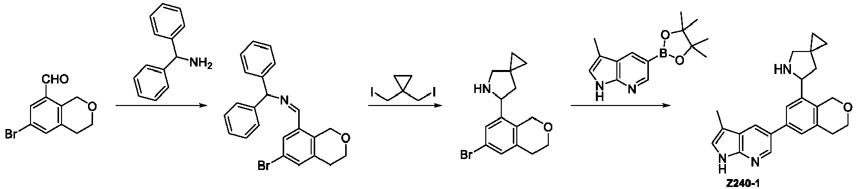

Step 1: Magnesium sulfate (330 mg, 2.74 mmol) and compound 6-bromoisochroman-8-carbaldehyde (300 mg, 1.24 mmol) were added successively to a solution ofbenzhydrylamine (228 mg, 1.24 mmol) in dichloromethane (10 mL) and stirred at room temperature overnight. The reaction mixture was filtered and concentrated to give (Z)-N-benzoyl-1-(6-bromoisochroman-8-yl)toluidine (505 mg, crude product) which was directly used in the next reaction without purification.

Step 2: In the presence of protective nitrogen, 1 M potassium tert-butoxide in tetrahydrofuran (0.148 mL) was slowly added to a solution of the (Z)-N-benzoyl-1-(6-bromoisochroman-8-yl)toluidine (50 mg, 0.12 mmol) in tetrahydrofuran (1 mL) at -65°C and stirred for 0.5 h. 1,1-Bis(iodomethyl)cyclopropane (119 mg, 0.37 mmol) was then quickly added to the reaction mixture and stirred for 1 h. The reaction mixture was then slowly warmed to room temperature and stirred overnight. The reaction mixture was quenched with water (10 mL), and extracted with dichloromethane (10 mLX3). The organic phase was dried over anhydrous sodium sulfate, filtered, and concentrated. The obtained residue was dissolved in acetone (1 mL). 3 M Hydrochloric acid solution (0.21 mL) was added and stirred at room temperature for 2 h. The reaction mixture was adjusted to pH 8 with saturated aqueous sodium bicarbonate solution, extracted with dichloromethane (10 mLX3), and concentrated to give 6-(6-bromoisochroman-8-yl)-5-azaspiro[2.4]heptane (40 mg) as a yellow oil. ES-API: [M+H]⁺ = 308.0, 310.1.

Step 3: In the presence of protective nitrogen, a mixed solution of the 6-(6-bromoisochroman-8-yl)-5-azaspiro[2.4]heptane (40 mg, 0.13 mmol), 3-methyl-5-(4,4,5,5-tetramethyl-dioxaborolan-2-yl)-1*H*-pyrrolo[2,3-*b*]pyridine (30 mg, 0.12 mmol), chloro (2-dicyclohexylphosphino-2',6'-dimethoxy-1,1'-biphenylyl)(2'-amino-1,1'-biphenyl-2-yl) palladium (II) (10 mg, 14 µmol), 2-dicyclohexylphosphine-2',6'-dimethoxybiphenyl (5 mg, 12 µmol) and potassium carbonate (54 mg, 0.39 mmol) in 1,4-dioxane (2 mL) and water (0.4 mL) was stirred at 120°C for 2 h. Ethyl acetate (10 mL) was poured into the reaction mixture, and the obtained mixture was washed with saturated brine (5 mL) and water (5 mL) successively. The organic phase was dried over anhydrous sodium sulfate, filtered, concentrated, and purified by prep-HPLC (ammonium bicarbonate) to give 5-(8-(5-azaspiro[2.4]heptan-6-yl)isochroman-6-yl)-3-methyl-1H-pyrrolo[2,3-b]pyridine (Z240-1, 0.7 mg, purity: 93%, yield: 1.4%) as a white solid. ES-API: [M+H]⁺ = 360.2.

### Example 157. Synthesis of compound Z219-1 and compound Z219-2

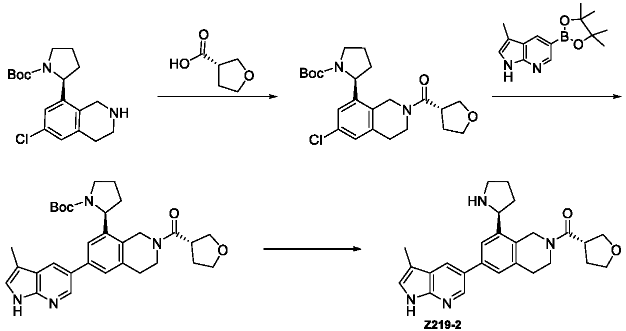

Step 1: (S)-Tetrahydrofuran-3-carboxylic acid (34.47 mg, 0.3 mmol), 1-propylphosphonic anhydride (566 mg, 0.89 mmol, 50% in ethyl acetate) and triethylamine (89.95 mg, 0.89 mmol) were added to a solution of tert-butyl (S)-2-(6-chloro-1,2,3,4-tetrahydroisoquinolin-8-yl)pyrrolidine-1-carboxylate (100 mg, 0.29 mmol) in dichloromethane (3 mL) and stirred at 25°C for 2 h. The reaction was monitored by LCMS for completion, and quenched by the addition of water. The reaction mixture was extracted with ethyl acetate, washed with saturated brine, dried over anhydrous sodium sulfate, filtered, and concentrated to give a product tert-butyl (S)-2-(6-chloro-2-((S)-tetrahydrofuran-3-carbonyl)-1,2,3,4-tetrahydroisoquinolin-8-yl)pyrr olidine-1-carboxylate (125 mg, crude product). ES-API:[M+H-100]⁺= 335.1.

Step 2: 3-Methyl-5-(4,4,5,5-tetramethyl-1,3,2-dioxaborolan-2-yl)-1H-pyrrolo[2,3-b]pyridine (89.02 mg, 0.34 mmol), chloro (2-dicyclohexylphosphino-2',6'-dimethoxy-1,1'-biphenylyl)(2'-amino-1,1'-biphenyl-2-yl) palladium (II) (20.68 mg, 0.029 mmmol) and potassium carbonate (118.98 mg, 0.86 mmmol) were added to a solution of the compound tert-butyl (S)-2-(6-chloro-2-((S)-tetrahydrofuran-3-carbonyl)-1,2,3,4-tetrahydroisoquinolin-8-yl)pyrr olidine-1-carboxylate (125 mg, 0.28 mmol) in dioxane/water (2 mL/0.5 mL), and the mixture was heated to 110°C and stirred for 2 h in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by silica gel column chromatography (0-10% methanol/dichloromethane, dichloromethane/methanol = 10/1, Rf = 0.6) to give a product tert-butyl (S)-2-(6-(3-methyl-1H-pyrrolo[2,3-b]pyridin-5-yl)-2-((S)-tetrahydrofuran-3-carbonyl)-1,2, 3,4-tetrahydroisoquinolin-8-yl)pyrrolidine-1-carboxylate (120 mg, yield: 78.7%) as a yellow powder. ES-API:[M+H-100]⁺= 531.4.

Step 3: Trifluoroacetic acid (0.5 mL) was added to a solution of the compound tert-butyl (S)-2-(6-(3-methyl-1H-pyrrolo[2,3-b]pyridin-5-yl)-2-((S)-tetrahydrofuran-3-carbonyl)-1,2, 3,4-tetrahydroisoquinolin-8-yl)pyrrolidine-1-carboxylate (130 mg, 0.24 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 ml/min, gradient: B/A = 20%-90% over 50 min, wavelength: 214 nm, column temperature: room temperature) to give (S)-(6-(3-methyl-1H-pyrrolo[2,3-b]pyridin-5-yl)-8-(pyrrolidin-2-yl)-3,4-dihydroisoquinoli n-2(1H)-yl)((S)-tetrahydrofuran-3-yl)methanone (Z219-2, 30.3 mg, yield: 28.7%) as a white powder. ES-API:[M+H]⁺=431.3. ¹H NMR (400 MHz, CDCl₃) δ 9.11 (s, 1H), 8.50-8.47 (m, 1H), 8.04-8.00 (m, 1H), 7.77-7.63 (m, 1H), 7.33-7.27 (m, 1H), 7.10 (s, 1H),5.11-4.60 (m, 2H), 4.39-4.21 (m,1H), 4.15-4.04 (m, 1H), 4.00-3.80 (m, 4H), 3.80-3.74 (m, 1H), 3.47-3.20 (m, 2H),3.11-2.92 (m, 3H), 2.35 (s, 3H), 2.33-2.08 (m, 2H), 2.17-2.07 (m, 1H), 2.02-1.83 (m, 2H), 1.69-1.54 (m, 1H).

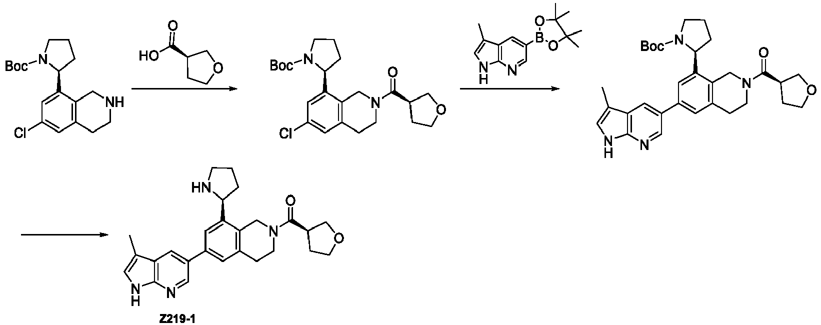

Step 1: (R)-Tetrahydrofuran-3-carboxylic acid (34.47 mg, 0.3 mmol), 1-propylphosphonic anhydride (566.4 mg, 0.9 mmol, 50% in ethyl acetate) and triethylamine (89.95 mg, 0.9 mmol) was added to a solution of tert-butyl (S)-2-(6-chloro-1,2,3,4-tetrahydroisoquinolin-8-yl)pyrrolidine-1-carboxylate (100 mg, 0.3 mmol) in dichloromethane (3 mL), and the mixture was stirred at 25°C for 2 h. The reaction was monitored by LCMS for completion, and quenched by the addition of water. The reaction mixture was extracted with dichloromethane, dried over anhydrous sodium sulfate, filtered, and concentrated to give a product tert-butyl (S)-2-(6-chloro-2-((R)-tetrahydrofuran-3-carbonyl)-1,2,3,4-tetrahydroisoquinolin-8-yl)pyr rolidine-1-carboxylate (130 mg, crude product). ES-API:[M+H-100]⁺= 335.1.

Step 2: 3-Methyl-5-(4,4,5,5-tetramethyl-1,3,2-dioxaborolan-2-yl)-1H-pyrrolo[2,3-b]pyridine (92.58 mg, 0.36 mmol), chloro (2-dicyclohexylphosphino-2',6'-dimethoxy-1,1'-biphenylyl)(2'-amino-1,1'-biphenyl-2-yl) palladium (II) (21.51 mg, 0.03 mmol) and potassium carbonate (123.74 mg, 0.9 mmol) were added to a solution of the compound tert-butyl (S)-2-(6-chloro-2-((R)-tetrahydrofuran-3-carbonyl)-1,2,3,4-tetrahydroisoquinolin-8-yl)pyr rolidine-1-carboxylate (130 mg, 0.3 mmol) in dioxane/water (3 mL/0.6 mL), and the mixture was heated to 110°C and stirred in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by silica gel column chromatography (mobile phase 0-10% methanol/dichloromethane, dichloromethane/methanol = 10/1, Rf = 0.6) to give a product tert-butyl (S)-2-(6-(3-methyl-1H-pyrrolo[2,3-b]pyridin-5-yl)-2-((R)-tetrahydrofuran-3-carbonyl)-1,2 ,3,4-tetrahydroisoquinolin-8-yl)pyrrolidine-1-carboxylate (130 mg, yield: 81.97%). ES-API:[M+H]⁺= 531.3.

Step 3: Trifluoroacetic acid (1.5 mL) was added to a solution of the compound tert-butyl (S)-2-(6-(3-methyl-1H-pyrrolo[2,3-b]pyridin-5-yl)-2-((R)-tetrahydrofuran-3-carbonyl)-1,2 ,3,4-tetrahydroisoquinolin-8-yl)pyrrolidine-1-carboxylate (130 mg, 0.24 mmol) in dichloromethane (3 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 ml/min, gradient: B/A = 20%-90% over 50 min, wavelength: 214 nm, column temperature: room temperature) to give (S)-(6-(3-methyl-1H-pyrrolo[2,3-b]pyridin-5-yl)-8-(pyrrolidin-2-yl)-3,4-dihydroisoquinoli n-2(1H)-yl)((R)-tetrahydrofuran-3-yl)methanone (Z219-1, 38.5 mg, yield: 36.5%) as a white powder. ES-API:[M+H]⁺=431.2. ¹H NMR (400 MHz, CDCl₃) δ 9.48 (s, 1H), 8.50 (s, 1H), 8.04 (s, 1H), 7.81-7.64 (m, 1H),7.31 (d, *J* = 11.3 Hz, 1H), 7.15-7.04 (m, 1H), 5.01-4.72 (m, 2H), 4.42-4.23 (m, 1H), 4.09 (t, *J* = 8.3 Hz, 1H),3.99-3.72 (m, 5H), 3.49-3.21 (m, 2H), 3.12-2.94 (m, 3H),2.36 (s, 3H), 2.34-2.09 (m, 4H), 1.86-1.54 (m, 2H).

### Example 158. Synthesis of compound Z243

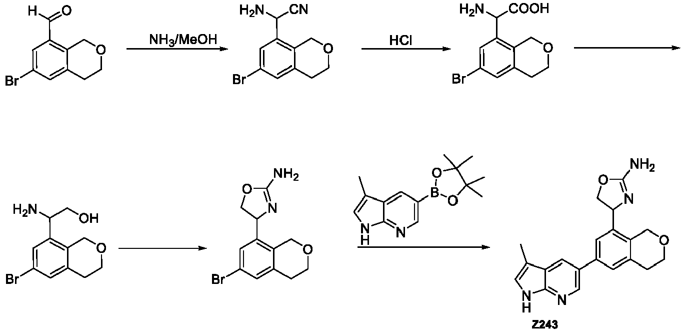

Step 1: Trimethylsilyl cyanide (2.04 g, 20.53 mmol) was added to a solution of 6-bromoisochroman-8-carbaldehyde (3.3 g, 13.69 mmol) in ammonia-methanol (7M, 80 mL) at 0°C and stirred for 1 h. The ice bath was then removed, and the mixture was stirred at 40°C for 16 h. The reaction was checked by LCMS for completion. A solvent was spun to dryness in vacuum, and the obtained mixture was purified by a flash silica gel column (dichloromethane: methanol = 10:1) to give 2-amino-2-(6-bromoisochroman-8-yl)acetonitrile (2.80 g, yield: 76.58%) as a yellow solid. ES-API:[M+H]⁺=267.0/269.0.

Step 2: The 2-amino-2-(6-bromoisochroman-8-yl)acetonitrile (2.8 g, 10.48 mmol) in 25% hydrochloric acid (80 mL) was stirred at 100°C for 16 h. The solution was cooled to room temperature and then stirred at 0°C for 1 h. The reaction was checked by LCMS for completion, and the reaction mixture was filtered. The obtained solid was collected and dried to give 2-amino-2-(6-bromoisochroman-8-yl)acetic acid (2.50 g, crude product) as a brown solid. ES-API:[M+H]⁺=286.0/288.1.

Step 3: In the presence of protective nitrogen, lithium borohydride (251.24 mg, 11.53 mmol) was dissolved in tetrahydrofuran (10 mL). Trimethylsilyl chloride (2.85 g, 26.21 mmol) and the 2-amino-2-(6-bromoisochroman-8-yl)acetic acid (1.5 g, 5.24 mmol) were added, and the mixture was stirred at 30°C for 16 h. The reaction was checked by LCMS for completion, and methanol was slowly added under an ice bath condition. The obtained yellow solution was concentrated and purified by a flash silica gel column (dichloromethane:methanol = 10:1) to give 2-amino-2-(6-bromoisochroman-8-yl)ethanol (900.00 mg, yield: 63.08%) as a yellow oil. ES-API:[M+H]⁺=272.1/274.0.

Step 4: Anhydrous potassium carbonate (243.77 mg, 1.76 mmol) was added to a solution of the 2-amino-2-(6-bromoisochroman-8-yl)ethanol (0.4 g, 1.47 mmol) in tetrahydrofuran (15 mL) at 0°C, and then cyanogen bromide (155.69 mg, 1.47 mmol) in tetrahydrofuran (5 mL) was added. The ice bath was removed, and the system continued to be stirred at 30°C for 16 h. The reaction was checked by LCMS for completion. The mixture was diluted with ethyl acetate (50 mL) and washed with water (100 mL). The aqueous phase was back extracted with ethyl acetate (100 mL). The combined organic layer was washed with water (50 mL) and saturated brine (100 mL) successively, dried over anhydrous sodium sulfate, filtered, and concentrated in vacuum. The residue was purified by silica gel column chromatography (dichloromethane:methanol = 10:1) to give 4-(6-bromoisochroman-8-yl)-4,5-dihydrooxazol-2-amine (300.00 mg, yield: 54.95%) as a yellow oil. ES-API:[M+H]⁺=297.0/298.9.

Step 5: 3-Methyl-5-(4,4,5,5-tetramethyl-1,3,2-dioxaborolan-2-yl)-1H-pyrrolo[2,3-b]pyridine (62.54 mg, 242.30 µmol), chloro (2-dicyclohexylphosphino-2',6'-dimethoxy-1,1'-biphenylyl)(2'-amino-1,1'-biphenyl-2-yl) palladium (II) (14.53 mg, 20.19 µmol) and potassium carbonate (83.72 mg, 605.76 µmol) were added to a solution of the 4-(6-bromoisochroman-8-yl)-4,5-dihydrooxazol-2-amine (0.06 g, 201.92 µmol) in 1,4-dioxane (2.5 mL) and water (0.5 mL) and stirred at 100°C for 2 h. The reaction was checked by LCMS for completion, and the reaction mixture was concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 mL/min, gradient: B/A = 20%-90% over 50 min, wavelength: 214 nm, column temperature: room temperature) to give 4-[6-(3-methyl-1H-pyrrolo[2,3-b]pyridin-5-yl)isochroman-8-yl]-4,5-dihydrooxazol-2-ami ne (Z243, 5.17 mg, yield: 7.28%) as a white solid. ES-API:[M+H]⁺=349.2. ¹H NMR (400 MHz, CDCl₃) δ 9.09 (s, 1H), 8.46 (d, *J* = 2.0 Hz, 1H), 8.00 (d, *J* = 2.0 Hz, 1H), 7.54 (s, 1H), 7.29 (s, 1H),7.04 (s, 1H), 5.22-5.16 (m, 1H), 4.93 (d, *J* = 15.0 Hz, 1H), 4.72-4.62 (m, 2H), 4.08-3.95 (m, 3H), 3.00-2.93 (m, 2H), 2.29 (s, 3H).

### Example 159. Synthesis of compound Z250

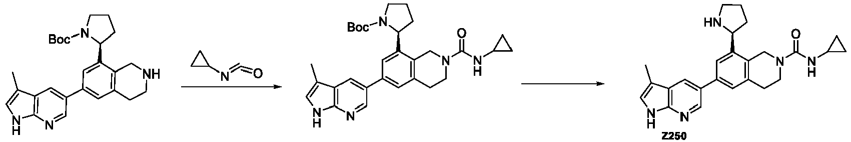

Step 1: Isocyanatocyclopropane (6.92 mg, 0.083 mmol) and triethylamine (21.01 mg, 0.21 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in tetrahydrofuran (1 mL) and stirred at 0°C for 0.2 h. The reaction was monitored by LCMS for completion, and quenched by the addition of water. The reaction mixture was extracted with ethyl acetate, washed with saturated brine, dried over anhydrous sodium sulfate, filtered, and concentrated to give a product tert-butyl (S)-2-(2-(cyclopropylcarbamoyl)-6-(3-methyl-1H-pyrrolo[2,3-b]pyridin-5-yl)-1,2,3,4-tetra hydroisoquinolin-8-yl)pyrrolidine-1-carboxylate (30 mg, crude product). ES-API:[M+H]⁺= 516.3.

Step 2: Trifluoroacetic acid (0.5 mL) was added to a solution of the compound tert-butyl (S)-2-(2-(cyclopropylcarbamoyl)-6-(3-methyl-1H-pyrrolo[2,3-b]pyridin-5-yl)-1,2,3,4-tetra hydroisoquinolin-8-yl)pyrrolidine-1-carboxylate (30 mg, 0.058 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sulfuric acid, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 ml/min, gradient: B/A = 20%-90% over 50 min, wavelength: 214 nm, column temperature: room temperature) to give a product (S)-N-cyclopropyl-6-(3-methyl-1H-pyrrolo[2,3-b]pyridin-5-yl)-8-(pyrrolidin-2-yl)-3,4-dih ydroisoquinoline-2(1H)-carboxamide (Z250, 4.4 mg, yield: 18.2%) as a white powder. ES-API:[M+H]⁺=416.2.

### Example 160. Synthesis of compound Z251

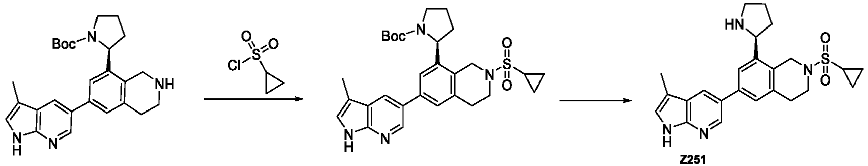

Step 1: Cyclopropanesulfonyl chloride (11.7 mg, 0.083 mmol) and triethylamine (21.01 mg, 0.21 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in tetrahydrofuran (1 mL) and stirred at 0°C for 0.2 h. The reaction was monitored by LCMS for completion, and quenched by the addition of water. The reaction mixture was extracted with ethyl acetate, washed with saturated brine, dried over anhydrous sodium sulfate, filtered, and concentrated to give a product tert-butyl (S)-2-(2-(cyclopropylsulfonyl)-6-(3-methyl-1H-pyrrolo[2,3-b]pyridin-5-yl)-1,2,3,4-tetrahy droisoquinolin-8-yl)pyrrolidine-1-carboxylate (30 mg, crude product). ES-API:[M+H]⁺= 537.3.

Step 2: Trifluoroacetic acid (0.5 mL) was added to a solution of the compound tert-butyl (S)-2-(2-(cyclopropylsulfonyl)-6-(3-methyl-1H-pyrrolo[2,3-b]pyridin-5-yl)-1,2,3,4-tetrahy droisoquinolin-8-yl)pyrrolidine-1-carboxylate (30 mg, 0.056 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 ml/min, gradient: B/A = 20%-90% over 50 min, wavelength: 214 nm, column temperature: room temperature) to give a product (S)-2-(cyclopropylsulfonyl)-6-(3-methyl-1H-pyrrolo[2,3-b]pyridin-5-yl)-8-(pyrrolidin-2-y l)-1,2,3,4-tetrahydroisoquinoline (Z251, 2.1 mg, yield: 8.61%) as a white powder. ES-API:[M+H]⁺=437.2.

### Example 161. Synthesis of compound Z249

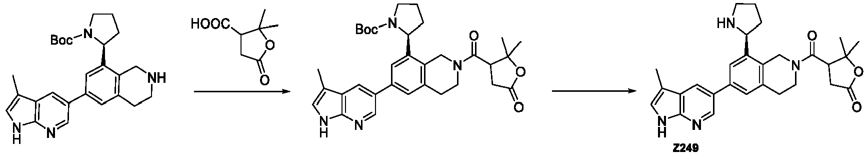

Step 1: 2,2-Dimethyl-5-oxotetrahydrofuran-3-carboxylic acid (13.16 mg, 0.083 mmol), 1-propylphosphonic anhydride (132.3 mg, 0.21 mmol, 50% solution in ethyl acetate) and triethylamine (21.01 mg, 0.21mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in dichloromethane (1 mL) and stirred at 25°C for 2 h. The reaction was monitored by LCMS for completion, and quenched by the addition of water. The reaction mixture was extracted with ethyl acetate, washed with saturated brine, dried over anhydrous sodium sulfate, filtered, and concentrated to give a crude product. The crude product was purified by silica gel column chromatography (0-6% methanol/dichloromethane) to give a product (2S)-tert-butyl 2-(2-(2,2-dimethyl-5-oxotetrahydrofuran-3-carbonyl)-6-(3-methyl-1H-pyrrolo[2,3-b]pyrid in-5-yl)-1,2,3,4-tetrahydroisoquinolin-8-yl)pyrrolidine-1-carboxylate (30 mg, yield: 75.53%). ES-API:[M+H]⁺= 573.4.

Step 2: Trifluoroacetic acid (0.5 mL) was added to a solution of the compound (2S)-tert-butyl 2-(2-(2,2-dimethyl-5-oxotetrahydrofuran-3-carbonyl)-6-(3-methyl-1H-pyrrolo[2,3-b]pyrid in-5-yl)-1,2,3,4-tetrahydroisoquinolin-8-yl)pyrrolidine-1-carboxylate (30 mg, 0.052 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 mL/min, gradient: B/A = 20%-90% over 50 min, wavelength: 214 nm, column temperature: room temperature) to give a product 5,5-dimethyl-4-(6-(3-methyl-1H-pyrrolo[2,3-b]pyridin-5-yl)-8-((S)-pyrrolidin-2-yl)-1,2,3, 4-tetrahydroisoquinoline-2-carbonyl)dihydrofuran-2(3H)-one (Z249, 8.3 mg, yield: 33.53%) as a white powder. ES-API:[M+H]⁺=473.3. ¹H NMR (400 MHz, CDCl₃) δ 8.83 (s, 1H), 8.49-8.46 (m, 1H), 8.04-7.99 (m, 1H), 7.79-7.58 (m, 1H),7.31 (d, *J* = 13.8 Hz, 1H), 7.09 (s, 1H), 5.20-4.78 (m, 2H), 4.65-4.31 (m, 1H), 4.21-3.74 (m, 2H), 3.73-3.57 (m, 1H), 3.35-3.20 (m, 2H), 3.09-2.96 (m, 3H), 2.75-2.63 (m, 1H), 2.35 (s, 3H),2.32-2.16 (m, 1H), 2.03-1.81 (m, 3H), 1.62-1.57 (m, 3H), 1.38-1.29 (m, 3H).

### Example 162. Synthesis of compound Z139

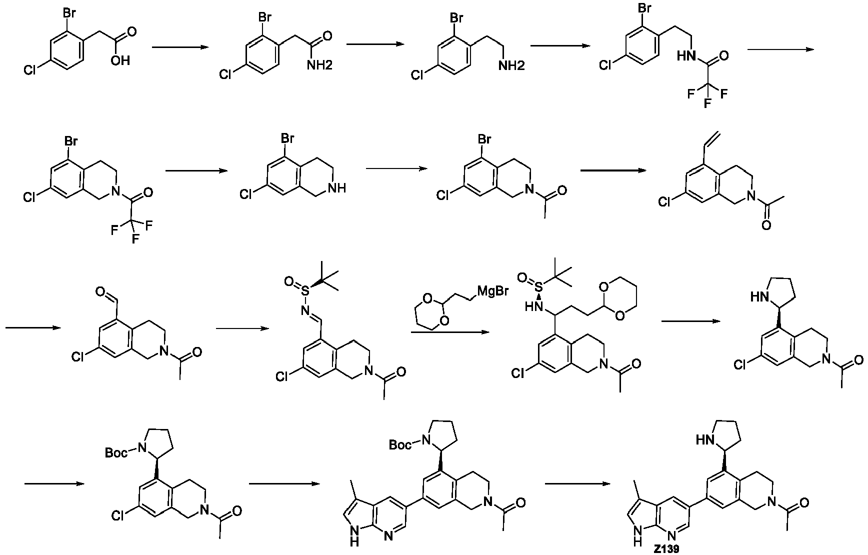

Step 1: 2-(2-Bromo-4-chlorophenyl)acetic acid (10 g, 40.1 mmol), N,N-dimethylformamide (1 mL) and tetrahydrofuran (70 mL) were added to a 250 mL three-neck flask. Oxalyl chloride (6 g, 47.24 mmol) was slowly added dropwise at 0°C in the presence of protective nitrogen, and the system reacted at 0°C for 1 h. The reaction mixture was spun to dryness, dissolved in dichloromethane (30 mL), added dropwise to a solution of ammonia water (50 mL) in dichloromethane (50 mL), stirred for 30 min, and slowly poured into ice water (500 mL). As a result, a large amount of solid was precipitated, filtered, and spun to dryness to give a desired product 2-(2-bromo-4-chlorophenyl)acetamide (8.5 g, yield: 85%). ES-API: [M+H]⁺=247.8.

Step 2: The 2-(2-bromo-4-chlorophenyl)acetamide (9 g, 36.3 mmol) and tetrahydrofuran (80 mL) were added to a 250 mL single-neck round-bottom flask. 1M borane in tetrahydrofuran (220 mL) was slowly added dropwise at 0°C in the presence of protective nitrogen, and the system was warmed to 75°C overnight. The reaction mixture was spun to dryness, dissolved in dichloromethane (30 mL), added dropwise to a solution of ammonia water (50 mL) in dichloromethane (50 mL), stirred for 30 min, and cooled to 0°C. 1M Hydrochloric acid solution (20 mL) was slowly added and stirred at 75°C for 1 h. Ethyl acetate (200 mL) was added. The obtained mixture was washed with saturated brine (200 mLx3), dried over anhydrous sodium sulfate, filtered, and concentrated. The crude product was purified by a flash silica gel column (methanol:dichloromethane = 10:100) to give a desired product 2-(2-bromo-4-chlorophenyl)ethan-1-amine (7.7 g, yield: 90%). ES-API: [M+H]⁺=239.9.

Step 3: The 2-(2-bromo-4-chlorophenyl)ethan-1-amine (8.1 g, 34.6 mmol), triethylamine (10.4 g, 103 mmol), trifluoroacetic anhydride (7.23 g, 34.4 mmol) and dichloromethane (80 mL) were added to a 250 mL single-neck round-bottom flask, and the system reacted at room temperature for 2 h. Dichloromethane (200 mL) was added to the reaction mixture. The obtained mixture was washed with saturated brine (200 mLX3), dried over anhydrous sodium sulfate, filtered, and concentrated. The crude product was purified by a flash silica gel column (methanol:dichloromethane = 10:100) to give a desired product N-(2-bromo-4-chlorophenethyl)-2,2,2-trifluoroacetamide (9.2 g, yield: 81%). ES-API: [M+H]⁺=330.

Step 4: N-(2-Bromo-4-chlorophenethyl)-2,2,2-trifluoroacetamide (4 g, 12.1 mmol) and acetic acid (50 mL) were added to a 250 mL single-neck round-bottom flask. Concentrated sulfuric acid (40 mL) was slowly added at 0°C and stirred at room temperature for 48 h. The reaction mixture was slowly poured into water (200 mL), extracted with dichloromethane (200 mLX3), dried over anhydrous sodium sulfate, filtered, and concentrated. The crude product was purified by a flash silica gel column (ethyl acetate:petroleum ether = 30:100) to give a desired product 1-(5-bromo-7-chloro-3,4-dihydroisoquinolin-2(1H)-yl)-2,2,2-trifluoroethan-1-one (1.2 g, yield: 31%). ES-API: [M+H]⁺=341.9.

Step 5: The 1-(5-bromo-7-chloro-3,4-dihydroisoquinolin-2(1H)-yl)-2,2,2-trifluoroethan-1-one (1.2 g, 3.5 mmol), potassium carbonate (485 mg, 3.5 mmol), ethanol (15 mL) and water (3 mL) were added to a 50 mL single-neck round-bottom flask and stirred at 80°C for 2 h. The reaction mixture was slowly poured into water (20 mL), extracted with dichloromethane (20 mLX3), dried over anhydrous sodium sulfate, filtered, and concentrated. The crude product was purified by a flash silica gel column (ethyl acetate:petroleum ether = 30:100) to give a desired product 5-bromo-7-chloro-1,2,3,4-tetrahydroisoquinoline (860 mg, yield: 99%). ES-API: [M+H]⁺=245.9.

Step 6: The 5-bromo-7-chloro-1,2,3,4-tetrahydroisoquinoline (492 mg, 2 mmol), triethylamine (606 mg, 6 mmol), and dichloromethane (10 mL) were added to a 50 mL single-neck round-bottom flask. Acetic anhydride (310 mg, 3 mmol) was slowly added at 0°C, and the system reacted at 0°C for 1 h. Dichloromethane (20 mL) was added to the reaction mixture. The obtained mixture was washed with saturated brine (20 mLX3), dried over anhydrous sodium sulfate, filtered, and concentrated. The crude product was purified by a flash silica gel column (methanol:dichloromethane = 10:100) to give a desired product 1-(5-bromo-7-chloro-3,4-dihydroisoquinolin-2(1H)-yl)ethan-1-one (582 mg, yield: 100%). ES-API: [M+H]⁺= 288.0.

Step 7: The 1-(5-bromo-7-chloro-3,4-dihydroisoquinolin-2(1H)-yl)ethan-1-one (540 mg, 1.88 mmol), potassium vinylfluoroborate (251 mg, 1.89 mmol), tetrakis(triphenylphosphine)palladium (216 mg, 0.18 mmol), potassium carbonate (776 mg, 5.6 mmol), dioxane (10 mL) and water (1 mL) were added to a 25 mL three-neck round-bottom flask. The system was replaced with nitrogen three times, protected with a nitrogen balloon, and then reacted at 110°C for 4 h. Ethyl acetate (100 mL) was added to the reaction mixture. The obtained mixture was washed with saturated brine (50 mLX3), dried over anhydrous sodium sulfate, filtered, and concentrated. The crude product was purified by a flash silica gel column (ethyl acetate:petroleum ether = 40:100) to give a desired product 1-(7-chloro-5-vinyl-3,4-dihydroisoquinolin-2(1H)-yl)ethan-1-one (470 mg, crude product). ES-API: [M+H]⁺=236.1.

Step 8: The 1-(7-chloro-5-vinyl-3,4-dihydroisoquinolin-2(1H)-yl)ethan-1-one (470 mg, 2 mmol), sodium periodate (2.14 g, 10 mmol), tetrahydrofuran (10 mL) and water (5 mL) were added to a 25 mL single-neck round-bottom flask. Potassium osmate dihydrate (147 mg, 0.4 mmol) was added in batches under an ice-water bath condition and then stirred at 0°C for 1 h. Ethyl acetate (30 mL) was added to the reaction mixture. The obtained mixture was washed with saturated brine (30 mLX3), dried over anhydrous sodium sulfate, filtered, and concentrated. The crude product was purified by a flash silica gel column (ethyl acetate:petroleum ether = 30:100) to give a desired product 2-acetyl-7-chloro-1,2,3,4-tetrahydroisoquinoline-5-carbaldehyde (200 mg, yield: 42%). ES-API: [M+H]⁺=238.0.

Step 9: The 2-acetyl-7-chloro-1,2,3,4-tetrahydroisoquinoline-5-carbaldehyde (200 mg, 0.84 mmol) and dichloromethane (10 mL) were added to a 50 mL single-neck round-bottom flask. (S)-2-Methylpropane-2-sulfinamide (203 mg, 1.68 mmol) and tetraethyl titanate (1 mL) were added under an ice-water bath condition and stirred at room temperature for 3 h. Ethyl acetate (30 mL) was added to the reaction mixture. The obtained mixture was washed with saturated brine (30 mLX3), dried over anhydrous sodium sulfate, filtered, and concentrated. The crude product was purified by a flash silica gel column (ethyl acetate:petroleum ether = 20:100) to give a desired product (S,E)-N-((2-acetyl-7-chloro-1,2,3,4-tetrahydroisoquinolin-5-yl)methylene)-2-methylpropa ne-2-sulfinamide (350 mg, yield: 83%). ES-API: [M+H]⁺=341.1.

Step 10: The (S,E)-N-((2-acetyl-7-chloro-1,2,3,4-tetrahydroisoquinolin-5-yl)methylene)-2-methylpropa ne-2-sulfinamide (200 mg, 0.56 mmol) and anhydrous tetrahydrofuran (3 mL) were added to a 5 mL three-neck round-bottom flask. The system was replaced with nitrogen three times and then protected with a nitrogen balloon. (2-(1,3-dioxan-2-yl)ethyl)magnesium bromide in tetrahydrofuran (3.5 mL, 0.5M) was added at -78°C and continued to be stirred for 2 h. The reaction mixture was quenched by the addition of saturated ammonium chloride (5 mL) solution. Ethyl acetate (30 mL) was added. The obtained mixture was washed with saturated brine (30 mLX3), dried over anhydrous sodium sulfate, filtered, and concentrated. The crude product was purified by a flash silica gel column (ethyl acetate:petroleum ether = 20:100) to give a desired product (S)-N-(1-(2-acetyl-7-chloro-1,2,3,4-tetrahydroisoquinolin-5-yl)-3-(1,3-dioxan-2-yl)propyl )-2-methylpropane-2-sulfinamide (210 mg, yield: 78%). ES-API: [M+H]⁺=457.2.

Step 11: The (S)-N-(1-(2-acetyl-7-chloro-1,2,3,4-tetrahydroisoquinolin-5-yl)-3-(1,3-dioxan-2-yl)propyl )-2-methylpropane-2-sulfinamide (270 mg, 0.59 mmol), trifluoroacetic acid (3 mL) and water (0.15 mL) were added to a 25 mL single-neck round-bottom flask. Trimethylsilane (687 mg, 5.9 mmol) was added at 0°C and stirred for 18 h. After the reaction mixture was spun to dryness, ethyl acetate (30 mL) was added. The obtained mixture was washed with saturated brine (30 mLX3), dried over anhydrous sodium sulfate, filtered, and concentrated to give a desired product (S)-1-(7-chloro-5-(pyrrolidin-2-yl)-3,4-dihydroisoquinolin-2(1H)-yl) ethan-1-one (160 mg, yield: 97%) as a crude product. ES-API: [M+H]⁺=279.1.

Step 12: The (S)-1-(7-chloro-5-(pyrrolidin-2-yl)-3,4-dihydroisoquinolin-2(1H)-yl)ethan-1-one (160 mg, 0.574 mmol), di-tert-butyl dicarbonate (1 mL), triethylamine (174 mg, 1.7 mmol) and dichloromethane (5 mL) were added to a 25 mL single-neck round-bottom flask and stirred at room temperature for 1 h. Dichloromethane (20 mL) was added to the reaction. The obtained mixture was washed with saturated brine (20 mLX3), dried over anhydrous sodium sulfate, filtered, and concentrated to give a desired product tert-butyl (S)-2-(2-acetyl-7-chloro-1,2,3,4-tetrahydroisoquinolin-5-yl)pyrrolidine-1-carboxylate (80 mg, yield: 37%). ES-API: [M+H]⁺=379.2.

Step 13: The tert-butyl (S)-2-(2-acetyl-7-chloro-1,2,3,4-tetrahydroisoquinolin-5-yl)pyrrolidine-1-carboxylate (80 mg, 0.21 mmol), 3-methyl-5-(4,4,5,5-tetramethyl-1,3,2-dioxaborolan-2-yl)-1H-pyrrolo[2,3-b]pyridine (81 mg, 0.32 mmol), chloro (2-dicyclohexylphosphino-2',6'-dimethoxy-1,1'-biphenylyl)(2'-amino-1,1'-biphenyl-2-yl) palladium (II) (15 mg, 0.02 mmol), potassium carbonate (88 mg, 0.63 mmol), dioxane (30 ml) and water (4 mL)were added to a 25 mL single-neck round-bottom flask. The system was replaced with nitrogen three times, protected with a nitrogen balloon, and then reacted under microwave at 110°C for 50 min. Ethyl acetate (10 mL) was added to the reaction mixture. The obtained mixture was washed with 5 mL of saturated brine (5 mLX3), dried over anhydrous sodium sulfate, filtered, and concentrated. The crude product was purified by a flash silica gel column (methanol:dichloromethane = 10:100) to give a desired product tert-butyl (S)-2-(2-acetyl-7-(3-methyl-1H-pyrrolo[2,3-b]pyridin-5-yl)-1,2,3,4-tetrahydroisoquinolin-5-yl)pyrrolidine-1-carboxylate (70 mg, yield: 70%). ES-API: [M+H]⁺=475.3.

Step 14: The tert-butyl (S)-2-(2-acetyl-7-(3-methyl-1H-pyrrolo[2,3-b]pyridin-5-yl)-1,2,3,4-tetrahydroisoquinolin-5-yl)pyrrolidine-1-carboxylate (70 mg, 0.15 mmol), trifluoroacetic acid (3 mL) and dichloromethane (6 mL) were added to a 5 mL single-neck round-bottom flask and stirred at room temperature for 30 min. The reaction mixture was spun to dryness, and the crude product was purified by prep-HPLC (ammonium bicarbonate) to give (S)-1-(7-(3-methyl-1H-pyrrolo[2,3-b]pyridin-5-yl)-5-(pyrrolidin-2-yl)-3,4-dihydroisoquin olin-2(1H)-yl)ethan-1-one (Z139, 20 mg, yield: 36%). ES-API: [M+H]⁺=375.2.

### Example 163. Synthesis of compound Z252

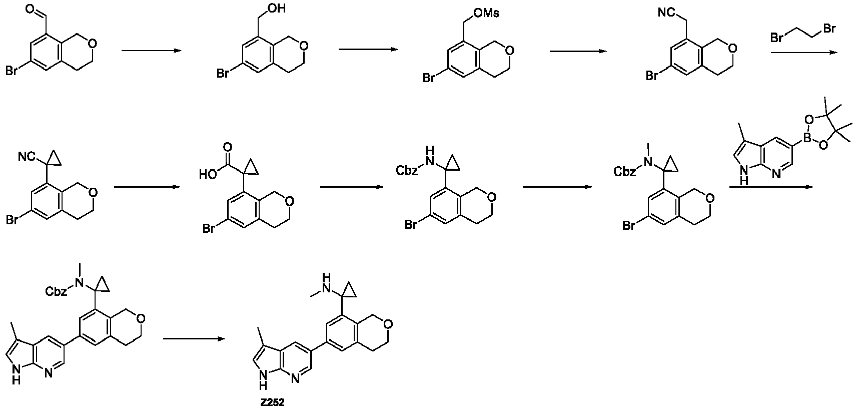

Step 1: 6-Bromoisochroman-8-carbaldehyde (1.6 g, 6.67 mmol) was dissolved in methanol (20 mL). Sodium borohydride (760 mg, 19.99 mmol) was slowly added under an ice-water bath condition and stirred for 1 h. Upon completion of the reaction, the reaction was quenched by the addition of water (20 mL). The reaction mixture was extracted with ethyl acetate (20 mlx3), washed with saturated brine (30 mLx1), dried over anhydrous sodium sulfate, filtered, and concentrated to give (6-bromoisochroman-8-yl)methanol (1.61 g, crude product). ES-API: [M+H]⁺=243.0.

Step 2: The (6-bromoisochroman-8-yl)methanol (1.61 g, crude product) was dissolved in tetrahydrofuran (30 mL). Triethylamine (2.02 g, 20.0 mmol) and methanesulfonyl chloride (1.14 g, 10 mmol) were added successively under an ice-water bath condition, and the system reacted at room temperature for 2 h. Upon completion of the reaction, the reaction was quenched by the addition of water (20 mL). The reaction mixture was extracted with ethyl acetate (20 ml x 3), washed with saturated brine (30 mL x 1), dried over anhydrous sodium sulfate, filtered, and concentrated to give methyl (6-bromoisochroman-8-yl)methanesulfonate (1.8 g, crude product). ES-API: [M+H]⁺ = 321.1.

Step 3: The methyl (6-bromoisochroman-8-yl)methanesulfonate (1.5 g, crude product) was dissolved in acetonitrile (30 mL). Trimethylsilyl cyanide (696 mg, 7.03 mmol) and tetrabutylammonium fluoride (1.83 g, 7.03 mmol) were added successively, and the system reacted at room temperature for 2 h. The reaction was quenched by the addition of water (20 mL). The reaction mixture was extracted with ethyl acetate (20 mlx3), washed with saturated brine (30 mLx1), dried over anhydrous sodium sulfate, filtered, and concentrated to give a crude product. The crude product was purified by silica gel column chromatography (ethyl acetate/petroleum ether = 1/5) to give 2-(6-bromoisochroman-8-yl)acetonitrile (0.96 g, yield of the 3 steps: 57.3%). ES-API: [M+H]⁺ = 252.2.

Step 4: The 2-(6-bromoisochroman-8-yl)acetonitrile (250 mg, 1.0 mmol) was dissolved in N,N-dimethylformamide (8 ml), and placed in an ice-water bath. 60% sodium hydride (200 mg, 5 mmol) was added, and the system reacted under the ice-water bath condition for 0.5 h. 1,2-Dibromoethane (188 mg, 1 mmol) was then added and stirred for 2 h. The reaction was quenched by the addition of aqueous ammonium chloride solution (20 mL). The reaction mixture was extracted with ethyl acetate (20 mlx3), washed with saturated brine (30 mLx1), dried over anhydrous sodium sulfate, filtered, and concentrated to give a crude product. The crude product was purified by silica gel column chromatography (ethyl acetate/petroleum ether = 1/5) to give 1-(6-bromoisochroman-8-yl)cyclopropane-1-carbonitrile (200 mg, yield: 72.2%). ES-API: [M+H]⁺ = 278.

Step 5: The 1-(6-bromoisochroman-8-yl)cyclopropane-1-carbonitrile (200 mg, 0.722 mmol) was dissolved in ethanol (4 mL) and water (4 mL). Sodium hydroxide (144.4 mg, 3.61 mmol) was added, and the system reacted under microwave at 100°C for 15 h. The reaction was cooled to room temperature, and quenched by the addition of diluted hydrochloric acid. The reaction mixture was extracted with ethyl acetate (20 mlx3), washed with saturated brine (30 mLx1), dried over anhydrous sodium sulfate, filtered, and concentrated to give a crude product. The crude product was purified by silica gel column chromatography (ethyl acetate/petroleum ether = 1/1) to give 1-(6-bromoisochroman-8-yl)cyclopropane-1-carboxylic acid (160 mg, yield: 74.8%). ES-API: [M+H]⁺ = 297.

Step 6: Toluene (5 mL) and benzyl alcohol (982.59 mg, 9.086 mmol) were added to a flask charged with the 1-(6-bromoisochroman-8-yl)cyclopropane-1-carboxylic acid (90 mg, 0.303 mmol). Diphenylphosphoryl azide (125.03 mg, 0.454 mmol) and triethylamine (0.084 mL, 0.606 mmol) were then added, and the mixture was stirred at 110°C for 15 h. The reaction mixture was diluted with water, extracted with ethyl acetate (30 mLx3), washed with saturated sodium chloride solution (30 mLx1), dried over anhydrous sodium sulfate, filtered, and concentrated to give a crude product. The crude product was purified by silica gel column chromatography (ethyl acetate/petroleum ether = 1/1) to give benzyl (1-(6-bromoisochroman-8-yl)cyclopropyl)carbamate (110 mg, yield: 90.5%). ES-API: [M+H]⁺ = 402.0.

Step 7: The benzyl (1-(6-bromoisochroman-8-yl)cyclopropyl)carbamate (100 mg, 0.249 mmol) was added dropwise to a suspension of sodium hydride (29.83 mg, 0.746 mmol) in tetrahydrofuran (5 mL) under an ice-water bath condition, and the mixture was stirred for 10 min. Iodomethane (176.42 mg, 1.243 mmol) was then added, and the reaction mixture was warmed to room temperature and stirred for 3 h. The obtained mixture was poured into a saturated ammonium chloride ice water solution, extracted with ethyl acetate (30 mL x3), washed with saturated sodium chloride solution (30 mLx1), dried over anhydrous sodium sulfate, filtered, and concentrated to give a crude product. The crude product was purified by silica gel column chromatography (ethyl acetate/petroleum ether = 1/1) to give benzyl N-methyl-(1-(6-bromoisochroman-8-yl)cyclopropyl)carbamate (90 mg, yield: 87%). ES-API: [M+H]⁺ = 416.0.

Step 8: The benzyl N-methyl-(1-(6-bromoisochroman-8-yl)cyclopropyl)carbamate (90 mg, 0.216 mmol), 3-methyl-5-(4,4,5,5-tetramethyl-1,3,2-dioxaborolan-2-yl)-1H-pyrrolo[2,3-b]pyridine (111.51 mg, 0.432 mmol), (2-dicyclohexylphosphino-2',6'-dimethoxy-1,1'-biphenylyl)(2'-amino-1,1'-biphenyl-2-yl) palladium (II) mesylate (15.57 mg, 0.022 mmol) and potassium carbonate (89.64 mg, 0.649 mmol) were added to 1,4-dioxane (3 mL) and water (0.5 mL). The system was replaced with nitrogen and reacted under microwave at 120°C for 0.5 h. Upon completion of the reaction, ethyl acetate (20 mL) was added. The obtained mixture was washed with water (10 mL) and saturated brine (10 mL) successively, and dried over anhydrous sodium sulfate to give a crude product. The crude product was purified by preparative thin layer chromatography (petroleum ether/ethyl acetate = 1/1) to give benzyl N-methyl-(1-(6-(3-methyl-1H-pyrrolo[2,3-b]pyridin-5-yl)isochroman-8-yl)cyclopropyl)ca rbamate (77 mg, yield: 76.3%). ES-API: [M+H]⁺=468.2.

Step 9: The benzyl N-methyl-(1-(6-(3-methyl-1H-pyrrolo[2,3-b]pyridin-5-yl)isochroman-8-yl)cyclopropyl)ca rbamate (77 mg, 0.164 mmol) was dissolved in dichloromethane (5 mL). Palladium chloride (5.08 mg, 0.01 mmol), triethylamine (16.5 mg, 0.164 mmol) and triethylsilane (76 mg, 0.656 mmol) were then added at 0°C, and the mixture was stirred at room temperature for 2 h. The reaction mixture was filtered and concentrated to give a crude product. The crude product was purified by prep-HPLC alkaline process (ammonia water) to give N-methyl-1-(6-(3-methyl-1H-pyrrolo[2,3-b]pyridin-5-yl)isochroman-8-yl)cyclopropan-1-a mine (Z252, 2.8 mg, yield: 5.1%). ES-API: [M+H]⁺=334.1.

### Example 164. Synthesis of compound Z238

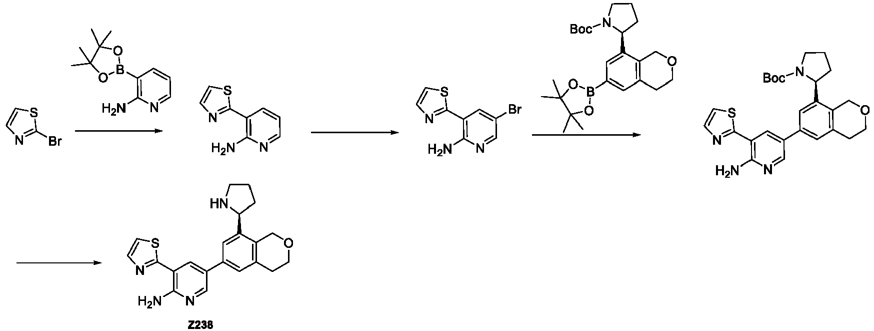

Step 1: 3-(4,4,5,5-Tetramethyl-1,3,2-dioxobenzofuran-2-yl)pyridin-2-amine (402 mg, 1.83 mmol), 2-bromo-1,3-thiazole (250 mg, 1.52 mmol), [1,1'-bis(diphenylphosphino)ferrocene]dichloropalladium (111 mg, 0.15 mmol), potassium carbonate (632 mg, 4.57 mmol), 1,4-dioxane (6 mL) and water (1.5 mL) were added to a 20 mL microwave tube. The system was purged with nitrogen for 1 min and reacted in a microwave reactor at 110°C for 1 h. The reaction mixture was cooled to room temperature, poured into water (30 mL), and extracted with ethyl acetate (50 mL). The organic layer was washed with saturated brine (25 mL), dried over anhydrous sodium sulfate, filtered, and concentrated to give a crude product. The crude product was purified by a flash silica gel column (methanol/dichloromethane: 0-10%) to give a desired product 3-(thiazol-2-yl)pyridin-2-amine (210 mg, yield: 77.7%) as an off-white solid. ES-API: [M+H]⁺=178.1.

Step 2: The 3-(thiazol-2-yl)pyridin-2-amine (185 mg, 1.04 mmol) was dissolved in tetrahydrofuran (15 mL). N-Bromosuccinimide (204 mg, 1.15 mmol) was added under an ice bath condition and stirred under the ice bath for 1 h. Ethyl acetate (50 mL) was added to the reaction mixture. The obtained mixture was washed with saturated sodium bicarbonate (20 mL) and saturated brine (20 mL), dried over anhydrous sodium sulfate, filtered, and concentrated to give a crude product. The crude product was purified by a flash silica gel column (methanol/dichloromethane: 0-5%) to give a desired product 5-bromo-3-(thiazol-2-yl)pyridin-2-amine (230 mg, yield: 86.0%) as a pale yellow solid. ES-API: [M+H]⁺=256.0, 258.0. ¹H NMR (500 MHz, DMSO-*d₆*) δ 8.15 (d, *J* = 2.5 Hz, 1H), 8.10 (d, *J* = 2.5 Hz, 1H), 7.97 (d, *J* = 3.5 Hz, 1H), 7.84 (d, *J* = 3.5 Hz, 1H), 7.78 (s, 2H).

Step 3: The 5-bromo-3-(thiazol-2-yl)pyridin-2-amine (50 mg, 0.20 mmol), tert-butyl (S)-2-(6-(4,4,5,5-tetramethyl-1,3,2-dioxaborolan-2-yl)isochroman-8-yl)pyrrolidine-1-carb oxylate (101 mg, 0.24 mmol), 2-dicyclohexylphosphine-2',6'-dimethoxy-biphenyl (4 mg, 0.01 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol), potassium carbonate (81 mg, 0.59 mmol), 1,4-dioxane (1.5 mL) and water (0.3 mL) were added to a 5 mL microwave tube. The mixture was purged with nitrogen for 30 sec, and the system reacted in a microwave reactor at 110°C for 45 min. The reaction was cooled to room temperature. The reaction mixture was poured into water (8 mL), and extracted with ethyl acetate (50 mL). The organic layer was washed with saturated brine (15 mL), dried over anhydrous sodium sulfate, filtered, and concentrated to give a crude product. The crude product was purified by a flash silica gel column (methanol/dichloromethane: 0-4%) to give a desired product tert-butyl (S)-2-(6-(6-amino-5-(thiazol-2-yl)pyridin-3-yl)isochroman-8-yl)pyrrolidine-1-carboxylate (55 mg, yield: 58.9%) as a pale yellow solid. ES-API: [M+H]⁺=479.3.

Step 4: The tert-butyl (S)-2-(6-(6-amino-5-(thiazol-2-yl)pyridin-3-yl)isochroman-8-yl)pyrrolidine-1-carboxylate (55 mg, 0.11 mmol) was dissolved in methanol (1 mL). 4.0M Hydrogen chloride-dioxane solution (2 mL, 8.0 mmol) was added, and the system reacted at room temperature for 1.5 h. The reaction mixture was concentrated. 7.0M Ammonia-methanol solution (5 mL) was added, and a solvent was spun to dryness to give a crude product. The crude product was purified by prep-HPLC (formic acid) to give a desired product (S)-5-(8-(pyrrolidin-2-yl)isochroman-6-yl)-3-(thiazol-2-yl)pyridin-2-amine (Z238, formate, 31 mg, yield: 63.5%) as a pale yellow solid. ES-API: [M+H]⁺=379.2 (free base). ¹H NMR (500 MHz, DMSO-*d₆*) δ 8.43 (d, *J* = 2.5 Hz, 1H), 8.29 (s, 1H), 8.14 (d, *J* = 2.5 Hz, 1H), 7.97 (d, *J =* 3.5 Hz, 1H), 7.81 (d, *J =* 3.5 Hz, 1H), 7.76 - 7.68 (m, 2H), 7.65 (s, 1H), 7.36 (s, 1H), 4.88 (d, *J* = 15.5 Hz, 1H), 4.76 (d, *J* = 15.5 Hz, 1H), 4.20 (t, *J* = 8.0 Hz, 1H), 3.91 - 3.83 (m, 2H), 3.24 - 3.18 (m, 1H), 3.07 - 2.98 (m, 1H), 2.96 - 2.82 (m, 2H), 2.25 - 2.14 (m, 1H), 1.98 - 1.79 (m, 2H), 1.69 - 1.57 (m, 1H).

### Example 165. Synthesis of compound Z245

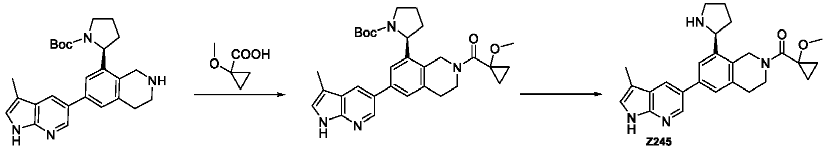

Step 1: 1-Methoxycyclopropane carboxylic acid (9.66 mg, 0.083 mmol), 1-propylphosphonic anhydride (133.6 mg, 208.07 µmol, 50% in ethyl acetate) and triethylamine (21.01 mg, 208.07 µmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in dichloromethane (1 mL) and stirred at 25°C for 2 h. The reaction was monitored by LCMS for completion, and quenched by the addition of water. The reaction mixture was extracted with ethyl acetate, washed with saturated brine, dried over anhydrous sodium sulfate, filtered, and concentrated to give a crude product. The crude product was purified by silica gel column chromatography (0-6% methanol/dichloromethane) to give a product tert-butyl (S)-2-(2-(1-methoxycyclopropanecarbonyl)-6-(3-methyl-1H-pyrrolo[2,3-b]pyridin-5-yl)-1 ,2,3,4-tetrahydroisoquinolin-8-yl)pyrrolidine-1-carboxylate (30 mg, yield: 80%). ES-API:[M+H]⁺= 531.4.

Step 2: Trifluoroacetic acid (0.5 mL) was added to a solution of the compound tert-butyl (S)-2-(2-(1-methoxycyclopropanecarbonyl)-6-(3-methyl-1H-pyrrolo[2,3-b]pyridin-5-yl)-1 ,2,3,4-tetrahydroisoquinolin-8-yl)pyrrolidine-1-carboxylate (30 mg, 0.056 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 mL/min, gradient: B/A = 20%-90% over 50 min, wavelength: 214 nm, column temperature: room temperature) to give a product (S)-(1-methoxycyclopropyl)(6-(3-methyl-1H-pyrrolo[2,3-b]pyridin-5-yl)-8-(pyrrolidin-2-y 1)-3,4-dihydroisoquinolin-2(1H)-yl)methanone (Z245, 8.2 mg, yield: 34%) as a white powder. ES-API:[M+H]⁺=431.3. ¹H NMR (400 MHz, CDCl₃) δ 8.87 (s, 1H), 8.50 (d, *J =* 2.0 Hz, 1H), 8.04 (d, *J=* 2.0 Hz, 1H), 7.75 (s, 1H), 7.30 (s, 1H), 7.09 (s, 1H),5.37-4.50 (m, 2H), 4.39-4.31 (m, 1H), 4.13-3.71 (m, 2H), 3.33 (s, 3H), 3.29-3.23 (m, 1H),3.10-2.94 (m, 3H), 2.35 (s, 3H), 2.33-2.25 (m, 1H), 2.05-1.81 (m, 3H), 1.19-1.10 (m, 2H), 1.04-0.94 (m, 2H).

### Example 166. Synthesis of compound Z75-1

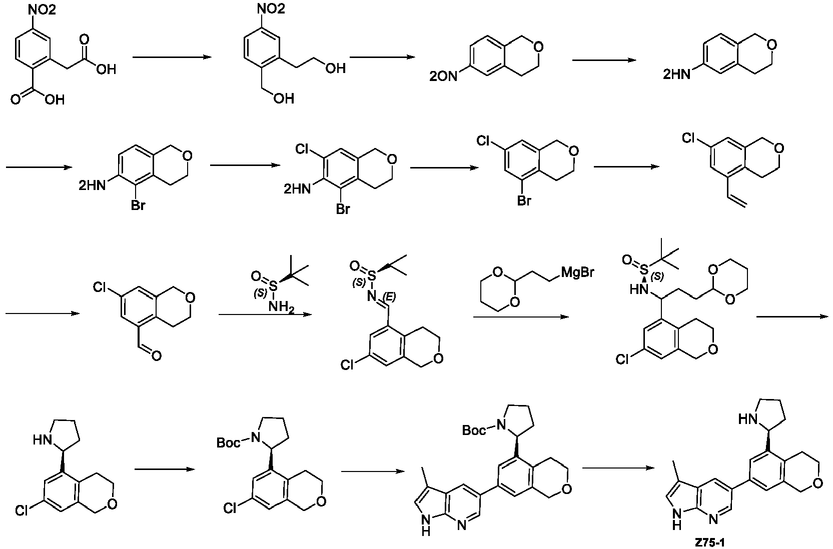

Step 1: 2-(Carboxymethyl)-4-nitrobenzoic acid (9 g, 40 mmol), sodium borohydride (4.6 g, 121 mmol) and tetrahydrofuran (100 mL) were added to a 500 mL single-neck round-bottom flask. 1M boron trifluoride in tetrahydrofuran (19 mL) was slowly added dropwise at 0°C in the presence of protective nitrogen, and the system reacted at 0°C for 3 h. Ethyl acetate (200 mL) was added to the reaction mixture. The obtained mixture was washed with saturated brine (200 mLX3), dried over anhydrous sodium sulfate, filtered, and concentrated to give a crude product. The crude product was purified by a flash silica gel column (methanol:dichloromethane = 10:100) to give a desired product 2-(2-(hydroxymethyl)-5-nitrophenyl)ethan-1-ol (6.6 g, yield: 83%). ES-API: [M+H-18]⁺=180.0.

Step 2: The 2-(2-(hydroxymethyl)-5-nitrophenyl)ethan-1-ol (198 g, 1 mmol), p-toluenesulfonic acid (384 mg, 2 mmol) and toluene (10 mL) were added to a 25 mL single-neck round-bottom flask, and the system reacted at 120°C overnight. Ethyl acetate (50 mL) was added to the reaction mixture. The obtained mixture was washed with saturated brine (50 mLX3), dried over anhydrous sodium sulfate, filtered, and concentrated to give a crude product. The crude product was purified by a flash silica gel column (methanol:dichloromethane = 10:100) to give a desired product 6-nitroisochromane (170 g, yield: 94%). ES-API: [M+H]⁺=180.0.

Step 3: The 6-nitroisochromane (680 mg, 3.78 mmol), palladium on carbon (70 mg) and methanol (10 mL) were added to a 50 mL single-neck round-bottom flask, and the system reacted overnight in the presence of hydrogen. The reaction mixture was filtered and spun to dryness to give a desired product 6-aminoisochromane (550 mg, yield: 97%). ES-API: [M+H]⁺=150.0.

Step 4: The 6-aminoisochromane (550 mg, 3.67 mmol), N-bromosuccinimide (594 mg, 3.3 mmol) and acetonitrile (20 mL) were added to a 50 mL single-neck round-bottom flask and stirred at 0°C for 1 h. The reaction mixture was poured into water (50 mL), extracted with ethyl acetate (50 mL X 3), dried over anhydrous sodium sulfate, filtered, and concentrated to give a crude product. The crude product was purified by a flash silica gel column (ethyl acetate:petroleum ether = 30:100) to give a desired product 5-bromoisochroman-6-amine (600 mg, yield: 71%). ES-API: [M+H]⁺=228.0.

Step 5: The 5-bromoisochroman-6-amine (600 mg, 2.63 mmol), N-chlorosuccinimide (351 mg, 2.63 mmol) and N,N-dimethylformamide (10 mL) were added to a 50 mL single-neck round-bottom flask and stirred at 80°C for 1 h. The reaction mixture was poured into water (30 mL), extracted with 30 mL of ethyl acetate 3 times, dried, and concentrated. The crude product was purified by a flash silica gel column (ethyl acetate:petroleum ether = 30:100) to give a desired product 5-bromo-7-chloroisochroman-6-amine (420 mg, yield: 61%). ES-API: [M+H]⁺=262.0.

Step 6: The 5-bromo-7-chloroisochroman-6-amine (400 mg, 1.52 mmol) and aqueous hypophosphorous acid solution (40 mL) were added to a 50 mL single-neck round-bottom flask. Sodium nitrite (316 mg, 4.58 mmol) was slowly added at 0°C, and the system reacted at 0°C for 2 h. The reaction mixture was added to water (20 mL), adjusted to pH 9-10 with sodium bicarbonate solution, extracted with ethyl acetate (20 mLX3), dried over anhydrous sodium sulfate, filtered, and concentrated to give a crude product. The crude product was purified by a flash silica gel column (methanol:dichloromethane = 10:100) to give a desired product 5-bromo-7-chloroisochromane (320 mg, yield: 85%). ES-API: [M+H]⁺=247.0.

Step 7: The 5-bromo-7-chloroisochromane (320 mg, 1.35 mmol), potassium vinylfluoroborate (180 mg, 1.35 mmol), tetrakis(triphenylphosphine)palladium (156 mg, 0.4 mmol), potassium carbonate (558 mg, 4.1 mmol), dioxane (10 mL) and water (1 mL) were added to a 25 mL three-neck round-bottom flask. The system was replaced with nitrogen three times, protected with a nitrogen balloon, and then reacted at 110°C for 4 h. Ethyl acetate (100 mL) was added to the reaction mixture. The obtained mixture was washed with saturated brine (50 mLX3), dried over anhydrous sodium sulfate, filtered, and concentrated to give a crude product. The crude product was purified by a flash silica gel column (ethyl acetate:petroleum ether = 40:100) to give a desired product 7-chloro-5-vinylisochromane (250 mg, yield: 93%). ES-API: [M+H]⁺=195.1.

Step 8: The 7-chloro-5-vinylisochromane (270 mg, 1.39 mmol), sodium periodate (1.78 g, 8.3 mmol), tetrahydrofuran (10 mL) and water (5 mL) were added to a 25 mL single-neck round-bottom flask. Potassium osmate dihydrate (102 mg, 0.27 mmol) was added in batches under an ice-water bath condition and then stirred at 0°C for 1 h. Ethyl acetate (30 mL) was added to the reaction mixture. The obtained mixture was washed with saturated brine (30 mLX3), dried over anhydrous sodium sulfate, filtered, and concentrated to give a crude product. The crude product was purified by a flash silica gel column (ethyl acetate:petroleum ether = 30:100) to give a desired product 7-chloroisochromane-5-carbaldehyde (110 mg, yield: 40%). ES-API: [M+H]⁺=197.0.

Step 9: The 7-chloroisochromane-5-carbaldehyde (110 mg, 0.56 mmol) and dichloromethane (10 mL) were added to a 50 mL single-neck round-bottom flask. (S)-2-methylpropane-2-sulfinamide (135.6 mg, 1.12 mmol) and tetraethyl titanate (1 mL) were added under an ice-water bath condition and stirred at room temperature for 3 h. Ethyl acetate (30 mL) was added to the reaction mixture. The obtained mixture was washed with saturated brine (30 mLX3), dried over anhydrous sodium sulfate, filtered, and concentrated to give a crude product. The crude product was purified by a flash silica gel column (ethyl acetate:petroleum ether = 20:100) to give a desired product (S,E)-N-((7-chloroisochroman-5-yl)methylene)propane-2-sulfinamide (155 mg, yield: 92%). ES-API: [M+H]⁺=286.1.

Step 10: The (S,E)-N-((7-chloroisochroman-5-yl)methylene)propane-2-sulfinamide (155 mg, 0.56 mmol) and anhydrous tetrahydrofuran (3 mL) were added to a 5 mL three-neck round-bottom flask. The system was replaced with nitrogen three times and then protected with a nitrogen balloon. (2-(1,3-dioxan-2-yl)ethyl)magnesium bromide in tetrahydrofuran (0.5M, 3.1 mL) was added at -78°C and continued to be stirred for 2 h. The reaction mixture was quenched by the addition of saturated ammonium chloride solution (5 mL), and ethyl acetate (30 mL) was added to the mixed solution. The obtained mixture was washed with saturated brine (30 mLX3), dried over anhydrous sodium sulfate, filtered, and concentrated to give a crude product. The crude product was purified by a flash silica gel column (ethyl acetate:petroleum ether = 20:100) to give a desired product (S)-N-(1-(7-chloroisochroman-5-yl)-3-(1,3-dioxan-2-yl)propyl)-2-methylpropane-2-sulfin amide (180 mg, yield: 84%). ES-API: [M+H]⁺=416.2.

Step 11: The (S)-N-(1-(7-chloroisochroman-5-yl)-3-(1,3-dioxan-2-yl)propyl)-2-methylpropane-2-sulfin amide (180 mg, 0.43 mmol), trifluoroacetic acid (3 mL) and water (0.15 mL) were added to a 25 mL single-neck round-bottom flask. Trimethylsilane (503 mg, 4.3 mmol) was added at 0°C and stirred for 18 h. After the reaction mixture was spun to dryness, ethyl acetate (30 mL) was added. The obtained mixture was washed with saturated brine (30 mLX3), dried over anhydrous sodium sulfate, filtered, and concentrated to give a desired product (S)-2-(7-chloroisochroman-5-yl)pyrrolidine (100 mg, yield: 97%). ES-API: [M+H]⁺=238.1.

Step 12: The (S)-2-(7-chloroisochroman-5-yl)pyrrolidine (130 mg, 0.42 mmol), di-tert-butyl dicarbonate (1 mL), triethylamine (174 mg, 1.7 mmol) and dichloromethane (5 mL) were added to a 25 mL single-neck round-bottom flask and stirred for 1 h. Dichloromethane (20 mL) was added to the reaction. The obtained mixture was washed with saturated brine (20 mL X 3), dried over anhydrous sodium sulfate, filtered, and concentrated to give a desired product tert-butyl (S)-2-(7-chloroisochroman-5-yl)pyrrolidine-1-carboxylate (130 mg, yield: 91%). ES-API: [M+H]⁺=338.2.

Step 13: The tert-butyl (S)-2-(7-chloroisochroman-5-yl)pyrrolidine-1-carboxylate (130 mg, 0.38 mmol), 3-methyl-5-(4,4,5,5-tetramethyl-1,3,2-dioxaborolan-2-yl)-1H-pyrrolo[2,3-b]pyridine (149 mg, 0.57 mmol), chloro (2-dicyclohexylphosphino-2',6'-dimethoxy-1,1'-biphenylyl)(2'-amino-1,1'-biphenyl-2-yl) palladium (II) (28 mg, 0.04 mmol), potassium carbonate (160 mg, 1.15 mmol), dioxane (20 mL) and water (3 mL) were added to a 25 mL single-neck round-bottom flask. The system was replaced with nitrogen three times, protected with a nitrogen balloon, and then reacted under microwave at 110°C for 50 min. Ethyl acetate (10 mL) was added to the reaction mixture. The obtained mixture was washed with saturated brine (5 ml X 3), dried over anhydrous sodium sulfate, filtered, and concentrated to give a crude product. The crude product was purified by a flash silica gel column (methanol:dichloromethane = 10:100) to give a desired product tert-butyl (S)-2-(7-(3-methyl-1H-pyrrolo[2,3-b]pyridin-5-yl)isochroman-5-yl)pyrrolidine-1-carboxyl ate (150 mg, yield: 90%). ES-API: [M+H]⁺=434.3.

Step 14: The tert-butyl (S)-2-(7-(3-methyl-1H-pyrrolo[2,3-b]pyridin-5-yl)isochroman-5-yl)pyrrolidine-1-carboxyl ate (150 mg, 0.15 mmol), trifluoroacetic acid (3 mL) and dichloromethane (6 mL) were added to a 5 mL single-neck round-bottom flask and stirred at room temperature for 30 min. The reaction mixture was spun to dryness to give a crude product. The crude product was purified by prep-HPLC (ammonium bicarbonate) to give (S)-3-methyl-5-(5-(pyrrol-2-yl)isochroman-7-yl)-1H-pyrrolo[2,3-b]pyridine (Z75-1, 100 mg, yield: 65%). ES-API: [M+H]⁺=334.2.

### Example 167. Synthesis of compound Z244

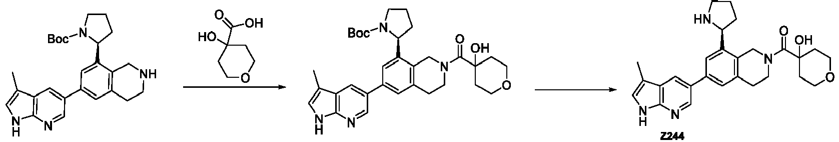

Step 1: 1-(3-Dimethylaminopropyl)-3-ethylcarbodiimide hydrochloride (39.89 mg, 0.21 mmol), 4-hydroxytetrahydropyran-4-carboxylic acid (12.16 mg, 0.083 mmol) and 1-hydroxybenzotriazole (14.06 mg, 0.1 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (30 mg, 0.069 mmol) in N,N-dimethylformamide (1 mL) and stirred at 25°C for 2 h. The reaction was monitored by LCMS for completion, and quenched by the addition of water. The reaction mixture was extracted with ethyl acetate, washed with saturated brine, dried over anhydrous sodium sulfate, filtered, and concentrated to give a crude product. The crude product was purified by silica gel column chromatography (0-6% methanol/dichloromethane) to give a product tert-butyl (S)-2-(2-(2-hydroxy-2-(tetrahydro-2H-pyran-4-yl)acetyl)-6-(3-methyl-1H-pyrrolo[2,3-b]p yridin-5-yl)-1,2,3,4-tetrahydroisoquinolin-8-yl)pyrrolidine-1-carboxylate (20 mg, 51%). ES-API:[M+H]⁺= 561.4

Step 2: Trifluoroacetic acid (0.5 mL) was added to a solution of the compound tert-butyl (S)-2-(2-(2-hydroxy-2-(tetrahydro-2H-pyran-4-yl)acetyl)-6-(3-methyl-1H-pyrrolo[2,3-b]p yridin-5-yl)-1,2,3,4-tetrahydroisoquinolin-8-yl)pyrrolidine-1-carboxylate (20 mg, 0.036 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 ml/min, gradient: B/A = 20%-90% over 50 min, wavelength: 214 nm, column temperature: room temperature) to give a product (S)-(4-hydroxytetrahydro-2H-pyran-4-yl)(6-(3-methyl-1H-pyrrolo[2,3-b]pyridin-5-yl)-8-( pyrrolidin-2-yl)-3,4-dihydroisoquinolin-2(1H)-yl)methanone (Z244, 3.6 mg, yield: 22%) as a white powder. ES-API:[M+H]⁺=461.3.

### Example 168. Synthesis of compound Z242

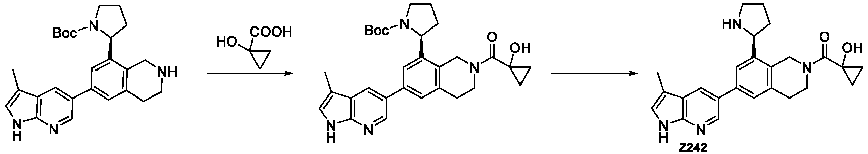

Step 1: 1-Hydroxycyclopropane carboxylic acid (5.66 mg, 0.055 mmol), 1-(3-dimethylaminopropyl)-3-ethylcarbodiimide hydrochloride (26.59 mg, 0.14 mmol) and 1-hydroxybenzotriazole (18.74 mg, 0.14 mmol) were added to a solution of tert-butyl (S)-2-(6-(3-methyl-1H-pyrrolo[2,3-b]pyridin-5-yl)-1,2,3,4-tetrahydroisoquinolin-8-yl)pyrr olidine-1-carboxylate (20 mg, 0.0646 mmol) in N,N-dimethylformamide (1 mL) and stirred at 25°C for 2 h. The reaction was monitored by LCMS for completion, and quenched by the addition of water. The reaction mixture was extracted with ethyl acetate, washed with saturated brine, dried over anhydrous sodium sulfate, filtered, and concentrated to give a crude product. The crude product was purified by silica gel column chromatography (0-6% methanol/dichloromethane) to give a product tert-butyl (S)-2-(2-(1-hydroxycyclopropanecarbonyl)-6-(3-methyl-1H-pyrrolo[2,3-b]pyridin-5-yl)-1, 2,3,4-tetrahydroisoquinolin-8-yl)pyrrolidine-1-carboxylate (30 mg, crude product). ES-API:[M+H]⁺= 517.3.

Step 2: Trifluoroacetic acid (0.5 mL) was added to a solution of the compound tert-butyl (S)-2-(2-(1-hydroxycyclopropanecarbonyl)-6-(3-methyl-1H-pyrrolo[2,3-b]pyridin-5-yl)-1, 2,3,4-tetrahydroisoquinolin-8-yl)pyrrolidine-1-carboxylate (30 mg, 0.058 mmol) in dichloromethane (1 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase system: A: purified water (0.05% ammonia water) B: pure acetonitrile, flow rate: 80 mL/min, gradient: B/A = 20%-90% over 50 min, wavelength: 214 nm, column temperature: room temperature) to give a product (S)-(1-hydroxycyclopropyl)(6-(3-methyl-1H-pyrrolo[2,3-b]pyridin-5-yl)-8-(pyrrolidin-2-y 1)-3,4-dihydroisoquinolin-2(1H)-yl)methanone (Z242, 6.5 mg, yield: 27%) as a white powder. ES-API:[M+H]⁺=417.2.

### Example 169. Synthesis of compound Z436

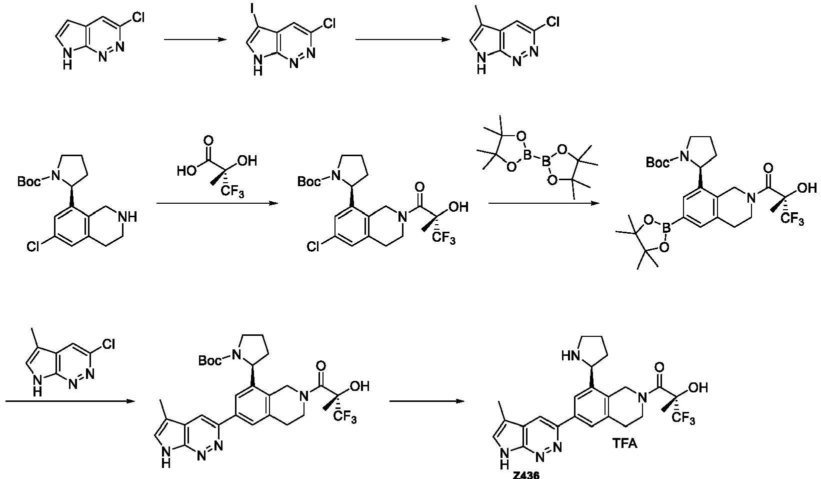

Step 1: N-Iodosuccinimide (879 mg, 3.91 mmol) was slowly added to a solution of 3-chloro-7H-pyrrolo[2,3-c]pyridazine (500 mg, 3.26 mmol) in acetonitrile (20 mL) and stirred at room temperature for 2 h. Upon completion of the reaction, the reaction was quenched with sodium thiosulfate (20 mL) solution, and the reaction mixture was extracted with ethyl acetate (20 mLX3). The organic phases were combined, dried over anhydrous sodium sulfate, filtered, concentrated, and purified by a flash silica gel column (0-8% methanol/dichloromethane) to give 3-chloro-5-iodo-7H-pyrrolo[2,3-c]pyridazine (750 mg, yield: 82%) as a yellow solid. ES-API: [M+H]⁺ = 280.0.

Step 2: In the presence of protective nitrogen, trimethylaluminum in tetrahydrofuran (5.37 mL, 1 M) was added to a mixed solution of the 3-chloro-5-iodo-7H-pyrrolo[2,3-c]pyridazine (750 mg, 2.68 mmol) and 1,1'-bis(diphenylphosphino)ferrocene dichloropalladium (II) (98 mg, 0.13 mmol) in tetrahydrofuran (20 mL) and stirred at 80°C overnight. The reaction mixture was quenched with 1 M hydrochloric acid (20 mL) and spun to dryness. The residue was dissolved in dichloromethane (20 mL), filtered, concentrated, and purified by a flash silica gel column (0-8% methanol/dichloromethane) to give 3-chloro-5-methyl-7H-pyrrolo[2,3-c]pyridazine (250 mg, yield: 56%) as a yellow solid. ES-API: [M+H]⁺ = 168.1.

Step 3: N,N-diisopropylethyl amine (1.29 mL, 7.42 mmol) and 2-(7-azobenzotriazole)-N,N,N',N'-tetramethylurea hexafluorophosphate (847 mg, 2.23 mmol) were added successively to a solution of tert-butyl (S)-2-(6-chloro-1,2,3,4-tetrahydroisoquinolin-8-yl)pyrrolidine-1-carboxylate (500 mg, 1.48 mmol) and (S)-2-trifluoromethyl-2-hydroxypropanoic acid (469 mg, 2.97 mmol) in dichloromethane (20 mL) and stirred at room temperature for 1 h. Upon completion of the reaction, the reaction mixture was concentrated and purified by a flash silica gel column (1-100% tetrahydrofuran/petroleum ether) to give tert-butyl (S)-2-(6-chloro-2-((S)-3,3,3-trifluoro-2-hydroxy-2-methylpropanoyl)-1,2,3,4-tetrahydroiso quinolin-8-yl)pyrrolidine-1-carboxylate (650 mg, yield: 92%) as a clear oil. ES-API: [M+H-Boc]⁺ = 377.2.

Step 4: In the presence of protective nitrogen, a mixture of the tert-butyl (S)-2-(6-chloro-2-((S)-3,3,3-trifluoro-2-hydroxy-2-methylpropanoyl)-1,2,3,4-tetrahydroiso quinolin-8-yl)pyrrolidine-1-carboxylate (215 mg, 0.45 mmol), bis(pinacolato)diboron (343 mg, 1.35 mmol), chloro (2-dicyclohexylphosphino-2',6'-dimethoxy-1,1'-biphenylyl)(2'-amino-1,1'-biphenyl-2-yl) palladium (II) (32 mg, 0.04 mmol) and potassium acetate (133 mg, 1.35 mmol) in 1,4-dioxane (5 mL) was stirred at 120°C for 2 h. Upon completion of the reaction, the reaction mixture was filtered, concentrated, and purified by a flash silica gel column (0-100% tetrahydrofuran/petroleum ether) to give tert-butyl (S)-2-(6-(4,4,5,5-tetramethyl-1,3,2-dioxaborolan-2-yl)-2-((S)-3,3,3-trifluoro-2-hydroxy-2-methylpropanoyl)-1,2,3,4-tetrahydroisoquinolin-8-yl)pyrrolidine-1-carboxylate (220 mg, yield: 86%) as a clear oil. ES-API: [M+H-Boc]⁺ = 469.2.

Step 5: In the presence of protective nitrogen, a mixed solution of the tert-butyl (S)-2-(6-(4,4,5,5-tetramethyl-1,3,2-dioxaborolan-2-yl)-2-((S)-3,3,3-trifluoro-2-hydroxy-2-methylpropanoyl)-1,2,3,4-tetrahydroisoquinolin-8-yl)pyrrolidine-1-carboxylate (100 mg, 0.18 mmol), 3-chloro-5-methyl-7H-pyrrolo[2,3-c]pyridazine (60 mg, 0.36 mmol), chloro (2-dicyclohexylphosphino-2',6'-dimethoxy-1,1'-biphenylyl)(2'-amino-1,1'-biphenyl-2-yl) palladium (II) (13 mg, 0.02 mmol) and potassium carbonate (73 mg, 0.53 mmol) in 1,4-dioxane (2 mL) and water (0.4 mL) was stirred at 100°C for 2 h. The reaction mixture was poured into ethyl acetate (10 mL) and washed with saturated brine (5 mL) and water (5 mL) successively. The organic phase was dried over anhydrous sodium sulfate, filtered, concentrated, and purified by a flash silica gel column (0-10% methanol/dichloromethane) to give tert-butyl (S)-2-(6-(5-methyl-7H-pyrrolo[2,3-c]pyridazin-3-yl)-2-((S)-3,3,3-trifluoro-2-hydroxy-2-m ethylpropanoyl)-1,2,3,4-tetrahydroisoquinolin-8-yl)pyrrolidine-1-carboxylate (50 mg, yield: 50%). ES-API: [M+H-Boc]⁺ = 474.2.

Step 6: Trifluoroacetic acid (0.5 mL) was added dropwise to a solution of the tert-butyl (S)-2-(6-(5-methyl-7H-pyrrolo[2,3-c]pyridazin-3-yl)-2-((S)-3,3,3-trifluoro-2-hydroxy-2-m ethylpropanoyl)-1,2,3,4-tetrahydroisoquinolin-8-yl)pyrrolidine-1-carboxylate (50 mg, 87 µmol) in dichloromethane (1 mL) under an ice bath condition and stirred at room temperature for 1 h. The reaction mixture was concentrated, neutralized with 7M amine-methanol (5 mL), concentrated, and purified by prep-HPLC (trifluoroacetic acid) to give (S)-3,3,3-trifluoro-2-hydroxy-2-methyl-1-(6-(5-methyl-7H-pyrrolo[2,3-c]pyridazin-3-yl)-8 -((S)-pyrrolidin-2-yl)-3,4-dihydroisoquinolin-2(1H)-yl)propan-1-one trifluoroacetate (Z436, 2.5 mg, purity: 100%, yield: 5%) as a white solid. ES-API: [M+H]⁺ = 474.2.

### Example 170. Synthesis of compound Z405

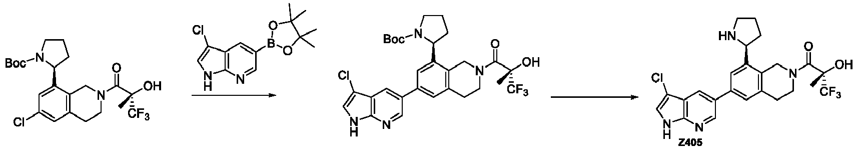

Step 1: tert-butyl (S)-2-(6-chloro-2-((S)-3,3,3-trifluoro-2-hydroxy-2-methylpropanoyl)-1,2,3,4-tetrahydroiso quinolin-8-yl)pyrrolidine-1-carboxylate (55 mg, 0.116 mmol), 3-chloro-5-(4,4,5,5-tetramethyl-1,3,2-dioxaborolan-2-yl)-1H-pyrrolo[2,3-b]pyridine (96 mg, 0.34 mmol), chloro (2-dicyclohexylphosphino-2',6'-dimethoxy-1,1'-biphenylyl)(2'-amino-1,1'-biphenyl-2-yl) palladium (II) (8 mg, 0.012 mmol) and potassium carbonate (48 mg, 0.34 mmol) were dissolved in 1,4-dioxane (5 ml) and stirred at 110°C for 2 h. The reaction mixture was concentrated to dryness under reduced pressure to give a crude product. The crude product was purified by silica gel column chromatography (petroleum ether/ethyl acetate = 50/50) to give tert-butyl (S)-2-(6-(3-chloro-1H-pyrrolo[2,3-b]pyridin-5-yl)-2-((S)-3,3,3-trifluoro-2-hydroxy-2-met hylpropanoyl)-1,2,3,4-tetrahydroisoquinolin-8-yl)pyrrolidine-1-carboxylate (35 mg, yield: 51%). ES-API: [M+H]⁺=593.

Step 2: The tert-butyl (S)-2-(6-(3-chloro-1H-pyrrolo[2,3-b]pyridin-5-yl)-2-((S)-3,3,3-trifluoro-2-hydroxy-2-met hylpropanoyl)-1,2,3,4-tetrahydroisoquinolin-8-yl)pyrrolidine-1-carboxylate (35 mg, 0.06 mmol) was dissolved in anhydrous dichloromethane (0.5 mL). Trifluoroacetic acid (0.2 mL) was added, and the system reacted at room temperature for 2 h. The reaction was monitored by LC-MS for completion, and the reaction mixture was concentrated to dryness under reduced pressure. After neutralization with ammonia-methanol solution (1 mL), the obtained mixture was concentrated again to give a crude product. The crude product was purified by prep-HPLC acid process (formic acid) to give (S)-1-(6-(3-chloro-1H-pyrrolo[2,3-b]pyridin-5-yl)-8-((S)-pyrrolidin-2-yl)-3,4-dihydroisoq uinolin-2(1H)-yl)-3,3,3-trifluoro-2-hydroxy-2-methylpropan-1-one (Z405, 18.6 mg, yield: 58%). ES-API: [M+H]⁺=493. ¹H NMR (400 MHz, DMSO-d₆) δ 12.07 (s, 1H), 8.61 (d, *J* = 1.6 Hz, 1H), 8.30 (s, 1H), 8.12 (s, 1H), 7.79 (s, 1H), 7.73 (s, 1H), 7.48 (s, 1H), 5.25 - 5.15 (m, 1H), 4.87 - 4.75 (m, 1H), 4.32 (s, 1H), 4.17 - 4.07 (m, 1H), 3.87 - 3.83 (m, 1H), 3.63 - 3.60 (m, 1H), 3.20 (s, 2H), 3.01 - 2.93 (m, 3H), 2.27 - 2.17 (m, 1H), 1.89 - 1.88 (m, 2H), 1.59 (s, 3H).

### Example 171. Synthesis of compound Z367-1

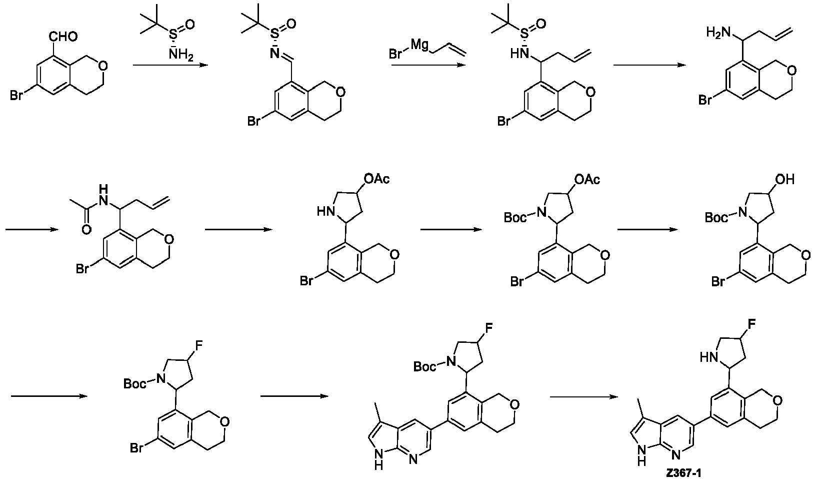

Step 1: (S)-Tert-butylsulfinamide (337 mg, 2.78 mmol) was added to a solution of 6-bromoisochromene-8-carbaldehyde (670 mg, 2.78 mmol) in tetrahydrofuran (20 mL). Tetraisopropyl titanate (2.34 g, 8.34 mmol) was added under an ice bath condition and stirred at room temperature for 3 h. The reaction was monitored by LCMS for completion, and saturated brine (20 mL) was added. The obtained mixture was filtered by suction, and the phase was separated. The organic phase was dried over anhydrous sodium sulfate, filtered, and concentrated under reduced pressure to give (S,E)-N-((6-bromoisochromen-8-yl)methylene)-2-methylpropane-2-sulfonamide (850 mg, yield: 88%). ES-API: [M+H]⁺=344.

Step 2: The (S,E)-N-((6-bromoisochromen-8-yl)methylene)-2-methylpropane-2-sulfonamide (500 mg, 1.4 mmol) was dissolved in tetrahydrofuran (15 mL). Allylmagnesium bromide (4.3 mL, 4.3 mmol) was added at -78°C, and the system reacted at room temperature for 2 h. A saturated ammonium chloride solution (10 mL) was added, and the obtained mixture was extracted with ethyl acetate (10 mLX3). The organic phases were combined, washed with saturated brine (15 mL), dried over anhydrous sodium sulfate, and concentrated under reduced pressure to give a crude product. The crude product was purified by silica gel column chromatography (petroleum ether/ethyl acetate = 50/50) to give (S)-N-(1-(6-bromoisochromen-8-yl)but-3-en-1-yl)-2-methylpropane-2-sulfonamide (400 mg, yield: 71%). ES-API: [M+H]⁺=386.

Step 3: The (S)-N-(1-(6-bromoisochromen-8-yl)but-3-en-1-yl)-2-methylpropane-2-sulfonamide (400 mg, 1 mmol) was dissolved in methanol (10 mL). Hydrochloric acid/1,4-dioxane (0.7 ml, 3 mmol) was added, and the system reacted at room temperature for 16 h. Upon completion of the reaction, the reaction mixture was concentrated to dryness under reduced pressure, and water (10 mL) was added. The obtained mixture was adjusted to pH 9 with saturated sodium carbonate solution, and extracted with ethyl acetate (10 mLX3). The organic phases were combined, washed with saturated brine (15 mL), dried over anhydrous sodium sulfate, and concentrated under reduced pressure to give 1-(6-bromoisochromen-8-yl)but-3-en-1-amine (230 mg, yield: 78%). ES-API: [M+H]⁺=282.

Step 4: The 1-(6-bromoisochromen-8-yl)but-3-en-1-amine (230 mg, 0.8 mmol) was dissolved in dichloromethane (10 mL). Triethylamine (123 mg, 1.2 mmol) was added, followed by acetic anhydride (91 mg, 0.9 mmol), and the system reacted at room temperature for 16 h. The reaction mixture was diluted with dichloromethane (10 mL), washed with water (10 mL) and saturated brine (10 mL), dried over anhydrous sodium sulfate, concentrated to dryness under reduced pressure to give N-(1-(6-bromoisochromen-8-yl)but-3-en-1-yl)acetamide (230 mg, yield: 88%). ES-API: [M+H]⁺=324.

Step 5: The N-(1-(6-bromoisochromen-8-yl)but-3-en-1-yl)acetamide (170 mg, 0.5 mmol) was dissolved in tetrahydrofuran/water (15 mL). Iodine (333 mg, 1.3 mmol) was added, and the system reacted at room temperature for 16 h. Saturated sodium sulfite solution (5 mL) and saturated sodium bicarbonate solution (5 mL) were added to the reaction mixture. The obtained mixture was extracted with ethyl acetate (10 mLX33), washed with water (10 mL) and saturated brine (10 mL), dried over anhydrous sodium sulfate, and concentrated to dryness under reduced pressure to give 5-(6-bromoisochromen-8-yl)pyrrolidin-3-yl acetate (140 mg, yield: 78%). ES-API: [M+H]⁺=340.

Step 6: The 5-(6-bromoisochromen-8-yl)pyrrolidin-3-yl acetate (556 mg, 1.6 mmol) was dissolved in dichloromethane (15 mL). Triethylamine (247 mg, 2.4 mmol) was added, followed by di-tert-butyl carbonate (356 mg, 1.6 mmol), and the system reacted at room temperature for 16 h. The reaction mixture was diluted with dichloromethane (10 mL), washed with water (10 mL) and saturated brine (10 mL), dried over anhydrous sodium sulfate, concentrated to dryness under reduced pressure to give tert-butyl 4-acetoxy-2-(6-bromoisochromen-8-yl)pyrrolidine-1-carboxylate (633 mg, yield: 88%). ES-API: [M+H]⁺=440.

Step 7: The tert-butyl 4-acetoxy-2-(6-bromoisochromen-8-yl)pyrrolidine-1-carboxylate (600 mg, 1.3 mmol) was dissolved in methanol (5 mL). 1N Sodium hydroxide solution (1.5 ml, 1.5 mmol) was added, and the system reacted at room temperature for 16 h. The reaction mixture was concentrated under reduced pressure, diluted with dichloromethane (10 mL), washed with water (10 mL) and saturated brine (10 mL), dried over anhydrous sodium sulfate, and concentrated to dryness under reduced pressure to give tert-butyl 2-(6-bromoisochromen-8-yl)-4-hydroxypyrrolidine-1-carboxylate (440 mg, yield: 81%). ES-API: [M+H]⁺=398.

Step 8: The tert-butyl 2-(6-bromoisochromen-8-yl)-4-hydroxypyrrolidine-1-carboxylate (200 mg, 0.5 mmol) was dissolved in dichloromethane (5 mL). Diethylaminosulfur trifluoride (161 mg, 1.0 mmol) was added at -78°C, and the system reacted at room temperature for 16 h. The reaction mixture was concentrated under reduced pressure, and diluted with dichloromethane (10 mL). A saturated sodium bicarbonate solution (5 mL) was added, and the phase was separated. The organic phase was washed with water (10 mL) and saturated brine (10 mL), dried over anhydrous sodium sulfate, and concentrated to dryness under reduced pressure to give a crude product. The crude product was purified by silica gel column chromatography (petroleum ether/ethyl acetate = 80/20) to give tert-butyl 2-(6-bromoisochromen-8-yl)-4-fluoropyrrolidine-1-carboxylate (160 mg, yield: 80%). ES-API: [M+H]⁺=400.

Step 9: The tert-butyl 2-(6-bromoisochromen-8-yl)-4-fluoropyrrolidine-1-carboxylate (110 mg, 0.27 mmol), 3-methyl-7-azaindole-5-boronic acid pinacol ester (212 mg, 0.82 mmol) and potassium carbonate (114 mg, 0.82 mmol) were dissolved in 1,4-dioxane/water (3 mL). 1,1-bis(diphenylphosphino)ferrocene dichloropalladium (10 mg) was added, and the system reacted at 110°C in the presence of protective nitrogen for 2 h. The reaction mixture was concentrated under reduced pressure to give a crude product. The crude product was purified by silica gel column chromatography (petroleum ether/ethyl acetate = 50/50) to give tert-butyl 4-fluoro-2-(6-(3-methyl-1H-pyrrolo[2,3-b]pyridin-5-yl)isochromen-8-yl)pyrrolidine-1-car boxylate (90 mg, yield: 72%). ES-API: [M+H]⁺=452.

Step 10: The tert-butyl 4-fluoro-2-(6-(3-methyl-1H-pyrrolo[2,3-b]pyridin-5-yl)isochromen-8-yl)pyrrolidine-1-car boxylate (20 mg, 0.044 mmol) was dissolved in anhydrous dichloromethane (0.5 mL). Trifluoroacetic acid (0.2 mL) was added, and the system reacted at room temperature for 2 h. The reaction was monitored by LC-MS for completion, and the reaction mixture was concentrated to dryness under reduced pressure. After neutralization with ammonia-methanol solution (1 mL), the obtained mixture was concentrated again to give a crude product. The crude product was purified by prep-HPLC acid process (formic acid) to give 5-(8-(4-fluoropyrrolidin-2-yl)isochromen-6-yl)-3-methyl-1H-pyrrolo[2,3-b]pyridine (Z367-1, 10.6 mg, yield: 56%). ES-API: [M+H]⁺=352. ¹H NMR (400 MHz, DMSO-d₆) δ 11.32 (s, 1H), 8.46 (d, *J=* 2.0 Hz, 1H), 8.24 (s, 1H), 8.09 (d, *J=* 2.0 Hz, 1H), 7.69 (s, 1H), 7.37 (s, 1H), 7.25 (s, 1H), 5.42 - 5.39 (m, 1H), 5.27 (s, 1H), 4.91 - 4.87 (m, 1H), 4.78 - 4.74 (m, 1H), 4.32 (dd, J = 10.0, 6.0 Hz, 1H), 3.95 - 3.81 (m, 3H), 3.40 - 3.36 (m, 1H), 3.15 - 3.07 (m, 1H), 2.96 - 2.83 (m, 2H), 2.31 (s, 3H), 1.82 - 1.66 (m, 1H).

### Example 172. Synthesis of compound Z362

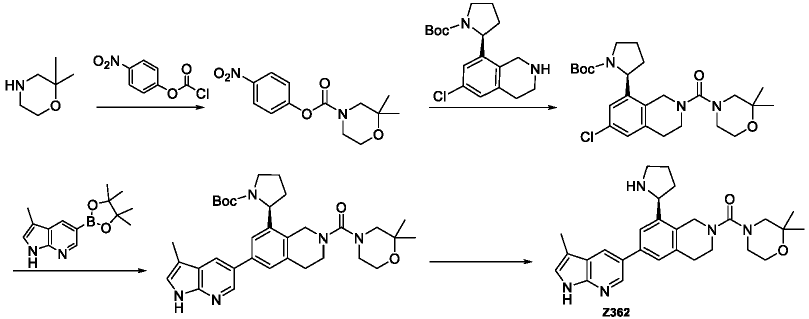

Step 1: In the presence of protective nitrogen, triethylamine (350 mg, 3.47 mmol) and (4-nitrophenyl)carbonyl chloride (480 mg, 2.08 mmol) were added to a solution of 2,2-dimethylmorpholine (200 mg, 1.74 mmol) in anhydrous dichloromethane (10 mL) at 0°C, and the mixture was warmed to 20°C and stirred for 3 h. The reaction was monitored by LCMS for completion, and the reaction mixture was concentrated to give a crude product. The crude product was purified by silica gel column chromatography (petroleum ether/ethyl acetate = 10:90) to give 4-nitrophenyl 2,2-dimethylmorpholine-4-carboxylate (480 mg, yield: 98.62%) as a yellow solid. ES-API:[M+1]⁺=281.1.

Step 2: The 4-nitrophenyl 2,2-dimethylmorpholine-4-carboxylate (87.36 mg, 0.31 mmol) and potassium carbonate (57.44 mg, 0.42 mmol) were added to a solution of tert-butyl (S)-2-(6-chloro-1,2,3,4-tetrahydroisoquinolin-8-yl)pyrrolidine-1-carboxylate (70 mg, 0.21 mmol) in N,N-dimethylacetamide (1 mL), and the mixture was warmed to 130°C and stirred for 3 h. The reaction was monitored by LCMS for completion. The reaction mixture was diluted with water and extracted with ethyl acetate. The combined organic phase was washed with saturated brine, dried over anhydrous sodium sulfate, filtered, and concentrated to give a crude product. The crude product was purified by a preparative thin layer chromatographic column (petroleum ether:ethyl acetate = 1:1) to give tert-butyl (S)-2-(6-chloro-2-(2,2-dimethylmorpholine-4-carbonyl)-1,2,3,4-tetrahydroisoquinolin-8-yl )pyrrolidine-1-carboxylate (70.00 mg, yield: 70.47%) as a yellow oil. ES-API:[M+1]⁺=478.3.

Step 3: 3-Methyl-5-(4,4,5,5-tetramethyl-1,3,2-dioxaborolan-2-yl)-1H-pyrrolo[2,3-b]pyridine (45.36 mg, 0.18 mmol), chloro (2-dicyclohexylphosphino-2',6'-dimethoxy-1,1'-biphenylyl)(2'-amino-1,1'-biphenyl-2-yl) palladium (II) (10.54 mg, 14.64 µmol) and potassium carbonate (60.62 mg, 0.44 mmol) were added to a solution of the tert-butyl (S)-2-(6-chloro-2-(2,2-dimethylmorpholine-4-carbonyl)-1,2,3,4-tetrahydroisoquinolin-8-yl )pyrrolidine-1-carboxylate (70 mg, 0.15 mmol) in dioxane/water (2 mL/0.4 mL), and the system was heated to 100°C for 2 h. Upon completion of the reaction, the reaction mixture was spun to dryness to give a crude product. The crude product was purified by a preparative thin layer chromatographic column (dichloromethane/methanol = 10/1) to give a product tert-butyl (S)-2-(2-(2,2-dimethylmorpholine-4-carbonyl)-6-(3-methyl-1H-pyrrolo[2,3-b]pyridin-5-yl )-1,2,3,4-tetrahydroisoquinolin-8-yl)pyrrolidine-1-carboxylate (60.00 mg, yield: 71.42%) as a yellow oil. ES-API:[M+1]⁺=574.4.

Step 4: The tert-butyl (S)-2-(2-(2,2-dimethylmorpholine-4-carbonyl)-6-(3-methyl-1H-pyrrolo[2,3-b]pyridin-5-yl )-1,2,3,4-tetrahydroisoquinolin-8-yl)pyrrolidine-1-carboxylate (60 mg, 0.11 mmol) was dissolved in dichloromethane (2.0 mL). Trifluoroacetic acid (1.0 mL) was added. The system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 8/1) to give a product (S)-(2,2-dimethylmorpholine)(6-(3-methyl-1H-pyrrolo[2,3-b]pyridin-5-yl)-8-(pyrrolidin-2 -yl)-3,4-dihydroisoquinolin-2(1H)-yl)methanone (Z362, 14.5 mg, yield: 29.28%) as a white solid. ES-API:[M+1]⁺=474.3. ¹H NMR (400 MHz, CDCl₃) δ 9.36 (s, 1H), 8.50 (s, 1H), 7.98 (s, 1H), 7.76 (s, 1H), 7.29 (s, 1H), 7.08 (s, 1H),4.64 (d, *J* = 16.3 Hz, 1H), 4.55-4.38 (m, 2H), 3.84-3.73 (m, 2H), 3.61-3.33 (m, 3H), 3.32-3.25 (m, 2H),3.21-310 (m, 3H), 3.05-2.90 (m, 2H), 2.34 (s, 3H), 2.32-2.26 (m, 1H), 2.12-1.78 (m, 3H), 1.27 (s, 6H).

### Example 173. Synthesis of compound Z345

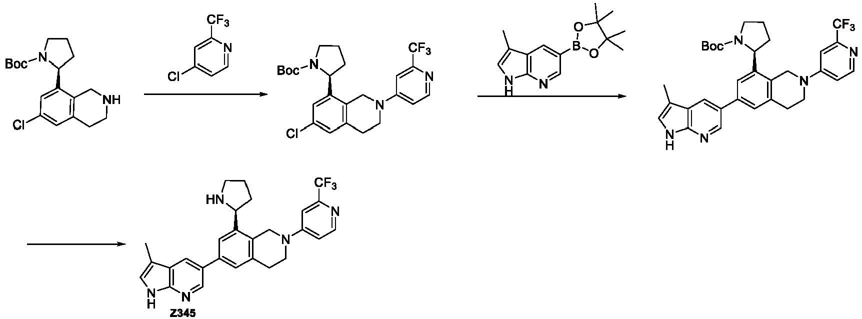

Step 1: Diisopropylethyl amine (115.12 mg, 890.59 µmol) and 4-chloro-2-(trifluoromethyl)pyridine (161.69 mg, 890.59 µmol) were added to a solution of tert-butyl (S)-2-(6-chloro-1,2,3,4-tetrahydroisoquinolin-8-yl)pyrrolidine-1-carboxylate (100 mg, 296.86 µmol) in N-methylpyrrolidinone (1 mL), and the system reacted at 130°C for 15 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (0-5% methanol in dichloromethane) to give a product tert-butyl (S)-2-(6-chloro-2-(2-(trifluoromethyl)pyridin-4-yl)-1,2,3,4-tetrahydroisoquinolin-8-yl)pyrr olidine-1-carboxylate (100 mg, yield: 70%). ES-API:[M+H]⁺=483.2.

Step 2: The compound tert-butyl (S)-2-(6-chloro-2-(2-(trifluoromethyl)pyridin-4-yl)-1,2,3,4-tetrahydroisoquinolin-8-yl)pyrr olidine-1-carboxylate (100 mg, 207.58 µmol) was dissolved in dioxane/water (2 mL/0.4 mL). 3-Methyl-5-(4,4,5,5-tetramethyl-1,3,2-dioxaborolan-2-yl)-1*H*-pyrrolo[2,3-*b*]pyridine (69.77 mg, 270.29 mmol), chloro (2-dicyclohexylphosphino-2',6'-dimethoxy-1,1'-biphenylyl)(2'-amino-1,1'-biphenyl-2-yl) palladium (II) (14.94 mg, 20.76 µmol) and potassium carbonate (85.94 mg, 622.74 µmol) were added. The system was heated to 100°C for 2 h. The reaction mixture was extracted with dichloromethane/water, dried over anhydrous sodium sulfate, filtered, and concentrated to give a crude product. The crude product was purified by a preparative thin layer chromatographic column (dichloromethane/methanol = 10/1) to give a product tert-butyl (S)-2-(6-(3-methyl-1*H*-pyrrolo[2,3-*b*]pyridin-5-yl)-2-(2-(trifluoromethyl)pyridin-4-yl)-1,2, 3,4-tetrahydroisoquinolin-8-yl)pyrrolidine-1-carboxylate (79 mg, yield: 66.6%). ES-API: [M+H]⁺=578.3.

Step 3: The tert-butyl (S)-2-(6-(3-methyl-1*H*-pyrrolo[2,3-*b*]pyridin-5-yl)-2-(2-(trifluoromethyl)pyridin-4-yl)-1,2, 3,4-tetrahydroisoquinolin-8-yl)pyrrolidine-1-carboxylate (79 mg, 136.76 µmol) was dissolved in dichloromethane (1 mL). Trifluoroacetic acid (1 mL) was added, and the system reacted at room temperature for 1 h. Upon completion of the reaction, the reaction mixture was spun to dryness, extracted with dichloromethane and saturated aqueous sodium bicarbonate solution, dried over anhydrous sulfuric acid, filtered, and spun to dryness to give a crude product. The crude product was purified by a preparative thin layer chromatographic column (dichloromethane/methanol = 10/1) to give a product (S)-6-(3-methyl-1H-pyrrolo[2,3-b]pyridin-5-yl)-8-(pyrrolidin-2-yl)-2-(2-(trifluoromethyl) pyridin-4-yl)-1,2,3,4-tetrahydroisoquinoline (Z345, 20.12 mg, yield: 31%). ES-API:[M+1]⁺=478.2. ¹H NMR (400 MHz, DMSO-*d₆*) δ 11.32 (s, 1H), 8.46 (d, *J* = 2.0 Hz, 1H), 8.29 (d, *J* = 6.0 Hz, 1H), 8.10 (d, *J* = 2.0 Hz, 1H), 7.73 (d, *J* = 2.0 Hz, 1H), 7.46-7.41 (m, 1H), 7.29 (d, *J* = 2.4 Hz, 1H), 7.25-7.22 (m, 1H), 7.13-7.10 (m, 1H), 4.78-4.59 (m, 2H), 4.47-4.33 (m, 1H), 3.82-3.59 (m, 2H), 3.17-3.10 (m, 1H), 3.04-2.94 (m, 3H), 2.28 (s, 3H), 2.26-2.20 (m, 1H), 1.90-1.77 (m, 2H), 1.60-1.50 (m, 1H).

### Example 174. Synthesis of compound Z359-1 and compound Z359-2

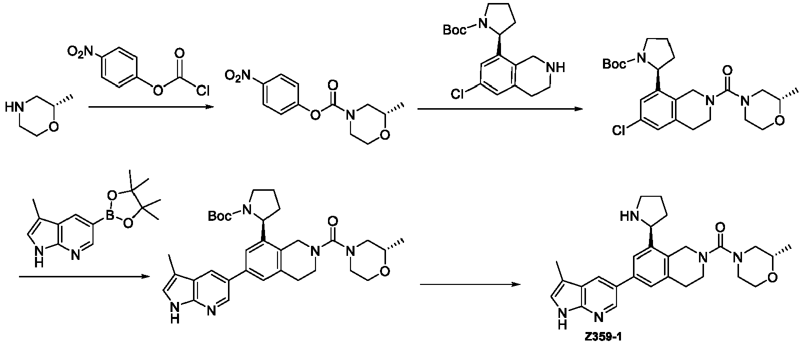

Step 1: In the presence of protective nitrogen, triethylamine (0.4 g, 3.95 mmol) and (4-nitrophenyl)carbonyl chloride (0.48 g, 2.37 mmol) were added to a solution of (S)-2-methylmorpholine (0.2 g, 1.98 mmol) in anhydrous dichloromethane (5 mL) at 0°C, and the mixture was warmed to 20°C and stirred for 3 h. The reaction was monitored by LCMS for completion, and the reaction mixture was concentrated to give a crude product. The crude product was purified by silica gel column chromatography (petroleum ether/ethyl acetate = 10:90) to give (4-nitrophenyl)(S)-2-methylmorpholine-4-carboxylate (0.45 g, yield: 85.48%) as a yellow solid. ES-API:[M+1]⁺=267.1.

Step 2: The (4-nitrophenyl)(S)-2-methylmorpholine-4-carboxylate (82.99 mg, 0.31 mmol) and potassium carbonate (57.44 mg, 0.42 mmol) were added to a solution of tert-butyl (S)-2-(6-chloro-1,2,3,4-tetrahydroisoquinolin-8-yl)pyrrolidine-1-carboxylate (70 mg, 0.21 mmol) in N,N-dimethylacetamide (1 mL), and the mixture was warmed to 150°C and stirred for 3 h. The reaction was monitored by LCMS for completion. The reaction mixture was diluted with water and extracted with ethyl acetate. The combined organic phase was washed with saturated brine, dried over anhydrous sodium sulfate, filtered, and concentrated to give a crude product. The crude product was purified by a preparative thin layer chromatographic column (petroleum ether:ethyl acetate = 1:1) to give tert-butyl (S)-2-[6-chloro-2-[(*S*)-2-methylmorpholine-4-carbonyl]-1,2,3,4-tetrahydroisoquinolin-8-yl ]pyrrolidine-1-carboxylate (67.00 mg, yield: 69.49%) as a yellow oil. ES-API:[M+1]⁺=464.3.

Step 3: 3-Methyl-5-(4,4,5,5-tetramethyl-1,3,2-dioxaborolan-2-yl)-1*H*-pyrrolo[2,3-*b*]pyridine (44.73 mg, 0.17 mmol), chloro (2-dicyclohexylphosphino-2',6'-dimethoxy-1,1'-biphenylyl)(2'-amino-1,1'-biphenyl-2-yl) palladium (II) (10.4 mg, 14.44 µmol) and potassium carbonate (59.78 mg, 0.43 mmol) were added to a solution of the tert-butyl (S)-2-[6-chloro-2-[(*S*)-2-methylmorpholine-4-carbonyl]-1,2,3,4-tetrahydroisoquinolin-8-yl ]pyrrolidine-1-carboxylate (67 mg, 0.14 mmol) in dioxane/water (4 mL/0.8 mL), and the system was heated to 100°C for 1 h. Upon completion of the reaction, the reaction mixture was spun to dryness to give a crude product. The crude product was purified by a preparative thin layer chromatographic column (dichloromethane/methanol = 10/1) to give a product tert-butyl (S)-2-[2-[(*S*)-2-methylmorpholine-4-carbonyl]-6-(3-methyl-1*H*-pyrrolo[2,3-*b*]pyridin-5-yl )-1,2,3,4-tetrahydroisoquinolin-8-yl]pyrrolidine-1-carboxylate (64.50 mg, yield: 79.81%) as yellow oil. ES-API:[M+1]⁺=560.3.

Step 4: Tert-butyl (*S*)-2-[2-[(*S*)-2-methylmorpholine-4-carbonyl]-6-(3-methyl-1*H*-pyrrolo[2,3-*b*]pyridin-5-yl )-1,2,3,4-tetrahydro-1*H*-isoquinolin-8-yl]pyrrolidine-1-carboxylate (64.5 mg, 0.12 mmol) was dissolved in dichloromethane (2.0 mL). Trifluoroacetic acid (2.0 mL) was added, and the system reacted at room temperature for 1 h. Upon completion of the reaction, the mixture was quenched with saturated aqueous sodium bicarbonate solution, and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 10/1) to give a product (6-(3-methyl-1H-pyrrolo[2,3-b]pyridin-5-yl)-8-((S)-pyrrolidin-2-yl)-3,4-dihydroisoquinoli n-2(1H)-yl)((S)-2-methylmorpholin)methanone (Z359-1, 23.40 mg, yield: 44.18%) as a yellow solid. ES-API:[M+1]⁺=460.3. ¹H NMR (400 MHz, CDCl₃) *δ* 10.28 (s, 1H), 8.47 (d, *J* = 2.0 Hz, 1H), 7.97 (d, *J* = 2.0 Hz, 1H), 7.73 (d, *J* = 1.4 Hz, 1H), 7.23 (s, 1H), 7.10 (s, 1H), 4.61 (d, *J* = 16.3 Hz, 1H), 4.53-4.40 (m, 2H), 3.88-3.83 (m, 1H), 3.67-3.51 (m, 5H), 3.49-3.31 (m, 2H), 3.21-2.84 (m, 4H), 2.73-2.66 (m, 1H), 2.33-2.25 (m, 4H), 2.08-2.01 (m, 1H), 1.97-1.88 (m, 1H), 1.84-1.76 (m, 1H), 1.17 (d, *J* = 6.0 Hz, 3H).

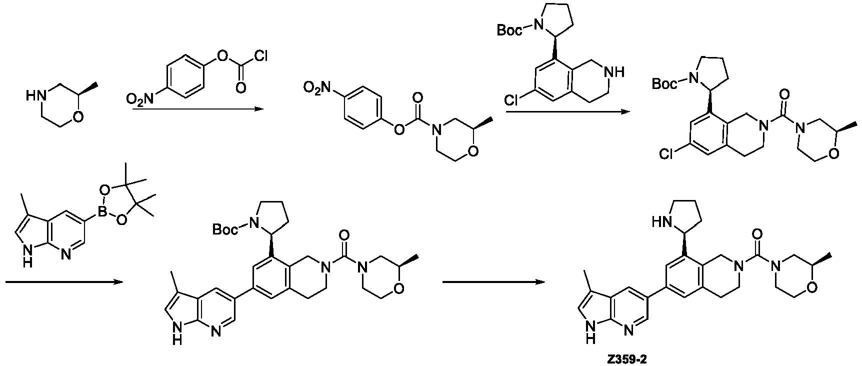

Step 1: A solution of (R)-2-methylmorpholine (200.00 mg, 1.98 mmol) in dichloromethane (10 mL) was cooled in an ice bath. Triethylamine (400.17 mg, 3.95 mmol) and (4-nitrophenyl)carbonyl chloride (478.27 mg, 2.37 mmol) were then added successively, and the mixture was warmed to room temperature and stirred for 3 h. The reaction mixture was spun to dryness to remove a solvent and obtain a crude product. The crude product was purified by silica gel column chromatography (petroleum ether/ethyl acetate = 5/1) to give (4-nitrophenyl)(R)-2-methylmorpholine-4-carboxylate (380 mg, yield: 72.74%). ES-API: [M+H]⁺ = 267.10.

Step 2: Diisopropylethyl amine (97.08 mg, 751.18 µmol) and tert-butyl (S)-2-(6-chloro-1,2,3,4-tetrahydroisoquinolin-8-yl)pyrrolidine-1-carboxylate (63.26 mg, 187.79 µmol) were added to a solution of the (4-nitrophenyl)(R)-2-methylmorpholine-4-carboxylate (50 mg, 187.79 µmol) in toluene (2 mL), and the obtained mixture was warmed to 100°C and stirred for 48 h. The reaction mixture was spun to dryness to remove a solvent and obtain a crude product. The crude product was purified by silica gel column chromatography (petroleum ether/ethyl acetate = 1/1) to give tert-butyl (S)-2-[6-chloro-2-[(R)-2-methylmorpholine-4-carbonyl]-1,2,3,4-tetrahydroisoquinolin-8-y 1]pyrrolidine-1-carboxylate (35 mg, yield: 36.15%) as a yellow oil. ES-API: [M+H]⁺ = 464.23.

Step 3: Tert-butyl (*S*)-2-[6-chloro-2-[(2)-2-methylmorpholine-4-carbonyl]-1,2,3,4-tetrahydroisoquinolin-8-yl ]pyrrolidine-1-carboxylate (35.00 mg, 75.43 µmol), 3-methyl-5-(4,4,5,5-tetramethyl-1,3,2-dioxaborolan-2-yl)-1*H*-pyrrolo[2,3-*b*]pyridine (44.06 mg, 170.69 µmol), chloro (2-dicyclohexylphosphino-2',6'-dimethoxy-1,1'-biphenylyl)(2'-amino-1,1'-biphenyl-2-yl) palladium (II) (10.24 mg, 14.22 µmol) and potassium carbonate (39.32 mg, 0.28 mmol) were dissolved in 1,4-dioxane solution (1 mL) and water (0.1 mL), and the system was warmed to 100°C for 2 h. Upon completion of the reaction, the reaction mixture was quenched by the addition of water, and extracted with ethyl acetate. The organic phase was washed with brine, dried over anhydrous sodium sulfate, filtered, and concentrated to give a crude product. The crude product was purified by a thin layer chromatographic column (dichloromethane/methanol = 10/1) to give tert-butyl (*S*)-2-[2-[(*R*)-2-methylmorpholine-4-carbonyl]-6-(3-methyl-1*H*-pyrrolo[2,3-*b*]pyridin-5-yl )-1,2,3,4-tetrahydroisoquinolin-8-yl]pyrrolidine-1-carboxylate (40.00 mg, yield: 46.22%) as a white solid. ES-API: [M+H]⁺ = 560.32.

Step 4: The tert-butyl (*S*)-2-[2-[(*R*)-2-methylmorpholine-4-carbonyl]-6-(3-methyl-1*H*-pyrrolo[2,3-*b*]pyridin-5-yl )-1,2,3,4-tetrahydroisoquinolin-8-yl]pyrrolidine-1-carboxylate (25 mg, 44.67 µmol) was dissolved in dichloromethane (2 mL) and trifluoroacetic acid solution (2 mL) and stirred at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a thin layer chromatographic column (dichloromethane/methanol = 5/1) to give a product (6-(3-methyl-1H-pyrrolo[2,3-b]pyridin-5-yl)-8-((S)-pyrrolidin-2-yl)-3,4-dihydroisoquinoli n-2(1H)-yl)((R)-2-methylmorpholin)methanone (Z359-2, 5.00 mg, yield: 23.87%) as a white solid. ES-API: [M+H]⁺ = 460.27.

### Example 175. Synthesis of compound Z437

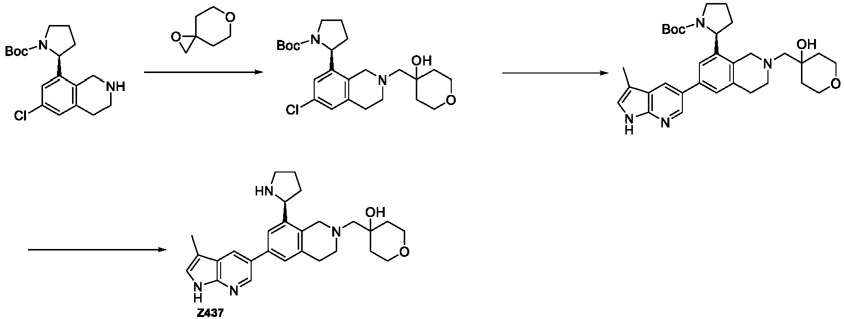

Step 1: tert-butyl (S)-2-(6-chloro-1,2,3,4-tetrahydroisoquinolin-8-yl)pyrrolidine-1-carboxylate (50 mg, 0.15 mmol) was dissolved in ethanol (0.5 mL). 1,6-Dioxaspiro[2.5]octane (34 mg, 0.30 mmol) and triethylamine (45 mg, 0.44 mmol) were added, and the system was heated to 75°C for 16 h. The reaction mixture was spun to dryness to give a crude product. The crude product was purified by a preparative thin layer chromatographic column (petroleum ether/ethyl acetate = 1/1) to give a product (87 mg, crude product). ES-API:[M+H]⁺= 451.3.

Step 2: tert-butyl (S)-2-(6-chloro-2-((4-hydroxytetrahydro-2H-pyran-4-yl)methyl)-1,2,3,4-tetrahydroisoquin olin-8-yl)pyrrolidine-1-carboxylate (87 mg, 0.19 mmol) was dissolved in dioxane/water (2 mL/0.4 mL). 3-Methyl-5-(4,4,5,5-tetramethyl-1,3,2-dioxaborolan-2-yl)-1*H*-pyrrolo[2,3-*b*]pyridine (60 mg, 0.23 mmol), chloro (2-dicyclohexylphosphino-2',6'-dimethoxy-1,1'-biphenylyl)(2'-amino-1,1'-biphenyl-2-yl) palladium (II) (14 mg, 0.019 mmol) and potassium carbonate (53 mg, 0.38 mmol) were added, and the system was heated to 100°C for 2 h. Upon completion of the reaction, the reaction mixture was Spun to dryness to give a crude product. The crude product was purified by a preparative thin layer chromatographic column (dichloromethane/methanol = 20/1) to give a product (90 mg, yield: 85%). ES-API:[M+1]⁺=547.3.

Step 3: tert-butyl (S)-2-(2-((4-hydroxytetrahydro-2H-pyran-4-yl)methyl)-6-(3-methyl-1H-pyrrolo[2,3-b]pyri din-5-yl)-1,2,3,4-tetrahydroisoquinolin-8-yl)pyrrolidine-1-carboxylate (90 mg, 0.16 mmol) was dissolved in dichloromethane (2 mL). Trifluoroacetic acid (1 mL) was added, and the system reacted at room temperature for 1 h. Upon completion of the reaction, the reaction mixture was spun to dryness, extracted with dichloromethane and saturated aqueous sodium carbonate solution, dried over anhydrous sodium sulfate, filtered, and spun to dryness to give a crude product. The crude product was purified by a preparative thin layer chromatographic column (dichloromethane/methanol = 8/1) to give a product (S)-4-((6-(3-methyl-1H-pyrrolo[2,3-b]pyridin-5-yl)-8-(pyrrol-2-yl)-3,4-dihydroisoquinolin -2(1H)-yl)methyl)tetrahydro-2H-pyran-4-ol (Z437, 25.2 mg, yield: 34%). ES-API:[M+1]⁺=447.3. ¹H NMR (400 MHz, DMSO-d6) δ 11.33-11.27 (m, 1H), 8.42 (d, *J* = 2.0 Hz, 1H), 8.05 (d, *J* = 2.0 Hz, 1H), 7.67 (d, *J* = 1.6 Hz, 1H), 7.29-7.25 (m, 1H), 7.24-7.20 (m, 1H), 4.32-4.21 (m, 1H), 4.17-4.10 (m, 1H), 3.88-3.81 (m, 1H), 3.72-3.55 (m, 6H), 3.14-3.09 (m, 1H), 2.92-2.82 (m, 4H), 2.80 -2.74 (m, 1H), 2.28 (s, 3H), 2.21-2.09 (m, 1H), 1.87-1.71 (m, 2H), 1.65-1.56 (m, 2H), 1.47-1.39 (m, 3H).

### Example 176. Synthesis of compound Z339

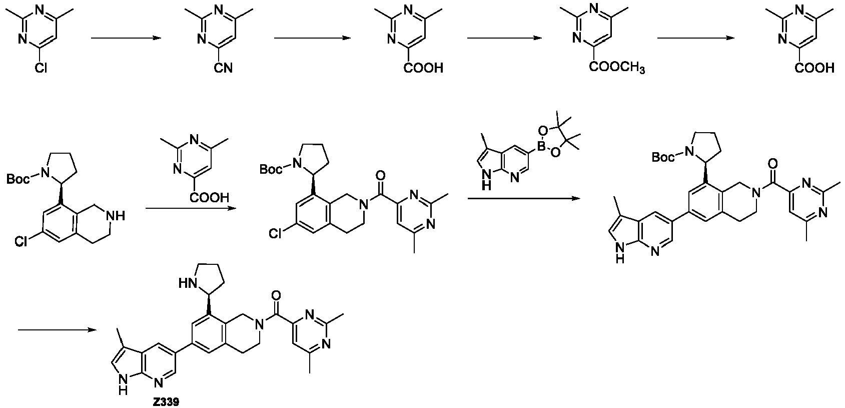

Step 1: 2-Dicyclohexylphosphine-2', 4', 6'-triisopropylbiphenyl (595.63 mg, 1.40 mmol), tris(dibenzylideneacetone)dipalladium (321.11 mg, 350.67 µmol), zinc cyanide (494.10 mg, 4.21 mmol) and zinc dust (91.17 mg, 1.40 mmol) were added to a solution of 4-chloro-2,6-dimethyl-pyrimidine (1 g, 7.01 mmol) in N,N-dimethylacetamide (2 mL), and the mixture was stirred at 25°C for 0.5 h and then at 90°C for 1 h. The reaction was monitored by LCMS for completion. The reaction mixture was treated with ethyl acetate/water, dried over anhydrous sodium sulfate, filtered, and concentrated to give a crude product. The crude product was purified by silica gel column chromatography (0-20% ethyl acetate in petroleum ether) to give a product 2,6-dimethylpyrimidine-4-carbonitrile (450.00 mg, yield: 48.19%) as a yellow solid. ES-API:[M+1]⁺=134.1.

Step 2: Sodium hydroxide (1.05 g, 26.29 mmol) was added to a solution of the 2,6-dimethylpyrimidine-4-carbonitrile (350 mg, 2.63 mmol) in ethanol (4 mL)/water (2 mL), and the mixture was stirred at 90°C for 2 h. The reaction was monitored by LCMS for completion, and the reaction mixture was treated with ethyl acetate/water. Water was then removed, and the obtained mixture was freeze-dried to give a product 2,6-dimethylpyrimidine-4-carboxylic acid (400.00 mg, crude product) as a yellow solid. ES-API:[M+1]⁺=153.1.

Step 3: The 2,6-dimethylpyrimidine-4-carboxylic acid (400 mg, 2.63 mmol) in (4M) hydrochloric acid/methanol (10 mL) was stirred at 25°C for 2 h. The reaction was monitored by LCMS for completion. The reaction mixture was spun to dryness to remove a solvent, and treated with ethyl acetate/water. The organic layer was dried over anhydrous sodium sulfate and concentrated to give a crude product. The crude product was purified by silica gel column chromatography (0-40% ethyl acetate in petroleum ether) to give a product methyl 2,6-dimethylpyrimidine-4-carboxylate (280.00 mg, yield: 64.09%) as a yellow solid. ES-API:[M+1]⁺=167.1.

Step 4: The methyl 2,6-dimethylpyrimidine-4-carboxylate (280 mg, 1.68 mmol) was dissolved in tetrahydrofuran (2 mL)/methanol (1 mL)/water (2 mL). Lithium hydroxide monohydrate (353.6 mg, 8.42 mmol) was added and stirred at 25°C for 2 h. The reaction was monitored by LCMS for completion. The reaction mixture was spun to dryness to remove a solvent and treated with ethyl acetate/water. Water was then removed, and the obtained mixture was freeze-dried to give a product 2,6-dimethylpyrimidine-4-carboxylic acid (256.00 mg, crude product) as a yellow oil. The crude product was purified by prep-HPLC (ammonia water) (37 mg, yield: 14.45%), ES-API:[M+1]⁺=153.1.

Step 5: The 2,6-dimethylpyrimidine-4-carboxylic acid (36.13 mg, 0.24 mmol), 1-propylphosphonic anhydride (229 mg, 0.36 mmol, 50% in ethyl acetate) and triethylamine (36.05 mg, 0.36 mmol) were added to a solution of tert-butyl (S)-2-(6-chloro-1,2,3,4-tetrahydroisoquinolin-8-yl)pyrrolidine-1-carboxylate (40 mg, 0.12 mmol) in dichloromethane (1 mL), and the mixture was stirred at 25°C for 2 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (mobile phase 0-50% ethyl acetate/petroleum ether, ethyl acetate/petroleum ether = 1/1, Rf = 0.3) to give a product tert-butyl (S)-2-(6-chloro-2-(2,6-dimethylpyrimidine-4-carbonyl)-1,2,3,4-tetrahydroisoquinolin-8-yl )pyrrolidine-1-carboxylate (34 mg, yield: 60.79%). ES-API:[M+H-100]⁺= 471.3.

Step 6: 3-Methyl-5-(4,4,5,5-tetramethyl-1,3,2-dioxaborolan-2-yl)-1*H*-pyrrolo[2,3-*b*]pyridine (22.36 mg, 0.087 mmol), chloro (2-dicyclohexylphosphino-2',6'-dimethoxy-1,1'-biphenylyl)(2'-amino-1,1'-biphenyl-2-yl) palladium (II) (5.19 mg, 0.007 mmmol) and potassium carbonate (29.89 mg, 0.22 mmmol) were added to a solution of the compound tert-butyl (S)-2-(6-chloro-2-(2,6-dimethylpyrimidine-4-carbonyl)-1,2,3,4-tetrahydroisoquinolin-8-yl )pyrrolidine-1-carboxylate (34 mg, 0.072 mmol) in dioxane/water (2 mL/0.4 mL), and the mixture was heated to 110°C and stirred in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by silica gel column chromatography (mobile phase 0-10% methanol/dichloromethane, dichloromethane/methanol = 10/1, Rf = 0.6) to give a product tert-butyl (S)-2-(2-(2,6-dimethylpyrimidine-4-carbonyl)-6-(3-methyl-1*H*-pyrrolo[2,3-*b*]pyridin-5-yl) -1,2,3,4-tetrahydroisoquinolin-8-yl)pyrrolidine-1-carboxylate (33 mg, yield: 80.67%). ES-API:[M+H]⁺= 567.4.

Step 7: Trifluoroacetic acid (1 ml) was added to a solution of the compound tert-butyl (S)-2-(2-(2,6-dimethylpyrimidine-4-carbonyl)-6-(3-methyl-1H-pyrrolo[2,3-b]pyridin-5-yl) -1,2,3,4-tetrahydroisoquinolin-8-yl)pyrrolidine-1-carboxylate (33 mg, 0.06 mmol) in dichloromethane (2 ml) and stirred at room temperature for 1 h. The reaction was monitored by LCMS for completion. The reaction mixture was adjusted to pH 7 with saturated sodium bicarbonate, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 mL/min, gradient: B/A = 20%-90% over 50 min, wavelength: 214 nm, column temperature: room temperature) to give (*S*)-(2,6-dimethylpyrimidin-4-yl)(6-(3-methyl-1*H*-pyrrolo[2,3-*b*]pyridin-5-yl)-8-(pyrrolidi n-2-yl)-3,4-dihydroisoquinolin-2(1*H*)-yl)methanone (Z339, 15.1 mg, 55.58%) as a white powder. ES-API:[M+H]⁺=467.3. ¹H NMR (400 MHz, CDCl₃) δ 8.55-8.50 (m, 1H), 8.01-7.74 (m, 2H), 7.25-7.24 (m, 1H), 7.16-7.11 (m, 1H), 7.05 (s, 1H), 5.11-4.83 (m, 2H), 4.74-4.01 (m, 1H), 3.86-3.08 (m, 4H), 3.07-2.80 (m, 2H), 2.76-2.72 (m, 3H), 2.59-2.54 (m, 3H), 2.31 (s, 3H), 2.25-1.93 (m, 4H).

### Example 177. Synthesis of compound Z368

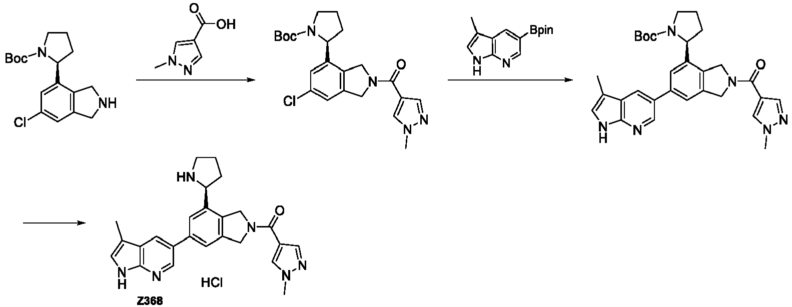

Step 1: 1-Methyl-1*H*-pyrazole-5-carboxylic acid (47 mg, 0.37 mmol) was dissolved in N,N-dimethylformamide (1 mL). N,N-Diisopropylethyl amine (0.2 mL) and 2-(7-azobenzotriazole)-N,N,N',N'-tetramethylurea hexafluorophosphate (353 mg, 0.93 mmol) were then added and stirred for 1 min. tert-butyl (S)-2-(6-chloroisoindolin-4-yl)pyrrolidine-1-carboxylate (0.1 g, 0.31 mol) was added and stirred at room temperature for 12 h. Upon completion of the reaction, the reaction mixture was added to ice water (5 mL), and extracted with ethyl acetate (5 mL X 3). The organic phase was dried over anhydrous sodium sulfate, filtered, and concentrated to give tert-butyl (S)-2-(6-chloro-2-(1-methyl-1*H*-pyrazole-4-carbonyl)isoindolin-4-yl)pyrrolidine-1-carbox ylate (0.12 g) as a yellow oil. ES-API: [M+H]⁺ = 431.1.

Step 2: The tert-butyl (S)-2-(6-chloro-2-(1-methyl-1*H*-pyrazole-4-carbonyl)isoindolin-4-yl)pyrrolidine-1-carbox ylate (0.12 g, 0.23 mmol) was dissolved in dioxane (1 mL). 3-Methyl-5-(4,4,5,5-tetramethyl-dioxaborolan-2-yl)-1*H*-pyrrolo[2,3-*b*]pyridine (72 mg, 0.28 mmol) was added, followed by water (0.1 mL) and potassium carbonate (64 mg, 0.46 mmol). Chloro (2-dicyclohexylphosphino-2',6'-dimethoxy-1,1'-biphenylyl)(2'-amino-1,1'-biphenyl-2-yl) palladium (II) (17 mg, 0.02 mmol) was finally added, and the system was heated and stirred for 12 h. Upon completion of the reaction, the reaction mixture was added to ice water (5 mL), and extracted with ethyl acetate (5 mL X 3). The organic phase was dried over anhydrous sodium sulfate, filtered and concentrated to give tert-butyl (S)-2-(2-(1-methyl-1*H*-pyrazole-4-carbonyl)-6-(3-methyl-1*H*-pyrrolo[2,3-b]pyridin-5-yl)is oindolin-4-yl)pyrrolidine-1-carboxylate (0.09 g) as yellow oil. ES-API: [M+H]⁺ = 527.3.

Step 3: The tert-butyl (S)-2-(2-(1-methyl-1*H*-pyrazole-4-carbonyl)-6-(3-methyl-1*H*-pyrrolo[2,3-b]pyridin-5-yl)is oindolin-4-yl)pyrrolidine-1-carboxylate (0.1 g) was dissolved in ethyl acetate (1 mL). Hydrochloric acid-ethyl acetate (4.0 mol/L, 1.0 mL) was added and stirred at room temperature for 12 h. Upon completion of the reaction, the reaction mixture was concentrated to give a crude product. The crude product was purified by prep-HPLC (hydrochloric acid) to give (S)-(1-methyl-1*H*-pyrazol-4-yl)(6-(3-methyl-1*H*-pyrrolo[2,3-*b*]pyridin-5-yl)-4-(pyrrolidin-2-yl)isoindolin-2-yl)methanone hydrochloride (Z368, 25.76 mg, yield: 31.8%) as a white solid. ES-API: [M+H]⁺ = 427.3. ¹H NMR (400 MHz, CD₃OD) δ 8.46 - 8.50 (m, 1H), 8.29-8.22 (m, 2H), 8.04-8.03(m, 1H), 7.72-7.71 (m, 1H),7.61 - 7.58 (m, 1H), 7.19 (s, 1H), 5.28 - 5.21 (m, 2H), 5.05 - 5.02 (m, 2H), 4.27 - 4.23 (m, 1H), 4.00 - 3.98 (m, 3H), 3.29 - 3.27 (m, 1H), 3.24 - 3.05 (m, 1H), 2.37 (s, 3H), 2.35-2.33 (m, 1H), 2.04-1.98 (m, 3H).

### Example 178. Synthesis of compound Z388

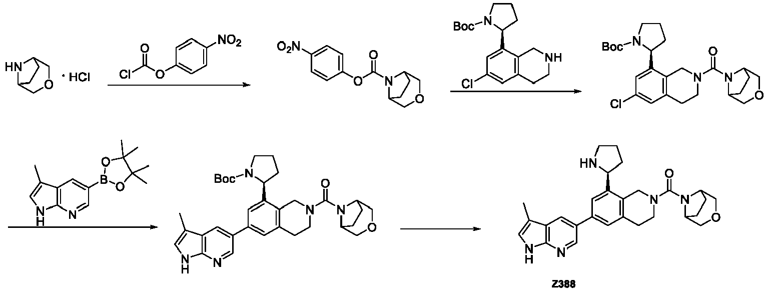

Step 1: In the presence of protective nitrogen, triethylamine (0.55 g, 5.42 mmol) and (4-nitrophenyl)carbonyl chloride (0.33 g, 1.63 mmol) were added to a solution of 3-oxa-8-azabicyclo[3.2.1]octane (0.2 g, 1.35 mmol) in anhydrous dichloromethane (10 mL) at 0°C, and the mixture was warmed to 20°C and stirred for 3 h. The reaction was monitored by LCMS for completion, and the reaction mixture was concentrated to give a crude product. The crude product was purified by silica gel column chromatography (petroleum ether/ethyl acetate = 10:90) to give (4-nitrophenyl) 3-oxa-8-azabicyclo[3.2.1]octane-8-carboxylate (0.3 g, yield: 75.59%) as a yellow solid. ES-API: [M+1]⁺=279.1.

Step 2: The(4-nitrophenyl) 3-oxa-8-azabicyclo[3.2.1]octane-8-carboxylate (86.74 mg, 0.31 mmol) and potassium carbonate (57.44 mg, 0.42 mmol) were added to a solution of tert-butyl (S)-2-(6-chloro-1,2,3,4-tetrahydroisoquinolin-8-yl)pyrrolidine-1-carboxylate (70 mg, 0.21 mmol) in N,N-dimethylacetamide (1 mL), and the mixture was warmed to 150°C and stirred for 3 h. The reaction was monitored by LCMS for completion. The reaction mixture was diluted with water, and extracted with ethyl acetate. The combined organic phase was washed with saturated brine, dried over anhydrous sodium sulfate, filtered, and concentrated to give a crude product. The crude product was purified by a preparative thin layer chromatographic column (petroleum ether:ethyl acetate = 1:1) to give tert-butyl (*S*)-2-[6-chloro-2-(3-oxa-8-azabicyclo[3.2.1]octane-8-carbonyl)-1,2,3,4-tetrahydroisoquin olin-8-yl]pyrrolidine-1-carboxylate (70.00 mg, yield: 70.77%) as a yellow oil. ES-API:[M+1]⁺=476.3.

Step 3: 3-Methyl-5-(4,4,5,5-tetramethyl-1,3,2-dioxaborolan-2-yl)-1*H*-pyrrolo[2,3-*b*]pyridine (45.55 mg, 0.18 mmol), chloro (2-dicyclohexylphosphino-2',6'-dimethoxy-1,1'-biphenylyl)(2'-amino-1,1'-biphenyl-2-yl) palladium (II) (10.6 mg, 14.71 µmol) and potassium carbonate (60.88 mg, 0.44 mmol) were added to a solution of the tert-butyl (*S*)-2-[6-chloro-2-(3-oxa-8-azabicyclo[3.2.1]octane-8-carbonyl)-1,2,3,4-tetrahydroisoquin olin-8-yl]pyrrolidine-1-carboxylate (70 mg, 0.15 mmol) in dioxane/water (4 mL/0.8 mL), and the system was heated to 100°C for 1 h. Upon completion of the reaction, the reaction mixture was spun to dryness to give a crude product. The crude product was purified by a preparative thin layer chromatographic column (dichloromethane/methanol = 10/1) to give a product tert-butyl (*S*)-2-[6-(3-methyl-1*H*-pyrrolo[2,3-b]pyridin-5-yl)-2-(3-oxa-8-azabicyclo[3.2.1]octane-8-c arbonyl)-1,2,3,4-tetrahydroisoquinolin-8-yl]pyrrolidine-1-carboxylate (70.00 mg, yield: 83.26%) as yellow oil. ES-API:[M+1]⁺=572.4.

Step 4: The tert-butyl (*S*)-2-[6-(3-methyl-1*H*-pyrrolo[2,3-b]pyridin-5-yl)-2-(3-oxa-8-azabicyclo[3.2.1]octane-8-c arbonyl)-1,2,3,4-tetrahydroisoquinolin-8-yl]pyrrolidine-1-carboxylate (70 mg, 0.12 mmol) was dissolved in dichloromethane (2.0 mL). Trifluoroacetic acid (2.0 mL) was added, and the system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 8/1) to give a product (3-oxa-8-azabicyclo[3.2.1]octan-8-yl)(6-(3-methyl-1H-pyrrolo[2,3-b]pyridin-5-yl)-8-((S)-pyrrolidin-2-yl)-3,4-dihydroisoquinolin-2(1H)-yl)methanone (Z388, 16.30 mg, yield: 28.23%) as a white solid. ES-API:[M+1]⁺=472.4¹H NMR (400 MHz, CDCl₃) *δ* 10.28 (s, 1H), 8.48 (d, *J*=2.0 Hz, 1H), 8.01 (d, J=2.0 Hz, 1H), 7.72 (d, J=1.4 Hz, 1H), 7.27 (s,1H), 7.11 (s, 1H), 4.72 (d, J=16.4 Hz, 1H), 4.60 (d, J=16.4 Hz, 1H), 4.46-4.32 (m, 1H), 3.95 (s, 2H), 3.81 (d, J = 10.7 Hz, 2H), 3.71-3.54 (m, 4H), 3.38-3.27 (m, 1H), 3.14-2.94 (m, 4H), 2.34 (s, 3H), 2.31-2.25 (m,1H), 2.05-1.85 (m, 7H), 1.81-1.64 (m, 1H).

### Example 179. Synthesis of compound Z389

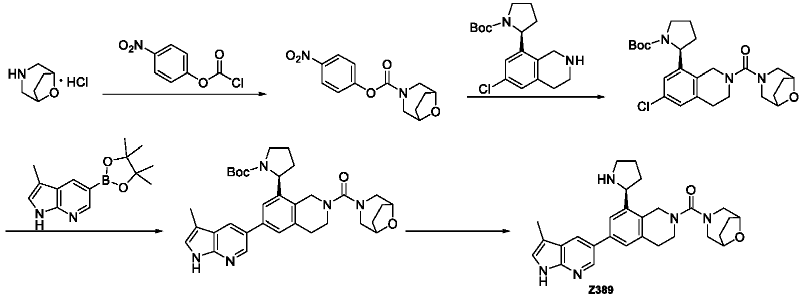

Step 1: In the presence of protective nitrogen, triethylamine (0.55 g, 5.42 mmol) and (4-nitrophenyl)carbonyl chloride (0.33 g, 1.63 mmol) were added to a solution of 8-oxa-3-azabicyclo[3.2.1]octane hydrochloride (0.2 g, 1.35 mmol) in anhydrous dichloromethane (6 mL) at 0°C, and the mixture was warmed to 20°C and stirred for 3 h. The reaction was monitored by LCMS for completion, and the reaction mixture was concentrated to give a crude product. The crude product was purified by silica gel column chromatography (petroleum ether/ethyl acetate = 10:90) to give (4-nitrophenyl) 8-oxa-3-azabicyclo[3.2.1]octane-3-carboxylate (0.3 g, yield: 79.57%) as a yellow solid. ES-API:[M+1]⁺=279.1.

Step 2: The (4-nitrophenyl) 8-oxa-3-azabicyclo[3.2.1]octane-3-carboxylate (86.74 mg, 0.31 mmol) and potassium carbonate (57.44 mg, 0.42 mmol) were added to a solution of tert-butyl (S)-2-(6-chloro-1,2,3,4-tetrahydroisoquinolin-8-yl)pyrrolidine-1-carboxylate (70 mg, 0.21 mmol) in N,N-dimethylacetamide (1 mL), and the mixture was warmed to 150°C and stirred for 3 h. The reaction was monitored by LCMS for completion. The reaction mixture was diluted with water and extracted with ethyl acetate. The combined organic phase was washed with saturated brine, dried over anhydrous sodium sulfate, filtered, and concentrated to give a crude product. The crude product was purified by a preparative thin layer chromatographic column (petroleum ether:ethyl acetate = 1:1) to give tert-butyl (2S)-2-[6-chloro-2-(8-oxa-3-azabicyclo[3.2.1]octane-3-carbonyl)-1,2,3,4-tetrahydroisoqui nolin-8-yl]pyrrolidine-1-carboxylate (70.00 mg, yield: 70.77%) as a yellow oil. ES-API:[M+1]⁺=476.3.

Step 3: 3-Methyl-5-(4,4,5,5-tetramethyl-1,3,2-dioxaborolan-2-yl)-1*H*-pyrrolo[2,3-*b*]pyridine (45.55 mg, 0.18 mmol), chloro (2-dicyclohexylphosphino-2',6'-dimethoxy-1,1'-biphenylyl)(2'-amino-1,1'-biphenyl-2-yl) palladium (II) (10.6 mg, 14.71 µmol) and potassium carbonate (60.88 mg, 0.44 mmol) were added to a solution of the tert-butyl (2S)-2-[6-chloro-2-(8-oxa-3-azabicyclo[3.2.1]octane-3-carbonyl)-1,2,3,4-tetrahydroisoqui nolin-8-yl]pyrrolidine-1-carboxylate (70 mg, 0.15 mmol) in dioxane/water (4 mL/0.8 mL), and the system was heated to 100°C for 1 h. Upon completion of the reaction, the reaction mixture was spun to dryness to give a crude product. The crude product was purified by a preparative thin layer chromatographic column (dichloromethane/methanol = 10/1) to give a product tert-butyl (*S*)-2-[6-(3-methyl-1*H*-pyrrolo[2,3-*b*]pyridin-5-yl)-2-(8-oxa-3-azabicyclo[3.2.1]octane-3-c arbonyl)-1,2,3,4-tetrahydroisoquinolin-8-yl]pyrrolidine-1-carboxylate (70.00 mg, yield: 83.26%) as yellow oil. ES-API:[M+1]⁺=572.5.

Step 4: The tert-butyl (*S*)-2-[6-(3-methyl-1*H*-pyrrolo[2,3-*b*]pyridin-5-yl)-2-(8-oxa-3-azabicyclo[3.2.1]octane-3-c arbonyl)-1,2,3,4-tetrahydroisoquinolin-8-yl]pyrrolidine-1-carboxylate (70 mg, 0.12 mmol) was dissolved in dichloromethane (2.0 mL). Trifluoroacetic acid (2.0 mL) was added. The system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 8/1) to give a product (8-oxa-3-azabicyclo[3.2.1]octan-3-yl)(6-(3-methyl-1H-pyrrolo[2,3-b]pyridin-5-yl)-8-((S)-pyrrolidin-2-yl)-3,4-dihydroisoquinolin-2(1H)-yl)methanone (Z389, 27.10 mg, yield: 46.93%) as a white solid. ES-API:[M+1]⁺=472.4. ¹H NMR (400 MHz, CDCl₃) *δ*10.77 (s, 1H), 8.42 (s, 1H), 7.78 (s, 2H), 7.00 (s, 2H), 4.93-4.86 (m, 1H), 4.53-4.25 (m, 4H), 3.79-3.72 (m, 1H), 3.63-3.37 (m, 4H), 3.32-3.20 (m, 2H), 3.12-3.04 (m, 1H), 2.92-2.76 (m, 1H), 2.64-2.55 (m, 1H), 2.50-2.29 (m, 3H), 2.24 (s, 3H), 2.22-2.10 (m, 1H), 1.99-1.75 (m, 4H).

### Example 180. Synthesis of compound Z357-1 and compound Z357-2

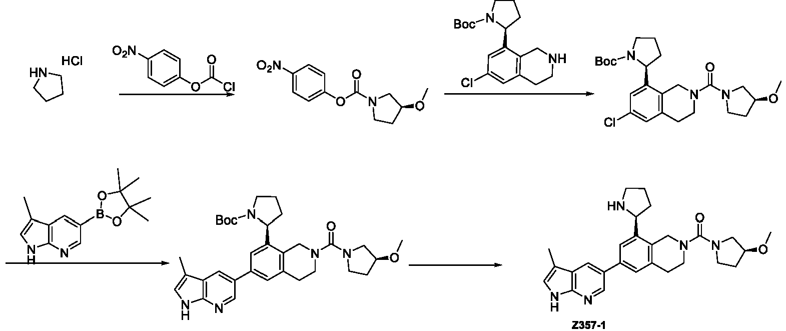

Step 1: In the presence of protective nitrogen, triethylamine (0.4 g, 3.64 mmol) and (4-nitrophenyl)carbonyl chloride (0.48 g, 2.18 mmol) were added to a solution of (S)-3-methoxypyrrolidine hydrochloride (0.25 g, 1.82 mmol) in anhydrous dichloromethane (10 mL) at 0°C, and the mixture was warmed to 20°C and stirred for 3 h. The reaction was monitored by LCMS for completion, and the reaction mixture was concentrated to give a crude product. The crude product was purified by silica gel column chromatography (petroleum ether/ethyl acetate = 10:90) to give (4-nitrophenyl)(S)-3-methoxypyrrolidine-1-carboxylate (0.4 g, yield: 82.59%) as a yellow solid. ES-API:[M+1]⁺=267.0.

Step 2: The (4-nitrophenyl)(S)-3-methoxypyrrolidine-1-carboxylate (82.99 mg, 0.31 mmol) and potassium carbonate (57.44 mg, 0.42 mmol) were added to a solution of tert-butyl (S)-2-(6-chloro-1,2,3,4-tetrahydroisoquinolin-8-yl)pyrrolidine-1-carboxylate (70 mg, 0.21 mmol) in N,N-dimethylacetamide (1 mL), and the mixture was warmed to 150°C and stirred for 3 h. The reaction was monitored by LCMS for completion. The reaction mixture was diluted with water and extracted with ethyl acetate. The combined organic phase was washed with saturated brine, dried over anhydrous sodium sulfate, filtered, and concentrated to give a crude product. The crude product was purified by a preparative thin layer chromatographic column (petroleum ether:ethyl acetate = 1:1) to give tert-butyl (S)-2-(6-chloro-2-((S)-3-methoxypyrrolidine-1-carbonyl)-1,2,3,4-tetrahydroisoquinolin-8-yl)pyrrolidine-1-carboxylate (70.00 mg, yield: 72.6%) as a yellow oil. ES-API:[M+1]⁺=464.3.

Step 3: 3-Methyl-5-(4,4,5,5-tetramethyl-1,3,2-dioxaborolan-2-yl)-1*H*-pyrrolo[2,3-*b*]pyridine (46.73 mg, 0.18 mmol), chloro (2-dicyclohexylphosphino-2',6'-dimethoxy-1,1'-biphenylyl)(2'-amino-1,1'-biphenyl-2-yl) palladium (II) (10.9 mg, 15.09 µmol) and potassium carbonate (62.46 mg, 0.45 mmol) were added to a solution of the tert-butyl (S)-2-(6-chloro-2-((*S*)-3-methoxypyrrolidine-1-carbonyl)-1,2,3,4-tetrahydroisoquinolin-8-yl)pyrrolidine-1-carboxylate (70 mg, 0.15 mmol) in dioxane/water (4 mL/0.8 mL), and the system was heated to 100°C for 1 h. Upon completion of the reaction, the reaction mixture was spun to dryness to give a crude product. The crude product was purified by a preparative thin layer chromatographic column (dichloromethane/methanol = 10/1) to give a product tert-butyl (*S*)-2-[2-[(*S*)-3-methoxypyrrolidine-1-carbonyl]-6-(3-methyl-1*H*-pyrrolo[2,3-*b*]pyridin-5-y l)-1,2,3,4-tetrahydroisoquinolin-8-yl]pyrrolidine-1-carboxylate (45.00 mg, yield: 53.29%) as a yellow oil. ES-API:[M+1]⁺=560.5.

Step 4: The tert-butyl (*S*)-2-[2-[(*S*)-3-methoxypyrrolidine-1-carbonyl]-6-(3-methyl-1*H*-pyrrolo[2,3-*b*]pyridin-5-y l)-1,2,3,4-tetrahydroisoquinolin-8-yl]pyrrolidine-1-carboxylate (45 mg, 80.40 µmol) was dissolved in dichloromethane (1.0 mL). Trifluoroacetic acid (1.0 mL) was added, and the system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 8/1) to give a product ((S)-3-methoxypyrrol-1-yl)(6-(3-methyl-1H-pyrrolo[2,3-b]pyridin-5-yl)-8-((S)-pyrrolidin-2-yl)-3,4-dihydroisoquinolin-2(1H)-yl)methanone (Z357-1, 10.30 mg, yield: 27.88%) as a white solid. ES-API: [M+1]+=460.3

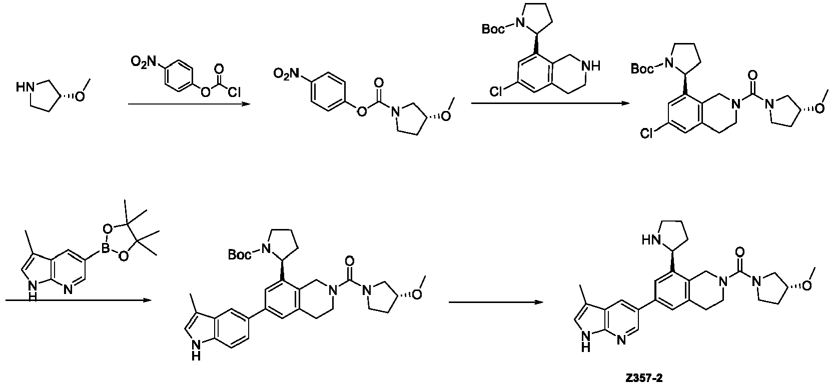

Step 1: In the presence of protective nitrogen, triethylamine (0.4 g, 3.95 mmol) and (4-nitrophenyl)carbonyl chloride (0.48 g, 2.37 mmol) were added to a solution of (R)-3-methoxypyrrolidine (0.2 g, 1.98 mmol) in anhydrous dichloromethane (10 mL) at 0°C. The mixture was warmed to 20°C and stirred for 3 h. The reaction was monitored by LCMS for completion, and the reaction mixture was concentrated to give a crude product. The crude product was purified by silica gel column chromatography (petroleum ether/ethyl acetate = 10:90) to give (R)-4-nitrophenyl 3-methoxypyrrolidine-1-carboxylate (0.4 g, yield: 75.98%) as a yellow solid. ES-API:[M+1]⁺=267.0.

Step 2: The (R)-4-nitrophenyl 3-methoxypyrrolidine-1-carboxylate (82.99 mg, 0.31 mmol) and potassium carbonate (57.44 mg, 0.42 mmol) were added to a solution of tert-butyl (S)-2-(6-chloro-1,2,3,4-tetrahydroisoquinolin-8-yl)pyrrolidine-1-carboxylate (70 mg, 0.21 mmol) in N,N-dimethylacetamide (1 mL), and the mixture was warmed to 150°C and stirred for 3 h. The reaction was monitored by LCMS for completion. The reaction mixture was diluted with water and extracted with ethyl acetate. The combined organic phase was washed with saturated brine, dried over anhydrous sodium sulfate, filtered, and concentrated to give a crude product. The crude product was purified by a preparative thin layer chromatographic column (petroleum ether:ethyl acetate = 1:1) to give tert-butyl (S)-2-(6-chloro-2-((R)-3-methoxypyrrolidine-1-carbonyl)-1,2,3,4-tetrahydroisoquinolin-8-yl)pyrrolidine-1-carboxylate (70.00 mg, yield: 72.6%) as a yellow oil. ES-API:[M+1]⁺=464.4.

Step 3: 3-Methyl-5-(4,4,5,5-tetramethyl-1,3,2-dioxaborolan-2-yl)-1*H*-pyrrolo[2,3-*b*]pyridine (46.73 mg, 0.18 mmol), chloro (2-dicyclohexylphosphino-2',6'-dimethoxy-1,1'-biphenylyl)(2'-amino-1,1'-biphenyl-2-yl) palladium (II) (10.86 mg, 15.09 µmol) and potassium carbonate (62.46 mg, 0.45 mmol) were added to a solution of the tert-butyl (S)-2-(6-chloro-2-((R)-3-methoxypyrrolidine-1-carbonyl)-1,2,3,4-tetrahydroisoquinolin-8-yl)pyrrolidine-1-carboxylate (70 mg, 0.15 mmol) in dioxane/water (2 mL/0.4 mL), and the system was heated to 100°C for 2 h. Upon completion of the reaction, the reaction mixture was spun to dryness to give a crude product. The crude product was purified by a preparative thin layer chromatographic column (dichloromethane/methanol = 10/1) to give a product tert-butyl (S)-2-(2-((*R*)-3-methoxypyrrolidine-1-carbonyl)-6-(3-methyl-1*H*-pyrrolo[2,3-*b*]pyridin-5-yl)-1,2,3,4-tetrahydroisoquinolin-8-yl)pyrrolidine-1-carboxylate (55.00 mg, yield: 65.14%) as yellow oil. ES-API:[M+1]⁺=560.5.

Step 4: The tert-butyl (S)-2-(2-((*R*)-3-methoxypyrrolidine-1-carbonyl)-6-(3-methyl-1*H*-pyrrolo[2,3-*b*]pyridin-5-yl)-1,2,3,4-tetrahydroisoquinolin-8-yl)pyrrolidine-1-carboxylate (55 mg, 0.10 mmol) was dissolved in dichloromethane (2.0 mL). Trifluoroacetic acid (1.0 mL) was added, and the system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 8/1) to give a product ((*R*)-3-methoxypyrrolidin-1-yl)(6-(3-methyl-1*H*-pyrrolo[2,3-*b*]pyridin-5-yl)-8-((*S*)-pyrroli din-2-yl)-3,4-dihydroisoquinolin-2(1*H*)-yl)methanone (Z357-2, 13.20 mg, yield: 30.15%) as a white solid. ES-API:[M+1]⁺=460.4. ¹H NMR (400 MHz, CDCl₃) δ 9.09 (s, 1H), 8.50 (d, *J =* 2.0 Hz, 1H), 8.02 (d, *J =* 2.0 Hz, 1H), 7.73 (s, 1H), 7.28 (s, 1H),7.09 (s, 1H), 4.63 (d, *J* = 16.3 Hz, 1H), 4.51 -4.37 (m, 2H), 3.99-3.94 (m, 1H), 3.67-3.54 (m, 3H),3.53-3.42 (m, 3H), 3.34 (s, 3H), 3.32-3.25 (m, 1H), 3.15-2.92 (m, 3H), 2.35 (s, 3H), 2.34-2.24 (m, 1H), 2.07-1.85 (m, 5H), 1.77-1.66 (m, 1H).

### Example 181. Synthesis of compound Z358-2 and compound Z358-1

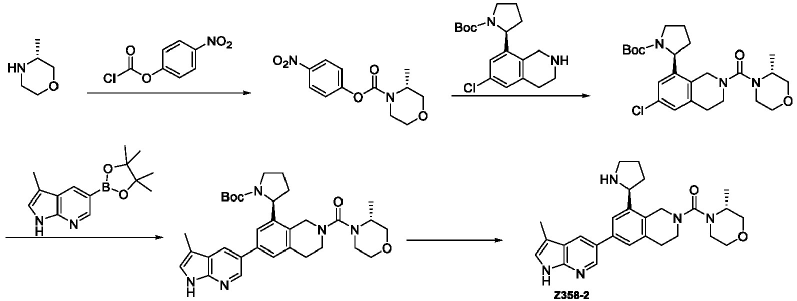

Step 1: In the presence of protective nitrogen, triethylamine (0.4 g, 3.95 mmol) and (4-nitrophenyl)carbonyl chloride (0.48 g, 2.37 mmol) were added to a solution of (R)-3-methylmorpholine (0.2 g, 1.98 mmol) in anhydrous dichloromethane (10 mL) at 0°C, and the mixture was warmed to 20°C and stirred for 3 h. The reaction was monitored by LCMS for completion, and the reaction mixture was concentrated to give a crude product. The crude product was purified by silica gel column chromatography (petroleum ether/ethyl acetate = 10:90) to give (4-nitrophenyl)(R)-3-methylmorpholine-4-carboxylate (0.47 g, yield: 89.28%) as a yellow solid. ES-API:[M+1]⁺=267.0.

Step 2: The (4-nitrophenyl)(R)-3-methylmorpholine-4-carboxylate (82.99 mg, 0.31 mmol) and potassium carbonate (57.44 mg, 0.42 mmol) were added to a solution of (tert-butyl (S)-2-(6-chloro-1,2,3,4-tetrahydroisoquinolin-8-yl)pyrrolidine-1-carboxylate (70 mg, 0.21 mmol) in N,N-dimethylacetamide (1 mL), and the mixture was warmed to 130°C and stirred for 3 h. The reaction was monitored by LCMS for completion. The reaction mixture was diluted with water and extracted with ethyl acetate. The combined organic phase was washed with saturated brine, dried over anhydrous sodium sulfate, filtered, and concentrated to give a crude product. The crude product was purified by a preparative thin layer chromatographic column (petroleum ether:ethyl acetate = 1:1) to give tert-butyl (*S*)-2-[6-chloro-2-[(*R*)-3-methylmorpholine-4-carbonyl]-1,2,3,4-tetrahydroisoquinolin-8-yl ]pyrrolidine-1-carboxylate (70.00 mg, yield: 72.6%) as a yellow oil. ES-API:[M+1]⁺=464.4.

Step 3: 3-Methyl-5-(4,4,5,5-tetramethyl-1,3,2-dioxaborolan-2-yl)-1*H*-pyrrolo[2,3-*b*]pyridine (46.73 mg, 0.18 mmol), chloro (2-dicyclohexylphosphino-2',6'-dimethoxy-1,1'-biphenylyl)(2'-amino-1,1'-biphenyl-2-yl) palladium (II) (10.86 mg, 15.09 µmol) and potassium carbonate (62.46 mg, 0.45 mmol) were added to a solution of the tert-butyl (*S*)-2-[6-chloro-2-[(*R*)-3-methylmorpholine-4-carbonyl]-1,2,3,4-tetrahydroisoquinolin-8-yl ]pyrrolidine-1-carboxylate (70 mg, 0.15 mmol) in dioxane/water (2 mL/0.4 mL), and the system was heated to 100°C for 2 h. Upon completion of the reaction, the reaction mixture was spun to dryness to give a crude product. The crude product was purified by a preparative thin layer chromatographic column (dichloromethane/methanol = 10/1) to give a product tert-butyl (*S*)-2-[2-[(*R*)-3-methylmorpholine-4-carbonyl]-6-(3-methyl-1*H*-pyrrolo[2,3-*b*]pyridin-5-yl )-1,2,3,4-tetrahydroisoquinolin-8-yl]pyrrolidine-1-carboxylate (60.00 mg, yield: 71.06%) as yellow oil. ES-API:[M+1]⁺=560.5.

Step 4: The tert-butyl (*S*)-2-[2-[(*R*)-3-methylmorpholine-4-carbonyl]-6-(3-methyl-1*H*-pyrrolo[2,3-*b*]pyridin-5-yl )-1,2,3,4-tetrahydroisoquinolin-8-yl]pyrrolidine-1-carboxylate (60 mg, 0.11 mmol) was dissolved in dichloromethane (2.0 mL). Trifluoroacetic acid (1.0 mL) was added, and the system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 8/1) to give a product (6-(3-methyl-1H-pyrrolo[2,3-b]pyridin-5-yl)-8-((S)-pyrrolidin-2-yl)-3,4-dihydroisoquinoli n-2(1H)-yl)((R)-3-methylmorpholin)methanone (Z358-2, 18.9 mg, yield: 38.36%) as a white solid. ES-API:[M+1]⁺=460.4. ¹H NMR (400 MHz, CDCl₃) δ 9.55 (s, 1H), 8.48 (d, *J* = 2.0 Hz, 1H), 8.01 (d, *J=* 2.0 Hz, 1H), 7.72 (s, 1H), 7.28 (s, 1H), 7.10 (s, 1H), 4.67-4.43 (m, 2H), 4.44-4.33 (m, 1H), 3.87-3.78 (m, 2H), 3.73-3.67 (m, 1H),3.67-3.52 (m, 3H), 3.49-3.39 (m, 1H), 3.39-3.26 (m, 3H), 3.13-2.94 (m, 3H), 2.35 (s, 3H), 2.33-2.24 (m, 1H), 2.05-1.84 (m, 2H), 1.79-1.68 (m, 1H), 1.32 (d, *J=* 6.8 Hz, 3H).

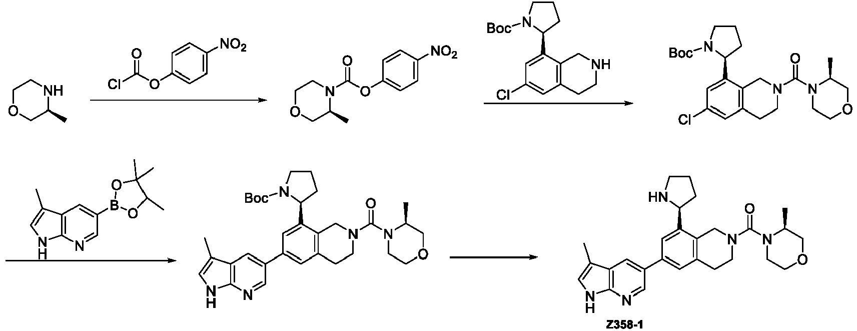

Step 1: In the presence of protective nitrogen, triethylamine (0.4 g, 3.95 mmol) and (4-nitrophenyl)carbonyl chloride (0.48 g, 2.37 mmol) were added to a solution of (S)-3-methylmorpholine (0.2 g, 1.98 mmol) in anhydrous dichloromethane (10 mL) at 0°C, and the mixture was warmed to 20°C and stirred for 3 h. The reaction was monitored by LCMS for completion, and the reaction mixture was concentrated to give a crude product. The crude product was purified by silica gel column chromatography (petroleum ether/ethyl acetate = 10:90) to give (4-nitrophenyl)(S)-3-methylmorpholine-4-carboxylate (0.45 g, yield: 85.48%) as a yellow solid. ES-API:[M+1]⁺=267.0.

Step 2: The (4-nitrophenyl)(S)-3-methylmorpholine-4-carboxylate (82.99 mg, 0.31 mmol) and potassium carbonate (57.44 mg, 0.42 mmol) were added to a solution of tert-butyl (S)-2-(6-chloro-1,2,3,4-tetrahydroisoquinolin-8-yl)pyrrolidine-1-carboxylate (70 mg, 0.21 mmol) in N,N-dimethylacetamide (1 mL), and the mixture was warmed to 150°C and stirred for 3 h. The reaction was monitored by LCMS for completion. The reaction mixture was diluted with water and extracted with ethyl acetate. The combined organic phase was washed with saturated brine, dried over anhydrous sodium sulfate, filtered, and concentrated to give a crude product. The crude product was purified by a preparative thin layer chromatographic column (petroleum ether:ethyl acetate = 1:1) to give tert-butyl (*S*)-2-[6-chloro-2-[(*S*)-3-methylmorpholine-4-carbonyl]-1,2,3,4-tetrahydroisoquinolin-8-yl ]pyrrolidine-1-carboxylate (60.00 mg, yield: 62.23%) as a yellow oil. ES-API:[M+1]⁺=464.4.

Step 3: 3-Methyl-5-(4,4,5,5-tetramethyl-1,3,2-dioxaborolan-2-yl)-1*H*-pyrrolo[2,3-*b*]pyridine (40.05 mg, 0.16 mmol), chloro (2-dicyclohexylphosphino-2',6'-dimethoxy-1,1'-biphenylyl)(2'-amino-1,1'-biphenyl-2-yl) palladium (II) (9.31 mg, 12.93 µmol) and potassium carbonate (53.53 mg, 0.39 mmol) were added to a solution of the tert-butyl (*S*)-2-[6-chloro-2-[(*S*)-3-methylmorpholine-4-carbonyl]-1,2,3,4-tetrahydroisoquinolin-8-yl ]pyrrolidine-1-carboxylate (60 mg, 0.13 mmol) in dioxane/water (4 mL/0.8 mL), and the system was heated to 100°C for 1 h. Upon completion of the reaction, the reaction mixture was spun to dryness to give a crude product. The crude product was purified by a preparative thin layer chromatographic column (dichloromethane/methanol = 10/1) to give a product tert-butyl (*S*)-2-[2-[(*S*)-3-methylmorpholine-4-carbonyl]-6-(3-methyl-1*H*-pyrrolo[2,3-*b*]pyridin-5-yl )-1,2,3,4-tetrahydroisoquinolin-8-yl]pyrrolidine-1-carboxylate (60.00 mg, yield: 82.90%) as a yellow oil. ES-API:[M+1]⁺=560.5.

Step 4: The tert-butyl (*S*)-2-[2-[(*S*)-3-methylmorpholine-4-carbonyl]-6-(3-methyl-1*H*-pyrrolo[2,3-*b*]pyridin-5-yl )-1,2,3,4-tetrahydroisoquinolin-8-yl]pyrrolidine-1-carboxylate (60 mg, 0.11 mmol) was dissolved in dichloromethane (2.0 mL). Trifluoroacetic acid (2.0 mL) was added, and the system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 8/1) to give a product (6-(3-methyl-1H-pyrrolo[2,3-b]pyridin-5-yl)-8-((S)-pyrrol-2-yl)-3,4-dihydroisoquinolin-2( 1H)-yl)((S)-3-methylmorpholin)methanone (Z358-1, 13.50 mg, yield: 27.40%) as a white solid. ES-API:[M+1]⁺=460.4. ¹H NMR (400 MHz, CDCl₃) δ 9.96 (s, 1H), 8.48 (d, J=2.1 Hz, 1H), 8.02 (d, J=2.1 Hz, 1H), 7.72 (d, J=1.7 Hz, 1H), 7.28 (d, 7=1.4 Hz, 1H),7.12 (s, 1H), 4.61 (d, *J* = 16.4 Hz, 1H), 4.49 (d, *J* = 16.4 Hz, 1H), 4.38 (t, J=7.6 Hz, 1H), 3.86-3.78 (m, 2H), 3.73-3.69 (m, 1H), 3.66 -3.48 (m, 4H), 3.41-3.26 (m, 3H), 3.13-2.98 (m, 3H), 2.35 (s, 3H), 2.32-2.25 (m, 1H), 2.04-1.86 (m, 2H), 1.78-1.67 (m,1H), 1.32 (d, J=6.8 Hz, 3H).

### Example 182. Synthesis of compound Z361

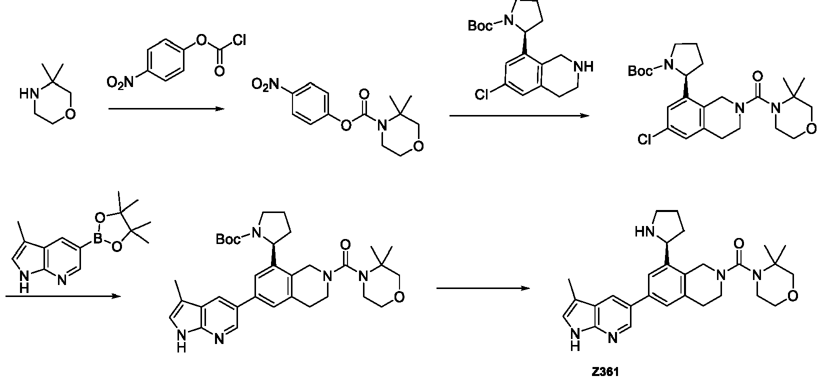

Step 1: A solution of (4-nitrophenyl)carbonyl chloride (350.02 mg, 1.74 mmol) in dichloromethane (10 mL) was cooled in an ice bath. Triethylamine (351.43 mg, 3.47 mmol) and 3,3-dimethylmorpholine (200 mg, 1.74 mmol) were then added successively, and the obtained mixture was warmed to room temperature and stirred for 3 h. The reaction mixture was spun to dryness to remove a solvent and obtain a crude product. The crude product was purified by silica gel column chromatography (petroleum ether/ethyl acetate = 5/1) to give 4-nitrophenyl 3,3-dimethylmorpholine-4-carboxylate (280 mg, yield: 56.38%). ES-API: [M+H]⁺ = 281.11.

Step 2: Diisopropylethyl amine (97.07 mg, 751.12 µmol) and tert-butyl (S)-2-(6-chloro-1,2,3,4-tetrahydroisoquinolin-8-yl)pyrrolidine-1-carboxylate (63.25 mg, 187.78 µmol) were added to a solution of (4-nitrophenyl) 3,3-dimethylmorpholine-4-carboxylate (52.63 mg, 187.78 µmol) in anhydrous N,N-dimethylformamide (2 mL), and the obtained mixture was warmed to 130°C and stirred for 5 h. The reaction mixture was spun to dryness to remove a solvent and obtain a crude product. The crude product was purified by silica gel column chromatography (petroleum ether/ethyl acetate = 1/) to give tert-butyl 2-[6-chloro-2-(3,3-dimethylmorpholine-4-carbonyl)-1,2,3,4-tetrahydroisoquinolin-8-yl]py rrolidine-1-carboxylate (33 mg, yield: 33%) as a yellow oil. ES-API: [M+H]⁺ = 478.25.

Step 3: The tert-butyl 2-[6-chloro-2-(3,3-dimethylmorpholine-4-carbonyl)-1,2,3,4-tetrahydroisoquinolin-8-yl]py rrolidine-1-carboxylate (36.06 mg, 75.43 µmol), 3-methyl-5-(4,4,5,5-tetramethyl-1,3,2-dioxaborolan-2-yl)-1*H*-pyrrolo[2,3-*b*]pyridine (44.06 mg, 170.69 µmol), chloro (2-dicyclohexylphosphino-2',6'-dimethoxy-1,1'-biphenylyl)(2'-amino-1,1'-biphenyl-2-yl) palladium (II) (10.24 mg, 14.22 µmol) and potassium carbonate (39.32 mg, 0.28 mmol) were dissolved in 1,4-dioxane solution (1 mL) and water (0.1 mL), and the system was warmed to 100°C for 2 h. Upon completion of the reaction, the reaction mixture was quenched by the addition of water and extracted with ethyl acetate. The organic phase was washed with brine, dried over anhydrous sodium sulfate, filtered, and concentrated to give a crude product. The crude product was purified by a thin layer chromatographic column (dichloromethane/methanol = 10/1) to give a product tert-butyl (*S*)-2-[2-(3,3-dimethylmorpholine-4-carbonyl)-6-(3-methyl)-1*H*-pyrrolo[2,3-*b*]pyridin-5-y l)-1,2,3,4-tetrahydroisoquinolin-8-yl]pyrrolidine-1-carboxylate (30.00 mg, yield: 34.92%). ES-API: [M+H]⁺ = 574.34.

Step 4: The tert-butyl (*S*)-2-[2-(3,3-dimethylmorpholine-4-carbonyl)-6-(3-methyl)-1*H*-pyrrolo[2,3-*b*]pyridin-5-y l)-1,2,3,4-tetrahydroisoquinolin-8-yl]pyrrolidine-1-carboxylate (30 mg, 49.68 µmol) was dissolved in dichloromethane (2 mL) and trifluoroacetic acid solution (2 mL) and stirred at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a thin layer chromatographic column (dichloromethane/methanol = 5/1) to give a product (*S*)-(3,3-dimethylmorpholino[6-(3-methyl-1*H*-pyrrolo[2,3-*b*]pyridin-5-yl)-8-[pyrrolidin-2-yl]-3,4-dihydroisoquinolin-2(1H)-yl]methanone (Z361, 8.20 mg, 16.97 µmol, yield: 38.94%) as a white solid. ES-API: [M+H]⁺ = 474.29.

### Example 183. Synthesis of compound Z343

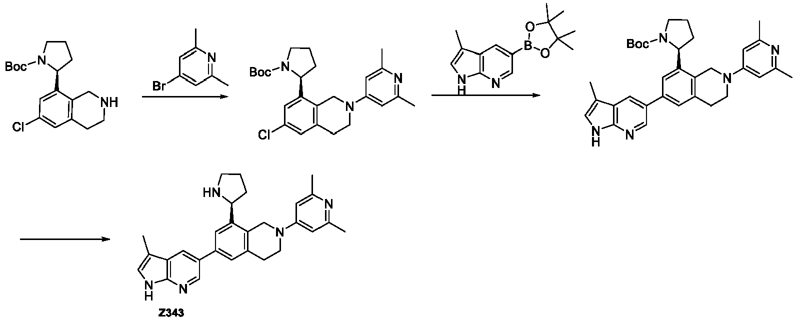

Step 1: 2-Dicyclohexylphosphino-2',6'-diisopropoxy-1,1'-biphenyl (27.12 mg, 59.37 µmol), (2-dicyclohexylphosphino-2',6'-diisopropoxy-1,1'-biphenylyl)(2-amino-1,1'-biphenyl-2-y l)palladium (II) mesylate (49.72 mg, 59.37 µmol), potassium carbonate (123.12 mg, 890.59 µmol) and 4-bromo-2,6-lutidine (165.69 mg, 890.59 µmol) were added to a solution of tert-butyl (S)-2-(6-chloro-1,2,3,4-tetrahydroisoquinolin-8-yl)pyrrolidine-1-carboxylate (100 mg, 296.86 µmol) in dioxane (1 mL), and the system reacted at 130°C for 15 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (0-5% methanol in dichloromethane) to give a product tert-butyl (S)-2-(6-chloro-2-(2,6-dimethylpyridin-4-yl)-1,2,3,4-tetrahydroisoquinolin-8-yl)pyrrolidin e-1-carboxylate (95 mg, yield: 65.2%). ES-API:[M+H]⁺=442.2.

Step 2: The compound tert-butyl (S)-2-(6-chloro-2-(2,6-dimethylpyridin-4-yl)-1,2,3,4-tetrahydroisoquinolin-8-yl)pyrrolidin e-1-carboxylate (100 mg, 226.24 µmol) was dissolved in dioxane/water (2 mL/0.4 mL). 3-Methyl-5-(4,4,5,5-tetramethyl-1,3,2-dioxaborolan-2-yl)-1*H*-pyrrolo[2,3-*b*]pyridine (69.77 mg, 270.29 mmol), chloro (2-dicyclohexylphosphino-2',6'-dimethoxy-1,1'-biphenylyl)(2'-amino-1,1'-biphenyl-2-yl) palladium (II) (16.28 mg, 22.62 µmol) and potassium carbonate (93.67 mg, 678 µmol) were added, and the system was heated to 100°C for 2 h. The reaction mixture was extracted with dichloromethane/water, dried over anhydrous sodium sulfate, filtered, and concentrated to give a crude product. The crude product was purified by a preparative thin layer chromatographic column (dichloromethane/methanol = 10/1) to give a product tert-butyl (S)-2-(2-(2,6-dimethylpyridin-4-yl)-6-(3-methyl-1*H*-pyrrolo[2,3-*b*]pyridin-5-yl)-1,2,3,4-te trahydroisoquinolin-8-yl)pyrrolidine-1-carboxylate (90 mg, yield: 66.6%). ES-API: [M+H]⁺=538.4.

Step 3: The tert-butyl (S)-2-(2-(2,6-dimethylpyridin-4-yl)-6-(3-methyl-1*H*-pyrrolo[2,3-*b*]pyridin-5-yl)-1,2,3,4-te trahydroisoquinolin-8-yl)pyrrolidine-1-carboxylate (55 mg, 102.9 µmol) was dissolved in dichloromethane (1 mL). Trifluoroacetic acid (1 mL) was added, and the system reacted at room temperature for 1 h. Upon completion of the reaction, the reaction mixture was spun to dryness, extracted with dichloromethane and saturated aqueous sodium bicarbonate solution, dried over anhydrous sodium sulfate, filtered, and spun to dryness to give a crude product. The crude product was purified by a preparative thin layer chromatographic column (dichloromethane/methanol = 10/1) to give a product (S)-2-(2,6-dimethylpyridin-4-yl)-6-(3-methyl-1H-pyrrolo[2,3-b]pyridin-5-yl)-8-(pyrrolidi n-2-yl)-1,2,3,4-tetrahydroisoquinoline (Z343, 12 mg, yield: 21.95%). (Rf = 0.4, dichloromethane/methanol = 10/1). ES-API:[M+1]⁺=438.2.

### Example 184. Synthesis of compound Z261

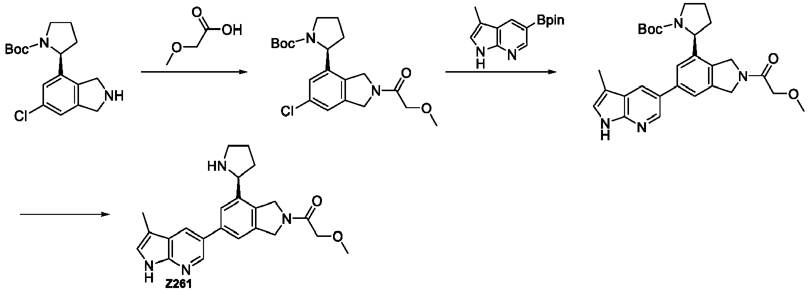

Step 1: Methoxyacetic acid (26.79 mg, 297.37 umol) was dissolved in N,N-dimethylformamide (1 mL). N,N-Diisopropylethyl amine and 2-(7-azobenzotriazole)-N,N,N',N'-tetramethylurea hexafluorophosphate (306.23 mg, 805.38 umol) were added and stirred for 1 min. tert-butyl (S)-2-(6-chloroisoindolin-4-yl)pyrrolidine-1-carboxylate (0.08 g, 247.81 umol) was then added and stirred at room temperature for 12 h. Upon completion of the reaction, the reaction mixture was added to ice water (5 mL) and extracted with ethyl acetate (5 mL X 3). The organic phase was dried over anhydrous sodium sulfate, filtered, and concentrated to give tert-butyl (S)-2-(6-chloro-2-(2-methoxyacetyl)isoindolin-4-yl)pyrrolidine-1-carboxylate (0.09 g) as a yellow oil. ES-API: [M+H]⁺ = 395.2.

Step 2: The tert-butyl (S)-2-(6-chloro-2-(2-methoxyacetyl)isoindolin-4-yl)pyrrolidine-1-carboxylate (0.09 g, 227.91 umol) was dissolved in dioxane (1 mL). 3-Methyl-5-(4,4,5,5-tetramethyl-dioxaborolan-2-yl)-1H-pyrrolo[2,3-b]pyridine (64.71 mg, 250.70 umol) was added, followed by 0.1 water (0.1 mL) and potassium carbonate (94.50 mg, 683.73 umol). Chloro (2-dicyclohexylphosphino-2',6'-dimethoxy-1,1'-biphenylyl)(2'-amino-1,1'-biphenyl-2-yl) palladium (II) (16.42 mg, 22.79 umol) was finally added, and the system was heated and stirred for 12 h. Upon completion of the reaction, the reaction mixture was added to ice water (5 mL), and then extracted with ethyl acetate (5 mL X 3). The organic phase was dried over anhydrous sodium sulfate, filtered, and concentrated to give tert-butyl tert-butyl (S)-2-(2-(2-methoxyacetyl)-6-(3-methyl-1*H*-pyrrolo[2,3-*b*]pyridin-5-yl)isoindolin-4-yl)py rrolidine-1-carboxylate (0.1 g) as a yellow oil. ES-API: [M+H]⁺ = 491.3.

Step 3: The tert-butyl tert-butyl (S)-2-(2-(2-methoxyacetyl)-6-(3-methyl-1*H*-pyrrolo[2,3-*b*]pyridin-5-yl)isoindolin-4-yl)py rrolidine-1-carboxylate (0.1 g) was dissolved in ethyl acetate (1 mL). Hydrochloric acid-ethyl acetate (4.0 M, 1.0 mL) was added and stirred at room temperature for 12 h. Upon completion of the reaction, the reaction mixture was concentrated to give a crude product. The crude product was purified by prep-HPLC (hydrochloric acid) to give (S)-2-methoxy-1-(6-(3-methyl-1H-pyrrolo[2,3-b]pyridin-5-yl)-4-(pyrrolidin-2-yl)isoindoli n-2-yl)ethan-1-one hydrochloride (Z261, 17.8 mg, yield: 22.4%) as a white solid. ES-API: [M+H]⁺ = 391.2.

### Example 185. Synthesis of compound Z262

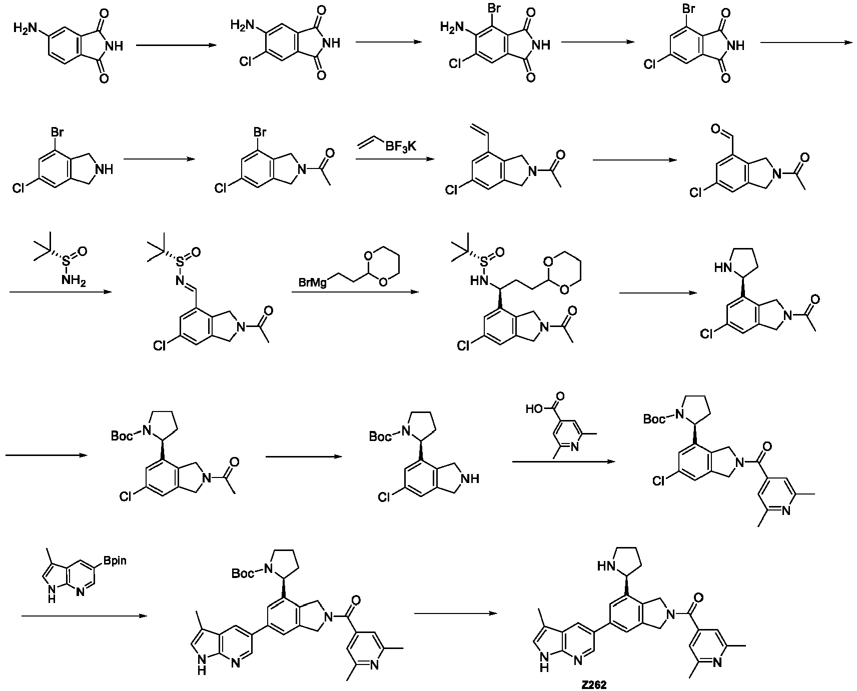

Step 1: 5-Aminoisoindoline-1,3-dione (200 g, 1.23 mmol) was dissolved in N,N-dimethylformamide (350 mL) and dioxane (1000 mL). N-Chlorosuccinimide (164.12 g, 1.23 mol) was slowly added and stirred at room temperature for 16 h. Upon completion of the reaction, the reaction mixture was slowly poured into ice water and filtered to give 5-amino-6-chloroisoindoline-1,3-dione (230 g) as a yellow solid. ES-API: [M+H⁺] = 197.1.

Step 2: The 5-amino-6-chloroisoindoline-1,3-dione (230 g, 1.17 mol) was dissolved in N,N-dimethylformamide (1200 mL). N-Bromosuccinimide (249.88 g, 1.40 mol) was slowly added and stirred at room temperature for 2 h. Upon completion of the reaction, the reaction mixture was slowly poured into ice water and filtered to give 5-amino-4-bromo-6-chloroisoindoline-1,3-dione (280 g) as a yellow solid. ES-API: [M+H⁺] = 275.0,277.0.

Step 3: The 5-amino-4-bromo-6-chloroisoindoline-1,3-dione (285 g, 1.03 mol) was added to sulfuric acid (57.47 mL) at a controlled temperature of 0-5°C. Sodium nitrite (214.13 g, 3.1 mmol) was added in batches and stirred at a controlled temperature of 0-5°C for 0.5 h. Phosphorous acid (132.38 g, 50% in water, 1.03 mol) was slowly added dropwise and stirred at 0-5°C for 2 h. Upon completion of the reaction, the reaction mixture was slowly poured into ice water and filtered to give 4-bromo-6-chloroisoindoline-1,3-dione (170 g) as a yellow solid. ES-API: [M+H⁺] = 259.9.

Step 4: In the presence of protective nitrogen, the 4-bromo-6-chloroisoindoline-1,3-dione (66 g, 253.39 mmol) was added to tetrahydrofuran (350 mL). 1M Borane in tetrahydrofuran (1.52 L) was added dropwise at 0-10°C, and the system was heated to 80°C and stirred for 36 h. Upon completion of the reaction, methanol (500 mL) was added, and the obtained mixture was concentrated to give 4-bromo-6-chloro-isoindoline (40 g). ES-API: [M+H]⁺ = 231.8,233.

Step 5: The 4-bromo-6-chloro-isoindoline (80 g, 344.08 mmol) was added to a tetrahydrofuran (560 mL) solution, and potassium carbonate (237.77 g, 1.72 mol) was added. After the system was cooled to 0°C, acetyl chloride (36.83 mL, 516.12 mmol) was slowly added and stirred at room temperature for 12 h. Upon completion of the reaction, the reaction mixture was slowly added to ice water (800 mL) and extracted with ethyl acetate (100 mLX3). The organic phase was dried over anhydrous sodium sulfate, filtered, and concentrated to give 1-(4-bromo-6-chloro-isoindoline)-2-ethanone (50 g). ES-API: [M+H]⁺ = 276.0,274.0.

Step 6: The 1-(4-bromo-6-chloro-isoindoline)-2-ethanone (50 g, 182.12 mmol) and potassium vinyltrifluoroborate (48.79 g, 364.21 mmol) were added to an ethanol (350 mL) solution. Triethylamine (25.35 mL, 182.12 mmol) was then added, and the system was replaced with nitrogen three times. [1,1 Bis(diphenylphosphino)ferrocene]dichloropalladium-dichloromethane complex (7.44 g, 9.11 mmol) was finally added, and the system was heated to 80°C for 16 h. Upon completion of the reaction, the reaction mixture was slowly poured into ice water (800 mL) and extracted with ethyl acetate (200 mLX3). The organic phase was dried over anhydrous sodium sulfate, filtered, and concentrated to give a crude product. The crude product was purified by a flash silica gel column (0-100% ethyl acetate/petroleum ether) to give 1-(6-chloro-4-vinyl-isoindoline)-2-ethanone (30 g, yield: 74%) as a yellow oil. ES-API: [M+H]⁺ = 222.1.

Step 7: The 1-(6-chloro-4-vinyl-isoindoline)-2-ethanone (30 g, 135.33 mmol) was added to tetrahydrofuran (90 mL) and water (40 mL). Sodium periodate (173.67 g, 811.97 mmol) and potassium osmate dihydrate (997.25 mg, 2.71 mmol) were added and stirred at room temperature for 12 h. Upon completion of the reaction, the reaction mixture was filtered. The obtained liquid was slowly poured into ice water (400 mL) and extracted with ethyl acetate (200 mLx3). The organic phase was dried over anhydrous sodium sulfate, filtered, concentrated, and purified by a flash silica gel column (0-100% ethyl acetate/petroleum ether) to give 2-acetyl-6-chloro-isoindoline-4-carbaldehyde (20 g, yield: 66%) as a yellow solid. ES-API: [M+H]⁺ = 224.1.

Step 8: The 2-acetyl-6-chloro-isoindoline-4-carbaldehyde (20 g, 135.33 mmol) was dissolved in dichloromethane (140 mL). S-(-)-2-Methyl-2-propanesulfinamide (13.01 g, 103.71 mmol) and tetraethyl titanate (61.19 g, 2.68 mmol) were then added and stirred at room temperature for 12 h. Upon completion of the reaction, the reaction mixture was filtered, slowly added to ice water (100 mL), and extracted with dichloromethane (500 mLX3). The organic phase was dried over anhydrous sodium sulfate, filtered, and concentrated to give (E,S)-N-[(2-acetyl-6-chloroisoindolin-4-yl)methylene]-2-methylpropane-2-sulfinamide (21 g) as a yellow solid. ES-API: [M+H]⁺ = 327.1.

Step 9: The (E,S)-N-[(2-acetyl-6-chloroisoindolin-4-yl)methylene]-2-methylpropane-2-sulfinamide (11 g, 33.66 mmol) was dissolved in tetrahydrofuran (75 mL). The system was replaced with nitrogen three times and cooled to -70°C. (1,3-Dioxane-2-ethyl)magnesium bromide (0.5 M, 134.62 mL) was added and stirred for 2 h. Upon completion of the reaction, the reaction mixture was filtered, slowly added to aqueous ammonium chloride solution (100 mL), and extracted with ethyl acetate (80 mLx3). The organic phase was dried over anhydrous sodium sulfate, filtered, and concentrated to give (S)-N-((S)-1-(2-acetyl-6-chloroisoindolin-4-yl)-3-(1,3-dioxan-2-yl)propyl)-2-methylpropa ne-2-sulfinamide (12.5 g) as a yellow solid. ES-API: [M+H]⁺ = 443.1.

Step 10: The (S)-N-((S)-1-(2-acetyl-6-chloroisoindolin-4-yl)-3-(1,3-dioxan-2-yl)propyl)-2-methylpropa ne-2-sulfinamide (12 g, 27.09 mmol) was dissolved in trifluoroacetic acid (60 mL). Water (3 mL) was added and stirred at room temperature for 0.5 h. Triethylsilane (31.50 g, 270.88 mmol) was then added and stirred at room temperature for 12 h. Upon completion of the reaction, the reaction mixture was directly concentrated to give (S)-1-(6-chloro-4-(pyrrolidin-2-yl)isoindol-2-yl)ethan-1-one (7.0 g) as a yellow oil. ES-API: [M+H]⁺ = 265.1.

Step 11: The (S)-1-(6-chloro-4-(pyrrolidin-2-yl)isoindol-2-yl)ethan-1-one (7.0 g, 27.09 mmol) was dissolved in tetrahydrofuran (14 mL). Triethylamine (11 mL) and di-tert-butyl dicarbonate (9.11 mL) was then added and stirred at room temperature for 12 h. Upon completion of the reaction, the reaction mixture was slowly added to ice water (50 mL) and extracted with ethyl acetate (30 mL × 3). The organic phase was dried over anhydrous sodium sulfate, filtered, concentrated, and purified by a flash silica gel column (0-100% ethyl acetate/petroleum ether) to give tert-butyl (S)-2-(2-acetyl-6-chloroisoindolin-4-yl)pyrrolidine-1-carboxylate (6.0 g, yield: 62%) as a yellow oil. ES-API: [M+H]⁺ = 365.0.

Step 12: The tert-butyl (S)-2-(2-acetyl-6-chloroisoindolin-4-yl)pyrrolidine-1-carboxylate (2.0 g, 5.48 mmol) was dissolved in ethanol (14 mL). 10% Sodium hydroxide (3 mL) was added, and the system was heated to 95°C and stirred for 12 h. Upon completion of the reaction, the reaction mixture was concentrated, added to ice water (20 mL), and extracted with ethyl acetate (15 mL × 3). The organic phase was dried over anhydrous sodium sulfate, filtered, concentrated, and purified by a flash silica gel column (0-100% ethyl acetate/petroleum ether) to give tert-butyl (S)-2-(6-chloroisoindolin-4-yl)pyrrolidine-1-carboxylate (1.5 g) as a yellow foamy solid. ES-API: [M+H]⁺ = 322.9.

Step 13: 2,6-Dimethylisonicotinic acid (93 mg, 0.60 mmol) was dissolved in N,N-dimethylformamide (1 mL). N,N-Diisopropylethyl amine and 2-(7-azobenzotriazol)-N,N,N',N'-tetramethylurea hexafluorophosphate (306 mg, 0.8 mmol) were added and stirred for 1 min. The tert-butyl (S)-2-(6-chloroisoindolin-4-yl)pyrrolidine-1-carboxylate (0.13 g, 0.4 mmol) was added and stirred at room temperature for 12 h. Upon completion of the reaction, the reaction mixture was added to ice water (5 mL) and extracted with ethyl acetate (5 mL × 3). The organic phase was dried over anhydrous sodium sulfate, filtered, and concentrated to give tert-butyl (S)-2-(6-chloro-2-(2,6-dimethylisonicotinoyl)isoindolin-4-yl)pyrrolidine-1-carboxylate (0.18 g) as a yellow oil. ES-API: [M+H]⁺ = 456.2.

Step 14: The tert-butyl (S)-2-(6-chloro-2-(2,6-dimethylisonicotinoyl)isoindolin-4-yl)pyrrolidine-1-carboxylate (0.18 g, 0.39 mmol) was dissolved in dioxane (1 mL). 3-Methyl-5-(4,4,5,5-tetramethyl-dioxaborolan-2-yl)-1H-pyrrolo[2,3-b]pyridine (112 mg, 0.4 mmol) was added, followed by water (0.1 mL) and potassium carbonate (164 mg, 1.18 mmol). Chloro (2-dicyclohexylphosphino-2',6'-dimethoxy-1,1'-biphenylyl)(2'-amino-1,1'-biphenyl-2-yl) palladium (II) (28.45 mg, 0.04 mmol) was finally added, and the system was heated and stirred for 12 h. Upon completion of the reaction, the reaction mixture was added to ice water (5 mL) and extracted with ethyl acetate (5 mL X 3). The organic phase was dried over anhydrous sodium sulfate, filtered and concentrated to give tert-butyl (S)-2-(2-(2,6-dimethylisonicotinoyl)-6-(3-methyl-1*H*-pyrrolo[2,3-*b*]pyridin-5-yl)isoindoli n-4-yl)pyrrolidine-1-carboxylate (0.2 g) as a yellow oil. ES-API: [M+H]⁺ = 552.3.

Step 15: The tert-butyl (S)-2-(2-(2,6-dimethylisonicotinoyl)-6-(3-methyl-1*H*-pyrrolo[2,3-*b*]pyridin-5-yl)isoindoli n-4-yl)pyrrolidine-1-carboxylate (0.2 g) was dissolved in ethyl acetate (1 mL). Hydrochloric acid-ethyl acetate (4.0M, 1.0 mL) was added and stirred at room temperature for 12 h. Upon completion of the reaction, the reaction mixture was concentrated to give a crude product. The crude product was purified by prep-HPLC (hydrochloric acid) to give (S)-(2,6-dimethylpyridin-4-yl)(6-(3-methyl-1H-pyrrolo[2,3-b]pyridin-5-yl)-4-(pyrrolidin-2-yl)isoindolin-2-yl)methanone hydrochloride (Z262, 59.48 mg, yield: 36.3%) as a white solid. ES-API: [M+H]⁺ = 452.1. ¹H NMR (400 MHz, CD₃OD) δ 9.08 - 9.06 (m, 1H), 8.82 (s, 1H), 8.12-8.06 (m, 1H), 7.96 - 7.80 (m, 3H), 7.55-7.54 (m, 1H), 5.28 - 5.14 (m, 2H), 5.02 - 4.99 (m, 1H), 4.87 - 4.83 (m, 1H), 4.65 - 4.64 (m, 1H), 3.71 - 3.47 (m, 2H), 2.87-2.86 (6H), 2.53 -2.51(m, 1H), 2.49-2.48 (m, 3H), 2.46- 2.13 (m, 3H).

### Example 186. Synthesis of compound Z438

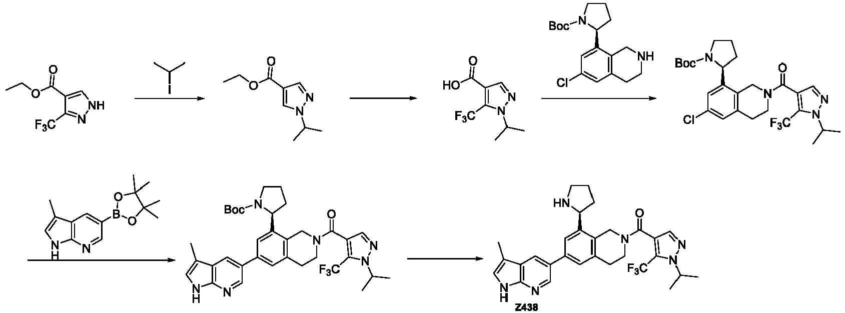

Step 1: Ethyl 3-(trifluoromethyl)-1H-pyrazole-4-carboxylate (1.0 g, 4.80 mmol) was dissolved in acetonitrile (10 mL) and stirred at room temperature. 2-Iodopropane (1.23 g, 7.21 mmol) was added to this solution, followed by potassium carbonate (1.0 g, 7.21 mmol), and the reaction mixture was stirred at 80°C for 6 h. The reaction was monitored by LCMS for completion, and quenched by the addition of water. The reaction mixture was extracted with ethyl acetate, dried over anhydrous sodium sulfate, filtered, and concentrated to give a crude product. The crude product was purified by a thin layer chromatographic column (petroleum ether/ethyl acetate = 10/1) to give ethyl 1-isopropyl-5-(trifluoromethyl)pyrazole-4-carboxylate (0.1 g, yield: 8%) as a yellow oil. ES-API:[M+1]⁺=251.1.

Step 2: Sodium hydroxide (79.92 mg, 2.00 mmol) was added to a solution of the ethyl 1-isopropyl-5-(trifluoromethyl)pyrazole-4-carboxylate (0.1 g, 0.4 mmol) in methanol/water (6 mL/1 mL), and the mixture was stirred at 20°C for 3 h. The reaction mixture was concentrated, diluted with water, and then adjusted to pH 3 with 1N hydrochloric acid to form a precipitate. The precipitate was filtered, and the obtained solid was dried to give 1-isopropyl-5-(trifluoromethyl)pyrazole-4-carboxylic acid (70.00 mg, crude product) as a white solid. ES-API:[M+1]⁺=223.0.

Step 3: The 1-isopropyl-5-(trifluoromethyl)pyrazole-4-carboxylic acid (69.25 mg, 311.71 µmol), 1-propylphosphonic anhydride (197 mg, 0.31 mmol, 50% in ethyl acetate) and triethylamine (63.08 mg, 623.41 µmol) were added to a solution of tert-butyl (S)-2-(6-chloro-1,2,3,4-tetrahydroisoquinolin-8-yl)pyrrolidine-1-carboxylate (70 mg, 0.21 mmol) in dichloromethane (6 mL), and the mixture was stirred at 25°C for 2 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by a preparative thin layer chromatographic column (petroleum ether/ethyl acetate = 1/1) to give a product tert-butyl (S)-2-[6-chloro-2-[1-isopropyl-3-(trifluoromethyl)-1H-pyrazole-4-carbonyl]-1,2,3,4-tetrah ydroisoquinolin-8-yl]pyrrolidine-1-carboxylate (60.00 mg, yield: 53.37%) as a yellow oil. ES-API: [M+1-100]⁺=441.2.

Step 4: The tert-butyl (S)-2-[6-chloro-2-[1-isopropyl-3-(trifluoromethyl)-1H-pyrazole-4-carbonyl]-1,2,3,4-tetrah ydroisoquinolin-8-yl]pyrrolidine-1-carboxylate (60 mg, 0.11 mmol) was dissolved in dioxane/water (4 mL/0.8 mL). 3-Methyl-5-(4,4,5,5-tetramethyl-1,3,2-dioxaborolan-2-yl)-1*H*-pyrrolo[2,3-*b*]pyridine (34.35 mg, 133.09 µmol), chloro (2-dicyclohexylphosphino-2',6'-dimethoxy-1,1'-biphenylyl)(2'-amino-1,1'-biphenyl-2-yl) palladium (II) (7.99 mg, 11.09 µmol) and potassium carbonate (45.91 mg, 332.71 µmol) were added, and the system was heated to 100°C for 1 h. Upon completion of the reaction, the reaction mixture was spun to dryness to give a crude product. The crude product was purified by a preparative thin layer chromatographic column (dichloromethane/methanol = 10/1) to give a product tert-butyl (*S*)-2-[2-[1-isopropyl-5-(trifluoromethyl)-1H-pyrazole-4-carbonyl]-6-(3-methyl-1*H*-pyrrol o[2,3-*b*]pyridin-5-yl)-1,2,3,4-tetrahydroisoquinolin-8-yl]pyrrolidine-1-carboxylate (55.00 mg, yield: 77.89%) as yellow oil. ES-API:[M+1]⁺=637.4.

Step 5: The tert-butyl (*S*)-2-[2-[1-isopropyl-5-(trifluoromethyl)-1H-pyrazole-4-carbonyl]-6-(3-methyl-1*H*-pyrrol o[2,3-*b*]pyridin-5-yl)-1,2,3,4-tetrahydroisoquinolin-8-yl]pyrrolidine-1-carboxylate (55 mg, 86.38 µmol) was dissolved in dichloromethane (2.0 mL). Trifluoroacetic acid (2.0 mL) was added, and the system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 10/1) to give a product (*S*)-[1-isopropyl-5-(trifluoromethyl)-1H-pyrazol-4-yl]-[6-(3-methyl-1*H*-pyrrolo[2,3-*b*]pyri din-5-yl)-8-[pyrrolidin-2-yl]-3,4-dihydroisoquinolin-2(1H)-yl]methanone (Z438, 18.00 mg, yield: 38.83%) as a yellow solid. ES-API:[M+1]⁺=537.3¹H NMR (400 MHz, CDCl₃) δ 9.61-9.38 (m, 1H), 8.55-8.49 (m, 1H), 8.02-7.98 (m, 1H), 7.84 (s, 1H),7.61-7.59 (m, 1H), 7.23 (s, 1H), 7.09 (s, 1H), 5.05 (d, *J* = 17.6 Hz, 1H), 4.89 (d, *J* = 17.5 Hz, 1H), 4.73-4.65 (m, 2H),4.45 (t, *J* = 5.0 Hz, 1H), 4.19-3.98 (m, 1H), 3.63-3.53 (m, 1H), 3.41-3.33 (m, 1H), 3.21-2.85 (m, 3H), 2.34 (s, 3H), 2.12-1.98 (m, 1H), 1.94-1.87 (m, 1H), 1.81-1.68 (m, 1H),1.55 (d, *J* = 5.8 Hz, 6H).

### Example 187. Synthesis of compound Z344

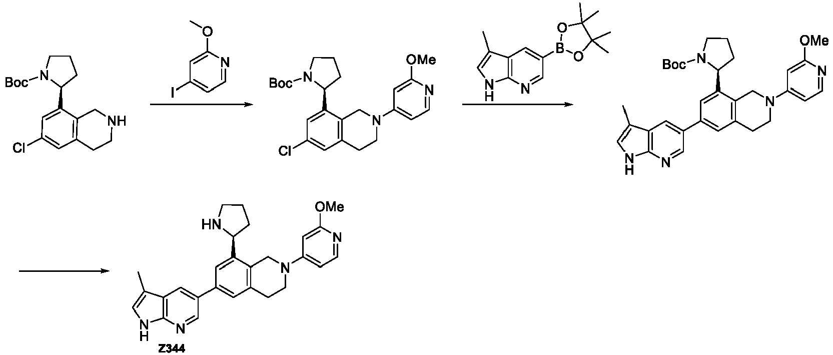

Step 1: 2-Dicyclohexylphosphino-2',6'-diisopropoxy-1,1'-biphenyl (27.12 mg, 59.37 µmol), (2-dicyclohexylphosphino-2',6'-diisopropoxy-1,1'-biphenylyl)(2-amino-1,1'-biphenyl-2-y l)palladium (II) mesylate (49.72 mg, 59.37 µmol), potassium carbonate (123.12 mg, 890.59 µmol) and 4-iodo-2-methoxypyridine (209.31 mg, 890.59 µmol) were added to a solution of tert-butyl (S)-2-(6-chloro-1,2,3,4-tetrahydroisoquinolin-8-yl)pyrrolidine-1-carboxylate (100 mg, 296.86 µmol) in dioxane (1 mL), and the system reacted at 130°C for 15 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (0-5% methanol in dichloromethane) to give a product tert-butyl (S)-2-(6-chloro-2-(2-methoxypyridin-4-yl)-1,2,3,4-tetrahydroisoquinolin-8-yl)pyrrolidine-1-carboxylate (90 mg, yield: 61.5%). ES-API:[M+H]⁺=444.3.

Step 2: The compound tert-butyl (S)-2-(6-chloro-2-(2-methoxypyridin-4-yl)-1,2,3,4-tetrahydroisoquinolin-8-yl)pyrrolidine-1-carboxylate (100 mg, 225.24 µmol) was dissolved in dioxane/water (2 mL/0.4 mL). 3-Methyl-5-(4,4,5,5-tetramethyl-1,3,2-dioxaborolan-2-yl)-1*H*-pyrrolo[2,3-*b*]pyridine (69.77 mg, 270.29 mmol), chloro (2-dicyclohexylphosphino-2',6'-dimethoxy-1,1'-biphenylyl)(2'-amino-1,1'-biphenyl-2-yl) palladium (II) (16.21 mg, 22.5 µmol) and potassium carbonate (96.7 mg, 675 µmol) were added, and the system was heated to 100°C for 2 h. The obtained mixture was extracted with dichloromethane/water, dried over anhydrous sodium sulfate, filtered, and concentrated to give a crude product. The crude product was purified by a preparative thin layer chromatographic column (dichloromethane/methanol = 10/1) to give a product tert-butyl (S)-2-(2-(2-methoxypyridin-4-yl)-6-(3-methyl-1*H*-pyrrolo[2,3-*b*]pyridin-5-yl)-1,2,3,4-tetr ahydroisoquinolin-8-yl)pyrrolidine-1-carboxylate (90 mg, yield: 68.1%). ES-API: [M+H]⁺=539.5.

Step 3: The tert-butyl (S)-2-(2-(2-methoxypyridin-4-yl)-6-(3-methyl-1*H*-pyrrolo[2,3-*b*]pyridin-5-yl)-1,2,3,4-tetr ahydroisoquinolin-8-yl)pyrrolidine-1-carboxylate (75 mg, 139 µmol) was dissolved in dichloromethane (1 mL). Trifluoroacetic acid (1 mL) was added, and the system reacted at room temperature for 1 h. Upon completion of the reaction, the reaction mixture was spun to dryness, extracted with dichloromethane and saturated aqueous sodium bicarbonate solution, dried over anhydrous sodium sulfate, filtered, and spun to dryness to give a crude product. The crude product was purified by a preparative thin layer chromatographic column (dichloromethane/methanol = 10/1) to give a product (S)-2-(2-methoxypyridin-4-yl)-6-(3-methyl-1H-pyrrolo[2,3-b]pyridin-5-yl)-8-(pyrrolidin-2-yl)-1,2,3,4-tetrahydroisoquinoline (Z344, 20 mg, yield: 40.95%). ES-API:[M+1]⁺=440.3.

### Example 188. Synthesis of compound Z348

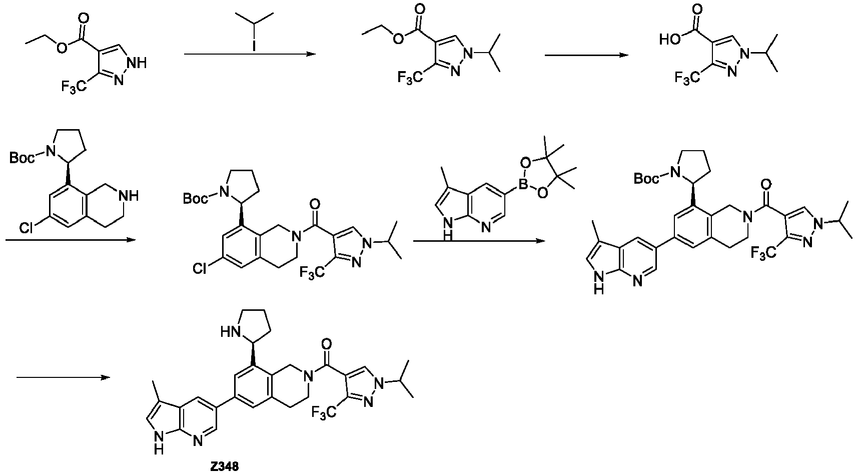

Step 1: Ethyl 3-(trifluoromethyl)-1*H*-pyrazole-4-carboxylate (1.0 g, 4.80 mmol) was dissolved in acetonitrile (10 mL) and stirred at room temperature. 2-Iodopropane (1.23 g, 7.21 mmol) was added to this solution, followed by potassium carbonate (1.0 g, 7.21 mmol), and the reaction mixture was stirred at 80°C for 6 h. The reaction was monitored by LCMS for completion, and quenched by the addition of water. The reaction mixture was extracted with ethyl acetate, dried over anhydrous sodium sulfate, filtered, and concentrated to give a crude product. The crude product was purified by silica gel column chromatography (petroleum ether/ethyl acetate = 10/1) to give ethyl 1-isopropyl-3-(trifluoromethyl)-1H-pyrazole-4-carboxylate (0.9 g, yield: 75%) as a white solid. ES-API:[M+1]⁺=251.1.

Step 2: Sodium hydroxide (0.72 mg, 17.98 mmol) was added to a solution of the ethyl 1-isopropyl-3-(trifluoromethyl)-1H-pyrazole-4-carboxylate (0.9 g, 3.60 mmol) in methanol/water (6 mL/1 mL), and the mixture was stirred at 20°C for 3 h. The reaction mixture was concentrated, diluted with water, and then adjusted to pH 3 with 1N hydrochloric acid to form a precipitate. The precipitate was filtered, and the obtained solid was dried to give 1-isopropyl-3-(trifluoromethyl)-1H-pyrazole-4-carboxylic acid (0.6 g, crude product) as a white solid. ES-API:[M+1]⁺=223.1.

Step 3: The 1-isopropyl-3-(trifluoromethyl)-1H-pyrazole-4-carboxylic acid (92.33 mg, 0.42 mmol), 1-propylphosphonic anhydride (197 mg, 0.31 mmol, 50% in ethyl acetate) and triethylamine (63.08 mg, 0.62 mmol) were added to a solution of tert-butyl (S)-2-(6-chloro-1,2,3,4-tetrahydroisoquinolin-8-yl)pyrrolidine-1-carboxylate (70 mg, 0.21 mmol) in dichloromethane (6 mL), and the mixture was stirred at 25°C for 2 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by a preparative thin layer chromatographic column (petroleum ether/ethyl acetate = 1/1) to give a product tert-butyl (S)-2-[6-chloro-2-[1-isopropyl-3-(trifluoromethyl)-1H-pyrazole-4-carbonyl]-1,2,3,4-tetrah ydroisoquinolin-8-yl]pyrrolidine-1-carboxylate (90 mg, yield: 80.05%) as a yellow oil. ES-API: [M+1-100]⁺=441.2.

Step 4: The tert-butyl (S)-2-[6-chloro-2-[1-isopropyl-3-(trifluoromethyl)-1H-pyrazole-4-carbonyl]-1,2,3,4-tetrah ydroisoquinolin-8-yl]pyrrolidine-1-carboxylate (90 mg, 0.17 mmol) was dissolved in dioxane/water (4 mL/0.8 mL). 3-Methyl-5-(4,4,5,5-tetramethyl-1,3,2-dioxaborolan-2-yl)-1H-pyrrolo[2,3-b]pyridine (51.53 mg, 0.2 mmol), chloro (2-dicyclohexylphosphino-2',6'-dimethoxy-1,1'-biphenylyl)(2'-amino-1,1'-biphenyl-2-yl) palladium (II) (11.98 mg, 16.64 µmol) and potassium carbonate (68.87 mg, 0.5 mmol) were added, and the system was heated to 100°C for 1 h. Upon completion of the reaction, the reaction mixture was spun to dryness to give a crude product. The crude product was purified by a preparative thin layer chromatographic column (dichloromethane/methanol = 10/1) to give a product tert-butyl (*S*)-2-[2-[1-isopropyl-3-(trifluoromethyl)-1H-pyrazole-4-carbonyl]-6-(3-methyl-1*H*-pyrrol o[2,3-*b*]pyridin-5-yl)-1,2,3,4-tetrahydroisoquinolin-8-yl]pyrrolidine-1-carboxylate (90 mg, yield: 84.97%) as yellow oil. ES-API:[M+1]⁺=637.5.

Step 5: The tert-butyl (*S*)-2-[2-[1-isopropyl-3-(trifluoromethyl)-1H-pyrazole-4-carbonyl]-6-(3-methyl-1*H*-pyrrol o[2,3-*b*]pyridin-5-yl)-1,2,3,4-tetrahydroisoquinolin-8-yl]pyrrolidine-1-carboxylate (90 mg, 0.14 mmol) was dissolved in dichloromethane (2.0 mL). Trifluoroacetic acid (2.0 mL) was added, and the system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 10/1) to give a product (*S*)-[1-isopropyl-3-(trifluoromethyl)-1H-pyrazol-4-yl]-[6-(3-methyl-1*H*-pyrrolo[2,3-*b*]pyri din-5-yl)-8-[pyrrolidin-2-yl]-3,4-dihydroisoquinolin-2(1H)-yl]methanone (Z348, 27.6 mg, yield: 36.39%) as a yellow solid. ES-API:[M+1]⁺=537.3. ¹H NMR (400 MHz, CDCl₃) δ 10.32 (s, 1H), 8.48 (s, 1H), 8.02 (s, 1H), 7.84-7.59 (m, 2H), 7.27 (s, 1H), 7.12 (s, 1H), 5.21-4.61 (m, 2H), 4.61-4.51 (m, 1H), 4.46-3.46 (m, 3H), 3.36-2.45 (m, 6H), 2.34 (s, 3H), 2.04-1.60 (m, 3H), 1.53 (d, *J* = 5.9 Hz, 6H).

### Example 189. Synthesis of compound Z350

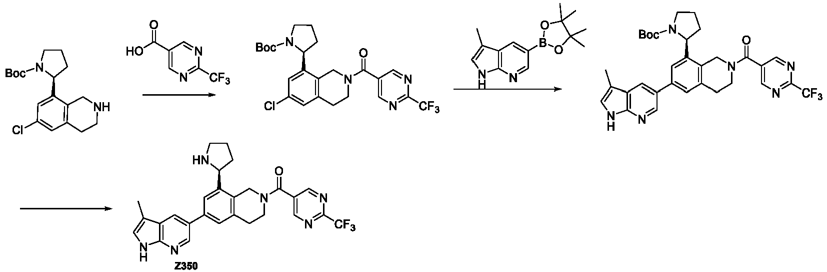

Step 1: 2-(Trifluoromethyl)-pyrimidine-5-carboxylic acid (41.06 mg, 213.74 µmol), 1-propylphosphonic anhydride (52.63 mg, 267.18 µmol) and triethylamine (0.05 mL) were added to a solution of tert-butyl (S)-2-(6-chloro-1,2,3,4-tetrahydroisoquinolin-8-yl)pyrrolidine-1-carboxylate (60 mg, 178.12 µmol) in dichloromethane (2 mL), and the mixture was stirred at 20°C for 2 h. The reaction mixture was spun to dryness to remove a solvent and obtain a crude product. The crude product was purified by silica gel column chromatography (petroleum ether/ethyl acetate = 1/1) to give tert-butyl (*S*)-2-[6-chloro-2-[2-(trifluoromethyl)pyrimidine-5-carbonyl]-1,2,3,4-tetrahydroisoquinoli n-8-yl]pyrrolidine-1-carboxylate as a yellow oil (65.00 mg, yield: 67.85%). ES-API: [M+H]⁺ = 511.17.

Step 2: The tert-butyl (*S*)-2-[6-chloro-2-[2-(trifluoromethyl)pyrimidine-5-carbonyl]-1,2,3,4-tetrahydroisoquinoli n-8-yl]pyrrolidine-1-carboxylate (55.00 mg, 0.11 mmol), 3-methyl-5-(4,4,5,5-tetramethyl-1,3,2-dioxaborolan-2-yl)-1*H*-pyrrolo[2,3-*b*]pyridine (44.06 mg, 0.17 mmol), chloro (2-dicyclohexylphosphino-2',6'-dimethoxy-1,1'-biphenylyl)(2'-amino-1,1'-biphenyl-2-yl) palladium (II) (10.24 mg, 0.014 mmol) and potassium carbonate (39.32 mg, 0.28 mmol) were dissolved in 1,4-dioxane solution (1 mL) and water (0.1 mL), and the system was warmed to 100°C for 2 h. Upon completion of the reaction, the reaction mixture was quenched by the addition of water and extracted with ethyl acetate. The organic phase was washed with brine, dried over anhydrous sodium sulfate, filtered, and concentrated to give a crude product. The crude product was purified by a thin layer chromatographic column (dichloromethane/methanol = 10/1) to give tert-butyl (*S*)-2-[6-(3-methyl-1H-pyrrolo[2,3-b]pyridin-5-yl)-2-[2-(trifluoromethyl)pyrimidine-5-car bonyl]-1,2,3,4-tetrahydroisoquinolin-8-yl]pyrrolidine-1-carboxylate (60.00 mg, yield: 66.06%) as a white solid. ES-API: [M+H]⁺ = 607.26.

Step 3: The tert-butyl (*S*)-2-[6-(3-methyl-1H-pyrrolo[2,3-b]pyridin-5-yl)-2-[2-(trifluoromethyl)pyrimidine-5-car bonyl]-1,2,3,4-tetrahydroisoquinolin-8-yl]pyrrolidine-1-carboxylate (60.00 mg, 93.96 µmol) was dissolved in dichloromethane (2 mL) and trifluoroacetic acid solution (2 mL) and stirred at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a thin layer chromatographic column (dichloromethane/methanol = 5/1) to give a product (*S*)-[6-(3-methyl-1H-pyrrolo[2,3-*b*]pyridin-5-yl)-8-[pyrrolidin-2-yl]-3,4-dihydroisoquinoli n-2(1H)-yl]-[2-(trifluoromethyl)pyrimidin-5-yl]methanone (Z350, 18.80 mg, yield: 44.13%) as a pale yellow solid. ES-API: [M+H]⁺ = 507.21. ¹H NMR (400 MHz, CDCl₃) δ 9.83-9.49 (m, 1H), 9.09-8.95 (m, 2H), 8.52-8.33 (m, 1H), 8.04-7.56 (m, 2H), 7.18 (s, 1H), 7.08 (s, 1H), 5.18 -4.84 (m, 2H), 4.22-3.97 (m, 1H), 3.65-3.44 (m, 2H), 3.35-3.23 (m, 1H), 3.15-2.75 (m, 3H), 2.44-2.35 (m, 1H), 2.32 (s, 3H), 2.22-2.12 (m, 1H), 2.07-2.01 (m, 1H), 1.94-1.81 (m, 1H).

### Example 190. Synthesis of compound Z354

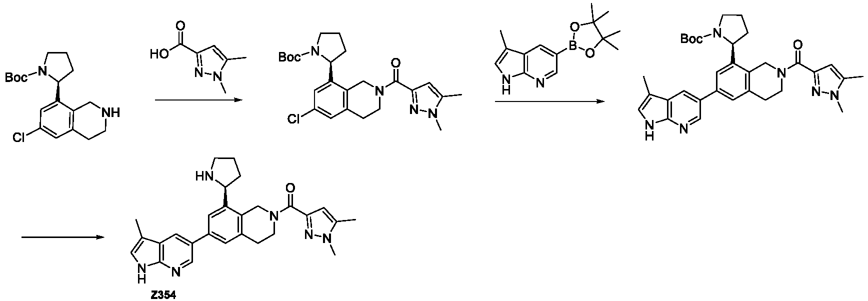

Step 1: were added to a solution of tert-butyl (S)-2-(6-chloro-1,2,3,4-tetrahydroisoquinolin-8-yl)pyrrolidine-1-carboxylate (60 mg, 178.12 µmol) in dichloromethane (2 mL) 1,5-Dimethyl-1*H*-pyrazole-3-carboxylic acid (29.95 mg, 213.74 µmol), 1-propylphosphonic anhydride (169.8 mg, 267.18 µmol, 50% in ethyl acetate), triethylamine (0.05 mL) and the mixture was stirred at 20°C for 2 h. The reaction mixture was spun to dryness to remove a solvent and obtain a crude product. The crude product was purified by silica gel column chromatography (petroleum ether/ethyl acetate = 1/1) to give tert-butyl (S)-2-[6-chloro-2-(1,5-dimethyl-1H-pyrazole-3-carbonyl)-1,2,3,4-tetrahydroisoquinolin-8-yl]pyrrolidine-1-carboxylate (60.00 mg, yield: 69.72%) as a yellow oil. ES-API: [M+H]⁺ = 459.2.

Step 2: The tert-butyl (S)-2-[6-chloro-2-(1,5-dimethyl-1H-pyrazole-3-carbonyl)-1,2,3,4-tetrahydroisoquinolin-8-yl]pyrrolidine-1-carboxylate (50.00 mg, 0.1 1 mmol), 3-methyl-5-(4,4,5,5-tetramethyl-1,3,2-dioxaborolan-2-yl)-1*H*-pyrrolo[2,3-*b*]pyridine (28.12 mg, 0.11 mmol), chloro (2-dicyclohexylphosphino-2',6'-dimethoxy-1,1'-biphenylyl)(2'-amino-1,1'-biphenyl-2-yl) palladium (II) (7.84 mg, 0.011 mmol) and potassium carbonate (30.11 mg, 0.22 mmol) were dissolved in 1,4-dioxane solution (1 mL) and water (0.1 mL), and the system was warmed to 100°C for 2 h. Upon completion of the reaction, the reaction mixture was quenched by the addition of water and extracted with ethyl acetate. The organic phase was washed with brine, dried over anhydrous sodium sulfate, filtered, and concentrated to give a crude product. The crude product was purified by a thin layer chromatographic column (dichloromethane/methanol = 10/1) to give tert-butyl (2)-2-[2-(1,5-dimethyl-1H-pyrazole-3-carbonyl)-6-(3-methyl-1*H*-pyrrolo[2,3-*b*]pyridin-5-yl)-1,2,3,4-tetrahydroisoquinolin-8-yl]pyrrolidine-1-carboxylate (45.00 mg, 65.75 µmol, yield: 70.75%) as a white solid. ES-API: [M+H]⁺ = 555.31.

Step 3: The tert-butyl (2)-2-[2-(1,5-dimethyl-1H-pyrazole-3-carbonyl)-6-(3-methyl-1*H*-pyrrolo[2,3-*b*]pyridin-5-yl)-1,2,3,4-tetrahydroisoquinolin-8-yl]pyrrolidine-1-carboxylate (45.00 mg, 65.75 µmol) was dissolved in dichloromethane (2 mL) and trifluoroacetic acid solution (2 mL) and stirred at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a thin layer chromatographic column (dichloromethane/methanol = 5/1) to give a product (*S*)-(1,5-dimethyl-1H-pyrazol-3-yl)-6-(3-methyl-1*H*-pyrrolo[2,3-*b*]pyridin-5-yl)-8-[pyrroli din-2-yl]-3,4-dihydroisoquinolin-2(1H)-yl]-methanone (Z354, 22.80 mg, yield: 54.53%) as a pale yellow solid. ES-API: [M+H]⁺ = 455.26. ¹H NMR (400 MHz, CDCl₃) δ 9.71 (s, 1H), 8.52 (s, 1H), 8.09-7.71 (m, 2H), 7.14 (s, 1H), 7.06-6.96 (m, 1H), 6.55-6.39 (m, 1H), 5.50-4.51 (m, 2H), 4.68-4.50 (m, 1H), 4.20-3.95 (m, 1H), 3.82 (s, 3H), 3.77-3.48 (m, 2H), 3.43-3.17 (m, 1H), 3.02-2.78 (m, 2H), 2.41-2.33 (m, 1H), 2.32-2.27 (m, 6H), 2.21-2.08 (m, 1H), 2.05-1.80 (m, 2H).

### Example 191. Synthesis of compound Z349

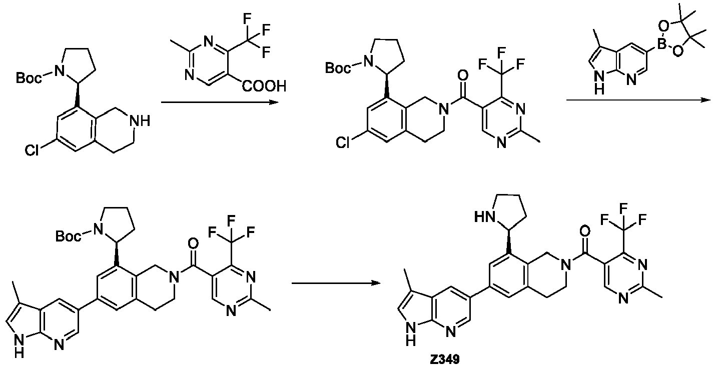

Step 1: 2-Methyl-4-(trifluoromethyl)pyrimidine-5-carboxylic acid (73.43 mg, 0.36 mmol), 1-propylphosphonic anhydride (337 mg, 0.53 mmol, 50% in ethyl acetate) and triethylamine (54.07 mg, 0.53 mmol) were added to a solution of tert-butyl (S)-2-(6-chloro-1,2,3,4-tetrahydroisoquinolin-8-yl)pyrrolidine-1-carboxylate (60 mg, 0.18 mmol) in dichloromethane (2 mL), and the mixture was stirred at 25°C for 2 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (mobile phase 0-50% ethyl acetate/petroleum ether, ethyl acetate/petroleum ether = 1/1) to give a product tert-butyl (S)-2-(6-chloro-2-(2-methyl-4-(trifluoromethyl)pyrimidine-5-carbonyl)-1,2,3,4-tetrahydroi soquinolin-8-yl)pyrrolidine-1-carboxylate (40 mg, yield: 42.78%). ES-API:[M+H-100]⁺= 425.2.

Step 2: 3-Methyl-5-(4,4,5,5-tetramethyl-1,3,2-dioxaborolan-2-yl)-1*H*-pyrrolo[2,3-*b*]pyridine (23.60 mg, 0.09 mmol), chloro (2-dicyclohexylphosphino-2',6'-dimethoxy-1,1'-biphenylyl)(2'-amino-1,1'-biphenyl-2-yl) palladium (II) (5.48 mg, 0.008 mmmol) and potassium carbonate (31.55 mg, 0.23 mmmol) were added to a solution of the compound tert-butyl (S)-2-(6-chloro-2-(2-methyl-4-(trifluoromethyl)pyrimidine-5-carbonyl)-1,2,3,4-tetrahydroi soquinolin-8-yl)pyrrolidine-1-carboxylate (40 mg, 0.076 mmol) in dioxane/water (2 mL/0.4 mL), and the mixture was heated to 110°C and stirred in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by silica gel column chromatography (mobile phase 0-10% methanol/dichloromethane, dichloromethane/methanol = 10/1, Rf = 0.6) to give a product tert-butyl (S)-2-(6-(3-methyl-1*H*-pyrrolo[2,3-b]pyridin-5-yl)-2-(2-methyl-4-(trifluoromethyl)pyrimi dine-5-carbonyl)-1,2,3,4-tetrahydroisoquinolin-8-yl)pyrrolidine- 1-carboxylate (40 mg, yield: 84.58%). ES-API:[M+H]⁺= 621.4.

Step 3: Trifluoroacetic acid (1 mL) was added to a solution of the compound tert-butyl (S)-2-(6-(3-methyl-1*H*-pyrrolo[2,3-b]pyridin-5-yl)-2-(2-methyl-4-(trifluoromethyl)pyrimi dine-5-carbonyl)-1,2,3,4-tetrahydroisoquinolin-8-yl)pyrrolidine- 1-carboxylate (40 mg, 0.06 mmol) in dichloromethane (2 mL) and stirred at room temperature for 1 h. The reaction was monitored by LCMS for completion. The reaction mixture was adjusted to pH 7 with saturated sodium bicarbonate, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 mL/min, gradient: B/A = 20%-90% over 50 min, wavelength: 214 nm, column temperature: room temperature ) to give (*S*)-(6-(3-methyl-1*H*-pyrrolo[2,3-*b*]pyridin-5-yl)-8-(pyrrolidin-2-yl)-3,4-dihydroisoquinoli n-2(1*H*)-yl)(2-methyl-4-(trifluoromethyl)pyrimidin-5-yl)methanone (Z349, 17.9 mg, yield: 53.36%) as a white powder. ES-API:[M+H]⁺=521.3. ¹H NMR (400 MHz, CDCl₃) δ 8.90 (s, 1H), 8.83-8.75 (m, 1H), 8.51-8.46 (m, 1H), 8.04-7.98 (m, 1H),7.84 (s, 1H), 7.32 (s, 1H), 7.09 (s, 1H), 5.44-4.44 (m, 2H), 4.44-4.36 (m, 1H), 4.20-3.86 (m, 1H), 3.56-3.42 (m, 1H),3.38-3.27 (m, 1H), 3.20-2.89 (m, 3H), 2.88 (s, 3H), 2.35 (s, 3H), 2.12-1.81 (m, 4H).

### Example 192. Synthesis of compound Z351

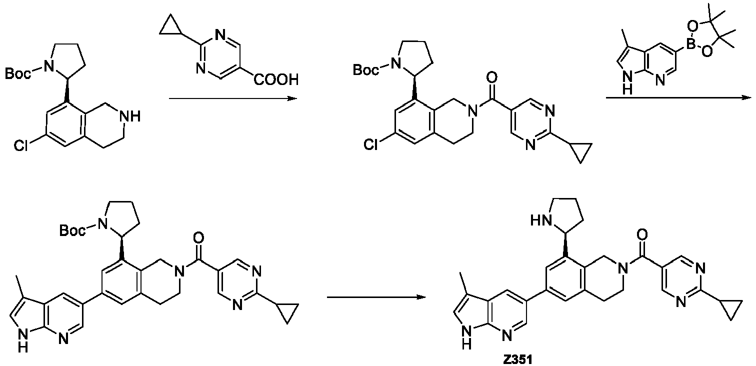

Step 1: 2-Cyclopropylpyrimidine-5-carboxylic acid (58.48 mg, 0.36 mmol), 1-propylphosphonic anhydride (337.1 mg, 0.53 mmol, 50% in ethyl acetate) and triethylamine (54.07 mg, 0.53 mmol) were added to a solution of tert-butyl (S)-2-(6-chloro-1,2,3,4-tetrahydroisoquinolin-8-yl)pyrrolidine-1-carboxylate (60 mg, 0.18 mmol) in dichloromethane (2 ml), and the mixture was stirred at 25°C for 2 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (mobile phase 0-50% ethyl acetate/petroleum ether, ethyl acetate/petroleum ether = 1/1, Rf = 0.4) to give a product tert-butyl (S)-2-(6-chloro-2-(2-cyclopropylpyrimidine-5-carbonyl)-1,2,3,4-tetrahydroisoquinolin-8-y l)pyrrolidine-1-carboxylate (45 mg, yield: 52.31%). ES-API:[M+H]⁺= 483.3.

Step 2: 3-Methyl-5-(4,4,5,5-tetramethyl-1,3,2-dioxaborolan-2-yl)-1*H*-pyrrolo[2,3-*b*]pyridine (28.86 mg, 0.11 mmol), chloro (2-dicyclohexylphosphino-2',6'-dimethoxy-1,1'-biphenylyl)(2'-amino-1,1'-biphenyl-2-yl) palladium (II) (6.7 mg, 0.009 mmmol) and potassium carbonate (38.57 mg, 0.238 mmmol) were added to a solution of the compound tert-butyl (S)-2-(6-chloro-2-(2-cyclopropylpyrimidine-5-carbonyl)-1,2,3,4-tetrahydroisoquinolin-8-y l)pyrrolidine-1-carboxylate (45 mg, 0.093 mmol) in dioxane/water (2 mL/0.4 mL), and the mixture was heated to 110°C and stirred in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by silica gel column chromatography (mobile phase 0-10% methanol/dichloromethane, dichloromethane/methanol = 10/1, Rf = 0.6) to give a product tert-butyl (S)-2-(2-(2-cyclopropylpyrimidine-5-carbonyl)-6-(3-methyl-1*H*-pyrrolo[2,3-*b*]pyridin-5-y l)-1,2,3,4-tetrahydroisoquinolin-8-yl)pyrrolidine-1-carboxylate (45 mg, yield: 83.46%). ES-API:[M+H]⁺= 579.5.

Step 3: Trifluoroacetic acid (1 mL) was added to a solution of the compound tert-butyl (S)-2-(2-(2-cyclopropylpyrimidine-5-carbonyl)-6-(3-methyl-1*H*-pyrrolo[2,3-*b*]pyridin-5-y l)-1,2,3,4-tetrahydroisoquinolin-8-yl)pyrrolidine-1-carboxylate (45 mg, 0.08 mmol) in dichloromethane (2 mL) and stirred at room temperature for 1 h. The reaction was monitored by LCMS for completion. The reaction mixture was adjusted to pH 7 with saturated sodium bicarbonate, treated with dichloromethane/water, dried over anhydrous sodium sulfate, filtered, and concentrated to give a crude product. The crude product was purified by prep-HPLC (column: Ultimate XB-C18, 50*250 mm, 10 um, mobile phase: A: purified water (0.05% ammonia water) B: pure acetonitrile, flow rate: 80 mL/min, gradient: B/A = 20%-90% over 50 min, wavelength: 214 nm, column temperature: room temperature) to give (*S*)-(2-cyclopropylpyrimidin-5-yl)(6-(3-methyl-1*H*-pyrrolo[2,3-*b*]pyridin-5-yl)-8-(pyrrolid in-2-yl)-3,4-dihydroisoquinolin-2(1*H*)-yl)methanone (Z351, 17.6 mg, yield: 47.29%) as a white powder. ES-API:[M+H]⁺=479.3. ¹H NMR (400 MHz, CDCl₃) δ 9.58 (s, 1H), 8.70 (s, 2H), 8.48 (s, 1H), 8.01 (s, 1H), 7.86-7.59 (m, 1H), 7.10 (s, 1H), 5.12-4.82 (m, 2H),4.53-3.94 (m, 1H), 3.94-3.59 (m, 2H), 3.41-2.92 (m, 4H), 2.34 (s, 3H), 2.11-1.59 (m, 4H), 1.35-1.07 (m, 5H).

### Example 193. Synthesis of compound Z376

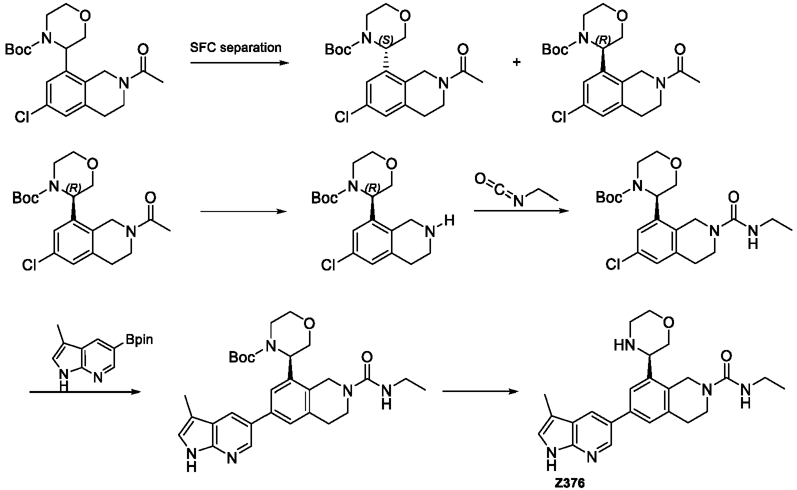

Step 1: Tert-butyl 3-(2-acetyl-6-chloro-1,2,3,4-tetrahydroisoquinolin-8-yl)morpholine-4-carboxylate (1.1 g, 2.79 mmol) was resolved by SFC (column type: IC: 5 µm, 4.6*250 mm, mobile phase: carbon dioxide:isopropyl alcohol = 70:30, flow rate: 3 mL/min, column temperature: 40°C, column pressure: atmospheric pressure) to give two stereoisomer, wherein a structure of one isomer was arbitrarily assigned as tert-butyl (S)-3-(2-acetyl-6-chloro-1,2,3,4-tetrahydroisoquinolin-8-yl)morpholine-4-carboxylate (406 mg, yield: 37%, retention time: 8.10 min, purity: 100%, ee value: 100%), ES-API: [M+H]⁺ = 395.0; a structure of the other isomer was arbitrarily assigned as tert-butyl (R)-3-(2-acetyl-6-chloro-1,2,3,4-tetrahydroisoquinolin-8-yl)morpholine-4-carboxylate (471 mg, yield: 43%, retention time: 9.83 min, purity: 100%, ee value: 100%). ES-API: [M+H]⁺ = 395.1.

Step 2: The compound tert-butyl (R)-3-(2-acetyl-6-chloro-1,2,3,4-tetrahydroisoquinolin-8-yl)morpholine-4-carboxylate (471 mg, 1.19 mmol) and sodium hydroxide (167 mg, 4.18 mmol) in ethanol (5 mL) and water (1 mL) was stirred at 80°C overnight. Upon completion of the reaction, the reaction mixture was concentrated, and ethyl acetate (30 mL) was added. The organic phase was washed with water (15 mL) and saturated brine (15 mL), dried over anhydrous sodium sulfate, filtered, and concentrated to give tert-butyl (R)-3-(6-chloro-1,2,3,4-tetrahydroisoquinolin-8-yl)morpholine-4-carboxylate (260 mg, crude product) which was directly used in the next reaction without purification. ES-API: [M+H]⁺ = 353.0.

Step 3:The tert-butyl (R)-3-(6-chloro-1,2,3,4-tetrahydroisoquinolin-8-yl)morpholine-4-carboxylate (90 mg, 255 umol) and ethyl isocyanate (22 mg, 306 umol, 24 uL) were dissolved in dichloromethane (1 mL). N,N-Diisopropylethyl amine (66 mg, 510 umol, 89 uL) was then added and stirred at room temperature for 2 h. Upon completion of the reaction, the reaction mixture was added to water (1 mL) and extracted with dichloromethane (1 mL X 3). The organic phase was washed with saturated brine (1 mL), dried over anhydrous sodium sulfate, filtered, and concentrated to give tert-butyl (R)-3-[6-chloro-2-(ethylcarbamoyl)-1,2,3,4-tetrahydroisoquinolin-8-yl]morpholine-4-carb oxylate (100 mg) as a brown oil. ES-API: [M+H]⁺ = 424.2.

Step 4: The tert-butyl (R)-3-[6-chloro-2-(ethylcarbamoyl)-1,2,3,4-tetrahydroisoquinolin-8-yl]morpholine-4-carb oxylate (100 mg, 236 umol) and 3-methyl-5-(4,4,5,5-tetramethyl-1,3,2-dioxaborolan-2-yl)-1H-pyrrolo[2,3-b]pyridine (73 mg, 283 umol) were dissolved in dioxane (1 mL) and water (0.2 mL), and potassium carbonate (65 mg, 472 umol) was then added. [2-(2-Aminophenyl)phenyl]-chloropalladium; dicyclohexyl-[2-(2,6-dimethoxyphenyl)phenyl]phosphorus (17.00 mg, 24 umol) was added in the presence of protective nitrogen and stirred at 120°C for 3 h. Upon completion of the reaction, the reaction mixture was added to water (1 mL) and extracted with ethyl acetate (1 mL × 3). The organic phase was washed with saturated brine (1 mL), dried over anhydrous sodium sulfate, filtered, and concentrated to give tert-butyl (R)-3-[2-(ethylcarbamoyl)-6-(3-methyl-1H-pyrrolo[2,3-b]pyridin-5-yl)-1,2,3,4-tetrahydroi soquinolin-8-yl]morphinme-4-carboxylate (104 mg) as a brown oil. ES-API: [M+H]⁺ = 520.2.

Step 5: The tert-butyl (R)-3-[2-(ethylcarbamoyl)-6-(3-methyl-1H-pyrrolo[2,3-b]pyridin-5-yl)-1,2,3,4-tetrahydroi soquinolin-8-yl]morphinme-4-carboxylate (104 mg, 200 umol) was dissolved in ethyl acetate (2 mL). Hydrochloric acid-ethyl acetate (4 M, 1 mL) was then added and stirred at room temperature for 4 h. Upon completion of the reaction, the reaction mixture was filtered and purified by prep-HPLC (formic acid method) to give (R)-N-ethyl-6-(3-methyl-1H-pyrrolo[2,3-b]pyridin-5-yl)-8-(morpholin-3-yl)-3,4-dihydrois oquinoline-2(1H)-carboxamide formate (Z376, 26.68 mg, yield: 31.8%) as a gray solid. ES-API: [M+H]⁺ = 420.1. ¹H NMR (400 MHz, CD₃OD) δ = 8.45 (br s, 1H), 8.43 - 8.38 (m, 1H), 8.22 (d, *J* = 1.9 Hz, 1H), 7.73 (s, 1H), 7.55 (s, 1H), 7.20 (s, 1H), 4.82 (s, 1H), 4.60 (d, *J* = 16.3 Hz, 2H), 4.05 (br d, *J* = 12.1 Hz, 2H), 3.74 (s, 2H), 3.65 (t, *J* = 6.0 Hz, 2H), 3.42 - 3.34 (m, 1H), 3.26 (d, *J* = 7.1 Hz, 3H), 3.01 (br t, *J* = 5.9 Hz, 2H), 2.38 (s, 3H), 1.16 (t, *J* = 7.2 Hz, 3H).

### Example 194. Synthesis of compound Z375

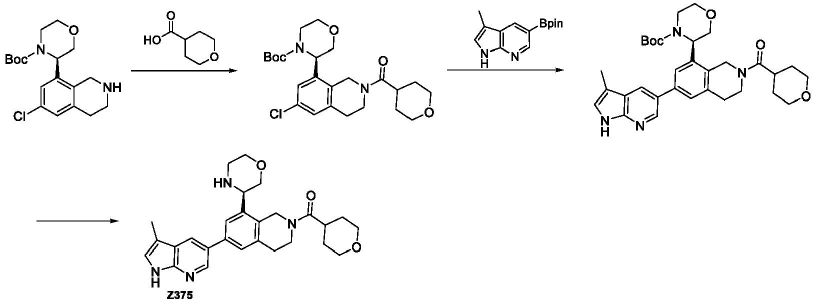

Step 1: Tert-butyl (R)-3-(6-chloro-1,2,3,4-tetrahydroisoquinolin-8-yl)morpholine-4-carboxylate (0.09 g, 255 umol) and tetrahydropyran-4-carboxylic acid (66 mg, 0.51 mmol) were dissolved in N,N-dimethylformamide (2 mL). N,N-Diisopropylethyl amine (98.9 mg, 765 umol, 133 uL) and O-(7-azabenzotriazol-1-yl)-N,N,N,N-tetramethylurea hexafluorophosphate (194 mg, 510 umol) were added, and the system reacted at room temperature for 2 h. Upon completion of the reaction, the reaction mixture was added to water (0.5 mL) and extracted with ethyl acetate (1 mL). The organic phase was washed with saturated brine (0.4 mL), dried over anhydrous sodium sulfate, filtered, and concentrated to give tert-butyl (R)-3-[6-chloro-2-(tetrahydropyran-4-carbonyl)-1,2,3,4-tetrahydroisoquinolin-8-yl]morph oline-4-carboxylate (105 mg) as a brown solid. ES-API: [M+H]⁺ = 465.1.

Step 2: The tert-butyl (R)-3-[6-chloro-2-(tetrahydropyran-4-carbonyl)-1,2,3,4-tetrahydroisoquinolin-8-yl]morph oline-4-carboxylate (105 mg, 226 umol) was dissolved in dioxane (1 mL). 3-Methyl-5-(4,4,5,5-tetramethyl-1,3,2-dioxaborolan-2-yl)-1H-pyrrolo[2,3-b]pyridine (70 mg, 271 umol) was added, followed by water (0.2 mL) and potassium carbonate (62 mg, 452 umol). Chloro (2-dicyclohexylphosphino-2',6'-dimethoxy-1,1'-biphenylyl)(2'-amino-1,1'-biphenyl-2-yl) palladium (II) (163 mg, 226 umol) was added, and the system was heated to 120°C and stirred for 12 h in the presence of protective nitrogen. Upon completion of the reaction, the reaction mixture was added to ice water (2 mL) and extracted with ethyl acetate (5 mL). The organic phase was washed with saturated brine (1 mL), dried over anhydrous sodium sulfate, filtered, and concentrated to give tert-butyl (R)-3-(6-(3-methyl-1H-pyrrolo[2,3-b]pyridin-5-yl)-2-(tetrahydropyran-4-carbonyl)-1,2,3,4 -tetrahydroisoquinolin-8-yl)morpholine-4-carboxylate (100 mg) as a yellow oil. ES-API: [M+H]⁺ = 561.3.

Step 3: The tert-butyl (R)-3-(6-(3-methyl-1H-pyrrolo[2,3-b]pyridin-5-yl)-2-(tetrahydropyran-4-carbonyl)-1,2,3,4 -tetrahydroisoquinolin-8-yl)morpholine-4-carboxylate (100 mg, 178 umol) was dissolved in ethyl acetate (1 mL). Hydrochloric acid-ethyl acetate (4.0 M, 1.0 mL) was added and stirred at room temperature for 2 h. Upon completion of the reaction, the reaction mixture was filtered. The filter cake was washed with ethyl acetate (1 mL), spun to dryness, and purified by prep-HPLC (formic acid method) to give (R)-(6-(3-methyl-1H-pyrrolo[2,3-b]pyridin-5-yl)-8 [morpholin-3-yl)-3,4-dihydro-1H-isoquinolin-2(1H)-yl)-tetrahydropyran-4-yl]methanone (Z375, formate, 29.2 mg, yield: 35.6%) as a black solid. ES-API: [M+H]⁺ = 461.1. ¹H NMR (400 MHz, CD₃OD) δ 8.45 (s, 1H), 8.22 (s, 1H), 7.75 (s, 1H), 7.57 - 7.53 (d, *J =* 18.0 Hz, 1H), 7.21 (s, 1H), 5.02 - 4.98 (d,J =16.8 Hz, 2H), 4.79 - 4.74 (d, J = 16.8 Hz, 1H), 4.47 - 4.45 (d, J = 8.8 Hz, 1H), 4.05-3.98 (m, 4H), 3.90 - 3.89 (d, J=4.0 Hz, 2H), 3.85 - 3.78 (m, 1H), 3.73 - 3.67 (m, 1H), 3.59-3.56 (d, J = 11.6 Hz, 2H), 3.35 (s, 2H), 3.13-3.09 (m, 2H), 2.38(s, 3H), 1.86-1.80(m, 2H), 1.70-1.67(d, J=12.0 Hz, 2H).

### Example 195. Synthesis of compound Z372

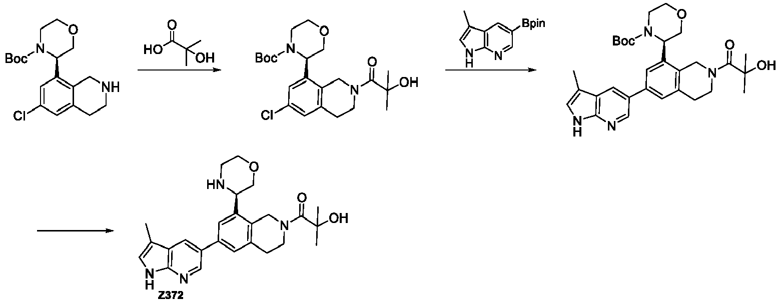

Step 1: tert-butyl (R)-3-(6-chloro-1,2,3,4-tetrahydroisoquinolin-8-yl)morphinme-4-carboxylate (90 mg, 255 umol) and 2-hydroxy-2-methylpropanoic acid (27 mg, 255 umol) were dissolved in N,N-dimethylformamide (1 mL). N,N-Diisopropylethyl amine (66 mg, 510 umol, 89 uL) and tetramethylurea hexafluorophosphate (97 mg, 255 umol) were then added and stirred at room temperature for 2 h. Upon completion of the reaction, the reaction mixture was added to water (1 mL) and extracted with ethyl acetate (1 mL × 3). The organic phase was washed with saturated brine (1 mL), dried over anhydrous sodium sulfate, filtered, and concentrated to give tert-butyl (R)-3-[6-chloro-2-(2-hydroxy-2-methylpropanoyl)-1,2,3,4-tetrahydroisoquinolin-8-yl]mor phinme-4-carboxylate (100 mg) as a brown oil. ES-API: [M+H]⁺ = 439.2.

Step 2: The tert-butyl (R)-3-[6-chloro-2-(2-hydroxy-2-methylpropanoyl)-1,2,3,4-tetrahydroisoquinolin-8-yl]mor phinme-4-carboxylate (100 mg, 228 umol) and 3-methyl-5-(4,4,5,5-tetramethyl-1,3,2-dioxaborolan-2-yl)-1H-pyrrolo[2,3-b]pyridine (71 mg, 273 umol) were dissolved in dioxane (1 mL) and water (0.2 mL), and potassium carbonate (63 mg, 456 umol) was added. Chloro (2-dicyclohexylphosphino-2',6'-dimethoxy-1,1'-biphenylyl)[2-(2'-amino-1,1'-biphenylyl] palladium (II) (16 mg, 23 umol) was added in the presence of protective nitrogen and stirred at 120°C for 3 h. Upon completion of the reaction, the reaction mixture was added to water (1 mL) and extracted with ethyl acetate (1 mL × 3). The organic phase was washed with saturated brine (1 mL), dried over anhydrous sodium sulfate, filtered, and concentrated to give tert-butyl (R)-3-[2-(2-hydroxy-2-methylpropanoyl)-6-(3-methyl-1H-pyrrolo[2,3-b]pyridin-5-yl)-1,2, 3,4-tetrahydroisoquinolin-8-yl]morphinme-4-carboxylate (110 mg) as a brown oil. ES-API: [M+H]⁺ = 535.3.

Step 3: The tert-butyl (R)-3-[2-(2-hydroxy-2-methylpropanoyl)-6-(3-methyl-1H-pyrrolo[2,3-b]pyridin-5-yl)-1,2, 3,4-tetrahydroisoquinolin-8-yl]morphinme-4-carboxylate (110 mg, 206 umol) was dissolved in ethyl acetate (2 mL). Hydrochloric acid-ethyl acetate solution (4 M, 1 mL) was then added and stirred at room temperature for 4 h. Upon completion of the reaction, the reaction mixture was filtered and purified by prep-HPLC (formic acid method) to give (R)-2-hydroxy-2-methyl-1-[6-(3-methyl-1H-pyrrolo[2,3-b]pyridin-5-yl)-8-[morphin-3-yl]-3,4-dihydroisoquinolin-2(1H)-yl]propan-1-one (Z372, formate, 21.64 mg, yield: 24.1%) as a white solid. ES-API: [M+H]⁺ = 435.1. ¹H NMR (400 MHz, CD₃OD) δ = 8.45 (d, *J* = 1.9 Hz, 1H), 8.39 (br s, 1H), 8.22 (d, *J* = 2.0 Hz, 1H), 7.74 (d, *J* = 1.5 Hz, 1H), 7.55 (s, 1H), 7.20 (d, *J* = 0.9 Hz, 1H), 5.05 - 4.93 (m, 1H), 4.80 (br d, *J* = 5.3 Hz, 1H), 4.51 - 4.41 (m, 1H), 4.36 - 4.16 (m, 1H), 4.05 (br dd, *J* = 2.8, 12.3 Hz, 2H), 3.83 (br d, *J* = 1.6 Hz, 2H), 3.50 - 3.32 (m, 2H), 3.30 - 3.23 (m, 1H), 3.07 (br s, 2H), 2.38 (d, *J* = 1.0 Hz, 3H), 1.56 - 1.43 (m, 6H).

### Example 196. Synthesis of compound Z382

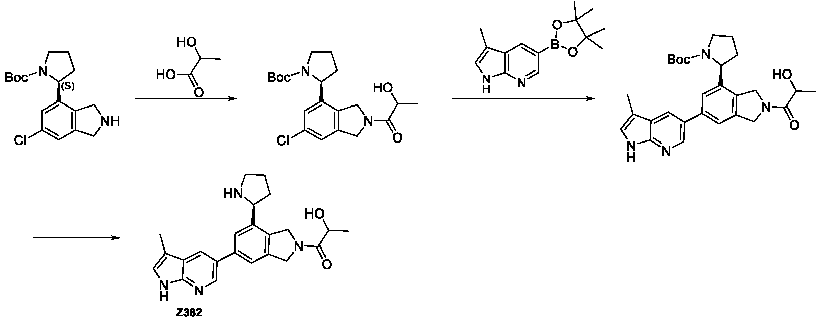

Step 1: Lactic acid (25 mg, 0.28 mmol) and tert-butyl (S)-2-(6-chloroisoindolin-4-yl)pyrrolidine-1-carboxylate were dissolved in anhydrous dichloromethane (1.0 mL). N,N-Diisopropylethyl amine and 2-(7-azobenzotriazole)-N,N,N',N'-tetramethylurea hexafluorophosphate (306 mg, 0.81 mmol) were added and stirred at room temperature for 12 h. Upon completion of the reaction, the reaction mixture was added to ice water (5.0 mL) and extracted with ethyl acetate (5.0 mL × 3). The organic phase was dried over anhydrous sodium sulfate, filtered, and concentrated to give rac-(2R)-tert-butyl 2-(6-chloro-2-(2-hydroxypropanoyl)isoindolin-4-yl)pyrrolidine-1-carboxylate (70 mg) as a yellow oil. ES-API: [M+H]⁺ = 395.1.

Step 2: The rac-(2R)-tert-butyl 2-(6-chloro-2-(2-hydroxypropanoyl)isoindolin-4-yl)pyrrolidine-1-carboxylate (70 mg, 0.18 mmol) was dissolved in dioxane (1.0 mL). 3-Methyl-5-(4,4,5,5-tetramethyl-1,3,2-dioxaborolan-2-yl)-1H-pyrrolo[2,3-b]pyridine (69 mg, 0.27 mmol) was added, followed by water (1.0 mL) and potassium carbonate (73.5 mg, 0.68 mmol). Chloro (2-dicyclohexylphosphino-2',6'-dimethoxy-1,1'-biphenylyl)[2-(2'-amino-1,1'-biphenylyl) palladium (II) (13 mg, 0.018 mmol) was finally added, and the system was heated to 120°C and stirred for 12 h. Upon completion of the reaction, the reaction mixture was added to ice water (5.0 mL) and then extracted with ethyl acetate (5.0 mL × 3). The organic phase was dried over anhydrous sodium sulfate, filtered, and concentrated to give rac-(2R)-tert-butyl 2-(2-(2-hydroxypropanoyl)-6-(3-methyl-1H-pyrrolo[2,3-b]pyridin-5-yl)isoindolin-4-yl)pyr rolidine-1-carboxylate (80 mg) as a yellow oil. ES-API: [M+H]⁺ = 491.2.

Step 3: The rac-(2R)-tert-butyl 2-(2-(2-hydroxypropanoyl)-6-(3-methyl-1H-pyrrolo[2,3-b]pyridin-5-yl)isoindolin-4-yl)pyr rolidine-1-carboxylate (80 mg) was dissolved in ethyl acetate (1.0 mL). Hydrochloric acid-ethyl acetate (4.0 M, 1.0 mL) was added and stirred at room temperature for 12 h. Upon completion of the reaction, the reaction mixture was concentrated and purified by prep-HPLC (formic acid method) to give rac-2-hydroxy-1-(6-(3-methyl-1H-pyrrolo [2,3-b]pyridin-5-yl)-4-((R)-pyrrolidin-2-yl)isoin dolin-2-yl)propan-1-one (Z382, formate, 1.38 mg, yield: 2.2%). ES-API: [M+H]⁺ = 391.2.

### Example 197. Synthesis of compound Z443

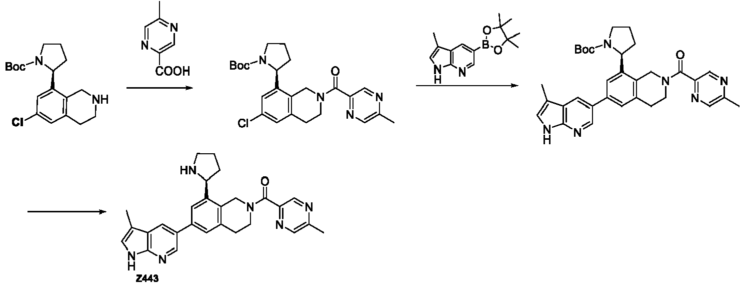

Step 1: 5-Methylpyrazine-2-carboxylic acid (57.41 mg, 0.42 mmol), 1-propylphosphonic anhydride (394.3 mg, 0.62 mmol, 50% in ethyl acetate) and triethylamine (63.08 mg, 0.62 mmol) were added to a solution of tert-butyl (S)-2-(6-chloro-1,2,3,4-tetrahydroisoquinolin-8-yl)pyrrolidine-1-carboxylate (70 mg, 0.21 mmol) in dichloromethane (2 mL), and the mixture was stirred at 25°C for 2 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (0-50% ethyl acetate/petroleum ether, ethyl acetate/petroleum ether = 1/1, Rf = 0.3) to give a product tert-butyl (S)-2-(6-chloro-2-(5-methylpyrazine-2-carbonyl)-1,2,3,4-tetrahydroisoquinolin-8-yl)pyrro lidine-1-carboxylate (50 mg, yield: 52.65%). ES-API:[M+H-100]⁺= 357.2.

Step 2: 3-Methyl-5-(4,4,5,5-tetramethyl-1,3,2-dioxaborolan-2-yl)-1*H*-pyrrolo[2,3-*b*]pyridine (33.89 mg, 0.13 mmol), chloro (2-dicyclohexylphosphino-2',6'-dimethoxy-1,1'-biphenylyl)(2'-amino-1,1'-biphenyl-2-yl) palladium (II) (7.87 mg, 0.011 mmol) and potassium carbonate (45.30 mg, 0.33 mmol) were added to a solution of the compound tert-butyl (S)-2-(6-chloro-2-(5-methylpyrazine-2-carbonyl)-1,2,3,4-tetrahydroisoquinolin-8-yl)pyrro lidine-1-carboxylate (50 mg, 0.11 mmol) in dioxane/water (2 mL/0.4 mL), and the mixture was heated to 110°C and stirred for 2 h in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by silica gel column chromatography (0-10% methanol/dichloromethane, dichloromethane/methanol = 10/1, Rf = 0.6) to give a product tert-butyl (S)-2-(6-(3-methyl-1*H*-pyrrolo[2,3-*b*]pyridin-5-yl)-2-(5-methylpyrazine-2-carbonyl)-1,2,3 ,4-tetrahydroisoquinolin-8-yl)pyrrolidine-1-carboxylate (35 mg, yield: 57.88%). ES-API:[M+H]⁺= 553.4.

Step 3: Trifluoroacetic acid (1 mL) was added to a solution of the compound tert-butyl (S)-2-(6-(3-methyl-1*H*-pyrrolo[2,3-*b*]pyridin-5-yl)-2-(5-methylpyrazine-2-carbonyl)-1,2,3 ,4-tetrahydroisoquinolin-8-yl)pyrrolidine-1-carboxylate (35 mg, 0.063 mmol) in dichloromethane (2 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by a preparative thin layer chromatographic column (dichloromethane/methanol = 8/1) to give a product (*S*)-(6-(3-methyl-1*H*-pyrrolo[2,3-*b*]pyridin-5-yl)-8-(pyrrolidin-2-yl)-3,4-dihydroisoquinoli n-2(1*H*)-yl)(5-methylpyrazin-2-yl)methanone (Z443, 15.5 mg, yield: 53.38%) as a white solid. ES-API:[M+H]⁺=453.3. ¹H NMR (400 MHz, CDCl₃) δ 8.99 (s, 1H), 8.92-8.86 (m, 1H), 8.55-8.42 (m, 2H), 8.02-7.95 (m, 1H), 7.83-7.65 (m, 1H), 7.28-7.26 (m, 1H), 7.07 (s, 1H), 5.21-4.87 (m, 2H), 4.55-4.11 (m, 1H), 4.03-3.78 (m, 2H), 3.44-2.98 (m, 4H), 2.67-2.61 (m, 3H), 2.40-2.30 (m, 4H), 2.17-1.93 (m, 3H).

### Example 198. Synthesis of compound Z404

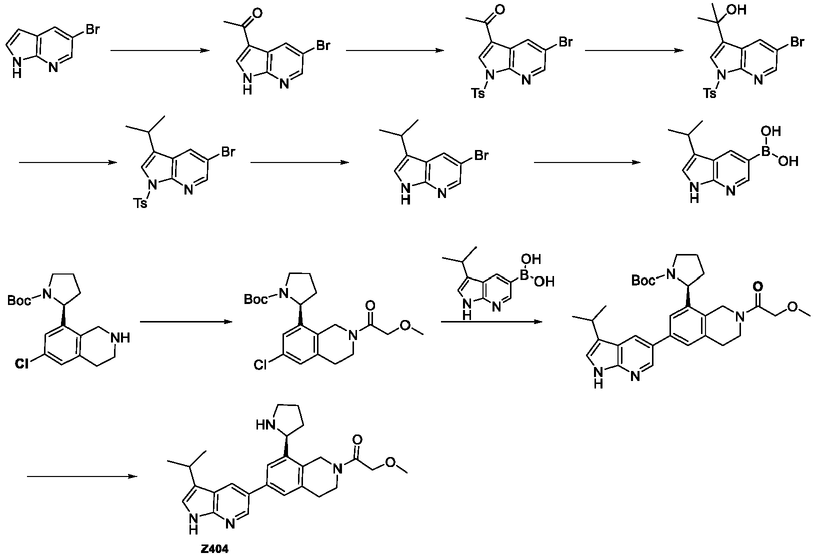

Step 1: In the presence of protective nitrogen, 5-bromo-1*H*-pyrrolo[2,3-b]pyridine (2 g, 10.15 mmol) was added to a stirred solution of aluminum trichloride (6.77 g, 50.77 mmol) suspended in anhydrous dichloromethane (100 mL), and the reaction mixture was stirred at 25°C for 1 h. Acetyl chloride (3.98 g, 50.75 mmol) was then added dropwise, and the reaction mixture was stirred for 5 h. The reaction was cooled to 0°C in an ice bath and carefully quenched with methanol until the solution became clear. The reaction mixture was concentrated, and water was added. 1N Sodium hydroxide was added dropwise to adjust pH to 4, and the obtained product was extracted into ethyl acetate. The organic layer was dried over anhydrous sodium sulfate and concentrated in vacuum to give a product 1-(5-bromo-1*H*-pyrrolo[2,3-*b*]pyridin-3-yl)ethanone (1.80 g, crude product) as a yellow solid. ES-API:[M+1]⁺=239.0.

Step 2: Sodium hydride (1.29 g, 32.21 mmol, purity: 60%) was added to a solution of the 1-(5-bromo-1*H*-pyrrolo[2,3-*b*]pyridin-3-yl)ethanone (7 g, 29.28 mmol) in tetrahydrofuran (60 mL) under ice bath cooling. After 1 h, p-toluenesulfonyl chloride (6.70 g, 35.14 mmol) in tetrahydrofuran (4 mL) was added dropwise, and the mixture was stirred at 25°C for 32 h. The reaction was monitored by LCMS for completion. The mixture was extracted with ethyl acetate and water, dried over anhydrous sodium sulfate, filtered, and concentrated. The residue was beaten with petroleum ether/ethyl acetate (5/1) to give 1-[5-bromo-1-(p-toluenesulfonyl)pyrrolo[2,3-*b*]pyridin-3-yl]ethanone (9.00 g, yield: 78.16%) as a yellow solid. ES-API:[M+1]⁺=395.1.

Step 3: Methylmagnesium bromide (1M, 11.44 mmol, 11.44 mL) was added dropwise to a solution of the 1-[5-bromo-1-(p-toluenesulfonyl)pyrrolo[2,3-*b*]pyridin-3-yl]ethanone (900 mg, 2.29 mmol) in tetrahydrofuran (30 mL) under an ice bath condition, and the obtained mixture was stirred at 0°C for 2 h. The mixture was poured into ice water and extracted with ethyl acetate (10 mLX3). The combined organic layer was washed with brine, dried over sodium sulfate, filtered, and concentrated in vacuum to dryness. The obtained residue was purified by silica gel column chromatography (0-30% ethyl acetate in petroleum ether) to give a product 2-[5-bromo-1-(p-toluenesulfonyl)pyrrolo[2,3-*b*]pyridin-3-yl]propan-2-ol (560.00 mg, yield: 59.78%) as a yellow solid. ES-API:[M+1]⁺=411.0.

Step 4: The 2-[5-bromo-1-(p-toluenesulfonyl)pyrrolo[2,3-*b*]pyridin-3-yl]propan-2-ol (500 mg, 1.22 mmol) was dissolved in anhydrous dichloromethane (10 mL). Triethylsilane (354.27 mg, 3.05 mmol) and trifluoroacetic acid (696.44 mg, 6.11 mmol) were added dropwise at 0°C. The obtained mixture was warmed to 25°C and stirred for 16 h. The mixture was poured into ice water, adjusted to pH 4-5 with saturated aqueous sodium bicarbonate solution, and extracted with dichloromethane (3 × 10 mL). The combined organic layer was washed with brine, dried over anhydrous sodium sulfate, filtered, and concentrated in vacuum to dryness. The obtained residue was purified by silica gel column chromatography (0-30% ethyl acetate in petroleum ether) to give a product 5-bromo-3-isopropyl-1-(p-toluenesulfonyl)pyrrolo[2,3-*b*]pyridine (380.00 mg, yield: 79.09%) as a white solid. ES-API:[M+1]⁺=395.0.

Step 5: 6N Sodium hydroxide (7.5 ml) was added to a solution of the 5-bromo-3-isopropyl-1-(p-toluenesulfonyl)pyrrolo[2,3-*b*]pyridine (380 mg, 0.97 mmol) in methanol, and the system heated to reflux and stirred for 2 h. The reaction mixture was concentrated, and the residue was poured into ice water. The mixture was adjusted to pH 5 with saturated citric acid solution and filtered. The filter cake was dissolved in ethyl acetate, dried over anhydrous sodium sulfate, filtered, and concentrated in vacuum to dryness to give 5-bromo-3-isopropyl-1H-pyrrolo[2,3-b]pyridine (225.00 mg, yield: 97.39%) as a yellow solid. ES-API:[M+1]⁺=239.0.

Step 6: Bis(pinacolato)diboron (165.67 mg, 652.42 µmol), 1,1-bis(diphenylphosphino)ferrocene dichloropalladium (36.42 mg, 50.19 µmol) and potassium acetate (246.26 mg, 2.51 mmol) were added to a solution of the 5-bromo-3-isopropyl-1*H*-pyrrolo[2,3-*b*]pyridine (120 mg, 501.86 µmol) in acetonitrile (1 mL), and the mixture was heated to 90°C under a nitrogen atmosphere and stirred overnight. The reaction mixture was filtered. The filter cake was washed with ethyl acetate, and the filtrate was concentrated in vacuum to dryness. The obtained residue was purified by silica gel column chromatography (5%-30% ethyl acetate in petroleum ether) to give (3-isopropyl-1*H*-pyrrolo[2,3-*b*]pyridin-5-yl)boronic acid (60.00 mg, yield: 41.78%) as a yellow solid. ES-API:[M+H]⁺= 205.1.

Step 7: 2-Methoxyacetic acid (37.44 mg, 0.42 mmol), 1-propylphosphonic anhydride (389.3 mg, 0.62 mmol, 50% in ethyl acetate) and triethylamine (63.08 mg, 0.62 mmol) were added to a solution of tert-butyl (S)-2-(6-chloro-1,2,3,4-tetrahydroisoquinolin-8-yl)pyrrolidine-1-carboxylate (70 mg, 0.21 mmol) in dichloromethane (2 mL), and the mixture was stirred at 25°C for 2 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (0-50% ethyl acetate/petroleum ether, ethyl acetate/petroleum ether = 1/1, Rf = 0.3) to give a product tert-butyl (S)-2-(6-chloro-2-(2-methoxyacetyl)-1,2,3,4-tetrahydroisoquinolin-8-yl)pyrrolidine-1-carb oxylate (60 mg, yield: 70.61%). ES-API:[M+H-100]⁺= 309.2.

Step 8: The (3-isopropyl-1*H*-pyrrolo[2,3-*b*]pyridin-5-yl)boronic acid (35.92 mg, 0.18 mmol), chloro (2-dicyclohexylphosphino-2',6'-dimethoxy-1,1'-biphenylyl)(2'-amino-1,1'-biphenyl-2-yl) palladium (II) (10.56 mg, 0.015 mmol) and potassium carbonate (60.75 mg, 0.45 mmol) were added to a solution of the compound tert-butyl (S)-2-(6-chloro-2-(2-methoxyacetyl)-1,2,3,4-tetrahydroisoquinolin-8-yl)pyrrolidine-1-carb oxylate (60 mg, 0.15 mmol) in dioxane/water (2 mL/0.4 mL), and the mixture was heated to 110°C and stirred for 2 h in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by silica gel column chromatography (0-10% methanol/dichloromethane, dichloromethane/methanol = 10/1, Rf = 0.6) to give a product tert-butyl (S)-2-(6-(3-isopropyl-1*H*-pyrrolo[2,3-*b*]pyridin-5-yl)-2-(2-methoxyacetyl)-1,2,3,4-tetrahy droisoquinolin-8-yl)pyrrolidine-1-carboxylate (25 mg, yield: 31.99%). ES-API:[M+H]⁺= 533.4.

Step 9: Trifluoroacetic acid (1 mL) was added to a solution of the compound tert-butyl (S)-2-(6-(3-isopropyl-1*H*-pyrrolo[2,3-*b*]pyridin-5-yl)-2-(2-methoxyacetyl)-1,2,3,4-tetrahy droisoquinolin-8-yl)pyrrolidine-1-carboxylate (20 mg, 0.05 mmol) in dichloromethane (2 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by a preparative thin layer chromatographic column (dichloromethane/methanol = 8/1) to give (*S*)-1-(6-(3-isopropyl-1H-pyrrolo[2,3-*b*]pyridin-5-yl)-8-(pyrrolidin-2-yl)-3,4-dihydroisoqu inolin-2(1*H*))-yl)-2-methoxyethan-1-one (Z404, 2.4 mg, yield: 11.82%) as a white powder. ES-API: [M+H]⁺=433.3.

### Example 199. Synthesis of compound Z387

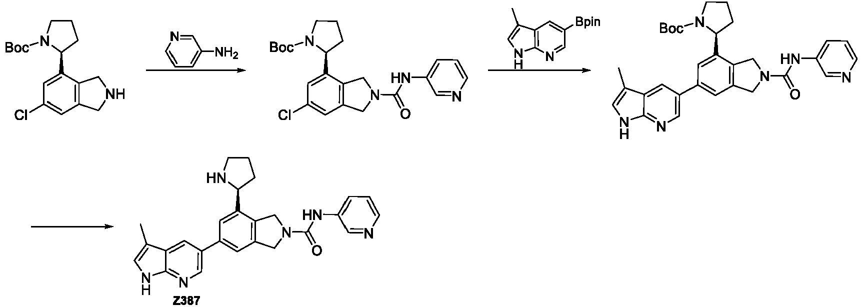

Step 1: 3-Aminopyridine (0.15 g, 1.59 mmol) was dissolved in anhydrous dichloromethane (1.0 mL). N,N-Diisopropylethyl amine (830 uL) was then added, and the system was cooled to 0°C. Triphosgene (0.39 g, 1.31 mmol) was added and stirred at room temperature for 2 h. Upon completion of the reaction, tert-butyl (S)-2-(6-chloroisoindolin-4-yl)pyrrolidine-1-carboxylate (0.15 g, 0.46 mmol) was added directly, and the system was heated to 25°C and stirred for 12 h. Upon completion of the reaction, the reaction mixture was added to ice water (5 mL) and extracted with ethyl acetate (5 mLX3). The organic phase was dried over anhydrous sodium sulfate, filtered, and concentrated to give tert-butyl (S)-2-(6-chloro-2-(pyridin-3-ylcarbamoyl)isoindolin-4-yl)pyrrolidine-1-carboxylate (0.1 g) as a yellow oil. ES-API: [M+H]⁺ = 443.3.

Step 2: The tert-butyl (S)-2-(6-chloro-2-(pyridin-3-ylcarbamoyl)isoindolin-4-yl)pyrrolidine-1-carboxylate (0.1 g, 0.23 mmol) was dissolved in dioxane (1 mL). 3-Methyl-5-(4,4,5,5-tetramethyl-1,3,2-dioxaborolan-2-yl)-1H-pyrrolo[2,3-b]pyridine (64 mg, 0.27 mmol) was added, followed by water (0.1 mL) and potassium carbonate (95 mg, 0.69 mmol). Chloro (2-dicyclohexylphosphino-2',6'-dimethoxy-1,1'-biphenylyl)[2-(2'-amino-1,1'-biphenylyl) palladium ((II) (16 mg, 0.02 mmol) was finally added, and the system was heated to 120°C and stirred for 12 h. Upon completion of the reaction, the reaction mixture was added to ice water (5 mL) and extracted with ethyl acetate (5 mLX3). The organic phase was dried over anhydrous sodium sulfate, filtered, and concentrated to give tert-butyl (S)-2-(6-(3-methyl-1H-pyrrolo[2,3-b]pyridin-5-yl)-2-(pyridin-3-ylcarbamoyl)isoindolin-4-yl)pyrrolidine-1-carboxylate (0.05 g) as a yellow oil. ES-API: [M+H]⁺ = 539.2.

Step 3: The tert-butyl (S)-2-(6-(3-methyl-1H-pyrrolo[2,3-b]pyridin-5-yl)-2-(pyridin-3-ylcarbamoyl)isoindolin-4-yl)pyrrolidine-1-carboxylate (0.05 g) was dissolved in ethyl acetate (1 mL). Hydrochloric acid-ethyl acetate (4.0 M, 1.0 mL) was added and stirred at room temperature for 12 h. Upon completion of the reaction, the reaction mixture was concentrated to give a crude product. The crude product was purified by prep-HPLC (formic acid method) to give (S)-6-(3-methyl-1H-pyrrolo[2,3-b]pyridin-5-yl)-N-(pyridin-3-yl)-4-(pyrrolidin-2-yl)isoind oline-2-carboxamide (Z387, formate, 1.38 mg, yield: 3.4%) as a white solid. ES-API: [M+H]⁺ = 439.2.

### Example 200. Synthesis of compound Z385

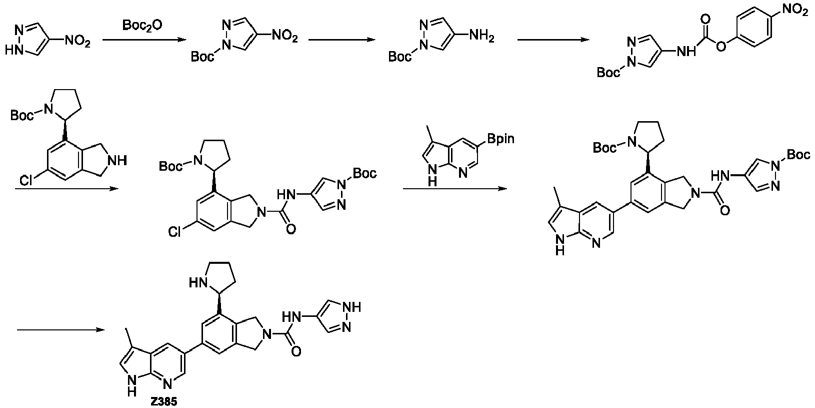

Step 1: 4-Nitropyrazole (5.0 g, 44.22 mmol) and di-tert-butyl dicarbonate (10.62 g, 48.64 mmol) were dissolved in tetrahydrofuran (35 mL). N,N-Diisopropylethyl amine (8.47 mL) and 4-dimethylamino pyridine (540.21 mg, 4.42 mmol) were then added and stirred at room temperature for 12 h. Upon completion of the reaction, the reaction mixture was added to ice water (50 mL) and extracted with ethyl acetate (30 mLX3). The organic phase was dried over anhydrous sodium sulfate, filtered, and concentrated. The crude product was beaten with petroleum ether (20 mL) to give tert-butyl 4-nitro-1H-pyrazole-1-carboxylate (9.0 g) as a yellow solid. ES-API: [M+H]⁺ = 214.9.

Step 2: The tert-butyl 4-nitro-1H-pyrazole-1-carboxylate (9.0 g, 44.22 mmol) was dissolved in ethanol (60 mL). Wet palladium on carbon (0.9 g, 10%) was then added, and the system was heated to 30°C and stirred for 12 h. Upon completion of the reaction, the reaction mixture was filtered. The filter cake was rinsed with ethyl acetate (50 mL) 3 to 5 times and concentrated. The obtained mixture was then beaten with methyl tert-butyl ether (20 mL) to give tert-butyl 4-amino-1H-pyrazole-1-carboxylate (5.0 g) as a yellow solid. ES-API: [M+H⁺-56] = 128.0.

Step 3: The tert-butyl 4-amino-1H-pyrazole-1-carboxylate (0.2 g, 1.36 mmol) and 4-nitrophenol chloroformate (302.55 mg, 1.50 mmol) were dissolved in anhydrous dichloromethane (1.0 mL). N,N-Diisopropylethyl amine (1.19 mL) was then added and stirred at room temperature for 2 h. Upon completion of the reaction, the reaction mixture was concentrated directly to give tert-butyl 4-(((4-nitrophenoxy) carbonyl)amino)-1H-pyrazole-1-carboxylate (0.3 g) as a yellow solid.

Step 4: The tert-butyl 4-(((4-nitrophenoxy) carbonyl)amino)-1H-pyrazole-1-carboxylate (309.76 ug, 371.71 umol) was dissolved in N,N-dimethylacetamide (1.0 mL). N,N-Diisopropylethyl amine (404.65 uL) was added and stirred for 1 min. tert-butyl (S)-2-(6-chloroisoindolin-4-yl)pyrrolidine-1-carboxylate (0.15 g, 464.64 umol) was then added, and the system was heated to 80°C and stirred for 12 h. Upon completion of the reaction, the reaction mixture was added to ice water (5 mL) and then extracted with ethyl acetate (5 mLx3). The organic phase was dried over anhydrous sodium sulfate, filtered, and concentrated to give tert-butyl (S)-4-(4-(1-(tert-butoxycarbonyl)pyrrolidin-2-yl)-6-chloroisoindoline-2-carboxamido)-1H -pyrazole-1-carboxylate (0.1 g) as a yellow oil. ES-API: [M+H⁺-200] = 331.9.

Step 5: The tert-butyl (S)-4-(4-(1-(tert-butoxycarbonyl)pyrrolidin-2-yl)-6-chloroisoindoline-2-carboxamido)-1H -pyrazole-1-carboxylate (0.1 g, 187.96 umol) was dissolved in dioxane (1 mL). 3-Methyl-5-(4,4,5,5-tetramethyl-dioxaborolan-2-yl)-1H-pyrrolo[2,3-b]pyridine (48.52 mg, 187.96 umol) was added, followed by water (0.1 mL) and potassium carbonate (25.98 mg, 187.96 umol). Chloro (2-dicyclohexylphosphino-2',6'-dimethoxy-1,1'-biphenylyl)(2'-amino-1,1'-biphenyl-2-yl) palladium (II) (13.55 mg, 18.80 umol) was finally added, and the system was heated to 120°C and stirred for 12 h. Upon completion of the reaction, the reaction mixture was added to ice water (5 mL) and then extracted with ethyl acetate (5 mLX3). The organic phase was dried over anhydrous sodium sulfate, filtered, and concentrated to give tert-butyl (S)-4-(4-(1-(tert-butoxycarbonyl)pyrrolidin-2-yl)-6-(3-methyl-1H-pyrrolo[2,3-b]pyridin-5-yl)isoindoline-2-carboxamide)-1H-pyrazole-1-carboxylate (0.1 g) as a yellow oil.

Step 6: The S)-tert-butyl 4-(4-(1-(tert-butoxycarbonyl)pyrrolidin-2-yl)-6-(3-methyl-1H-pyrrolo[2,3-b]pyridin-5-yl)i soindoline-2-carboxamide)-1H-pyrazole-1-carboxylate (0.1g) was dissolved in ethyl acetate (1 mL). Hydrochloric acid-ethyl acetate (4.0 M, 1.0 mL) was added and stirred at room temperature for 12 h. Upon completion of the reaction, the reaction mixture was concentrated to give a crude product. The crude product was purified by prep-HPLC (formic acid method) to give (S)-6-(3-methyl-1H-pyrrolo[2,3-b]pyridin-5-yl)-N-(1*H*-pyrazol-4-yl)-4-(pyrrolidin-2-yl))i soindoline-2-carboxamide (Z385, formate, 2.28 g, yield: 3%) as a white solid. ES-API: [M+H]⁺ = 428.2.

### Example 201. Synthesis of compound Z386

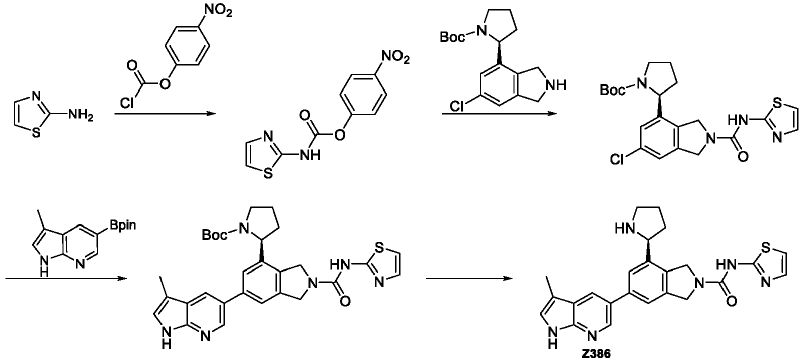

Step 1: 2-Aminothiazole (0.1 g, 1.36 mmol) and 4-nitrophenol chloroformate (201 mg, 1 mmol) were dissolved in anhydrous dichloromethane (1.0 mL). N,N-Diisopropylethyl amine (870 uL) was added and stirred at room temperature for 2 h. Upon completion of the reaction, the reaction mixture was concentrated directly to give 4-nitrophenylthiazol-2-ylcarbamate (0.15 g) as a yellow solid.

Step 2: The 4-nitrophenylthiazol-2-ylcarbamate (0.2 g, 0.75 mmol) was dissolved in *N*,*N*-dimethylacetamide (1.0 mL). N,N-Diisopropylethyl amine (240 uL) was added and stirred for 1 min. tert-butyl (S)-2-(6-chloroisoindolin-4-yl)pyrrolidine-1-carboxylate (221 mg, 0.69 mmol) was then added, and the system was heated to 80°C and stirred for 12 h. Upon completion of the reaction, the reaction mixture was added to ice water (5 mL) and then extracted with ethyl acetate (5 mLX3). The organic phase was dried over anhydrous sodium sulfate, filtered, and concentrated to give tert-butyl (S)-2-(6-chloro-2-(thiazol-2-ylcarbamoyl)isoindolin-4-yl)pyrrolidine-1-carboxylate (0.1g) as a yellow oil. ES-API: [M-200] ⁺ = 449.1.

Step 3: The tert-butyl (S)-2-(6-chloro-2-(thiazol-2-ylcarbamoyl)isoindolin-4-yl)pyrrolidine-1-carboxylate (107 mg, 0.24 mmol) was dissolved in dioxane (1 mL). 3-Methyl-5-(4,4,5,5-tetramethyl-1,3,2-dioxaborolan-2-yl)-1*H*-pyrrolo[2,3-*b*]pyridine (74 mg, 0.29 mmol) was added, followed by water (0.1 mL) and potassium carbonate (99 mg, 0.72 mmol). Chloro (2-dicyclohexylphosphino-2',6'-dimethoxy-1,1'-biphenylyl)[2-(2'-amino-1,1'-biphenylyl) palladium ((II) (17 mg, 0.02 mmol) was finally added, and the system was heated to 120°C and stirred for 12 h. Upon completion of the reaction, the reaction mixture was added to ice water (5 mL) and then extracted with ethyl acetate (5 mLx3). The organic phase was dried over anhydrous sodium sulfate, filtered, and concentrated to give tert-butyl (S)-2-(6-(3-methyl-1H-pyrrolo[2,3-b]pyridin-5-yl)-2-(thiazol-2-ylcarbamoyl)isoindolin-4-yl)pyrrolidine-1-carboxylate (0.05 g) as a yellow oil. ES-API: [M+H]⁺ = 545.2.

Step 4: The tert-butyl (S)-2-(6-(3-methyl-1H-pyrrolo[2,3-b]pyridin-5-yl)-2-(thiazol-2-ylcarbamoyl)isoindolin-4-yl)pyrrolidine-1-carboxylate (0.1g) was dissolved in ethyl acetate (1 mL). Hydrochloric acid-ethyl acetate (4.0 M, 1.0 mL) was added and stirred at room temperature for 12 h. Upon completion of the reaction, the reaction mixture was concentrated to give a crude product. The crude product was purified by prep-HPLC (ammonium bicarbonate method) to give (S)-6-(3-methyl-1H-pyrrolo[2,3-b]pyridin-5-yl)-4-(pyrrolidin-2-yl)-N-(thiazol-2-yl)isoind oline-2-carboxamide (Z386, 8.45 mg, yield: 10.3%) as a white solid. ES-API: [M+H]⁺ = 445.1.

### Example 202. Synthesis of compound Z439

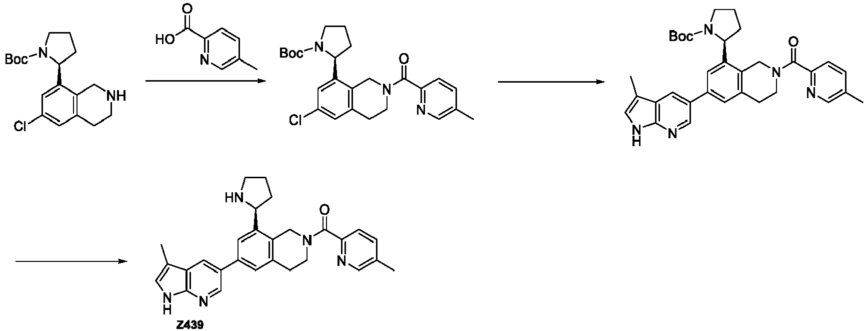

Step 1: tert-butyl (S)-2-(6-chloro-1,2,3,4-tetrahydroisoquinolin-8-yl)pyrrolidine-1-carboxylate (70 mg, 0.21 mmol) was dissolved in dichloromethane (2 ml). 5-Methylpicolinic acid (57 mg, 0.42 mmol), 1-propylphosphonic anhydride (396 mg, 0.62 mmol, 50% in ethyl acetate) and triethylamine (63 mg, 0.62 mmol) were added, and the system reacted at room temperature for 16 h. Upon completion of the reaction, the reaction was quenched by the addition of water. The reaction mixture was extracted with dichloromethane, washed with saturated brine, dried over anhydrous sodium sulfate, filtered, and spun to dryness to give a crude product. The crude product was purified by a preparative thin layer chromatographic column (petroleum ether/ethyl acetate = 2/1) to give a product tert-butyl (S)-2-(6-chloro-2-(5-methylpicolinoyl)-1,2,3,4-tetrahydroisoquinolin-8-yl)pyrrolidine-1-c arboxylate (90 mg, yield: 95%). ES-API:[M+H]⁺= 456.2.

Step 2: The tert-butyl (S)-2-(6-chloro-2-(5-methylpicolinoyl)-1,2,3,4-tetrahydroisoquinolin-8-yl)pyrrolidine-1-c arboxylate (90 mg, 0.20 mmol) was dissolved in dioxane/water (2 mL/0.4 mL). 3-Methyl-5-(4,4,5,5-tetramethyl-1,3,2-dioxaborolan-2-yl)-1*H*-pyrrolo[2,3-*b*]pyridine (61 mg, 0.24 mmol), chloro (2-dicyclohexylphosphino-2',6'-dimethoxy-1,1'-biphenylyl)(2'-amino-1,1'-biphenyl-2-yl) palladium (II) (14 mg, 0.020 mmol) and potassium carbonate (54 mg, 0.39 mmol) were added, and the system was heated to 100°C for 2 h. Upon completion of the reaction, the reaction mixture was spun to dryness to give a crude product. The crude product was purified by a preparative thin layer chromatographic column (dichloromethane/methanol = 20/1) to give a product tert-butyl (S)-2-(6-(3-methyl-1*H*-pyrrolo[2,3-*b*]pyridin-5-yl)-2-(5-methylpicolinoyl)-1,2,3,4-tetrahy droisoquinolin-8-yl)pyrrolidine-1-carboxylate (100 mg, yield: 92%). ES-API:[M+1]⁺=552.3.

Step 3: The tert-butyl (S)-2-(6-(3-methyl-1*H*-pyrrolo[2,3-*b*]pyridin-5-yl)-2-(5-methylpicolinoyl)-1,2,3,4-tetrahy droisoquinolin-8-yl)pyrrolidine-1-carboxylate (100 mg, 0.18 mmol) was dissolved in dichloromethane (2 mL). Trifluoroacetic acid (1 mL) was added, and the system reacted at room temperature for 2 h. Upon completion of the reaction, the reaction mixture was spun to dryness, extracted with dichloromethane and saturated aqueous sodium carbonate solution, dried over anhydrous sodium sulfate, filtered, and spun to dryness to give a crude product. The crude product was purified by a preparative thin layer chromatographic column (dichloromethane/methanol = 8/1) to give a product (S)-(6-(3-methyl-1H-pyrrolo[2,3-b]pyridin-5-yl)-8-(pyrrolidin-2-yl)-3,4-dihydroisoquinoli n-2(1H)-yl)(5-methylpyridin-2-yl)methanone (Z439, 31.4 mg, yield: 38%). ES-API:[M+1]⁺=452.2. ¹H NMR (400 MHz, CDCl₃) δ 9.69 (s, 1H), 8.47 (d, *J* = 14.4 Hz, 2H), 7.98 (d, *J* = 14.7 Hz, 1H), 7.83-7.60 (m, 3H), 7.25-7.2 (m, 1H), 7.08 (s, 1H), 5.20-4.84 (m, 2H), 4.57-3.75 (m, 3H), 3.42-2.99 (m, 4H), 2.65-2.57 (m, 1H), 2.41-2.33 (m, 6H), 2.05-1.73 (m, 3H).

### Example 203. Synthesis of compound Z440

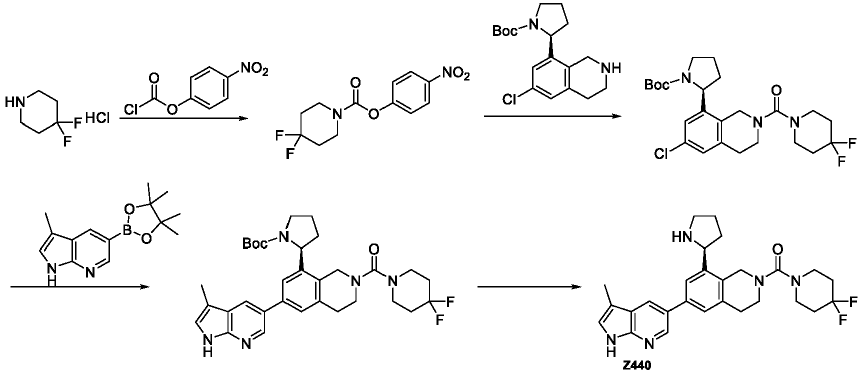

Step 1: In the presence of protective nitrogen, triethylamine (0.5 g, 4.95 mmol) and (4-nitrophenyl)carbonyl chloride (0.49 g, 2.48 mmol) were added to a solution of 4,4-difluoropiperidine hydrochloride (0.2 g, 1.65 mmol) in anhydrous dichloromethane (5 mL) at 0°C. The mixture was warmed to 20°C and stirred for 3 h. The reaction was monitored by LCMS for completion, and the reaction mixture was concentrated to give a crude product. The crude product was purified by silica gel column chromatography (petroleum ether/ethyl acetate = 10:90) to give 4-nitrophenyl-4,4-difluoropiperidine-1-carboxylate (300.00 mg, yield: 63.48%) as a yellow solid. ES-API:[M+1]⁺=287.1.

Step 2: The 4-nitrophenyl-4,4-difluoropiperidine-1-carboxylate (71.38 mg, 0.25 mmol) and potassium carbonate (86.16 mg, 0.62 mmol) were added to a solution of tert-butyl (S)-2-(6-chloro-1,2,3,4-tetrahydroisoquinolin-8-yl)pyrrolidine-1-carboxylate (70 mg, 0.21 mmol) in N,N-dimethylacetamide (1 mL), and the mixture was warmed to 150°C and stirred for 3 h. The reaction was monitored by LCMS for completion. The reaction mixture was diluted with water and extracted with ethyl acetate. The combined organic phase was washed with saturated brine, dried over anhydrous sodium sulfate, filtered, and concentrated to give a crude product. The crude product was purified by a preparative thin layer chromatographic column (ethyl acetate:petroleum ether = 1:1) to give tert-butyl (*S*)-2-(6-chloro-2-(4,4-difluoropiperidine-1-carbonyl)-1,2,3,4-tetrahydroisoquinolin-8-yl)p yrrolidine-1-carboxylate (77.00 mg, yield: 76.56%) as a yellow oil. ES-API:[M+1-100]⁺=384.2.

Step 3: 3-Methyl-5-(4,4,5,5-tetramethyl-1,3,2-dioxaborolan-2-yl)-1*H*-pyrrolo[2,3-*b*]pyridine (49.28 mg, 0.19 mmol), chloro (2-dicyclohexylphosphino-2',6'-dimethoxy-1,1'-biphenylyl)(2'-amino-1,1'-biphenyl-2-yl) palladium (II) (11.46 mg, 15.91 µmol) and potassium carbonate (65.87 mg, 0.48 mmol) were added to a solution of the (*S*)-2-[6-chloro-2-(4,4-difluoropiperidine-1-carbonyl)-1,2,3,4-tetrahydroisoquinolin-8-yl]p yrrolidine-1-carboxylate (77.00 mg, 0.16 mmol) in dioxane/water (4 mL/0.8 mL), and the system was heated to 100°C for 1 h. Upon completion of the reaction, the reaction mixture was spun to dryness to give a crude product. The crude product was purified by a preparative thin layer chromatographic column (dichloromethane/methanol = 10/1) to give a product tert-butyl (*S*)-2-(2-(4,4-difluoropiperidine-1-carbonyl)-6-(3-methyl-1*H*-pyrrolo[2,3-*b*]pyridin-5-yl)-1,2,3,4-tetrahydroisoquinolin-8-yl)pyrrolidine-1-carboxylate (70.00 mg, yield: 75.90%) as a yellow oil. ES-API:[M+1]⁺=580.4.

Step 4: The tert-butyl (*S*)-2-[2-(4,4-difluoropiperidine-1-carbonyl)-6-(3-methyl-1*H*-pyrrolo[2,3-*b*]pyridin-5-yl)-1,2,3,4-tetrahydroisoquinolin-8-yl]pyrrolidine-1-carboxylate (70 mg, 0.12 mmol) was dissolved in dichloromethane (2.0 mL). Trifluoroacetic acid (2.0 mL) was added, and the system reacted at room temperature for 1 h. Upon completion of the reaction, the mixture was quenched with saturated aqueous sodium bicarbonate solution and extracted with dichloromethane. The combined organic layer was washed with saturated sodium chloride, dried over anhydrous sodium sulfate, filtered, and concentrated to give a crude product. The crude product was purified by a preparative thin layer chromatographic column (dichloromethane/methanol = 10/1) to give a product (*S*)-(4,4-difluoropiperidin-1-yl)(6-(3-methyl-1*H*-pyrrolo[2,3-*b*]pyridin-5-yl)-8(pyrrolidin-2-yl)-3,4-dihydroisoquinolin-2(1H)-yl)methanone (Z440, 17.90 mg, yield: 30.91%) as a white solid. ES-API:[M+1]⁺=480.3. ¹H NMR (400 MHz, CDCl₃) δ 9.63 (s, 1H), 8.48 (d, *J* = 2.4 Hz, 1H), 8.02 (d, *J* = 2.0 Hz, 1H), 7.71 (d, *J* = 1.6 Hz, 1H), 7.28 (s, 1H), 7.11 (s, 1H), 4.64 (d, *J* = 16.4 Hz, 1H), 4.50 (d, *J* = 16.4 Hz, 1H), 4.34 (t, *J* = 6.0 Hz, 1H), 3.57-3.50 (m, 2H), 3.46-3.39 (m, 4H), 3.36-3.25 (m, 1H), 3.13-3.05 (m, 1H), 3.04-2.99 (m, 2H), 2.35 (s, 3H), 2.31-2.24 (m, 1H), 2.08-1.95 (m, 5H), 1.94-1.85 (m, 1H), 1.81-1.65 (m, 1H).

### Example 204. Synthesis of compound Z414

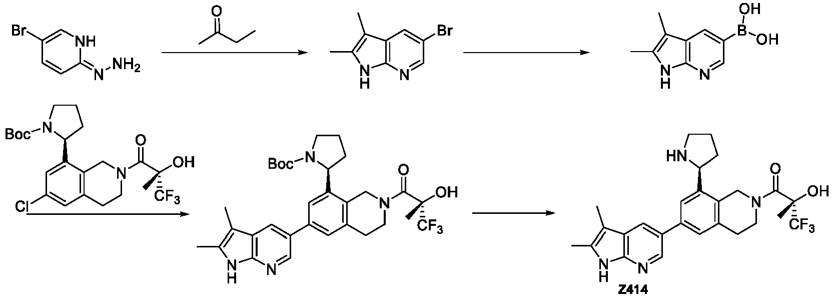

Step 1: 2-Hydrazino-5-bromopyridine (5.61 g, 30.00 mmol) was dissolved in ethanol (100 mL). 2-Butanone (2.16 g, 30.00 mmol) was added at room temperature and stirred at reflux for 2 h. The reaction was checked by LC-MS for completion, and the above mixture was spun to dryness. Polyphosphoric acid (50.00 g) was added, and the system reacted in an oil bath at 170°C for 1 h. The reaction was checked by LC-MS for completion and cooled to 30°C. Water (100 mL) was added. The mixture was cooled to 0°C and adjusted to pH 7-8 with 4 mol/L of sodium hydroxide solution. A solid was precipitated and filtered to give a filter cake. The filter cake was then rinsed with ice water (100 mL) and a mixed solution (150 mL) (methanol:water = 1:5), air-dried, extracted with a mixed solution (dichloromethane:methanol = 10:1) (150 mL×2), dried over anhydrous sodium sulfate, filtered, and concentrated to give a product 5-bromo-2,3-dimethyl-1H-pyrrolo[2,3-b]pyridine (4.21 g, yield: 62%) as a white solid. ES-API: [M+H]⁺=225.2.

Step 2: The 5-bromo-2,3-dimethyl-1H-pyrrolo[2,3-b]pyridine (300.0 mg, 1.33 mmol) was dissolved in dioxane (4 mL). Bis(pinacolato)diboron (372.5 mg, 1.47 mmol), [[1,1'-bis(diphenylphosphino)ferrocene]dichloropalladium (93.7 mg, 0.13 mmol) and potassium acetate (260.7 mg, 2.66 mmol) were added at room temperature. The system was replaced with nitrogen for protection and stirred in an oil bath at 100°C for 16 h. The reaction was checked by LC-MS for completion, and water (10 mL) was added. The obtained mixture was extracted with a mixed solution (dichloromethane:methanol = 10:1) (50 mL×2), dried over anhydrous sodium sulfate, filtered, concentrated, and purified by silica gel column chromatography (dichloromethane:methanol = 3%-6%) to give a product (2,3-dimethyl-1H-pyrrolo[2,3-b]pyridin-5-yl)boronic acid (210.3 mg, yield: 83%). ES-API: [M+H]⁺=191.2.

Step 3: Tert-butyl (S)-2-(6-chloro-2-((S)-3,3,3-trifluoro-2-hydroxy-2-methylpropanoyl)-1,2,3,4-tetrahydroiso quinolin-8-yl)pyrrolidine-1-carboxylate (100 mg, 0.21 mmol) was dissolved in dioxane (2 mL) and water (0.5 mL). The (2,3-dimethyl-1H-pyrrolo[2,3-b]pyridin-5-yl)boronic acid (47.9 mg, 0.25 mmol), chloro (2-dicyclohexylphosphino-2',6'-dimethoxy-1,1'-biphenylyl)(2'-amino-1,1'-biphenyl-2-yl) palladium (II) (14.4 mg, 0.02 mmol) and potassium carbonate (57.9 mg, 0.42 mmol) were added at room temperature and stirred under microwave irradiation at 110°C for 2 h in the presence of protective nitrogen. The reaction mixture was extracted with a mixed solution (dichloromethane:methanol = 10:1) (10 mL), washed with water (10 mL) and saturated brine (10 mL) successively, dried over anhydrous sodium sulfate, filtered, concentrated, and purified by silica gel column chromatography (dichloromethane:methanol = 3%-7%) to give a product tert-butyl (S)-2-(6-(2,3-dimethyl-1H-pyrrolo[2,3-b]pyridin 5-yl)-2-((S)-3,3,3-trifluoro-2-hydroxy-2-methylpropanoyl)-1,2,3,4-tetrahydroisoquinolin-8 -yl)pyrrolidine-1-carboxylate (112.1 mg, yield: 91%). ES-API: [M+H]⁺ = 587.2.

Step 4: The (*S*)-2-(6-(2,3-dimethyl-1H-pyrrolo[2,3-b]pyridin 5-yl)-2-((S)-3,3,3-trifluoro-2-hydroxy-2-methylpropanoyl)-1,2,3,4-tetrahydroisoquinolin-8 -yl)pyrrolidine-1-carboxylate (112.1 mg, 0.19 mmol) was dissolved in dichloromethane (4.0 mL). Trifluoroacetic acid (2.0 mL) was added, and the system reacted at room temperature for 2 h. The reaction mixture was concentrated. 7.0M Ammonia-methanol solution (5.0 mL) was added, and a solvent was spun to dryness to a crude product. The crude product was purified by prep-HPLC (hydrochloric acid) to give a desired product (*S*)-1-(6-(2,3-dimethyl-1H-pyrrolo[2,3-b]pyridin-5-yl)-8-((S)-pyrrolidin-2-yl)-3,4-dihydroi soquinolin-2(1H)-yl)-3,3,3-trifluoro-2-hydroxy-2-methylpropan-1-one (Z414, 45.3 mg, yield: 49%) as a white solid. ES-API: [M+H]⁺=487.2. ¹H NMR (400 MHz, DMSO-d₆) *δ* 11.97 (s, 1H), 10.00-9.70 (m, 1H), 9.60 -9.20 (m, 1H), 8.68 (s, 1H), 8.57 (s, 1H), 8.03 (s, 1H), 7.68(s, 1H), 7.50-6.90(m, 1H), 5.02-4.80 (m, 1H), 4.79-4.68 (m, 2H), 4.24-4.14(s, 2H), 4.12-4.00 (m, 2H), 3.60-3.30 (m, 2H), 3.02 (s, 2H), 2.39 (s, 3H), 2.27 (s, 3H), 2.21-1.97 (m, 3H), 1.61 (s, 3H).

### Example 205. Synthesis of compound Z441

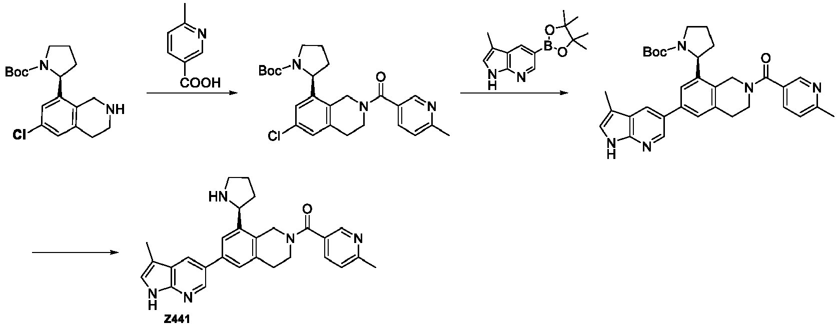

Step 1: 6-Methylnicotinic acid (56.99 mg, 0.42 mmol), 1-propylphosphonic anhydride (394.3 mg, 0.62 mmol, 50% in ethyl acetate) and triethylamine (63.08 mg, 0.62 mmol) were added to a solution of tert-butyl (S)-2-(6-chloro-1,2,3,4-tetrahydroisoquinolin-8-yl)pyrrolidine-1-carboxylate (70 mg, 0.21 mmol) in dichloromethane (2 mL), and the mixture was stirred at 25°C for 2 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (0-50% ethyl acetate/petroleum ether, ethyl acetate/petroleum ether = 1/1, Rf = 0.3) to give a product tert-butyl (S)-2-(6-chloro-2-(6-methylnicotinoyl)-1,2,3,4-tetrahydroisoquinolin-8-yl)pyrrolidine-1-c arboxylate (65 mg, yield: 68.60%). ES-API:[M+H]⁺= 456.3.

Step 2: 3-Methyl-5-(4,4,5,5-tetramethyl-1,3,2-dioxaborolan-2-yl)-1*H*-pyrrolo[2,3-*b*]pyridine (44.15 mg, 0.17 mmol), chloro (2-dicyclohexylphosphino-2',6'-dimethoxy-1,1'-biphenylyl)(2'-amino-1,1'-biphenyl-2-yl) palladium (II) (10.26 mg, 0.014 mmol) and potassium carbonate (59.02 mg, 0.43 mmol) were added to a solution of the compound tert-butyl (S)-2-(6-chloro-2-(6-methylnicotinoyl)-1,2,3,4-tetrahydroisoquinolin-8-yl)pyrrolidine-1-c arboxylate (65 mg, 0.14 mmol) in dioxane/water (2 mL/0.4 mL), and the mixture was heated to 110°C and stirred for 2 h in the presence of protective nitrogen. The reaction was monitored by LCMS for completion. The reaction mixture was treated with ethyl acetate/water, dried over anhydrous sodium sulfate, filtered, and concentrated to give a crude product. The crude product was purified by silica gel column chromatography (0-10% methanol/dichloromethane, dichloromethane/methanol = 10/1, Rf = 0.6) to give a product tert-butyl (S)-2-(6-(3-methyl-1*H*-pyrrolo[2,3-*b*]pyridin-5-yl)-2-(6-methylnicotinoyl)-1,2,3,4-tetrahy droisoquinolin-8-yl)pyrrolidine-1-carboxylate (60 mg, yield: 76.29%). ES-API:[M+H]⁺= 552.4.

Step 3: Trifluoroacetic acid (1 mL) was added to a solution of the compound tert-butyl (S)-2-(6-(3-methyl-1*H*-pyrrolo[2,3-*b*]pyridin-5-yl)-2-(6-methylnicotinoyl)-1,2,3,4-tetrahy droisoquinolin-8-yl)pyrrolidine-1-carboxylate (60 mg, 0.06 mmol) in dichloromethane (2 mL) and stirred at room temperature for 1 h. The reaction was monitored by LCMS for completion. The reaction mixture was adjusted to pH 7 with saturated aqueous sodium bicarbonate solution, treated with dichloromethane/water, dried over anhydrous sodium sulfate, filtered, and concentrated to give a crude product. The crude product was purified by a preparative thin layer chromatographic column (dichloromethane/methanol = 8/1) to give a product (S)-(6-(3-methyl-1H-pyrrolo[2,3-b]pyridin-5-yl)-8-(pyrrolidin-2-yl)-3,4-dihydroisoquinoli n-2(1H)-yl)(6-methylpyridin-3-yl)methanone (15.5 mg, yield: 31.56%) as a white solid. ES-API:[M+H]⁺=452.3. ¹H NMR (400 MHz, CDCl₃) δ 9.34 (s, 1H), 8.63 (d, *J=* 1.8 Hz, 1H), 8.49 (s, 1H), 8.02 (s, 1H), 7.88-7.61 (m, 2H),7.31-7.26 (m, 1H), 7.24-7.21 (m, 1H), 7.10 (s, 1H), 5.14-4.73 (m, 2H), 4.54-3.60 (m, 3H), 3.42-2.82 (m, 4H),2.62 (s, 3H), 2.35 (s, 3H), 2.18-1.98 (m, 4H).

### Example 206. Synthesis of compound Z442

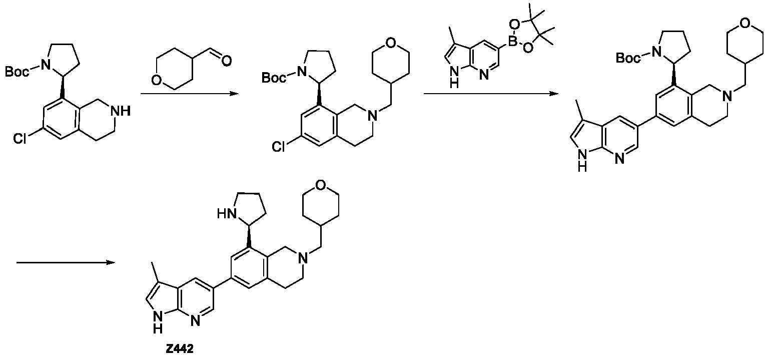

Step 1: Tetrahydro-2*H*-pyran-4-carbaldehyde (18.64 mg, 0.16 mmol) and sodium triacetoxyborohydride (29 mg, 0.24 mmol) were added to a solution of tert-butyl (S)-2-(6-chloro-1,2,3,4-tetrahydroisoquinolin-8-yl)pyrrolidine-1-carboxylate (50 mg, 0.18 mmol) in dichloromethane (2 mL), and the mixture was stirred at 25°C for 2 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (mobile phase 0-50% ethyl acetate/petroleum ether, ethyl acetate/petroleum ether = 1/1, Rf = 0.4) to give a product tert-butyl (S)-2-(6-chloro-2-((tetrahydro-2*H*-pyran-4-yl)methyl)-1,2,3,4-tetrahydroisoquinolin-8-yl) pyrrolidine-1-carboxylate (50 mg, yield: 82%). ES-API:[M+H]⁺= 435.3.

Step 2: 3-Methyl-5-(4,4,5,5-tetramethyl-1,3,2-dioxaborolan-2-yl)-1H-pyrrolo[2,3-b]pyridine (35.6 mg, 0.14 mmol), chloro (2-dicyclohexylphosphino-2',6'-dimethoxy-1,1'-biphenylyl)(2'-amino-1,1'-biphenyl-2-yl) palladium (II) (8.27 mg, 0.011 mmol) and potassium carbonate (47.57 mg, 0.344 mmol) were added to a solution of the compound tert-butyl (S)-2-(6-chloro-2-((tetrahydro-2*H*-pyran-4-yl)methyl)-1,2,3,4-tetrahydroisoquinolin-8-yl) pyrrolidine-1-carboxylate (50 mg, 0.16 mmol) in dioxane/water (2 mL/0.4 mL), and the mixture was heated to 110°C and stirred for 2 h in the presence of protective nitrogen. The reaction was monitored by LCMS for completion. The reaction mixture was extracted with ethyl acetate/water, dried over anhydrous sodium sulfate, filtered, and concentrated to give a crude product. The crude product was purified by silica gel column chromatography (mobile phase 0-10% methanol/dichloromethane, dichloromethane/methanol = 10/1, Rf = 0.6) to give a product tert-butyl (S)-2-(6-(3-methyl-1*H*-pyrrolo[2,3-*b*]pyridin-5-yl)-2-((tetrahydro-2*H*-pyran-4-yl)methyl)-1,2,3,4-tetrahydroisoquinolin-8-yl)pyrrolidine-1-carboxylate (65 mg, yield: 100%). ES-API: [M+H]⁺=531.3.

Step 3: Trifluoroacetic acid (1 mL) was added to a solution of the compound tert-butyl (S)-2-(6-(3-methyl-1*H*-pyrrolo[2,3-*b*]pyridin-5-yl)-2-((tetrahydro-2*H*-pyran-4-yl)methyl)-1,2,3,4-tetrahydroisoquinolin-8-yl)pyrrolidine-1-carboxylate (50 mg, 0.09 mmol) in dichloromethane (2 mL) and stirred at room temperature for 1 h. The reaction was monitored by LCMS for completion. The reaction mixture was adjusted to pH 7 with saturated aqueous sodium bicarbonate solution, extracted with dichloromethane/water, dried over anhydrous sodium sulfate, filtered, and concentrated to give a crude product. The crude product was purified by a preparative thin layer chromatographic column (dichloromethane/methanol = 8/1) to give (*S*)-6-(3-methyl-1*H*-pyrrolo[2,3-*b*]pyridin-5-yl)-8-(pyrrolidin-2-yl)-2-((tetrahydro-2*H*-pyr an-4-yl)methyl)-1,2,3,4-tetrahydroisoquinoline (10.5 mg, yield: 25.88%) as a yellow powder. ES-API:[M+H]⁺=431.3. ¹H NMR (400 MHz, CDCl₃) δ 9.47 (s, 1H), 8.42 (s, 1H), 7.98 (d, *J* = 2.0 Hz, 1H), 7.63 (s, 1H), 7.21 (s, 1H),7.08 (s, 1H), 4.37 (t, *J* = 5.3 Hz, 1H), 4.02-3.95 (m, 2H), 3.79 (d, *J* = 15.2 Hz, 1H), 3.62 (d, *J* = 15.1 Hz, 1H), 3.47-3.38 (m, 2H), 3.38-3.28 (m, 1H), 3.19-3.09 (m, 1H),3.00-2.93 (m, 2H), 2.73-2.63 (m, 2H), 2.43 (d, *J* = 6.4 Hz, 2H), 2.34-2.31 (m, 3H), 2.28-2.21 (m, 1H), 2.05-1.88 (m, 3H), 1.78-1.69 (m, 3H), 1.47-1.11 (m, 2H).

### Example 207. Synthesis of compound Z433

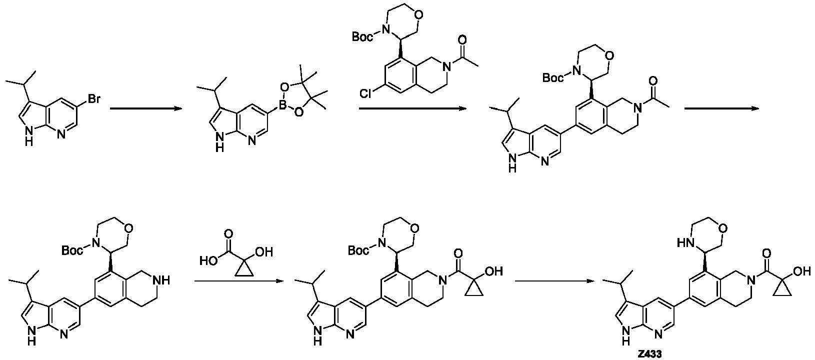

Step 1: 5-Bromo-3-isopropyl-1H-pyrrolo[2,3-b]pyridine (94 mg, 0.4 mmol), bis(pinacolato)diboron (200 mg, 0.8 mmol) and potassium acetate (120 mg, 1.2 mmol) were dissolved in 1,4-dioxane (3 mL). [1,1'-bis(diphenylphosphino)ferrocene]dichloropalladium (20 mg) was added, and the system reacted at 110°C for 2 h in the presence of protective nitrogen. The reaction mixture was concentrated under reduced pressure to give a crude product. The crude product was purified by silica gel column chromatography (petroleum ether/ethyl acetate = 50%-60%) to give 3-isopropyl-5-(4,4,5,5-tetramethyl-1,3,2-dioxaborolan-2-yl)-1H-pyrrolo[2,3-b]pyridine (80 mg, yield: 70%). ES-API: [M+H]⁺=287.2.

Step 2: In the presence of protective nitrogen, a mixture of tert-butyl (R)-3-(2-acetyl-6-chloro-1,2,3,4-tetrahydroisoquinolin-8-yl)morpholine-4-carboxylate (70 mg, 0.17 mmol), the 3-isopropyl-5-(4,4,5,5-tetramethyl-1,3,2-dioxaborolan-2-yl)-1H-pyrrolo[2,3-b]pyridine (80 mg, 0.28 mmol), chloro (2-dicyclohexylphosphino-2',6'-dimethoxy-1,1'-biphenylyl)(2'-amino-1,1'-biphenyl-2-yl) palladium (II) (13 mg, 0.02 mmol) and potassium carbonate (74 mg, 0.54 mmol) in 1,4-dioxane (2 mL) and water (0.4 mL) was stirred at 120°C for 2 h. Upon completion of the reaction, ethyl acetate (20 mL) was poured into the reaction mixture, and the obtained mixture was washed with saturated brine (10 mL) and water (10 mL) successively. The organic phase was dried over anhydrous sodium sulfate, filtered, concentrated, and purified by a flash silica gel column (0-10% methanol/dichloromethane) to give tert-butyl (R)-3-(2-acetyl-6-(3-isopropyl-1H-pyrrolo[2,3-b]pyridin-5-yl)-1,2,3,4-tetrahydroisoquinol in-8-yl)morpholine-4-carboxylate (70 mg, yield: 76%) as a colorless oil. ES-API: [M+H]⁺ = 519.2.

Step 3: The tert-butyl (R)-3-(2-acetyl-6-(3-isopropyl-1*H*-pyrrolo[2,3-*b*]pyridin-5-yl)-1,2,3,4-tetrahydroisoquinol in-8-yl)morpholine-4-carboxylate (70 mg, 0.14 mmol) was dissolved in ethanol (3.5 mL). 5.0 mol/L Sodium hydroxide (2 mL) was added at room temperature, and the system was stirred at 85°C in a sealed tube for 6 h. The reaction was checked by LC-MS for completion. The reaction mixture was extracted with a mixed solution of dichloromethane:methanol (10:1, 10 mL), washed with water (10 mL) and saturated brine (10 mL) successively, dried over anhydrous sodium sulfate, filtered, and concentrated to give a crude product. The crude product was purified by silica gel column chromatography (dichloromethane:methanol containing 5 ‰ ammonia = 3%-5%) to give a product tert-butyl (R)-3-(6-(3-isopropyl-1*H*-pyrrolo[2,3-b]pyridin-5-yl)-1,2,3,4-tetrahydroisoquinolin-8-yl)-morpholine-4-carboxylate (55.3 mg, yield: 83%). ES-API: [M+H]⁺ = 477.2.

Step 4: 1-Hydroxycyclopropane-1-carboxylic acid (11.9 mg, 0.11 mmol) was dissolved in dichloromethane (1 mL). 1-(3-Dimethylaminopropyl)-3-ethylcarbodiimide hydrochloride (21.1 mg, 0.11 mmol) and 1-hydroxybenzotriazole (14.9 mg, 0.11 mmol) were added at room temperature and stirred at room temperature for 3 min. The tert-butyl (R)-3-(6-(3-isopropyl-1*H*-pyrrolo[2,3-b]pyridin-5-yl)-1,2,3,4-tetrahydroisoquinolin-8-yl)-morpholine-4-carboxylate (55.3 mg, 0.11 mol) was then added and stirred at room temperature for 2 h. The reaction was checked by LC-MS for completion, and water (5 mL) was added. The obtained mixture was extracted with a mixed solution of dichloromethane:methanol (10:1, 10 mL×2), dried over anhydrous sodium sulfate, filtered, concentrated, and purified by silica gel column chromatography (dichloromethane:methanol = 3%-6%) to give a product tert-butyl (R)-3-(2-(1-hydroxycyclopropane-1-carbonyl)-6-(3-isopropyl-1*H*-pyrrolo [2,3-b]pyridin-5-yl)-1,2,3,4-tetrahydroisoquinolin-8-yl-morpholine-4-carboxylate (43.2 mg, yield: 70%). ES-API: [M+H]⁺=561.2.

Step 5: The tert-butyl (R)-3-(2-(1-hydroxycyclopropane-1-carbonyl)-6-(3-isopropyl-1*H*-pyrrolo[2,3-b]pyridin-5-yl)-1,2,3,4-tetrahydroisoquinolin-8-yl-morpholine-4-carboxylate (43.2 mg, 0.08 mmol) was dissolved in dichloromethane (4.0 mL). Trifluoroacetic acid (2.0 mL) was added, and the system reacted at room temperature for 2 h. The reaction mixture was concentrated. 7.0M Ammonia-methanol solution (5.0 mL) was added, and a solvent was spun to dryness to give a crude product. The crude product was purified by prep-HPLC (ammonia water) to give (*R*)-(1-hydroxycyclopropyl)(6-(3-isopropyl-1*H*-pyrrolo[2,3-b]pyridin-5-yl)-8-(morpholin-3-yl)-3,4-dihydroisoquinolin-2(1*H*)-yl)methanone (Z433, 15.6 mg, yield: 44%) as a white solid. ES-API: [M+H]⁺=461.2.

### Example 208. Synthesis of compound Z424-1

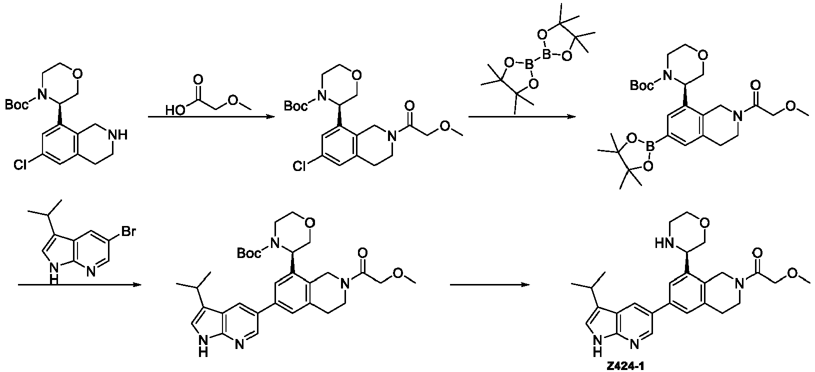

Step 1: N,N-Diisopropylethyl amine (476 mg, 3.68 mmol) and 2-(7-azobenzotriazole)-N,N,N',N'-tetramethylurea hexafluorophosphate (210 mg, 0.55 mmol) were added successively to a solution of tert-butyl (R)-3-(6-chloro-1,2,3,4-tetrahydroisoquinolin-8-yl)morpholine-4-carboxylate (130 mg, 0.37 mmol) and methoxyacetic acid (66 mg, 0.73 mmol) in N,N-dimethylformamide (1 mL) and stirred at room temperature for 2 h. Upon completion of the reaction, the reaction mixture was diluted with ethyl acetate (20 mL) and washed with saturated brine (20 mL). The organic phase was dried over anhydrous sodium sulfate, filtered, concentrated, and purified by a flash silica gel column (0-80% tetrahydrofuran/petroleum ether) to give tert-butyl (R)-3-(6-chloro-2-(2-methoxyacetyl)-1,2,3,4-tetrahydroisoquinolin-8-yl)morpholine-4-car boxylate (130 mg, yield: 83%) as an oil. ES-API: [M+H]⁺ = 425.1.

Step 2: In the presence of protective nitrogen, a mixture of the tert-butyl (R)-3-(6-chloro-2-(2-methoxyacetyl)-1,2,3,4-tetrahydroisoquinolin-8-yl)morpholine-4-car boxylate (80 mg, 0.19 mmol), bis(pinacolato)diboron (96 mg, 0.38 mmol), chloro (2-dicyclohexylphosphino-2',6'-dimethoxy-1,1'-biphenylyl)(2'-amino-1,1'-biphenyl-2-yl) palladium (II) (14 mg, 0.02 mmol) and potassium acetate (55 mg, 0.57 mmol) in 1,4-dioxane (2 mL) was stirred at 110°C for 2 h. Upon completion of the reaction, the reaction mixture was filtered, concentrated, and purified by a flash silica gel column (0-100% tetrahydrofuran/petroleum ether) to give tert-butyl (R)-3-(2-(2-methoxyacetyl)-6-(4,4,5,5-tetramethyl-1,3,2-dioxaborolan-2-yl)-1,2,3,4-tetrah ydroisoquinolin-8-yl)morpholine-4-carboxylate (70 mg, yield: 72%) as a colorless oil. ES-API: [M+H]⁺ = 517.3.

Step 3: In the presence of protective nitrogen, a mixture of the tert-butyl (R)-3-(2-(2-methoxyacetyl)-6-(4,4,5,5-tetramethyl-1,3,2-dioxaborolan-2-yl)-1,2,3,4-tetrah ydroisoquinolin-8-yl)morpholine-4-carboxylate (70 mg, 0.14 mmol), 5-bromo-3-isopropyl-1H-pyrrolo[2,3-b]pyridine (32 mg, 0.14 mmol), chloro (2-dicyclohexylphosphino-2',6'-dimethoxy-1,1'-biphenylyl)(2'-amino-1,1'-biphenyl-2-yl) palladium (II) (10 mg, 0.01 mmol) and potassium carbonate (56 mg, 0.41 mmol) in 1,4-dioxane (2 mL) and water (0.4 mL) was stirred at 110°C for 2 h. Upon completion of the reaction, the reaction mixture was dissolved in ethyl acetate (20 mL) and washed with saturated brine (10 mL) and water (10 mL) successively. The organic phase was dried over anhydrous sodium sulfate, filtered, concentrated, and purified by a flash silica gel column (0-10% methanol/dichloromethane) to give tert-butyl (R)-3-(6-(3-isopropyl-1H-pyrrolo[2,3-b]pyridin-5-yl)-2-(2-methoxyacetyl)-1,2,3,4-tetrahy droisoquinolin-8-yl)morpholine-4-carboxylate (55 mg, yield: 74%) as a colorless oil. ES-API: [M+H]⁺ = 549.3.

Step 4: Trifluoroacetic acid (0.5 mL) was added dropwise to a solution of the tert-butyl (R)-3-(6-(3-isopropyl-1H-pyrrolo[2,3-b]pyridin-5-yl)-2-(2-methoxyacetyl)-1,2,3,4-tetrahy droisoquinolin-8-yl)morpholine-4-carboxylate (20 mg, 34 µmol) in dichloromethane (1 mL) under an ice bath condition and stirred at room temperature for 1 h. The reaction mixture was concentrated, neutralized with 7M amine-methanol (5 mL), concentrated, and then purified by prep-HPLC (ammonium bicarbonate) to give (R)-1-(6-(3-isopropyl-1H-pyrrolo[2,3-b]pyridin-5-yl)-8-(morpholin-3-yl)-3,4-dihydroisoq uinolin-2(1H))-yl)-2-methoxyethan-1-one (Z424-1, 27.32 mg, purity: 100%, yield: 61%) as a white solid. ES-API: [M+H]⁺ = 449.3. ¹H NMR (400 MHz, DMSO-*d₆*) δ 11.42 (s, 1H), 8.62 (d, *J* = 1.6 Hz, 1H), 8.37 (s, 1H), 8.21 - 8.11 (m, 1H), 7.67 (s, 1H), 7.25 (d, *J* = 1.6 Hz, 1H), 5.02 - 4.83 (m, 1H), 4.74 - 4.53 (m, 2H), 4.41 - 4.26 (m, 1H), 4.23 (s, 1H), 4.11 - 3.98 (m, 3H), 3.97 - 3.67 (m, 2H), 3.67 - 3.45 (m, 3H), 3.34 (s, 3H), 3.27 - 3.18 (m, 1H), 3.06 - 2.85 (m, 2H), 1.35 (d, *J* = 3.2 Hz, 3H), 1.33 (d, *J* = 3.2 Hz, 3H).

### Example 209. Synthesis of compound Z418-1

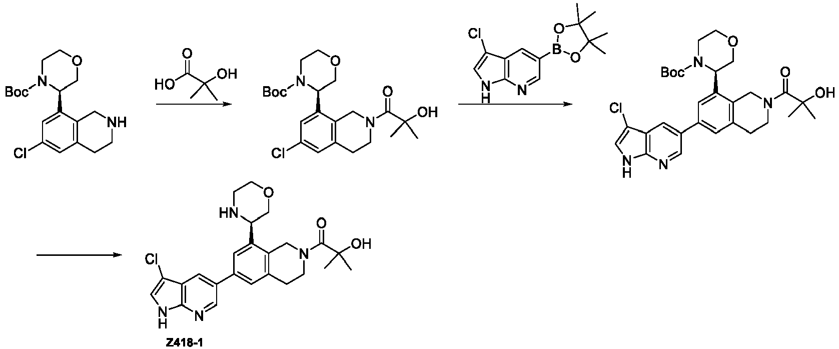

Step 1: tert-butyl (R)-3-(6-chloro-1,2,3,4-tetrahydroisoquinolin-8-yl)morpholine-4-carboxylate (35 mg, 0.1 mmol) and 2-hydroxy-2-methylpropanoic acid (10 mg, 0.1 mmol) was dissolved in N,N-dimethylformamide (1 mL). 2-(7-Azobenzotriazole)-N,N,N',N'-tetramethylurea hexafluorophosphate (57 mg, 0.15 mmol) and N,N-diisopropylethyl amine (40 mg, 0.3 mmol) were added, and the system reacted at room temperature for 2 h. The reaction mixture was poured into water and extracted with ethyl acetate. The organic phases were combined, washed with saturated brine, and concentrated to dryness under reduced pressure to give a crude product. The crude product was purified by silica gel column chromatography (petroleum ether/ethyl acetate = 50/50) to give tert-butyl (R)-3-(6-chloro-2-(2-hydroxy-2-methylpropanoyl)-1,2,3,4-tetrahydroisoquinolin-8-yl)mor pholine-4-carboxylate (35 mg, yield: 79%). ES-API: [M+H]⁺=439.

Step 2: The tert-butyl (R)-3-(6-chloro-2-(2-hydroxy-2-methylpropanoyl)-1,2,3,4-tetrahydroisoquinolin-8-yl)mor pholine-4-carboxylate (35 mg, 0.08 mmol), 3-chloro-5-(4,4,5,5-tetramethyl-1,3,2-dioxaborolan-2-yl)-1H-pyrrolo[2,3-b]pyridine (68 mg, 0.24 mmol), chloro (2-dicyclohexylphosphino-2',6'-dimethoxy-1,1'-biphenylyl)(2'-amino-1,1'-biphenyl-2-yl) palladium (II) (8 mg, 0.012 mmol) and potassium carbonate (48 mg, 0.34 mmol) were dissolved in 1,4-dioxane (2 mL) and water (0.5 mL) and stirred at 110°C for 2 h. The obtained mixture was concentrated to dryness under reduced pressure to give a crude product. The crude product was purified by silica gel column chromatography (petroleum ether/ethyl acetate = 50/50) to give tert-butyl (R)-3-(6-(3-chloro-1H-pyrrolo[2,3-b]pyridin-5-yl)-2-(2-hydroxy-2-methylpropanoyl)-1,2, 3,4-tetrahydroisoquinolin-8-yl)morpholine-4-carboxylate (22 mg, yield: 51%). ES-API: [M+H]⁺=555.

Step 3: The tert-butyl (R)-3-(6-(3-chloro-1H-pyrrolo[2,3-b]pyridin-5-yl)-2-(2-hydroxy-2-methylpropanoyl)-1,2, 3,4-tetrahydroisoquinolin-8-yl)morpholine-4-carboxylate (22 mg, 0.04 mmol) was dissolved in anhydrous dichloromethane (0.5 mL). Trifluoroacetic acid (0.2 mL) was added, and the system reacted at room temperature for 2 h. The reaction was monitored by LC-MS for completion, and the reaction mixture was concentrated to dryness under reduced pressure. After neutralization with ammonia-methanol solution (1 mL), the obtained mixture was concentrated again and purified by prep-HPLC (ammonium bicarbonate) to give (R)-1-(6-(3-chloro-1H-pyrrolo[2,3-b]pyridin-5-yl)-8-(morpholin-3-yl)-3,4-dihydroisoquin olin-2(1H))-yl)-2-methyl-2-hydroxy-propan-1-one (Z418-1, 8.8 mg, yield: 45%) ES-API: [M+H]⁺=455.1. ¹H NMR (400 MHz, CDCl₃) δ 9.33 (s, 1H), 8.45- 8.24 (m, 1H), 8.08 (s, 1H), 7.79- 7.72 (m, 1H), 7.36 (s, 1H), 5.31- 5.26 (m, 1H), 4.91- 4.72 (m, 1H), 4.25- 4.18 (m, 1H), 4.09- 4.04 (m, 1H), 3.97- 3.85 (m, 2H), 3.75- 3.71 (m, 1H), 3.53- 3.48 (m, 1H), 3.28- 3.22 (m, 1H), 3.11- 2.93(m, 2H), 1.77- 1.67(m, 2H), 1.63 (s, 6H).

### Example 210. Synthesis of compound Z374

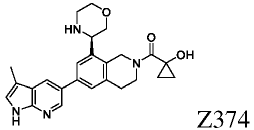

Referring to the synthesis procedure of compound Z372, except replacing 2-hydroxy-2-methylpropanoic acid in step 1 with 1-hydroxycyclopropane carboxylic acid, compound Z374 was finally obtained. ES-API: [M+H]⁺ = 433.2.

### Example 211. Synthesis of compound Z419

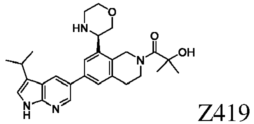

Referring to the synthesis procedure of compound Z372, except replacing 3-methyl-5-(4,4,5,5-tetramethyl-1,3,2-dioxaborolan-2-yl)-1H-pyrrolo[2,3-b]pyridine with 3-isopropyl-5-(4,4,5,5-tetramethyl-1,3,2-dioxaborolan-2-yl)-1H-pyrrolo[2,3-b]pyridine, compound Z419 was finally obtained. ES-API: [M+H]⁺ = 463.3.

### Example 212. Synthesis of compound Z422

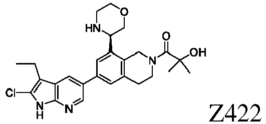

Referring to the synthesis procedure of compound Z372, except replacing 3-methyl-5-(4,4,5,5-tetramethyl-1,3,2-dioxaborolan-2-yl)-1H-pyrrolo[2,3-b]pyridine in step 2 with 2-chloro-3-ethyl-5-(4,4,5,5-tetramethyl-1,3,2-dioxaborolan-2-yl)-1H-pyrrolo[2,3-b]pyridi ne, compound Z422 was finally obtained. ES-API: [M+H]⁺ = 483.2.

### Example 213. Synthesis of compound Z425

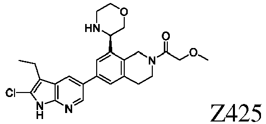

Referring to the synthesis procedure of compound Z424-1, except replacing 5-bromo-3-isopropyl-1H-pyrrolo[2,3-b]pyridine in step 3 with 5-bromo-2-chloro-3-ethyl-1H-pyrrolo[2,3-b]pyridine, compound Z425 was finally obtained. ES-API: [M+H]⁺ = 469.2.

### Example 214. Synthesis of compound Z428

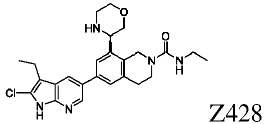

Referring to the synthesis procedure of compound Z376, except replacing 3-methyl-5-(4,4,5,5-tetramethyl-1,3,2-dioxaborolan-2-yl)-1H-pyrrolo[2,3-b]pyridine in step 4 with 2-chloro-3-ethyl-5-(4,4,5,5-tetramethyl-1,3,2-dioxaborolan-2-yl)-1H-pyrrolo[2,3-b]pyridi ne, compound Z428 was finally obtained. ES-API: [M+H]⁺ =468.2.

### Example 215. Synthesis of compound Z429

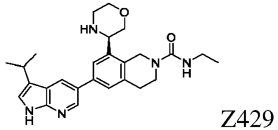

Referring to the synthesis procedure of compound Z376, except replacing 3-methyl-5-(4,4,5,5-tetramethyl-1,3,2-dioxaborolan-2-yl)-1H-pyrrolo[2,3-b]pyridine in step 4 with 3-isopropyl-5-(4,4,5,5-tetramethyl-1,3,2-dioxaborolan-2-yl)-1H-pyrrolo[2,3-b]pyridine, compound Z429 was finally obtained. ES-API: [M+H]⁺ = 448.3.

### Example 216. Synthesis of compound Z430

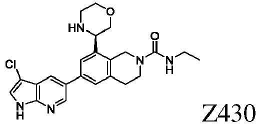

Referring to the synthesis procedure of compound Z376, except replacing 3-methyl-5-(4,4,5,5-tetramethyl-1,3,2-dioxaborolan-2-yl)-1H-pyrrolo[2,3-b]pyridine in step 4 with 3-chloro-5-(4,4,5,5-tetramethyl-1,3,2-dioxaborolan-2-yl)-1H-pyrrolo[2,3-b]pyridine, compound Z430 was finally obtained. ES-API: [M+H]⁺ =440.2.

### Example 217. Synthesis of compound Z435

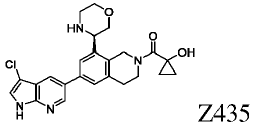

Referring to the synthesis procedure of compound Z433, except replacing 5-bromo-3-isopropyl-1H-pyrrolo[2,3-b]pyridine in step 1 with 5-bromo-3-chloro-1H-pyrrolo[2,3-b]pyridine, compound Z435 was finally obtained. ES-API: [M+H]⁺ =453.2.

### Example 218. Synthesis of compound Z410

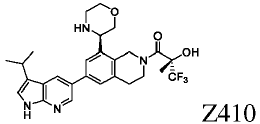

Referring to the synthesis procedure of compound Z424-1, except replacing methoxyacetic acid in step 1 with (S)-3,3,3-trifluoro-2-hydroxy-2-methylpropanoic acid, compound Z410 was finally obtained. ES-API: [M+H]⁺ =517.2.

### Example 219. Synthesis of compound Z413

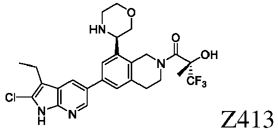

Referring to the synthesis procedure of compound Z424-1, except replacing 5-bromo-3-isopropyl-1H-pyrrolo[2,3-b]pyridine in step 3 with 5-bromo-2-chloro-3-ethyl-1H-pyrrolo[2,3-b]pyridine, compound Z413 was finally obtained. ES-API: [M+H]⁺ =537.2.

### Example 220. Synthesis of compound Z406

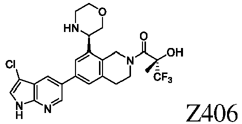

Referring to the synthesis procedure of compound Z418-1, except replacing 2-hydroxy-2-methylpropanoic acid in step 1 with (S)-3,3,3-trifluoro-2-hydroxy-2-methylpropanoic acid, compound Z406 was finally obtained. ES-API: [M+H]⁺ =509.2.

### Example 221. Synthesis of compound Z373

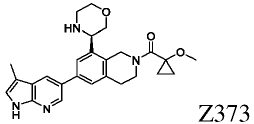

Referring to the synthesis procedure of compound Z372, except replacing 2-hydroxy-2-methylpropanoic acid in step 1 with 1-methoxycyclopropane-1-carboxylic acid, compound Z373 was finally obtained. ES-API: [M+H]⁺ =447.2.

### Example 222. Synthesis of compound Z444

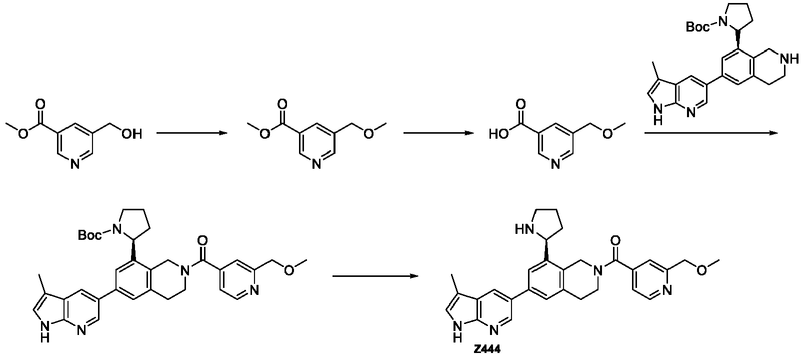

Step 1: Sodium hydride (313.42 mg, 13.06 mmol) was added to a solution of methyl 5-(hydroxymethyl)nicotinate (1.0 g, 6.53 mmol) in N,N-dimethylformamide (10 mL) at 0°C and stirred for 1 h. Iodomethane (813 µL, 13.06 mmol) was then added and stirred for 1 h. The reaction was monitored by LCMS for completion, and quenched by the addition of water. The reaction mixture was extracted with dichloromethane, dried over anhydrous sodium sulfate, filtered, and concentrated to give a crude product. The crude product was purified by silica gel column chromatography (petroleum ether: ethyl acetate = 9:1-1:1) to give a product methyl 5-(methoxymethyl)nicotinate (700 mg, yield: 64.13%). ES-API:[M+H]⁺=182.1.

Step 2: Sodium hydroxide (334.98 mg, 8.38 mmol) was added to a solution of the methyl 5-(methoxymethyl)nicotinate (0.7 g, 4.19 mmol) in methanol (12 mL) and water (8 mL). The mixture was stirred at 50°C for 2 h. The reaction was monitored by LCMS for completion, and the reaction mixture was concentrated. 1N aqueous hydrochloric acid solution was added to give a solid. The obtained mixture was filtered to give 5-(methoxymethyl)pyridine-3-carboxylic acid (0.5 g, crude product) as a white solid. ES-API:[M+H]⁺=168.0.

Step 3: The 5-(methoxymethyl)pyridine-3-carboxylic acid (17.39 mg, 104.03 µmol), 1-propylphosphonic anhydride (133.5 mg, 208.07 µmol, 50% in ethyl acetate) and triethylamine (21.01 mg, 208.07 µmol) were added to a solution of tert-butyl (S)-2-[6-(3-methyl-1*H*-pyrrolo[2,3-*b*]pyridin-5-yl)-1,2,3,4-tetrahydroisoquinolin-8-yl]pyrr olidine-1-carboxylate (0.03 g, 69.36 µmol) in dichloromethane (1.5 mL) and stirred at room temperature for 2 h. The reaction was monitored by LCMS for completion. The reaction mixture was adjusted to pH 7 with saturated aqueous sodium bicarbonate solution, extracted with dichloromethane/water, dried over anhydrous sodium sulfate, filtered, and concentrated to give a crude product. The crude product was purified by a preparative thin layer chromatographic column (dichloromethane:methanol = 20:1) to give a product tert-butyl (S)-2-(2-(2-(methoxymethyl)isonicotinoyl)-6-(3-methyl-1H-pyrrolo[2,3-b]pyridin-5-yl)-1, 2,3,4-tetrahydroisoquinolin-8-yl)pyrrolidine-1-carboxylate (17 mg, yield: 42.14%) as a yellow oil. ES-API:[M+H]⁺= 582.3.

Step 4: Trifluoroacetic acid (1 mL) was added to a solution of the tert-butyl (S)-2-(2-(2-(methoxymethyl)isonicotinoyl)-6-(3-methyl-1H-pyrrolo[2,3-b]pyridin-5-yl)-1, 2,3,4-tetrahydroisoquinolin-8-yl)pyrrolidine-1-carboxylate (0.014 g, 24.07 µmol) in dichloromethane (1 mL) and stirred at 30°C for 2 h. The reaction was monitored by LCMS for completion, and the reaction mixture was concentrated to give a crude product. The crude product was purified by a preparative thin layer chromatographic column (dichloromethane: methanol = 10:1) to give (*S*)-[2-(methoxymethyl)pyridyl-4-yl]-[6-(3-methyl-1*H*-pyrrolo[2,3-*b*]pyridin-5-yl)-8-[pyrr olidin-2-yl]-3,4-dihydroisoquinolin-2(1H)-yl]methanone (5.00 mg, yield: 42.41%) as a white solid. ES-API:[M+H]⁺= 482.2.

Referring to the preparation methods of the above Examples, the compounds shown in Table (A) were prepared by modifying some of the starting materials:

**Table (A)**

| Structure and Number | MS [M+H]⁺ | Structure and Number | MS [M+H]⁺ | Structure and Number | MS [M+H]⁺ |
|---|---|---|---|---|---|
| 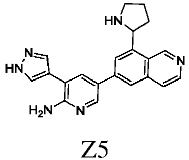 | 357.2 | 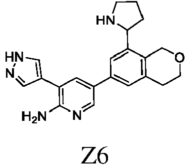 | 362.2 | 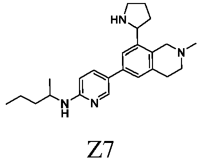 | 379.3 |
| 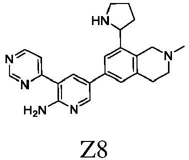 | 387.2 | 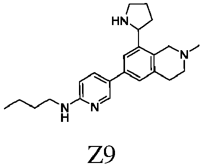 | 365.3 | 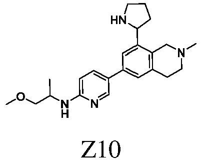 | 381.3 |
| 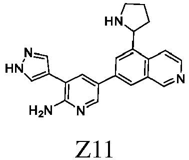 | 357.2 | 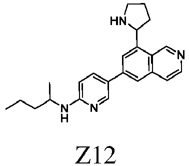 | 361.2 | 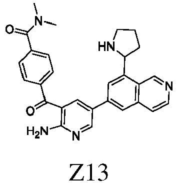 | 466.2 |
| 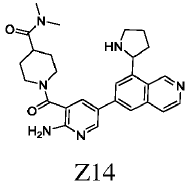 | 473.2 | 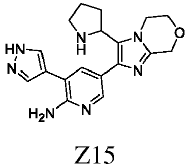 | 351.2 | 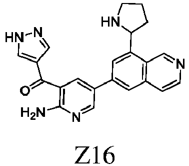 | 385.2 |
| 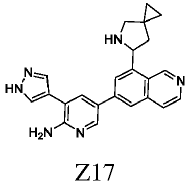 | 383.2 | 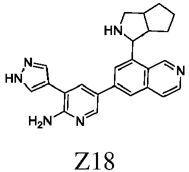 | 397.2 | 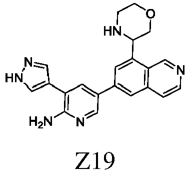 | 373.2 |
| 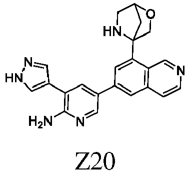 | 385.2 | 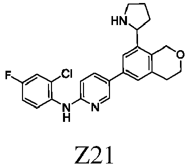 | 424.2 | 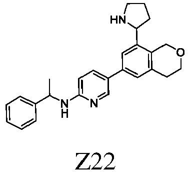 | 400.2 |
| 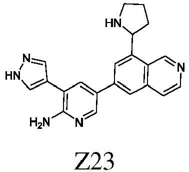 | 357.2 | 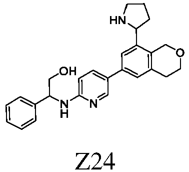 | 416.2 | 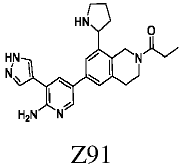 | 417.2 |
| 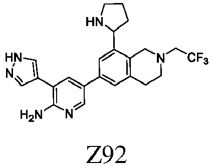 | 443.2 | 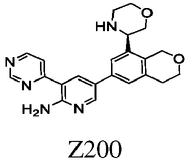 | 390.2 | 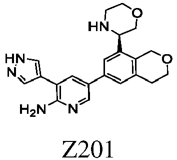 | 378.2 |
| 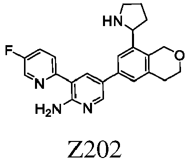 | 391.2 | 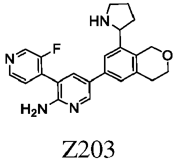 | 391.2 | 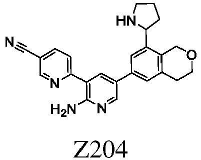 | 398.2 |
| 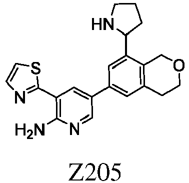 | 379.2 | 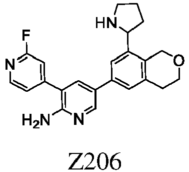 | 391.2 | 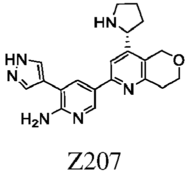 | 363.2 |
| 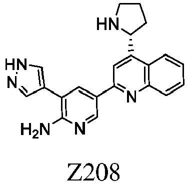 | 357.2 | 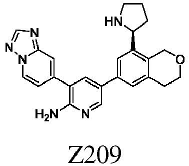 | 413.2 | | |

Referring to the preparation methods of the above Examples, the target compounds shown in Table (B) were prepared by modifying some of the starting materials:

**Table (B)**

| Structure and Number | MS [M+H] + | Structure and Number | MS [M+H] + | Structure and Number | MS [M+H] + |
|---|---|---|---|---|---|
| 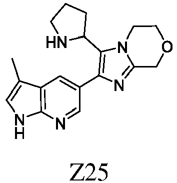 | 324.2 | 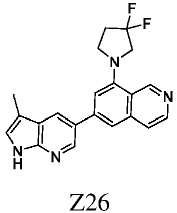 | 365.1 | 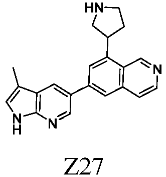 | 329.2 |
| 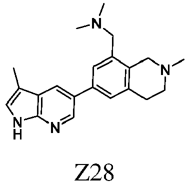 | 335.2 | 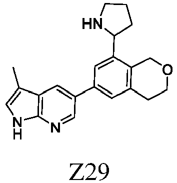 | 334.2 | 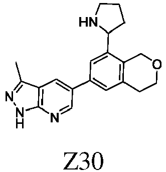 | 335.2 |
| 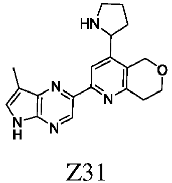 | 336.2 | 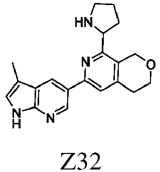 | 335.2 | 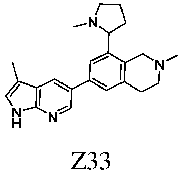 | 361.2 |
| 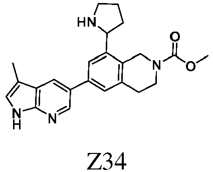 | 391.2 | 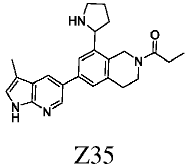 | 389.2 | 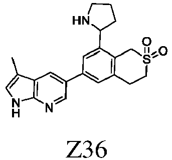 | 382.2 |
| 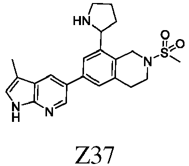 | 411.2 | 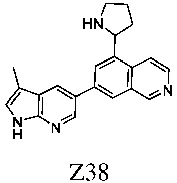 | 329.2 | 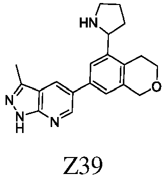 | 335.2 |
| 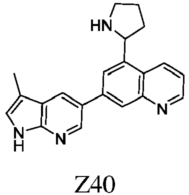 | 329.2 | 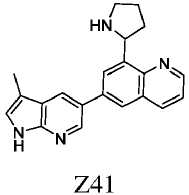 | 329.2 | 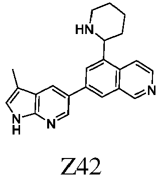 | 343.2 |
| 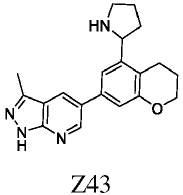 | 335.2 | 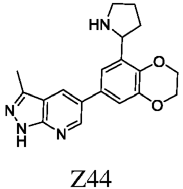 | 337.2 | 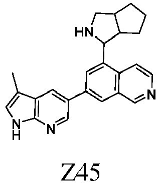 | 369.2 |
| 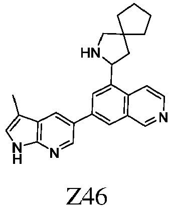 | 383.2 | 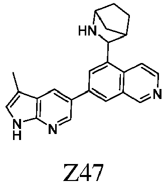 | 355.2 | 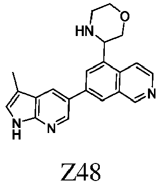 | 345.2 |
| 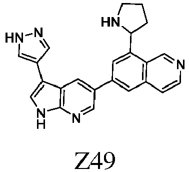 | 381.2 | 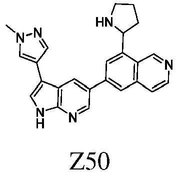 | 395.2 | 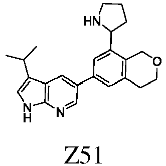 | 362.2 |
| 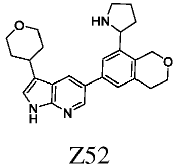 | 404.2 | 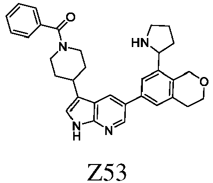 | 507.3 | 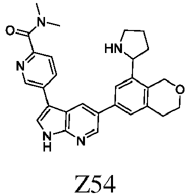 | 468.2 |
| 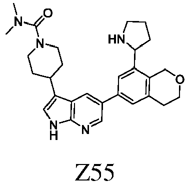 | 474.3 | 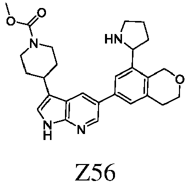 | 461.2 | 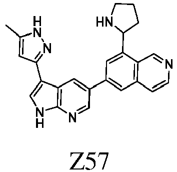 | 395.2 |
| 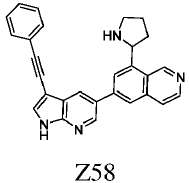 | 415.2 | 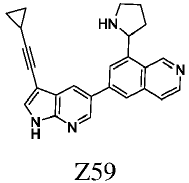 | 379.2 | 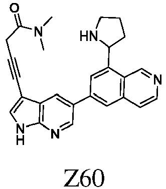 | 424.2 |
| 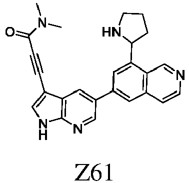 | 410.2 | 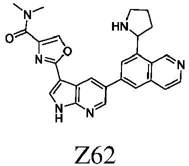 | 453.2 | 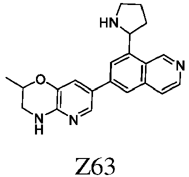 | 347.2 |
| 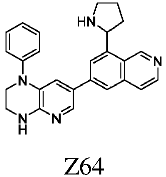 | 408.2 | 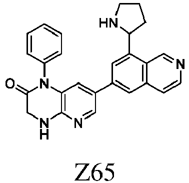 | 422.2 | 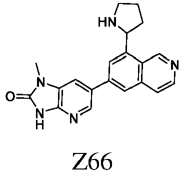 | 346.2 |
| 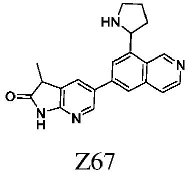 | 345.2 | 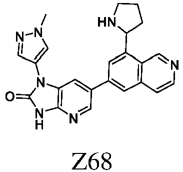 | 412.2 | 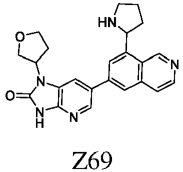 | 402.2 |
| 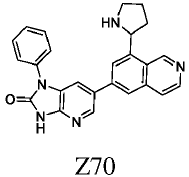 | 408.2 | 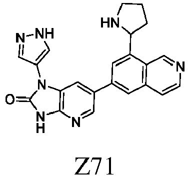 | 398.2 | 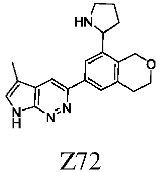 | 335.2 |
| 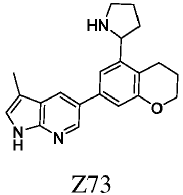 | 334.2 | 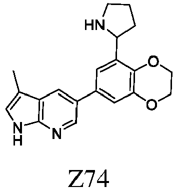 | 336.2 | 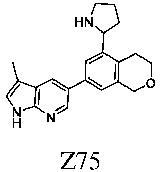 | 334.2 |
| 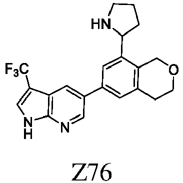 | 338.2 | 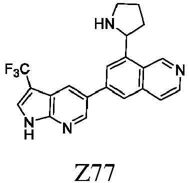 | 383.1 | 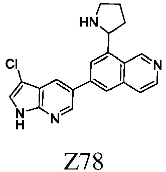 | 349.1 |
| 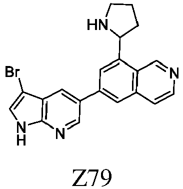 | 393.1 | 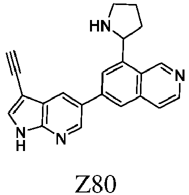 | 339.2 | 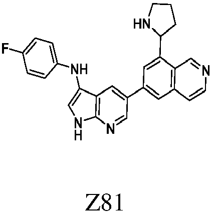 | 424.2 |
| 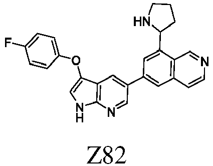 | 425.2 | 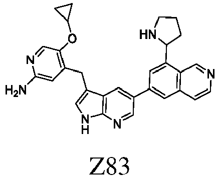 | 477.2 | 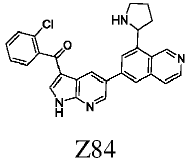 | 453.1 |
| 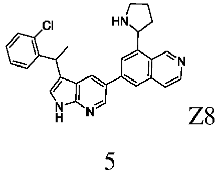 | 453.2 | 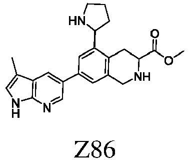 | 391.2 | 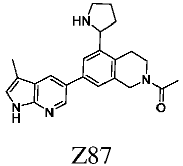 | 375.2 |
| 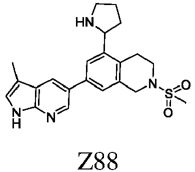 | 411.2 | 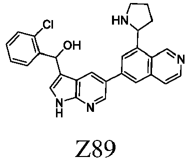 | 455.2 | 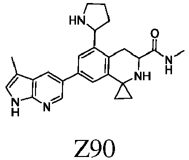 | 416.2 |
| 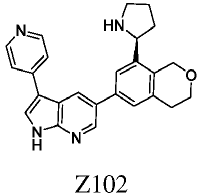 | 397.2 | 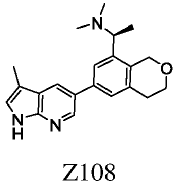 | 336.2 | 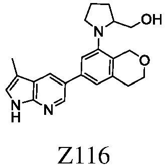 | 364.2 |
| 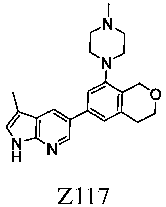 | 363.2 | 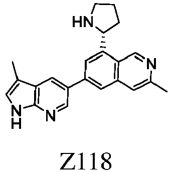 | 343.2 | 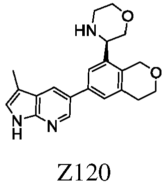 | 350.2 |
| 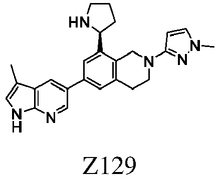 | 413.2 | 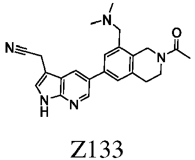 | 388.2 | 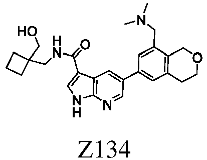 | 449.3 |
| 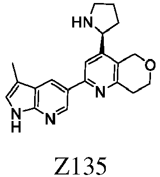 | 335.2 | 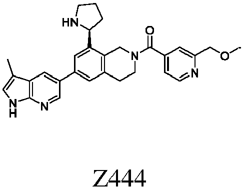 | 481.3 | 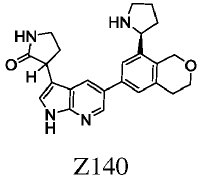 | 403.2 |
| 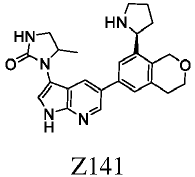 | 418.2 | 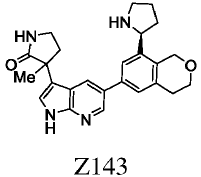 | 417.2 | 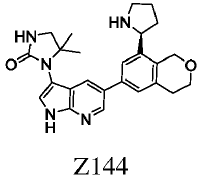 | 432.2 |
| 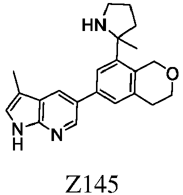 | 348.2 | 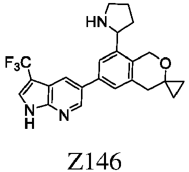 | 414.2 | 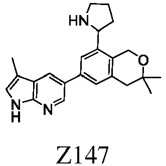 | 362.2 |
| 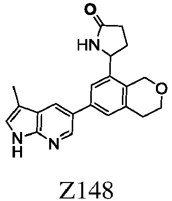 | 348.2 | 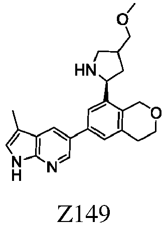 | 378.2 | 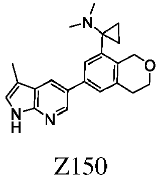 | 348.2 |
| 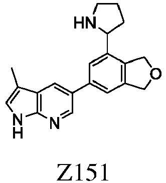 | 320.2 | 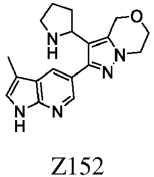 | 324.2 | 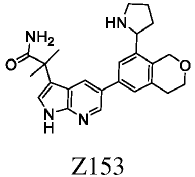 | 405.2 |
| 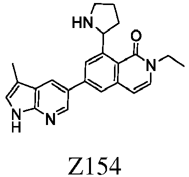 | 373.2 | 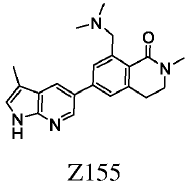 | 349.2 | 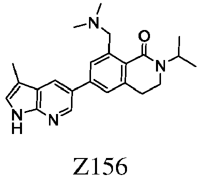 | 377.2 |
| 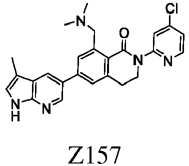 | 446.2 | 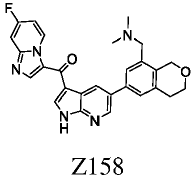 | 470.2 | 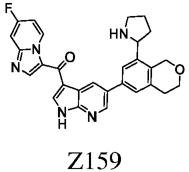 | 482.2 |
| 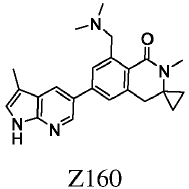 | 375.2 | 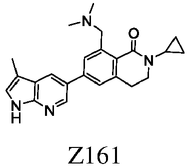 | 375.2 | 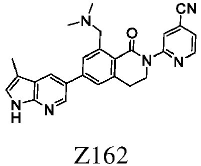 | 437.2 |
| 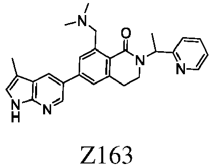 | 440.2 | 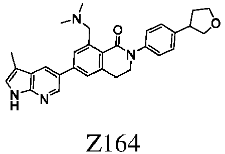 | 481.3 | 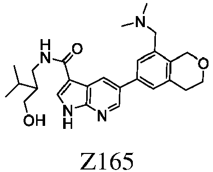 | 451.3 |
| 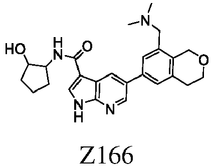 | 435.2 | 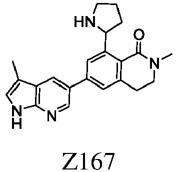 | 361.2 | 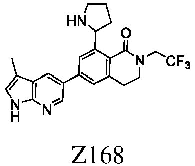 | 429.2 |
| 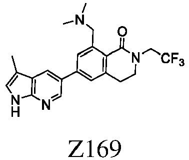 | 417.2 | 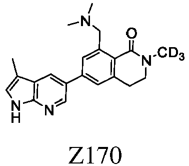 | 352.2 | 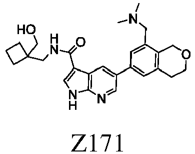 | 449.3 |
| 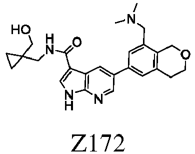 | 435.2 | 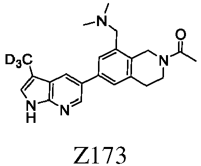 | 366.2 | 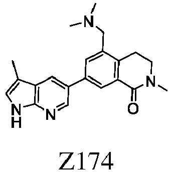 | 349.2 |
| 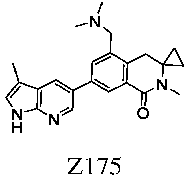 | 375.2 | 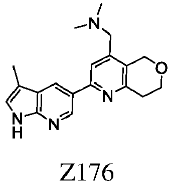 | 323.2 | 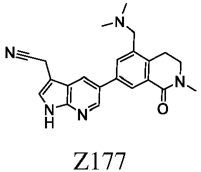 | 374.2 |
| 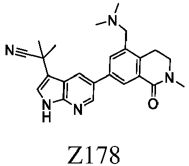 | 402.2 | 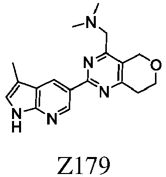 | 324.2 | 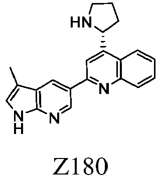 | 329.2 |
| 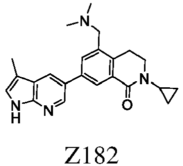 | 375.2 | 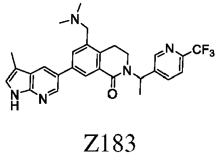 | 508.2 | 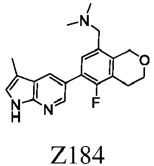 | 340.2 |
| 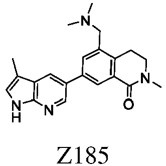 | 349.2 | 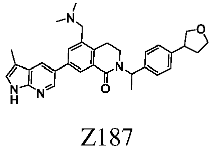 | 509.3 | 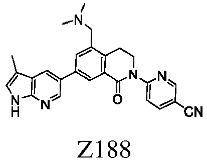 | 437.2 |
| 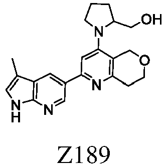 | 365.2 | 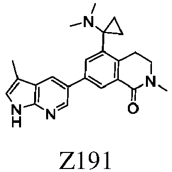 | 375.2 | 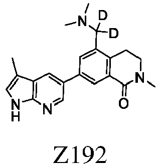 | 351.2 |
| 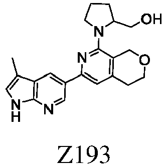 | 365.2 | 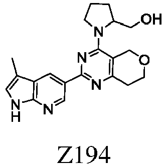 | 366.2 | 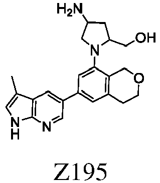 | 379.2 |
| 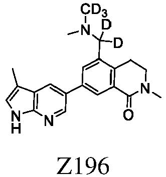 | 354.2 | 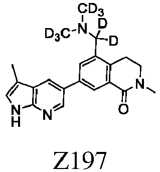 | 357.3 | 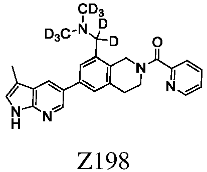 | 434.3 |
| 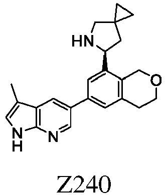 | 360.2 | 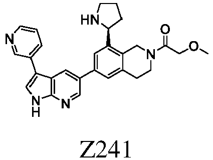 | 468.2 | 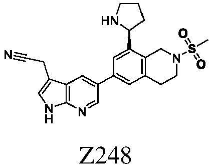 | 436.2 |
| 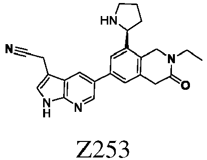 | 400.2 | 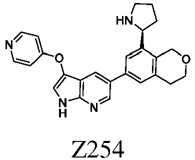 | 413.2 | 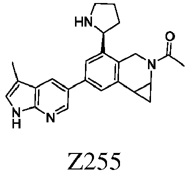 | 387.2 |
| 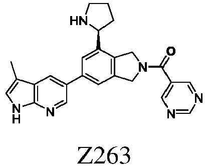 | 425.2 | 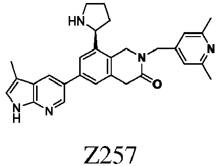 | 466.2 | 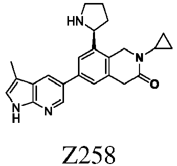 | 387.2 |
| 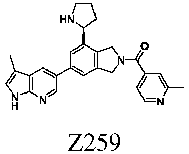 | 438.2 | 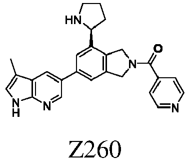 | 424.2 | 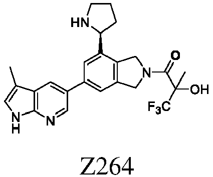 | 459.2 |
| 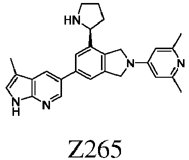 | 424.2 | 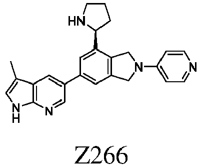 | 396.2 | 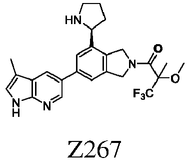 | 473.2 |
| 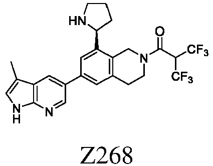 | 511.2 | 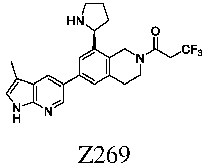 | 443.2 | 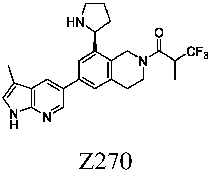 | 457.2 |
| 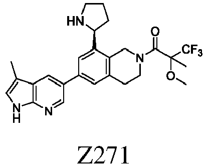 | 487.2 | 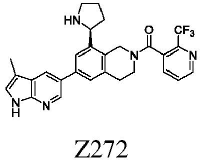 | 506.2 | 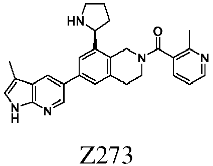 | 452.2 |
| 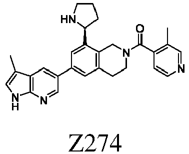 | 452.2 | 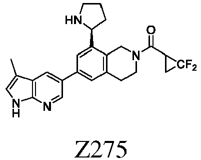 | 437.2 | 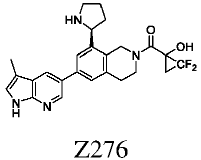 | 453.2 |
| 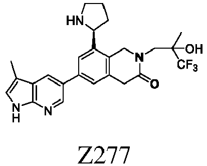 | 473.2 | 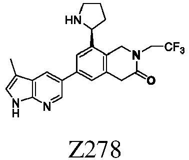 | 429.2 | 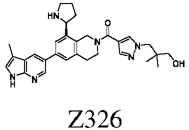 | 513.3 |
| 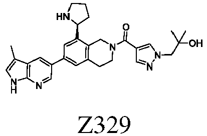 | 499.3 | 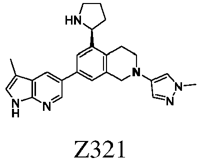 | 413.2 | 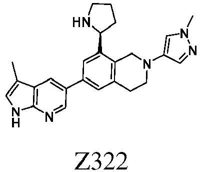 | 413.2 |
| 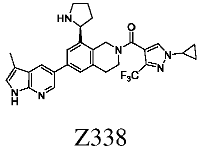 | 363.2 | 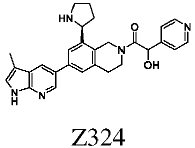 | 468.2 | 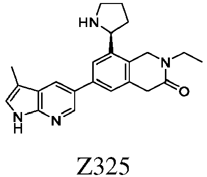 | 391.2 |
| 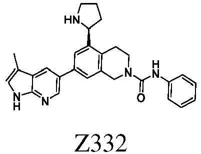 | 452.2 | 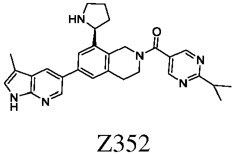 | 481.2 | 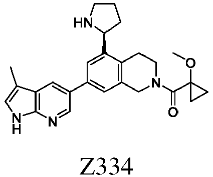 | 431.2 |
| 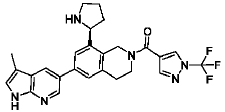 | 495.2 | 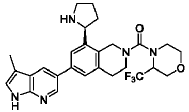 | 514.2 | 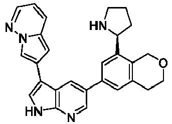 | 436.2 |
| 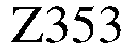 | | 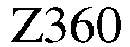 | | 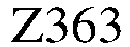 | |
| 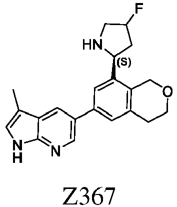 | 352.2 | 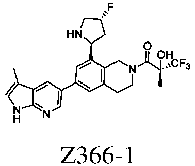 | 491.2 | 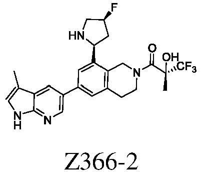 | 491.2 |
| 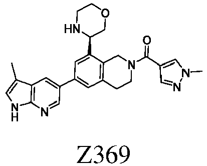 | 457.2 | 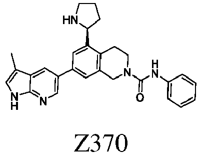 | 452.2 | 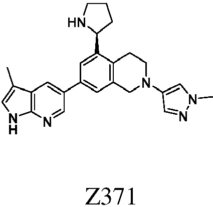 | 413.2 |
| 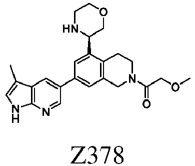 | 421.2 | 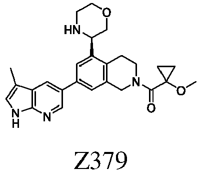 | 447.2 | 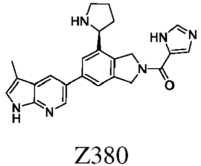 | 413.2 |
| 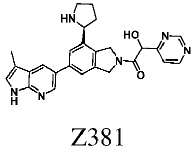 | 455.2 | 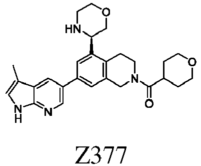 | 461.2 | 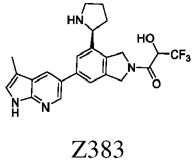 | 445.2 |
| 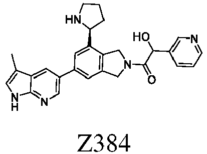 | 454.2 | 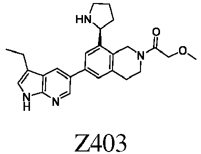 | 419.2 | 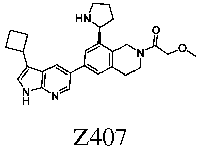 | 445.3 |
| 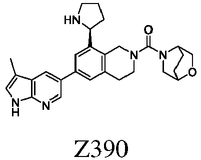 | 472.3 | 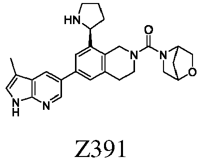 | 458.3 | 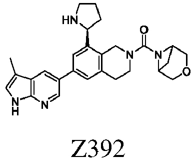 | 458.3 |
| 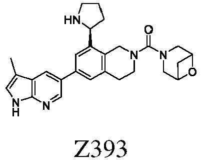 | 458.3 | 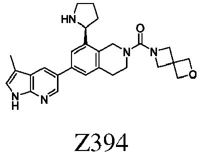 | 458.3 | 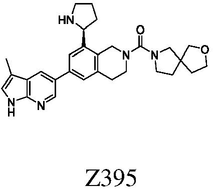 | 486.3 |
| 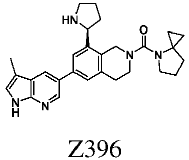 | 456.3 | 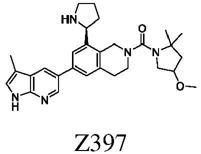 | 488.3 | 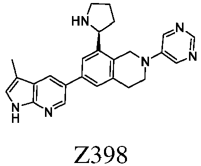 | 411.2 |
| 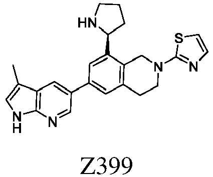 | 416.2 | 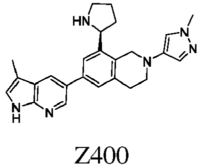 | 413.2 | 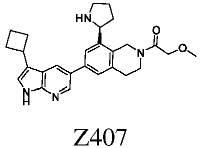 | 445.3 |
| 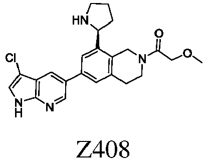 | 425.2 | 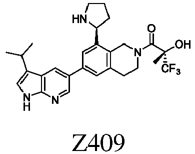 | 501.2 | 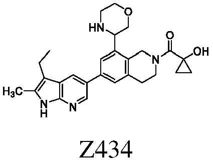 | 461.3 |
| 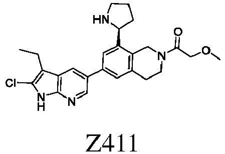 | 453.2 | 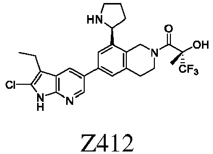 | 521.2 | 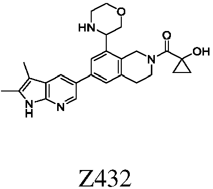 | 447.2 |
| 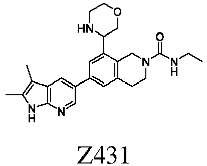 | 434.3 | 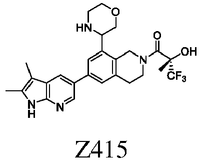 | 503.2 | 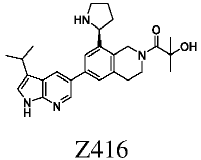 | 447.3 |
| 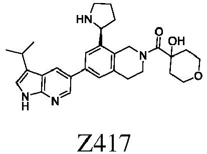 | 489.3 | 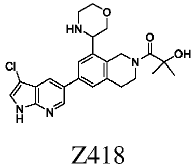 | 455.2 | 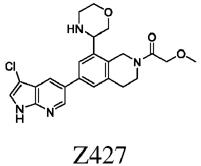 | 441.2 |
| 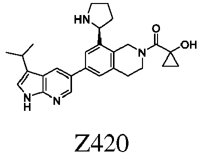 | 445.3 | 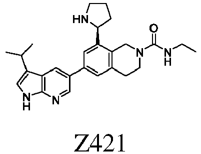 | 432.3 | 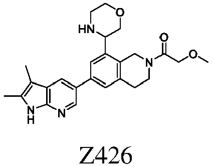 | 435.2 |
| 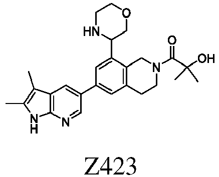 | 449.3 | 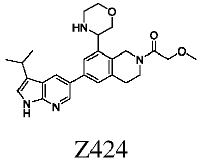 | 449.3 | | |

### Test Example 1. SLP76 phosphorylation assay

RPMI-1640: purchased from Gibco; Cat. No: 11875-093; FBS: purchased from Gibco; Cat. No: 10099-141C; PS: purchased from Gibco; Cat. No: 15140-122; PBS: purchased from Hyclone; Cat. No: SH30256.01; EDTA, 0.5M, pH 8.0: purchased from absin; Cat. No: abs9133; Dynabeads anti-human CD3: purchased from Thermo Fisher; Cat. No: 11151D; 96 well F-bottom TC plate: purchased from Corning; Cat. No: 3599; 96 well V-storage plate: purchased from Corning; Cat. No: 3894; Halt Protease Inhibitor Cocktail, EDTA-Free (100X): purchased from Thermo Fisher; Cat. No: 78439; PathScan Phospho-SLP-76 (Ser376) Sandwich ELISA Kit: purchased from CST; Cat. No: 78222CA;
Intracellular HPK1 inhibitory activity was tested by the detection of SLP76 phosphorylation at S376. The cells used in this experiment were Jurkat, Clone E6-1 which expressed HPK1 and SLP76.

Cell culture: Jurkat, Clone E6-1 cells (purchased from ATCC; Cat. No: TIB-512; complete medium: RPMI-1640+10% FBS+1% PS), in RPMI-1640 complete medium containing 10% fetal bovine serum and 1% double antibiotics (a mixed solution of penicillin and streptomycin), were placed in a 5% CO₂ incubator at 37°C for culture, passed 2-3 times per week, and maintained a culture density at 1×10⁵/mL ~ 1×10⁶/mL.

Preparation of 1000× drug stock plate: a 1000× compound stock plate was prepared. 10 mM or 5 mM of compound stock solution was diluted with DMSO, with an initial concentration of 10 mM (5 mM), 9 concentration gradients, and 3.162-fold dilution. The compound stock plate was sealed with a sealing film and stored in a -20°C refrigerator for later use.

Preparation of Beads wash buffer: 0.1 mL FBS and 0.4 mL 0.5 M EDTA (with a final concentration of 2 mM) were added to 100 mL PBS and stored in a 4°C refrigerator for later use.

Preparation of 5× protein lysis buffer: 10× Cell lysis buffer was taken and diluted to 5× with deionized water. 100× Protease Inhibitor Cocktail in 1/20 total volume was added and stored on wet ice for later use. The buffer was ready to use.

Cell inoculation and dosing: the 1000× drug stock plate was taken and melted at room temperature in the dark. Then 10× drug intermediate plate was prepared using fresh RPMI-1640 complete medium and mixed well. Jurkat cells in the logarithmic growth phase were selected and collected. After centrifugation, the pellet was resuspended using fresh complete medium, counted, and adjusted the cell density to 2.22×10⁶/mL. The cells were inoculated into a 96-well plate (the volume was 90 µL per well), and a blank control well (without cells inoculation, and in which 110 µL of medium was added) was set. 10 µL of a drug-containing culture medium (10×) was transferred from the 10× drug intermediate plate into the 96-well cell plate (with a final concentration of 0.1% DMSO), and set a DMSO control group (in which 0.1% DMSO was added to the treated cells), a stimulated control group (in which 10 µL of culture medium was added to treat cells) and an unstimulated group (in which 10 µL of culture medium was added to treat cells). The wells were duplicated, gently patted, mixed, and then placed into a 5% CO₂ incubator at 37°C for 1 h.

Anti-CD3 beads stimulation: Dynabeads anti-human CD3 was taken, shaken, and mixed for 60 s. The required volume of beads was pipetted, and an equal volume or at least 1 mL of washing solution was added to wash once. Fresh complete medium was then added to resuspend and adjust the density of the beads to 4×10⁷/mL, and the beads were placed on ice for later use. At the end of drug pre-incubation, the cell culture plate was taken out. 10 µL Dynabeads anti-human CD3 was added to each well to treat the cells, and 10 µL the culture medium was added to the unstimulated group. The cell culture plate was shaken to mix well at 2500 rpm for 10 s, and then placed into a 5% CO₂ incubator at 37°C and continued to culture for 20 min.

Detection of p-SLP76 protein level: PathScan Phospho-SLP-76 (Ser376) Sandwich ELISA Kit was applied to detect p-SLP76 protein level. At the end of the Anti-CD3 beads stimulation, the cell culture plate was removed from the incubator. 27 µL of 5× protein lysis buffer pre-chilled on ice was added to each well, and the plate was placed on ice for 30 min. After lysis, the cell culture plate was centrifuged at 4°C, 400× g for 5 min. 25 µL of sample and 75 µL of sample diluent were added to each well, and the plate was placed in a 5% CO₂ incubator at 37°C for 2 h. The subsequent detection steps were carried out following the kit instructions, and the absorbance (OD value) of each well was read at 450 nm by a microplate reader.

Data analysis processing: the inhibition rate of p-SLP76 protein level was calculated according to the following formula: Inhibition rate%=[1-(OD _{Compound}-OD _{Blank control}) / (ODDMSO _{control} - OD _{Blank control})] × 100%, wherein OD _{Compound} is the reading of the well treated with compound , OD _{Blank control} is the reading of the blank control well, and OD _{DMSO control} is the reading of the cells DMSO control well. The "Dose response one site/f(x) 205[fit=(A+((B-A)/(1+((C/x)^AD))))]" model was used to fit the pharmacodynamics inhibition rate curves with XLfit 5.5.x software, and the IC₅₀ values of the drugs were calculated.

It may be seen from the experimental results that the compounds of the present disclosure have a high inhibitory activity on SLP76 phosphorylation, with an IC₅₀ value of less than 5000 nM (e.g. 1 nM to 5000 nM); IC₅₀ values for some compounds are even less than 3000 nM (e.g. 1 nM to 3000 nM) or less than 1000 nM (e.g. 1 nM to 1000 nM). The experimental results of some compounds are shown in Table 1.

**Table 1**

| Number | p-SLP76 IC₅₀(nM) | Number | p-SLP76 IC₅₀(nM) | Number | p-SLP76 IC₅₀(nM) | Number | p-SLP76 IC₅₀(nM) |
|---|---|---|---|---|---|---|---|
| Z2 | 2897.1 | Z3 | 853.1 | Z3-1 | > 10000 | Z3-2 | 603.8 |
| Z4 | 3574.9 | Z93 | 438.6 | Z93-2 | 235.0 | Z94 | 2846.4 |
| Z94-2 | 1388.0 | Z95 | 1077.0 | Z95-2 | 623.3 | Z96 | 1001.1 |
| Z97 | 377.0 | Z97-2 | 164.3 | Z98 | 237.3 | Z98-2 | 151.5 |
| Z100 | 211.6 | Z100-2 | 327.9 | Z101 | 162.6 | Z101-2 | 110.9 |
| Z103 | 545.4 | Z104 | 77.0 | Z105 | 198.5 | Z106 | 132.8 |
| Z107 | 525.3 | Z223 | 301.0 | Z225 | 1337.0 | Z104-2 | 196.9 |
| Z137 | 159.41 | Z199 | 116.6 | Z181 | 1705.0 | Z228-1 | 84.4 |
| Z136 | 7909.7 | Z229 | 275.7 | Z231 | > 10000 | Z109 | 1061.0 |
| Z112 | 304.9 | Z110 | 2838.1 | Z108-1 | 570.7 | Z236 | 3810.4 |
| Z237 | 100.2 | Z138 | 132.1 | Z244 | 96.58 | Z238 | 354.42 |
| Z139 | 158.7 | Z251 | 131.41 | Z250 | 193.07 | Z243 | 2508.09 |
| Z219-2 | 122.06 | Z279 | 161.97 | Z271-2 | 379.75 | Z281 | 2029.08 |
| Z283 | 285.8 | Z285 | 322.74 | Z132 | 352.2 | Z121 | 185.2 |
| Z122 | 179.1 | Z126 | 252.0 | Z125 | 301.0 | Z123 | 343.0 |
| Z124 | 260.1 | Z127 | 204.29 | Z130 | 1805.4 | Z210 | 226.9 |
| Z211 | 463.1 | Z212 | 909.4 | Z119 | 358.0 | Z213 | 318.2 |
| Z214 | 180.5 | Z215 | 443.6 | Z216 | 107.5 | Z34-1 | 265.4 |
| Z119-1 | 390.2 | Z54-1 | 400.6 | Z217 | 133.0 | Z37-1 | 143.3 |
| Z218 | 367.4 | Z219 | 257.4 | Z220 | 469.0 | Z221 | 215.0 |
| Z222 | 201.0 | Z224 | 333.0 | Z104-1 | 126.2 | Z186 | 3760.6 |
| Z190 | 494.43 | Z226 | 10697.0 | Z227 | 118.6 | Z228-2 | 49.3 |
| Z131 | 663.79 | Z230 | 437.7 | Z232 | 1658.59 | Z111 | 814.7 |
| Z113 | 10005.8 | Z233 | 520.0 | Z235 | 735.4 | Z242 | 175.45 |
| Z245 | 118.68 | Z252 | 1772.47 | Z249 | 208.06 | Z444 | 334.63 |
| Z75-1 | 475.43 | Z219-1 | 173.74 | Z240-1 | 769.82 | Z271-1 | 330.83 |
| Z280 | 315.7 | Z282 | 471.4 | Z284 | 366.02 | Z239 | 239.29 |
| Z286 | 244.37 | Z287 | 338.14 | Z246 | 278.73 | Z288 | 320.8 |
| Z289 | 378.33 | Z290 | 272.12 | Z291 | 161.98 | Z292 | 272.3 |
| Z293 | 112.87 | Z294 | 95.33 | Z285-1 | 239.14 | Z88-1 | 91.27 |
| Z295 | 213.75 | Z296 | 223.27 | Z297 | 1011.49 | Z298 | 668.33 |
| Z299 | 376.23 | Z300 | 274.42 | Z301 | 253.61 | Z302 | 125.33 |
| Z303 | 236.39 | Z304 | 388.62 | Z305 | 297.3 | Z306 | 169.79 |
| Z307 | 276.56 | Z151-1 | 739.21 | Z308 | 440.76 | Z38-1 | 719.14 |
| Z309-1 | 82.59 | Z309-2 | - | Z310 | 567.19 | Z311 | 146 |
| Z312 | 88.37 | Z313 | - | Z314 | 452.46 | Z315 | 224.81 |
| Z316 | 313.53 | Z317 | 199.9 | Z318 | 528.15 | Z319 | 110.54 |
| Z320 | 121.45 | Z247 | 150.6 | Z327 | 149.93 | Z333 | 205.23 |
| Z365 | 315.06 | Z364 | 327.49 | Z323 | 203.64 | Z330 | 204.66 |
| Z331 | 174.4 | Z328 | 90.73 | Z336 | 174.49 | Z335 | 169.98 |
| Z356 | 62.06 | Z256 | 279.28 | Z402 | 453.52 | Z337 | 359.42 |
| Z401 | 462.05 | Z405 | 89.76 | Z367-1 | 999.27 | Z362 | 92.92 |
| Z345 | 764.77 | Z359-1 | 135.45 | Z437 | 119.11 | Z339 | 148.52 |
| Z368 | 417.36 | Z388 | 43.09 | Z389 | 47.14 | Z357-1 | 99.3 |
| Z357-2 | 80.74 | Z358-2 | 53.25 | Z361 | 101.55 | Z359-2 | 98.08 |
| Z358-1 | 67.4 | Z343 | 176.69 | Z261 | 842.27 | Z262 | 627.45 |
| Z438 | 338.8 | Z344 | 386.05 | Z348 | 182.88 | Z350 | 177.6 |
| Z354 | 268.63 | Z349 | 240.47 | Z351 | 125.59 | Z376 | 211.09 |
| Z375 | 257.72 | Z372 | 105.73 | Z382 | 474.26 | Z443 | 210.82 |
| Z404 | 112.51 | Z387 | 2402.03 | Z385 | - | Z386 | 632.29 |
| Z439 | 209.96 | Z440 | 141.99 | Z414 | 2608.75 | Z441 | 197.04 |
| Z442 | 411.91 | Z433 | 111.45 | Z424-1 | 128.88 | Z418-1 | 61.3 |
| Z340 | 240.65 | Z355 | 195.52 | Z346 | 225.84 | Z341 | 492.46 |
| Z314-1 | 969.45 | Z347 | 318.58 | Z342 | 270.8 | Z374 | 265 |
| Z429 | 159 | Z385 | 3.3 | | | | |

### Test Example 2. HPK1 kinase activity assay

Adp-GloTM Kinase Assay was purchased from Promega; Cat. No: V9102; HPK1 was purchased from Carna; Cat. No: 07-410; ATP was purchased from Promega; BSA was purchased from Sigma; Cat. No: B2064-100G; MgCl2 was purchased from Shyuanye; Cat. No: R21455; MBP was purchased from Millipore; Cat. No: 13-110; Briji-35 was purchased from Sigma; Cat. No: B4184-100ML; Tris-HCl was purchased from Sigma; DTT was purchased from Thermo Fisher; Cat. No: R0861; V-storage plate was purchased from corning; Cat. No: 3894; ProxiPlate-384-Plates was purchased from PerkinElmer; Cat. No: 6008289.

Preparation of buffer: 1× reaction buffer (40 mM Tris-HCl, 20 mM MgCl₂, 0.01% Brij35, 50 µM DTT, 0.1 mg/ml BSA) was prepared using sterile water, which was prepared and used on the day of the experiment.

Preparation of 1000× drug stock plate: a 1000× compound stock plate was prepared. 10 mM of compound stock solution was diluted with DMSO, with an initial concentration of 1 mM, 10 concentration gradients, and 3.162-fold dilution. The compound stock plate was sealed with a sealing film and stored in a -20°C refrigerator for later use.

Compound preparation and dosing: the 1000× drug stock plate was taken and melted at room temperature in the dark. Then 5× drug intermediate plate was prepared using reaction buffer and mixed well. 2 µL was transferred from the 5× drug intermediate plate into a 384-well plate, and negative control well and positive control well (in which 2 µL reaction buffer containing 0.5% DMSO was added) were set. Each point was repeated twice.

Preparation and incubation of reaction mixture: HPK1 protein was diluted to a 2.5× working solution with reaction buffer. 4 µL 2.5× HPK1 protein was added to each well containing test compound and positive control well, and 4 µL of reaction buffer without HPK1 protein was added to the negative control well. The obtained mixture was centrifuged at 1000 rpm for 1 min and then incubated in a 20°C incubator for 15 min. A 2.5× mixed working solution of MBP protein and ATP was prepared with reaction buffer at concentrations of 0.5 µg/µL and 50 µM, respectively. 4µL of the mixed working solution of MBP and ATP was added to each well, centrifuged at 1000 rpm for 1 min, and then incubated in a 20°C incubator for 90 min.

ADP-Glo detection and reading plate: 10 µl ADP-Glo was added to each well of the test plate. The resulting mixture was centrifuged at 1000 rpm for 1 min and incubated in a 20°C incubator for 60 min. Then 10 µL of the final incubation solution was transferred from the test plate to a new 384-well plate. 10 µL of kinase detection reagent was added to each well, centrifuged at 400 g for 1 min, and incubated in a 20°C incubator for 60 min. Chemiluminescent signals were read by a microplate reader.

Data analysis processing: Inhibition rate%=(1-(test well-negative control well)/(positive control well-negative control well))* 100%; negative control well: 10 µM ATP + 0.1 µg/µL MBP + DMSO; positive control well: 1 nM HPK1 + 10 µM ATP + 0.1 µg/µL MBP + DMSO; test well: 1 nM HPK1 + 10 µM ATP + 0.1 µg/µL MBP + a compound; the data were analyzed using Graphpad Prism 8.0.1; pharmacodynamics inhibition rate curves were fitted using a four-parameter method, and IC₅₀ values for the drugs were calculated. It may be seen from the experimental results that the compounds of the present disclosure have a high inhibitory activity on HPK1 kinase, with an IC₅₀ value of less than 500 nM (e.g. 0.1 nM to 500 nM); IC₅₀ values for some compounds are even less than 100 nM (e.g. 0.1 nM to 100 nM) or less than 50 nM (e.g. 0.1 nM to 50 nM), more even less than 10 nM (e.g. 0.1 nM to 10 nM). The experimental results of some compounds are shown in Table 2.

**Table 2**

| Number | HPK1 Kinase activity IC₅₀ (nM) | Number | HPK1 Kinase activity IC₅₀ (nM) | Number | HPK1 Kinase activity IC₅₀ (nM) | Number | HPK1 Kinase activity IC₅₀ (nM) |
|---|---|---|---|---|---|---|---|
| Z2 | 2.72 | Z3 | 4.34 | Z3-1 | 230.6 | Z3-2 | 3.37 |
| Z4 | 48.68 | Z93 | 1.62 | Z93-1 | 162.9 | Z93-2 | 0.71 |
| Z94 | 4.34 | Z94-1 | 92.7 | Z94-2 | 1.53 | Z95 | 3.11 |
| Z95-1 | 18.38 | Z95-2 | 1.45 | Z96 | 4.13 | Z97 | 1.54 |
| Z97-1 | 120.08 | Z97-2 | 0.67 | Z98 | 0.90 | Z98-1 | 49.41 |
| Z98-2 | 0.50 | Z100 | 4.26 | Z100-1 | 61.88 | Z100-2 | 1.52 |
| Z101 | 0.49 | Z101-1 | 42.70 | Z101-2 | 0.99 | Z103 | 2.65 |
| Z104 | 0.29 | Z105 | 0.47 | Z106 | 0.53 | Z107 | 5.79 |
| Z104-1 | 0.62 | Z186 | 34.24 | Z190 | 1.89 | Z226 | 3.25 |
| Z227 | 0.52 | Z228-2 | 0.41 | Z131 | 7.54 | Z230 | 1.04 |
| Z232 | 20.53 | Z114 | 138.43 | Z111 | 5.81 | Z113 | 62.20 |
| Z110 | 26.95 | Z233 | 4.01 | Z234-1 | 154.34 | Z235 | 1.32 |
| Z138 | 0.81 | Z242 | 0.52 | Z245 | 0.45 | Z252 | 5.1 |
| Z249 | 0.38 | Z444 | 0.65 | Z75-1 | 1.7 | Z219-1 | 0.42 |
| Z240 | 2.28 | Z271-1 | 1.03 | Z280 | 0.54 | Z282 | 0.38 |
| Z284 | 0.83 | Z239 | - | Z132 | 3.64 | Z121 | 0.50 |
| Z122 | 0.71 | Z126 | 0.78 | Z125 | 1.43 | Z123 | 1.35 |
| Z124 | 0.33 | Z127 | 0.58 | Z128 | 167.95 | Z130 | 3.06 |
| Z210 | 0.62 | Z211 | 1.70 | Z212 | 3.76 | Z119 | 1.54 |
| Z213 | 0.64 | Z214 | 1.04 | Z215 | 0.51 | Z216 | 0.41 |
| Z34-1 | 0.55 | Z119-1 | 1.06 | Z119-2 | 90.03 | Z54-1 | 0.60 |
| Z217 | 0.44 | Z37-1 | 0.82 | Z218 | 0.81 | Z219 | 0.66 |
| Z220 | 2.26 | Z221 | 1.27 | Z222 | 0.80 | Z223 | 1.60 |
| Z224 | 1.11 | Z225 | 0.72 | Z104-2 | 0.74 | Z137 | 0.37 |
| Z199 | 0.32 | Z181 | 14.60 | Z228-1 | 0.48 | Z136 | 31.23 |
| Z229 | 0.46 | Z231 | 35.26 | Z109 | 9.78 | Z115 | 257.03 |
| Z112 | 3.55 | Z116-1 | 843.42 | Z116-2 | > 1000 | Z108-1 | 4.12 |
| Z234-2 | > 1000 | Z236 | 29.59 | Z237 | 0.71 | Z244 | 0.35 |
| Z238 | 1.48 | Z139 | 0.39 | Z251 | 0.49 | Z250 | 0.42 |
| Z243 | 16.49 | Z219-2 | 0.35 | Z279 | 0.44 | Z271-2 | 1.06 |
| Z281 | - | Z283 | 0.3 | Z285 | 0.91 | Z286 | 0.46 |
| Z287 | 0.4 | Z246 | 0.48 | Z288 | 0.69 | Z289 | 1.35 |
| Z290 | 0.77 | Z291 | 0.6 | Z292 | 0.97 | Z293 | 0.47 |
| Z294 | 0.52 | Z285-1 | 0.87 | Z88-1 | 0.38 | Z295 | 1.22 |
| Z296 | 0.86 | Z297 | 2.37 | Z298 | 3.3 | Z299 | 1.06 |
| Z300 | 0.64 | Z301 | 0.61 | Z302 | 0.93 | Z303 | 0.83 |
| Z304 | 1.18 | Z305 | 0.84 | Z306 | 0.64 | Z307 | 0.53 |
| Z151-1 | 2.52 | Z308 | 2.06 | Z38-1 | 2.97 | Z309-1 | 0.58 |
| Z309-2 | 22.67 | Z310 | 1.79 | Z311 | 0.56 | Z312 | 0.47 |
| Z313 | - | Z314 | 2.42 | Z315 | 0.29 | Z316 | 0.74 |
| Z317 | 0.88 | Z318 | 1.06 | Z319 | 0.28 | Z320 | 0.73 |
| Z247 | 0.61 | Z347 | 0.61 | Z342 | 0.64 | Z327 | 0.33 |
| Z333 | 0.49 | Z365 | 0.97 | Z364 | 1.01 | Z323 | 0.49 |
| Z330 | 0.73 | Z331 | 0.7 | Z328 | 0.33 | Z336 | 0.36 |
| Z335 | 0.25 | Z356 | 0.28 | Z256 | 0.8 | Z402 | 1.2 |
| Z337 | 1.57 | Z401 | 3.19 | Z405 | 0.23 | Z367-1 | 1.44 |
| Z362 | 0.22 | Z345 | 1.11 | Z359-1 | 0.27 | Z437 | 0.58 |
| Z339 | 0.58 | Z368 | 1.41 | Z436 | 39.1 | Z389 | 0.21 |
| Z357-1 | 0.31 | Z357-2 | 0.26 | Z358-2 | 0.23 | Z361 | 0.36 |
| Z359-2 | 0.36 | Z358-1 | 0.23 | Z343 | 0.4 | Z261 | 2.63 |
| Z262 | 1.72 | Z438 | 1.13 | Z344 | 1.52 | Z348 | 0.75 |
| Z350 | 0.67 | Z354 | 1.02 | Z349 | 0.47 | Z351 | 0.87 |
| Z386 | 2.74 | Z439 | 0.78 | Z440 | 0.37 | Z414 | 20.63 |
| Z441 | 0.52 | Z442 | 1.5 | Z376 | 0.33 | Z375 | 0.59 |
| Z372 | 0.18 | Z382 | 1.35 | Z418-1 | 0.19 | Z424-1 | 0.31 |
| Z433 | 0.22 | Z404 | 0.32 | Z443 | 0.65 | Z340 | 0.57 |
| Z355 | 0.45 | Z346 | 0.43 | Z341 | 1.09 | Z314-1 | 2.62 |
| Z374 | 0.38 | Z429 | 0.41 | Z387 | 3.8 | | |

the present disclosure have been described in detail with reference to specific embodiments, but those skilled in the art will understand that various modifications and alterations can be made to the technical solution of the present disclosure based on all teachings of the disclosure without departing from the scope of the present disclosure. The appended claims and any equivalents thereof define the full scope of the present disclosure.

## Claims

1. A compound of formula (IA), or a pharmaceutically acceptable salt, a stereoisomer, a solvate or a prodrug thereof: 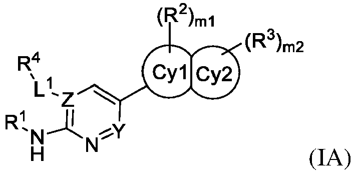 wherein
Y is CH or N;
R¹ is H, C₁₋₆ alkyl, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-NR^{d}-S(=O)₂-R^{c}, -C₁₋₄ alkyl-NR^{d}-S(=O)₂-NR^{a}R^{b}, -C₁₋₄ alkyl-C(=O)-NR^{a}R^{b}, -C₁₋₄ alkyl-S(=O)₂-R^{c}, -C₁₋₄ alkyl-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -S(=O)₂-R^{c}, or -S(=O)₂-NR^{a}R^{b}; wherein the C₁₋₆ alkyl and the C₁₋₆ alkoxy are each independently optionally substituted with halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8-to 10-membered bicyclic heteroaryl each independently contains 1, 2, 3, or 4 heteroatom(s) independently selected from N, O, and S as ring atoms;
Z, L¹ and R⁴ are selected from one of the following combinations:
(a) Z is N; -L¹-R⁴ is absent;
(b) Z is C;
L¹ is a bond, -C₁₋₄ alkyl-, -C₃₋₆ monocyclic cycloalkyl-, -O-, -S-, -NH-, -C(=O)-, -S(=O)-, or -S(=O)₂-;
R⁴ is selected from the group consisting of H, halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C₁₋₄ alkyl-hydroxyl, -C₁₋₄ alkyl-cyano, -C₁₋₄ alkyl-carboxyl, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3-to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}; the C₁₋₆ alkyl and the C₁₋₆ alkoxy are each independently optionally substituted with halogen, deuterium, cyano, or hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8-to 10-membered bicyclic heteroaryl each independently contains 1, 2, 3, or 4 heteroatom(s) independently selected from N, O, and S as ring atoms; 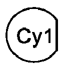 is phenyl or 5- or 6-membered monocyclic heteroaryl; the 5- or 6-membered monocyclic heteroaryl contains 1, 2, or 3 heteroatom(s) independently selected from N, O, and S as ring atoms;
the phenyl or the 5- or 6-membered monocyclic heteroaryl are each independently optionally substituted with m1 R² group(s); m1 is 1, 2, 3, or 4;
the R² is 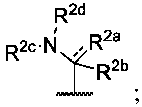 wherein
R^{2a} is H, deuterium, C₁₋₆ alkyl, phenyl, or deuterated C₁₋₆ alkyl, and R^{2b} is H, deuterium, C₁₋₆ alkyl, or deuterated C₁₋₆ alkyl; or R^{2a} and R^{2b} together with the carbon atom to which the R^{2a} and R^{2b} are attached form a C₃₋₆ monocyclic cycloalkyl group; and the bond between R^{2a} and the carbon atom is a single bond; R^{2c} is H, deuterium, C₁₋₆ alkyl, deuterated C₁₋₆ alkyl, -C₁₋₄ alkyl-C₃₋₆ monocyclic cycloalkyl, or C₃₋₆ monocyclic cycloalkyl; R^{2d} is H, deuterium, C₁₋₆ alkyl, deuterated C₁₋₆ alkyl, C₃₋₂₀ cycloalkyl, or 3- to 20-membered heterocyclyl; or
when R^{2a} and R^{2d} together with the nitrogen atom to which the R^{2a} and R^{2d} are attached form a 3- to 20-membered heterocyclyl group, a 5- or 6-membered monocyclic heteroaryl group, or an 8- to 10-membered bicyclic heteroaryl group, R^{2c} is H, deuterium, C₁₋₆ alkyl, or deuterated C₁₋₆ alkyl; R^{2b} is H, deuterium, C₁₋₆ alkyl, or deuterated C₁₋₆ alkyl, and the bond between R^{2a} and the carbon atom is a single bond; or R^{2b} is absent, and the bond between R^{2a} and the carbon atom is a double bond; the 3- to 20-membered heterocyclyl, the 5- or 6-membered monocyclic heteroaryl, or the 8- to 10-membered bicyclic heteroaryl each independently contains one nitrogen atom and optionally 1 or 2 heteroatom(s) independently selected from N, O, and S as ring atoms; and the 3- to 20-membered heterocyclyl, the 5- or 6-membered monocyclic heteroaryl and the 8- to 10-membered bicyclic heteroaryl are each independently optionally substituted with 1, 2, 3, or 4 group(s) selected from group S1; or
when R^{2c} and R^{2d} together with the nitrogen atom to which the R^{2c} and R^{2d} are attached form a 3- to 20-membered heterocyclyl group, a 5- or 6-membered monocyclic heteroaryl group, or an 8- to 10-membered bicyclic heteroaryl group, R^{2a} is H, deuterium, C₁₋₆ alkyl, or deuterated C₁₋₆ alkyl, R^{2b} is H, deuterium, C₁₋₆ alkyl, or deuterated C₁₋₆ alkyl, and the bond between R^{2a} and the carbon atom is a single bond; the 3- to 20-membered heterocyclyl, the 5- or 6-membered monocyclic heteroaryl, or the 8- to 10-membered bicyclic heteroaryl each independently contains one nitrogen atom and optionally 1 or 2 heteroatom(s) independently selected from N, O, and S as ring atoms; and the 3- to 20-membered heterocyclyl, the 5- or 6-membered monocyclic heteroaryl and the 8- to 10-membered bicyclic heteroaryl are each independently optionally substituted with 1, 2, 3, or 4 group(s) selected from group S1; or
the R² is 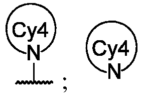 is 3- to 20-membered heterocyclyl, 5- or 6-membered monocyclic heteroaryl, or 8- to 10-membered bicyclic heteroaryl containing one nitrogen atom and attached to other moieties of a molecule through the nitrogen atom; 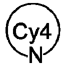 further optionally contains 1 or 2 heteroatom(s) independently selected from N, O, and S as ring atoms; the 3- to 20-membered heterocyclyl, the 5- or 6-membered monocyclic heteroaryl and the 8- to 10-membered bicyclic heteroaryl are each independently optionally substituted with 1, 2, 3, or 4 group(s) selected from group S1; 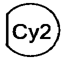
is phenyl or 5- or 6-membered monocyclic heteroaryl; the 5- or 6-membered monocyclic heteroaryl contains 1, 2, or 3 heteroatom(s) independently selected from N, O, and S as ring atoms; the phenyl and the 5- or 6-membered monocyclic heteroaryl are each independently optionally substituted with m2 R³ group(s);
m2 is 1, 2, 3, or 4; each R³ is independently selected from the group consisting of H, oxo (=O), halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C₁₋₄ alkyl-carboxy, -C(=O)-C₁₋₆ alkyl, -C(=O)O-C₁₋₆ alkyl, -C(=O)-C₁₋₄ alkyl-hydroxy, -C(=O)-C₁₋₄ alkyl-C₁₋₆ alkoxy, -C(=O)-C₁₋₄ alkyl-O-C₃₋₂₀ cycloalkyl, -C(=O)-C₃₋₂₀ cycloalkyl, -C(=O)O-C₃₋₂₀ cycloalkyl, -C(=O)-C₁₋₄ alkyl-C₃₋₂₀ cycloalkyl, -C(=O)-3- to 20-membered heterocyclyl, -C(=O)-C₁₋₄ alkyl-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)(C₁₋₆ alkyl)₂, -P(=O)(C₁₋₆ alkyl)₂; wherein the C₁₋₆ alkyl and the C₁₋₆ alkoxy are each independently optionally substituted with 1, 2, 3, or 4 group(s) selected from halogen, deuterium, cyano, 5- to 6-membered heterocyclyl, and hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S2; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; or 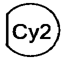
is C₅₋₇ monocyclic cycloalkyl or 5-, 6- or 7-membered monocyclic heterocyclyl;
the 5-, 6- or 7-membered heterocyclyl each independently contains 1, 2, or 3 heteroatom(s) independently selected from N, O, and S as ring atoms; the C₅₋₇ monocyclic cycloalkyl and the 5-, 6- or 7-membered monocyclic heterocyclyl are each independently optionally substituted with m2 R³ group(s);
m2 is 1, 2, 3, or 4; each R³ is independently selected from the group consisting of H, oxo (=O), halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C₁₋₄ alkyl-carboxy, -C(=O)-C₁₋₆ alkyl, -C(=O)O-C₁₋₆ alkyl, -C(=O)-C₁₋₄ alkyl-hydroxy, -C(=O)-C₁₋₄ alkyl-C₁₋₆ alkoxy, -C(=O)-C₁₋₄ alkyl-O-C₃₋₂₀ cycloalkyl, -C(=O)-C₃₋₂₀ cycloalkyl, -C(=O)O-C₃₋₂₀ cycloalkyl, -C(=O)-C₁₋₄ alkyl-C₃₋₂₀ cycloalkyl, -C(=O)-3- to 20-membered heterocyclyl, -C(=O)-C₁₋₄ alkyl-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)(C₁₋₆ alkyl)₂, -P(=O)(C₁₋₆ alkyl)₂, =CR^{3a}R^{3b}; wherein two hydrogen atoms attached to a same carbon atom can be substituted with two R³ groups such that the two R³ groups together with the carbon atom to which the two R³ groups are attached form a C₃₋₆ monocyclic cycloalkyl group or a 3- to 6-membered monocyclic heterocyclyl group; or two hydrogen atoms attached to different carbon atoms are substituted with two R³ groups, and the two R³ groups join to form -CH₂-, -CH₂CH₂-, or -CH₂CH₂CH₂-; wherein the C₁₋₆ alkyl and the C₁₋₆ alkoxy are each independently optionally substituted with 1, 2, 3, or 4 group(s) selected from halogen, deuterium, cyano, 5- to 6-membered heterocyclyl, and hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S2; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; wherein R^{3a} and R^{3b} are each independently hydrogen, halogen, cyano, C₁₋₆ alkyl, halogenated C₁₋₆ alkyl, C₆₋₁₄ aryl, or 5- or 6-membered monocyclic heteroaryl; the C₆₋₁₄ aryl or the 5- or 6-membered monocyclic heteroaryl is optionally substituted with 1, 2, or 3 group(s) selected from halogen, cyano, C₁₋₄ alkyl, C₁₋₄ alkoxy, halogenated C₁₋₄ alkyl, halogenated C₁₋₄ alkoxy; or R^{3a} and R^{3b} together with the carbon atom to which the R^{3a} and R^{3b} are attached form a C₃₋₆ monocyclic cycloalkyl group or a 3- to 6-membered monocyclic heterocyclyl group;
the C₃₋₆ monocyclic cycloalkyl or the 3- to 6-membered monocyclic heterocyclyl is optionally substituted with 1, 2, or 3 group(s) selected from halogen, cyano, C₁₋₄ alkyl, C₁₋₄ alkoxy, halogenated C₁₋₄ alkyl, halogenated C₁₋₄ alkoxy, C₆₋₁₄ aryl, 5- or 6-membered monocyclic heteroaryl;
among the above groups, each group from group S1 is independently selected from the group consisting of oxo (=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C=C-3- to 20-membered heterocyclyl, -C≡C-C₆₋₁₄ aryl, -C=C-5- or 6-membered monocyclic heteroaryl, -C=C-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -S(=O)₂-5- or 6-membered monocyclic heteroaryl, -S(=O)₂-8- to 10-membered bicyclic heteroaryl, -C(=O)O-C₁₋₆ alkyl, -C(=O)O-C₃₋₂₀ cycloalkyl, -C(=O)O-3- to 20-membered heterocyclyl, -C(=O)O-5- or 6-membered monocyclic heteroaryl, -C(=O)O-8- to 10-membered bicyclic heteroaryl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1} -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1};
wherein the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S3; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms;
among the above groups, each group from group S2 is independently selected from the group consisting of oxo (=O), halogen, cyano, hydroxyl, carboxyl, nitro, C₁₋₆ alkyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-3- to 20-membered heterocyclyl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8-to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -S(=O)₂-5- or 6-membered monocyclic heteroaryl, -S(=O)₂-8- to 10-membered bicyclic heteroaryl, -C(=O)O-C₁₋₆ alkyl, -C(=O)O-C₃₋₂₀ cycloalkyl, -C(=O)O-3- to 20-membered heterocyclyl, -C(=O)O-5- or 6-membered monocyclic heteroaryl, -C(=O)O-8- to 10-membered bicyclic heteroaryl, -NR^{a1}R^{b1}, -C(-O)-NR^{a1}R^{b1}, -OR^{c1}, -C₁₋₄ alkyl-C(=O)O-C₁₋₆ alkyl, -C₁₋₄ alkyl-C(=O)O-C₃₋₂₀ cycloalkyl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}; wherein the C₁₋₆ alkyl, the C₁₋₆ alkoxy, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S3; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms;
among the above groups, each R^{a}, R^{b}, R^{a1}, and R^{b1} is independently H, C₁₋₆ alkyl, halogenated C₁₋₆ alkyl, deuterated C₁₋₆ alkyl, -C₁₋₄ alkyl-hydroxy, -C₁₋₄ alkyl-cyano, -C₁₋₄ alkyl-halogenated C₁₋₆ alkyl, -C₁₋₄ alkyl-deuterated C₁₋₆ alkyl, -C₁₋₄ alkyl-C₁₋₆ alkoxy, -C₁₋₄ alkyl-halogenated C₁₋₆ alkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8-to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C(=O)-C₁₋₆ alkyl, -C(=O)-C₃₋₆ monocyclic cycloalkyl, -C(=O)-3- to 6-membered monocyclic heterocyclyl;
wherein the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂; or
each R^{a} and R^{b} together with the nitrogen atom to which the R^{a} and R^{b} are attached form a 3- to 20-membered heterocyclyl group; each R^{a1} and R^{b1} together with the nitrogen atom to which the R^{a1} and R^{b1} are attached form a 3- to 20-membered heterocyclyl group;
wherein the 3- to 20-membered heterocyclyl is each independently optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂;
among the above groups, each R^{d} and R^{d1} are independently H, C₁₋₆ alkyl, or deuterated C₁₋₆ alkyl;
among the above groups, each R^{c} and R^{c1} are independently H, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -C₁₋₄ alkyl-halogenated C₁₋₆ alkyl, -C₁₋₄ alkyl-deuterated C₁₋₆ alkyl, -C₁₋₄ alkyl-C₁₋₆ alkoxy, -C₁₋₄ alkyl-halogenated C₁₋₆ alkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂;
among the above groups, each group from group S3 is independently selected from the group consisting of oxo (C=O), cyano, halogen, hydroxy, carboxy, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂, 3- to 6-membered monocyclic heterocyclyl;
among the above groups, the -C₁₋₄ alkyl- or the -C₃₋₆ monocyclic cycloalkyl- is unsubstituted; or hydrogen atoms of the -C₁₋₄ alkyl- are each independently substituted with a group selected from halogen, cyano, hydroxy, C₁₋₆ alkyl, halogenated C₁₋₆ alkyl, deuterated C₁₋₆ alkyl, -CH₂-hydroxy, -CH₂-cyano, and phenyl, or two hydrogen atoms attached to a same carbon atom of the C₁₋₄ alkyl are simultaneously substituted with -(CH₂)ⱼ- to form a cycloalkyl group, wherein j is 2, 3, 4, 5, or 6; hydrogen atoms of the -C₃₋₆ monocyclic cycloalkyl- are each independently substituted with a group selected from halogen, cyano, hydroxy, C₁₋₆ alkyl, halogenated C₁₋₆ alkyl, deuterated C₁₋₆ alkyl.

2. The compound, or the pharmaceutically acceptable salt, the stereoisomer, the solvate or the prodrug thereof claim 1, wherein the compound is a compound of formula (IA-1); 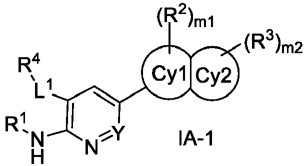
wherein R¹, Y, 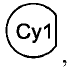 R², m1, 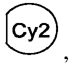 R³, and m2 are as defined in claim 1;
L¹ is a bond, -C₁₋₄ alkyl-, -C₃₋₆ monocyclic cycloalkyl-, -O-, -S-, -NH-, -C(=O)-, -S(=O)-, or -S(=O)₂-;
R⁴ is selected from the group consisting of H, halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C₁₋₄ alkyl-hydroxyl, -C₁₋₄ alkyl-cyano, -C₁₋₄ alkyl-carboxyl, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3-to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}; the C₁₋₆ alkyl and the C₁₋₆ alkoxy are each independently optionally substituted with halogen, deuterium, cyano, or hydroxy; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8-to 10-membered bicyclic heteroaryl each independently contains 1, 2, 3, or 4 heteroatom(s) independently selected from N, O, and S as ring atoms; wherein R^{a}, R^{b}, R^{c}, R^{d}, and group S1 are each as defined in claim 1.

3. The compound, or the pharmaceutically acceptable salt, the stereoisomer, the solvate or the prodrug thereof of claim 1, wherein the compound is a compound of formula (IA1), a compound of formula (IA2), a compound of formula (IA3), a compound of formula (IA4), a compound of formula (IA5), or a compound of formula (IA6); 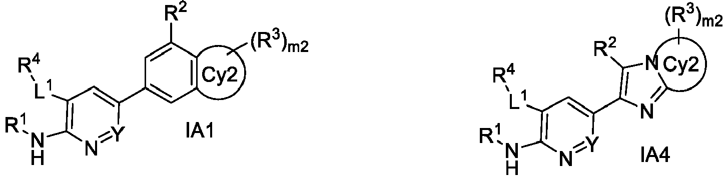 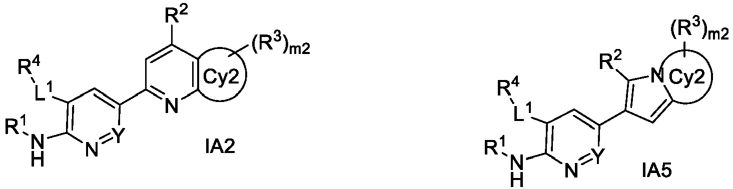 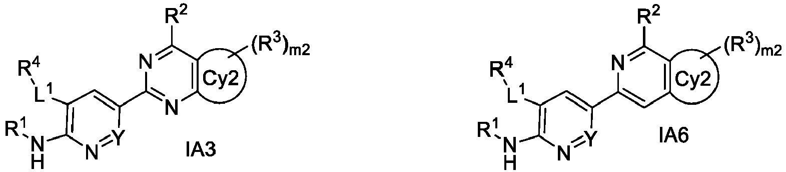 in each formula, Y, L¹, R¹, R², 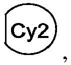 R³, R⁴, and m2 are each independently as defined in claim 2.

4. The compound, or the pharmaceutically acceptable salt, the stereoisomer, the solvate or the prodrug thereof of claim 1, wherein the compound is a compound of formula (IB); 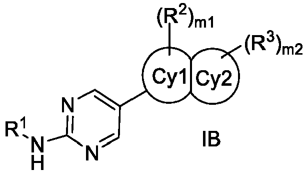 wherein R¹, 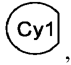 R², m1, 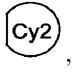 R³, and m2 are as defined in claim 1.

5. The compound, or the pharmaceutically acceptable salt, the stereoisomer, the solvate or the prodrug thereof of claim 4, wherein the compound is a compound of formula (IB1), a compound of formula (IB2), a compound of formula (IB3), a compound of formula (IB4), a compound of formula (IB5), or a compound of formula (IB6); 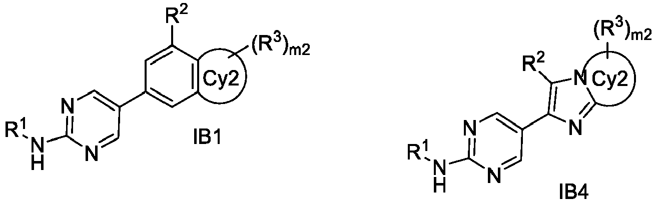 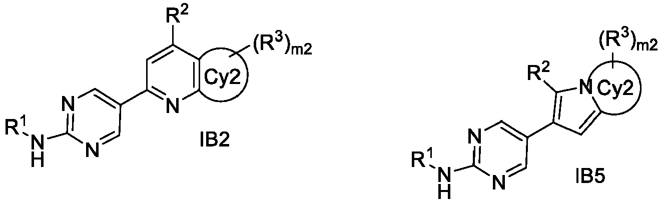 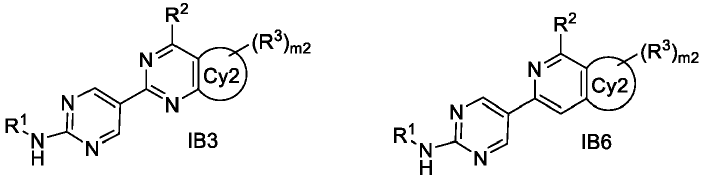 in each formula, R¹, R², 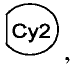 R³, and m2 are each independently as defined in claim 4.

6. The compound, or the pharmaceutically acceptable salt, the stereoisomer, the solvate or the prodrug thereof of any one of claims 1-5, wherein
R¹ is 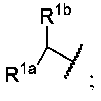 wherein R^{1a} is C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -C₁₋₄ alkyl-hydroxy, -C₁₋₄ alkyl-cyano, -C₁₋₄ alkyl-C₁₋₆ alkoxy, -C₁₋₄ alkyl-halogenated C₁₋₆ alkoxy, or -C₁₋₄ alkyl-deuterated C₁₋₆ alkoxy; R^{1b} is H, halogen, C₁₋₃ alkyl, halogenated C₁₋₃ alkyl, deuterated C₁₋₃ alkyl, C₁₋₃ alkoxy, halogenated C₁₋₃ alkoxy, or deuterated C₁₋₃ alkoxy;
or
R¹ is 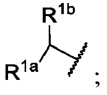 wherein R^{1a} is C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl; R^{1b} is H, halogen, C₁₋₃ alkyl, halogenated C₁₋₃ alkyl, deuterated C₁₋₃ alkyl, C₁₋₃ alkoxy, halogenated C₁₋₃ alkoxy, or deuterated C₁₋₃ alkoxy; wherein the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are each independently optionally substituted with 1, 2, 3, or 4 group(s) selected from group S1.

7. A compound of formula (IC), or a pharmaceutically acceptable salt, a stereoisomer, a solvate or a prodrug thereof: 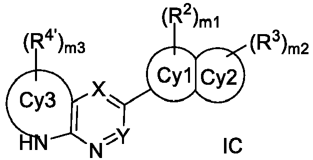 wherein
X is CR⁵ or N; Y is CH or N; and X and Y cannot both be N; R⁵ is H, C₁₋₆ alkyl, or C₃₋₆ monocyclic cycloalkyl; 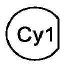 is phenyl or 5- or 6-membered monocyclic heteroaryl; the 5- or 6-membered monocyclic heteroaryl contains 1, 2, or 3 heteroatom(s) independently selected from N, O, and S as ring atoms; the phenyl or the 5- or 6-membered monocyclic heteroaryl are each independently optionally substituted with m1 R² group(s); m1 is 1, 2, 3, or 4;
the R² is 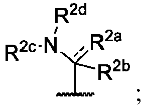 wherein R^{2a} is H, deuterium, C₁₋₆ alkyl, phenyl, or deuterated C₁₋₆ alkyl, and R^{2b} is H, deuterium, C₁₋₆ alkyl, or deuterated C₁₋₆ alkyl; or R^{2a} and R^{2b} together with the carbon atom to which the R^{2a} and R^{2b} are attached form a C₃₋₆ monocyclic cycloalkyl group, and a bond between R^{2a} and the carbon atom is a single bond; R^{2c} is H, deuterium, C₁₋₆ alkyl, deuterated C₁₋₆ alkyl, -C₁₋₄ alkyl-C₃₋₆ monocyclic cycloalkyl, or C₃₋₆ monocyclic cycloalkyl; R^{2d} is H, deuterium, C₁₋₆ alkyl, deuterated C₁₋₆ alkyl, C₃₋₂₀ cycloalkyl, or 3- to 20-membered heterocyclyl; or
when R^{2a} and R^{2d} together with the nitrogen atom to which the R^{2a} and R^{2d} are attached form a 3- to 20-membered heterocyclyl group, a 5- or 6-membered monocyclic heteroaryl group, or an 8- to 10-membered bicyclic heteroaryl group, R^{2c} is H, deuterium, C₁₋₆ alkyl, or deuterated C₁₋₆ alkyl; R^{2b} is H, deuterium, C₁₋₆ alkyl, or deuterated C₁₋₆ alkyl, and the bond between R^{2a} and the carbon atom is a single bond; or R^{2b} is absent, and the bond between R^{2a} and the carbon atom is a double bond; the 3- to 20-membered heterocyclyl, the 5- or 6-membered monocyclic heteroaryl, or the 8- to 10-membered bicyclic heteroaryl each independently contains one nitrogen atom and optionally 1 or 2 heteroatom(s) independently selected from N, O, and S as ring atoms; and the 3- to 20-membered heterocyclyl, the 5- or 6-membered monocyclic heteroaryl, and the 8- to 10-membered bicyclic heteroaryl are each independently optionally substituted with 1, 2, 3, or 4 group(s) selected from group S1; or
when R^{2c} and R^{2d} together with the nitrogen atom to which the R^{2c} and R^{2d} are attached form a 3- to 20-membered heterocyclyl group, a 5- or 6-membered monocyclic heteroaryl group, or an 8- to 10-membered bicyclic heteroaryl group, R^{2a} is H, deuterium, C₁₋₆ alkyl, or deuterated C₁₋₆ alkyl; R^{2b} is H, deuterium, C₁₋₆ alkyl, or deuterated C₁₋₆ alkyl; and the bond between R^{2a} and the carbon atom is a single bond; the 3- to 20-membered heterocyclyl, the 5- or 6-membered monocyclic heteroaryl, or the 8- to 10-membered bicyclic heteroaryl each independently contains one nitrogen atom and optionally 1 or 2 heteroatom(s) independently selected from N, O, and S as ring atoms; and the 3- to 20-membered heterocyclyl, the 5- or 6-membered monocyclic heteroaryl, and the 8- to 10-membered bicyclic heteroaryl are each independently optionally substituted with 1, 2, 3, or 4 group(s) selected from group S1; or
the R² is 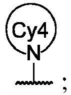 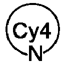 is 3- to 20-membered heterocyclyl, 5- or 6-membered monocyclic heteroaryl, or 8-to 10-membered bicyclic heteroaryl containing one nitrogen atom and attached to other moieties of a molecule through the nitrogen atom; 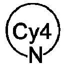 further optionally contains 1 or 2 heteroatom(s) independently selected from N, O, and S as ring atoms; the 3- to 20-membered heterocyclyl, the 5- or 6-membered monocyclic heteroaryl, and the 8- to 10-membered bicyclic heteroaryl are each independently optionally substituted with 1, 2, 3, or 4 group(s) selected from group S1; 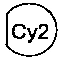
is phenyl or 5- or 6-membered monocyclic heteroaryl; the 5- or 6-membered monocyclic heteroaryl contains 1, 2, or 3 heteroatom(s) independently selected from N, O, and S as ring atoms; the phenyl and the 5- or 6-membered monocyclic heteroaryl are each independently optionally substituted with m2 R³ group(s);
m2 is 1, 2, 3, or 4; each R³ is independently selected from the group consisting of H, oxo (=O), halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl,
-C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C₁₋₄ alkyl-carboxy, -C(=O)-C₁₋₆ alkyl, -C(=O)O-C₁₋₆ alkyl, -C(=O)-C₁₋₄ alkyl-hydroxy, -C(=O)-C₁₋₄ alkyl-C₁₋₆ alkoxy, -C(=O)-C₁₋₄ alkyl-O-C₃₋₂₀ cycloalkyl, -C(=O)-C₃₋₂₀ cycloalkyl, -C(=O)O-C₃₋₂₀ cycloalkyl, -C(=O)O-C₃₋₂₀ cycloalkyl, -C(=O)-C₁₋₄ alkyl-C₃₋₂₀ cycloalkyl, -C(=O)-3- to 20-membered heterocyclyl, -C(=O)-C₁₋₄ alkyl-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)(C₁₋₆ alkyl)₂, -P(=O)(C₁₋₆ alkyl)₂; wherein the C₁₋₆ alkyl and the C₁₋₆ alkoxy are each independently optionally substituted with 1, 2, 3, or 4 group(s) selected from halogen, deuterium, cyano, 5- to 6-membered heterocyclyl, and hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S2; the 3- to 20-membered heterocyclyl, the 5- or 6-membered monocyclic heteroaryl, and the 8-to 10-membered bicyclic heteroaryl each independently contains 1, 2, 3, or 4 heteroatom(s) independently selected from N, O, and S as ring atoms; or 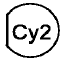
is C₅₋₇ monocyclic cycloalkyl or 5-, 6- or 7-membered monocyclic heterocyclyl;
the 5-, 6- or 7-membered heterocyclyl each independently contains 1, 2, or 3 heteroatom(s) independently selected from N, O, and S as ring atoms; the C₅₋₇ monocyclic cycloalkyl and the 5-, 6- or 7-membered monocyclic heterocyclyl are each independently optionally substituted with m2 R³ group(s);
m2 is 1, 2, 3, or 4; each R³ is independently selected from the group consisting of H, oxo (=O), halogen, cyano, hydroxyl, carboxyl, nitro, C₁₋₆ alkyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C₁₋₄ alkyl-carboxy, -C(=O)-C₁₋₆ alkyl, -C(=O)O-C₁₋₆ alkyl, -C(=O)-C₁₋₄ alkyl-hydroxy, -C(=O)-C₁₋₄ alkyl-C₁₋₆ alkoxy, -C(=O)-C₁₋₄ alkyl-O-C₃₋₂₀ cycloalkyl, -C(=O)-C₃₋₂₀ cycloalkyl, -C(=O)O-C₃₋₂₀ cycloalkyl, -C(=O)-C₁₋₄ alkyl-C₃₋₂₀ cycloalkyl, -C(=O)-3- to 20-membered heterocyclyl, -C(=O)-C₁₋₄ alkyl-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)(C₁₋₆ alkyl)₂, -P(=O)(C₁₋₆ alkyl)₂, =CR^{3a}R^{3b}; wherein two hydrogen atoms attached to a same carbon atom can be substituted with two R³ groups such that the two R³ groups together with the carbon atom to which the two R³ groups are attached form a C₃₋₆ monocyclic cycloalkyl group or a 3- to 6-membered monocyclic heterocyclyl group; or two hydrogen atoms attached to different carbon atoms are substituted with two R³ groups, and the two R³ groups join to form -CH₂-, -CH₂CH₂-, or -CH₂CH₂CH₂-; wherein the C₁₋₆ alkyl and the C₁₋₆ alkoxy are each independently optionally substituted with 1, 2, 3, or 4 group(s) selected from halogen, deuterium, cyano, 5- to 6-membered heterocyclyl, and hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S2; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; wherein R^{3a} and R^{3b} are each independently hydrogen, halogen, cyano, C₁₋₆ alkyl, halogenated C₁₋₆ alkyl, C₆₋₁₄ aryl, or 5- or 6-membered monocyclic heteroaryl; the C₆₋₁₄ aryl or the 5- or 6-membered monocyclic heteroaryl is optionally substituted with 1, 2, or 3 group(s) selected from halogen, cyano, C₁₋₄ alkyl, C₁₋₄ alkoxy, halogenated C₁₋₄ alkyl, halogenated C₁₋₄ alkoxy; or R^{3a} and R^{3b} together with the carbon atom to which the R^{3a} and R^{3b} are attached form a C₃₋₆ monocyclic cycloalkyl group or a 3- to 6-membered monocyclic heterocyclyl group;
the C₃₋₆ monocyclic cycloalkyl or the 3- to 6-membered monocyclic heterocyclyl is optionally substituted with 1, 2, or 3 group(s) selected from halogen, cyano, C₁₋₄ alkyl, C₁₋₄ alkoxy, halogenated C₁₋₄ alkyl, halogenated C₁₋₄ alkoxy, C₆₋₁₄ aryl, 5- or 6-membered monocyclic heteroaryl; 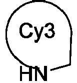
is 5- to 7-membered heteroaromatic ring; wherein the m3 hydrogen atom(s) of 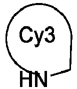
are optionally substituted with m3 R^{4'} group(s); m3 is 1, 2, 3, or 4; R^{4'} is selected from the group consisting of H, oxo (C=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, 5- or 6-membered monocyclic heteroaryl, 8- to 10-membered bicyclic heteroaryl, -C≡C-C₃₋₂₀ cycloalkyl, -C=C-3- to 20-membered heterocyclyl, -C≡C-C₆₋₁₄ aryl, -C=C-5- or 6-membered monocyclic heteroaryl, -C=C-8- to 10-membered bicyclic heteroaryl, -C≡C-C(=O)-NR^{a}R^{b}, -C≡C-C₁₋₄ alkyl-C(=O)-NR^{a}R^{b}, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5-or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b} -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)-(C₁₋₆ alkyl)₂, -P(=O)-(C₁₋₆ alkyl)₂;
wherein the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; or 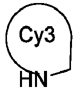
is 5- to 7-membered heterocyclic ring; wherein the m3 hydrogen atom(s) of 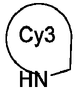
are optionally substituted with m3 R^{4'} group(s); m3 is 1, 2, 3, or 4; R^{4'} is selected from the group consisting of H, oxo (C=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C≡C-C₃₋₂₀ cycloalkyl, -C=C-3- to 20-membered heterocyclyl, -C≡C-C₆₋₁₄ aryl, -C=C-5- or 6-membered monocyclic heteroaryl, -C=C-8- to 10-membered bicyclic heteroaryl, -C≡C-C(=O)-NR^{a}R^{b}, -C≡C-C₁₋₄ alkyl-C(=O)-NR^{a}R^{b}, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3-to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)-(C₁₋₆ alkyl)₂, -P(=O)-(C₁₋₆ alkyl)₂; the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms;
among the above groups, each group from group S1 is independently selected from the group consisting of oxo (=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C=C-3- to 20-membered heterocyclyl, -C≡C-C₆₋₁₄ aryl, -C≡C-5-or 6-membered monocyclic heteroaryl, -C=C-8- to 10-membered bicyclic heteroaryl, -C₁₋₄ alkyl-hydroxy, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5-or 6-membered monocyclic heteroaryl, -C₁₋₄ alkyl-8- to 10-membered bicyclic heteroaryl, -C₁₋₄ alkyl-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -OR^{c1}, -C₁₋₄ alkyl-S(=O)₂-C₁₋₆ alkyl, -C₁₋₄ alkyl-S(=O)₂-C₃₋₂₀ cycloalkyl, -C₁₋₄ alkyl-S(=O)₂-3- to 20-membered heterocyclyl, -C₁₋₄ alkyl-C(=O)O-C₁₋₆ alkyl, -C₁₋₄ alkyl-C(=O)O-C₃₋₂₀ cycloalkyl, -C₁₋₄ alkyl-C(=O)-C₁₋₆ alkyl, -C₁₋₄ alkyl-C(=O)-C₃₋₂₀ cycloalkyl, -C₁₋₄ alkyl-C(=O)-C₆₋₁₄ aryl, -C(=O)-5- or 6-membered monocyclic heteroaryl, -C(=O)-8- to 10-membered bicyclic heteroaryl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1};
wherein the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S3; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms;
among the above groups, each group from group S2 is independently selected from the group consisting of oxo (=O), halogen, cyano, hydroxyl, carboxyl, nitro, C₁₋₆ alkyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-3- to 20-membered heterocyclyl, -O-5- or 6-membered monocyclic heteroaryl, -O-8- to 10-membered bicyclic heteroaryl, -C₁₋₄ alkyl-hydroxyl, -C₁₋₄ alkyl-cyano, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8-to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -S(=O)₂-5- or 6-membered monocyclic heteroaryl, -S(=O)₂-8- to 10-membered bicyclic heteroaryl, -C(=O)O-C₁₋₆ alkyl, -C(=O)O-C₃₋₂₀ cycloalkyl, -C(=O)O-3- to 20-membered heterocyclyl, -C(=O)O-5- or 6-membered monocyclic heteroaryl, -C(=O)O-8- to 10-membered bicyclic heteroaryl, -NR^{a1}R^{b1}, -C(-O)-NR^{a1}R^{b1}, -OR^{c1}, -C₁₋₄ alkyl-C(=O)O-C₁₋₆ alkyl, -C₁₋₄ alkyl-C(=O)O-C₃₋₂₀ cycloalkyl, -C₁₋₄ alkyl-NR^{a1}R^{b1}, -C₁₋₄ alkyl-C(=O)-NR^{a1}R^{b1}, -C≡C-C(=O)-NR^{a1}R^{b1}, -C≡C-C₁₋₄ alkyl-C(=O)-NR^{a1}R^{b1}, -C₁₋₄ alkyl-OR^{c1}, -C₁₋₄ alkyl-P(=O)-(C₁₋₆ alkyl)₂, -P(=O)-(C₁₋₆ alkyl)₂, -C₁₋₄ alkyl-NR^{d1}-C(=O)-R^{c1}, -C₁₋₄ alkyl-NR^{d1}-C(=O)-NR^{a1}R^{b1}, -C₁₋₄ alkyl-NR^{d1}-S(=O)₂-R^{c1}, -C₁₋₄ alkyl-S(=O)₂-NR^{a1}R^{b1}, -C₁₋₄ alkyl-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}; wherein the C₁₋₆ alkyl, the C₁₋₆ alkoxy, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S3; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms;
among the above groups, each R^{a}, R^{b}, R^{a1}, and R^{b1} is independently H, C₁₋₆ alkyl, halogenated C₁₋₆ alkyl, deuterated C₁₋₆ alkyl, -C₁₋₄ alkyl-hydroxy, -C₁₋₄ alkyl-cyano, -C₁₋₄ alkyl-halogenated C₁₋₆ alkyl, -C₁₋₄ alkyl-deuterated C₁₋₆ alkyl, -C₁₋₄ alkyl-C₁₋₆ alkoxy, -C₁₋₄ alkyl-halogenated C₁₋₆ alkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, -C₁₋₄ alkyl-8-to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C(=O)-C₁₋₆ alkyl, -C(=O)-C₃₋₆ monocyclic cycloalkyl, -C(=O)-3- to 6-membered monocyclic heterocyclyl; wherein the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂; or each R^{a} and R^{b} together with the nitrogen atom to which the R^{a} and R^{b} are attached form a 3- to 20-membered heterocyclyl group; each R^{a1} and R^{b1} together with the nitrogen atom to which the R^{a1} and R^{b1} are attached form a 3- to 20-membered heterocyclyl group;
wherein the 3- to 20-membered heterocyclyl is each independently optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂;
among the above groups, each R^{d} and R^{d1} are independently H, C₁₋₆ alkyl, or deuterated C₁₋₆ alkyl;
among the above groups, each R^{c} and R^{c1} are independently H, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -C₁₋₄ alkyl-halogenated C₁₋₆ alkyl, -C₁₋₄ alkyl-deuterated C₁₋₆ alkyl, -C₁₋₄ alkyl-C₁₋₆ alkoxy, -C₁₋₄ alkyl-halogenated C₁₋₆ alkoxy, -C₁₋₄ alkyl-deuterated C₁₋₆ alkoxy, C₃₋₆ monocyclic cycloalkyl, -C₁₋₄ alkyl-C₃₋₆ monocyclic cycloalkyl, -C₁₋₄ alkyl-O-C₃₋₆ monocyclic cycloalkyl, 3- to 6-membered monocyclic heterocyclyl, -C₁₋₄ alkyl-3- to 6-membered monocyclic heterocyclyl, -C₁₋₄ alkyl-O-3- to 6-membered monocyclic heterocyclyl, phenyl, -C₁₋₄ alkyl-phenyl, 5- or 6-membered monocyclic heteroaryl, -C₁₋₄ alkyl-5- or 6-membered monocyclic heteroaryl, 8- to 10-membered bicyclic heteroaryl, or -C₁₋₄ alkyl-8- to 10-membered bicyclic heteroaryl; the C₃₋₆ monocyclic cycloalkyl, the 3- to 6-membered monocyclic heterocyclyl, the phenyl, the 5- or 6-membered monocyclic heteroaryl, and the 8- to 10-membered bicyclic heteroaryl are optionally substituted with 1 or 2 group(s) selected from the group consisting of halogen, hydroxyl, carboxyl, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂;
among the above groups, each group from group S3 is independently selected from the group consisting of oxo (C=O), halogen, cyano, hydroxy, carboxy, nitro, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, -NH₂, -NHC₁₋₆ alkyl, -N(C₁₋₆ alkyl)₂, -C₁₋₄ alkyl-P(=O)-(C₁₋₆ alkyl)₂, -P(=O)-(C₁₋₆ alkyl)₂, 3- to 6-membered monocyclic heterocyclyl;
among the above groups, the -C₁₋₄ alkyl- or the -C₃₋₆ monocyclic cycloalkyl- is unsubstituted, or hydrogen atoms of the -C₁₋₄ alkyl- are each independently substituted with a group selected from halogen, cyano, hydroxy, C₁₋₆ alkyl, halogenated C₁₋₆ alkyl, deuterated C₁₋₆ alkyl, -CH₂-hydroxy, -CH₂-cyano, and phenyl, or two hydrogen atoms attached to a same carbon atom of the C₁₋₄ alkyl are simultaneously substituted with -(CH₂)ⱼ- to form a cycloalkyl group, wherein j is 2, 3, 4, 5, or 6; hydrogen atoms of the -C₃₋₆ monocyclic cycloalkyl- are each independently substituted with a group selected from halogen, cyano, hydroxy, C₁₋₆ alkyl, halogenated C₁₋₆ alkyl, deuterated C₁₋₆ alkyl.

8. The compound, or the pharmaceutically acceptable salt, the stereoisomer, the solvate or the prodrug thereof of claim 7, wherein the compound is a compound of formula (IC1); 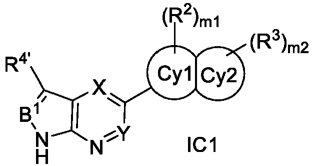 wherein
X, Y, 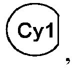 R², m1, 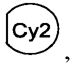 R³, and m2 are as defined in claim 7;
B¹ is CH or N;
R^{4'} is selected from the group consisting of H, oxo (C=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, 5- or 6-membered monocyclic heteroaryl, 8- to 10-membered bicyclic heteroaryl, -C≡C-C₃₋₂₀ cycloalkyl, -C=C-3- to 20-membered heterocyclyl, -C≡C-C₆₋₁₄ aryl, -C=C-5- or 6-membered monocyclic heteroaryl, -C=C-8- to 10-membered bicyclic heteroaryl, -C≡C-C(=O)-NR^{a}R^{b}, -C≡C-C₁₋₄ alkyl-C(=O)-NR^{a}R^{b}, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3-to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)-(C₁₋₆ alkyl)₂, -P(=O)-(C₁₋₆ alkyl)₂; wherein the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; wherein R^{a}, R^{b}, R^{c}, R^{d}, and group S1 are each as defined in claim 7.

9. The compound, or the pharmaceutically acceptable salt, the stereoisomer, the solvate or the prodrug thereof of claim 8, wherein the compound is a compound of formula (IC1a), a compound of formula (IC1b), a compound of formula (IC1c), a compound of formula (IC1d), a compound of formula (IC1e), or a compound of formula (IC1f); 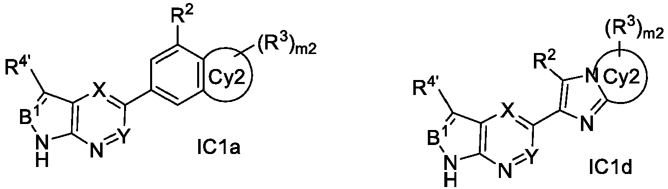 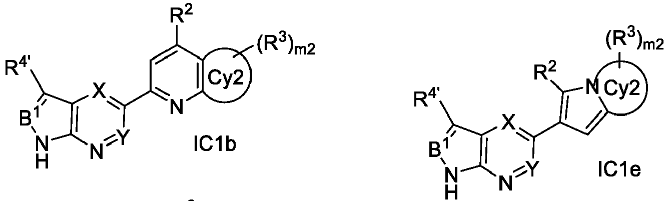 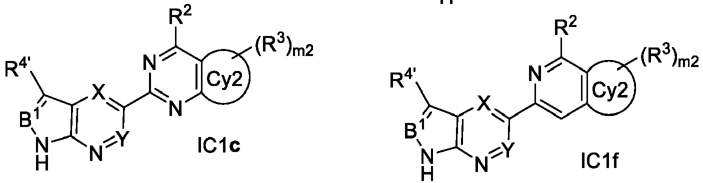 in each formula, X, Y, R², 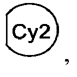 R³, m2, B¹, and R^{4'} are each independently as defined in claim 8.

10. The compound, or the pharmaceutically acceptable salt, the stereoisomer, the solvate or the prodrug thereof of claim 7, wherein the compound is a compound of formula (IC3); 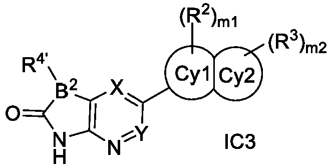
wherein X, Y, 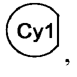 R², m1, 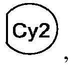 R³, and m2 are as defined in claim 7; B² is N or CH;
R^{4'} is selected from the group consisting of H, oxo (C=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C≡C-C₃₋₂₀ cycloalkyl, -C=C-3- to 20-membered heterocyclyl, -C≡C-C₆₋₁₄ aryl, -C=C-5- or 6-membered monocyclic heteroaryl, -C=C-8- to 10-membered bicyclic heteroaryl, -C≡C-C(=O)-NR^{a}R^{b}, -C≡C-C₁₋₄ alkyl-C(=O)-NR^{a}R^{b}, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3-to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5- or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)-(C₁₋₆ alkyl)₂, -P(=O)-(C₁₋₆ alkyl)₂; the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; wherein R^{a}, R^{b}, R^{c}, R^{d}, and group S1 are each as defined in claim 7.

11. The compound, or the pharmaceutically acceptable salt, the stereoisomer, the solvate or the prodrug thereof of claim 10, wherein the compound is a compound of formula (IC3a), a compound of formula (IC3b), a compound of formula (IC3c), a compound of formula (IC3d), a compound of formula (IC3e), or a compound of formula (IC3f); 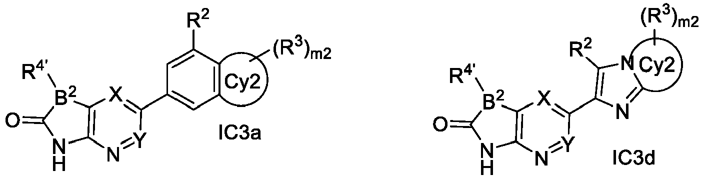 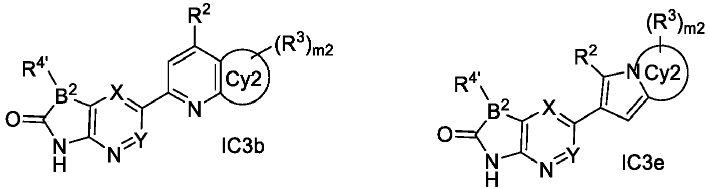 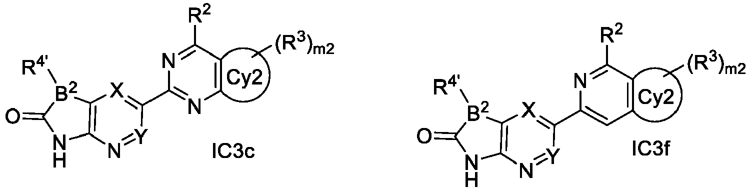 in each formula, X, Y, R², 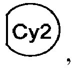 R³, m2, B², and R^{4'} are each independently as defined in claim 10.

12. The compound, or the pharmaceutically acceptable salt, the stereoisomer, the solvate or the prodrug thereof of claim 7, wherein the compound is a compound of formula (IC4); 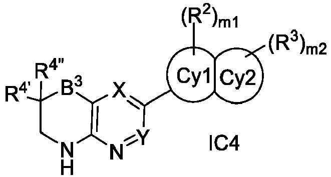
wherein X, Y, 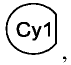 R², m1, 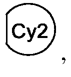 NR^{4‴}, R³, and m2 are as defined in claim 7; B³ is CHR^{4‴}, NR^{4‴}, or O;
R⁴, R^{4"}, and R^{4‴} are each independently selected from the group consisting of H, oxo (C=O), halogen, cyano, hydroxyl, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -C≡C-C₃₋₂₀ cycloalkyl, -C=C-3- to 20-membered heterocyclyl, -C≡C-C₆₋₁₄ aryl, -C=C-5- or 6-membered monocyclic heteroaryl, -C=C-8- to 10-membered bicyclic heteroaryl, -C≡C-C(=O)-NR^{a}R^{b}, -C≡C-C₁₋₄ alkyl-C(=O)-NR^{a}R^{b}, -C₁₋₄ alkyl-hydroxy, -C₁₋₄ alkyl-cyano, -C₁₋₄ alkyl-carboxy, -C₁₋₄ alkyl-C₁₋₆ alkyl, -C₁₋₄ alkyl-C₁₋₆ alkoxy, -C₁₋₄ alkyl-C₃₋₂₀ cycloalkyl, -C₁₋₄ alkyl-O-C₃₋₂₀ cycloalkyl, -C₁₋₄ alkyl-3- to 20-membered heterocyclyl, -C₁₋₄ alkyl-O-3- to 20-membered heterocyclyl, -C₁₋₄ alkyl-C₆₋₁₄ aryl, -C₁₋₄ alkyl-O-C₆₋₁₄ aryl, -C₁₋₄ alkyl-5- or 6-membered monocyclic heteroaryl, -C₁₋₄ alkyl-O-5-or 6-membered monocyclic heteroaryl, -C₁₋₄ alkyl-8- to 10-membered bicyclic heteroaryl, -C₁₋₄ alkyl-O-8- to 10-membered bicyclic heteroaryl, -C₁₋₄ alkyl-NR^{a}R^{b}, -C₁₋₄ alkyl-C(=O)-NR^{a}R^{b}, -C₁₋₄ alkyl-NR^{d}-C(=O)-R^{c}, -C₁₋₄ alkyl-NR^{d}-C(=O)-NR^{a}R^{b}, -C₁₋₄ alkyl-S(=O)₂-R^{c}, -C₁₋₄ alkyl-NR^{d}-S(=O)₂-R^{c}, -C₁₋₄ alkyl-S(=O)₂-NR^{a}R^{b}, -C₁₋₄ alkyl-NR^{d}-S(=O)₂-NR^{a}R^{b}, -C(=O)-C₁₋₆ alkyl, -C(=O)-C₃₋₂₀ cycloalkyl, -C(=O)-3- to 20-membered heterocyclyl, -C(=O)-C₆₋₁₄ aryl, -C(=O)-5- or 6-membered monocyclic heteroaryl, -C(=O)-8- to 10-membered bicyclic heteroaryl, -C(=O)-NR^{a}R^{b}, -NR^{d}-C(=O)-R^{c}, -NR^{d}-C(=O)-NR^{a}R^{b}, -S(=O)₂-R^{c}, -NR^{d}-S(=O)₂-R^{c}, -S(=O)₂-NR^{a}R^{b}, -NR^{d}-S(=O)₂-NR^{a}R^{b}, -NR^{a}R^{b}, -OR^{c}, -C₁₋₄ alkyl-P(=O)-(C₁₋₆ alkyl)₂, -P(=O)-(C₁₋₆ alkyl)₂;
the C₁₋₆ alkyl, the C₁₋₆ alkoxy, the C₂₋₆ alkenyl, and the C₂₋₆ alkynyl are each independently optionally substituted with 1, 2, or 3 group(s) selected from halogen, deuterium, cyano, or hydroxyl; the C₃₋₂₀ cycloalkyl, the 3- to 20-membered heterocyclyl, the C₆₋₁₄ aryl, the 5- or 6-membered monocyclic heteroaryl, and the 8- to 10-membered bicyclic heteroaryl are each independently optionally substituted with 1, 2, 3, or 4 group(s) selected from group S1; the 3- to 20-membered heterocyclyl, the 5- or 6-membered monocyclic heteroaryl, and the 8- to 10-membered bicyclic heteroaryl each independently contains 1, 2, 3, or 4 heteroatom(s) independently selected from N, O, and S as ring atoms; or
R^{4'} and R^{4"} together with the carbon atom to which the R^{4'} and R^{4"} are attached form a carbonyl group (C=O);
wherein R^{a}, R^{b}, R^{c}, R^{d}, and group S1 are each as defined in claim 7.

13. The compound, or the pharmaceutically acceptable salt, the stereoisomer, the solvate or the prodrug thereof of claim 12, wherein the compound is a compound of formula (IC4a), a compound of formula (IC4b), a compound of formula (IC4c), a compound of formula (IC4d), a compound of formula (IC4e), or a compound of formula (IC4f); 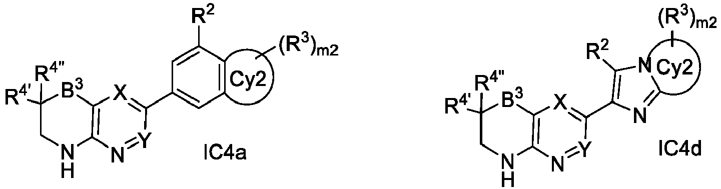 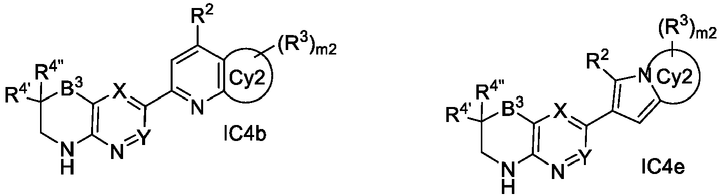 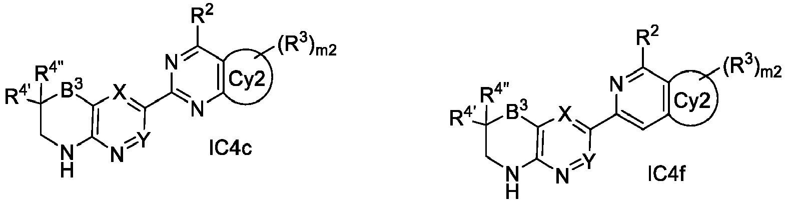 in each formula, X, Y, R², 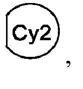 R³, m2, B³, R^{4'}, and R^{4"} are each independently as defined in claim 12.

14. The compound, or the pharmaceutically acceptable salt, the stereoisomer, the solvate or the prodrug thereof of claim 7, wherein the compound is selected from the group consisting of

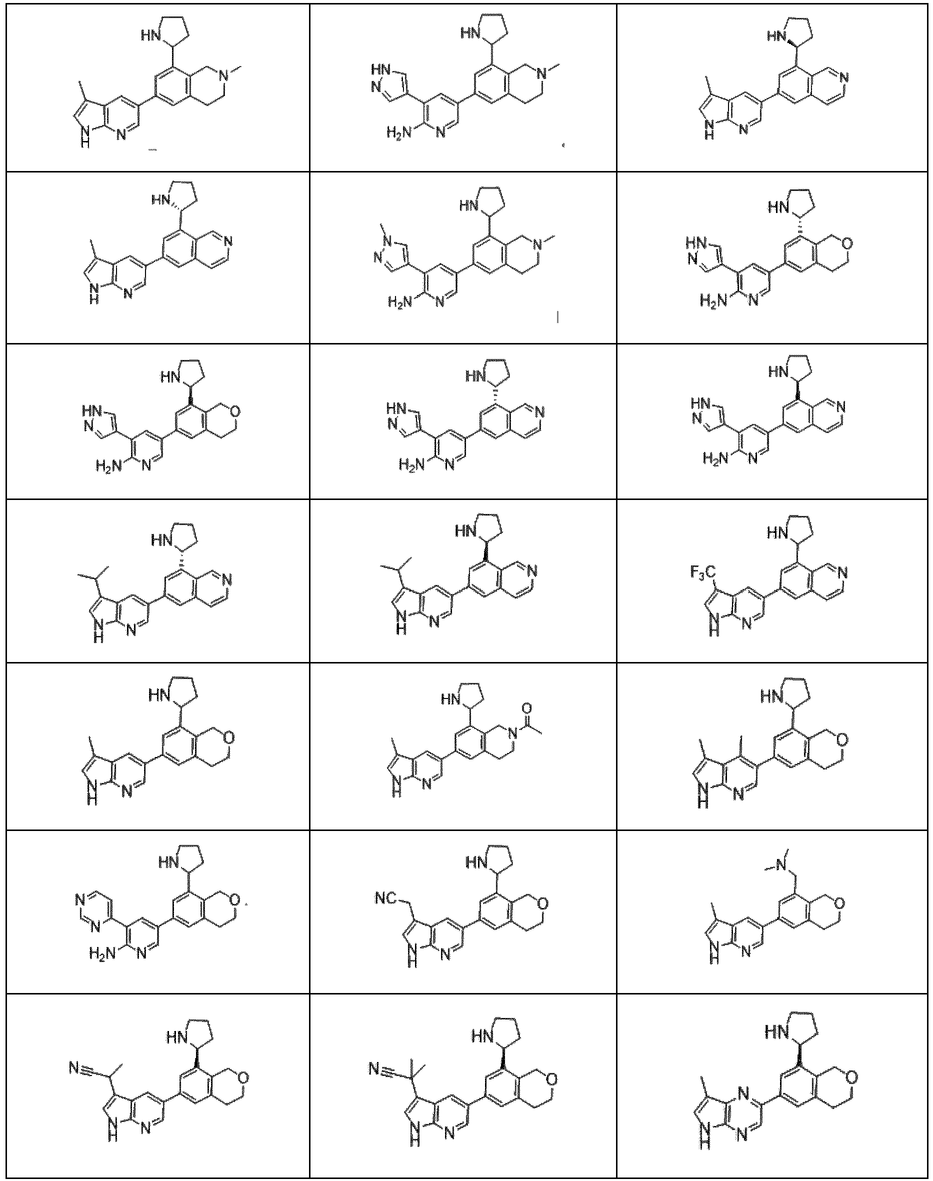

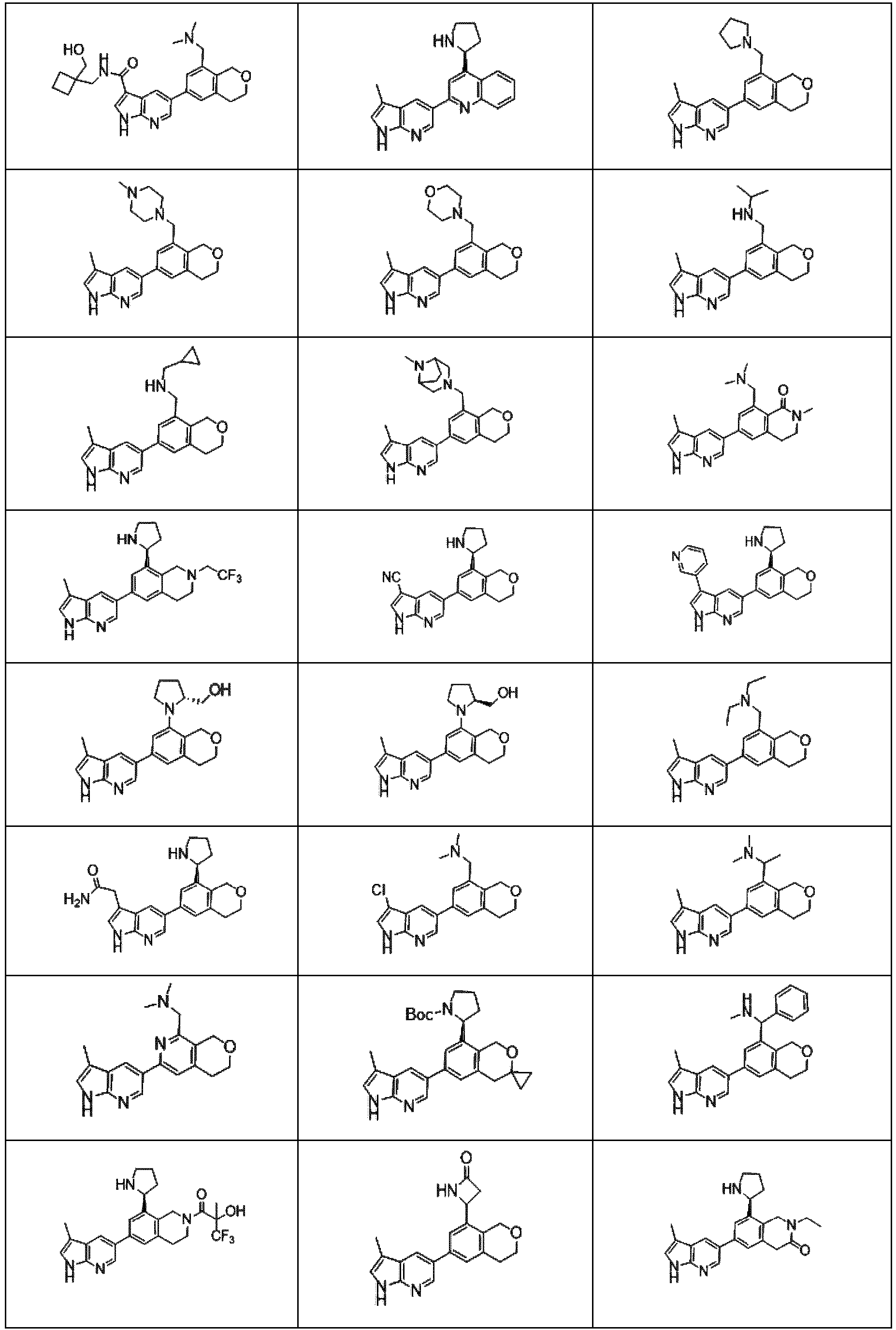

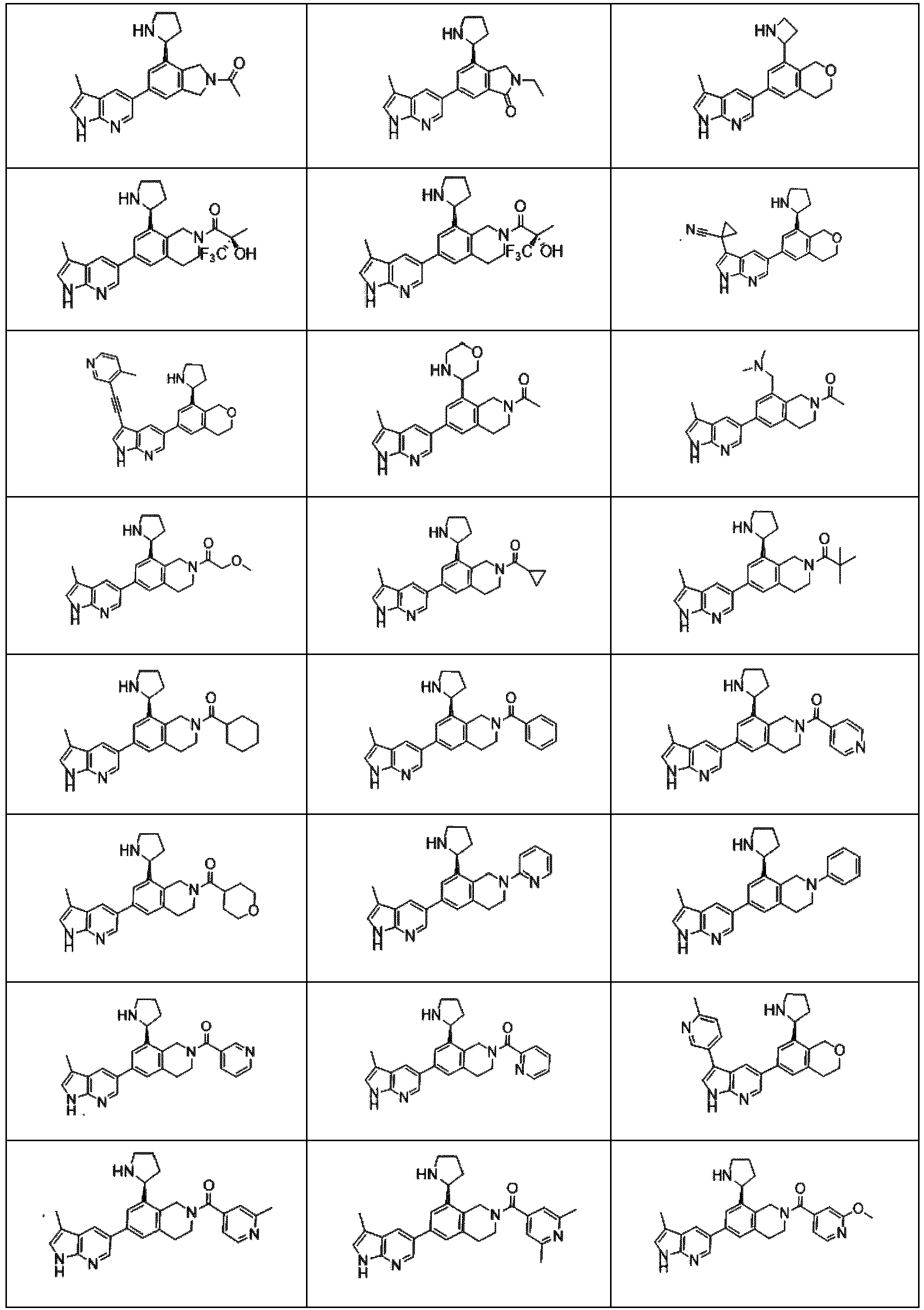

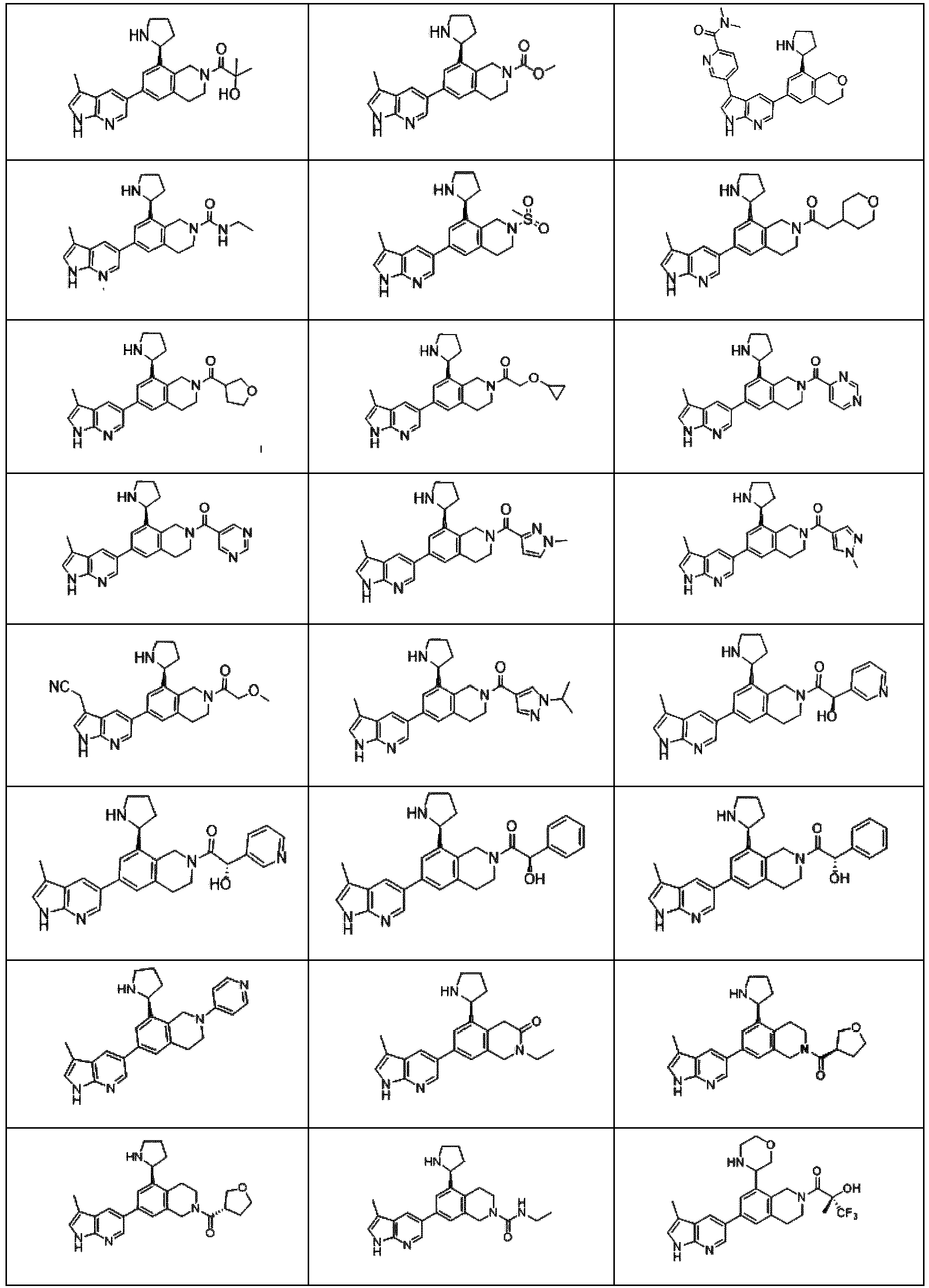

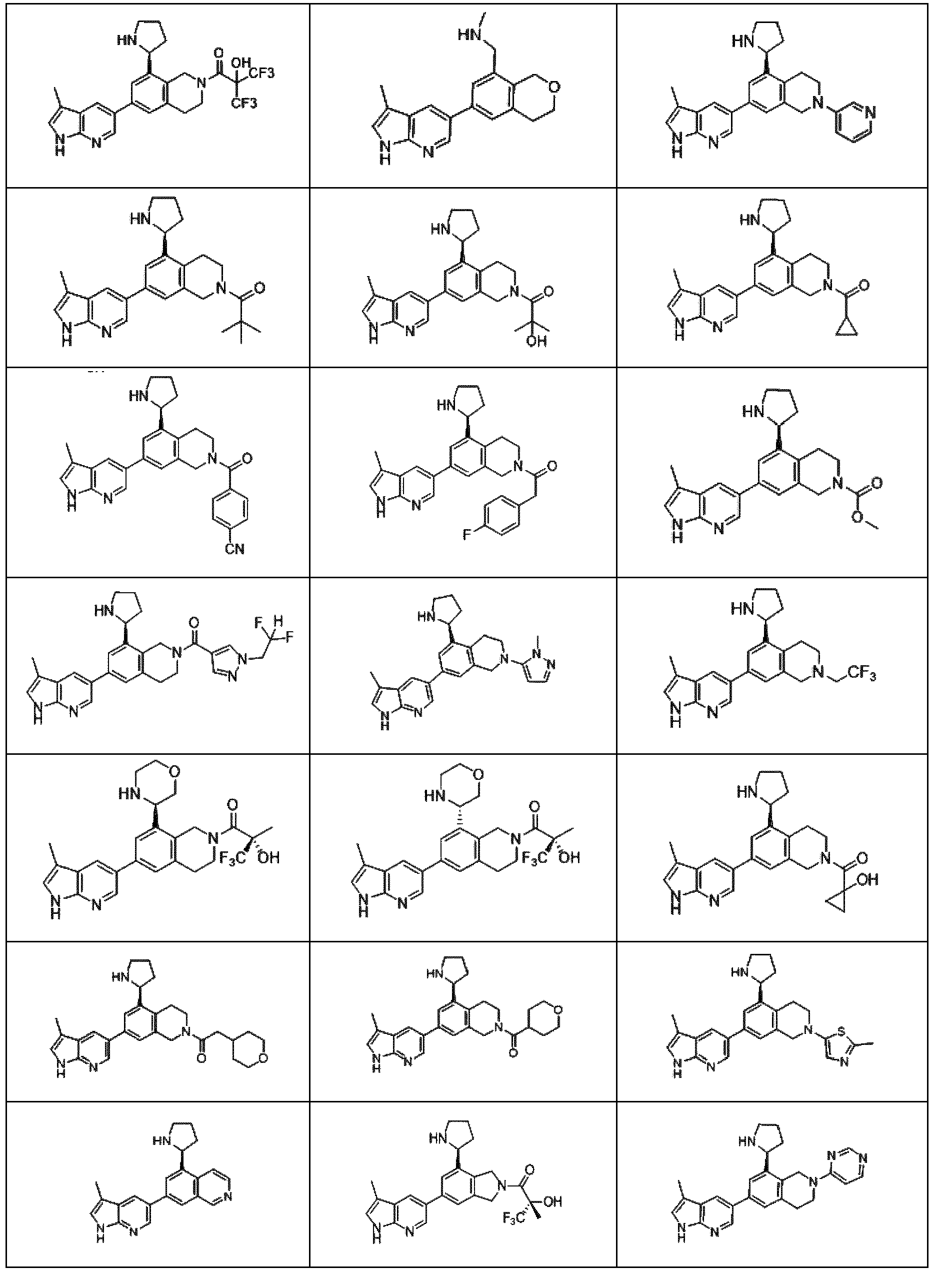

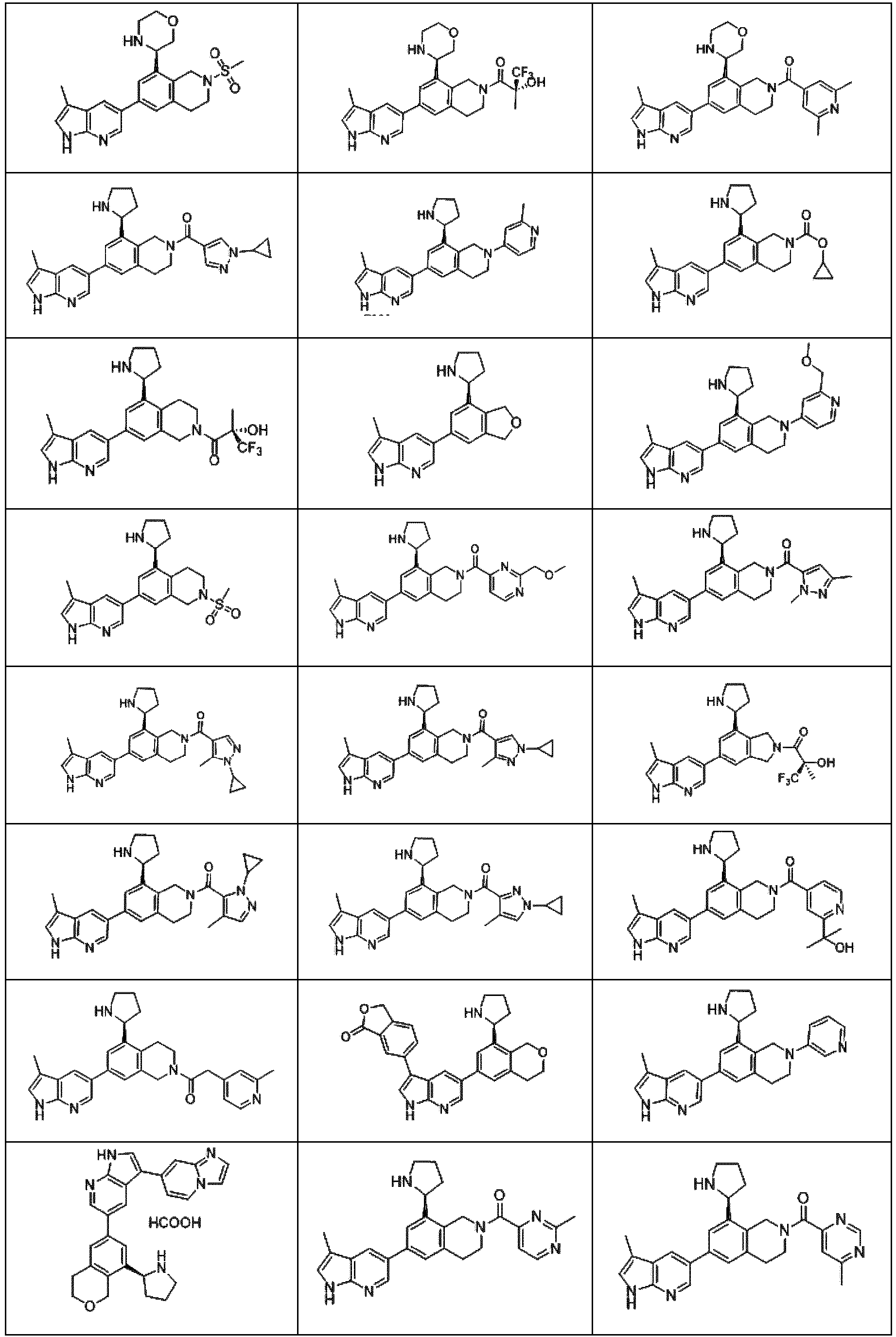

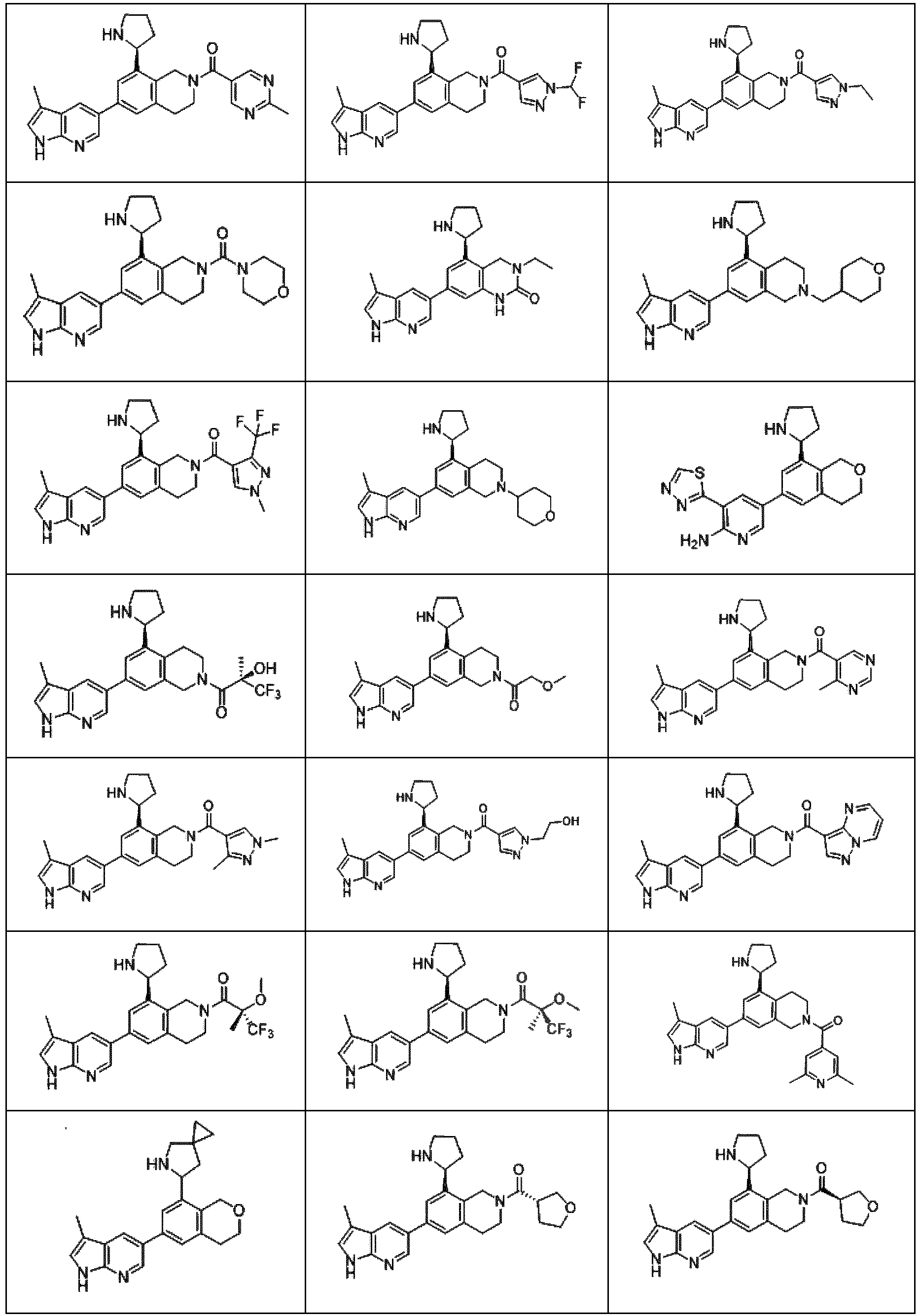

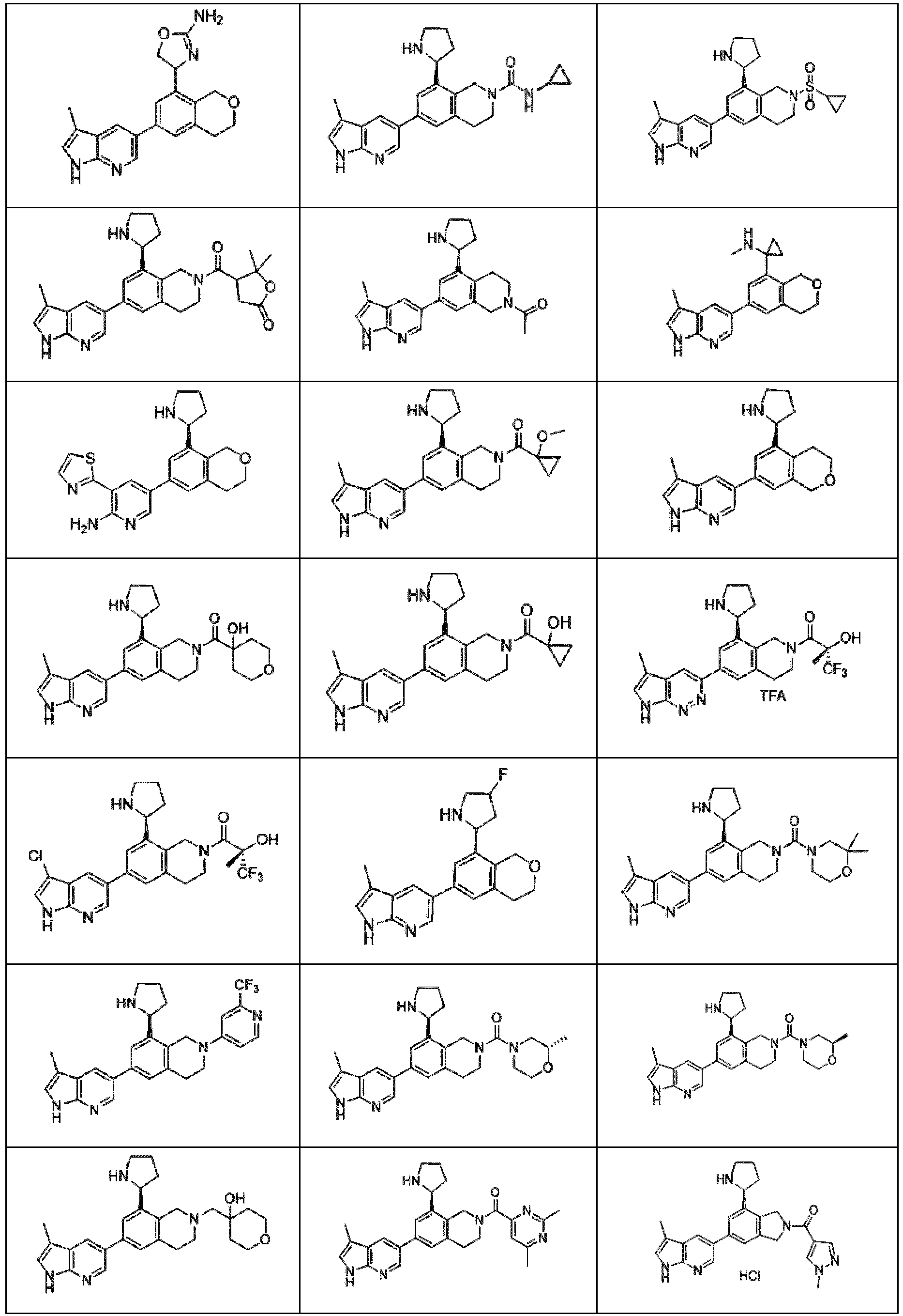

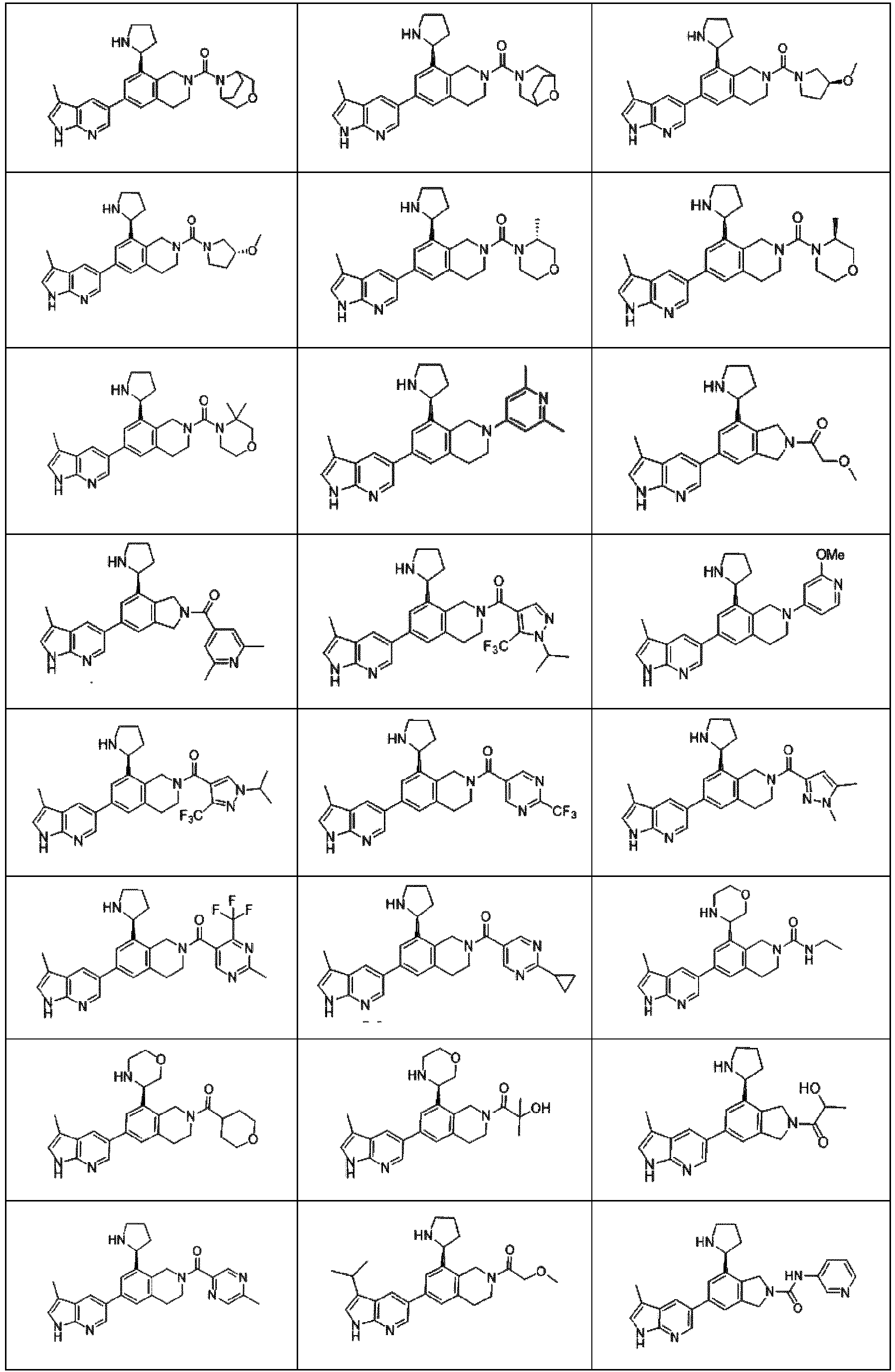

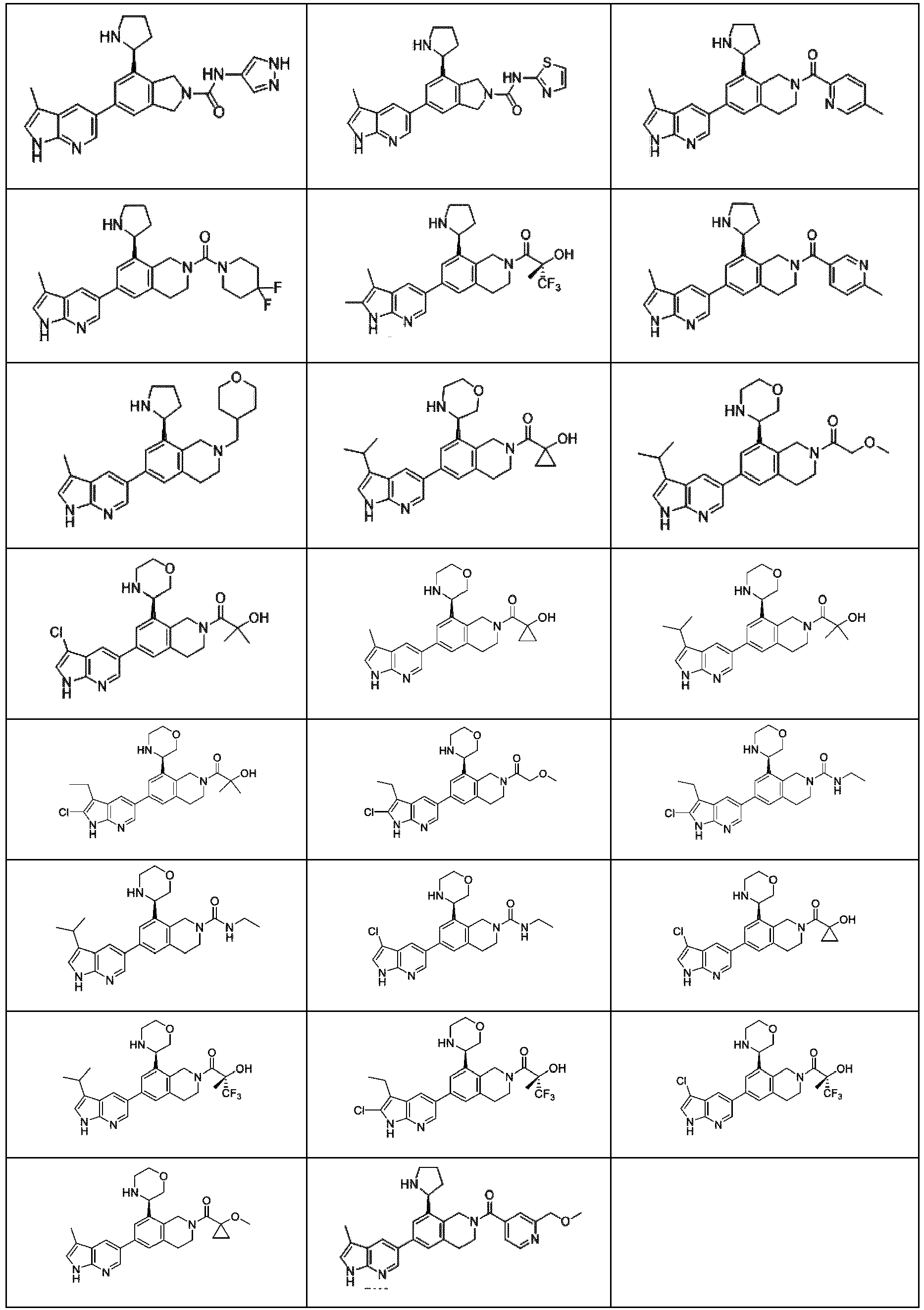

15. A method for preparing the compound, or the pharmaceutically acceptable salt, the stereoisomer, the solvate or the prodrug thereof of claim 7, comprising: 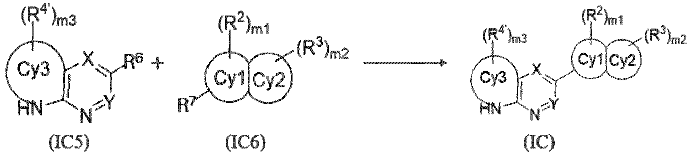
wherein X, Y, R², R³, R^{4'}, 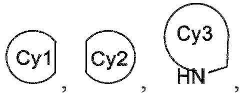 m1, m2, and m3 are as defined in claim 7;
R⁶ and R⁷ are different groups optionally selected from -CHO, -COCH₂, -COOC₂H₅, -OCH₃, -CN, -NO₂, -F, -Cl, Br, a boronic acid group, or a borate group;
the compound of formula (IC), or a pharmaceutically acceptable salt, a stereoisomer, a solvate or a prodrug thereof, is prepared by a Suzuki reaction of R⁶ in a compound of formula (ICS) with R⁷ in a compound of formula (IC6) in the presence of a palladium catalyst, or by a Suzuki reaction of a compound without the -R⁴ group in the compound of formula (ICS) with a compound without the -R² and/or -R³ groups in the compound of formula (IC6) to obtain an intermediate free of the -R², -R³ and -R⁴ groups, followed by a substitution reaction of the intermediate with a compound having the -R², -R³ and -R⁴ groups.

16. A pharmaceutical composition comprising the compound, or the pharmaceutically acceptable salt, the stereoisomer, the solvate or the prodrug thereof of any one of claims 1 to 14; and a pharmaceutically acceptable carrier.

17. Use of the compound, or the pharmaceutically acceptable salt, the stereoisomer, the solvate or the prodrug thereof of any one of claims 1 to 14, or the pharmaceutical composition of claim 16, in the preparation of a medicament for preventing and/or treating a disease or condition associated with HPK1 activity.

18. The use of claim 17, wherein the diseases or conditions associated with HPK1 activity are cancer.
